# EUROPÄISCHE PATENTANMELDUNG

(11) **EP 2 388 258 A1**
(43) Veröffentlichungstag der Anmeldung: **23.11.2011**
(21) Anmeldenummer: 11006751.9
(22) Anmeldetag: 19.10.2006
(51) Int. Cl.: C07D 401/04, C07D 401/12, C07D 409/04, C07D 213/74, C07D 213/40, C07D 213/64, C07D 295/12, C07C 311/08, A61K 31/444, A61P 29/00

(54) **Neue Vanilloidrezeptor-Liganden und ihre Verwendung zur Herstellung von Arzneimitteln**

(30) Priorität: 19.10.2005 DE 102005050408; 19.10.2005 US 727859 P; 18.11.2005 DE 102005055486
(62) Teilanmeldung aus: 06806372.6
(71) Anmelder: Grünenthal GmbH, 52078 Aachen (DE)
(72) Erfinder: Frank, Robert, Dr., 52070 Aachen (DE); Bahrenberg, Gregor, Dr., 52156 Monschau-Konzen (DE); de Vry, Jean, Dr., 5223 Stolberg (DE); Christoph, Thomas, Dr., 52080 Aachen (DE); Saunders, Derek John, Dr., 52072 Aachen (DE); Schiene, Klaus, Dr., 41363 Jüchen (DE); Sundermann, Bernd, Dr., 61381 Friedrichsdorf (DE); Ryu, Hyung, Chul, Ansan-Si, Gyenggi-do 425-838 (KR); Lee, Jeewoo, Prof. Dr., Seoul 151-742 (KR)
(74) Vertreter: Bülle, Jan

(57) **Zusammenfassung**

Die vorliegende Erfindung betrifft neue Vanilloid-Rezeptor-Liganden, Verfahren zu ihrer Herstellung, Arzneimittel enthaltend diese Verbindungen und die Verwendung dieser Verbindungen zur Herstellung von Arzneimitteln.

## Beschreibung

Die vorliegende Erfindung betrifft neue Vanilloid-Rezeptor-Liganden, Verfahren zu ihrer Herstellung, Arzneimittel enthaltend diese Verbindungen und die Verwendung dieser Verbindungen zur Herstellung von Arzneimitteln.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Ein geeigneter Ansatzpunkt zur Behandlung von Schmerz; insbesondere von Schmerz ausgewählt aus der Gruppe bestehend aus akutem Schmerz, chronischem Schmerz, neuropathischem Schmerz und visceralem Schmerz, besonders bevorzugt von neurophatischem Schmerz; stellt der Vanilloid-Rezeptor vom Subtyp 1 (VR1/TRPV1) dar, der häufig auch als Capsaicin-Rezeptor bezeichnet wird. Dieser Rezeptor wird u.a. durch Vanilloide wie z.B. Capsaicin, Hitze und Protonen stimuliert und spielt eine zentrale Rolle bei der Schmerzentstehung. Darüber hinaus ist er für eine Vielzahl weiterer physiologischer und pathophysiologischer Prozesse von Bedeutung wie beispielsweise Migräne; Depressionen; neurodegenerativen Erkrankungen; kognitiven Erkrankungen; Angstzuständen; Epilepsie; Husten; Diarrhöe; Pruritus; Entzündungen; Störungen des kardiovaskulären Systems; Störungen der Nahrungsaufnahme; Medikamentenabhängigkeit; Medikamentenmißbrauch und insbesondere Harninkontinenz.

Eine Aufgabe der vorliegenden Erfindung bestand daher darin, neue Verbindungen zur Verfügung zu stellen, die sich insbesondere als pharmakologische Wirkstoffe in Arzneimitteln eignen, vorzugsweise in Arzneimitteln zur Behandlung von Störungen oder Krankheiten, die zumindest teilweise durch Vanilloid-Rezeptoren 1 (VR1/TRPV1-Rezeptoren) vermittelt werden.

Überraschenderweise wurde nun gefunden, dass die substituierten Verbindungen der nachstehend angegebenen allgemeinen Formeln A und I eine ausgezeichnete Affinität zum Vanilloid-Rezeptor vom Subtyp 1 (VR1/TRPV1-Rezeptor) aufweisen und sich daher insbesondere zur Prophylaxe und/oder Behandlung von Störungen oder Krankheiten eignen, die zumindest teilweise durch Vanilloid-Rezeptoren 1 (VR1/TRPV1) vermittelt werden. Ebenfalls verfügen die substituierten Verbindungen der nachstehend angegebenen allgemeinen Formeln A und I über eine antientzündliche Aktivität.

Ein Gegenstand der vorliegenden Erfindung sind daher substituierte Verbindungen der allgemeinen Formel A, 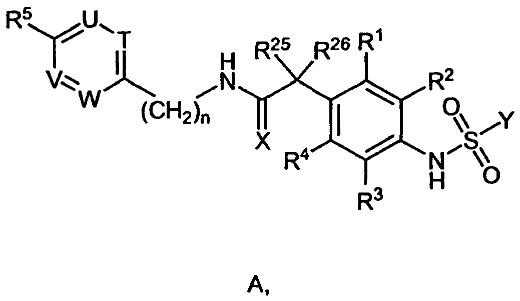 worin
X für O, S oder N-C≡N steht;
Y für -NH₂; -NHR³⁰; -NR³¹R³² oder für einen linearen oder verzweigten, gesättigten oder ungesättigten, unsubstituierten oder wenigstens einfach substituierten aliphatischen C₁₋₁₀ Rest steht;
n für 0, 1, 2, 3 oder 4 steht;
R¹, R², R³ und R⁴, unabhängig voneinander, jeweils für H; F; Cl; Br; I; -SF₅; -NO₂; -CF₃; -CN; -NH₂; -OH; -SH; -C(=O)-NH₂; -S(=O)₂-NH₂-; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³; -S(=O)-R²⁴; - S(=O)₂-R²⁴ oder für einen linearen oder verzweigten, gesättigten oder ungesättigten, unsubstituierten oder wenigstens einfach substituierten aliphatischen C₁₋₁₀ Rest stehen;
R⁵ für H; F; Cl; Br; I; -SF₅; -NO₂; -CF₃; -CF₂Cl; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; - C(=O)-OR²²; -C(=O)-R²³, -S(=O)₂-R²⁴; -S(=O)-R²⁴;
für einen linearen oder verzweigten, gesättigten oder ungesättigten, unsubstituierten oder wenigstens einfach substituierten aliphatischen C₁₋₁₀ Rest;
oder für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest steht, der jeweils über ein Kohlenstoffatom im Ring des cycloaliphatischen Restes an das Grundgerüst gebunden ist;
T für C-R⁶ und U für C-R⁷ und V für N und W für C-R⁸
oder
T für C-R⁶ und U für N und V für C-R⁹ und W für C-R⁸
oder
T für N und U für C-R⁷ und V für C-R⁹ und W für C-R⁸
oder
T für N und U für N und V für C-R⁹ und W für C-R⁸
oder
T für N und U für C-R⁷ und V für N und W für C-R⁸
oder
T für C-R⁶ und U für N und V für N und W für C-R⁸ oder
T für C-R⁶ und U für C-R⁷ und V für C-R⁹ und W für C-R¹⁰
steht;
R⁶ und R⁷, unabhängig voneinander, jeweils für H; F; Cl; Br; I; -SF₅; -NO₂-; -CF₃; -CF₂Cl; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; - C(=O)-NR¹⁷R¹⁸; -S(=O)₂-NHR¹⁹; -S(=O)₂-NR²⁰R²¹; -C(=O)-OR²²; -C(=O)-R²³, -S(=O)-R²⁴; -S(=O)₂-R²⁴; für einen linearen oder verzweigten, gesättigten oder ungesättigten, unsubstituierten oder wenigstens einfach substituierten aliphatischen C₁₋₁₀ Rest;
oder für einen unsubstituierten oder wenigstens einfach substituierten 6- oder 10-gliedrigen Aryl-Rest, der über eine lineare oder verzweigte, unsubstituierte oder wenigstens einfach substituierte C₁₋₆-Alkylen-Gruppe oder C-₂₋₆-Alkenylen-Gruppe oder C₂₋₆-Alkinylen-Gruppe gebunden sein kann, stehen;
R⁸ für H; F; Cl; Br, I; -SF₅; -NO₂; -CF₃; -CF₂Cl; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; - C(=O)-OR²²; -C(=O)-R²³; -S(=O)-R²⁴; -S(=O)₂-R²⁴; -C(=NH)-NH₂; -C(=NH)-NH-R²⁷; - N=C(NH₂)₂; -N=C(NHR²⁸)(NHR²⁹);
für einen linearen oder verzweigten, gesättigten oder ungesättigten, unsubstituierten oder wenigstens einfach substituierten aliphatischen C₁₋₁₀ Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder C-₂₋₆-Alkenylen-Gruppe oder C₂₋₆-Alkinylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder C-₂₋₆-Alkenylen-Gruppe oder C₂₋₆-Alkinylen-Gruppe gebunden sein kann, steht;
R⁹ für H; F; Cl; Br; I; -SF₅; -NO₂; -CF₃, -CF₂Cl; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; - C(=O)-OR²²; -C(=O)-R²³; -S(=O)-R²⁴; -S(=O)₂-R²⁴ oder für einen linearen oder verzweigten, gesättigten oder ungesättigten, unsubstituierten oder wenigstens einfach substituierten aliphatischen C₁₋₁₀ Rest steht;
R¹⁰ für -SF₅; -NO₂; -CF₃; -CF₂Cl; -CN; -NH₂; -OH; -SH; -C(=O)-NH₂; -S(=O)₂-NH₂; - C(=O)-NH-OH; -C(=O)-OH; -C(=O)-H; -S(=O)₂-OH; -NHR¹¹; -NR¹²R¹³; -OR¹⁴; -SR¹⁵; - C(=O)-NHR¹⁶; -C(=O)-NR¹⁷R¹⁸; -S(=O)₂-NHR¹⁹; -S(=O)₂-NR²⁰R²¹; -C(=O)-OR²²; - C(=O)-R²³; -S(=O)-R²⁴; -S(=O)₂-R²⁴; -C(=NH)-NH₂; -C(=NH)-NH-R²⁷; -N=C(NH₂)₂; - N=C(NHR²⁸)(NHR²⁹);
für einen linearen oder verzweigten, gesättigten oder ungesättigten aliphatischen C₁₋₁₀ Rest, der jeweils mit ggf. 1, 2, 3, 4, 5, 6, 7, 8 oder 9 Substituenten unabhängig voneinander ausgewählt aus der Gruppe bestehend aus -CN, -NO₂, -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), -OCF₃ und - SCF₃ substituiert ist;
für einen unsubstituierten C₂₋₁₀ Alkenyl-Rest oder einen unsubstituierten C₂₋₁₀-Akinyl-Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe gebunden sein kann, steht;
R¹¹, R¹², R¹³, R¹⁴, R¹⁵, R¹⁶, R¹⁷, R¹⁸, R¹⁹, R²⁰, R²¹, R²², R²³, R²⁴, R²⁷, R²⁸ und R²⁹, unabhängig voneinander, jeweils
für einen linearen oder verzweigten, gesättigten oder ungesättigten, unsubstituierten oder wenigstens einfach substituierten aliphatischen C₁₋₁₀ Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder 2- bis 6-gliedrige Heteroalkylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder 2- bis 6-gliedrige Heteroalkylen-Gruppe gebunden sein kann, stehen;
oder
R¹² und R¹³ jeweils zusammen mit dem sie verbindenden Stickstoffatom als Ringglied einen gesättigten oder ungesättigten, unsubstituierten oder wenigstens einfach substituierten 4-, 5-, 6-, 7-, 8- oder 9-gliedrigen, ggf. wenigstens ein weiteres Heteroatom als Ringglied aufweisenden heterocycloaliphatischen Rest bilden, der mit einem gesättigten oder ungesättigten, unsubstituierten oder wenigstens einfach substituierten mono- oder polyzyklischen Ringsystem kondensiert sein kann;
und
R²⁵ und R²⁶, unabhängig voneinander, jeweils für einen Wasserstoff-Rest;
für einen linearen oder verzweigten, gesättigten oder ungesättigten, unsubstituierten oder wenigstens einfach substituierten aliphatischen C₁₋₁₀ Rest;
für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder C-₂₋₆-Alkenylen-Gruppe oder C₂₋₆-Alkinylen-Gruppe gebunden sein kann;
oder für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest stehen;
mit der Maßgabe, dass R²⁵ und R²⁶ nicht jeweils für einen Wasserstoff-Rest stehen;
oder
R²⁵ und R²⁶ jeweils zusammen mit dem sie verbindenden Kohlenstoffatom als Ringglied einen gesättigten oder ungesättigten, unsubstituierten oder wenigstens einfach substituierten 3-, 4-, 5- oder 6-gliedrigen cycloaliphatischen Rest bilden;
und R³⁰, R³¹ und R³², unabhängig voneinander, jeweils für einen
für einen linearen oder verzweigten, gesättigten oder ungesättigten, unsubstituierten oder wenigstens einfach substituierten aliphatischen C₁₋₁₀ Rest stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Sofern nicht anders angegeben, können die vorstehend genannten aliphatischen C₁₋₁₀ Reste vorzugsweise ggf. jeweils mit 1, 2, 3, 4, 5, 6, 7, 8 oder 9 Substituenten unabhängig voneinander ausgewählt aus der Gruppe bestehend aus -C(=O)-O-C₁₋₅-Alkyl, -O-C(=O)-C₁₋₅-Alkyl, -O-Phenyl, Phenyl, F, Cl, Br, I, -CN, -NO₂, -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), -OCF₃ und - SCF₃ substituiert sein.

Bevorzugt können die vorstehend genannten C₁₋₆-Alkylen-Gruppen, C₂₋₆-Alkenylen-Gruppen und C₂₋₆-Alkinylen-Gruppen ggf. jeweils mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, -O(C₁₋₅-Alkyl), -S(C₁₋₅-Alkyl), -NH(C₁₋₅-Alkyl), - N(C₁₋₅-Alkyl)(C₁₋₅Alkyl), -OCF₃ und -SCF₃ substituiert sein.

Der Begriff "Heteroalkylen" bezeichnet eine wie vorstehend beschriebene AlkylenKette, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroalkylen-Gruppen können bevorzugt 1, 2 oder 3 Heteroatom(e), besonders bevorzugt ein Heteroatom, unabhängig voneinander ausgewählt aus der Gruppe bestehend aus Sauerstoff, Schwefel und Stickstoff (NH) als Kettenglied(er) aufweisen. Heteroalkylen-Gruppen können bevorzugt 2- bis 6-gliedrig, besonders bevorzugt 2- oder 3-gliedrig, sein.

Beispielhaft seien Heteroalkylen-Gruppen wie -CH₂-CH₂-O-CH₂-, -CH₂-CH(CH₃)-O-CH₂-, -(CH₂)-O-, -(CH₂)₂-O-, -(CH₂)₃-O-, -(CH₂)₄-O-, -O-(CH₂)-, -O-(CH₂)₂-, -O-(CH₂)₃-, -O-(CH₂)₄-, -C(C₂H₅)(H)-O-, -O-C(C₂H₅)(H)-, -CH₂-O-CH₂-, -CH₂-S-CH₂-, -CH₂-NH-CH₂-, -CH₂-NH- und -CH₂-CH₂-NH-CH₂-CH₂ genannt.

Bevorzugt können die 2- bis 6-gliedrigen Heteroalkylen-Gruppen ggf. jeweils mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), -OCF₃ und -SCF₃ substituiert sein.

Bevorzugt können die vorstehend genannten (hetero)cycloaliphatischen Reste ggf. jeweils mit 1, 2, 3, 4 oder 5 Substituenten unabhängig voneinander ausgewählt aus der Gruppe bestehend aus -C₁₋₆-Alkylen-OH, =CH₂, -O-C₁₋₅₋Akylen-Oxetanyl, -C₁₋₅-Alkylen-O-C₁₋₅-Alkylen-Oxetanyl, -CH₂-NH-C₁₋₅-Alkyl, -CH₂-N(C₁₋₅-Alkyl)₂, -N[C(=O)-C₁₋₅-Alkyl]-Phenyl, -CH₂-O-C₁₋₅-Alkyl, Oxo (=O), Thioxo (=S), F, Cl, Br, I, -CN, -CF₃, - SF₅, -OH, -O-C₁₋₅-Alkyl, -O-C(=O)-C₁₋₅-Alkyl, -NH₂, -NO₂, -O-CF₃, -S-CF₃, -SH, -S-C₁-₅-Alkyl, -C₁₋₅-Alkyl, -C(=O)-C₁₋₅-Alkyl, -C(=O)-OH, -C(=O)-O-C₁₋₅-Alkyl, -NH-C₁₋₅-Alkyl, -N(C₁₋₅-Alkyl)₂, (4,5)-Dihydroisoxazolyl, Thiazolyl, (1,2,5)-Thiadiazolyl, Thiophenyl, Phenethyl, -NH-Phenyl, -N(C₁₋₅-Alkyl)Phenyl, Cyclohexyl, Cyclopentyl, Piperidinyl, Pyrrolidinyl, -(CH₂)-Pyridinyl, Pyridinyl, -O-Phenyl, -O-Benzyl, Phenyl und Benzyl substituiert sein, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N[C(=O)-C₁₋₅-Alkyl]-Phenyl, -NH-Phenyl, -N(C₁₋₅-Alkyl)-Phenyl, -(CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -C₁₋₅-Alkyl, -O-C₁₋₅-Alkyl, -O-CF₃, -S-CF₃, Phenyl und -O-Benzyl substituiert sein kann.

Ebenfalls bevorzugt können die vorstehend genannten (hetero)cycloaliphatischen Reste jeweils ggf. 1, 2 oder 3 (weitere) Heteroatom(e) unabhängig voneinander ausgewählt aus der Gruppe bestehend aus Sauerstoff, Stickstoff und Schwefel aufweisen.

Vorzugsweise können die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-C₁₋₅-Alkyl, -C(=O)-OH, -C(=O)-O-C₁₋₅-Alkyl, -NH-C₁₋₅-Alkyl, -N(C₁-₅-Alkyl)₂, -O-Phenyl, -O-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, - C₁₋₅-Alkyl, -O-C₁₋₅-Alkyl, -O-CF₃, -S-CF₃, Phenyl und -O-Benzyl substituiert sein kann.

Vorzugsweise sind die Ringe der vorstehend genannten mono- oder polyzyklischen Ringsysteme jeweils 5-, 6- oder 7-gliedrig und können jeweils ggf. 1, 2, 3, 4 oder 5 Heteroatom(e) als Ringglied(er) aufweisen, die unabhängig voneinander aus der Gruppe bestehend aus Sauerstoff, Stickstoff und Schwefel ausgewählt sind.

Ebenfalls bevorzugt können die vorstehend genannten Aryl- oder Heteroaryl-Reste ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -NH-C₁₋₅-Alkyl, -N(C₁₋₅-Alkyl)₂, -NH-S(=O)₂-C₁₋₅-Alkyl, -NH-C(=O)-O-C₁₋₅-Alkyl, - C(=O)-H, -C(=O)-C₁₋₅-Alkyl, -C(=O)-NH₂, -C(=O)-NH-C₁₋₅-Alkyl, -C(=O)-N-(C₁₋₅-Alkyl)₂, -O-Phenyl, -O-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann.

Ebenfalls bevorzugt weisen die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) auf.

Sofern einer oder mehrere der vorstehend genannten Reste für einen gesättigten oder ungesättigten C₁₋₁₀ aliphatischen Rest, d.h. für einen C₁₋₁₀-Alkyl-, C₂₋₁₀-Alkenyl-oder C₂₋₁₀-Alkinyl-Rest, stehen, kann dieser bevorzugt mit ggf. 1, 2, 3, 4, 5, 6, 7, 8 oder 9 Substituenten unabhängig voneinander ausgewählt aus der Gruppe bestehend aus -O-Phenyl, F, Cl, Br, I, -CN, -NO₂, -OH, -NH₂, -SH, -O(C₁₋₅-Alkyl), - S(C₁₋₅-Alkyl), -NH(C₁₋₅-Alkyl), -N(C₁₋₅-Alkyl)(C₁₋₅-Alkyl), -C(=O)-O-C₁₋₅-Alkyl, -OCF₃ und -SCF₃ substituiert sein. C₂₋₁₀-Alkenyl-Reste weisen wenigstens eine, vorzugsweise 1, 2, 3 oder 4 C-C-Doppelbindungen und C₂₋₁₀-Alkinyl-Reste wenigstens eine, vorzugsweise 1, 2, 3 oder 4 C-C-Dreifachbindungen auf.

Bevorzugt sind Alkyl-Reste ausgewählt aus der Gruppe bestehend aus Methyl, Ethyl, n-Propyl, Isopropyl, n-Butyl, sek-Butyl, Isobutyl, tert-Butyl, n-Pentyl, 3-Methyl-but-1-yl, 2-Pentyl, 3-Pentyl, sec-Pentyl, neo-Pentyl, 4-Methyl-pent-1-yl, (3,3)-Dimethyl-but-1-yl, n-Hexyl, n-Heptyl, 2-Heptyl, 3-Heptyl, 4-Heptyl, n-Octyl, n-Nonyl, 2-Nonyl, 3-Nonyl, 4-Nonyl, 5-Nonyl und (2,6)-Dimethyl-hept-4-yl, die ggf. mit 1, 2, 3, 4, 5, 6, 7, 8 oder 9 Substituenten unabhängig voneinander ausgewählt aus der Gruppe bestehend aus - O-Phenyl, -O-C(=O)-CH₃, -O-C(=O)-C₂H₅, -O-C(=O)-CH(CH₃)₂, -O-C(=O)-C(CH₃)₃, - C(=O)-O-CH₃, -C(=O)-O-C₂H₅, -C(=O)-O-CH(CH₃)₂, -C(=O)-O-C(CH₃)₃, F, Cl, Br, I,-CN, -NO₂, -OH, -NH₂, -SH, -O-CH₃, -O-C₂H₅, -O-CH(CH₃)₂, -O-C(CH₃)₃, -S-CH₃, -S-C₂H₅, -S-CH(CH₃)₂, -S-C(CH₃)₃, -NH-CH₃, -NH-C₂H₅, -NH-C(CH₃)₃, -N(CH₃)₂, - N(C₂H₅)₂, -N(CH₃)(C₂H₅), -OCF₃ und -SCF₃ substituiert sein können.

Ebenfalls bevorzugt sind Alkenyl-Reste ausgewählt aus der Gruppe bestehend aus Vinyl, 1-Propenyl, 2-Propenyl, 1-Butenyl, 2-Butenyl, 3-Butenyl, 2-Methyl-propen-1-yl, 3-Methyl-but-2-en-1-yl, (3,3)-Dimethyl-but-1-enyl, 2-Methyl-buten-2-yl, 1-Pentenyl, 2-Pentenyl, 3-Pentenyl, 4-Pentenyl, 1-Hexenyl, 1-Heptenyl und 1-Octenyl, die ggf. mit 1, 2 oder 3 Substituenten unabhängig voneinander ausgewählt aus der Gruppe bestehend aus F, Cl, Br, I, -CN, -NO₂, -OH, -NH₂, -SH, -O-CH₃, -O-C₂H₅, -O-CH(CH₃)₂, -O-C(CH₃)₃, -S-CH₃, -S-C₂H₅, -S-CH(CH₃)₂, -S-C(CH₃)₃, -NH-CH₃, -NH-C₂H₅, -NH-C(CH₃)₃, -N(CH₃)₂, -N(C₂H₅)₂, -N(CH₃)(C₂H₅), -OCF₃ und -SCF₃ substituiert sein können.

Weiterhin bevorzugt sind Alkinyl-Reste ausgewählt aus der Gruppe bestehend aus (3,3)-Dimethyl-but-1-inyl, 4-Methyl-pent-1-inyl, 1-Hexinyl, Ethinyl, 1-Propinyl, 2-Propinyl, 1-Butinyl, 2-Butinyl, 3-Butinyl, 1-Pentinyl, 2-Pentinyl, 3-Pentinyl und 4-Pentinyl, die ggf. mit 1, 2 oder 3 Substituenten unabhängig voneinander ausgewählt aus der Gruppe bestehend aus F, Cl Br, I, -CN, -NO₂, -OH, -NH₂, -SH, -O-CH₃, -O-C₂H₅, -O-CH(CH₃)₂, -O-C(CH₃)₃, -S-CH₃, -S-C₂H₅, -S-CH(CH₃)₂, -S-C(CH₃)₃, -NH-CH₃, -NH-C₂H₅, -NH-C(CH₃)₃, -N(CH₃)₂, -N(C₂H₅)₂, -N(CH₃)(C₂H₅), -OCF₃ und -SCF₃ substituiert sein können.

Besonders bevorzugte ggf. substituierte C₁₋₁₀aliphatische Rest sind ausgewählt aus der Gruppe bestehend aus Methyl, -CF₃, -CHF₂, -CH₂F, -CF₂Cl, -CCl₂F, -CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃, -CH₂-N(CH₃)₂, -CH₂-N(C₂H₅)₂, -CH₂-N(CH₃)(C₂H₅), Ethyl, -CF₂-CH₃, -CHF-CF₂Cl, -CF₂-CFCl₂, -CFCl-CF₂Cl, -CFCl-CFCl₂, -CH₂-CH₂-NH₂, -CH₂-CH₂-OH, -CH₂-CH₂-SH, -CH₂-CH₂-NH-CH₃, -CH₂-CH₂-N(CH₃)₂, -CH₂-CH₂-N(C₂H₅)₂, -CH₂-CH₂-N(CH₃)(C₂H₅), -CH₂-CF₃, -C₂F₅, -CH₂-CCl₃, -CH₂-CBr₃, -CH₂-CH₂-CN, n-Propyl, - CH₂-CH₂-CH₂-OH, -CH₂-CH₂-CH₂-SH, -CH₂-CH₂-CH₂-NH₂, -CH₂-CH₂-CH₂-NH-CH₃, - CH₂-CH₂-CH₂-N(CH₃)₂, -CH₂-CH₂-CH₂-N(C₂H₅)₂, -CH₂-CH₂-CH₂-N(CH₃)(C₂H₅), -CH₂-CH₂-O-CH₃, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-Butyl, - CF₂-CF₂-CF₂-CF₃, -CH₂-CH₂-CH₂-CH₂-CN, -CH₂-CH₂-CH₂-CF₃, -CH₂-CH₂-CH₂-CH₂-CF₃, -CH₂-O-C(=O)-CH₃, -CH₂-O-C(=O)-C₂H₅, -CH₂-O-C(=O)-CH(CH₃)₂, -CH₂-O-C(=O)-C(CH₃)₃, -CH₂-C(=O)-O-CH₃, -CH₂-C(=O)-O-C₂H₅, -CH₂-C(=O)-O-C(CH₃)₃, - CH₂-CH₂-O-CH₃, -CH₂-CH₂-O-C₂H₅, -CH₂-CH₂-O-Phenyl, -CH₂-CH₂-CH₂-O-CH₃, sek-Butyl, Isobutyl, tert-Butyl, n-Pentyl, sec-Pentyl, neo-Pentyl, n-Hexyl, Vinyl, 1-Propenyl, 2-Propenyl, 1-Butenyl, 2-Butenyl, 3-Butenyl, 2-Methyl-buten-2-yl, (1,1,2)-Trifluor-1-butenyl, 1-Pentenyl, 2-Pentenyl, 3-Pentenyl, 4-Pentenyl, -CF=CF₂, - CCl=CCl₂, -CH₂-CF=CF₂, -CH₂-CCl=CCl₂, -C=C-I, -C=C-F und -C≡C-Cl.

Sofern einer oder mehrere der vorstehend genannten Substituenten für einen (hetero)cycloaliphatischen Rest stehen, der ggf. mit einem gesättigten oder ungesättigten, unsubstituierten oder wenigstens einfach substituierten mono- oder polyzyklischen Ringsystem kondensiert sein kann, kann dieser bevorzugt ausgewählt werden aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Oxetanyl, (1,2,3,6)-Tetrahydropyridinyl, Azepanyl, Azocanyl, Diazepanyl, Dithiolanyl, (1,3,4,5)-Tetrahydropyrido[4,3-b]indolyl, (3,4)-Dihydro-1H-isochinolinyl, (1,3,4,9)-Tetrahydro-[b]-carbolinyl und (1,3)-Thiazolidinyl.

Als geeignete (hetero)cycloaliphatische Reste, die unsubstituiert oder einfach oder mehrfach substituiert sein können, und mit einem mono- bzw. bizyklischem Ringsystem kondensiert sind, seien beispielhaft (4,5,6,7)-Tetrahydroisoxazolo[5,4-c]pyridinyl, (2,3)-Dihydro-1 H-indenyl, 3-Aza-bicyclo[3.1.1]heptyl, 3-Aza-bicyclo[3.2.1]octyl, 6-Aza-bicyclo[3.3.1]heptyl, 8-Aza-bicyclo[3.2.1]octyl, Isoindolyl, Indolyl, (1,2,3,4)-Tetrahydrochinolinyl, (1,2,3,4)-Tetrahydroisochinolinyl, (2,3)-Dihydro-1H-isoindolyl, (1,2,3,4)-Tetrahydronaphthyl, (2,3)-Dihydro-benzo[1.4]dioxinyl, Benzo[1.3]dioxolyl, (1,4)-Benzodioxanyl, (2,3)-dihydrothieno[3,4-b][1,4]dioxinyl, (3,4)-Dihydro-2H-benzo[1.4]oxazinyl, Octahydro-1 H-isoindolyl und Octahydro-pyrrolo[3,4-c]pyrrolyl genannt.

(Hetero)kycloaliphatische Reste können im Sinne der vorliegenden Erfindung mit einem weiteren (hetero)cycloaliphatischen Rest über ein gemeinsames Kohlenstoffatom in beiden Ringen einen spirozyklischen Rest bilden.

Als geeignete spirozyklischer Reste seien beispielsweise ein 6-Aza-spiro[2.5]octyl-Rest, 8-Azaspiro[4.5]decyl-Rest und ein 1-Oxa-2,8-diaza-spiro[4.5]dec-2-enyl-Rest genannt.

Besonders bevorzugt können die (hetero)cycloaliphatischen Reste ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, - S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH -C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), - CH₂-OH, -CH₂-CH₂-OH, =CH₂, -CH₂-O-CH₂-Oxetanyl, -O-CH₂-Oxetanyl, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, -NH-Phenyl, -N(CH₃)-Phenyl, - N(C₂H₅)-Phenyl, -N(C₂H₅)-Phenyl, -O-CH₂-CH₂-CH₂-CH₃, (4,5)-Dihydroisoxazolyl, Thiazolyl, (1,2,5)-Thiadiazolyl, Thiophenyl, Phenethyl, Cyclohexyl, Cyclopentyl, Piperidinyl, Pyrrolidinyl, -O-C(=O)-CH₃, -O-C(=O)-C₂H₅, -O-C(=O)-C(CH₃)₃, -(CH₂)-Pyridinyl, Pyridinyl, -O-Phenyl, -O-Benzyl, Phenyl und Benzyl substituiert sein, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N-[C(=O)-C₂H₅]-Phenyl, -N-[C(=O)-CH₃]-Phenyl, -NH-Phenyl, -N(CH₃)-Phenyl, -N(C₂H₅)-Phenyl, -(CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sek-Butyl, Isobutyl, tert-Butyl, -O-CH₃, -O-C₂H₅, -O-CH(CH₃)₂, -O-C(CH₃)₃, -O-CF₃, -S-CF₃, Phenyl und -O-Benzyl substituiert sein kann.

Sofern einer oder mehrere der vorstehend genannten Substituenten für einen Aryl-Rest stehen, kann dieser bevorzugt ausgewählt werden aus der Gruppe bestehend aus Phenyl und Naphthyl (1-Naphthyl und 2-Naphthyl).

Sofern einer oder mehrere der vorstehend genannten Substituenten für einen Heteroaryl-Rest stehen, kann dieser bevorzugt ausgewählt werden aus der Gruppe bestehend aus Tetrazolyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzoxazolyl, Benzisoxazolyl, Thiazol, Oxazolyl, Isoxazolyl, Pyridazinyl, Pyrazinyl, Pyrimidinyl, Indazolyl, Chinoxalinyl, Chinolinyl und Isochinolinyl.

Als geeignete Aryl- und Heteroaryl-Reste, die unsubstituiert oder einfach oder mehrfach substituiert sein können, und mit einem mono- bzw. bizyklischem Ringsystem kondensiert sind, seien beispielhaft Isoindolyl, Indolyl, (1,2,3,4)-Tetrahydrochinolinyl, (1,2,3,4)-Tetrahydroisochinolinyl, (2,3)-Dihydro-1H-isoindolyl, (1,2,3,4)-Tetrahydronaphthyl, (2,3)-Dihydro-benzo[1.4]dioxinyl, (2,3)-Dihydrothieno[3,4-b][1,4]dioxinyl, Benzo[1.3]dioxolyl und (1,4)-Benzodioxanyl genannt.

Besonders bevorzugt können die Aryl- oder Heteroaryl-Reste ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), - NH-S(=O)-CH₃, -NH-S(=O₂)-C₂H₅, -NH-S(=O)₂-CH(CH₃)₂, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann.

Unter einem mono- oder polyzyklischen Ringsystem werden im Sinne der vorliegenden Erfindung mono- oder polyzyklische Kohlenwasserstoffreste verstanden, die gesättigt oder ungesättigt sein und ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind. Ein solches mono- bzw. polyzyklisches Ringsystem kann beispielsweise mit einem Aryl-Rest oder einem Heteroaryl-Rest kondensiert (anneliert) sein.

Sofern ein polyzyklisches Ringsystem wie beispielsweise ein bizyklisches Ringsystem vorliegt, können die verschiedenen Ringe, jeweils unabhängig voneinander, einen unterschiedlichen Sättigungsgrad aufweisen, d.h. gesättigt oder ungesättigt sein. Bevorzugt ist ein polyzyklisches Ringsystem ein bizyklisches Ringsystem.

Beispielhaft für Aryl-Reste, die mit einem mono- bzw. polyzyklischen Ringsystem kondensiert sind, seien (1,3)-Benzodioxolyl und (1,4)-Benzodioxanyl genannt.

Sofern einer oder mehrere der vorstehend genannten Substituenten ein mono- oder polyzyklisches Ringsystem aufweisen, kann dieses bevorzugt mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, - N(CH₃)(C₂H₅), -NH-C(=O)-O-CH₃, -NH-C(=O)-O-C₂H₅, -NH-C(=O)-O-C(CH₃)₃, - C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, - CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann.

Sofern einer oder mehrere der vorstehend genannten Substituenten eine lineare oder verzweigte C₁₋₆-Alkylen-Gruppe aufweisen, kann diese bevorzugt ausgewählt werden aus der Gruppe bestehend aus -(CH₂)-, -(CH₂)₂-, -C(H)(CH₃)-, -(CH₂)₃-, - (CH₂)₄-, -(CH₂)₅-, -C(H)(C(H)(CH₃)₂)- und -C(C₂H₅)(H)-.

Bevorzugt sind Verbindungen der allgemeinen Formel A, worin
X für O steht;
Y für -NH₂; -NHR³⁰; -NR³¹R³²; für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl steht;
n für 1 steht;
R¹, R³ und R⁴ jeweils für H stehen;
R² für Methyl; -O-CH₃; F; Cl; Br oder I steht;
R⁵ für einen Rest ausgewählt aus der Gruppe bestehend aus Methyl, Ethyl, -CF₃, -CCl₃, -CBr₃, -CHF₂, -CH₂F, -CF₂Cl, -CCl₂F, -C(CH₃)₂(CH₂OH), tert-Butyl, -O-CF₃, -O-CCl₃, -O-CBr₃, -O-CHF₂, -O-CH₂F, -S-CF₃, -S-CCl₃, -S-CBr₃, -S-CHF₂ und -S-CH₂F steht;
T für CH und U für CH und V für N und W für C-R⁸
oder
T für CH und U für N und V für CH und W für C-R⁸
oder
T für N und U für CH und V für CH und W für C-R⁸
oder
T für N und U für N und V für CH und W für C-R⁸
oder
T für N und U für CH und V für N und W für C-R⁸
oder
T für CH und U für N und V für N und W für C-R⁸
oder
T für CH und U für CH und V für CH und W für C-R¹⁰
steht;
R⁸ für H; F; Cl; Br, I**;** -CN; -OH; -NH₂; -NO₂; -NHR¹¹; -NR¹²R¹³; -OR¹⁴; -SR¹⁵;
oder für einen Rest ausgewählt aus der Gruppe bestehend aus Methyl, -CF₃, -CCl₃, - CBr₃, -CHF₂, -CH₂F, -CF₂Cl, -CCl₂F, Ethyl, -CF₂-CH₃, -CH₂-CF₃, -C₂F₅, -CH₂-CCl₃, - CH₂-CBr₃, -CHF-CF₂Cl, -CF₂-CF₂Cl, -CFCl-CF₂Cl, n-Propyl, -CF₂-CF₂-CF₃, - CF(CF₃)₂, Isopropyl, sek-Butyl, Isobutyl, tert-Butyl, -CH₂-CH₂-CH₂-CF₃, -CH₂-CH₂-CH₂-CH₂-CF₃, -CH₂-C(=O)-O-CH₃, -CH₂-C(=O)-C₂H₅, -CH₂-C(=O)-C(CH₃)₃, -CH₂-O-C(=O)-CH₃, -CH₂-O-C(=O)-C₂H₅, -CH₂-O-C(=O)-CH(CH₃)₂, -CH₂-O-C(=O)-C(CH₃)₃, n-Butyl, 3-Methyl-but-1-yl, 4-Methyl-pent-1-yl, n-Pentyl, n-Pentyl, n-Hexyl, (3,3)-Dimethyl-but-1-yl, (3,3)-Dimethyl-but-1-inyl, 4-Methyl-pent-1-inyl, 1-Hexinyl, Pentinyl, Butinyl, Propinyl, Ethinyl, 2-Methyl-propen-1-yl, 3-Methyl-but-2-en-1-yl, 1-Pentenyl, 1-Octenyl, 1-Heptenyl, 1-Hexenyl und (3,3)-Dimethyl-but-1-enyl;
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂. -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Tetrazolyl, (1,3)-Benzodioxolyl, (1,4)-Benzodioxanyl, (2,3)-Dihydrothieno[3,4-b][1,4]dioxinyl, Benzo[b]furanyl, Indolyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, - C≡C-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - N(CH₃)₂, -N(C₂H₅)₂, -NH-S(=O)₂-CH₃, -NH-S(=O₂)-C₂H₅, -NH-S(=O)₂-CH(CH₃)₂, - NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R¹⁰ für-CN; -NH₂; -NO₂; -NHR¹¹; -NR¹²R¹³; -OR¹⁴; -SR¹⁵;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der
jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R¹¹, R¹², R¹³, R¹⁴ und R¹⁵, unabhängig voneinander, jeweils für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus 2,3-Dihydro-1H-indenyl, Oxetanyl, Piperidinyl, Pyrrolidinyl, Cyclopropyl, Cyclobutyl, Cyclopentyl und Cyclohexyl stehen, der jeweils über eine -CH₂-O-, -CH₂-CH₂-O-, -CH₂-CH₂-O-CH₂-, - CH₂-CH(CH₃)-O-CH₂, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, - (CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF3, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen; oder
R¹² und R¹³ jeweils zusammen mit dem sie verbindenden Stickstoffatom als Ringglied einen Rest ausgewählt aus der Gruppe bestehend aus 3-Aza-bicyclo[3.1.1]heptyl 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl,-CH₂-OH, -CH₂-CH₂-OH, =CH₂, -C(=O)-O-CH₃, -C(=O)-O-C₂H₅, - C(=O)-O-C(CH₃)₃, -C(=O)-CH₃, -C(=O)-C₂H₅, -C(=O)-C(CH₃)₃, -CN, -CH₂-N(CH₃)₂, - CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, -NH₂, -NH-CH₃, -NH-C₂H₅, -N(CH₃)₂, - N(C₂H₅)₂, -NH-Phenyl, -N(CH₃)-Phenyl, -N(C₂H₅)-Phenyl, Oxo (=O), Thioxo (=S), - OH, F, Cl, Br, -CF₃, -O-CH₃, -O-C₂H₅, -O-C(CH₃)₃, -O-CH(CH₃)₂, -O-CH₂-CH₂-CH₂-CH₃, Methyl, Ethyl, Isopropyl, n-Propyl, n-Butyl, tert-Butyl, sek-Butyl, Cyclohexyl, Cyclopentyl, Piperidinyl, Pyrrolidinyl, -O-Phenyl, -O-C(=O)-CH₃, -O-C(=O)-C₂H₅, -O-C(=O)-C(CH₃)₃, -(CH₂)-Pyridinyl, Pyridinyl, Phenyl und Benzyl substituiert sein kann, wobei jeweils der zyklische Teil der Reste Oxetanyl, -N-[C(=O)-C₂H₅]-Phenyl, -N-[C(=O)-CH₃]-Phenyl, -NH-Phenyl, -N(CH₃)-Phenyl, -N(C₂H₅)-Phenyl, -(CH₂)-Pyridinyl, Pyridinyl, Phenyl, -O-Phenyl und Benzyl ggf. mit 1, 2, 3, 4 oder 5 Substituenten unabhängig voneinander ausgewählt aus der Gruppe bestehend aus -CF₃, -OH, -O-CH₃, -O-C₂H₅, F, Cl, Br, Methyl, Ethyl, Isopropyl, n-Propyl, n-Butyl, tert-Butyl und sek-Butyl substituiert sein kann;
und
R²⁵ für einen Alkyl-Rest ausgewählt aus der Gruppe bestehend aus -CH₂-OH, -CH₂-CH₂-OH, -CH₂-CH₂-CH₂-OH, -CH₂-CH₂-CH₂-CH₂-OH, Isopropyl, n-Butyl, sek-Butyl, Isobutyl, Methyl, Ethyl und n-Propyl oder für einen Rest ausgewählt aus der Gruppe bestehend aus Phenyl, Benzyl, Phenethyl, Cyclopropyl, Cyclobutyl, Cyclopentyl und Cyclohexyl steht;
R²⁶ für einen Wasserstoff-Rest oder für einen Rest ausgewählt aus der Gruppe bestehend aus Methyl, Ethyl und n-Propyl steht;
oder
R²⁵ und R²⁶ jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
und R³⁰, R³¹ und R³², unabhängig voneinander, jeweils für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ein weiterer Gegenstand der Erfindung sind Verbindungen der allgemeinen Formel I**,** 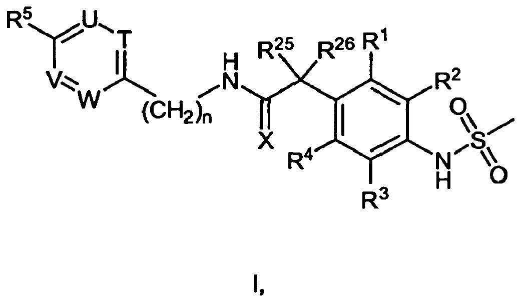 worin

X für O, S oder N-C≡N steht; n für 0, 1, 2, 3 oder 4 steht;
R¹, R², R³ und R⁴, unabhängig voneinander, jeweils für H; F; Cl; Br; I; -SF₅; -NO₂; -CF₃; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³; -S(=O)-R²⁴; S(=O)₂-R²⁴ oder für einen linearen oder verzweigten, gesättigten oder ungesättigten, unsubstituierten oder wenigstens einfach substituierten aliphatischen C₁₋₁₀ Rest stehen;
R⁵ für H; F; Cl; Br; I; -SF₅; -NO₂; -CF₃; -CF₂Cl; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³ -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; - C(=O)-OR²²; -C(=O)-R²³, -S(=O)-R²⁴; -S(=O)₂-R²⁴
für einen linearen oder verzweigten, gesättigten oder ungesättigten, unsubstituierten oder wenigstens einfach substituierten aliphatischen C₁₋₁₀ Rest;
oder für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest steht, der jeweils über ein Kohlenstoffatom im Ring des cycloaliphatischen Restes an das Grundgerüst gebunden ist;
T für C-R⁶ und U für C-R⁷ und V für N und W für C-R⁸
oder
T für C-R⁶ und U für N und V für C-R⁹ und W für C-R⁸
oder
T für N und U für C-R⁷ und V für C-R⁹ und W für C-R⁸
oder
T für N und U für N und V für C-R⁹ und W für C-R⁸
oder
T für N und U für C-R⁷ und V für N und W für C-R⁸
oder
T für G-R⁶ und U für N und V für N und W für C-R⁸
oder
T für C-R⁶ und U für C-R⁷ und V für C-R⁹ und W für C-R¹⁰
steht;
R⁶ und R⁷, unabhängig voneinander, jeweils für H; F; Cl; Br; I; -SF₅; -NO₂; -CF₃; -CF₂Cl; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; - C(=O)-NR¹⁷R¹⁸; -S(=O)₂-NHR¹⁹_{;} -S(=O)₂-NR²⁰R²¹; -C(=O)-OR²²; -C(=O)-R²³, -S(=O)-R²⁴; -S(=O)₂-R²⁴; für einen linearen oder verzweigten, gesättigten oder ungesättigten, unsubstituierten oder wenigstens einfach substituierten aliphatischen C₁₋₁₀ Rest;
oder für einen unsubstituierten oder wenigstens einfach substituierten 6- oder 10-gliedrigen Aryl-Rest, der über eine lineare oder verzweigte, unsubstituierte oder wenigstens einfach substituierte C₁₋₆-Alkylen-Gruppe oder C-₂₋₆-Alkenylen-Gruppe oder C₂₋₆-Alkinylen-Gruppe gebunden sein kann, stehen;
R⁸ für H; F; Cl; Br; I; -SF₅; -NO₂; -CF₃; -CF₂Cl; -CN; -NH₂; -OH; -SH; -C(=O)-NH₂; -S(=O)₂-NH₂: -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; - C(=O)-OR²²; -C(=O)-R²³; -S(=O)-R²⁴; -S(=O)₂-R²⁴; -C(=NH)-NH₂; -C(=NH)-NH-R²⁷; - N=C(NH₂)₂: -N=C(NHR²⁸)(NHR²⁹);
für einen linearen oder verzweigten, gesättigten oder ungesättigten, unsubstituierten oder wenigstens einfach substituierten aliphatischen C₁₋₁₀ Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder C-₂₋₆-Alkenylen-Gruppe oder C₂₋₆-Alkinylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder C-₂₋₆-Alkenylen-Gruppe oder C₂₋₆-Alkinylen-Gruppe gebunden sein kann, steht;
R⁹ für H; F; Cl; Br; I; -SF₅; -NO₂; -CF₃; -CF₂Cl; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; - C(=O)₂-OR²²; -C(=O)-R²³; -S(=O)-R²⁴; -S(=O)₂-R²⁴ oder für einen linearen oder verzweigten, gesättigten oder ungesättigten, unsubstituierten oder wenigstens einfach substituierten aliphatischen C₁₋₁₀ Rest steht; R¹⁰ für -SF₅; -NO₂; -CF₃; -CF₂Cl; -CN; -NH₂; -OH; -SH; -C(=O)-NH₂; -S(=O)₂-NH₂; - C(=O)-NH-OH; -C(=O)-OH; -C(=O)-H; -S(=O)₂-OH; -NHR¹¹; -NR¹²R¹³; -OR¹⁴; -SR¹⁵; - C(=O)-NHR¹⁶; -C(=O)-NR¹⁷R¹⁸; -S(=O)₂-NHR¹⁹; -S(=O)₂-NR²⁰R²¹; -C(=O)-OR²²; - C(=O)-R²³; -S(=O)-R²⁴; -S(=O)₂-R²⁴; -C(=NH)-NH₂; -C(=NH)-NH-R²⁷; -N=C(NH₂)₂; - N=C(NHR²⁸)(NHR²⁹);
für einen linearen oder verzweigten, gesättigten oder ungesättigten aliphatischen C₁₋₁₀ Rest, der jeweils mit ggf. 1, 2, 3, 4, 5, 6, 7, 8 oder 9 Substituenten unabhängig voneinander ausgewählt aus der Gruppe bestehend aus -CN, -NO₂, -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), -OCF₃ und - SCF₃ substituiert ist;
für einen unsubstituierten C₂₋₁₀ Alkenyl-Rest oder einen unsubstituierten C₂₋₁₀-Akinyl-Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe gebunden sein kann, steht;
R¹¹, R¹², R¹³, R¹⁴, R¹⁵, R¹⁶, R¹⁷, R¹⁸, R¹⁹, R²⁰, R²¹ , R²², R²³, R²⁴, R²⁷ , R²⁸ und R²⁹, unabhängig voneinander, jeweils
für einen linearen oder verzweigten, gesättigten oder ungesättigten, unsubstituierten oder wenigstens einfach substituierten aliphatischen C₁₋₁₀ Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder 2- bis 6-gliedrige Heteroalkylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder 2- bis 6-gliedrige Heteroalkylen-Gruppe gebunden sein kann, stehen;
oder
R¹² und R¹³ jeweils zusammen mit dem sie verbindenden Stickstoffatom als Ringglied einen gesättigten oder ungesättigten, unsubstituierten oder wenigstens einfach substituierten 4-, 5-, 6-, 7-, 8- oder 9-gliedrigen, ggf. wenigstens ein weiteres Heteroatom als Ringglied aufweisenden heterocycloaliphatischen Rest bilden, der mit einem gesättigten oder ungesättigten, unsubstituierten oder wenigstens einfach substituierten mono- oder polyzyklischen Ringsystem kondensiert sein kann;
und
R²⁵ und R²⁶, unabhängig voneinander, jeweils für einen Wasserstoff-Rest;
für einen linearen oder verzweigten, gesättigten oder ungesättigten, unsubstituierten oder wenigstens einfach substituierten aliphatischen C₁₋₁₀ Rest;
für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder C-₂₋₆-Alkenylen-Gruppe oder C₂₋₆-Alkinylen-Gruppe gebunden sein kann;
oder für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest stehen;
mit der Maßgabe, dass R²⁵ und R²⁶ nicht jeweils für einen Wasserstoff-Rest stehen;
oder
R²⁵ und R²⁶ jeweils zusammen mit dem sie verbindenden Kohlenstoffatom als Ringglied einen gesättigten oder ungesättigten, unsubstituierten oder wenigstens einfach substituierten 3-, 4-, 5- oder 6-gliedrigen cycloaliphatischen Rest bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Bevorzugt sind Verbindungen der vorstehend angegebenen allgemeinen Formeln A und I, worin
n, X, Y, T, U, V, W, R¹ bis R⁷, R⁹ und R¹¹ bis R³² wie vorstehend definiert sind und
R⁸ für H; F; Cl; Br; I; -SF₅; -NO₂; -CF₃; -CF₂Cl; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -C(=O)-NHR¹⁶; - C(=O)-NR¹⁷R¹⁸_{;} -S(=O)₂-NHR¹⁹_{;} -S(=O)₂-NR²⁰R²¹; -C(=O)-OR²²; -C(=O)-R²³; -S(=O)-R²⁴; -S(=O)₂-R²⁴; -C(=NH)-NH₂; -C(=NH)-NH-R²⁷; -N=C(NH₂)₂; -N=C(NHR²⁸)(NHR²⁹);
für eine ungesättigte oder gesättigte, unsubstituierte oder wenigstens einfach substituierte Kette, die 1 bis 7 Kohlenstoffatome als Kettenglieder aufweist, wobei 1, 2 oder 3 Kohlenstoffatome durch Heteroatome ausgewählt aus der Gruppe bestehend aus Sauerstoff, Schwefel und Stickstoff (NH) ersetzt werden können;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder C-₂₋₆-Alkenylen-Gruppe oder C₂₋₆-Alkinylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder C-₂₋₆-Alkenylen-Gruppe oder C₂₋₆-Alkinylen-Gruppe gebunden sein kann, steht;
und
R¹⁰ für -SF₅; -NO₂; -CF₃; -CF₂Cl; -CN; -NH₂; -OH; -SH; -C(=O)-NH₂; -S(=O)₂-NH₂; - C(=O)-NH-OH; -C(=O)-OH; -C(=O)-H; -S(=O)₂-OH; -C(=O)-NHR¹⁶; -C(=O)-NR¹⁷R¹⁸; - S(=O)₂-NHR¹⁹; -S(=O)₂-NR²⁰R²¹; -C(=O)-OR²²; -C(=O)-R²³; -S(=O)-R²⁴; -S(=O)₂-R²⁴; -C(=NH)-NH₂; -C(=NH)-NH-R²⁷; -N=C(NH₂)₂; -N=C(NHR²⁸)(NHR²⁹);
für eine ungesättigte oder gesättigte, unsubstituierte oder wenigstens einfach substituierte Kette, die 1 bis 7 Kohlenstoffatome als Kettenglieder aufweist, wobei 1, 2 oder 3 Kohlenstoffatome durch Heteroatome ausgewählt aus der Gruppe bestehend aus Sauerstoff, Schwefel und Stickstoff (NH) ersetzt werden können und die in Abwesenheit von Heteroatomen als Keftengliedem jeweils mit ggf. 1, 2, 3, 4, 5, 6, 7, 8 oder 9 Substituenten unabhängig voneinander ausgewählt aus der Gruppe bestehend aus -CN, -NO₂, -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), -OCF₃ und -SCF₃ substituiert ist;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe gebunden sein kann, steht.

Bevorzugt weist die Kette 5 bis 7 Kohlenstoffatome als Kettenglieder auf, wobei 1, 2 oder 3 Kohlenstoffatome durch Heteroatome ausgewählt aus der Gruppe bestehend aus Sauerstoff und Schwefel ersetzt werden können.

Sofern einer oder mehrere der vorstehend genannten Reste für eine 1- bis 7-gliedrige Kette oder eine 5- bis 7-gliedrige Kette stehen, kann diese bevorzugt mit ggf. 1, 2, 3, 4, 5, 6, 7, 8 oder 9 Substituenten unabhängig voneinander ausgewählt aus der Gruppe bestehend aus -C₁₋₅-Alkyl, F, Cl, Br, -CN, -NO₂, -OH, -NH₂, -SH, - O(C₁₋₅-Alkyl), -S(C₁₋₅-Alkyl), -NH(C₁₋₅-Alkyl), -N(C₁₋₅-Alkyl)(C₁₋₅-Alkyl), -OCF₃, -SCF₃, - O-Phenyl, -S-Phenyl, -NH-Phenyl, Oxetanyl, Piperidinyl, Pyrrolidinyl, Cyclopropyl, Cyclobutyl, Cyclopentyl und Cyclohexyl substituiert sein, wobei jeweils die zyklischen Reste mit ggf. 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sek-Butyl, Isobutyl, tert-Butyl, -O-CH₃, -O-C₂H₅, -O-CH(CH₃)₂, -O-C(CH₃)₃, -O-CF₃, -S-CF₃, -C(=O)-CH₂, -C(=O)-C₂H₅, Phenyl und -O-Benzyl substituiert sein können.

Beispielhaft für 5-bis 7-gliedrige substituierte oder unsubstituierte Ketten seien n-Pentyl, n-Hexyl, 4-Methyl-pent-1-inyl, 1-Hexinyl, Pentinyl, 1-Pentenyl, 1-Heptenyl, 1-Hexenyl, -O-CH₂-CH₂-CH₂-O-CH₃, -S-CH₂-CH₂-CH₂-O-CH₃, -S-CH₂-CH₂-CH₂-S-CH₃, -O-CH₂-CH(CH₃)-O-CH₂-Oxetanyl und -S-CH₂-CH(CH₃)-O-CH₂-Oxetanyl genannt.

Bevorzugt sind Verbindungen der vorstehend angegebenen allgemeinen Formel A und I, worin
X, n, R¹ bis R²⁹, T, U, V und W wie vorstehend definiert sind;
wobei
sofern nicht anders angegeben, die vorstehend genannten aliphatischen C₁₋₁₀ Reste ggf. jeweils mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, - O(C₁₋₅-Alkyl), -S(C₁₋₅-Alkyl), -NH(C₁₋₅-Alkyl), -N(C₁₋₅-Alkyl)(C₁₋₅-Alkyl), -C(=O)-O-C₁₋₅-Alkyl, -O-C(=O)-C₁₋₅-Alkyl, -O-Phenyl, Phenyl, -OCF₃ und -SCF₃ substituiert sein können;
die vorstehend genannten 2- bis 6-gliedrigen Heteroalkylen-Gruppen, C₁₋₆-AlkylenGruppen und C₂₋₆-Alkenylen-Gruppen und C₂₋₆-Alkinylen-Gruppen ggf. jeweils mit 1,
2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), -OCF₃ und -SCF₃ substituiert sein können;
die vorstehend genannten Heteroalkylen-Gruppen jeweils ggf. 1, 2 oder 3 Heteroatom(e) unabhängig voneinander ausgewählt aus der Gruppe bestehend aus Sauerstoff, Schwefel und Stickstoff (NH) als Kettenglied(er) aufweisen;
die vorstehend genannten (hetero)cycloaliphatischen Reste ggf. jeweils mit 1, 2, 3, 4 oder 5 Substituenten unabhängig voneinander ausgewählt aus der Gruppe bestehend aus -C₁₋₆-Alkylen-OH, =CH₂, -O-C₁₋₅₋Alkylen-Oxetanyl, -C₁₋₅-Alkylen-O-C₁₋₅-Alkylen-Oxetanyl,-CH₂-NH-C₁₋₅-Alkyl, -CH₂-N(C₁₋₅-Alkyl)₂, -N[C(=O)-C₁₋₅-Alkyl]-Phenyl, -CH₂-O-C₁₋₅-Alkyl, Oxo (=O), Thioxo (=S), F, Cl, Br, I, -CN, -CF₃, -SF₅, -OH, - O-C₁₋₅-Alkyl, -O-C(=O)-C₁₋₅-Alkyl, -NH₂, -NO₂, -O-CF₃, -S-CF₃, -SH, -S-C₁₋₅-Alkyl, -C₁₋₅-Alkyl, -C(=O)-C₁₋₅-Alkyl, -C(=O)-OH, -C(=O)-O-C₁₋₅-Alkyl, -NH-C₁₋₅-Alkyl, -N(C₁₋₅-Alkyl)₂, -NH-Phenyl, -N(C₁₋₅-Alkyl)-Phenyl, Cyclohexyl, Cyclopentyl, (4,5)-Dihydroisoxazolyl, Thiazolyl, (1,2,5)-Thiadiazolyl, Thiophenyl, Phenethyl, Piperidinyl, Pyrrolidinyl, -(CH₂)-Pyridinyl, Pyridinyl, -O-Phenyl, -O-Benzyl, Phenyl und Benzyl substituiert sein, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N[C(=O)-C₁₋₅-Alkyl]-Phenyl, -NH-Phenyl, -N(C₁₋₅-Alkyl)-Phenyl, -(CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -C₁₋₅-Alkyl, -O-C₁₋₅-Alkyl, -O-CF₃, -S-CF₃, Phenyl und -O-Benzyl substituiert sein kann,-und die vorstehend genannten (hetero)cycloaliphatischen Reste jeweils ggf. 1, 2 oder 3 (weitere) Heteroatom(e) unabhängig voneinander ausgewählt aus der Gruppe bestehend aus Sauerstoff, Stickstoff und Schwefel aufweisen können;
die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-C₁₋₅-Alkyl, -C(=O)-OH, -C(=O)-O-C₁₋₅-Alkyl, -NH-C₁₋₅-Alkyl, -N(C₁₋₅-Alkyl)₂, -O-Phenyl, -O-Benzyl, Phenyl und Benzyl substituiert sein können, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
und die Ringe der vorstehend genannten mono- oder polyzyklischen Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
und die vorstehend genannten Aryl- oder Heteroaryl-Reste ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂, -NO₂, -O-CF₃, - S-CF₃, -SH, -S-C₁₋₅-Alkyl, -C₁₋₅-Alkyl, -C(=O)-OH, -C(=O)-O-C₁₋₅-Alkyl, -NH-C₁₋₅-Alkyl, -N(C₁₋₅-Alkyl)₂, -NH-S(=O)₂-C₁₋₅-Alkyl, -NH-C(=O)-O-C₁₋₅-Alkyl, -C(=O)-H, -C(=O)-C₁-₅-Alkyl, -C(=O)-NH₂, -C(=O)-NH-C₁₋₅-Alkyl, -C(=O)-N-(C₁₋₅-Alkyl)₂, -O-Phenyl, -O-Benzyl, Phenyl und Benzyl substituiert sein können, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
und
die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ein weiterer Gegenstand der Erfindung sind Verbindung der allgemeinen Formel B1, 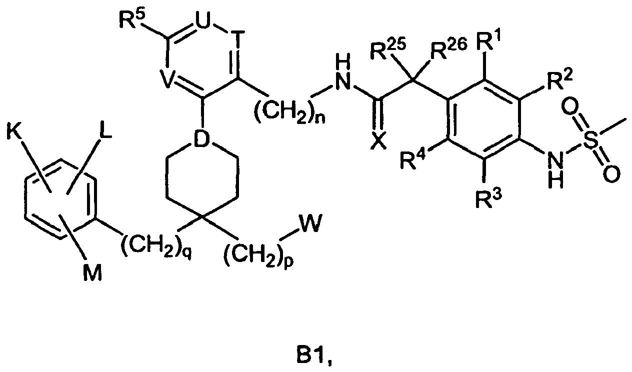 worin
U, T, V, X, n, R¹, R², R³, R⁴, R⁵, R²⁵ und R²⁶ wie vorstehend definiert sind;
D ist CH oder N;
p ist 0, 1, 2 oder 3;
q ist 0, 1, 2 oder 3;
K, L und M, unabhängig voneinander, jeweils für H, F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -NH-C₁₋₅-Alkyl, -N(C₁₋₅-Alkyl)₂, -NH-S(=O)₂-C₁₋₅-Alkyl, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, - C(=O)-N-(C₁₋₅-Alkyl)₂, -O-Phenyl, -O-Benzyl, Phenyl und Benzyl stehen, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann;
W für -CN, -NR³⁴R³⁵, -C(=O)-R³⁶ oder -C(=O)-OR³⁷ steht;
und R³⁴, R³⁵, R³⁶ und R³⁷, unabhängig voneinander, jeweils für Wasserstoff, oder für einen linearen oder verzweigten, gesättigten oder ungesättigten aliphatischen C₁₋₁₀ Rest;
für einen ungesättigten oder gesättigten, unsubstituierten oder wenigstens einfach substituierten 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder C-₂₋₆-Alkenylen-Gruppe oder C₂₋₆-Alkinylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder C-₂₋₆-Alkenylen-Gruppe oder C₂₋₆-Alkinylen-Gruppe gebunden sein kann, stehen.

Ein weiterer Gegenstand der Erfindung sind Verbindungen der allgemeinen Formel B2, 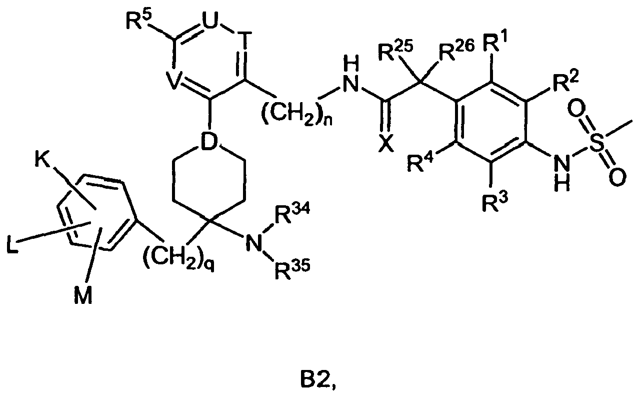 worin
U, T, V, X, n, R¹, R², R³, R⁴, R⁵, R²⁵ und R²⁶ wie vorstehend definiert sind;
D ist CH oder N;
q ist 0, 1, 2 oder 3;
K, L und M, unabhängig voneinander, jeweils für H, F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -NH-C₁₋₅-Alkyl, -N(C₁₋₅-Alkyl)₂, -NH-S(=O)₂-C₁₋₅-Alkyl, -NH-C(=O)-O-C₁₋₅-A)kyl, -C(=O)-H, -C(=O)-C₁₋₅-Alkyl, -C(=O)-NH₂, -C(=O)-NH-C₁₋₅-Alkyl, - C(=O)-N-(C₁₋₅-Alkyl)₂, -O-Phenyl, -O-Benzyl, Phenyl und Benzyl stehen, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann;
und R³⁴ und R³⁵, unabhängig voneinander, jeweils für Wasserstoff oder für einen linearen oder verzweigten, gesättigten oder ungesättigten aliphatischen C₁₋₁₀ Rest;
für einen ungesättigten oder gesättigten, unsubstituierten oder wenigstens einfach substituierten 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder C-₂₋₆-Alkenylen-Gruppe oder C₂₋₆-Alkinylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder C-₂₋₆-Alkenylen-Gruppe oder C₂₋₆-Alkinylen-Gruppe gebunden sein kann, stehen.
Bevorzugt sind Verbindungen der vorstehend angebenen allgemeinen Formeln I, B1 und B2, worin
X für O, S oder N-C≡N steht;
n für 0, 1, 2, 3 oder 4 steht;
R¹, R², R³ und R⁴, unabhängig voneinander, jeweils für H; F; Cl; Br; I; -SF₅; -NO₂; -CN; -CF₃; -NH₂; -OH; -SH; -C(=O)-NH₂; -S(=O)₂-NH₂, -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(-O)₂-NHR¹⁹; -S(=O)₂-NR²⁰R²¹; -C(=O)-OR²²; -C(=O)-R²³; -S(=O)-R²⁴; S(=O)₂-R²⁴ oder für einen Alkyl-Rest ausgewählt aus der Gruppe bestehend aus Methyl, Ethyl, n-Propyl, Isopropyl, tert-Butyl, n-Butyl, sek-Butyl, Isobutyl, n-Pentyl, n-Hexyl und n-Heptyl stehen;
R⁵ für H; F; Cl; Br; I; -SF₅; -NO₂; -CF₃; -CF₂Cl; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; - C(=O)-OR²²; -C(=O)-R²³; -S(=O)-R²⁴; -S(=O)₂-R²⁴;
für einen Alkyl-Rest ausgewählt aus der Gruppe bestehend aus Methyl, Ethyl, n-Propyl, Isopropyl, tert-Butyl, n-Butyl, sek-Butyl, Isobutyl, n-Pentyl, n-Hexyl und n-Heptyl;
oder für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste an das Grundgerüst gebunden ist und ggf. jeweils mit 1, 2, 3, 4 oder 5 Substituenten unabhängig voneinander ausgewählt aus der Gruppe bestehend aus -CH₂-O-CH₃, -CH₂-O-CH₂-CH₃, Oxo (=O), Thioxo (=S), F, Cl, Br, I,-CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, - C(=O)-C₂H₅, -C(=O)-CH(CH₃)₂, -C(=O)C(CH₃)₃, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, - N(CH₃)₂, -N(C₂H₅)₂, -N(CH₃)(C₂H₅), -NH-Phenyl, -N(CH₃)-Phenyl, -N(C₂H₅)-Phenyl, - N(C₂H₅)-Phenyl, -O-CH₂-CH₂-CH₂-CH₃, Piperidinyl, Pyrrolidinyl, -O-C(=O)-CH₃, -O-C(=O)-C₂H₅, -O-C(=O)-C(CH₃)₃, -(CH₂)-Pyridinyl, Pyridinyl, -O-Phenyl, -O-Benzyl, Phenyl und Benzyl substituiert sein, wobei jeweils der zyklische Teil der Reste -NH-Phenyl, -N(CH₃)-Phenyl, -N(C₂H₅)-Phenyl, -(CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, Methyl, Ethyl, n-Propyl, Isopropyl, n-Butyl, sek-Butyl, Isobutyl, tert-Butyl, - O-CH₃, -O-C₂H₅, -O-CH(CH₃)₂, -O-C(CH₃)₃, -O-CF₃, -S-CF₃, Phenyl und -O-Benzyl substituiert sein kann, steht;
T für C-R⁶ und U für C-R⁷ und V für N und W für C-R⁸
oder
T für C-R⁶ und U für N und V für C-R⁹ und W für C-R⁸ oder
T für N und U für C-R⁷ und V für C-R⁹ und W für C-R⁸
oder
T für N und U für N und V für C-R⁹ und W für C-R⁸
oder
T für N und U für C-R⁷ und V für N und W für C-R⁸
oder
T für C-R⁶ und U für N und V für N und W für C-R⁸
oder
T für C-R⁶ und U für C-R⁷ und V für C-R⁹ und W für C-R¹⁰
steht;
R⁶ und R⁷, unabhängig voneinander, jeweils für H; F; Cl; Br; I; -SF₅; -NO₂; -CF₃; -CF₂Cl; -CN; -NH₂; -OH; -SH; -C(=O)-NH₂; -S(=O)₂-NH₂; -C(=O)-OH; - C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³, -S(=O)-R²⁴; - S(=O)₂-R²⁴; für einen Alkyl-Rest ausgewählt aus der Gruppe bestehend aus Methyl, Ethyl, n-Propyl, Isopropyl, tert-Butyl, n-Butyl, sek-Butyl, Isobutyl, n-Pentyl, n-Hexyl und n-Heptyl oder für einen Phenyl-Rest stehen, der über eine -(CH=CH)-, -C≡C-, - (CH₂)-, -(CH₂)₂- oder -(CH₂)₃-Gruppe gebunden sein kann und/oder ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl und n-Pentyl substituiert sein kann;
R⁸ für H; F; Cl; Br; I; -SF₅, -NO₂; -CF₃; -CF₂Cl; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹;-C(=O)-OR²², -C(=O)-R²³; -S(=O)-R²⁴; -S(=O)₂-R²⁴, -C(=NH)-NH₂; -C(=NH)-NH-R²⁷; - N=C(NH₂)₂; -N=C(NHR²⁸)(NHR²⁹);
für einen Alkyl-Rest ausgewählt aus der Gruppe bestehend aus Methyl, Ethyl, n-Propyl, Isopropyl, tert-Butyl, n-Butyl, sek-Butyl, Isobutyl, n-Pentyl, 3-Methyl-but-1-yl, 4-Methyl-pent-1-yl, (3,3)-Dimethyl-but-1-yl, n-Hexyl und n-Heptyl;
für einen Alkenyl-Rest ausgewählt aus der Gruppe bestehend aus 2-Methyl-propen-1-yl, 3-Methyl-but-2-en-1-yl, (3,3)-Dimethyl-but-1-enyl, Ethenyl, Propenyl, Butenyl, 1-Octenyl, 1-Heptenyl, 1-Hexenyl und 1-Pentenyl;
für einen Alkinyl-Rest ausgewählt aus der Gruppe bestehend aus Ethinyl, Propinyl, Butinyl, (3,3)-Dimethyl-but-1-inyl, 4-Methyl-pent-1-inyl, 1-Hexinyl und Pentinyl;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, - C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, - N(CH₃)₂, -N(C₂H₅)₂, -N(CH₃)(C₂H₅), -NH-Phenyl, -N(CH₃)-Phenyl, -N(C₂H₅)-Phenyl, - N(C₂H₅)-Phenyl, -O-CH₂-CH₂-CH₂-CH₃, Piperidinyl, Pyrrolidinyl, -O-C(=O)-CH₃, -O-C(=O)-C₂H₅, -O-C(=O)-C(CH₃)₃, -(CH₂)-Pyridinyl, Pyridinyl, -O-Phenyl, -O-Benzyl, Phenyl und Benzyl substituiert sein, wobei jeweils der zyklische Teil der Reste -NH-Phenyl, -N(CH₃)-Phenyl, -N(C₂H₅)-Phenyl, -(CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, Methyl, Ethyl, n-Propyl, Isopropyl, n-Butyl, sek-Butyl, Isobutyl, tert-Butyl, - O-CH₃, -O-C₂H₅, -O-CH(CH₃)₂, -O-C(CH₃)₃, -O-CF₃, -S-CF₃, Phenyl und -O-Benzyl substituiert sein kann, steht;
oder für einen Rest ausgewählt aus der Gruppe bestehend aus Tetrazolyl, Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, (2,3)-Dihydrothieno[3,4-b][1,4]dioxinyl, Benzo[b]thiophenyl, Benzoxazolyl, Benzisoxazolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl, der jeweils über eine -(CH=CH)-, -C≡C-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, - S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, -C(=O)-O-CH₃, -C(=O)-O-C₂H₅, - C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -NH-C(=O)-O-CH₃, -NH-S(=O)₂-CH3, -NH-S(=O₂)-C₂H₅, - NH-S(=O)₂-CH(CH₃)₂, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein kann, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -C₁₋₅-Alkyl, -O-C₁₋₅-Alkyl, -O-CF₃, -S-CF₃, Phenyl und -O-Benzyl substituiert sein kann, steht;
R⁹ für H; F; Cl; Br; I; -SF₅; -NO₂; -CF₃; -CF₂Cl; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; - C(=O)-OR²²; -C(=O)-R²³; -S(=O)-R²⁴; -S(=O)₂-R²⁴ oder für einen Alkyl-Rest ausgewählt aus der Gruppe bestehend aus Methyl, Ethyl, n-Propyl, Isopropyl, tert-Butyl, n-Butyl, sek-Butyl, Isobutyl, n-Pentyl, n-Hexyl und n-Heptyl steht;
R¹⁰ für -SF₅; -NO₂; -CF₃; -CF₂Cl; -CN; -NH₂; -OH; -SH; -C(=O)-NH₂; -S(=O)₂-NH₂; - C(=O)-NH-OH; -C(=O)-OH; -C(=O)-H; -S(=O)₂-OH; -NHR¹¹; -NR¹²R¹³; -OR¹⁴; -SR¹⁵; - C(=O)-NHR¹⁶; -C(=O)-NR¹⁷R¹⁸; -S(=O)₂-NHR¹⁹; -S(=O)₂-NR²⁰R²¹; -C(=O)-OR²²; - C(=O)-R²³; -S(=O)-R²⁴; -S(=O)₂-R²⁴; -C(=NH)-NH₂; -C(=NH)-NH-R²⁷; -N=C(NH₂)₂; -N=C(NHR²⁸)(NHR²⁹);
für einen Alkyl-Rest ausgewählt aus der Gruppe bestehend aus Methyl, Ethyl, n-Propyl, Isopropyl, tert-Butyl, n-Butyl, sek-Butyl, Isobutyl, n-Pentyl, n-Hexyl und n-Heptyl, der jeweils mit ggf. 1, 2, 3, 4, 5, 6, 7, 8 oder 9 Substituenten unabhängig voneinander ausgewählt aus der Gruppe bestehend aus -CN, -NO₂, -OH, -NH₂, -SH, -O-CH₃, -O-C₂H₅, -O-CH(CH₃)₂, -O-C(CH₃)₃, -S-CH₃, -S-C₂H₅, -S-CH(CH₃)₂, -S-C(CH₃)₃, -NH-CH₃, -NH-C₂H₅, -NH-C(CH₃)₃, -N(CH₃)₂, -N(C₂H₅)₂, -N(CH₃)(C₂H₅), - OCF₃ und -SCF₃ substituiert ist;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, - C(=O)C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, - N(CH₃)₂, -N(C₂H₅)₂, -N(CH₃)(C₂H₅), -NH-Phenyl, -N(CH₃)-Phenyl, -N(C₂H₅)-Phenyl, - N(C₂H₅)-Phenyl, -O-CH₂-CH₂-CH₂-CH₃, Piperidinyl, Pyrrolidinyl, -O-C(=O)-CH₃, -O-C(=O)-C₂H₅, -O-C(=O)-C(CH₃)₃, -(CH₂)-Pyridinyl, Pyridinyl, -O-Phenyl, -O-Benzyl, Phenyl und Benzyl substituiert sein, wobei jeweils der zyklische Teil der Reste -NH-Phenyl, -N(CH₃)-Phenyl, -N(C₂H₅)-Phenyl, -(CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, Methyl, Ethyl, n-Propyl, Isopropyl, n-Butyl, sek-Butyl, Isobutyl, tert-Butyl, - O-CH₃, -O-C₂H₅, -O-CH(CH₃)₂, -O-C(CH₃)₃, -O-CF₃, -S-CF₃, Phenyl und -O-Benzyl substituiert sein kann, steht;
oder für einen Rest ausgewählt aus der Gruppe bestehend aus Tetrazolyl, Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, (2,3)-Dihydrothieno[3,4-b][1,4]dioxinyl, Benzo[b]thiophenyl, Benzoxazolyl, Benzisoxazolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl, der jeweils über eine -(CH=CH)-, -C≡C-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, - S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, -C(=O)-O-CH₃, -C(=O)-O-C₂H₅, - C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -NH-C(=O)-O-CH₃, -NH-S(=O)₂-CH3, -NH-S(=O₂)-C₂H₅, - NH-S(=O)₂-CH(CH₃)₂, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein kann, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -C₁₋₅-Alkyl, -O-C₁₋₅-Alkyl, -O-CF₃, -S-CF₃, Phenyl und -O-Benzyl substituiert sein kann, steht;
R¹¹, R¹², R¹³, R¹⁴, R¹⁵, R¹⁶, R¹⁷, R¹⁸, R¹⁹, R²⁰, R²¹, R²², R²³, R²⁴, R²⁷, R²⁸ und R²⁹, unabhängig voneinander, jeweils
für einen Alkyl-Rest ausgewählt aus der Gruppe bestehend aus Methyl, Ethyl, n-Propyl, Isopropyl, tert-Butyl, n-Butyl, sek-Butyl, Isobutyl, n-Pentyl, n-Hexyl, n-Heptyl, 3-Pentyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, Ethenyl, Propenyl, 2-Butenyl, 3-Butenyl, 2-Pentenyl und 3-Pentenyl;
für einen Rest ausgewählt aus der Gruppe bestehend aus 2,3-Dihydro-1H-indenyl, Cyclopropyl, Oxetanyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über eine -CH₂-O-, -CH₂-CH₂-O-, -CH₂-CH₂-O-CH₂-, -CH₂-CH(CH₃)-O-CH₂, -(CH₂)-, -(CH₂)₂- oder -(CH₂)₃-Gruppe gebunden sein kann und/oder ggf. mit 1, 2, 3, 4 oder 5 Substituenten unabhängig voneinander ausgewählt aus der Gruppe bestehend aus -CH₂-O-CH₃, -CH₂-O-CH₂-CH₃, Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H_{5,} -C(=O)-CH(CH₃)₂, -C(=O)-C(CH₃)₃, - C(=O)-OH, -C(=O)-O-CH₃, -C(=O)-O-C₂H₅, -C(=O)-O-CH(CH₃)_{2,} -C(=O)-O-C(CH₃)₃, - NH-CH₃, -NH-C₂H₅, -NH-C(CH₃)₃, -N(CH₃)₂, -N(C₂H₅)₂, -N(CH₃)(C₂H₅), -NH-Phenyl, - N(CH₃)-Phenyl, -N(C₂H₅)-Phenyl, -N(C₂H₅)-Phenyl, -O-CH₂-CH₂-CH₂-CH₃, Piperidinyl, Pyrrolidinyl, -O-C(=O)-CH₃, -O-C(=O)-C₂H₅, -O-C(=O)-C(CH₃)₃, -(CH₂)-Pyridinyl, Pyridinyl, -O-Phenyl, -O-Benzyl, Phenyl und Benzyl substituiert sein, wobei jeweils der zyklische Teil der Reste -NH-Phenyl, -N(CH₃)-Phenyl, -N(C₂H₅)-Phenyl, - (CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sek-Butyl, Isobutyl, tert-Butyl, -O-CH₃, -O-C₂H₅, -O-CH(CH₃)₂, -O-C(CH₃)₃, -O-CF₃, -S-CF₃, Phenyl und -O-Benzyl substituiert sein kann, steht;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzoxazolyl, Benzisoxazolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl, wobei der Rest jeweils über eine -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, - CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, - C(=O)-O-CH₃, -C(=O)-O-C₂H₅, -C(=O)-O-CH(CH₃)₂, -C(=O)-O-C(CH₃)₃, -NH-CH₃, - NH-C₂H₅, -NH-C(CH₃)₃, -N(CH₃)₂, -N(C₂H₅)₂, -N(CH₃)(C₂Hₛ), -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein kann, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann, stehen;
oder
R¹² und R¹³ jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, (1,3,4,5)-Tetrahydropyrido[4,3-b]indolyl, (3,4)-Dihydro-1H-isochinolinyl, (1,3,4,9)-Tetrahydro-[b]-carbolinyl, Imidazolidinyl, (1,3)-Thiazolidinyl, Piperazinyl, Morpholinyl, Azepanyl, Diazepanyl und Thiomorpholinyl bilden, der ggf. jeweils mit 1, 2, 3, 4 oder 5 Substituenten unabhängig voneinander ausgewählt aus der Gruppe bestehend aus - CH₂-O-CH₂-Oxetanyl, -CH₂-OH, -CH₂-CH₂-OH, =CH₂, -O-CH₂-Oxetanyl, (4,5)-Dihydroisoxazolyl, Thiazolyl, (1,2,5)-Thiadiazolyl, Thiophenyl, Phenethyl, -CN, -CH₂-N(CH₃)₂, -CH₂-N(C₂H₅)₂, -CH₂-NH-CH₃, -CH₂-NH-C₂H₅, -N-[C(=O)-C₂H_{5]}-Phenyl, -N-[C(=O)-CH₃]-Phenyl, -CH₂-O-CH₃, -CH₂-O-CH₂-CH₃, Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, - C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)_{2,} -C(=O)-O-C(CH₃)₂, -NH-CH₃, -NH-C₂H₅, -NH-C(CH₃)₃, - N(CH₃)₂, -N(C₂H₅)₂, -N(CH₃)(C₂H₅), -NH-Phenyl, -N(CH₃)-Phenyl, -N(C₂H₅)-Phenyl, - N(C₂H₅)-Pheny), -O-CH₂-CH₂-CH₂-CH₃, Piperidinyl, Pyrrolidinyl, Cyclohexyl, Cyclopentyl, -O-C(=O)-CH₃, -O-C(=O)-C₂H₅, -O-C(=O)-C(CH₃)₃, -(CH₂)-Pyridinyl, Pyridinyl, -O-Phenyl, -O-Benzyl, Phenyl und Benzyl substituiert sein, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N-[C(=O)-C₂H₅]-Phenyl, -N-[C(=O)-CH₃]-Phenyl, -NH-Phenyl, -N(CH₃)-Phenyl, -N(C₂H₅)-Phenyl, -(CH₂)-Pyridinyl, Pyridinyl, - O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, - CF₃, -SF₅, -CN, -NO₂, Methyl, Ethyl, n-Propyl, Isopropyl, n-Butyl, sek-Butyl, Isobutyl, tert-Butyl, -O-CH₃, -O-C₂H₅, -O-CH(CH₃)₂, -O-C(CH₃)₃, -O-CF₃, -S-CF₃, Phenyl und - O-Benzyl substituiert sein kann;
und
R²⁵ und R²⁶, unabhängig voneinander, jeweils für einen Wasserstoff-Rest;
für einen Alkyl-Rest ausgewählt aus der Gruppe bestehend aus Methyl, Ethyl, n-Propyl, Isopropyl, tert-Butyl, n-Butyl, sek-Butyl, Isobutyl, n-Pentyl, n-Hexyl und n-Heptyl;
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzoxazolyl, Benzisoxazolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl, wobei der Rest jeweils über eine -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I,-CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Cyclopropyl, Cyclobutyl, Cyclopentyl, Cyclohexyl, Cyclopentenyl und Cyclohexenyl stehen;
mit der Maßgabe, dass R²⁵ und R²⁶ nicht jeweils für einen Wasserstoff-Rest stehen;
oder
R²⁵ und R²⁶ jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl, Cycloheptyl, Cyclopentenyl und Cyclohexenyl bilden;
wobei
sofern nicht anders angegeben, die vorstehend genannten Alkyl-, Alkenyl- und Alkinyl-Reste jeweils ggf. mit 1, 2, 3, 4, 5, 6, 7, 8 oder 9 Substituenten unabhängig voneinander ausgewählt aus der Gruppe bestehend aus -C(=O)-O-CH₃, -C(=O)-O-C₂H₅, -C(=O)-O-C(CH₃)₃, -O-C(=O)-CH₃, -O-C(=O)-C₂H₅, -O-C(=O)-CH(CH₃)₂, -O-C(=O)-C(CH₃)₃, -O-Phenyl, Phenyl, F, Cl, Br, I, -CN, -NO₂, -OH, -NH₂, -SH, -O-CH₃, - O-C₂H₅, -O-CH(CH₃)₂, -O-C(CH₃)₃, -S-CH₃, -S-C₂H₅, -S-CH(CH₃)₂, -S-C(CH₃)₃, -NH-CH₃, -NH-C₂H₅, -NH-C(CH₃)₃, -N(CH₃)₂, -N(C₂H₅)₂, -N(CH₃)(C₂H₅), -OCF₃ und -SCF₃ substituiert sein können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind Verbindungen der vorstehend angegebenen allgemeinen Formeln A, I, B1 und B2, worin
X für O, S oder N-C≡N steht;
Y für -NH₂; -NHR³⁰; -NR³⁰R³²; für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl steht;
n für 0, 1, 2, 3 oder 4 steht;
R¹, R², R³ und R⁴, unabhängig voneinander, jeweils für H; F; Cl; Br; I; -SF₅; -NO₂; -CN; -NH₂; -OH; -SH; -C(=O)-NH₂; -S(=O)₂-NH₂; -C(=O)-NH-OH; -C(=O)-OH; - C(=O)-H; -S(=O)₂-OH; -NHR¹¹; -NR¹²R¹³; -OR¹⁴; -SR¹⁵; -S(=O)-R²⁴; -S(=O)₂-R²⁴ oder für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, n-Butyl, sek-Butyl, Isobutyl und tert-Butyl stehen;
R⁵ für F; Cl; Br; I; -SF₅; -OR¹⁴; -SR¹⁵; -S(=O)-R²⁴; -S(=O)₂-R²⁴;
für einen Rest ausgewählt aus der Gruppe bestehend aus Methyl, -CF₃, -CCl₃, - CBr₃, -CHF₂, -CH₂F, -CF₂Cl, -CCl₂F, -CH₂-CN, -CH₂-O-CH₃, -CH₂-O-CF₃, -CH₂-SF₃, Ethyl, -CF₂-CH₃, -CH₂-CF₃, -C₂F₅, -CH₂-CCl₃, -CH₂-CBr₃, -CHF-CF₂Cl, -CF₂-CF₂Cl, - CFCl-CF₂Cl, -CH₂-CH₂-CN, n-Propyl, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), - CH(CH₃)(S-CH₃), n-Butyl, -CF₂-CF₂-CF₂-CF₃, -CH₂-CH₂-CH₂-CH₂-CN, n-Butyl, sek-Butyl, Isobutyl, -C(CH₃)₂(CH₂OH), und tert-Butyl;
oder für einen Rest ausgewählt aus der Gruppe bestehend aus Cyclopropyl, Cyclobutyl, Cyclopentyl, Cyclohexyl, Cyclopentenyl und Cyclohexenyl, der ggf. jeweils mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl und n-Pentyl substituiert sein kann;
T für C-R⁶ und U für C-R⁷ und V für N und W für C-R⁸
oder
T für C-R⁶ und U für N und V für C-R⁹ und W für C-R⁸
oder
T für N und U für C-R⁷ und V für C-R⁹ und W für C-R⁸
oder
T für N und U für N und V für C-R⁹ und W für C-R⁸
oder
T für N und U für C-R⁷ und V für N und W für C-R⁸
oder
T für C-R⁶ und U für N und V für N und W für C-R⁸
oder
T für C-R⁶ und U für C-R⁷ und V für C-R⁹ und W für C-R¹⁰
steht;
R⁶ und R⁷, unabhängig voneinander, jeweils für H; F; Cl; Br; I; -SF₅; -NO₂; -CN; -NH₂; -OH; -SH; -C(=O)-NH₂; -S(=O)₂-NH₂; -C(=O)-NH-OH; -C(=O)-OH: - C(=O)-H; -S(=O)₂-OH; -NHR¹¹; -NR¹²R¹³; -OR¹⁴; -SR¹⁵; -C(=O)-OR²²; -S(=O)-R²⁴; - S(=O)₂-R²⁴; für einen Rest ausgewählt aus der Gruppe bestehend aus -CH₂-OH, Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, n-Butyl, sek-Butyl, Isobutyl und tert-Butyl oder für einen Phenyl-Rest stehen, der ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂. -O-C(CH₃)₃, -NH₂, -NO₂, -O-CF₃, -S-CF₃, -SH, - S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl und n-Pentyl substituiert sein kann;
R⁸ für H; F; Cl; Br; I; -SF₅; -NO₂; -CN; -NH₂; -OH; -SH; -C(=O)-NH₂; -S(=O)₂-NH₂; -C(=O)-NH-OH; -C(=O)-OH_{;} -C(=O)-H; -S(=O)₂-OH; -NHR¹¹; -NR¹²R¹³, -OR¹⁴; -SR¹⁵; -C(=O)-OR²²; -S(=O)-R²⁴; -S(=O)₂-R²⁴; -C(=NH)-NH₂; -C(=NH)-NH-R²⁷, -N=C(NH₂)₂; - N=C(NHR²⁸)(NHR²⁹);
oder für einen Rest ausgewählt aus der Gruppe bestehend aus Methyl, -CF₃, -CCl₃, - CBr₃, -CHF₂, -CH₂F, -CF₂Cl, -CCl₂F, Ethyl, -CF₂-CH₃, -CH₂-CF₃, -C₂F₅, -CH₂-CCl₃, - CH₂-CBr₃, -CHF-CF₂Cl, -CF₂-CF₂Cl, -CFCl-CF₂Cl, n-Propyl, -CF₂-CF₂-CF₃, - CF(CF₃)₂, -CH₂-CH₂-CH₂-CF₃, -CH₂-CH₂-CH₂-CH₂-CF₃, -CH₂-C(=O)-O-CH₃, -CH₂-C(=O)-C₂H₅, -CH₂-C(=O)-C(CH₃, -CH₂-O-C(=O)-CH₃, -CH₂-O-C(=O)-C₂H₅, -CH₂-O-C(=O)-CH(CH₃)₂, -CH₂-O-C(=O)-C(CH₃)₃, Isopropyl, n-Butyl, sek-Butyl, Isobutyl, tert-Butyl, 3-Methyl-but-1-yl, 4-Methyl-pent-1-yl, n-Pentyl, sec-Pentyl, neo-Pentyl, n-Hexyl, 2-Methyl-propen-1-yl, 3-Methyl-but-2-en-1-yl, (3,3)-Dimethyl-but-1-yl, (3,3)-Dimethyl-but-1-enyl, Ethenyl, Propenyl, Butenyl, 1-Pentenyl, 1-Octenyl, 1-Heptenyl, 1-Hexenyl, (3,3)-Dimethyl-but-1-inyl, 4-Methyl-pent-1-inyl, 1-Hexinyl, Ethinyl, Propinyl, Butinyl, Pentinyl, -CF=CF₂, -CCl=Cl₂, -CH₂-CF=CF₂, -CH₂-CCl=CCl₂, -C≡C-I, -C≡C-F und -C≡C-Cl;
für einen Rest ausgewählt aus der Gruppe bestehend aus Cyclopropyl, Cyclobutyl, Cyclopentyl, Cyclohexyl, Cyclohexenyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus-CN, -CH₂-N(CH₃)₂, -CH₂-N(C₂H₅)₂. -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), -OH, -O-CH₃, -O-C₂H₅, -O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, - C(=O)-O-CH₃, -C(=O)-O-C₂H₅, -C(=O)-O-CH(CH₃)₂ und -C(=O)-O-C(CH₃)₃ substituiert sein kann;
oder für einen Rest ausgewählt aus der Gruppe bestehend aus (1,3)-Benzodioxolyl, (1,4)-Benzodioxanyl, Tetrazolyl, (2,3)-Dihydrothieno[3,4-b][1,4]dioxinyl, Benzo[b]furanyl, Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isolndolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl, der jeweils über eine -(CH=CH)-, -C≡C-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, - CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -s-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, - C(=O)-O-CH₃, -C(=O)-O-C₂H₅, -C(=O)-O-CH(CH₃)₂, -C(=O)-O-C(CH₃)₃, -NH-CH₃, - NH-C₂H₅, -NH-C(CH₃)₃, -N(CH₃)₂, -N(C₂H₅)₂, -N(CH₃)(C₂H₅), -NH-C(=O)-O-CH₃, -NH-S(=O)₂-CH3, -NH-S(=O₂)-C₂H₅, -NH-S(=O)₂-CH(CH₃)₂, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein kann, steht;
R⁹ für H; F; Cl; Br, I; -SF₅; -NO₂; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -S(=O)-R²⁴; -S(=O)₂-R²⁴ oder für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, - CF₂-CF₂-CF₃, -CF(CF₃)₂, Isopropyl, n-Butyl, sek-Butyl, Isobutyl und tert-Butyl steht;
R¹⁰ für -SF₅, -NO_{2;} -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-OR²²; -S(=O)-R²⁴; -S(=O)₂-R²⁴; -C(=NH)-NH₂; -C(=NH)-NH-R²⁷; -N=C(NH₂)₂; - N=C(NHR²⁸)(NHR²⁹);
für einen Rest ausgewählt aus der Gruppe bestehend aus Cyclopropyl, Cyclobutyl, Cyclopentyl, Cyclohexyl, Cyclohexenyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus-CN, -CH₂-N(CH₃)_{2,} -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), -OH, -O-CH₃, -O-C₂H₅, -O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, - C(=O)-O-CH₃, -C(=O)-O-C₂H₅, -C(=O)-O-CH(CH₃)₂ und -C(=O)-O-C(CH₃)₃ substituiert sein kann;
oder für einen Rest ausgewählt aus der Gruppe bestehend aus (1,3)-Benzodioxolyl, (1,4)-Benzodioxanyl, Tetrazolyl, (2,3)-Dihydrothieno[3,4-b][1,4]dioxinyl, Benzo[b]furanyl, Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Thiazolyl, Oxazolyl, Isoxazolyl, Pyridazinyl, Pyrazinyl, Pyrimidinyl, Indazolyl, Chinoxalinyl, Chinolinyl und lsochinolinyl, der jeweils über eine -(CH=CH)-, -C≡C-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, - CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, - C(=O)-O-CH₃, -C(=O)-O-C₂H₅, -C(=O)-O-CH(CH₃)₂, -C(=O)-O-C(CH₃)₃, -NH-CH₃, - NH-C₂Hₛ, -NH-C(CH₃)₃, -N(CH₃)₂, -N(C₂H₅)₂, -N(CH₃)(C₂H₅), -NH-C(=O)-O-CH₃, -NH-S(=O)₂-CH3, -NH-S(=O₂)-C₂H₅, -NH-S(=O)₂-CH(CH₃)₂, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein kann, steht;
R¹¹, R¹², R¹³, R¹⁴, R¹⁵, R²², R²⁴, R²⁷, R²⁸ und R²⁹, unabhängig voneinander, jeweils
für einen Rest ausgewählt aus der Gruppe bestehend aus Methyl, -CF₃, -CCl₃, -CBr₃, -CHF₂, -CH₂F, -CF₂Cl, -CCl₂F, -CH₂-CN, -CH₂-O-CH₃, -CH₂-O-CF₃, -CH₂-SF₃, Ethyl, - CF₂-CH₃, _CH₂-CF₃, -C₂F₅, -CH₂-CCl₃, -CH₂-CBr₃, -CHF-CF₂Cl, -CF₂-CF₂Cl, -CFCI-CF₂Cl, -CH₂-CH₂-CN, n-Propyl, -CF₂-CF₂-CF₃, -CF(CF₃)₂. Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), - CH(CH₃)(S-CH₃), n-Butyl, -CF₂-CF₂-CF₂-CF₃, -CH₂-CH₂-CH₂-CH₂-CN, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus 2,3-Dihydro-1 H-indenyl, Cyclopropyl, Oxetanyl, Cyclobutyl, Cyclopentyl, Cyclohexyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über eine -CH₂-O-, -CH_{Z}-CH₂-O-, -CH₂-CH₂-O-CH₂-, -CH₂-CH(CH₃)-O-CH₂-, -(CH₂)-. - (CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, - C(=O)-O-C₂H₅, -C(=O)-O-CH(CH₃)₂ und -C(=O)-O-C(CH₃)₃ substituiert sein kann;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Thiazolyl, Oxazolyl und Isoxazolyl, wobei der Rest jeweils über eine - (CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -0-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), - NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein kann, stehen;
oder
R¹² und R¹³ jeweils zusammen mit dem sie verbindenden Stickstoffatom als Ringglied einen Rest ausgewählt aus der Gruppe bestehend aus 3-Aza-bicyclo[3.1.1]heptyl 3-Aza-bicyclo[3.1.1]heptyl, 6-Aza-spiro[2.5]octyl, 3-Azabicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl,(1,2,3,6)-Tetrahydropyridinyl, (4,5,6,7)-Tetrahydroisoxazolo[5,4-c]pyridinyl, Pyrrolidinyl, Piperidinyl, Piperazinyl, Morpholinyl, Azepanyl, Diazepanyl und Thiomorpholinyl bilden, der ggf. jeweils mit 1, 2, 3, 4 oder 5 Substituenten unabhängig voneinander ausgewählt aus der Gruppe bestehend aus -CH₂-O-CH₂-Oxetanyl, -CH₂-OH, -CH₂-CH₂-OH, =CH₂, -O-CH₂-Oxetanyl, (4,5)-Dihydroisoxazolyl, Thiazolyl, (1,2,5)-Thiadiazolyl, Thiophenyl, Phenethyl, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl,-CH₂-O-CH₃, -CH₂-O-CH₂-CH₃, Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, - C(=O)-O-CH₃, -C(=O)-O-C₂H₅, -C(=O)-O-CH(CH₃)₂, -C(=O)-O-C(CH₃)₃, -NH-CH₃, - NH-C₂H₅, -NH-C(CH₃)₃, -N(CH₃)₂, -N(C₂H₅)₂, -N(CH₃)(C₂H₅), -NH-Phenyl, -N(CH₃)-Phenyl, -N(C₂H₅)-Phenyl, -N(C₂H₅)-Phenyl, -O-CH₂-CH₂-CH₂-CH₃, Cyclohexyl, Cyclopentyl, Piperidinyl, Pyrrolidinyl, -O-C(=O)-CH₃, -O-C(=O)-C₂H₅, -O-C(=O)-C(CH₃)₃, -(CH₂)-Pyridinyl, Pyridinyl, -O-Phenyl, -O-Benzyl, Phenyl und Benzyl substituiert sein, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N-[C(=O)-C₂H₅]-Phenyl, -N-[C(=O)-CH₃]-Phenyl, -NH-Phenyl, -N(CH₃)-Phenyl, -N(C₂H₅)-Phenyl, -(CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus -CF₃, F, Cl, Br, -OH, -CF₃, -SF₅, -CN, -NO₂, Methyl, Ethyl, n-Propyl, Isopropyl, n-Butyl, sek-Butyl, Isobutyl, tert-Butyl, -O-CH₃, -O-C₂H₅, -O-CH(CH₃)₂, -O-C(CH₃)₃, -O-CF₃, -S-CF₃, Phenyl und -O-Benzyl substituiert sein kann,
und
R²⁵ und R²⁶, unabhängig voneinander, jeweils für einen Wasserstoff-Rest;
für einen Alkyl-Rest ausgewählt aus der Gruppe bestehend aus -CH₂-OH, -CH₂-CH₂-OH, -CH₂-CH₂-CH₂-OH, -CH₂-CH₂-CH₂-CH₂-OH, Isopropyl, n-Butyl, sek-Butyl, Isobutyl, Methyl, Ethyl und n-Propyl;
für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyi, Isoindolyl, Thiazolyl, Oxazolyl, Isoxazotyl, Pyridazinyl, Pyrazinyl, Pyrimidinyl, Indazolyl, Chinoxalinyl, Chinolinyl und Isochinolinyl, der jeweils über eine -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CHₛ)₃, Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Cyclopropyl, Cyclobutyl, Cyclopentyl und Cyclohexyl stehen;
mit der Maßgabe, dass R²⁵ und R²⁶ nicht jeweils für einen Wasserstoff-Rest stehen;
oder
R²⁵ und R²⁶ jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
und R³⁰, R³¹ und R³², unabhängig voneinander, jeweils für einen Alkyl-Restausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.
Weiterhin bevorzugt sind Verbindungen der vorstehend angegebenen allgemeinen Formeln A, I, B1 und B2, worin
X für O S oder N-C≡N steht;
Y für -NH₂; -NHR³⁰; -NR³¹R³²; für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl steht;
n für 0, 1 oder 2 steht;
R¹, R³ und R⁴, unabhängig voneinander, jeweils für H; F; Cl; Br, oder für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl und -CFCl-CF₂Cl stehen;
R² für F; Cl; Br, I oder für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, lsopropyl, sek-Butyl, Isobutyl, tert-Butyl, -O-CH₃, -O-CF₃, -O-CCl₃, -O-CBr₃, -O-CHF₂, -O-CH₂F, -O-CF₂Cl, -O-CCl₂F, -O-C₂H₅, -O-CF₂-CH₃, -O-CH₂-CF₃, -O-C₂F₅, -O-CH₂-CCl₃, -O-CH₂-CBr₃, -O-CHF-CF₂Cl, -O-CF₂-CF₂Cl, -O-CFCl-CF₂Cl, -O-CH₂-CH₂-CH₃, -O-CF₂-CF₂-CF₃, -O-CF(CF₃)₂, -O-CH(CH₃)₂, -O-C(CH₃)₃, -S-CH₃, -S-CF₃, -S-CCl₃, -S-CBr₃, -S-CHF₂, -S-CH₂F, -S-CF₂Cl, -S-CCl₂F, - S-C₂H₅, -S-CF₂-CH₃, -S-CH₂-CF₃, -S-C₂F₅, -S-CH₂-CCl₃, -S-CH₂-CBr₃, -S-CHF-CF₂Cl, -S-CF₂-CF₂Cl, -S-CFCl-CF₂Cl, -S-CH₂-CH₂-CH₃, -S-CF₂-CF₂-CF₃, -S-CF(CF₃)₂, -S-CH(CH₃)₂ und -S-C(CH₃)₃ steht;
R⁵ für F; Cl; Br, I; -SF₅;
für einen Rest ausgewählt aus der Gruppe bestehend aus Methyl, Ethyl, -CF₃, -CCl₃, -CBr₃, -CHF₂, -CH₂F, -CF₂Cl, -CCl₂F, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, -CF₂-CF₂-CF₃, -CF(CF₃)₂, sek-Butyl, Isobutyl, -C(CH₃)₂(CH₂OH), tert-Butyl, -O-CF₃, -O-CCl₃, -O-CBr₃, -O-CHF₂, -O-CH₂F, -O-CF₂Cl, -O-CCl₂F, -O-CF₂-CH₃, -O-CH₂-CF₃, -O-C₂F₅, -O-CH₂-CCl₃, -O-CH₂-CBr₃, -O-CHF-CF₂Cl, -O-CF₂-CF₂Cl, -O-CFCl-CF₂Cl, -O-CF₂-CF₂-CF₃, -O-CF(CF₃)₂, -O-CH(CH₃)₂, -O-C(CH₃)₃, -S-CF₃, -S-CCl₃, -S-CBr₃, -S-CHF₂, -S-CH₂F, -S-CF₂Cl, -S-CCl₂F, -S-CF₂-CH₃, -S-CH₂-CF₃, -S-C₂F₅, -S-CH₂-CCl₃, -S-CH₂-CBr₃, -S-CHF-CF₂Cl, -S-CF₂-CF₂Cl, -S-CFCl-CF₂Cl, -S-CF₂-CF₂-CF₃, -S-CF(CF₃)₂, -S-CH(CH₃)₂, -S-C(CH₃)₃, -S(=O)₂-CF₃, -S(=O)₂-CCl₃, -S(=O)₂-CBr₃, -S(=O)₂-CHF₂, -S(=O)₂-CH₂F, - S(=O)₂-CF₂Cl, -S(=O)₂-CCl₂F, -S(=O)₂-CF₂-CH₃, -S(=O)₂-CH₂-CF₃, -S(=O)₂-C₂F₅, - S(=O)₂-CH₂-CCl₃, -S(=O)₂-CH₂-CBr₃, -S(=O)₂-CHF-CF₂Cl, -S(=O)₂-CF₂-CF₂Cl, - S(=O)₂-CFCl-CF₂Cl, -S(=O)₂-CF₂-CF₂-CF₃, -S(=O)₂-CF(CF₃)₂, -S(=O)₂-CH(CH₃)₂ und -S(=O)₂-C(CH₃)₃;
oder für einen Rest ausgewählt aus der Gruppe bestehend aus Cyclopropyl, Cyclobutyl, Cyclopentyl, Cyclohexyl, Cyclopentenyl und Cyclohexenyl steht;
T für C-R⁶ und U für G-R⁷ und V für N und W für C-R⁸
oder
T für C-R⁶ und U für N und V für C-R⁹ und W für C-R⁸
oder
T für N und U für C-R⁷ und V für C-R⁹ und W für C-R⁸
oder
T für N und U für N und V für C-R⁹ und W für C-R⁸
oder
T für N und U für G-R⁷ und V für N und W für C-R⁸
oder
T für C-R⁶ und U für N und V für N und W für C-R⁸
oder
T für C-R⁶ und U für C-R⁷ und V für C-R⁹ und W für C-R¹⁰
steht;
R⁶ und R⁷, unabhängig voneinander, jeweils für H; F; Cl; Br; I; -NO₂; -CN; -C(=O)-OCH₃; -C(=O)-OC₂H₅; für einen Rest ausgewählt aus der Gruppe bestehend aus - CH₂-OH, Methyl, -CF₃, -CCl₃, -CBr₃, -CHF₂, -CH₂F, -CF₂Cl, -CCl₂F, Ethyl, n-Propyl, Isopropyl, sek-Butyl, Isobutyl und tert-Butyl oder für einen Phenyl-Rest stehen, der ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl und n-Pentyl substituiert sein kann;
R⁸ für H; F; Cl; Br; I; -OH; -CN; -NH₂; -NO₂-, -NHR¹¹, -NR¹²R¹³, -OR¹⁴, -SR¹⁵;-C(=O)-OR²²;
oder für einen Rest ausgewählt aus der Gruppe bestehend aus Methyl, -CF₃, -CCl₃, - CBr₃, -CHF₂, -CH₂F, -CF₂Cl, -CCl₂F, Ethyl, -CF₂-CH₃, -CH₂-CF₃, -C₂F₅, -CH₂-CCl₃, - CH₂-CBr₃, -CHF-CF₂Cl, -CF₂-CF₂Cl, -CFCl-CF₂Cl, n-Propyl, -CF₂-CF₂-CF₃, - CF(CF₃)₂, Isopropyl, sek-Butyl, Isobutyl, tert-Butyl, -CH₂-CH₂-CH₂-CF₃, -CH₂-CH₂-CH₂-CH₂-CF₃, -CH₂-C(=O)-O-CH₃, -CH₂-C(=O)-C₂H₅, -CH₂-C(=O)-C(CH₃)₃, -CH₂-O-C(=O)-CH₃, -CH₂-O-C(=O)-C₂H₅, -CH₂-O-C(=O)-CH(CH₃)₂, -CH₂-O-C(=O)-C(CH₃)₃, n-Butyl, 3-Methyl-but-1-yl, 4-Methyl-pent-1-yl, n-Pentyl, n-Hexyl, (3,3)-Dimethyl-but-1-yl, (3,3)-Dimethyl-but-1-inyl, 4-Methyl-pent-1-inyl, 1-Hexinyl, Propinyl, Ethinyl, Butinyl, Pentinyl, 2-Methyl-propen-1-yl, 3-Methyl-but-2-en-1-yl, 1-Pentenyl, 1-Octenyl, 1-Heptenyl, 1-Hexenyl und (3,3)-Dimethyl-but-1-enyl;
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂Hs, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus (1,3)-Benzodioxolyl, (1,4)-Benzodioxanyl, Indolyl, Tetrazolyl, (2,3rDihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂)-, - (CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, - N(C₂H₅)₂, -NH-S(=O)₂-CH3, -NH-S(=O₂)-C₂H₅, -NH-S(=O)₂-CH(CH₃)₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R⁹ für H; F; Cl; Br; I; -NO₂, -CN; oder für einen Rest ausgewählt aus der Gruppe bestehend aus Methyl, -CF₃, -CCl₃, -CBr₃, -CHF₂, -CH₂F, -CF₂Cl, -CCl₂F, Ethyl n-Propyl, -CF₂-CF₂-CF₃, -CF(CF₃)₂, Isopropyl, sek-Butyl, Isobutyl und tert-Butyl steht;
R¹⁰ für -CN; -OH; -NH₂; -NO₂; -NHR¹¹; -NR¹²R¹³; -OR¹⁴; -SR¹⁵; -C(=O)-OR²²;
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C=C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-(C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus (1,3)-Benzodioxolyl, (1,4)-Benzodioxanyl, Indolyl, Tetrazolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂)-, - (CHF2- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, - N(C₂H₅)₂, -NH-S(=O)₂-CH3, -NH-S(=O₂)-C₂H₅, -NH-S(=Oh-CH(CH₃)₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R¹¹, R¹², R¹³, R¹⁴, R¹⁵ und R²², unabhängig voneinander, jeweils
für einen Rest ausgewählt aus der Gruppe bestehend aus Methyl, Ethyl, n-Propyl, Isopropyl, n-Butyl, sek-Butyl, Isobutyl, tert-Butyl; n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus 2,3-Dihydro-1 H-indenyl, Cyclopropyl, Oxetanyl, Cyclobutyl, Cyclopentyl, Cyclohexyl, Pyrrolidinyl, Piperidinyl, Morpholinyl, Piperazinyl, Azepanyl, Diazepanyl, Azocanyl und Thiomorpholinyl stehen, der jeweils über eine -CH₂-O-, -CH₂-CH₂-O-, -CH₂-CH₂-O-CH₂-, -CH₂-CH(CH₃)-O-CH₂-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, lsopropyl, n-Butyl, sek-Butyl, -C(=O)-O-CH₃, -C(=O)-O-C₂H₅, -C(=O)-O-CH(CH₃n, -C(=O)-O-C(CH₃)₃ substituiert sein kann;
oder für einen Rest ausgewählt aus der Gruppe bestehend aus -(CH₂)-Pyridinyl, - (CH₂)₂-Pyridinyl, Benzyl, Phenethyl, Phenyl, Naphthyl, Oxazolyl, Thiazolyl, Imidazolyl, Pyrimidinyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, stehen;
oder
R¹² und R¹³ jeweils zusammen mit dem sie verbindenden Stickstoffatom als Ringglied einen Rest ausgewählt aus der Gruppe bestehend aus 3-Aza-bicyclo[3.1.1]heptyl 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, Piperazinyl, Morpholinyl, Azepanyl, Diazepanyl und Thiomorpholinyl bilden, der ggf. jeweils mit 1, 2, 3, 4 oder 5 Substituenten unabhängig voneinander ausgewählt aus der Gruppe bestehend aus -CH₂O-CH₂-Oxetanyl, -O-CH₂-Oxetanyl, -CH₂-OH, _CH₂-CH₂-OH, =CH₂, (4,5)-Dihydroisoxazolyl, Thiazolyl, (1,2,5)-Thiadiazolyl, Thiophenyl, Phenethyl,
-CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, - NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, - C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -NH-Phenyl, -N(CH₃)-Phenyl, - N(C₂H₅)-Phenyl, -N(C₂H₅)-Phenyl, -O-CH₂-CH₂-CH₂-CH₃, Cyclohexyl, Cyclopentyl, Piperidinyl, Pyrrolidinyl, -O-C(=O)-CH₃, -O-C(=O)-C₂H₅, -O-C(=O)-C(CH₃)₃, -(CH₂)-Pyridinyl, Pyridinyl, -O-Phenyl, -O-Benzyl, Phenyl und Benzyl substituiert sein, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N-[C(=O)-C₂H₅]-Phenyl, -N-[C(=O)-CH₃]-Phenyl, -NH-Phenyl, -N(CH₃)-Phenyl, -N(C₂H₅)-Phenyl, -(CH₂)-Pyridinyl, Pyridinyl, -O-Phenyl, -O-Benzyl, Phenyl und Benzyl mit 1, 2, 3, 4 oder 5
Substituenten unabhängig voneinander ausgewählt aus der Gruppe bestehend aus - CF₃, F, Cl, Br, -OH, -CF₃, -SF₅, -CN, -NO₂, Methyl, Ethyl, n-Propyl, Isopropyl, n-Butyl, sek-Butyl, Isobutyl, tert-Butyl, -O-CH₃, -O-C₂H₅, -O-CH(CH₃)₂, -O-C(CH₃)₃, -O-CF₃, - S-CF₃, Phenyl und -O-Benzyl substituiert sein kann;
und
R²⁵ und R²⁶, unabhängig voneinander, jeweils für einen Wasserstoff-Rest;
für einen Alkyl-Rest ausgewählt aus der Gruppe bestehend aus -CH₂-OH, -CH₂-CH₂-OH, -CH₂-CH₂-CH₂-OH, -CH₂-CH₂-CH₂-CH₂-OH, Isopropyl, n-Butyl, sek-Butyl, Isobutyl, Methyl, Ethyl und n-Propyl oder für einen Rest ausgewählt aus der Gruppe bestehend aus Phenyl, Benzyl, Phenethyl, Cyclopropyl, Cyclobutyl, Cyclopentyl und Cyclohexyl stehen;
mit der Maßgabe, dass R²⁵ und R²⁶ nicht jeweils für einen Wasserstoff-Rest stehen;
oder
R²⁵ und R²⁶ jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
und R³⁰, R³¹ und R³², unabhängig voneinander, jeweils für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind Verbindungen der vorstehend angegebenen allgemeinen Formeln I, B1 und B2, worin
X für O oder S steht;
n für 0, 1 oder 2 steht;
R¹, R³ und R⁴ jeweils für H stehen;
R² für F; Cl; Br; I oder für einen Rest ausgewählt aus der Gruppe bestehend aus Methyl, -CF₃, -CCl₃, -CBr₃, -CHF₂, -CH₂F, -O-CH₃, -O-CF₃, -O-CCl₃, -O-CBr₃, -O-CHF₂, -O-CH₂F, -S-CH₃, -S-CF₃, -S-CCl₃, -S-CBr₃, -S-CHF₂, -S-CH₂F, -S-CF₂Cl und - S-CCl₂F steht;
R⁵ für F; Cl; Br; I; -SF₅;
für einen Rest ausgewählt aus der Gruppe bestehend aus Methyl, Ethyl, -CF₃, -CCl₃, -CBr₃, -CHF₂, -CH₂F, -CF₂Cl, -CCl₂F, -C(CH₃)₂(CH₂OH), tert-Butyl, -O-CF₃, -O-CCl₃, - O-CBr₃, -O-CHF₂, -O-CH₂F, -O-CF₂Cl, -O-CCl₂F, -O-CF₂-CH₃, -S-CF₃, -S-CCl₃, -S-CBr₃, -S-CHF₂, -S-CH₂F, -S-CF₂Cl, -S-CCl₂F, -S-CF₂-CH₃, -S(=O)₂-CF₃, -S(=O)₂-CCl₃, -S(=O)₂-CBr₃, -S(=O)₂-CHF₂, -S(=O)₂-CH₂F und -S(=O)₂-CF₂Cl;
oder für einen Rest ausgewählt aus der Gruppe bestehend aus Cyclopropyl, Cyclobutyl, Cyclopentyl, Cyclohexyl, Cyclopentenyl und Cyclohexenyl steht;
T für C-R⁶ und U für C-R⁷ und V für N und W für C-R⁸
oder
T für C-R⁶ und U für N und V für C-R⁹ und W für C-R⁸
oder
T für N und U für C-R⁷ und V für C-R⁹ und W für C-R⁸
oder
T für N und U für N und V für C-R⁹ und W für C-R⁸
oder
T für N und U für C-R⁷ und V für N und W für C-R⁸
oder
T für C-R⁶ und U für N und V für N und W für C-R⁸
oder
T für C-R⁶ und U für C-R⁷ und V für C-R⁹ und W für C-R¹⁰
steht;
R⁶ und R⁷ jeweils für -CF₃; Phenyl; -C(=O)-OCH₃; -C(=O)-OC₂H₅; Methyl; -CH₂-OH; H; F; Cl; Br und I stehen;
R⁸ für H; F; Cl; Br; I; -CN; -OH; -NH₂; -NO₂; -NHR¹¹; -NR¹²R¹³; -OR¹⁴; -SR¹⁵; - C(=O)-OR²²;
oder für einen Rest ausgewählt aus der Gruppe bestehend aus Methyl, -CF₃, -CCl₃, - CBr₃, -CHF₂, -CH₂F, -CF₂Cl, -CCl₂F, Ethyl, -CF₂-CH₃, -CH₂-CF₃, -C₂F₅, -CH₂-CCl₃, - CH₂-CBr₃, -CHF-CF₂Cl, -CF₂-CF₂Cl, -CFCl-CF₂Cl, n-Propyl, -CF₂-CF₂-CF₃, - CF(CF₃)₂, Isopropyl, sek-Butyl, Isobutyl, tert-Butyl, -CH₂-CH₂-CH₂-CF₃, -CH₂-CH₂-CH₂-CH₂-CF₃, -CH₂-C(=O)-O-CH₃, -CH₂-C(=O)-C₂H₅, -CH₂-C(=O)-C(CH₃)₃, -CH₂-O-C(=O)-CH₃, -CH₂-O-C(=O)-C₂H₅, -CH₂-O-C(=O)-CH(CH₃)₂, -CH₂-O-C(=O)-C(CH₃)₃, n-Butyl, n-Pentyl, n-Hexyl, (3,3)-Dimethyl-but-1-yl, 4-Methyl-pent-1-yl, (3,3)-Dimethyl-but-1-inyl, 4-Methyl-pent-1-inyl, 1-Hexinyl, Propinyl, Ethinyl, Butinyl, Pentinyl, 2-Methyl-propen-1-yl, 3-Methyl-but-2-en-1-yl, 1-Pentenyl, 1-Octenyl, 1-Heptenyl, 1-Hexenyl und (3,3)-Dimethyl-but-1-enyl;
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine - (CH=CH)-, -C=C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, - C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus (1,3)-Benzodioxolyl, (1,4)-Benzodioxanyl, Indolyl, Tetrazolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂)-, - (CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, - N(C₂H₅)₂, -NH-S(=O)₂-CH3, -NH-S(=O₂)-C₂H₅, -NH-S(=O)₂-CH(CH₃)₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R⁹ für -CF₃; H; F; Cl; Br oder I steht;
R¹⁰ für -CN; -NH₂; -NO₂; -NHR¹¹; -NR¹²R¹³; -OR¹⁴; -SR¹⁵; -C(=O)-OR²²;
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine - (CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, - C(=O)-CH₃, -C(=O)-C₂H₅, -C(=O)-C(CH₃)₃, -CN, -CH₂-N(CH₃)₂, -CH₂-N(C₂H₅)₂. -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus (1,3)-Benzodioxolyl, (1,4)-Benzodioxanyl, Indolyl, Tetrazolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂)-, - (CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, - N(C₂H₅)₂, -NH-S(=O)₂-CH₃, -NH-S(=O₂)-C₂H₅, -NH-S(=O)₂-CH(CH₃)₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht; R¹¹, R¹², R¹³, R¹⁴, R¹⁵ und R²², unabhängig voneinander, jeweils für einen Rest ausgewählt aus der Gruppe bestehend aus Methyl, Ethyl, n-Propyl, Isopropyl, n-Butyl, sek-Butyl, Isobutyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl, 3-Pentenyl und tert-Butyl stehen;
für einen Rest ausgewählt aus der Gruppe bestehend aus 2,3-Dihydro-1H-indenyl, Piperidinyl, Pyrrolidinyl, Cyclopropyl, Oxetanyl, Cyclobutyl, Cyclopentyl und Cyclohexyl stehen, der jeweils über eine -CH₂-O-, -CH₂-CH₂-O-, -CH₂-CH₂-O-CH₂-, - CH₂-CH(CH₃)-O-CH₂-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, - (CH₂)₂-Pyridinyl,Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF3, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R¹² und R¹³ jeweils zusammen mit dem sie verbindenden Stickstoffatom als Ringglied einen Rest ausgewählt aus der Gruppe bestehend aus 3-Azabicyclo[3.1.1]heptyl 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl, -CH₂-OH, -CH₂-CH₂-OH, =CH₂, (4,5)-Dihydroisoxazolyl, Thiazolyl, (1,2,5)-Thiadiazolyl, Thiophenyl, Phenethyl, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl_{;} CH₂-O-CH₃, -CH₂-O-CH₂-CH₃, Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, - NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, - C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -NH-Phenyl, -N(CH₃)-Phenyl, - N(C₂H₅)-Phenyl, -N(C₂H₅)-Phenyl, -O-CH₂-CH₂-CH₂-CH₃, Cyclohexyl, Cyclopentyl, Piperidinyl, Pyrrolidinyl, -O-C(=O)-CH₃, -O-C(=O)-C₂H₅, -O-C(=O)-C(CH₃)₃, -(CH₂)-Pyridinyl, Pyridinyl, -O-Phenyl, -O-Benzyl, Phenyl und Benzyl substituiert sein, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N-[C(=O)-C₂H₅]-Phenyl, -N-[C(=O)-CH₃]-Phenyl, -NH-Phenyl, -N(CH₃)-Phenyl, -N(C₂H₅)-Phenyl, -(CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus - CF₃, F, Cl, Br, -OH, -CF₃, -SF₅, -CN, -NO₂, Methyl, Ethyl, n-Propyl, Isopropyl, n-Butyl, sek-Butyl, Isobutyl, tert-Butyl, -O-CH₃, -O-C₂H₅, -O-CH(CH₃)₂, -O-C(CH₃)₃, -O-CF₃, - S-CF₃, Phenyl und -O-Benzyl substituiert sein kann;
und
R²⁵ und R²⁶, unabhängig voneinander, jeweils für einen Wasserstoff-Rest; für einen Alkyl-Rest ausgewählt aus der Gruppe bestehend aus -CH₂-OH, -CH₂-CH₂-OH, -CH₂-CH₂-CH₂-OH, -CH₂-CH₂-CH₂-CH₂-OH, Isopropyl, n-Butyl, sek-Butyl, Isobutyl, Methyl, Ethyl und n-Propyl oder für einen Rest ausgewählt aus der Gruppe bestehend aus Phenyl, Benzyl, Phenethyl, Cyclopropyl, Cyclobutyl, Cyclopentyl und Cyclohexyl stehen;
mit der Maßgabe, dass R²⁵ und R²⁶ nicht jeweils für einen Wasserstoff-Rest stehen; oder
R²⁵ und R²⁶ jeweils zusammen mit dem sie verbindenden Kohlenstoffatom als Ringglied einen Rest ausgewählt aus der Gruppe bestehend aus Cyclopropyl,
Cyclobutyl, Cyclopentyl, Cyclohexyl und Cycloheptyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind Verbindungen der vorstehend angebenen allgemeinen Formeln I, B1 und B2, worin
X für O steht;
n für 1 steht;
R¹, R³ und R⁴ jeweils für H stehen;
R² für Methyl; -O-CH₃; F; Cl; Br oder I steht;
R⁵ für einen Rest ausgewählt aus der Gruppe bestehend aus Methyl, Ethyl, -CF₃, -CCl₃, -CBr₃, -CHF₂, -CH₂F, -CF₂Cl, -CCl₂F, -C(CH₃)₂(CH₂OH), tert-Butyl, -O-CF₃, -O-CCl₃, -O-CBr₃, -O-CHF₂, -O-CH₂F, -S-CF₃, -S-CCl₃, -S-CBr₃, -S-CHF₂ und -S-CH₂F steht;
oder für einen Rest ausgewählt aus der Gruppe bestehend aus Cyclopropyl, Cyclobutyl, Cyclopentyl, Cyclohexyl, Cyclopentenyl und Cyclohexenyl steht;
T für CH und U für CH und V für N und W für C-R⁸
oder
T für CH und U für N und V für CH und W für C-R⁸
oder
T für N und U für CH und V für CH und W für C-R⁸
oder
T für N und U für N und V für CH und W für C-R⁸
oder
T für N und U für CH und V für N und W für C-R⁸
oder
T für CH und U für N und V für N und W für C-R⁸
oder
T für CH und U für CH und V für CH und W für C-R¹⁰
steht;
R⁸ für H; F; Cl; Br; I; -CN; -OH; -NH₂, -NO₂-; -NHR¹¹; -NR¹²R¹³; -OR¹⁴; -SR¹⁵;
oder für einen Rest ausgewählt aus der Gruppe bestehend aus Methyl, -CF₃, -CCl₃, - CBr₃, -CHF₂, -CH₂F, -CF₂Cl, -CCl₂F, Ethyl, -CF₂-CH₃, -CH₂-CF₃, -C₂F₅, -CH₂-CCl₃, - CH₂-CBr₃, -CHF-CF₂Cl, -CF₂-CF₂Cl, -CFCl-CF₂Cl, n-Propyl, -CF₂-CF₂-CF₃, - CF(CF₃)₂, Isopropyl, sek-Butyl, Isobutyl, tert-Butyl, -CH₂-CH₂-CH₂-CF₃, -CH₂-CH₂-CH₂-CH₂-CF₃, -CH₂-C(=O)-O-CH₃, -CH₂-C(=O)-C₂H₅, -CH₂-C(=O)-C(CH₃)₃, -CH₂-O-C(=O)-CH₃, -CH₂-O-C(=O)-C₂H₅, -CH₂-O-C(=O)-CH(CH₃)₂, -CH₂-O-C(=O)-C(CH₃)₃, n-Butyl, 3-Methyl-but-1-yl, 4-Methyl-pent-1-yl, n-Pentyl, n-Pentyl, n-Hexyl, (3,3)-Dimethyl-but-1-yl, (3,3)-Dimethyl-but-1-inyl, 4-Methyl-pent-1-inyl, 1-Hexinyl, Propinyl, Ethinyl, Butinyl, Pentinyl, 2-Methyl-propen-1-yl, 3-Methyl-but-2-en-1-yl, 1-Pentenyl, 1-Octenyl, 1-Heptenyl, 1-Hexenyl und (3,3)-Dimethyl-but-1-enyl;
für einen Rest ausgewählt aus der Gruppe bestehend aus Cyclopropyl,
Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl,Oxo (=O), Thioxo (=S), Methyl, Ethyl, n-Propyl, Isopropyl, n-Butyl, sek-Butyl, Isobutyl, tert-Butyl und n-Pentyl substituiert sein kann; oder für einen Rest ausgewählt aus der Gruppe bestehend aus Tetrazolyl, (1,3)-Benzodioxolyl, (1,4)-Benzodioxanyl, Indolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂)-, - (CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, - N(C₂H₅)₂, -NH-S(=O)₂-CH3, -NH-S(=O₂)-C₂H₅, -NH-S(=O)₂-CH(CH₃)₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R¹⁰ für-CN; -NH₂; -NO₂; -NHR¹¹; -NR¹²R¹³; -OR¹⁴; -SR¹⁵;
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃. -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl,Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Tetrazolyl, (1,3)-Benzodioxolyl, (1,4)-Benzodioxanyl, Indolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂)-, - (CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, - N(C₂H₅)₂, -NH-S(=O)₂-CH3, -NH-S(=O₂)-C₂H₅, -NH-S(=O)₂-CH(CH₃)₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R¹¹, R¹², R¹³, R¹⁴ und R¹⁵, unabhängig voneinander, jeweils
für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus 2,3-Dihydro-1H-indenyl, Piperidinyl, Pyrrolidinyl, Cyclopropyl, Oxetanyl, Cyclobutyl, Cyclopentyl und Cyclohexyl stehen, der jeweils über eine -CH₂-O-, -CH₂-CH₂-O-, -CH₂-CH₂-O-CH₂-, - CH₂-CH(CH₃)-O-CH₂-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, - (CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF3, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R¹² und R¹³ jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl,(1,2,3,6)-Tetrahydropyridinyl, (4,5,6,7)-Tetrahydroisoxazolo[5,4-c]pyridinyl, Pyrrolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl, -CH₂-OH, -CH₂-CH₂-OH, =CH₂, -C(=O)-O-CH₃, -C(=O)-O-C₂H₅, - C(=O)-O-C(CH₃)₃, -C(=O)-CH₃, -C(=O)-C₂H₅, -C(=O)-C(CH₃)₃, -CN, -CH₂-N(CH₃)₂, - CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, -NH₂, -NH-CH₃, -NH-C₂H₅, -N(CH₃)₂, - N(C₂H₅)₂, -NH-Phenyl, -N(CH₃)-Phenyl, -N(C₂H₅)-Phenyl, Oxo (=O), Thioxo (=S), - OH, F, Cl, Br, -CF₃, -O-CH₃, -O-C₂H₅, -O-C(CH₃)₃, -O-CH(CH₃)₂, -O-CH₂-CH₂-CH₂-CH₃, Methyl, Ethyl, Isopropyl, n-Propyl, n-Butyl, tert-Butyl, sek-Butyl, Cyclohexyl, Cyclopentyl, Piperidinyl, Pyrrolidinyl, -O-Phenyl, -O-C(=O)-CH₃, -O-C(=O)-C₂H₅, -O-C(=O)-C(CH₃)₃, -(CH₂)-Pyridinyl, (4,5)-Dihydroisoxazolyl, Thiazolyl, (1,2,5)-Thiadiazolyl, Thiophenyl, Phenethyl, Pyridinyl, Phenyl und Benzyl substituiert sein kann, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N-[C(=O)-C₂H₅]-Phenyl, -N-[C(=O)-CH₃]-Phenyl, -NH-Phenyl, -N(CH₃)-Phenyl, -N(C₂H₅)-Phenyl, -(CH₂)-Pyridinyl, Pyridinyl, Phenyl, -O-Phenyl und Benzyl ggf. mit 1, 2, 3, 4 oder 5 Substituenten unabhängig voneinander ausgewählt aus der Gruppe bestehend aus -CF₃, -OH, -O-CH₃, -O-C₂H₅, F, Cl, Br, Methyl, Ethyl, Isopropyl, n-Propyl, n-Butyl, tert-Butyl und sek-Butyl substituiert sein kann;
und
R²⁵ für einen Alkyl-Rest ausgewählt aus der Gruppe bestehend aus -CH₂-OH, -CH₂-CH₂-OH, Methyl, Ethyl und n-Propyl oder für einen Rest ausgewählt aus der Gruppe bestehend aus Benzyl, Phenyl, Phenethyl, Cyclopropyl, Cyclobutyl, Cyclopentyl und Cyclohexyl steht;
R²⁶ für einen Wasserstoff-Rest oder für einen Rest ausgewählt aus der Gruppe bestehend aus Methyl, Ethyl und n-Propyl steht;
oder
R²⁵ und R²⁶ jeweils zusammen mit dem sie verbindenden Kohlenstoffatom als Ringglied einen Rest ausgewählt aus der Gruppe bestehend aus Cyclopropyl, Cyclobutyl, Cyclopentyl und Cyclohexyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind Verbindungen der allgemeinen Formel Ia1, 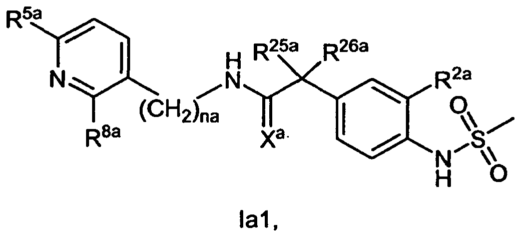 worin
X^{a} für O oder S steht;
na für 0, 1 oder 2 steht;
R^{2a} für F; Cl; Br; I oder für einen Rest ausgewählt aus der Gruppe bestehend aus Methyl, -CF₃, -CCl₃, -CBr₃, -CHF₂, -CH₂F, -O-CH₃, -O-CF₃, -O-CCl₃, -O-CBr₃, -O-CHF₂, -O-CH₂F, -S-CH₃, -S-CF₃, -S-CCl₃, -S-CBr₃, -S-CHF₂, -S-CH₂F, -S-CF₂Cl und - S-CCl₂F steht;
R^{5a} für F; Cl; Br; I; -SF₅;
für einen Rest ausgewählt aus der Gruppe bestehend aus Methyl, Ethyl, -CF₃, -CCl₃, -CBr₃, -CHF₂, -CH₂F, -CF₂Cl, -CCl₂F, -C(CH₃)₂-(CH₂OH), tert-Butyl, -O-CF₃, -O-CCl₃, -O-CBr₃, -O-CHF₂, -O-CH₂F, -O-CF₂Cl, -O-CCl₂F, -O-CF₂-CH₃, -S-CF₃, -S-CCl₃, -S-CBr₃, -S-CHF₂, -S-CH₂F, -S-CF₂Cl, -S-CCl₂F, -S-CF₂-CH₃, -S(=O)₂-CF₃, -S(=O)₂-CCl₃, -S(=O)₂-CBr₃, -S(=O)₂-CHF₂, -S(=O)₂-CH₂F und -S(=O)₂-CF₂Cl;
oder für einen Rest ausgewählt aus der Gruppe bestehend aus Cyclopropyl, Cyclobutyl, Cyclopentyl, Cyclohexyl, Cyclopentenyl und Cyclohexenyl steht;
R^{8a} für H; F; Cl; Br; I; -CN; -OH; -NH₂; -NO₂; -NHR^{11a}; -NR^{12a}R^{13a}; -OR^{14a}; -SR^{15a}; - C(=O)-OR^{22a};
oder für einen Rest ausgewählt aus der Gruppe bestehend aus Methyl, -CF₃, -CCl₃, - CBr₃, -CHF₂, -CH₂F, -CF₂Cl, -CCl₂F, Ethyl, -CF₂-CH₃, -CH₂-CF₃, -C₂F₅, -CH₂-CCl₃, - CH₂-CBr₃, -CHF-CF₂Cl, -CF₂-CF₂Cl, -CFCl-CF₂Cl, n-Propyl, -CF₂-CF₂-CF₃, - CF(CF₃)₂, Isopropyl, sek-Butyl, Isobutyl, tert-Butyl, -CH₂-CH₂-CH₂-CF₃, -CH₂-CH₂-CH₂-CH₂-CF₃, -CH₂-C(=O)-O-CH₃, -CH₂-C(=O)-C₂H₅, -CH₂-C(=O)-C(CH₃)₃, -CH₂-O-C(=O)-CH₃, -CH₂-O-C(=O)-C₂H₅, -CH₂-O-C(=O)-CH(CH₃)₂, -CH₂-O-C(=O)-C(CH₃)₃, n-Butyl, 3-Methyl-but-1-yl, 4-Methyl-pent-1-yl, n-Pentyl, n-Hexyl, (3,3)-Dimethyl-but-1-yl, (3,3)-Dimethyl-but-1-inyl, 4-Methyl-pent-l-inyl, 1-Hexinyl, Propinyl, Ethinyl, Butinyl, Pentinyl, 2-Methyl-propen-1-yl, 3-Methyl-but-2-en-1-yl, 1-Pentenyl, 1-Octenyl, 1-Heptenyl, 1-Hexenyl und (3,3)-Dimethyl-but-1-enyl;
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Tetrazolyl, (1,3)-Benzodioxolyl, (1,4)-Benzodioxanyl, Indolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂)-, - (CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, - N(C₂H₅)₂, -NH-S(=O)₂-CH3, -NH-S(=O₂)-C₂H₅, -NH-S(=O)₂-CH(CH₃)₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R^{11a}, R^{12a}, R^{13a}, R^{14a}, R^{15a} und R^{22a}, unabhängig voneinander, jeweils
für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus 2,3-Dihydro-1H-indenyl, Piperidinyl, Pyrrolidinyl, Cyclopropyl, Cyclobutyl, Cyclopentyl und Cyclohexyl stehen, der jeweils über eine -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, - (CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF₃, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R^{12a} und R^{13a} jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl,(1,2,3,6)-Tetrahydropyridinyl, (4,5,6,7)-Tetrahydroisoxazolo[5,4-c]pyridinyl, Pymolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl, -CH₂-OH, -CH₂-CH₂-OH, =CH₂, -C(=O)-O-CH₃, -C(=O)-O-C₂H₅, - C(=O)-O-C(CH₃)₃, -C(=O)-CH₃, -C(=O)-C₂H₅, -C(=O)-C(CH₃)₃, -CN, -CH₂-N(CH₃)₂, - CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl,-CH₂-O-CH₃, -CH₂-O-CH₂-CH₃, -NH₂, -NH-CH₃, -NH-C₂H₅, -N(CH₃)₂, - N(C₂H₅)₂, -NH-Phenyl, -N(CH₃)-Phenyl, -N(C₂H₅)-Phenyl, Oxo (=O), Thioxo (=S), - OH, F, CI, Br, -CF₃, -O-CH₃, -O-C₂H₅, -O-C(CH₃)₃, -O-CH(CH₃)₂, -O-CH₂-CH₂-CH₂-CH₃, Methyl, Ethyl, Isopropyl, n-Propyl, n-Butyl, tert-Butyl, sek-Butyl, Cyclohexyl, Cyclopentyl, (4,5)-Dihydroisoxazolyl, Thiazolyl, (1,2,5)-Thiadiazolyl, Thiophenyl, Phenethyl, Piperidinyl, Pyrrolidinyl, -O-Phenyl, -O-C(=O)-CH₃, -O-C(=O)-C₂H₅, -O-C(=O)-C(CH₃)₃, -(CH₂)-Pyridinyl, Pyridinyl, Phenyl und Benzyl substituiert sein kann, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N-[C(=O)-C₂H₅]-Phenyl, -N-[C(=O)-CH₃]-Phenyl,-NH-Phenyl, -N(CH₃)-Phenyl, -N(C₂H₅)-Phenyl, -(CH₂)-Pyridinyl, Pyridinyl, Phenyl, -O-Phenyl und Benzyl ggf. mit 1, 2, 3, 4 oder 5 Substituenten unabhängig voneinander ausgewählt aus der Gruppe bestehend aus -CF₃, -OH, -O-CH₃, -O-C₂H₅, F, Cl, Br, Methyl, Ethyl, Isopropyl, n-Propyl, n-Butyl, tert-Butyl und sek-Butyl substituiert sein kann;
R^{25a} und R^{26a}, unabhängig voneinander, jeweils für einen Wasserstoff-Rest;
für einen Alkyl-Rest ausgewählt aus der Gruppe bestehend aus -CH₂-OH, -CH₂-CH₂-OH, -CH₂-CH₂-CH₂-OH, -CH₂-CH₂-CH₂CH₂-OH, Isopropyl, n-Butyl, sek-Butyl, Isobutyl, Methyl, Ethyl und n-Propyl;
mit der Maßgabe, dass R^{25a} und R^{26a} nicht jeweils für einen Wasserstoff-Rest stehen; oder
R^{25a} und R^{26a} jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind Verbindungen der allgemeinen Formel Ia, 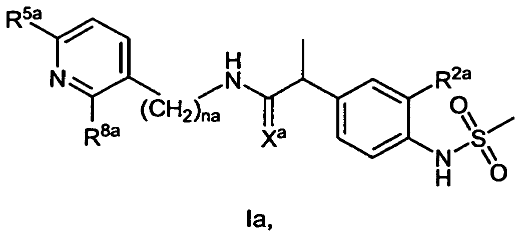 worin
X^{a}, R^{5a}, R^{8a}, na und R^{2a} wie vorstehend definiert sind;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls besonders bevorzugt sind Verbindungen der allgemeinen Formel la, worin X^{a} für O oder S steht;
na für 0, 1 oder 2 steht;
R^{2a} für F; Cl; Br; I oder für einen Rest ausgewählt aus der Gruppe bestehend aus Methyl, -CF₃, -CCl₃, -CBr₃, -CHF₂, -CH₂F, -O-CH₃, -O-CF₃, -O-CCl₃, -O-CBr₃, -O-CHF₂, -O-CH₂F, -S-CH₃, -S-CF₃, -S-CCl₃, -S-CBr₃, -S-CHF₂, -S-CH₂F, -S-CF₂Cl und - S-CCl₂F steht;
R^{5a} für F; Cl; Br, I; -SF₅;
für einen Rest ausgewählt aus der Gruppe bestehend aus Methyl, Ethyl, -CF₃, -CCl₃, -CBr₃, -CHF₂, -CH₂F, -CF₂Cl, -CCl₂F, -C(CH₃)₂-(CH₂OH), tert-Butyl, -O-CF₃, -O-CCl₃, -O-CBr₃, -O-CHF₂, -O-CH₂F, -O-CF₂Cl, -O-CCl₂F, -O-CF₂-CH₃, -S-CF₃, -S-CCl₃, -S-CBr₃, -S-CHF₂, -S-CH₂F, -S-CF₂Cl, -S-CCl₂F, -S-CF₂-CH₃, -S(=O)₂-CF₃, -S(=O)₂-CCl₃, -S(=O)₂-CBr₃, -S(=O)₂-CHF₂, -S(=O)₂-CH₂F und -S(=O)₂-CF₂Cl;
oder für einen Rest ausgewählt aus der Gruppe bestehend aus Cyclopropyl, Cyclobutyl, Cyclopentyl, Cyclohexyl, Cyclopentenyl und Cyclohexenyl steht;
R^{8a} für H; F; Cl; Br; I; -CN; -OH; -NH₂; -NO₂; -NHR^{11a}; -NR^{12a}R^{13a}; -OR^{14a}, -SR^{15a}; - C(=O)-OR^{22a};
oder für einen Rest ausgewählt aus der Gruppe bestehend aus Methyl, -CF₃, -CCl₃, - CBr₃, -CHF₂, -CH₂F, -CF₂Cl, -CCl₂F, Ethyl, -CF₂-CH₃, -CH₂-CF₃, -C₂F₅, -CH₂-CCl₃, - CH₂-CBr₃, -CHF-CF₂Cl, -CF₂-CF₂Cl, -CFCl-CF₂Cl, n-Propyl, -CF₂-CF₂-CF₃, - CF(CF₃)₂, Isopropyl, sek-Butyl, Isobutyl, tert-Butyl, -CH₂CH₂CH₂-CF₃, -CH₂-CH₂-CH₂-CH₂-CF₃, -CH₂-C(=O)-O-CH₃, -CH₂-C(=O)-C₂H₅, -CH₂-C(=O)-C(CH₃)₃, -CH₂-O-C(=O)-CH₃, -CH₂-O-C(=O)-C₂H₅, -CH₂-O-C(=O)-CH(CH₃)₂, -CH₂-O-C(=O)-C(CH₃)₃, n-Butyl, 3-Methyl-but-1-yl, 4-Methyl-pent-1-yl, n-Pentyl, n-Hexyl, (3,3)-Dimethyl-but-1-yl, (3,3)-Dimethyl-but-1-inyl, 4-Methyl-pent-1-inyl, 1-Hexinyl, Propinyl, Ethinyl, Butinyl, Pentinyl, 2-Methyl-propen-1-yl, 3-Methyl-but-2-en-1-yl, 1-Pentenyl, 1-Octenyl, 1-Heptenyl, 1-Hexenyl und (3,3)-Dimethyl-but-1-enyl;
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Tetrazolyl, (1,3)-Benzodioxolyl, (1,4)-Benzodioxanyl, (2,3)-Dihydrothieno[3,4-b][1,4]dioxinyl, Benzo[b]furanyl, Indolyl, Phenyl, Naphthyl, Oxazolyl, Thiazolyl; Imidazolyl, Pyrimidinyl, Thiophenyl, Furanyl und Pyridinyl, der jeweils über eine -(CH=CH)-, - C=C-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - N(CH₃)₂, -N(C₂H₅)₂, -NH-S(=O)₂-CH3, -NH-S(=O₂)-C₂H₅, -NH-S(=O)₂-CH(CH₃)₂, - N0₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R^{11a}, R^{12a}, R^{13a}, R^{14a}, R^{15a} und R^{22a}, unabhängig voneinander, jeweils
für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus 2,3-Dihydro-1 H-indenyl, Piperidinyl, Pyrrolidinyl, Cyclopropyl, Cyclobutyl, Oxetanyl, Cyclopentyl und Cyclohexyl stehen, der jeweils über eine -CH₂-O-, -CH₂-CH₂-O-, -CH₂-CH₂-O-CH₂-, - CH₂-CH(CH₃)-O-CH₂-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, - (CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF₃, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R¹², und R^{13a} jeweils zusammen mit dem sie verbindenden Stickstoffatom einen Rest bilden ausgewählt aus der Gruppe bestehend aus

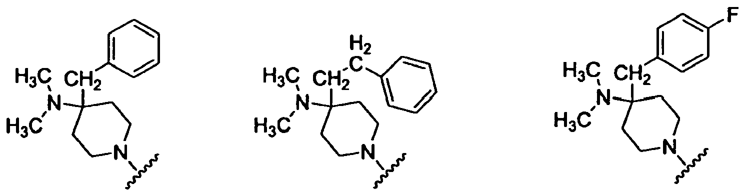

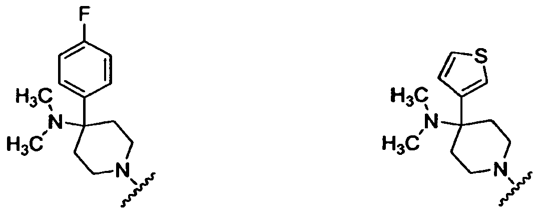

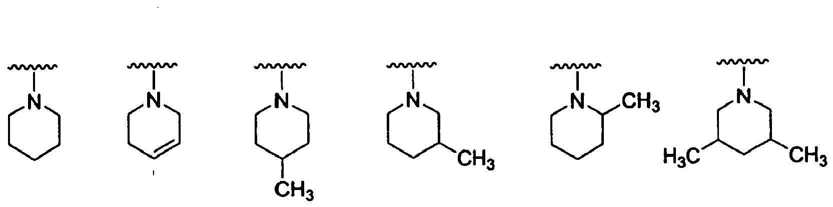

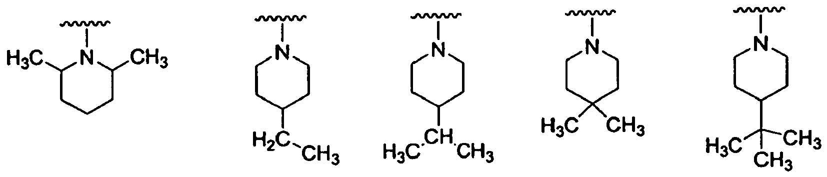

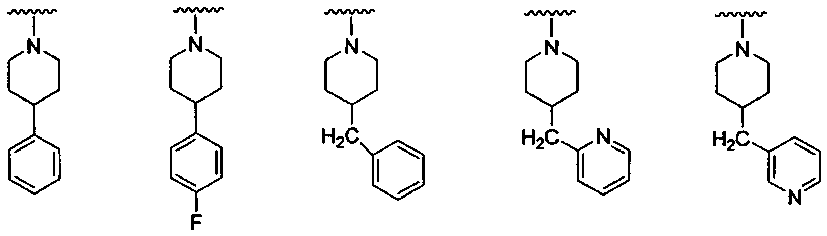

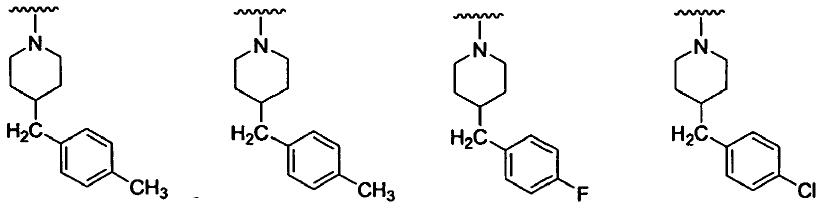

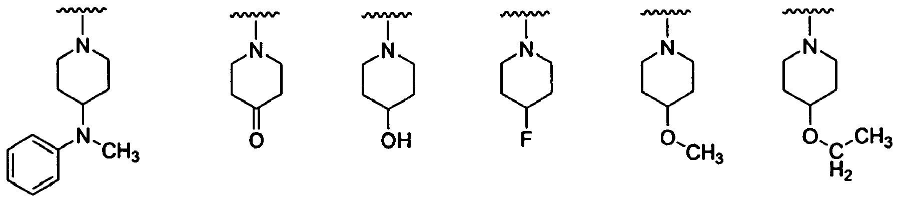

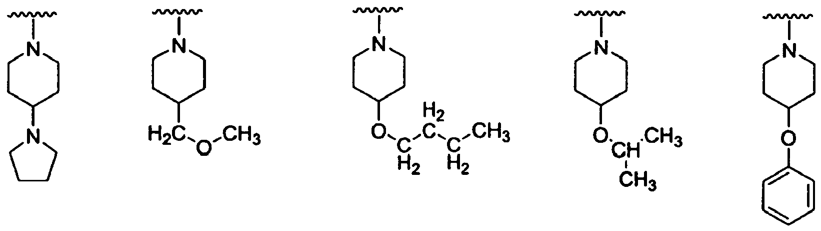

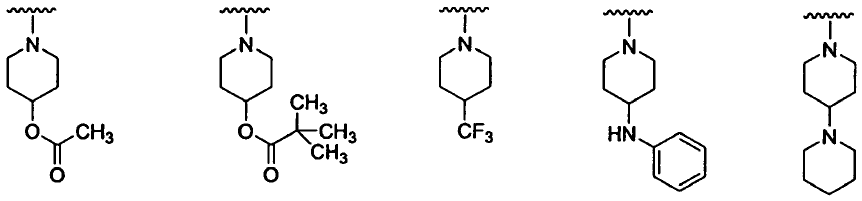

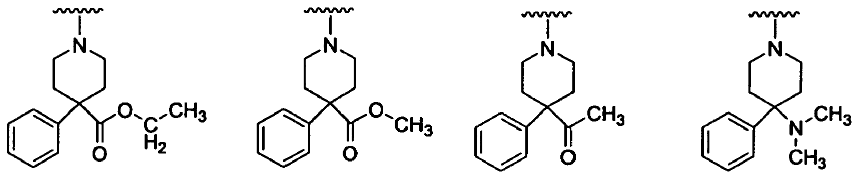

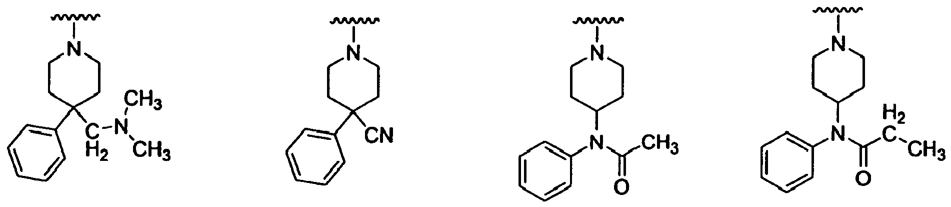

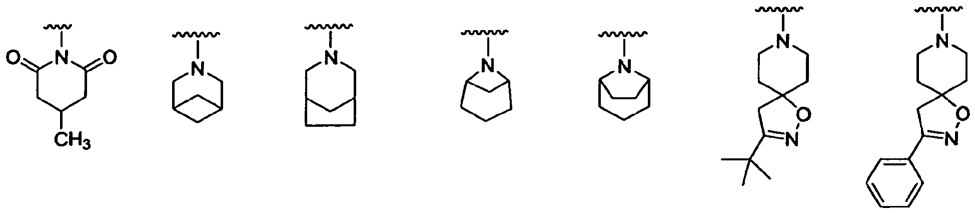

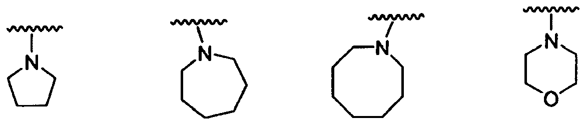

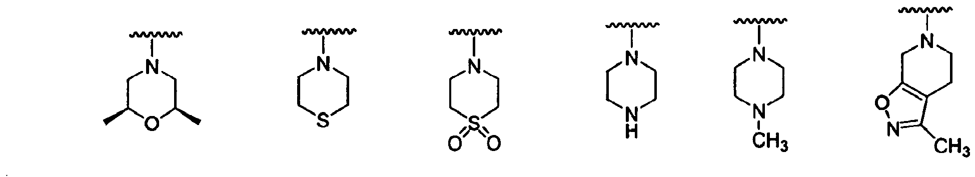

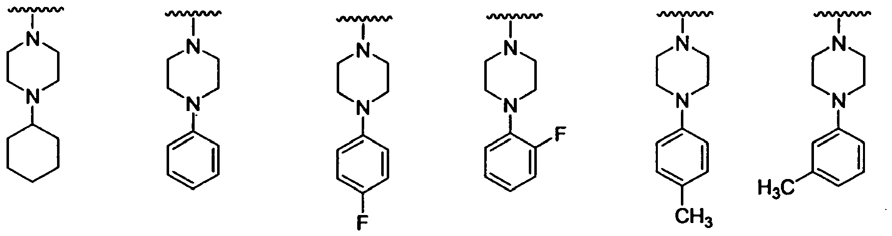

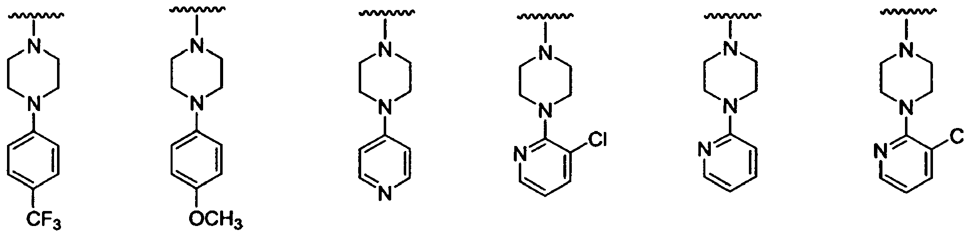

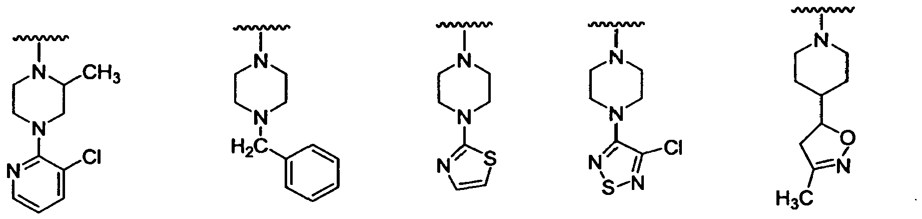

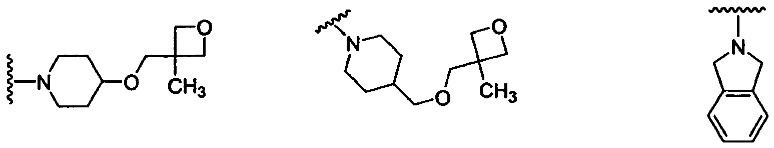

Sofern die Synthese der vorstehend genannten Reste in Position der Substituenten R¹² und R¹³ nicht im Experimentalteil angegeben ist, sind die entsprechenden Synthesen dem Fachmann bekannt.

Besonders bevorzugt sind Verbindungen der allgemeinen Formel C1. 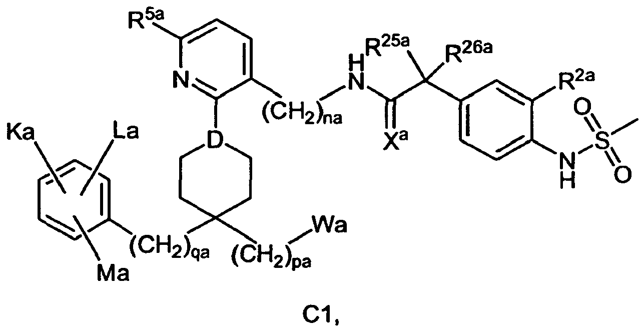 worin
na, R^{2a}, R^{25a}, R^{26a}, R^{5a} und X^{a} wie vorstehend definiert sind;
D für CH oder N steht;
pa für 0 steht,
qa für 0, 1 oder 2 steht;
Ka, La und Ma, unabhängig voneinander, jeweils für H, -CF₃, -OH, -O-CH₃, -O-C₂H₅, F, Cl, Br, Methyl, Ethyl, Isopropyl, n-Propyl, n-Butyl, tert-Butyl oder sek-Butyl stehen;
Wa für -NR^{34a}R^{35a}, -CN, -C(=O)-R^{36a} oder -C(=O)-OR^{37a} steht;
und R^{34a}, R^{35a}, R^{36a} und R^{37a}, unabhängig voneinander, jeweils für H oder für einen Rest ausgewählt aus der Gruppe bestehend aus Methyl, Ethyl, n-Propyl, Isopropyl, n-Butyl, sek-Butyl und Isobutyl stehen.

Ebenfalls besonders bevorzugt sind Verbindungen der allgemeinen Formel C2, 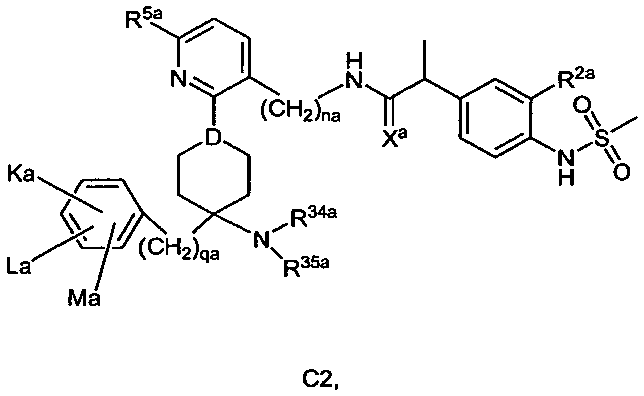 worin
na, R^{2a}, R^{5a} und X^{a} wie vorstehend definiert sind;
D für CH oder N steht;
qa für 0, 1 oder 2 steht,
Ka, La und Ma, unabhängig voneinander, jeweils für H, -CF₃, -OH, -O-CH₃, -O-C₂H₅, F, Cl, Br, Methyl, Ethyl, Isopropyl, n-Propyl, n-Butyl, tert-Butyl oder sek-Butyl stehen;
und R^{34a} und R^{35a}, unabhängig voneinander, jeweils für H oder für einen Rest ausgewählt aus der Gruppe bestehend aus Methyl, Ethyl, n-Propyl, Isopropyl, n-Butyl, sek-Butyl und Isobutyl stehen.

Die Verbindungen der allgemeinen Formeln B1, B2, C1 und C2 besitzen neben einer Affinität zum VR1-Rezeptor ebenfalls eine Affinität zum µ-Opioid-Rezeptor.

Besonders bevorzugt sind Verbindungen der allgemeinen Formel Ib1, worin 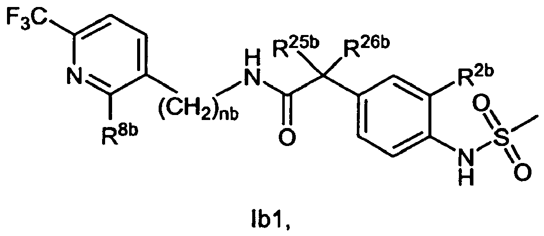 worin
nb für 0, 1 oder 2 steht;
R^{2b} für Methyl, -O-CH₃, F; Cl; Br oder I steht;
R^{8b} für H; F; Cl; Br; I; -CN; -OH; -NH₂; -NO₂; -NHR^{11b}; -NR^{12b}R^{13b}; -OR^{14b}; -SR^{15b};
für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, sek-Butyl, Isobutyl, tert-Butyl, -CH₂-CH₂-CH₂-CF₃, -CH₂-CH₂-CH₂-CH₂-CF₃, -CH₂-C(=O)-O-CH₃, -CH₂-C(=O)-C₂H₅, -CH₂-C(=O)-C(CH₃)₃, -CH₂-O-C(=O)-CH₃, - CH₂-O-C(=O)-C₂H₅, -CH₂-O-C(=O)-CH(CH₃)₂, -CH₂-O-C(=O)-C(CH₃)₃, n-Butyl, n-Pentyl, n-Hexyl, (3,3)-Dimethyl-but-1-yl, 3-Methyl-but-1-yl, 4-Methyl-pent-1-yl, (3,3)-Dimethyl-but-1-inyl, 4-Methyl-pent-1-inyl, 1-Hexinyl, Propinyl, Ethinyl, Butinyl, Pentinyl, 2-Methyl-propen-1-yl, 3-Methyl-but-2-en-1-yl, 1-Pentenyl, 1-Octenyl, 1-Heptenyl, 1-Hexenyl und (3,3)-Dimethyl-but-l-enyl;
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Tetrazolyl, (1,3)-Benzodioxolyl, (1,4)-Benzodioxanyl, Indolyl, (2,3)-Dihydrothieno[3,4-b][1 ,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂)-, - (CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, - N(C₂H₅)₂, -NH-S(=O)₂-CH3, -NH-S(=O₂)-C₂H₅, -NH-S(=O)₂-CH(CH₃)₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R^{11b}, R^{12b}, R^{13b}, R^{14b} und R^{15b}, unabhängig voneinander, jeweils
für einen Rest ausgewählt aus der Gruppe bestehend aus Methyl, Ethyl, n-Propyl, lsopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus Oxetanyl, 2,3-Dihydro-1 H-indenyl, Piperidinyl, Pyrrolidinyl, Cyclopropyl, Cyclobutyl, Cyclopentyl und Cyclohexyl stehen, der jeweils über eine -CH₂-O-, -CH₂-CH₂-O-, -CH₂-CH₂-O-CH₂-, -CH₂-CH(CH₃)-O-CH₂-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, - (CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF3, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R^{12b} und R^{13b} jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, lsoindolyl, Indolyl,(1,2,3,6)-Tetrahydropyridinyl, (4,5,6,7)-Tetrahydroisoxazolo[5,4-c]pyridinyl, Pyrrolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl, -CH₂-OH, -CH₂-CH₂-OH, =CH₂, -C(=O)-O-CH₃, -C(=O)-O-C₂H₅, - C(=O)-O-C(CH₃)₃, -C(=O)-CH₃, -C(=O)-C₂H₅, -C(=O)-C(CH₃)₃, -CN, -CH₂-N(CH₃)₂, - CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, -NH₂, -NH-CH₃, -NH-C₂H₅, -N(CH₃)₂, - N(C₂H₅)₂, -NH-Phenyl, -N(CH₃)-Phenyl, -N(C₂H₅)-Phenyl, Oxo (=O), Thioxo (=S), - OH, F, Cl, Br, -CF₃, -O-CH₃, -O-C₂H₅, -O-C(CH₃)₃, -O-CH(CH₃)₂, -O-CH₂-CH₂-CH₂-CH₃, Methyl, Ethyl, Isopropyl, n-Propyl, n-Butyl, tert-Butyl, sek-Butyl, (4,5)-Dihydroisoxazolyl, Thiazolyl, (1,2,5)-Thiadiazolyl, Thiophenyl, Phenethyl, Cyclohexyl, Cyclopentyl, Piperidinyl, Pyrrolidinyl, -O-Phenyl, -O-C(=O)-CH₃, -O-C(=O)-C₂H₅, -O-C(=O)-C(CH₃)₃, -(CH₂)-Pyridinyl, Pyridinyl, Phenyl und Benzyl substituiert sein kann, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N-[C(=O)-C₂H₅]-Phenyl, -N-[C(=O)-CH₃]-Phenyl, -NH-Phenyl, -N(CH₃)-Phenyl, -N(C₂H₅)-Phenyl, -(CH₂)-Pyridinyl, Pyridinyl, Phenyl, -O-Phenyl und Benzyl ggf. mit 1, 2, 3, 4 oder 5 Substituenten unabhängig voneinander ausgewählt aus der Gruppe bestehend aus -CF₃, -OH, -O-CH₃, -O-C₂H₅, F, Cl, Br, Methyl, Ethyl, Isopropyl, n-Propyl, n-Butyl, tert-Butyl und sek-Butyl substituiert sein kann;
R^{25b} und R^{26b}, unabhängig voneinander, jeweils für einen Wasserstoff-Rest; für einen Alkyl-Rest ausgewählt aus der Gruppe bestehend aus -CH₂-OH, -CH₂-CH₂-OH, -CH₂-CH₂-CH₂-OH, -CH₂-CH₂-CH₂-CH₂-OH, Isopropyl, n-Butyl, sek-Butyl, Isobutyl, Methyl, Ethyl und n-Propyl;
mit der Maßgabe, dass R^{25b} und R^{26b} nicht jeweils für einen Wasserstoff-Rest stehen;
oder
R^{25b} und R^{26b} jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind Verbindungen der allgemeinen Formel Ib, worin 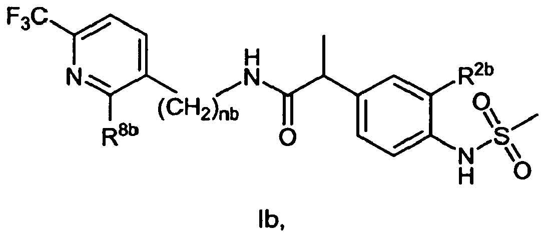 worin
nb, R^{8b} und R^{2b} wie vorstehend definiert sind,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls besonders bevorzugt sind Verbindungen der allgemeinen Formel Ib, worin
nb für 1 steht;
R^{2b} für F steht;
R^{8b} für H; F; Cl; Br, I; -CN; -OH; -NH₂; -NO₂; -NHR^{11b}, -NR^{12b}R^{13b}, -OR^{14b}, -SR^{15b};
für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, sek-Butyl, Isobutyl, tert-Butyl, -CH₂-CH₂-CH₂-CF₃, -CH₂-CH₂-CH₂-CH₂-CF₃, -CH₂-C(=O)-O-CH₃, -CH₂-C(=O)-C₂H₅, -CH₂-C(=O)-C(CH₃)₃, -CH₂-O-C(=O)-CH₃, - CH₂-O-C(=O)-C₂H₅, -CH₂-O-C(=O)-CH(CH₃)₂, -CH₂-O-C(=O)-C(CH₃)₃, n-Butyl, n-Pentyl, n-Hexyl, (3,3)-Dimethyl-but-1-yl, 3-Methyl-but-1-yl, 4-Methyl-pent-1-yl, (3,3)-Dimethyl-but-1-inyl, 4-Methyl-pent-1-inyl, 1-Hexinyl, Propinyl, Ethinyl, Butinyl, Pentinyl, 2-Methyl-propen-1-yl, 3-Methyl-but-2-en-1-yl, 1-Pentenyl, 1-Octenyl, 1-Heptenyl, 1-Hexenyl und (3,3)-Dimethyl-but-l-enyl;
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O=CH₃), -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Tetrazolyl, (1,3)-Benzodioxolyl, (1,4)-Benzodioxanyl, Indolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C=C-, -(CH₂)-, (CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, - N(C₂H₅)₂, -NH-S(=O)₂-CH3, -NH-S(=O₂)-C₂H₅, -NH-S(=O)₂-CH(CH₃)₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R^{11b}, R^{12b}, R^{13b}, R^{14b} und R^{15b}, unabhängig voneinander, jeweils
für einen Rest ausgewählt aus der Gruppe bestehend aus -(CH₂)-Pyridinyl, -(CH₂)₂ Pyridinyl, Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus 2,3-Dihydro-1H-indenyl, Oxetanyl, Piperidinyl, Pyrrolidinyl, Cyclopropyl, Cyclobutyl, Cyclopentyl und Cyclohexyl stehen, der jeweils über eine -CH₂-O-, -CH₂-CH₂-O-, -CH₂-CH₂-O-CH₂-, - CH₂-CH(CH₃)-O-CH₂-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, - (CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF3, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R^{12b} und R^{13b} jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl,(1,2,3,6)-Tetrahydropyridinyl, (4,5,6,7)-Tetrahydroisoxazolo[5,4-c]pyridinyl, Pyrrolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl, -CH₂-OH, -CH₂-CH₂-OH, =CH₂, -C(=O)-O-CH₃, -C(=O)-O-C₂H_{5,} - C(=O)-O-C(CH₃)_{3,} -C(=O)-CH₃, -C(=O)-C₂H₅, -C(=O)-C(CH₃)₃, -CN, -CH₂-N(CH₃)₂, - CH₂-N(C₂H₅)2, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, -NH₂, -NH-CH₃, -NH-C₂H₅, -N(CH₃)₂, - N(C₂H₅)₂, -NH-Phenyl, -N(CH₃)-Phenyl, -N(C₂H₅)-Phenyl, Oxo (=O), Thioxo (=S), - OH, F, Cl, Br, -CF₃, -O-CH₃, -O-C₂H₅, -O-C(CH₃)₃, -O-CH(CH₃)₂, -O-CH₂-CH₂-CH₂-CH₃, Methyl, Ethyl, Isopropyl, n-Propyl, n-Butyl, tert-Butyl, sek-Butyl, (4,5)-Dihydroisoxazolyl, Thiazolyl, (1,2,5)-Thiadiazolyl, Thiophenyl, Phenethyl, Cyclohexyl, Cyclopentyl, Piperidinyl, Pyrrolidinyl, -O-Phenyl, -O-C(=O)-CH₃, -O-C(=O)-C₂H₅, -O-C(=O)-C(CH₃)₃, -(CH₂)-Pyridinyl, Pyridinyl, Phenyl und Benzyl substituiert sein kann, wobei jeweils der zyklische Teil der Reste Oxetanyl, -N-[C(=O)-C₂H₅]-Phenyl, -N-[C(=O)-CH₃]-Phenyl, (4,5)-Dihydroisoxazolyl, Thiazolyl, (1,2,5)-Thiadiazolyl, Thiophenyl, Phenethyl, -NH-Phenyl, -N(CH₃)-Phenyl, -N(C₂H₅)-Phenyl, -(CH₂)-Pyridinyl, Pyridinyl, Phenyl, -O-Phenyl und Benzyl ggf. mit 1, 2, 3, 4 oder 5 Substituenten unabhängig voneinander ausgewählt aus der Gruppe bestehend aus - CF₃, -OH, -O-CH₃, -O-C₂H₅, F, Cl, Br, Methyl, Ethyl, Isopropyl, n-Propyl, n-Butyl, tert-Butyl und sek-Butyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind Verbindungen der allgemeinen Formel Ic1, 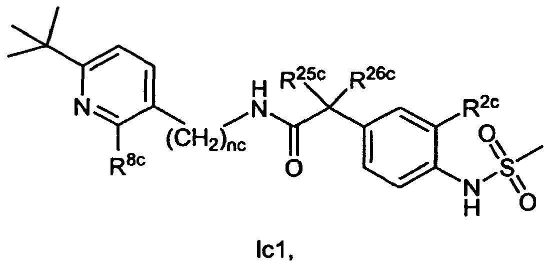 worin
nc für 0, 1 oder 2 steht;
R^{2c} für Methyl; -O-CH₃; F; Cl; Br oder I steht;
R^{8c} für H; F; Cl, Br; I; -CN; -OH; -NH₂; -NO₂; -NHR¹¹; -NR^{2c}R^{13c}; -OR^{14c}; -SR^{15c};
für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, sek-Butyl, Isobutyl, tert-Butyl, -CH₂-CH₂-CH₂-CF₃, -CH₂-CH₂-CH₂-CH₂-CF₃, -CH₂-C(=O)-O-CH₃, -CH₂-C(=O)-C₂H₅, -CH₂-C(=O)-C(CH₃)₃, -CH₂-O-C(=O)-CH₃, CH₂-O-C(=O)-C₂H₅, -CH₂-O-C(=O)-CH(CH₃)₂, -CH₂-O-C(=O)-C(CH₃)₃, n-Butyl, n-Pentyl, n-Hexyl, (3,3)-Dimethyl-but-1-yl, 3-Methyl-but-1-yl, 4-Methyl-pent-1-yl, (3,3)-Dimethyl-but-1-inyl, 4-Methyl-pent-1-inyl, 1-Hexinyl, Propinyl, Ethinyl, Butinyl, Pentinyl, 2-Methyl-propen-1-yl, 3-Methyl-but-2-en-1-yl, 1-Pentenyl, 1-Octenyl, 1-Heptenyl, 1-Hexenyl und (3,3)-Dimethyl-but-1-enyl;
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Tetrazolyl, (1,3)-Benzodioxolyl, (1,4)-Benzodioxanyl, Indolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂)-, - (CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, l, -CN, -CF₃, -SF₅, -OH, -O-CH₃, -O-C₂H₅, -NH₂, -N(CH₃)_{2,-}N(C₂H₅)₂, -NH-S(=O)₂-CH₃, -NH-S(=O₂)-C₂H₅, -NH-S(=O)₂-CH(CH₃)₂, -NO₂, -O-CF₃, - S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R^{11c}, R^{12c}, R^{13c}, R^{14c} und R^{15c}, unabhängig voneinander, jeweils
für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus 2,3-Dihydro-1H-indenyl, Piperidinyl, Pyrrolidinyl, Cyclopropyl, Cyclobutyl, Oxetanyl, Cyclopentyl und Cyclohexyl stehen, der jeweils über eine -CH₂-O-, -CH₂-CH₂-O-, -CH₂-CH₂-O-CH₂-, - CH₂-CH(CH₃)-O-CH₂-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, - (CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF₃, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R¹²_{,} und R^{13c} jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl, -CH₂-OH, -CH₂-CH₂-OH, =CH₂, -C(=O)-O-CH₃, -C(=O)-O-C₂H₅, - C(=O)-O-C(CH₃)₃, -C(=O)-CH₃, -C(=O)-C₂H₅, -C(=O)-C(CH₃)₃, -CN, -CH₂-N(CH₃)₂, - CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂O-CH₃, -CH₂-O-CH₂-CH₃, -NH₂, -NH-CH₃, -NH-C₂H₅, -N(CH₃)₂, - N(C₂H₅)₂, -NH-Phenyl, -N(CH₃)-Phenyl, -N(C₂H₅)-Phenyl, Oxo (=O), Thioxo (=S), - OH, F, Cl, Br, -CF₃, -O-CH₃, -O-C₂H₅, -O-C(CH₃)₃, -O-CH(CH₃)₂, -O-CH₂-CH₂-CH₂-CH₃, Methyl, Ethyl, Isopropyl, n-Propyl, n-Butyl, tert-Butyl, sek-Butyl, Cyclohexyl, Cyclopentyl, (4,5)-Dihydroisoxazolyl, Thiazolyl, (1,2,5)-Thiadiazolyl, Thiophenyl, Phenethyl, Piperidinyl, Pyrrolidinyl, -O-Phenyl, -O-C(=O)-CH₃, -O-C(=O)-C₂H₅, -O-C(=O)-C(CH₃)₃, -(CH₂)-Pyridinyl, Pyridinyl, Phenyl und Benzyl substituiert sein kann, wobei jeweils der zyklische Teil der Reste Oxetanyl, -N-[C(=O)-C₂H₅]-Phenyl, -N-[C(=O)-CH₃]-Phenyl, (4,5)-Dihydroisoxazolyl, Thiazolyl, (1,2,5)-Thiadiazolyl, Thiophenyl, Phenethyl, -NH-Phenyl, -N(CH₃)-Phenyl, -N(C₂H₅)-Phenyl, -(CH₂)-Pyridinyl, Pyridinyl, Phenyl, -O-Phenyl und Benzyl ggf. mit 1, 2, 3, 4 oder 5 Substituenten unabhängig voneinander ausgewählt aus der Gruppe bestehend aus - CF₃, -OH, -O-CH₃, -O-C₂H₅, F, Cl, Br, Methyl, Ethyl, Isopropyl, n-Propyl, n-Butyl, tert-Butyl und sek-Butyl substituiert sein kann;
R^{25c} und R^{26c}, unabhängig voneinander, jeweils für einen Wasserstoff-Rest; für einen Alkyl-Rest ausgewählt aus der Gruppe bestehend aus -CH₂-OH, -CH₂-CH₂-OH, -CH₂-CH₂-CH₂-OH, -CH₂-CH₂-CH₂-CH₂-OH, Isopropyl, n-Butyl, sek-Butyl, Isobutyl, Methyl, Ethyl und n-Propyl;
mit der Maßgabe, dass R^{25c} und R^{26c} nicht jeweils für einen Wasserstoff-Rest stehen;
oder
R^{25c} und R^{26c} jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind Verbindungen der allgemeinen Formel Ic, 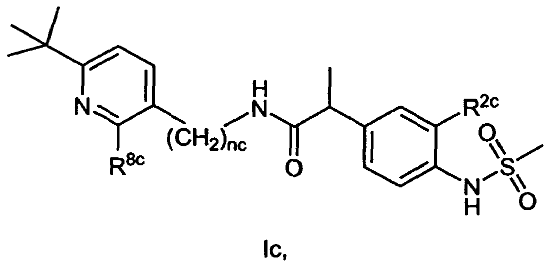 worin
nc, R^{8c} und R^{2c} wie vorstehend definiert sind;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls besonders bevorzugt sind Verbindungen der allgemeinen Formel Ic, worin
nc für 1 steht;
R^{2c} für F steht;
R^{8c} für H; F; Cl; Br; l; -CN; -OH; -NH₂; -NO_{2;} -NHR^{11c}; -NR^{12c}R^{13c}; -OR^{14c}; -SR^{15c};
für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, sek-Butyl, Isobutyl, tert-Butyl, -CH₂-CH₂-CH₂-CF₃, -CH₂-CH₂-CH₂-CH₂-CF_{3;} -CH₂-C(=O)-O-CH₃, -CH₂-C(=O)-C₂H₅, -CH₂-C(=O)-C(CH₃)₃, -CH₂-O-C(=O)-CH₃, - CH₂-O-C(=O)-C₂H₅, -CH₂-O-C(=O)-CH(CH₃)₂, -CH₂-O-C(=O)-C(CH₃)₃, n-Butyl, n-Pentyl, n-Hexyl, (3,3)-Dimethyl-but-1-yl, 3-Methyl-but-1-yl, 4-Methyl-pent-1-yl, (3,3)-Dimethyl-but-1-inyl, 4-Methyl-pent-1-inyl, 1-Hexinyl, Propinyl, Ethinyl, Butinyl, Pentinyl, 2-Methyl-propen-1-yl, 3-Methyl-but-2-en-1-yl, 1-Pentenyl, 1-Octenyl, 1-Heptenyl, 1-Hexenyl und (3,3)-Dimethyl-but-l-enyl;
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C=C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Tetrazolyl, (1,3)-Benzodioxolyl, (1,4)-Benzodioxanyl, Indolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂)-, - (CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, l, -CN, -CF₃, -SF₅, -OH, -O-CH₃, -O-C₂H₅, -NH₂, -N(CH₃)₂, - N(C₂H₅)₂, -NH-S(=O)₂-CH3, -NH-S(=O₂)-C₂H₅, -NH-S(=O)₂-CH(CH₃)₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R^{11c}, R^{12c}, R^{13c}, R^{14c} und R^{15c}, unabhängig voneinander, jeweils
für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus 2,3-Dihydro-1H-indenyl, Piperidinyl, Pyrrolidinyl, Cyclopropyl, Cyclobutyl, Oxetanyl, Cyclopentyl und Cyclohexyl stehen, der jeweils über eine -CH₂-O-, -CH₂-CH₂-O-, -CH₂-CH₂-O-CH₂-, - CH₂-CH(CH₃)-0-CH₂-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, - (CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF₃, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R^{12c} und R^{13c} jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl,(1,2,3,6)-Tetrahydropyridinyl, (4,5,6,7)-Tetrahydroisoxazolo[5,4-c]pyridinyl, Pyrrolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -C(=O)-O-CH₃, -C(=O)-O-C₂H_{5,} -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₂-Oxetanyl, -O-CH₂-Oxetanyl, -CH₂-OH, -CH₂-CH₂-OH, =CH₂, -CH₂-O-CH₃, -CH₂-O-CH₂-CH₃, -NH₂, -NH-CH₃, -NH-C₂H₅, -N(CH₃)₂, - N(C₂H₅)₂, -NH-Phenyl, -N(CH₃)-Phenyl, -N(C₂H₅)-Phenyl, Oxo (=O), Thioxo (=S), - OH, F, Cl, Br, -CF₃, -O-CH₃, -O-C₂H₅, -O-C(CH₃)₃, -O-CH(CH₃)₂, -O-CH₂-CH₂-CH₂-CH₃, Methyl, Ethyl, Isopropyl, n-Propyl, n-Butyl, tert-Butyl, sek-Butyl, Cyclohexyl, Cyclopentyl, (4,5)-Dihydroisoxazolyl, Thiazolyl, (1,2,5)-Thiadiazolyl, Thiophenyl, Phenethyl, Piperidinyl, Pyrrolidinyl, -O-Phenyl, -O-C(=O)-CH₃, -O-C(=O)-C₂H₅, -O-C(=O)-C(CH₃)₃, -(CH₂)-Pyridinyl, Pyridinyl, Phenyl und Benzyl substituiert sein kann, wobei jeweils der zyklische Teil der Reste Oxetanyl, -N-[C(=O)-C₂H₅]-Phenyl, -N-[C(=O)-CH₃]-Phenyl, (4,5)-Dihydroisoxazolyl, Thiazolyl, (1,2,5)-Thiadiazolyl, Thiophenyl, Phenethyl, -NH-Phenyl, -N(CH₃)-Phenyl, -N(C₂H₅)-Phenyl, -(CH₂)-Pyridinyl, Pyridinyl, Phenyl, -O-Phenyl und Benzyl ggf. mit 1, 2, 3, 4 oder 5 Substituenten unabhängig voneinander ausgewählt aus der Gruppe bestehend aus - CF₃, -OH, -O-CH₃, -O-C₂H₅, F, Cl, Br, Methyl, Ethyl, Isopropyl, n-Propyl, n-Butyl, tert-Butyl und sek-Butyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Bevorzugt sind Verbindungen der allgemeinen Formel Id1, worin 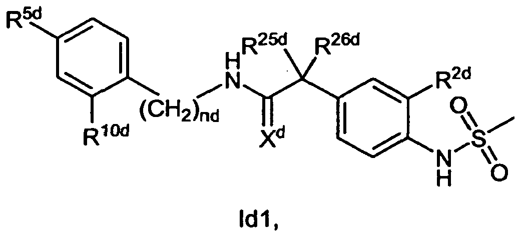 worin
X^{d} für O oder S steht;
nd für 0, 1 oder 2 steht;
R^{2d} für F; Cl; Br; l oder für einen Rest ausgewählt aus der Gruppe bestehend aus Methyl, -CF₃, -CCl₃, -CBr₃, -CHF₂, -CH₂F, -O-CH₃, -O-CF₃, -O-CCl₃, -O-CBr₃, -O-CHF₂, -O-CH₂F, -S-CH₃, -S-CF₃, -S-CCl₃, -S-CBr₃, -S-CHF₂, -S-CH₂F, -S-CF₂Cl und - S-CCl₂F steht;
R^{5d} für F; Cl; Br; l; -SF₅;
für einen Rest ausgewählt aus der Gruppe bestehend aus Methyl, Ethyl, -CF₃, -CCl₃, -CBr₃, -CHF₂, -CH₂F, -CF₂Cl, -CCl₂F, -C(CH₃)₂-(CH₂OH), tert-Butyl, -O-CF₃, -O-CCl₃, -O-CBr₃, -O-CHF₂, -O-CH₂F, -O-CF₂Cl, -O-CCl₂F, -O-CF₂-CH₃, -S-CF₃, -S-CCl₃, -S-CBr₃, -S-CHF₂, -S-CH₂F, -S-CF₂Cl, -S-CCl₂F, -S-CF₂-CH₃, -S(=O)₂-CF₃, -S(=O)₂-CCl₃, -S(=O)₂-CBr₃, -S(=O)₂-CHF₂, -S(=O)₂-CH₂F und -S(=O)₂-CF₂Cl;
oder für einen Rest ausgewählt aus der Gruppe bestehend aus Cyclopropyl, Cyclobutyl, Cyclopentyl, Cyclohexyl, Cyclopentenyl und Cyclohexenyl steht;
R^{10d} für -CN; -OH; -NH₂; -NO₂; -NHR^{11d}; -NR^{12d}R^{13d}; -OR^{14d}; -SR^{15d}; -C(=O)-OR^{22d};
für einen Rest ausgewählt aus der Gruppe bestehend aus Cyclopropyl, Cyclobutyl, Cyclopentyl, Cyclohexyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste an das Grundgerüst gebunden ist;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R^{11d}, R^{12d} , R^{13d}, R^{14d}, R^{15d} und R^{22d}, unabhängig voneinander, jeweils
für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus 2,3-Dihydro-1H-indenyl, Piperidinyl, Pyrrolidinyl, Cyclopropyl, Cyclobutyl, Oxetanyl, Cyclopentyl und Cyclohexyl stehen, der jeweils über eine -CH₂-O-, -CH₂-CH₂-O-, -CH₂-CH₂-O-CH₂-, - CH₂-CH(CH₃)-O-CH₂-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, - (CH₂)₂-Pyridinyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF3, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R^{12d} und R^{13d} jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl,(1,2,3,6)-Tetrahydropyridinyl, (4,5,6,7)-Tetrahydroisoxazolo[5,4-c]pyridinyl, Pyrrolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl, -CH₂-OH, -CH₂-CH₂-OH, =CH₂, -C(=O)-O-CH₃, -C(=O)-O-C₂H₅, - C(=O)-O-C(CH₃)₃, -C(=O)-CH₃, -C(=O)-C₂H₅, -C(=O)-C(CH₃)₃, -CN, -CH₂-N(CH₃)₂, - CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, -NH₂, -NH-CH₃, -NH-C₂H₅, -N(CH₃)₂, - N(C₂H₅)₂, -NH-Phenyl, -N(CH₃)-Phenyl, -N(C₂H₅)-Phenyl, Oxo (=O), Thioxo (=S), - OH, F, Cl, Br, -CF₃, -O-CH₃, -O-C₂H₅, -O-C(CH₃)₃, -O-CH(CH₃)₂, -O-CH₂-CH₂-CH₂-CH₃, Methyl, Ethyl, Isopropyl, n-Propyl, n-Butyl, tert-Butyl, sek-Butyl, Cyclohexyl, Cyclopentyl, (4,5)-Dihydroisoxazolyl, Thiazolyl, (1,2,5)-Thiadiazolyl, Thiophenyl, Phenethyl, Piperidinyl, Pyrrolidinyl, -O-Phenyl, -O-C(=O)-CH₃, -O-C(=O)-C₂H₅, -O-C(=O)-C(CH₃)₃, -(CH₂)-Pyridinyl, Pyridinyl, Phenyl und Benzyl substituiert sein kann, wobei jeweils der zyklische Teil der Reste Oxetanyl, -N-[C(=O)-C₂H₅]-Phenyl, -N-[C(=O)-CH₃]-Phenyl, (4,5)-Dihydroisoxazolyl, Thiazolyl, (1,2,5)-Thiadiazolyl, Thiophenyl, Phenethyl, -NH-Phenyl, -N(CH₃)-Phenyl, -N(C₂H₅)-Phenyl, -(CH₂₎₋Pyridinyl, Pyridinyl, Phenyl, -O-Phenyl und Benzyl ggf. mit 1, 2, 3, 4 oder 5 Substituenten unabhängig voneinander ausgewählt aus der Gruppe bestehend aus - CF₃, -OH, -O-CH₃, -O-C₂H₅. F, Cl, Br, Methyl, Ethyl, Isopropyl, n-Propyl, n-Butyl, tert-Butyl und sek-Butyl substituiert sein kann;
R^{25d} und R^{26d}, unabhängig voneinander, jeweils für einen Wasserstoff-Rest; für einen Alkyl-Rest ausgewählt aus der Gruppe bestehend aus -CH₂-OH, -CH₂-CH₂-OH, -CH₂-CH₂-CH₂-OH, -CH₂-CH₂-CH₂-CH₂-OH, Isopropyl, n-Butyl, sek-Butyl, Isobutyl, Methyl, Ethyl und n-Propyl;
mit der Maßgabe, dass R^{25d} und R^{26d} nicht jeweils für einen Wasserstoff-Rest stehen;
oder
R^{25d} und R^{26d} jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Bevorzugt sind Verbindungen der allgemeinen Formel Id, worin 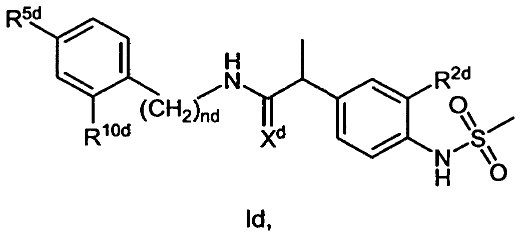 worin
X^{d}, R^{5d}, R^{10d}, nd und R^{2d} wie vorstehend definiert sind;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls besonders bevorzugt sind Verbindungen der allgemeinen Formel Ie1, 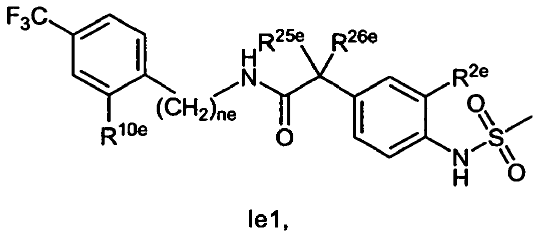 worin
ne für 0, 1 oder 2 steht;
R^{2e} für Methyl; -O-CH₃; F; Cl; Br oder I steht;
R^{10e} für -CN; -OH; -NH₂; -NO₂; -NHR^{11e}; -NR^{12e}R^{13e}; -OR^{14e}; -SR^{15e};
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Tetrazolyl, (1,3)-Benzodioxolyl, (1,4)-Benzodioxanyl, Indolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂)-, - (CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, - N(C₂H₅)₂, -NH-S(=O)₂-CH₃, -NH-S(=O₂)-C₂H₅, -NH-S(=O)₂-CH(CH₃)₂, -NO₂, -O-CF₃, - S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R^{11e}, R^{12e}, R^{13e}, R^{14e} und R^{15e}, unabhängig voneinander, jeweils für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus Oxetanyl, 2,3-Dihydro-1H-indenyl, Piperidinyl, Pyrrolidinyl, Cyclopropyl, Cyclobutyl, Cyclopentyl und Cyclohexyl stehen, der jeweils über eine -CH₂-O-, -CH₂-CH₂-O-, -CH₂-CH₂-O-CH₂-, -CH₂-CH(CH₃)-O-CH₂-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, - (CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF3, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R^{12e} und R^{13e} jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl, -CH₂-OH, -CH₂-CH₂-OH, =CH₂, -C(=O)-O-CH₃, -C(=O)-O-C₂H₅, - C(=O)-O-C(CH₃)₃, -C(=O)-CH₃, -C(=O)-C₂H₅, -C(=O)-C(CH₃)₃, -CN, -CH₂-N(CH₃)₂, - CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, -NH₂, -NH-CH₃, -NH-C₂H₅, -N(CH₃)₂, - N(C₂H₅)₂, -NH-Phenyl, -N(CH₃)-Phenyl, -N(C₂H₅)-Phenyl, Oxo (=O), Thioxo (=S), - OH, F, Cl, Br, -CF₃, -O-CH₃, -O-C₂H₅, -O-C(CH₃)₃, -O-CH(CH₃)₂, -O-CH₂-CH₂-CH₂-CH₃, Methyl, Ethyl, Isopropyl, n-Propyl, n-Butyl, tert-Butyl, sek-Butyl, Cyclohexyl, Cyclopentyl, (4,5)-Dihydroisoxazolyl, Thiazolyl, (1,2,5)-Thiadiazolyl, Thiophenyl, Phenethyl, Piperidinyl, Pyrrolidinyl, -O-Phenyl, -O-C(=O)-CH₃, -O-C(=O)-C₂H₅, -O-C(=O)-C(CH₃)₃, -(CH₂)-Pyridinyl, Pyridinyl, Phenyl und Benzyl substituiert sein kann, wobei jeweils der zyklische Teil der Reste Oxetanyl, -N-[C(=O)-C₂H₅]-Phenyl, -N-[C(=O)-CH₃]-Phenyl, (4,5)-Dihydroisoxazolyl, Thiazolyl, (1,2,5)-Thiadiazolyl, Thiophenyl, Phenethyl, -NH-Phenyl, -N(CH₃)-Phenyl, -N(C₂H₅)-Phenyl, -(CH₂)-Pyridinyl, Pyridinyl, Phenyl, -O-Phenyl und Benzyl ggf. mit 1, 2, 3, 4 oder 5 Substituenten unabhängig voneinander ausgewählt aus der Gruppe bestehend aus - CF₃, -OH, -O-CH₃, -O-C₂H₅, F, Cl, Br, Methyl, Ethyl, Isopropyl, n-Propyl, n-Butyl, tert-Butyl und sek-Butyl substituiert sein kann;
und R^{25e} und R^{26e}, unabhängig voneinander, jeweils für einen Wasserstoff-Rest; für einen Alkyl-Rest ausgewählt aus der Gruppe bestehend aus -CH₂-OH, -CH₂-CH₂-OH, -CH₂-CH₂-CH₂-OH, -CH₂-CH₂-CH₂-CH₂-OH, Isopropyl, n-Butyl, sek-Butyl, Isobutyl, Methyl, Ethyl und n-Propyl;
mit der Maßgabe, dass R^{25e} und R^{26e} nicht jeweils für einen Wasserstoff-Rest stehen;
oder
R^{25e} und R^{26e} jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls besonders bevorzugt sind Verbindungen der allgemeinen Formel Ie, 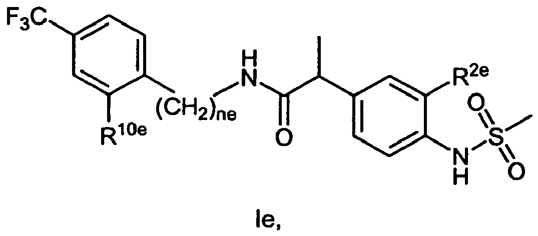 worin
ne, R^{10e} und R^{2e} wie vorstehend definiert sind;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls besonders bevorzugt sind Verbindungen der allgemeinen Formel Ie, worin
ne für 1 steht;
R^{2e} für F steht;
R^{10e} für -CN; -OH; -NH₂; -NO₂; -NHR^{11e}; -NR^{12e}R^{13e}; -OR^{14e}; -SR^{15e};
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Tetrazolyl, (1,3)-Benzodioxolyl, (1,4)-Benzodioxanyl, Indolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂)-, - (CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, - N(C₂H₅)₂, -NH-S(=O)₂-CH₃, -NH-S(=O₂)-C₂H₅, -NH-S(=O)₂-CH(CH₃)₂, -NO₂, -O-CF₃, - S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R^{11e}, R^{12e} , R^{13e}, R^{14e} und R^{15e}, unabhängig voneinander, jeweils
für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus Oxetanyl, 2,3-Dihydro-1 H-indenyl, Piperidinyl, Pyrrolidinyl, Cyclopropyl, Cyclobutyl, Cyclopentyl und Cyclohexyl stehen, der jeweils über eine -CH₂-O-, -CH₂-CH₂-O-, -CH₂-CH₂-O-CH₂-, -CH₂-CH(CH₃)-O-CH₂-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, - (CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF3, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R^{12e} und R^{13e} jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, (1,2,3.6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl, -CH₂-OH, -CH₂-CH₂-OH, =CH₂, -C(=O)-O-CH₃, -C(=O)-O-C₂H₅, - C(=O)-O-C(CH₃)₃, -C(=O)-CH₃, -C(=O)-C₂H₅, -C(=O)-C(CH₃)₃, -CN, -CH₂-N(CH₃)₂, - CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, -NH₂, -NH-CH₃, -NH-C₂H₅, -N(CH₃)₂, - N(C₂H₅)₂, -NH-Phenyl, -N(CH₃)-Phenyl, -N(C₂H₅)-Phenyl, Oxo (=O), Thioxo (=S), - OH, F, Cl, Br, -CF₃, -O-CH₃, -O-C₂H₅, -O-C(CH₃)₃, -O-CH(CH₃)₂, -O-CH₂-CH₂-CH₂-CH₃, Methyl, Ethyl, Isopropyl, n-Propyl, n-Butyl, tert-Butyl, sek-Butyl, Cyclohexyl, Cyclopentyl, (4,5)-Dihydroisoxazolyl, Thiazolyl, (1,2,5)-Thiadiazolyl, Thiophenyl, Phenethyl, Piperidinyl, Pyrrolidinyl, -O-Phenyl, -O-C(=O)-CH₃, -O-C(=O)-C₂H₅, -O-C(=O)-C(CH₃)₃, -(CH₂)-Pyridinyl, Pyridinyl, Phenyl und Benzyl substituiert sein kann, wobei jeweils der zyklische Teil der Reste Oxetanyl, N-[C(=O)-C₂H₅]-Phenyl, -N-[C(=O)-CH₃]-Phenyl, (4,5)-Dihydroisoxazolyl, Thiazolyl, (1,2,5)-Thiadiazolyl, Thiophenyl, Phenethyl, -NH-Phenyl, -N(CH₃)-Phenyl, -N(C₂H₅)-Phenyl, -(CH₂)-Pyridinyl, Pyridinyl, Phenyl, -O-Phenyl und Benzyl ggf. mit 1, 2, 3, 4 oder 5 Substituenten unabhängig voneinander ausgewählt aus der Gruppe bestehend aus - CF₃, -OH, -O-CH₃, -O-C₂H₅, F, Cl, Br, Methyl, Ethyl, Isopropyl, n-Propyl, n-Butyl, tert-Butyl und sek-Butyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind Verbindungen der allgemeinen Formel If1, worin 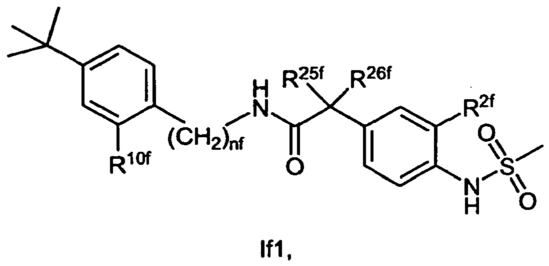 worin
nf für 0, 1 oder 2 steht;
R^{2f} für Methyl; -O-CH₃; F; Cl; Br oder I steht;
R^{10f} für -CN; -OH; -NH₂; -NO₂; -NHR^{11f}; -NR^{12f}R^{13f}; -OR^{14f}; -SR^{15f};
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus (1,3)-Benzodioxolyl, (1,4)-Benzodioxanyl, Indolyl, Tetrazolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂)-, - (CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, - N(C₂H₅)₂, -NH-S(=O)₂-CH₃, -NH-S(=O₂)-C₂H₅, -NH-S(=O)₂-CH(CH₃)₂, -NO₂, -O-CF₃, - S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R^{11f}, R^{12f}, R^{13f}, R^{14f} und R^{15f}, unabhängig voneinander, jeweils
für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus Oxetanyl, 2,3-Dihydro-1H-indenyl, Piperidinyl, Pyrrolidinyl, Cyclopropyl, Cyclobutyl, Cyclopentyl und Cyclohexyl stehen, der jeweils über eine -CH₂-O-, -CH₂-CH₂-O-, -CH₂-CH₂-O-CH₂-, -CH₂-CH(CH₃)-O-CH₂-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, - (CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF3, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R^{12f} und R^{13f} jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl, -CH₂-OH, -CH₂-CH₂-OH, =CH₂, -C(=O)-O-CH₃, -C(=O)-O-C₂H₅, - C(=O)-O-C(CH₃)₃, -C(=O)-CH₃, -C(=O)-C₂H₅, -C(=O)-C(CH₃)₃, -CN, -CH₂-N(CH₃)₂, - CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, -NH₂, -NH-CH₃, -NH-C₂H₅, -N(CH₃)₂, - N(C₂H₅)₂, -NH-Phenyl, -N(CH₃)-Phenyl, -N(C₂H₅)-Phenyl, Oxo (=O), Thioxo (=S), - OH, F, Cl, Br, -CF₃, -O-CH₃, -O-C₂H₅, -O-C(CH₃)₃, -O-CH(CH₃)₂, -O-CH₂-CH₂-CH₂-CH₃, Methyl, Ethyl, Isopropyl, n-Propyl, n-Butyl, tert-Butyl, sek-Butyl, Cyclohexyl, Cyclopentyl, (4,5)-Dihydroisoxazolyl, Thiazolyl, (1,2,5)-Thiadiazolyl, Thiophenyl, Phenethyl, Piperidinyl, Pyrrolidinyl, -O-Phenyl, -O-C(=O)-CH₃, -O-C(=O)-C₂H₅, -O-C(=O)-C(CH₃)₃, -(CH₂)-Pyridinyl, Pyridinyl, Phenyl und Benzyl substituiert sein kann, wobei jeweils der zyklische Teil der Reste Oxetanyl, -N-[C(=O)-C₂H₅]-Phenyl, -N-[C(=O)-CH₃]-Phenyl, (4,5)-Dihydroisoxazolyl, Thiazolyl, (1,2,5)-Thiadiazolyl, Thiophenyl, Phenethyl, -NH-Phenyl, -N(CH₃)-Phenyl, -N(C₂H₅)-Phenyl, -(CH₂)-Pyridinyl, Pyridinyl, Phenyl, -O-Phenyl und Benzyl ggf. mit 1, 2, 3, 4 oder 5 Substituenten unabhängig voneinander ausgewählt aus der Gruppe bestehend aus - CF₃, -OH, -O-CH₃, -O-C₂H₅, F, Cl, Br, Methyl, Ethyl, Isopropyl, n-Propyl, n-Butyl, tert-Butyl und sek-Butyl substituiert sein kann;
und R^{25f} und R^{26f}, unabhängig voneinander, jeweils für einen Wasserstoff-Rest; für einen Alkyl-Rest ausgewählt aus der Gruppe bestehend aus -CH₂-OH, -CH₂-CH₂-OH, -CH₂-CH₂-CH₂-OH, -CH₂-CH₂-CH₂-CH₂-OH, Isopropyl, n-Butyl, sek-Butyl, Isobutyl, Methyl, Ethyl und n-Propyl;
mit der Maßgabe, dass R^{25f} und R^{26f} nicht jeweils für einen Wasserstoff-Rest stehen;
oder
R^{25f} und R^{26f} jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind Verbindungen der allgemeinen Formel If, worin 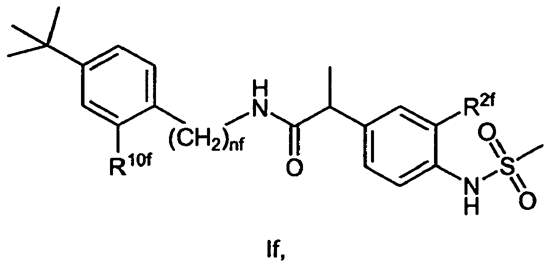 worin
nf, R^{10f} und R^{2f} wie vorstehend definiert sind;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls besonders bevorzugt sind Verbindungen der allgemeinen Formel If, worin
nf für 1 steht;
R^{2f} für F steht;
R^{10f} für -CN; -OH; -NH₂; -NO₂; -NHR^{11f}; -NR^{12f}R^{13f}; -OR^{14f}; -SR^{15f};
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C=C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus (1,3)-Benzodioxolyl, (1,4)-Benzodioxanyl, Indolyl, Tetrazolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂)-, - (CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, - N(C₂H₅)₂, -NH-S(=O)₂-CH₃, -NH-S(=O₂)-C₂H₅, -NH-S(=O)₂-CH(CH₃)₂, -NO₂, -O-CF₃, - S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R^{11f}, R^{12f} , R^{13f}, R^{14f} und R^{15f}, unabhängig voneinander, jeweils
für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus Oxetanyl, 2,3-Dihydro-1 H-indenyl, Piperidinyl, Pyrrolidinyl, Cyclopropyl, Cyclobutyl, Cyclopentyl und Cyclohexyl stehen, der jeweils über eine -CH₂-O-, -CH₂-CH₂-O-, -CH₂-CH₂-O-CH₂-, -CH₂-CH(CH₃)-O-CH₂-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, tert-Butyl, n-Pentyl, n-Hexyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, - (CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF₃, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R^{12f} und R^{13f} jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl, -CH₂-OH, -CH₂-CH₂-OH, =CH₂, -C(=O)-O-CH₃, -C(=O)-O-C₂H₅, - C(=O)-O-C(CH₃)₃, -C(=O)-CH₃, -C(=O)-C₂H₅, -C(=O)-C(CH₃)₃, -CN, -CH₂-N(CH₃)₂, - CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, -CH₂-O-CH₃, -NH₂, -NH-CH₃, -NH-C₂H₅, -N(CH₃)₂, -N(C₂H₅)₂, -NH-Phenyl, -N(CH₃)-Phenyl, -N(C₂H₅)-Phenyl, Oxo (=O), Thioxo (=S), -OH, F, Cl, Br, -CF₃, -O-CH₃, -O-C₂H₅, -O-C(CH₃)₃, -O-CH(CH₃)₂, -O-CH₂-CH₂-CH₂-CH₃, Methyl, Ethyl, Isopropyl, n-Propyl, n-Butyl, tert-Butyl, sek-Butyl, Piperidinyl, Pyrrolidinyl, -O-Phenyl, -O-C(=O)-CH₃, -O-C(=O)-C₂H₅, -O-C(=O)-C(CH₃)₃, -(CH₂)-Pyridinyl, Cyclohexyl, Cyclopentyl, (4,5)-Dihydroisoxazolyl, Thiazolyl, (1,2,5)-Thiadiazolyl, Thiophenyl, Phenethyl, Pyridinyl, Phenyl und Benzyl substituiert sein kann, wobei jeweils der zyklische Teil der Reste Oxetanyl, -N-[C(=O)-C₂H₅]-Phenyl, -N-[C(=O)-CH₃]-Phenyl, (4,5)-Dihydroisoxazolyl, Thiazolyl, (1,2,5)-Thiadiazolyl, Thiophenyl, Phenethyl,-NH-Phenyl, -N(CH₃)-Phenyl, -N(C₂H₅)-Phenyl, - (CH₂)-Pyridinyl, Pyridinyl, Phenyl, -O-Phenyl und Benzyl ggf. mit 1, 2, 3, 4 oder 5 Substituenten unabhängig voneinander ausgewählt aus der Gruppe bestehend aus - CF₃, -OH, -O-CH₃, -O-C₂H₅, F, Cl, Br, Methyl, Ethyl, Isopropyl, n-Propyl, n-Butyl, tert-Butyl und sek-Butyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind Verbindungen der allgemeinen Formel Ig, 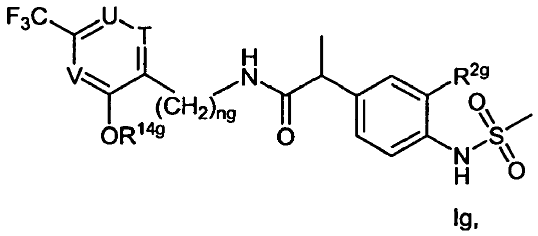 worin
ng für 0, 1 oder 2 steht;
R²⁹ für Methyl; -O-CH₃; F; Cl; Br oder I steht;
R^{14g} für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl steht;
T für CH und U für N und V für CH
oder
T für N und U für CH und V für CH
oder
T für N und U für N und V für CH
oder
T für N und U für CH und V für N
oder
T für CH und U für N und V für N
steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls besonders bevorzugt sind Verbindungen der allgemeinen Formel Ig, worin
ng für 1 steht;
R²⁹ für F steht;
R^{14g} für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl steht;
T für CH und U für N und V für CH
oder
T für N und U für CH und V für CH
oder
T für N und U für N und V für CH
oder
T für N und U für CH und V für N
oder
T für CH und U für N und V für N
steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ganz besonders bevorzugt sind Verbindungen der vorstehend angegebenen allgemeinen Formeln ausgewählt aus der Gruppe bestehend aus
1 2-(3-Fluor-4-(methylsulfonylamino)phenyl)-N-((2-methyl-6-(trifluormethyl)pyridin-3-yl)methyl)propanamid
2 2-(3-Fluor-4-(methylsulfonylamino)phenyl)-N-((2-(piperidin-1-yl)-6-(trifluormethyl)pyridin-3-yl)methyl)propanamid
3 2-(3-Fluor-4-(methylsulfonylamino)phenyl)-N-((2-piperidin-1-yl)-4-(trifluormethyl)benzyl)propanamid
4 2-(3-Fluor-4-(methylsulfonylamino)phenyl)-N-((2-fluor-6-(trifluormethyl)pyridin-3-yl)methyl)propanamid
5 N-((2-Chlor-6-(trifluormethyl)pyridin-3-yl)methyl)-2-(3-fluor-4-(methylsulfonylamino)phenyl)propanamid
6 N-((2-Brom-6-(trifluormethyl)pyridin-3-yl)methyl)-2-(3-fluor-4-(methylsulfonylamino)phenyl)propanamid
7 2-(3-Fluor-4-(methylsulfonylamino)phenyl)-N-((2-iod-6-(trifluormethyl)pyridin-3-yl)methyl)propanamid
8 N-((2-tert-Butyl-6-(trifluormethyl)pyridin-3-yl)methyl)-2-(3-fluor-4-(methylsulfonylamino)phenyl)propanamid
9 N-((2-Cyano-6-(trifluormethyl)pyridin-3-yl)methyl)-2-(3-fluor-4-(methylsulfonylamino)phenyl)propanamid
10 (S)-2-(3-Fluor-4-(methylsulfonylamino)phenyl)-N-((2-(piperidin-1-yl)-6-(trifluormethyl)pyridin-3-yl)methyl)propanamid
11 (R)-2-(3-Fluor-4-(methylsulfonylamino)phenyl)-N-((2-(piperidin-1-yl)-6-(trifluormethyl)pyridin-3-yl)methyl)propanamid
12 2-(3-Fluor-4-(methylsulfonylamino)phenyl)-N-((2-morpholino-6-(trifluormethyl)pyridin-3-yl)methyl)propanamid
13 2-(3-Fluor-4-(methylsulfonylamino)phenyl)-N-((2-(pyrrolidin-1-yl)-6-(trifluormethyl)pyridin-3-yl)methyl)propanamid
14 N-((2-(Dimethylamino)-6-(trifluormethyl)pyridin-3-yl)methyl)-2-(3-fluor-4-(methylsulfonylamino)phenyl)propanamid
15 N-((2-(Diethylamino)-6-(trifluormethyl)pyridin-3-yl)methyl)-2-(3-fluor-4-(methylsulfonylamino)phenyl)propanamid
16 N-((2-(Dipropylamino)-6-(trifluormethyl)pyridin-3-yl)methyl)-2-(3-fluor-4-(methylsulfonylamino)phenyl)propanamid
17 2-(3-Fluor-4-(methylsulfonylamino)phenyl)-N-((2-hydroxy-6-(trifluormethyl)pyridin-3-yl)methyl)propanamid
18 2-(3-Fluor-4-(methylsulfonylamino)phenyl)-N-((2-methoxy-6-(trifluormethyl)pyridin-3-yl)methyl)propanamid
19 N-((2-Butoxy-6-(trifluormethyl)pyridin-3-yl)methyl)-2-(3-fluor-4-(methylsulfonylamino)phenyl)propanamid
20 2-(3-Fluor-4-(methylsulfonylamino)phenyl)-N-((2-isopropoxy-6-(trifluormethyl)pyridin-3-yl)methyl)propanamid
21 N-((2-Cyclopentyloxy-6-(trifluormethyl)pyridin-3-yl)methyl)-2-(3-fluor-4-(methylsulfonylamino)phenyl)propanamid
22 2-(3-Fluor-4-(methylsulfonylamino)phenyl)-N-((2-phenyl-6-(trifluormethyl)pyridin-3-yl)methyl)propanamid
23 2-(3-Fluor-4-(methylsulfonylamino)phenyl)-N-((2-(4-fluor-phenyl)-6-(trifluormethyl)pyridin-3-yl)methyl)propanamid
24 2-(3-Fluor-4-(methylsulfonylamino)phenyl)-N-((6-(trifluormethyl)-2,2'-bipyridin-3-yl)methyl)propanamid
25 2-(3-Fluor-4-(methylsulfonylamino)phenyl)-N-((6-(trifluormethyl)-2,3'-bipyridin-3-yl)methyl)propanamid
26 2-(3-Fluor-4-(methylsulfonylamino)phenyl)-N-((2-(pyrimidin-2-yl)-6-(trifluormethyl)pyridin-3-yl)methyl)propanamid
27 2-(3-Fluor-4-(methylsulfonylamino)phenyl)-N-((2-(thiazol-2-yl)-6-(trifluormethyl)pyridin-3-yl)methyl)propanamid
28 2-(3-Fluor-4-(methylsulfonylamino)phenyl)-N-((2-(oxazol-2-yl)-6-(trifluormethyl)pyridin-3-yl)methyl)propanamid
29 N-((2-(1H-Imidazol-2-yl)-6-(trifluormethyl)pyridin-3-yl)methyl)-2-(3-fluor-4-(methylsulfonylamino)phenyl)propanamid
30 N-(2-Cyano-4-(trifluormethyl)benzyl)-2-(3-fluor-4-(methylsulfonylamino)phenyl)propanamid
31 (S)-2-(3-Fluor-4-(methylsulfonylamino)phenyl)-N-(2-(piperidin-1-yl)-4-(trifluormethyl)benzyl)propanamid
32 (R)-2-(3-Fluor-4-(methylsulfonylamino)phenyl)-N-(2-(piperidin-1-yl)-4-(trifluormethyl)benzyl)propanamid
33 2-(3-Fluor-4-(methylsulfonylamino)phenyl)-N-(2-morpholino-4-(trifluormethyl)benzyl)propanamid
34 2-(3-Fluor-4-(methylsulfonylamino)phenyl)-N-(2-(pyrrolidin-1-yl)-4-(trifluormethyl)benzyl)propanamid
35 N-(2-(Dimethylamino)-4-(trifluormethyl)benzyl)-2-(3-fluor-4-(methylsulfonylamino)phenyl)propanamid
36 N-(2-(Diethylamino)-4-(trifluormethyl)benzyl)-2-(3-fluor-4-(methylsulfonylamino)phenyl)propanamid
37 N-(2-(Dipropylamino)-4-(trifluormethyl)benzyl)-2-(3-fluor-4-(methylsulfonylamino)phenyl)propanamid
38 2-(3-Fluor-4-(methylsulfonylamino)phenyl)-N-(2-hydroxy-4-(trifluormethyl)benzyl)propanamid
39 2-(3-Fluor-4-(methylsulfonylamino)phenyl)-N-(2-methoxy-4-(trifluormethyl)benzyl)propanamid
40 N-(2-Butoxy-4-(trifluormethyl)benzyl)-2-(3-fluor-4-(methylsulfonylamino)phenyl)propanamid
41 2-(3-Fluor-4-(methylsulfonylamino)phenyl)-N-(2-isopropoxy-4-(trifluormethyl)benzyl)propanamid
42 N-(2-(Cyclopentyloxy)-4-(trifluormethyl)benzyl)-2-(3-fluor-4-(methylsulfonylamino)phenyl)propanamid
43 2-(3-Fluor-4-(methylsulfonylamino)phenyl)-N-((5-(trifluormethyl)biphenyl-2-yl)methyl)propanamid
44 2-(3-Fluor-4-(methylsulfonylamino)phenyl)-N-((4'-fluor-5-(trifluormethyl)biphenyl-2-yl)methyl)propanamid
45 2-(3-Fluor-4-(methylsulfonylamino)phenyl)-N-(2-(pyridin-2-yl)-4-(trifluormethyl)benzyl)propanamid
46 2-(3-Fluor-4-(methylsulfonylamino)phenyl)-N-(2-(pyridin-3-yl)-4-(trifluormethyl)benzyl)propanamid
47 2-(3-Fluor-4-(methylsulfonylamino)phenyl)-N-(2-(pyrimidin-2-yl)-4-(trifluormethyl)benzyl)propanamid
48 2-(3-Fluor-4-(methylsulfonylamino)phenyl)-N-(2-(thiazol-2-yl)-4-(trifluormethyl)benzyl)propanamid
49 2-(3-Fluor-4-(methylsulfonylamino)phenyl)-N-(2-(oxazol-2-yl)-4-(trifluormethyl)benzyl)propanamid
50 N-(2-(1H-Imidazol-2-yl)-4-(trifluormethyl)benzyl)-2-(3-fluor-4-(methylsulfonylamino)phenyl)propanamid
51 N-((6-tert-Butyl-2-(piperidin-1-yl)pyridin-3-yl)methyl)-2-(3-fluor-4-(methylsufonamido)phenyl)propanamid
52 2-(3-Fluor-4-(methylsulfonylamino)phenyl)-N-((4-(piperidin-1-yl)-6-(trifluormethyl)pyridin-3-yl)methyl)propanamid
53 2-(3-Fluor-4-(methylsulfonylamino)phenyl)-N-((3-(piperidin-1-yl)-5-(trifluormethyl)pyridin-2-yl)methyl)propanamid
54 2-(3-Fluor-4-(methylsulfonylamino)phenyl)-N-((4-(piperidin-1-yl)-2-(trifluormethyl)pyrimidin-5-yl)methyl)propanamid
55 2-(3-Fluor-4-(methylsulfonylamino)phenyl)-N-((3-(piperidin-1-yl)-5-(trifluormethyl)pyrazin-2-yl)methyl)propanamid
56 2-(3-Fluor-4-(methylsulfonylamino)phenyl)-N-((4-(piperidin-1-yl)-6-(trifluormethyl)pyridazin-3-yl)methyl)propanamid
57 2-(3-Fluor-4-(methylsulfonylamino)phenyl)-N-((2-piperidin-1-yl)-6-(trifluormethyl)pyridin-3-yl)propanamid
58 2-(3-Fluor-4-(methylsulfonylamino)phenyl)-N-((2-piperidin-1-yl)-4-(trifluormethyl)phenyl)propanamid
59 2-(3-Fluor-4-(methylsulfonylamino)phenyl)-N-(2-(2-(piperidin-1-yl)-6-(trifluormethyl)pyridin-3-yl)ethyl)propanamid
60 2-(3-Fluor-4-(methylsulfonylamino)phenyl)-N-(2-(piperidin-1-yl)-4-(trifluormethyl)phenethyl)propanamid
61 N-(2-Amino-4-(trifluormethyl)benzyl)-2-(3-fluor-4-(methylsulfonylamino)phenyl)propanamid
62 2-(3-Fluor-4-(methylsulfonylamino)phenyl)-N-(2-nitro-4-(trifluormethyl)benzyl)propanamid
63 N-(4-tert-Butyl-2-(piperidin-1-yl)benzyl)-2-(3-fluor-4-(methylsulfonylamino)phenyl)propanamid
64 2-(3-Chlor-4-(methylsulfonylamino)phenyl)-N-((2-(piperidin-1-yl)-6-(trifluormethyl)pyridin-3-yl)methyl)propanamid
65 2-(3-Chlor-4-(methylsulfonylamino)phenyl)-N-((2-(pyrrolidin-1-yl)-6-(trifluormethyl)pyridin-3-yl)methyl)propanamid
66 2-(3-Chlor-4-(methylsulfonylamino)phenyl)-N-(2-(piperidin-1-yl)-4-(trifluormethyl)benzyl)propanamid
67 2-(3-Brom-4-(methylsulfonylamino)phenyl)-N-((2-(piperidin-1-yl)-6-(trifluormethyl)pyridin-3-yl)methyl)propanamid
68 2-(3-Brom-4-(methylsulfonylamino)phenyl)-N-(2-(piperidin-1-yl)-4-(trifluormethyl)benzyl)propanamid
69 2-(3-Brom-4-(methylsulfonylamino)phenyl)-N-(2-(pyrrolidin-1-yl)-4-(trifluormethyl)benzyl)propanamid
70 N-(4-tert-Butyl-2-cyanobenzyl)-2-(3-fluor-4-(methylsulfonylamino)phenyl)propanamid
71 N-((6-(Chlordiflourmethyl)-2-(piperidin-1-yl)pyridin-3-yl)methyl)-2-(3-fluor-(4-methylsulfonylamino)phenyl)propanamid
72 (S)-2-(3-Fluor-4-(methylsulfonylamino)phenyl)-N-((2-morpholino-6-(trifluormethyl)pyridin-3-yl)methyl)propanamid
73 N-((2-(4-Benzylpiperazin-1-yl)-6-(trifluormethyl)pyridin-3-yl)methyl)-2-(3-fluor-4-(methylsulfonylamino)phenyl)propanamid
74 2-(3-Fluor-4-(methylsulfonylamino)phenyl)-N-((2-piperazin-1-yl)-6-(trifluormethyl)pyridin-3-yl)methyl)propanamid;
75 N-(2-Chlor-4-(trifluormethyl)benzyl)-2-(3-fluor-4-(methylsulfonylamino)phenyl)propanamid
76 N-((2-(Cyclohexyloxy)-6-(trifluormethyl)pyridin-3-yl)methyl-2-(3-fluor-4-methylsulfonylamino)phenyl)propanamid
77 N-((3-Chlor-5-(trifluormethyl)pyridin-2-yl)methyl-2-(3-fluor-4-(methylsulfonylamino)phenyl)propanamid
78 2-(3-Fluor-4-(methylsulfonylamino)phenyl)-N-((3-(pyrrolidin-1-yl)-5-(trifluormethyl)pyridin-2-yl)methyl)propanamid
79 N-((2-(3,5-Dimethylpiperidin-1-yl)-6-(trifluormethyl)pyridin-3-yl)methyl)-2-(3-fluor-4-(methylsulfonylamino)phenyl)propanamid
80 2-(3-Fluor-4-(methylsulfonylamino)phenyl)-N-((2-(4-methylpiperidin-1-yl)-6-(trifluormethyl)pyridin-3-yl)methyl)propanamid
81 N-((2-(Azepan-1-yl)-6-(trifluormethyl)pyridin-3-yl)methyl)-2-(3-fluor-4-(methylsulfonylamino)phenyl)propanamid
82 2-(3-Fluor-4-(methylsulfonylamino)phenyl)-N-(2-(4-methylpiperidin-1-yl)-4-(trifluormethyl)benzyl)propanamid;
83 (S)-2-(3-Fluor-4-methansulfonylamino-phenyl)-N-[2-(4-methyl-piperidin-1-yl)-4-trifluormethyl-benzyl]-propionamid
84 (R)-2-(3-Fluor-4-methansulfonylamino-phenyl)-N-[2-(4-methyl-piperidin-1-yl)-4-trifluormethyl-benzyl]-propionamid
85 N-(2-Dimethylamino-6-trifluormethyl-pyridin-3-ylmethyl)-2-(3-fluor-4-methansulfonylamino-phenyl)-propionamid
87 2-(3-Fluor-4-methansulfonylamino-phenyl)-N-(2-imidazol-1-yl-6-trifluormethyl-pyridin-3-ylmethyl)-propionamid
88 2-(3-Fluor-4-methansulfonylamino-phenyl)-N-(2-thiophen-2-yl-6-trifluormethyl-pyridin-3-ylmethyl)-propionamid
89 2-(3-Fluor-4-methansulfonylamino-phenyl)-N-[2-(4-fluor-phenyl)-6-trifluormethyl-pyridin-3-ylmethyl]-propionamid
90 N-(2-cyclohexylamino-6-trifluormethyl-pyridin-3-ylmethyl)-2-(3-fluor-4-methansulfonylamino-phenyl)-propionamid
91 2-(3-Fluor-4-methansulfonylamino-phenyl)-N-(2-hexyloxy-6-trifluormethyl-pyridin-3-ylmethyl)-propionamid
93 (S)-2-(3-Fluor-4-methansulfonylamino-phenyl)-N-(2-hexyloxy-6-trifluormethyl-pyridin-3-ylmethyl)-propionamid
94 (R)-2-(3-Fluor-4-methansulfonylamino-phenyl)-N-(2-hexyloxy-6-trifluormethyl-pyridin-3-ylmethyl)-propionamid
95 2-(3-Fluor-4-methansulfonylamino-phenyl)-N-(2-isobutoxy-6-trifluormethyl-pyridin-3-ylmethyl)-propionamid
96 (S)-2-(3-Fluor-4-methansulfonylamino-phenyl)-N-(2-isobutoxy-6-trifluormethyl-pyridin-3-ylmethyl)-propionamid
97 (R)-2-(3-Fluor-4-methansulfonylamino-phenyl)-N-(2-isobutoxy-6-trifluormethyl-pyridin-3-ylmethyl)-propionamid
98 2-(3-Fluor-4-methansulfonylamino-phenyl)-N-(2-isobutoxy-4-trifluormethyl-benzyl)-propionamid
99 (R)-2-(3-Fluor-4-methansulfonylamino-phenyl)-N-(2-isobutoxy-4-trifluormethyl-benzyl)-propionamid
100 N-(2-Cyclopropylmethoxy-6-trifluormethyl-pyridin-3-ylmethyl)-2-(3-fluor-4-methansulfonylamino-phenyl)-propionamid
101 N-(2-Cyclobutylmethoxy-6-trifluormethyl-pyridin-3-ylmethyl)-2-(3-fluor-4-methansulfonylamino-phenyl)-propionamid
102 2-(3-Chlor-4-methansulfonylamino-phenyl)-N-(2-pyrrolidin-1-yl-6-trifluormethyl-pyridin-3-ylmethyl)-propionamid
103 2-(3-Bromo-4-methansulfonylamino-phenyl)-N-(2-pyrrolidin-1-yl-6-trifluormethyl-pyridin-3-ylmethyl)-propionamid
104 N-(4-Benzy)-6'-trifluormethyl-3,4,5,6-tetrahydro-2H-[1,2']bipyridinyl-3'-ylmethyl)-2-(3-fluor-4-methansulfonylamino-phenyl)-propionamid
106 N-(2-Benzyloxy-6-trifluormethyl-pyridin-3-ylmethyl)-2-(3-fluor-4-methansulfonylamino-phenyl)-propionamid
107 2-(3-Fluor-4-methansulfonylamino-phenyl)-N-[2-(3-methoxy-benzyloxy)-6-trifluormethyl-pyridin-3-ylmethyl]-propionamid
108 N-(2-Butoxy-4-tert-butyl-benzyl)-2-(3-fluor-4-methansulfonylamino-phenyl)-propionamid
109 2-(3-Fluor-4-methansulfonylamino-phenyl)-N-[2-(4-phenyl-piperazin-1-yl)-6-trifluormethyl-pyridin-3-ylmethyl]-propionamid
110 2-(3-Fluor-4-methansulfonylamino-phenyl)-N-(2-phenylamino-6-trifluormethyl-pyridin-3-ylmethyl)-propionamid
111 2-(3-Fluor-4-methansulfonylamino-phenyl)-N-(2-propoxy-6-trifluormethyl-pyridin-3-ylmethyl)-propionamid
112 2-(3-Fluor-4-methansulfonylamino-phenyl)-N-[2-(4-fluor-phenylamino)-6-trifluormethyl-pyridin-3-ylmethyl]-propionamid
113 N-[2-(4-Chlor-phenylamino)-6-trifluormethyl-pyridin-3-ylmethyl]-2-(3-fluor-4-methansulfonylamino-phenyl)-propionamid
114 2-(3-Fluor-4-methansulfonylamino-phenyl)-N-(2-fluor-4-trifluormethyl-benzyl)-propionamid
115 N-(2-Benzylamino-6-trifluormethyl-pyridin-3-ylmethyl)-2-(3-fluor-4-methansulfonylamino-phenyl)-propionamid
116 N-(2-Butylamino-6-trifluormethyl-pyridin-3-ylmethyl)-2-(3-fluor-4-methansulfonylamino-phenyl)-propionamid
117 N-[2-(4-tert-Butyl-phenyl)-6-trifluormethyl-pyridin-3-ylmethyl]-2-(3-fluor-4-methansulfonylamino-phenyl)-propionamid
118 N-[2-(3-Chlor-4-fluor-phenyl)-6-trifluormethyl-pyridin-3-ylmethyl]-2-(3-fluor-4-methansulfonylamino-phenyl)-propionamid
120 (S)-N-[2-(3-Chlor-4-fluor-phenyl)-6-trifluormethyl-pyridin-3-ylmethyl]-2-(3-fluor-4-methansulfonylamino-phenyl)-propionamid
121 (R)-N-[2-(3-Chlor-4-fluor-phenyl)-6-trifluormethyl-pyridin-3-ylmethyl]-2-(3-fluor-4-methansulfonylamino-phenyl)-propionamid
122 N-(2-Butylsulfanyl-6-trifluormethyl-pyridin-3-ylmethyl)-2-(3-fluor-4-methansulfonylamino-phenyl)-propionamid
123 2-(3-Fluor-4-methansulfonylamino-phenyl)-N-[2-(3-methyl-butoxy)-6-trifluormethyl-pyridin-3-ylmethyl]-propionamid
124 2-(3-Fluor-4-methansulfonylamino-phenyl)-N-[2-(2-methyl-cyclopropylmethoxy)-6-trifluormethyl-pyridin-3-ylmethyl]-propionamid
125 N-[2-(3,3-Dimethyl-butoxy)-6-trifluormethyl-pyridin-3-ylmethyl]-2-(3-fluor-4-methansulfonylamino-phenyl)-propionamid
126 N-(2-Cyclohexylsulfanyl-6-trifluormethyl-pyridin-3-ylmethyl)-2-(3-fluor-4-methansulfonylamino-phenyl)-propionamid
127 2-(4-methansulfonylamino-3-methyl-phenyl)-N-(6'-trifluormethyl-3,4,5,6-tetrahydro-2H-[1,2']bipyridinyl-3'-ylmethyl)-propionamid
128 N-(2-Azocan-1-yl-6-trifluormethyl-pyridin-3-ylmethyl)-2-(3-fluor-4-methansulfonylamino-phenyl)-propionamid
129 2-(3-Fluor-4-methansulfonylamino-phenyl)-N-(2-pyrrolidin-1-yl-4-trifluormethyl-benzyl)-thiopropionamid
130 2-(3-Fluor-4-methansulfonylamino-phenyl)-N-[2-(4-fluor-phenyl)-6-trifluormethyl-pyridin-3-ylmethyl]-thiopropionamid
131 N-[6'-(Chlor-difluor-methyl)-methyl-3,4,5,6-tetrahydro-2H-[1,2]bipyridinyl-3'-ylmethyl]-2-(3-fluor-4-methansulfonylamino-phenyl)-propionamid
132 N-[2-Azepan-1-yl-6-(chlor-difluor-methyl)-pyridin-3-ylmethyl]-2-(3-fluor-4-methansulfonylamino-phenyl)-propionamid
133 N-(4-tert-Butyl-2-isobutoxy-benzyl)-2-(3-fluor-4-methansulfonylamino-phenyl)-propionamid 134 2-(3-Fluor-4-methansulfonylamino-phenyl)-N-[2-(4-methyl-piperazin-1-yl)-6-trifluormethyl-pyridin-3-ylmethyl]-propionamid
135 N-[2-(3,4-Dimethyl-phenylamino)-6-trifluormethyl-pyridin-3-ylmethyl]-2-(3-fluor-4-methansulfonylamino-phenyl)-propionamid
136 N-[2-(5-Chlor-2-methyl-phenylamino)-6-trifluormethyl-pyridin-3-ylmethyl]-2-(3-fluor-4-methansulfonylamino-phenyl)-propionamid
137 2-(3-Fluor-4-methansulfonylamino-phenyl)-N-(4-phenyl-6'-trifluormethyl-3,4,5,6-tetrahydro-2H-[1,2']bipyridinyl-3'-ylmethyl)-propionamid
138 2-(3-Fluor-4-methansulfonylamino-phenyl)-N-(4-fluor-6'-trifluormethyl-3,4,5,6-tetrahydro-2H-[1,2']bipyridinyl-3'-ylmethyl)-propionamid
139 2-(3-Fluor-4-methansulfonylamino-phenyl)-N-(6'-trifluormethyl-3,6-dihydro-2H-[1,2']bipyridinyl-3'-ylmethyl)-propionamid
140 N-[2-Butoxy-6-(chlor-difluor-methyl)-pyridin-3-ylmethyl]-2-(3-fluor-4-methansulfonylamino-phenyl)-propionamid
142 2-(3-Fluor-4-methansulfonylamino-phenyl)-N-(2-pentyl-6-trifluormethyl-pyridin-3-ylmethyl)-propionamid
144 (S)-2-(3-Fluor-4-methansulfonylamino-phenyl)-N-(2-pentyl-6-trifluormethyl-pyridin-3-ylmethyl)-propionamid
145 (R)-2-(3-Fluor-4-methansulfonylamino-phenyl)-N-(2-pentyl-6-trifluormethyl-pyridin-3-ylmethyl)-propionamid
147 N-[2-(4-Chlor-phenyl)-6-trifluormethyl-pyridin-3-ylmethyl]-2-(3-fluor-4-methansulfonylamino-phenyl)-propionamid
148 2-(3-Fluor-4-methansulfonylamino-phenyl)-N-[2-(3-fluor-phenyl)-6-trifluormethyl-pyridin-3-ylmethyl]-propionamid
149 N-[2-(3-Chlor-phenyl)-6-trifluormethyl-pyridin-3-ylmethyl]-2-(3-fluor-4-methansulfonylamino-phenyl)-propionamid
150 2-(3-Fluor-4-methansulfonylamino-phenyl)-N-[2-(2-fluor-phenyl)-6-trifluormethyl-pyridin-3-ylmethyl]-propionamid
151 2-(3-Fluor-4-methansulfonylamino-phenyl)-N-[2-(4-methoxy-phenyl)-6-trifluormethyl-pyridin-3-ylmethyl]-propionamid
152 N-[4-tert-Butyl-2-(2,2-dimethyl-propoxy)-benzyl]-2-(3-fluor-4-methansulfonylamino-phenyl)-propionamid
153 N-(4-tert-Butyl-2-pentyloxy-benzyl)-2-(3-fluor-4-methansulfonylamino-phenyl)-propionamid
154 N-(4-tert-Butyl-2-cyclohexyloxy-benzyl)-2-(3-fluor-4-methansulfonylamino-phenyl)-propionamid
155 N-(4-tert-Butyl-2-cyclopentyloxy-benzyl)-2-(3-fluor-4-methansulfonylamino-phenyl)-propionamid
156 N-(2-Cyclobutoxy-6-trifluormethyl-pyridin-3-ylmethyl)-2-(3-fluor-4-methansulfonylamino-phenyl)-propionamid
157 2-(3-Fluor-4-methansulfonylamino-phenyl)-N-[2-(4-methyl-cyclohexyloxy)-6-trifluormethyl-pyridin-3-ylmethyl]-propionamid
158 Essigsäure-3'-{[2-(3-fluor-4-methansulfonylamino-phenyl)-propionylamino]-methyl}-6'-trifluormethyl-3,4,5,6-tetrahydro-2H-[1,2']bipyridinyl-4-yl ester
159 2-(3-Fluor-4-methansulfonylamino-phenyl)-N-(4-methoxy-6'-trifluormethyl-3,4,5,6-tetrahydro-2H-[1,2']bipyridinyl-3'-ylmethyl)-propionamid
160 N-(4-Butoxy-6'-trifluormethyl-3,4,5,6-tetrahydro-2H-[1,2']bipyridinyl-3'-ylmethyl)-2-(3-fluor-4-methansulfonylamino-phenyl)-propionamid
161 N-(2-Cyclopentylmethoxy-6-trifluormethyl-pyridin-3-ylmethyl)-2-(3-fluor-4-methansulfonylamino-phenyl)-propionamid
162 2-(3-Fluor-4-methansulfonylamino-phenyl)-N-(4-isopropoxy-6'-trifluormethyl-3,4,5,6-tetrahydro-2H-[1,2']bipyridinyl-3'-ylmethyl)-propionamid
163 N-(2-Ethoxy-6-trifluormethyl-pyridin-3-ylmethyl)-2-(3-fluor-4-methansulfonylamino-phenyl)-propionamid
164 2-(3-Fluor-4-methansulfonylamino-phenyl)-N-(6"-trifluormethyl-3,4,5,6,3',4',5',6'-octahydro-2H,2'H-[1,4';1',2"]terpyridin-3"-ylmethyl)-propionamid
165 2-(3-Fluor-4-methansulfonylamino-phenyl)-N-(4-pyrrolidin-1-yl-6'-trifluormethyl-3,4,5,6-tetrahydro-2H-[1,2']bipyridinyl-3'-ylmethyl)-propionamid
166 N-[6-(Chlor-difluor-methyl)-2-cyclopentyloxy-pyridin-3-ylmethyl]-2-(3-fluor-4-methansulfonylamino-phenyl)-propionamid
167 N-[2-(Butyl-methyl-amino)-6-trifluormethyl-pyridin-3-ylmethyl]-2-(3-fluor-4-methansulfonylamino-phenyl)-propionamid
168 N-[6-(Chlor-difluor-methyl)-2-cyclohexyloxy-pyridin-3-ylmethyl]-2-(3-fluor-4-methansulfonylamino-phenyl)-propionamid
169 N-[2-Benzyloxy-6-(chlor-difluor-methyl)-pyridin-3-ylmethyl]-2-(3-fluor-4-methansulfonylamino-phenyl)-propionamid
170 N-[2-(4-tert-Butyl-cyclohexyloxy)-6-trifluormethyl-pyridin-3-ylmethyl]-2-(3-fluor-4-methansulfonylamino-phenyl)-propionamid
171 N-[2-(4-Ethyl-cyclohexyloxy)-6-trifluormethyl-pyridin-3-ylmethyl]-2-(3-fluor-4-methansulfonylamino-phenyl)-propionamid
172 2-(3-Fluor-4-methansulfonylamino-phenyl)-N-[2-(4-methyl-benzyloxy)-6-trifluormethyl-pyridin-3-ylmethyl]-propionamid
173 N-[2-(4-Chlor-benzylamino)-6-trifluormethyl-pyridin-3-ylmethyl]-2-(3-fluor-4-methansulfonylamino-phenyl)-propionamid
174 N-(2-Azepan-1-yl-4-trifluormethyl-benzyl)-2-(3-fluor-4-methansulfonylamino-phenyl)-propionamid
175 N-[2-(4-Fluor-benzyloxy)-6-trifluormethyl-pyridin-3-ylmethyl]-2-(3-fluor-4-methansulfonylamino-phenyl)-propionamid
176 2-(3-Fluor-4-methansulfonylamino-phenyl)-N-[2-(4-pyridin-4-yl-piperazin-1-yl)-6-trifluormethyl-pyridin-3-ylmethyl]-propionamid
177 2-(3-Fluor-4-methansulfonylamino-phenyl)-N-[2-(pyridin-4-ylmethoxy)-6-trifluormethyl-pyridin-3-ylmethyl]-propionamid
178 2-(3-Fluor-4-methansulfonylamino-phenyl)-N-(2-phenethyloxy-6-trifluormethyl-pyridin-3-ylmethyl)-propionamid
179 2-(3-Fluor-4-methansulfonylamino-phenyl)-N-{2-[4-(4-fluor-phenyl)-piperazin-1-yl]-6-trifluormethyl-pyridin-3-ylmethyl}-propionamid
180 N-[6-(Chlor-difluor-methyl)-2-hexyloxy-pyridin-3-ylmethyl]-2-(3-fluor-4-methansulfonylamino-phenyl)-propionamid
181 N-[6-(Chlor-difluor-methyl)-2-(pyridin-3-ylmethoxy)-pyridin-3-ylmethyl]-2-(3-fluor-4-methansulfonylamino-phenyl)-propionamid
182 N-[6-(Chlor-difluor-methyl)-2-(pyridin-2-ylmethoxy)-pyridin-3-ylmethyl]-2-(3-fluor-4-methansulfonylamino-phenyl)-propionamid
183 N-(2-Dibutylamino-6-trifluormethyl-pyridin-3-ylmethyl)-2-(3-fluor-4-methansulfonylamino-phenyl)-propionamid
184 2-(3-Fluor-4-methansulfonylamino-phenyl)-N-[6'-(4-fluor-phenyl)-4-methyl-3,4,5,6-tetrahydro-2H-[1,2']bipyridinyl-3'-ylmethyl]-propionamid
185 N-[2-Azepan-1-yl-6-(4-fluor-phenyl)-pyridin-3-ylmethyl]-2-(3-fluor-4-methansulfonylamino-phenyl)-propionamid
186 N-[6-(Chlor-difluor-methyl)-2-dipropylamino-pyridin-3-ylmethyl]-2-(3-fluor-4-methansulfonylamino-phenyl)-propionamid
187 N-[6'-(Chlor-difluor-methyl)-3,5-dimethyl-3,4,5,6-tetrahydro-2H-[1,2']bipyridinyl-3'-ylmethyl]-2-(3-fluor-4-methansulfonylamino-phenyl)-propionamid
188 N-[2-(1,3-Dihydro-isoindol-2-yl)-6-trifluormethyl-pyridin-3-ylmethyl]-2-(3-fluor-4-methansulfonylamino-phenyl)-propionamid
189 3'-{[2-(3-Fluor-4-methansulfonylamino-phenyl)-propionylamino]-methyl}-4-phenyl-6'-trifluormethyl-3,4,5,6-tetrahydro-2H-[1,2']bipyridinyl-4-carbonsäureethylester
190 N-(4,6'-Bis-trifluormethyl-3,4,5,6-tetrahydro-2H-[1,2']bipyridinyl-3'-ylmethyl)-2-(3-fluor-4-methansulfonylamino-phenyl)-propionamid
191 2-(3-Fluoro-4-(methylsulfonylamino)phenyl)-N-((2-styryl-6-(trifluoromethyl)pyridin-3-yl)methyl)propanamid
192 2-(3-Fluor-4-methansulfonylamino-phenyl)-N-(2-phenethyl-6-trifluormethyl-pyridin-3-ylmethyl)-propionamid
193 N-{2-[4-(3-Chlor-pyridin-2-yl)-piperazin-1-yl]-6-trifluormethyl-pyridin-3-ylmethyl}-2-(3-fluor-4-methansulfonylamino-phenyl)-propionamid
194 N-{2-[4-(3-Chlor-pyridin-2-yl)-2-methyl-piperazin-1-yl]-6-trifluormethyl-pyridin-3-ylmethyl}-2-(3-fluor-4-methansulfonylamino-phenyl)-propionamid
195 N-(4,6'-Bis-trifluormethyl-3,4,5,6-tetrahydro-2H-[1,2']bipyridinyl-3'-ylmethyl)-2-(4-methansulfonylamino-3-methyl-phenyl)-propionamid
196 2-(4-Methansulfonylamino-3-methyl-phenyl)-N-(4-methyl-6'-trifluormethyl-3,4,5,6-tetrahydro-2H-[1,2']bipyridinyl-3'-ylmethyl)-propionamid
197 N-(4-Ethyl-6'-trifluormethyl-3,4,5,6-tetrahydro-2H-[1,2']bipyridinyl-3'-ylmethyl-(3-fluor-4-methansulfonylamino-phenyl)-propionamid
198 2-(3-Fluor-4-methansulfonylamino-phenyl)-N-(2-phenoxy-6-trifluormethyl-pyridin-3-ylmethyl)-propionamid
199 2-(3-Fluor-4-methansulfonylamino-phenyl)-N-(4-methoxymethyl-6'-trifluormethyl-3,4,5,6-tetrahydro-2H-[1,2']bipyridinyl-3'-ylmethyl)-propionamid
200 2-(3-Fluor-4-methansulfonylamino-phenyl)-N-[4-(4-fluor-phenyl)-6'-trifluormethyl-3,4,5,6-tetrahydro-2H-[1,2']bipyridinyl-3'-ylmethyl]-propionamid
201 2-(3-Fluor-4-methansulfonylamino-phenyl)-N-{2-[4-(2-fluor-phenyl)-piperazin-1-yl]-6-trifluormethyl-pyridin-3-ylmethyl}-propionamid
202 2-(3-Fluor-4-methansulfonylamino-phenyl)-N-[2-(pyridin-2-ylmethoxy)-6-trifluormethyl-pyridin-3-ylmethyl]-propionamid
203 2-(4-Methansulfonylamino-3-methyl-phenyl)-N-[2-(4-phenyl-piperazin-1-yl)-6-trifluormethyl-pyridin-3-ylmethyl]-propionamid
204 N-(2-Benzyloxy-6-trifluormethyl-pyridin-3-ylmethyl)-2-(4-methansulfonylamino-3-methylphenyl)-propionamid
205 2-(3-Fluor-4-methansulfonylamino-phenyl)-N-[2-(methyl-phenyl-amino)-6-trifluormethyl-pyridin-3-ylmethyl]-propionamid
206 2-(3-Fluor-4-methansulfonylamino-phenyl)-N-[4-trifluormethyl-2-(4-trifluormethyl-benzyloxy)-benzyl]-propionamid
207 N-[6-(Chlor-difluor-methyl)-2-(4-phenyl-piperazin-1-yl)-pyridin-3-ylmethyl]-2-(3-fluor-4-methansulfonylamino-phenyl)-propionamid
208 N-[6-(Chlor-difluor-methyl)-2-isobutoxy-pyridin-3-ylmethyl]-2-(3-fluor-4-methansulfonylamino-phenyl)-propionamid
209 N-(2-Benzyloxy-4-trifluormethyl-benzyl)-2-(3-fluor-4-methansulfonylamino-phenyl)-propionamid
210 N-(4,4-Dimethyl-6'-trifluormethyl-3,4,5,6-tetrahydro-2H-[1,2']bipyridinyl-3'-ylmethyl)-2-(3-fluor-4-methansulfonylamino-phenyl)-propionamid
211 2-(3-Fluor-4-methansulfonylamino-phenyl)-N-[2-(pyridin-3-ylmethoxy)-6-trifluormethyl-pyridin-3-ylmethyl]-propionamid
212 2-(3-Fluor-4-methansulfonylamino-phenyl)-N-{6-trifluormethyl-2-[4-(3-trifluormethyl-pyridin-2-yl)-piperazin-1-yl]-pyridin-3-ylmethyl}-propionamid
213 2-(3-Fluor-4-methansulfonylamino-phenyl)-N-{6-trifluormethyl-2-[4-(3-trifluormethyl-pyridin-2-yl)-piperazin-1-yl]-pyridin-3-ylmethyl}-propionamid
214 2-(3-Fluor-4-methansulfonylamino-phenyl)-N-[2-(4-pyridin-2-yl-piperazin-1-yl)-6-trifluormethyl-pyridin-3-ylmethyl]-propionamid
215 2-(3-Fluor-4-methansulfonylamino-phenyl)-N-(6-trifluormethyl-pyridin-3-ylmethyl)-propionamid
216 2-(3-Fluor-4-methansulfonylamino-phenyl)-N-[2-(4-phenyl-piperazin-1-yl)-4-trifluormethyl-benzyl]-propionamid
217 N-(2-Azocan-1-yl-4-trifluormethyl-benzyl)-2-(3-fluor-4-methansulfonylamino-phenyl)-propionamid
218 N-[2-(4,4-Dimethyl-piperidin-1-yl)-4-trifluormethyl-benzyl]-2-(3-fluor-4-methansulfonylamino-phenyl)-propionamid
219 2-(3-Fluor-4-methansulfonylamino-phenyl)-N-[2-(4-p-tolyl-piperazin-1-yl)-6-trifluormethyl-pyridin-3-ylmethyl]-propionamid
220 2-(3-Fluor-4-methansulfonylamino-phenyl)-N-[2-(4-m-tolyl-piperazin-1-yl)-6-trifluormethyl-pyridin-3-ylmethyl]-propionamid
221 2-(3-Fluor-4-methansulfonylamino-phenyl)-N-{2-[4-(4-methoxy-phenyl)-piperazin-1-yl]-6-trifluormethyl-pyridin-3-ylmethyl}-propionamid
222 2-(3-Fluor-4-methansulfonylamino-phenyl)-N-{6-trifluormethyl-2-[4-(4-trifluormethyl-phenyl)-piperazin-1-yl]-pyridin-3-ylmethyl}-propionamid
223 N-(2-Benzyloxy-4-hydroxymethyl-6-trifluormethyl-pyridin-3-ylmethyl)-2-(3-fluor-4-methansulfonylamino-phenyl)-propionamid
225 2-(3-Fluor-4-methansulfonylamino-phenyl)-N-(2-pentyloxy-6-trifluormethyl-pyridin-3-ylmethyl)-propionamid
226 2,2-Dimethyl-propionsäure-3'-{[2-(3-fluor-4-methansulfonylamino-phenyl)-propionylamino]-methyl}-6'-trifluormethyl-3,4,5,6-tetrahydro-2H-[1,2']bipyridinyl-4-yl ester
227 2-(3-Fluor-4-methansulfonylamino-phenyl)-N-(4-oxo-6-trifluormethyl-3,4,5,6-tetrahydro-2H-[1,2']bipyridinyl-3'-ylmethyl)-propionamid
228 N-(4-Ethoxy-6'-trifluormethyl-3,4,5,6-tetrahydro-2H-[1,2']bipyridinyl-3'-ylmethyl)-2-(3-fluor-4-methansulfonylamino-phenyl)-propionamid
229 N-[2-(4-Ethyl-piperidin-1-yl)-4-trifluormethyl-benzyl]-2-(3-fluor-4-methansulfonylamino-phenyl)-propionamid
230 2-(3-Fluor-4-methansulfonylamino-phenyl)-N-[4-trifluormethyl-2-(4-trifluormethyl-piperidin-1-yl)-benzyl]-propionamid
231 N-[2-(4-Benzyl-piperidin-1-yl)-4-trifluormethyl-benzyl]-2-(3-fluor-4-methansulfonylamino-phenyl)-propionamid
233 N-(6-tert-Butyl-2-cyclohexyloxy-pyridin-3-ylmethyl)-2-(3-fluor-4-methansulfonylamino-phenyl)-propionamid
234 N-(6-tert-Butyl-2-cyclopentyloxy-pyridin-3-ylmethyl)-2-(3-fluor-4-methansulfonylamino-phenyl)-propionamid
235 N-(2-Butoxy-6-tert-butyl-pyridin-3-ylmethyl)-2-(3-fluor-4-methansulfonylamino-phenyl)-propionamid
236 N-(6-tert-Butyl-2-hexyloxy-pyridin-3-ylmethyl)-2-(3-fluor-4-methansulfonylamino-phenyl)-propionamid
237 N-(2-Benzyloxy-6-tert-butyl-pyridin-3-ylmethyl)-2-(3-fluor-4-methansulfonylamino-phenyl)-propionamid
238 N-(2-Cyclohexyloxy-4-trifluormethyl-benzyl)-2-(3-fluor-4-methansulfonylamino-phenyl)-propionamid
239 (R)-N-(2-Cyclohexyloxy-4-trifluormethyl-benzyl)-2-(3-fluor-4-methansulfonylamino-phenyl)-propionamid
240 N-(6-tert-Butyl-2-pyrrolidin-1-yl-pyridin-3-ylmethyl)-2-(3-fluor-4-methansulfonylamino-phenyl)-propionamid
241 N-(6'-tert-Butyl-4-methyl-3,4,5,6-tetrahydro-2H-[1,2']bipyridinyl-3'-ylmethyl)-2-(3-fluor-4-methansulfonylamino-phenyl)-propionamid
242 N-[2-(4-Ethyl-benzyloxy)-6-trifluormethyl-pyridin-3-ylmethyl]-2-(3-fluor-4-methansulfonylamino-phenyl)-propionamid
243 N-[2-(4-Butyl-benzyloxy)-6-trifluormethyl-pyridin-3-ylmethyl]-2-(3-fluor-4-methansulfonylamino-phenyl)-propionamid
244 N-[2-(4-tert-Butyl-benzyloxy)-6-trifluormethyl-pyridin-3-ylmethyl]-2-(3-fluor-4-methansulfonylamino-phenyl)-propionamid
245 2-(3-Fluor-4-methansulfonylamino-phenyl)-N-[2-(indan-2-yloxy)-6-trifluormethyl-pyridin-3-ylmethyl]-propionamid
246 2-(3-Fluor-4-methansulfonylamino-phenyl)-N-[2-(4-p-tolyl-piperazin-1-yl)-4-trifluormethyl-benzyl]-propionamid
247 2-(3-Fluor-4-methansulfonylamino-phenyl)-N-[2-(4-m-tolyl-piperazin-1-yl)-4-trifluormethyl-benzyl]-propionamid
248 2-(3-Fluor-4-methansulfonylamino-phenyl)-N-{4-trifluormethyl-2-[4-(4-trifluormethyl-phenyl)-piperazin-1-yl]-benzyl}-propionamid
249 2-(3-Fluor-4-methansulfonylamino-phenyl)-N-{2-[4-(4-methoxy-phenyl)-piperazin-1-yl]-4-trifluormethyl-benzyl}-propionamid
250 N-[2-(3,4-Dichlor-phenyl)-6-trifluormethyl-pyridin-3-ylmethyl]-2-(3-fluor-4-methansulfonylamino-phenyl)-propionamid
251 N-[2-(3-tert-Butyl-1-oxa-2,8-diaza-spiro[4.5]dec-2-en-8-yl)-6-trifluormethyl-pyridin-3-ylmethyl]-2-(3-fluor-4-methansulfonylamino-phenyl)-propionamid
252 2-(3-Fluor-4-methansulfonylamino-phenyl)-N-[2-(3-phenyl-1-oxa-2,8-diaza-spiro[4.5]dec-2-en-8-yl)-6-trifluormethyl-pyridin-3-ylmethyl]-propionamid
253 2-(3-Fluor-4-(pentafluorsulfanylsulfonamido)phenyl)-N-p-tolylpropanamid
254 2-(3-Fluor-4-methansulfonylamino-phenyl)-N-[2-(3-fluor-4-methoxy-phenyl)-6-trifluormethyl-pyridin-3-ylmethyl]-propionamid
255 2-(3-Fluor-4-methansulfonylamino-phenyl)-N-{2-[4-(4-fluor-phenyl)-piperidin-1-yl]-4-trifluormethyl-benzyl}-propionamid
256 N-(2-Butoxy-6-trifluormethyl-pyridin-3-ylmethyl)-2-(4-methansulfonylamino-3-methyl-phenyl)-propionamid
257 N-(2-Hexyloxy-6-trifluormethyl-pyridin-3-ylmethyl)-2-(4-methansulfonylamino-3-methyl-phenyl)-propionamid
258 N-[2-(4-Chlor-benzyloxy)-6-trifluormethyl-pyridin-3-ylmethyl]-2-(3-fluor-4-methansulfonylamino-phenyl)-propionamid
259 N-(4-Dimethylaminomethyl-4-phenyl-6'-trifluormethyl-3,4,5,6-tetrahydro-2H-[1,2']bipyridinyl-3'-ylmethyl)-2-(3-fluor-4-methansulfonylamino-phenyl)-propionamid
260 N-[2-(4-Cyclohexyl-piperazin-1-yl)-6-trifluormethyl-pyridin-3-ylmethyl]-2-(3-fluor-4-methansulfonylamino-phenyl)-propionamid
261 N-(6-tert-Butyl-2-cyclopentyloxy-4-hydroxymethyl-pyridin-3-ylmethyl)-2-(3-fluor-4-methansulfonylamino-phenyl)-propionamid
262 2-(4-Methansulfonylamino-phenyl)-N-(4-methyl-6'-trifluormethyl-3,4,5,6-tetrahydro-2H-[1,2']bipyridinyl-3'-ylmethyl)-propionamid
263 N-[2-(3,3-Dimethyl-butyl)-6-trifluormethyl-pyridin-3-ylmethyl]-2-(3-fluor-4-methansulfonylamino-phenyl)-propionamid
264 2-(3-Fluor-4-methansulfonylamino-phenyl)-N-[2-(2-p-tolyl-ethyl)-6-trifluormethyl-pyridin-3-ylmethyl]-propionamid
265 N-[2-(2,3-Dihydro-benzo[1,4]dioxin-6-yl)-6-trifluormethyl-pyridin-3-ylmethyl]-2-(3-fluor-4-methansulfonylamino-phenyl)-propionamid
266 N-(2-Benzo[1,3]dioxol-5-yl-6-trifluormethyl-pyridin-3-ylmethyl)-2-(3-fluor-4-methansulfonylamino-phenyl)-propionamid
267 2-(3-Fluor-4-methansulfonylamino-phenyl)-N-(2-hexyl-6-trifluormethyl-pyridin-3-ylmethyl)-propionamid
268 2-(3-Fluor-4-methansulfonylamino-phenyl)-N-[2-(4-methyl-pentyl)-6-trifluormethyl-pyridin-3-ylmethyl]-propionamid
269 2-(3-Fluor-4-methansulfonylamino-phenyl)-N-(4-hydroxy-6'-trifluormethyl-3,4,5,6-tetrahydro-2H-[1,2']bipyridinyl-3'-ylmethyl)-propionamid
270 N-(2-Cyclohexylmethoxy-6-trifluormethyl-pyridin-3-ylmethyl)-2-(3-fluor-4-methansulfonylamino-phenyl)-propionamid
271 2-(3-Fluor-4-methansulfonylamino-phenyl)-N-[2-(4-methyl-cyclohexylmethoxy)-6-trifluormethyl-pyridin-3-ylmethyl]-propionamid
272 2-(3-Fluor-4-methansulfonylamino-phenyl)-N-[2-(3-methansulfonylamino-phenyl)-6-trifluormethyl-pyridin-3-ylmethyl]-propionamid
273 2-(3-Fluor-4-methansulfonylamino-phenyl)-N-[2-(2-methyl-propenyl)-6-trifluormethyl-pyridin-3-ylmethyl]-propionamid
274 N-[2-(3,3-Dimethyl-but-1-enyl)-6-trifluormethyl-pyridin-3-ylmethyl]-2-(3-fluor-4-methansulfonylamino-phenyl)-propionamid
275 2-(3-Fluor-4-methansulfonylamino-phenyl)-N-[2-(1H-indol-6-yl)-6-trifluormethyl-pyridin-3-ylmethyl]-propionamid
276 2-(3-Fluor-4-methansulfonylamino-phenyl)-N-[2-(1H-indol-5-yl)-6-trifluormethyl-pyridin-3-ylmethyl]-propionamid
277 N-[2-(4-Chlor-3-fluor-phenyl)-6-trifluormethyl-pyridin-3-ylmethyl]-2-(3-fluor-4-methansulfonylamino-phenyl)-propionamid
278 2-(3-Fluor-4-methansulfonylamino-phenyl)-N-[2-(4-fluor-3-methyl-phenyl)-6-trifluormethyl-pyridin-3-ylmethyl]-propionamid
279 N-[2-(2,2-Dimethyl-cyclopropylmethoxy)-6-trifluormethyl-pyridin-3-ylmethyl]-2-(3-fluor-4-methansulfonylamino-phenyl)-propionamid
282 2-(3-Fluor-4-methansulfonylamino-phenyl)-N-(3-methyl-6'-trifluormethyl-3,4,5,6-tetrahydro-2H-[1,2']bipyridinyl-3'-ylmethyl)-propionamid
283 2-(3-Fluor-4-methansulfonylamino-phenyl)-N-(2-methyl-6'-trifluormethyl-3,4,5,6-tetrahydro-2H-[1,2']bipyridinyl-3'-ylmethyl)-propionamid
284 2-(3-Fluor-4-methansulfonylamino-phenyl)-N-[2-(4-methyl-6'-trifluormethyl-3,4,5,6-tetrahydro-2H-[1,2']bipyridinyl-3'-yl)-ethyl]-propionamid
285 N-(4-Cyano-4-phenyl-6'-trifluormethyl-3,4.5,6-tetrahydro-2H-[1,2']bipyridinyl-3'-ylmethyl)-2-(3-fluor-4-methansulfonylamino-phenyl)-propionamid
287 2-(4-Ethansulfonylamino-3-fluor-phenyl)-N-(4-methyl-6'-trifluormethyl-3,4,5,6-tetrahydro-2H-[1,2']bipyridinyl-3'-ylmethyl)-propionamid
288 2-(4-(N,N-dimethylsulfamoylamino)-3-fluorphenyl)-N-((2-(4-methylpiperidin-1-yl)-6-(trifluormethyl)pyridin-3-yl)methyl)propanamid
289 2-(4-Methansulfonylamino-3-methoxy-phenyl)-N-(4-methyl-6'-trifluormethyl-3,4,5,6-tetrahydro-2H-[1,2']bipyridinyl-3'-ylmethyl)-propionamid
290 2-(3-Fluor-4-methansulfonylamino-phenyl)-N-(4-phenylamino-6'-trifluormethyl-3,4,5,6-tetrahydro-2H-[1,2']bipyridinyl-3'-ylmethyl)-propionamid
291 N-(2-Cyclohexyl-6-trifluormethyl-pyridin-3-ylmethyl)-2-(3-fluor-4-methansulfonylamino-phenyl)-propionamid
292 2-(3-Fluor-4-methansulfonylamino-phenyl)-N-(4-phenyl-6-trifluormethyl-pyridin-3-ylmethyl)-propionamid
293 2-(3-Fluor-4-methansulfonylamino-phenyl)-N-(4-methyl-6'-trifluormethyl-3,4,5,6-tetrahydro-2H-[1,2']bipyridinyl-3'-ylmethyl)-thiopropionamid
294 N-(2-cyclohexylsulfanyl-6-trifluormethyl-pyridin-3-ylmethyl)-2-(3-fluor-4-methansulfonylamino-phenyl)-propionamid
295 2-(3-Fluor-4-methansulfonylamino-phenyl)-N-(4-phenyl-6'-trifluormethyl-3,4,5,6-tetrahydro-2H-[1,2']bipyridinyl-3'-ylmethyl)-propionamid
296 N-(2-Azepan-1-yl-6-tert-butyl-pyridin-3-ylmethyl)-2-(3-fluor-4-methansulfonylamino-phenyl)-propionamid
297 N-(6-tert-Butyl-2-dipropylamino-pyridin-3-ylmethyl)-2-(3-fluor-4-methansulfonylamino-phenyl)-propionamid
298 N-(2-But-2-enyloxy-6-trifluormethyl-pyridin-3-ylmethyl)-2-(3-fluor-4-methansulfonylamino-phenyl)-propionamid
299 2-(3-Fluor-4-methansulfonylamino-phenyl)-N-(2-pent-2-enyloxy-6-trifluormethyl-pyridin-3-ylmethyl)-propionamid
300 2-(3-Fluor-4-methansulfonylamino-phenyl)-N-(2-pent-1-enyl-6-trifluormethyl-pyridin-3-ylmethyl)-propionamid
301 2-(3-Fluor-4-methansulfonylamino-phenyl)-N-(2-pent-1-enyl-6-trifluormethyl-pyridin-3-ylmethyl)-propionamid
302 2-(3-Fluor-4-methansulfonylamino-phenyl)-N-(2-hexyloxy-4-methyl-6-trifluormethyl-pyridin-3-ylmethyl)-propionamid
303 2-(3-Fluor-4-methansulfonylamino-phenyl)-N-{2-[2-(4-fluor-phenyl)-ethyl]-6-trifluormethyl-pyridin-3-ylmethyl}-propionamid
304 N-(4-Acetyl-4-phenyl-6'-trifluormethyl-3,4,5,6-tetrahydro-2H-[1,2']bipyridinyl-3'-ylmethyl)-2-(3-fluor-4-methansulfonylamino-phenyl)-propionamid
307 2-(3-Fluor-4-methansulfonylamino-phenyl)-N-[4-(phenyl-propionyl-amino)-6'-trifluormethyl-3,4,5,6-tetrahydro-2H-[1,2']bipyridinyl-3'-ylmethyl]-propionamid
308 N-[2-(4-Dimethylamino-phenyl)-6-trifluormethyl-pyridin-3-ylmethyl]-2-(3-fluor-4-methansulfonylamino-phenyl)-propionamid
309 2-[3-Fluor-4-(propan-2-sulfonylamino)-phenyl]-N-(4-methyl-6'-trifluormethyl-3,4,5,6-tetrahydro-2H-[1,2']bipyridinyl-3'-ylmethyl)-propionamid
310 2-[3-Fluor-4-(2,2,2-trifluor-ethansulfonylamino)-phenyl]-N-(4-methyl-6'-trifluormethyl-3,4,5,6-tetrahydro-2H-[1,2']bipyridinyl-3'-ylmethyl)-propionamid
311 N-[2-(2,6-Dimethyl-morpholin-4-yl)-6-trifluormethyl-pyridin-3-ylmethyl]-2-(3-fluor-4-methansulfonylamino-phenyl)-propionamid
312 2-(3-Fluor-4-trifluormethansulfonylamino-phenyl)-N-(4-methyl-6'-trifluormethyl-3,4,5,6-tetrahydro-2H-[1,2']bipyridinyl-3'-ylmethyl)-propionamid
313 2-(3-Fluor-4-(sulfamoylamino)phenyl)-N-((2-(4-methylpiperidin-1-yl)-6-(trifluormethyl)pyridin-3-yl)methyl)propanamid
314 N-[2-(1,1-Dioxo-1/6-thiomorpholin-4-yl)-6-trifluormethyl-pyridin-3-ylmethyl]-2-(3-fluor-4-methansulfonylamino-phenyl)-propionamid
315 N-(6'-Difluormethyl-4-methyl-3,4,5,6-tetrahydro-2H-[1,2']bipyridinyl-3'-ylmethyl)-2-(3-fluor-4-methansulfonylamino-phenyl)-propionamid
316 N-(4,6'-Dimethyl-3,4,5,6-tetrahydro-2H-[1,2']ipyridinyl-3'-ylmethyl)-2-(3-fluor-4-methansulfonylamino-phenyl)-propionamid
317 2-(3-Fluor-4-methansulfonylamino-phenyl)-N-(4-phenyl-6'-trifluormethyl-3,6-dihydro-2H-[1,2']bipyridinyl-3'-ylmethyl)-propionamid
318 N-(4,4'-Dimethyl-6'-trifluormethyl-3,4,5,6-tetrahydro-2H-[1,2']bipyridinyl-3'-ylmethyl)-2-(3-fluor-4-methansulfonylamino-phenyl)-propionamid
319 N-[2-(4-Cyclohexyl-piperazin-1-yl)-4-trifluormethyl-benzyl]-2-(3-fluor-4-methansulfonylamino-phenyl)-propionamid
320 N-(4'-tert-Butyl-5-trifluormethyl-biphenyl-2-ylmethyl)-2-(3-fluor-4-methansulfonylamino-phenyl)-propionamid
321 2-(3-Fluor-4-methansulfonylamino-phenyl)-N-(4'-methoxy-5-trifluormethyl-biphenyl-2-ylmethyl)-propionamid
322 N-(3'-Chlor-5-trifluormethyl-biphenyl-2-ylmethyl)-2-(3-fluor-4-methansulfonylamino-phenyl)-propionamid
323 2-(3-Fluor-4-methansulfonylamino-phenyl)-N-(3'-fluor-5-trifluormethyl-biphenyl-2-ylmethyl)-propionamid
324 N-(3'-Chlor-4'-fluor-5-trifluormethyl-biphenyl-2-ylmethyl)-2-(3-fluor-4-methansulfonylamino-phenyl)-propionamid
325 N-(3',4'-Dimethoxy-5-trifluormethyl-biphenyl-2-ylmethyl)-2-(3-fluor-4-methansulfonylamino-phenyl)-propionamid
326 N-[2-(3,4-Dimethoxy-phenyl)-6-trifluormethyl-pyridin-3-ylmethyl]-2-(3-fluor-4-methansulfonylamino-phenyl)-propionamid
327 4-(3-{[2-(3-Fluor-4-methansulfonylamino-phenyl)-propionylamino]-methyl}-6-trifluormethyl-pyridin-2-yloxymethyl)-piperidin-1-carbonsäure tert-butyl ester
328 N-(6-tert-Butyl-2-pentyloxy-pyridin-3-ylmethyl)-2-(3-fluor-4-methansulfonylamino-phenyl)-propionamid
329 N-[6-tert-Butyl-2-(3-methyl-butoxy)-pyridin-3-ylmethyl]-2-(3-fluor-4-methansulfonylamino-phenyl)-propionamid
330 N-(4-Dimethylamino-4-phenyl-6'-trifluormethyl-3,4,5,6-tetrahydro-2H-[1,2']bipyridinyl-3'-ylmethyl)-2-(3-fluor-4-methansulfonylamino-phenyl)-propionamid
331 N-(2-Dipropylamino-6-trifluormethyl-pyridin-3-ylmethyl)-2-(4-methansulfonylamino-3-methylphenyl)-propionamid
332 2-(3-Fluor-4-methansulfonylamino-phenyl)-N-[4-(4-fluor-phenyl)-6'-trifluormethyl-3,6-dihydro-2H-[1,2']bipyridinyl-3'-ylmethyl]-propionamid
334 N-(2-Cyclohex-1-enyl-6-trifluormethyl-pyridin-3-ylmethyl)-2-(3-fluor-4-methansulfonylamino-phenyl)-propionamid
335 N-[2-(1-Ethyl-propoxy)-6-trifluormethyl-pyridin-3-ylmethyl]-2-(3-fluor-4-methansulfonylamino-phenyl)-propionamid
336 2-(3-Fluor-4-methansulfonylamino-phenyl)-N-[2-(1-propyl-butoxy)-6-trifluormethyl-pyridin-3-ylmethyl]-propionamid
337 2-(3-Fluor-4-methansulfonylamino-phenyl)-N-[2-(1-isobutyl-3-methyl-butoxy)-6-trifluormethyl-pyridin-3-ylmethyl]-propionamid
338 N-[2-(4,4-Dimethyl-cyclohexyloxy)-6-trifluormethyl-pyridin-3-ylmethyl]-2-(3-fluor-4-methansulfonylamino-phenyl)-propionamid
339 2-(3-Fluor-4-methansulfonylamino-phenyl)-N-[6-trifluormethyl-2-(4-trifluormethyl-cyclohexyloxy)-pyridin-3-ylmethyl]-propionamid
340 2-(3-Fluor-4-methansulfonylamino-phenyl)-N-[6-trifluormethyl-2-(4-trifluormethyl-cyclohexyloxy)-pyridin-3-ylmethyl]-propionamid
341 4-(3-{[2-(3-Fluor-4-methansulfonylamino-phenyl)-propionylamino]-methyl}-6-trifluormethyl-pyridin-2-yloxy)-piperidin-1-carbonsäure tert-butyl ester
342 4-[(3-{[2-(3-Fluor-4-methansulfonylamino-phenyl)-propionylamino]-methyl}-6-trifluormethyl-pyridin-2-ylamino)-methyl]-piperidin-1-carbonsäure tert-butyl ester
343 2-(3-Fluor-4-methansulfonylamino-phenyl)-N-[2-(piperidin-4-ylmethoxy)-6-trifluormethyl-pyridin-3-ylmethyl]-propionamid
344 2-(3-Fluor-4-methansulfonylamino-phenyl)-N-[2-(piperidin-4-yloxy)-6-trifluormethyl-pyridin-3-ylmethyl]-propionamid
345 2-(3-Fluor-4-methansulfonylamino-phenyl)-N-(2-p-tolyloxy-6-trifluormethyl-pyridin-3-ylmethyl)-propionamid
346 N-[2-(2-Cyclohexyl-vinyl)-6-trifluormethyl-pyridin-3-ylmethyl]-2-(3-fluor-4-methansulfonylamino-phenyl)-propionamid
347 2-(3-Fluor-4-methansulfonylamino-phenyl)-N-(4-methyl-6'-trifluormethyl-3,4 ,5,6-tetrahydro-2H-[1,2']bipyridinyl-3'-ylmethyl)-butyramid
348 N-[2-(3,5-Dimethoxy-phenyl)-6-trifluormethyl-pyridin-3-ylmethyl]-2-(3-fluor-4-methansulfonylamino-phenyl)-propionamid
349 N-(2-Cyclopentyloxy-4-methyl-6-trifluormethyl-pyridin-3-ylmethyl)-2-(3-fluor-4-methansulfonylamino-phenyl)-propionamid
350 N-(3',5'-Dimethoxy-5-trifluormethyl-biphenyl-2-ylmethyl)-2-(3-fluor-4-methansulfonylamino-phenyl)-propionamid
351 Ethyl 5-((2-(3-fluor-4-(methylsulfonylamino)phenyl)propanamido)methyl)-6-(4-methylpiperidin-1-yl)-2-(trifluormethyl)nicotinat
352 2-(3-Fluor-4-(methylsulfonylamino)phenyl)-N-((2-(nonan-5-yloxy)-6-(trifluormethyl)pyridin-3-yl)methyl)propanamid
353 N-((6-tert-Butyl-2-isobutoxypyridin-3-yl)methyl)-2-(3-fluor-4-(methylsulfonylamino)phenyl)propanamid
354 2-(3-Fluor-4-(methylsulfonylamino)phenyl)-N-((2-(phenylethinyl)-6-(trifluormethyl)pyridin-3-yl)methyl)propanamid
355 2-(3-Fluor-4-(methylsulfonylamino)phenyl)-N-((2-(3-methoxypropoxy)-6-(trifluormethyl)pyridin-3-yl)methyl)propanamid
356 N-((2-(4-Benzylpiperidin-1-yl)-4-methyl-6-(trifluormethyl)pyridin-3-yl)methyl)-2-(3-fluor-4-(methylsulfonylamino)phenyl)propanamid
357 2-(3-Fluoro-4-methansulfonylamino-phenyl)-N-(4-methylen-6'-trifluoromethyl-3,4,5,6-tetrahydro-2H-[1,2']bipyridinyl-3'-ylmethyl)-propionamid
358 N-[2-(6-Aza-spiro[2.5]oct-6-yl)-6-trifluoromethyl-pyridin-3-ylmethyl]-2-(3-fluoro-4-methansulfonylamino-phenyl)-propionamid
359 2-(3-Fluoro-4-methansulfonylamino-phenyl)-N-[2-(3-methyl-but-2-enyloxy)-6-trifluoromethyl-pyridin-3-ylmethyl]-propionamid
360 N-[2-(3-Cyclohexyl-propyl)-6-trifluoromethyl-pyridin-3-ylmethyl]-2-(3-fluoro-4-methansulfonylamino-phenyl)-propionamid
361 N-[2-(3-Ethoxy-propoxy)-6-trifluoromethyl-pyridin-3-ylmethyl]-2-(3-fluoro-4-methansulfonylamino-phenyl)-propionamid
362 2-(3-Fluoro-4-methansulfonylamino-phenyl)-N-[2-(2-phenoxy-ethoxy)-6-trifluoromethyl-pyridin-3-ylmethyl]-propionamid
363 N-[2-(3,5-Dimethoxy-benzyloxy)-6-trifluoromethyl-pyridin-3-ylmethyl]-2-(3-fluoro-4-methansulfonylamino-phenyl)-propionamid
364 2-(3-Fluoro-4-methansulfonylamino-phenyl)-N-(4-hydroxymethyl-6'-trifluoro methyl-3,4,5,6-tetrahydro-2H-[1,2']bipyridinyl-3'-ylmethyl)-propionamid
365 N-(6'-tert-Butyl-4-phenyl-3,4.5.6-tetrahydro-2H-[1,2']bipyridinyl-3'-ylmethyl)-2-(3-fluoro-4-methansulfonylamino-phenyl)-propionamid
366 N-{6-tert-Butyl-2-[4-(4-fluoro-phenyl)-piperazin-1-yl]-pyridin-3-ylmethyl}-2-(3-fluoro-4-methansulfonylamino-phenyl)-propionamid
367 2-(4-Methansulfonylamino-3-methyl-phenyl)-N-(2-pyrrolidin-1-yl-6-trifluoromethyl-pyridin-3-ylmethyl)-propionamid
368 N-((2-(1H-Indol-4-yl)-6-(trifluoromethyl)pyridin-3-yl)methyl)-2-(3-fluor-4-(methylsulfonylamino)phenyl)propanamid,
369 N-((6-tert-Butyl-2-propoxypyridin-3-yl)methyl)-2-(3-fluor-4-(methylsulfonylamino)phenyl)propanamid,
370 N-((6-tert-Butyl-2-(3-methoxypropoxy)pyridin-3-yl)methyl)-2-(3-fluor-4-(methylsulfonylamino)phenyl)propanamid,
371 N-((6-tert-Butyl-2-(4-(dimethylamino)-4-phenylpiperidin-1-yl)pyridin-3-yl)methyl)-2-(3-fluor-4-(methylsulfonylamino)phenyl)propanamid,
372 N-((6-tert-Butyl-2-methoxypyridin-3-yl)methyl)-2-(3-fluor-4-(methylsulfonylamino)phenyl)propanamide,
373 N-((6-tert-Butyl-2-ethoxypyridin-3-yl)methyl)-2-(3-fluor-4-(methylsulfonylamino)phenyl)propanamid,
374 N-((6-tert-Butyl-2-isopropoxypyridin-3-yl)methyl)-2-(3-fluor-4-(methylsulfonylamino)phenyl)propanamid,
375 2-(3-Fluor-4-(methylsulfonylamino)phenyl)-N-((2-(pentyloxy)-6-(trifluoromethyl)pyridin-3-yl)methyl)propanamid,
376 2-(3-Fluor-4-(methylsulfonylamino)phenyl)-N-((2-(hexyloxy)-6-(trifluoromethyl)pyridin-3-yl)methyl)propanamid,
377 N-((2-(3,5-Dimethylcyclohexyloxy)-6-(trifluoromethyl)pyridin-3-yl)methyl)-2-(3-fluor-4-(methylsulfonylamino)phenyl)propanamid,
378 N-((6-tert-Butyl-2-(2-ethoxyethoxy)pyridin-3-yl)methyl)-2-(3-fluor-4-(methylsulfonylamino)phenyl)propanamid,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiter bevorzugt sind die Verbindungen 126, 166, 174, 291, 83, 80, 89, 91, 104, 117, 118, 131, 137, 140, 142, 149, 160, 166, 167, 168, 172, 218, 235, 127, 196, 256, 257 und 204; noch weiter bevorzugt sind die Verbindungen 126, 166, 174, 291, 83, 80, 89, 91, 104, 117, 118, 131, 137, 140, 142, 149, 160, 166, 167, 168, 172, 218 und 235; am weitesten bevorzugt sind die Verbindungen 126, 166, 174 und 291.

Ein weiterer Gegenstand der vorliegenden Erfindung sind Verbindungen der allgemeinen Formel I, 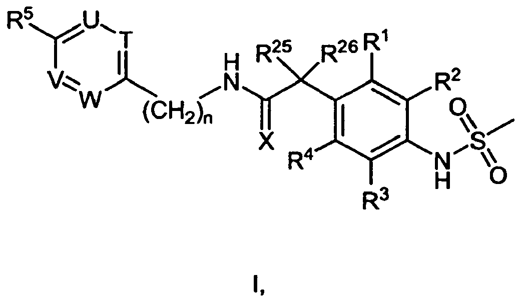 worin
X für O, S oder N-C≡N steht;
n für 0, 1, 2, 3 oder 4 steht;
R¹, R², R³ und R⁴, unabhängig voneinander, jeweils für H; F; Cl; Br; I; -SF₅; -NO₂; -CF₃; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³; -S(=O)₂-R²⁴ oder für einen linearen oder verzweigten, gesättigten oder ungesättigten, unsubstituierten oder wenigstens einfach substituierten aliphatischen C₁₋₁₀ Rest stehen;
R⁵ für H; F; Cl; Br; I; -SF₅: -NO₂; -CF₃; -CF₂Cl; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; - C(=O)OR²²; -C(=O)-R²³; -S(=O)₂-R²⁴;
für einen linearen oder verzweigten, gesättigten oder ungesättigten, unsubstituierten oder wenigstens einfach substituierten aliphatischen C₁₋₁₀ Rest;
oder für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest steht, der jeweils über ein Kohlenstoffatom im Ring des cycloaliphatischen Restes an das Grundgerüst gebunden ist;
T für C-R⁶ und U für C-R⁷ und V für N und W für C-R⁸
oder
T für C-R⁶ und U für N und V für C-R⁹ und W für C-R⁸
oder
T für N und U für C-R⁷ und V für C-R⁹ und W für C-R⁸
oder
T für N und U für N und V für C-R⁹ und W für C-R⁸
oder
T für N und U für C-R⁷ und V für N und W für C-R⁸
oder
T für C-R⁶ und U für N und V für N und W für C-R⁸
oder
T für C-R⁶ und U für C-R⁷ und V für C-R⁹ und W für C-R¹⁰
steht;
R⁶ und R⁷, unabhängig voneinander, jeweils für H; F; Cl; Br; I; -SF₅; -NO₂; -CF₃; - CF₂Cl; -CN; -NH₂; -OH; -SH; -C(=O)-NH₂; -S(=O)₂-NH₂; -C(=O)-NH-OH: -C(=O)-OH: -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³, -S(=O)₂-R²⁴ oder für einen linearen oder verzweigten, gesättigten oder ungesättigten, unsubstituierten oder wenigstens einfach substituierten aliphatischen C₁₋₁₀ Rest stehen;
R⁸ für H; F; Cl; Br; I; -SF₅; -NO₂; -CF₃; -CF₂Cl; -CN; -NH₂; -OH; -SH; -C(=O)-NH₂; - S(=O)₂-NH₂; -C(=O)-NH-OH; -C(=O)-OH; -C(=O)-H; -S(=O)₂-OH; -NHR¹¹; -NR¹²R¹³; - OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; - C(=O)-OR²²; -C(=O)-R²³; -S(=O)₂-R²⁴;
für einen linearen oder verzweigten, gesättigten oder ungesättigten, unsubstituierten oder wenigstens einfach substituierten aliphatischen C₁₋₁₀ Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe gebunden sein kann, steht;
R⁹ für H; F; Cl; Br; I; -SF₅; -NO₂; -CF₃; -CF₂Cl; -CN; -NH₂; -OH; -SH; -C(=O)-NH₂; -S(=O)₂-NH₂, -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; C(=O)OR²²; -C(=O)-R²³; -S(=O)₂-R²⁴ oder für einen linearen oder verzweigten, gesättigten oder ungesättigten, unsubstituierten oder wenigstens einfach substituierten aliphatischen C₁₋₁₀ Rest steht;
R¹⁰ für -SF₅; -NO₂; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³; -S(=O)₂-R²⁴;
für einen linearen oder verzweigten, gesättigten oder ungesättigten aliphatischen C₁₋₁₀ Rest, der jeweils mit ggf. 1, 2, 3, 4, 5, 6, 7, 8 oder 9 Substituenten unabhängig voneinander ausgewählt aus der Gruppe bestehend aus -CN, -NO₂, -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), -OCF₃ und - SCF₃ substituiert ist;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe gebunden sein kann;
oder für einen unsubstituierten oder wenigstens einfach substituierten 5- bis 14-gliedrigen Aryl- oder Heteroaryl-Rest; der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe gebunden sein kann, steht;
R¹¹, R¹², R¹³, R¹⁴, R¹⁵, R¹⁶, R¹⁷, R¹⁸, R¹⁹, R²⁰, R²¹, R²², R²³ und R²⁴, unabhängig voneinander, jeweils
für einen linearen oder verzweigten, gesättigten oder ungesättigten, unsubstituierten oder wenigstens einfach substituierten aliphatischen C₁₋₁₀ Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe gebunden sein kann;
oder für einen unsubstituierten oder wenigstens einfach substituierten 5- bis 14-gliedrigen Aryl- oder Heteroaryl-Rest; der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe gebunden sein kann; stehen;
oder
R¹² und R¹³ jeweils zusammen mit dem sie verbindenden Stickstoffatom als Ringglied einen gesättigten oder ungesättigten, unsubstituierten oder wenigstens einfach substituierten 4-, 5-, 6-, 7-, 8- oder 9-gliedrigen heterocycloaliphatischen Rest bilden, der mit einem gesättigten oder ungesättigten, unsubstituierten oder wenigstens einfach substituierten mono- oder polyzyklischen Ringsystem kondensiert sein kann;
und
R²⁵ und R²⁶, unabhängig voneinander, jeweils für einen Wasserstoff-Rest;
für einen linearen oder verzweigten, gesättigten oder ungesättigten, unsubstituierten oder wenigstens einfach substituierten aliphatischen C₁₋₁₀ Rest;
oder für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest stehen;
mit der Maßgabe, dass R²⁵ und R²⁶ nicht jeweils für einen Wasserstoff-Rest stehen;
oder
R²⁵ und R²⁶ zusammen mit dem sie verbindenden Kohlenstoffatom als Ringglied einen gesättigten oder ungesättigten, unsubstituierten oder wenigstens einfach substituierten 3-, 4-, 5- oder 6-gliedrigen cycloaliphatischen Rest bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Sofern nicht anders angegeben, können die vorstehend genannten aliphatischen C₁₋₁₀ Reste vorzugsweise ggf. jeweils mit 1, 2, 3, 4, 5, 6, 7, 8 oder 9 Substituenten unabhängig voneinander ausgewählt aus der Gruppe bestehend aus F, Cl, Br, I, - CN, -NO₂, -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), -OCF₃ und -SCF₃ substituiert sein.

Bevorzugt können die vorstehend genannten C₁₋₆-Alkylen-Gruppen ggf. jeweils mit 1, 2, 3, 4, 5, 6, 7, 8 oder 9 Substituenten unabhängig voneinander ausgewählt aus der Gruppe bestehend aus F, CI, Br, I, -CN, -NO₂, -OH, -NH₂, -SH, -O(C₁₋₅-Alkyl),S(C₁₋₅-Alkyl), -NH(C₁₋₅-Alkyl), -N(C₁₋₅-Alkyl)(C₁₋₅-Alkyl), -OCF₃ und -SCF₃ substituiert sein.

Bevorzugt können die vorstehend genannten (hetero)cycloaliphatischen Reste ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-C₁₋₅-Alkyl, -C(=O)-OH, -C(=O)-O-C₁₋₅-Alkyl, -NH-C₁₋₅-Alkyl, -N(C₁₋₅-Alkyl)₂, -O-Phenyl, -O-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, - CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann.

Ebenfalls bevorzugt können die vorstehend genannten (hetero)cycloaliphatischen Reste jeweils ggf. 1, 2 oder 3 (weitere) Heteroatom(e) unabhängig voneinander ausgewählt aus der Gruppe bestehend aus Sauerstoff, Stickstoff und Schwefel aufweisen.

Vorzugsweise können die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅Alkyl, -NH₂, -NO₂, -O-CF₃, -S-CF₃, -SH, -S-C₁₋₅-Alkyl, -C₁₋₅-Alkyl, -C(=O)-C₁₋₅-Alkyl, -C(=O)-OH, -C(=O)-O-C₁₋₅-Alkyl, -NH-C₁₋₅-Alkyl, -N(C₁₋₅-Alkyl)₂, -O-Phenyl, -O-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, - C₁₋₅-Alkyl, -O-C₁₋₅-Alkyl, -O-CF₃, -S-CF₃, Phenyl und -O-Benzyl substituiert sein kann.

Vorzugsweise sind die Ringe der vorstehend genannten mono- oder polyzyklischen Ringsysteme jeweils 5-, 6- oder 7-gliedrig und können jeweils ggf. 1, 2, 3, 4 oder 5 Heteroatom(e) als Ringglied(er) aufweisen, die unabhängig voneinander aus der Gruppe bestehend aus Sauerstoff, Stickstoff und Schwefel ausgewählt sind.

Ebenfalls bevorzugt können die vorstehend genannten Aryl- oder Heteroaryl-Reste ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, - C(=O)-NH₂, -C(=O)-NH-C₁₋₅-Alkyl, -C(=O)-N-(C₁₋₅-Alkyl)₂, -O-Phenyl, -O-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -C₁₋₅-Alkyl, -O-C₁₋₅-Alkyl, -O-CF₃, -S-CF₃, Phenyl und -O-Benzyl substituiert sein kann.

Ebenfalls bevorzugt weisen die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) auf.

Sofern einer oder mehrere der vorstehend genannten Reste für einen gesättigten oder ungesättigten C₁₋₁₀ aliphatischen Rest, d.h. für einen C₁₋₁₀-Alkyl-, C₂₋₁₀-Alkenyl-oder C₂₋₁₀-Alkinyl-Rest, stehen, kann dieser bevorzugt mit ggf. 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, -O(C₁₋₅-Alkyl), -S(C₁₋₅-Alkyl), - NH(C₁₋₅-Alkyl), -N(C₁₋₅-Alkyl)(C₁₋₅-Alkyl), -OCF₃ und -SCF₃ substituiert sein. C₂₋₁₀-Alkenyl-Reste weisen wenigstens eine, vorzugsweise 1, 2, 3 oder 4 C-C-Doppelbindungen und C₂₋₁₀-Alkinyl-Reste wenigstens eine, vorzugsweise 1, 2, 3 oder 4 C-C-Dreifachbindungen auf.

Bevorzugt sind Alkyl-Reste ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, neo-Pentyl, n-Hexyl und n-Heptyl, die ggf. mit 1, 2, 3, 4, 5, 6, 7, 8 oder 9 Substituenten unabhängig voneinander ausgewählt aus der Gruppe bestehend aus F, Cl, Br, I, - CN, -NO₂, -OH, -NH₂, -SH, -O-CH₃, -O-C₂H₅, -O-CH(CH₃)₂, -O-C(CH₃)₃, -S-CH₃, -S-C₂H₅, -S-CH(CH₃)₂, -S-C(CH₃)₃, -NH-CH₃, -NH-C₂H₅, -NH-C(CH₃)₃, -N(CH₃)₂, - N(C₂H₅)₂, -N(CH₃)(C₂H₅), -OCF₃ und -SCF₃ substituiert sein können.

Ebenfalls bevorzugt sind Alkenyl-Reste ausgewählt aus der Gruppe bestehend aus Vinyl, 1-Propenyl, 2-Propenyl, 1-Butenyl, 2-Butenyl, 3-Butenyl, 2-Methyl-buten-2-yl, 1-Pentenyl, 2-Pentenyl, 3-Pentenyl und 4-Pentenyl, die ggf. mit 1, 2 oder 3 Substituenten unabhängig voneinander ausgewählt aus der Gruppe bestehend aus F, Cl, Br, I, -CN, -NO₂, -OH, -NH₂, -SH, -O-CH₃, -O-C₂H₅, -O-CH(CH₃)₂, -O-C(CH₃)₃, - S-CH₃, -S-C₂H₅, -S-CH(CH₃)₂, -S-C(CH₃)₃, -NH-CH₃, -NH-C₂H₅, -NH-C(CH₃)₃, - N(CH₃)₂, -N(C₂H₅)₂, -N(CH₃)(C₂H₅), -OCF₃ und -SCF₃ substituiert sein können. Weiterhin bevorzugt sind Alkinyl-Reste ausgewählt aus der Gruppe bestehend aus Ethinyl, 1-Propinyl, 2-Propinyl, 1-Butinyl, 2-Butinyl, 3-Butinyl, 1-Pentinyl, 2-Pentinyl, 3-Pentinyl und 4-Pentinyl, die ggf. mit 1, 2 oder 3 Substituenten unabhängig voneinander ausgewählt aus der Gruppe bestehend aus F, Cl, Br, I, -CN, -NO₂, -OH, -NH₂, -SH, -O-CH₃, -O-C₂H₅, -O-CH(CH₃)₂, -O-C(CH₃)₃, -S-CH₃, -S-C₂H₅, -S-CH(CH₃)₂, -S-C(CH₃)₃, -NH-CH₃, -NH-C₂H₅, -NH-C(CH₃)₃, -N(CH₃)₂, -N(C₂H₅)₂, - N(CH₃)(C₂H₅), -OCF₃ und -SCF₃ substituiert sein können.

Besonders bevorzugte ggf. substituierte C₁₋₁₀ aliphatische Rest sind ausgewählt aus der Gruppe bestehend aus Methyl, -CF₃, -CHF₂, -CH₂F, -CF₂Cl, -CCl₂F, -CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃, -CH₂-N(CH₃)₂, -CH₂-N(C₂H₅)₂, -CH₂-N(CH₃)(C₂H₅), Ethyl, -CF₂-CH₃, -CHF-CF₂Cl, -CF₂-CFCl₂, -CFCl-CF₂Cl, -CFCl-CFCl₂, -CH₂-CH₂-NH₂, -CH₂-CH₂-OH, -CH₂-CH₂-SH, -CH₂-CH₂-NH-CH₃, -CH₂-CH₂-N(CH₃)₂, -CH₂-CH₂-N(C₂H₅)₂, -CH₂-CH₂-N(CH₃)(C₂H₅), -CH₂-CF₃, -C₂F₅, -CH₂-CCl₃, -CH₂-CBr₃, -CH₂-CH₂-CN, n-Propyl, - CH₂-CH₂-CH₂-OH, -CH₂-CH₂-CH₂-SH, -CH₂-CH₂-CH₂-NH₂, -CH₂-CH₂-CH₂-NH-CH₃, - CH₂-CH₂-CH₂-N(CH₃)₂, -CH₂-CH₂-CH₂-N(C₂H₅)₂, -CH₂-CH₂-CH₂-N(CH₃)(C₂H₅), -CH₂-CH₂-O-CH₃, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-Butyl, - CF₂-CF₂-CF₂-CF₃, -CH₂-CH₂-CH₂-CH₂-CN, sec-Butyl, Isobutyl, tert-Butyl, n-Pentyl, sec-Pentyl, neo-Pentyl, n-Hexyl, Vinyl, 1-Propenyl, 2-Propenyl, 1-Butenyl, 2-Butenyl, 3-Butenyl, 2-Methyl-buten-2-yl, (1,1,2)-Trifluor-1-butenyl, 1-Pentenyl, 2-Pentenyl, 3-Pentenyl, 4-Pentenyl, -CF=CF₂, -CCl=CCl₂, -CH₂-CF=CF₂, -CH₂-CCl=CCl₂, -C≡C-I, - C≡C-F und -C≡C-Cl.

Sofern einer oder mehrere der vorstehend genannten Substituenten für einen (hetero)cycloaliphatischen Rest stehen, der ggf. mit einem gesättigten oder ungesättigten, unsubstituierten oder wenigstens einfach substituierten mono- oder polyzyklischen Ringsystem kondensiert sein kann, kann dieser bevorzugt ausgewählt werden aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Azocanyl, Diazepanyl, Dithiolanyl, (1,3,4,5)-Tetrahydropyrido[4,3-b]indolyl, (3,4)-Dihydro-1H-isochinolinyl, (1,3,4,9)-Tetrahydro-[b]-carbolinyl und (1,3)-Thiazolidinyl.

Besonders bevorzugt können die (hetero)cycloaliphatischen Reste ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H_{5,} -O-CH(CH₃)₂, -O-C(CH₃)₃, -NH₂, -NO₂, -O-CF₃, -S-CF₃, -SH, -S-CH₃, -S-C₂H₅, - S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂. -N(CH₃)(C₂H₅), -O-Phenyl, -O-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CH(CH₃)₂, -O-C(CH₃)₃, -O-CF₃, -S-CF₃, Phenyl und -O-Benzyl substituiert sein kann.

Sofern einer oder mehrere der vorstehend genannten Substituenten für einen Aryl-Rest stehen, kann dieser bevorzugt ausgewählt werden aus der Gruppe bestehend aus Phenyl und Naphthyl (1-Naphthyl und 2-Naphthyl).

Sofern einer oder mehrere der vorstehend genannten Substituenten für einen Heteroaryl-Rest stehen, kann dieser bevorzugt ausgewählt werden aus der Gruppe bestehend aus Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzoxazolyl, Benzisoxazolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl.

Besonders bevorzugt können die Aryl- oder Heteroaryl-Reste ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), - NH-C(=O)-O-CH₃, -NH-C(=O)-O-C₂H₅, -NH-C(=O)-O-C(CH₃)₅, -C(=O)-H, -C(=O)-CH₃, -C(=O)-C₂H₅, -C(=O)-CH(CH₃)_{2,} -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein, wobei jeweils der zyklische Teil der Reste -O-Phenyl, - O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -C₁₋₅-Alkyl, -O-C₁₋₅-Alkyl, -O-CF₃, -S-CF₃, Phenyl und -O-Benzyl substituiert sein kann.

Unter einem mono- oder polyzyklischen Ringsystem werden im Sinne der vorliegenden Erfindung mono- oder polyzyklische Kohlenwasserstoffreste verstanden, die gesättigt oder ungesättigt sein und ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind.

Ein solches mono- bzw. polyzyklisches Ringsystem kann beispielsweise mit einem Aryl-Rest oder einem Heteroaryl-Rest kondensiert (anneliert) sein.

Sofern ein polyzyklisches Ringsystem wie beispielsweise ein bizyklisches Ringsystem vorliegt, können die verschiedenen Ringe, jeweils unabhängig voneinander, einen unterschiedlichen Sättigungsgrad aufweisen, d.h. gesättigt oder ungesättigt sein. Bevorzugt ist ein polyzyklisches Ringsystem ein bizyklisches Ringsystem.

Beispielhaft für Aryl-Reste, die mit einem mono- bzw. polyzyklischen Ringsystem kondensiert sind, seien (1,3)-Benzodioxolyl und (1,4)-Benzodioxanyl genannt.

Sofern einer oder mehrere der vorstehend genannten Substituenten ein mono- oder polyzyklisches Ringsystem aufweisen, kann dieses bevorzugt mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, - N(CH₃)(C₂H₅), -NH-C(=O)-O-CH₃, -NH-C(=O)-O-C₂H₅, -NH-C(=O)-O-C(CH₃)₃, - C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, - CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann.

Sofern einer oder mehrere der vorstehend genannten Substituenten eine lineare oder verzweigte C₁₋₆-Alkylen-Gruppe aufweisen, kann diese bevorzugt ausgewählt werden aus der Gruppe bestehend aus -(CH₂)-, -(CH₂)₂-, -C(H)(CH₃)-, -(CH₂)₃-, - (CH₂)₄-, -(CH₂)₅-, -C(H)(C(H)(CH₃)₂)- und -C(C₂H₅)(H)-.

In einer weiteren Ausführungsform sind substituierte Verbindungen der vorstehend angegebenen allgemeinen Formel I bevorzugt, worin
X für O, S oder N-C≡N steht;
n für 0, 1, 2, 3 oder 4 steht;
R¹, R², R³ und R⁴, unabhängig voneinander, jeweils für H; F; Cl; Br, I; -SF₅; -NO₂; -CF_{3;} -CN; -NH₂; -OH; -SH; -C(=O)-NH₂; -S(=O)₂-NH₂; -C(=O)-NH-OH; -C(=O)-OH; -C(=0)-H: -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³; -S(=O)₂-R²⁴ oder für einen Alkyl-Rest ausgewählt as der Gruppe bestehend aus Methyl, Ethyl, n-Propyl, Isopropyl, tert-Butyl, n-Butyl, sec-Butyl, Isobutyl, n-Pentyl, n-Hexyl und n-Heptyl stehen;
R⁵ für H; F; Cl; Br; I; -SF₅; -NO₂; -CF₃; -CF₂Cl; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹, -S(=O)₂-NR²⁰R²¹; C(=O)-OR²²; -C(=O)-R²³; -S(=O)₂-R²⁴;
für einen Alkyl-Rest ausgewählt aus der Gruppe bestehend aus Methyl, Ethyl, n-Propyl, Isopropyl, tert-Butyl, n-Butyl, sec-Butyl, Isobutyl, n-Pentyl, n-Hexyl und n-Heptyl;
oder für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste an das Grundgerüst gebunden ist und ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, - S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -O-Phenyl, -O-Benzyl, Phenyl und Benzyl substituiert sein kann, wobei jeweils der
zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CH(CH₃)₂, -O-C(CH₃)₃, -O-CF₃, -S-CF₃, Phenyl und -O-Benzyl substituiert sein kann, steht;
T für C-R⁶ und U für C-R⁷ und V für N und W für C-R³
oder
T für C-R⁶ und U für N und V für C-R⁹ und W für C-R⁸
oder
T für N und U für C-R⁷ und V für C-R⁹ und W für C-R⁸
oder
T für N und U für N und V für C-R⁹ und W für C-R⁸
oder
T für N und U für C-R⁷ und V für N und W für C-R⁸ oder
T für C-R⁶ und U für N und V für N und W für C-R⁸
oder
T für C-R⁶ und U für C-R⁷ und V für C-R⁹ und W für C-R¹⁰
steht;
R⁶ und R⁷, unabhängig voneinander, jeweils für H; F; Cl; Br; I; -SF₅; -NO₂; -CF₃; -CF₂Cl; -CN; -NH₂; -OH; -SH; -C(=O)-NH₂; -S(=O)₂-NH₂-, -C(=O)-OH; - C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³, -S(=O)₂-R²⁴ oder für einen Alkyl-Rest ausgewählt aus der Gruppe bestehend aus Methyl, Ethyl, n-Propyl, Isopropyl, tert-Butyl, n-Butyl, sec-Butyl, Isobutyl, n-Pentyl, n-Hexyl und n-Heptyl stehen;
R⁸ für H; F; Cl; Br; I; -SF₅; -CF₃; -CF₂Cl; -NO₂; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹-, -S(=O)₂-NR²⁰R²¹; - C(=O)-OR²²; -C(=O)-R²⁵; -S(=O)₂-R²⁴;
für einen Alkyl-Rest ausgewählt aus der Gruppe bestehend aus Methyl, Ethyl, n-Propyl, Isopropyl, tert-Butyl, n-Butyl, sec-Butyl, Isobutyl, n-Pentyl, n-Hexyl und n-Heptyl;
für einen Alkenyl-Rest ausgewählt aus der Gruppe bestehend aus Ethenyl, Propenyl, Butenyl und Pentenyl;
für einen Alkinyl-Rest ausgewählt aus der Gruppe bestehend aus Ethinyl, Propinyl, Butinyl und Pentinyl;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste an das Grundgerüst gebunden ist und ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, - S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -O-Phenyl, -O-Benzyl, Phenyl und Benzyl substituiert sein kann, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CH(CH₃)₂, -O-C(CH₃)₃, -O-CF₃, -S-CF₃, Phenyl und -O-Benzyl substituiert sein kann;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzoxazolyl, Benzisoxazolyl, Thiazolyl, Oxazolyl, Isoxazolyl, Pyridazinyl, Pyrazinyl, Pyrimidinyl, Indazolyl, Chinoxalinyl, Chinolinyl und Isochinollnyl, der jeweils über eine -(CH₂)-, -(CH₂)₂- oder -(CH₂)₃-Gruppe gebunden sein kann und/oder jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-OH, -C(=O)-O-CH₃, -C(=O)-O-C₂H₅, -C(=O)-O-CH(CH₃)₂, -C(=O)-O-C(CH₃)_{3,} -NH-CH₃, -NH-C₂H₅, -NH-C(CH₃)₃, -N(CH₃)₂, -N(C₂H₅)₂, - N(CH₃)(C₂H₅), -NH-C(-O)-O-CH₃, -NH-C(-O)-O-C₂H₅, -NH-C(=O)-O-C(CH₃)₃, - C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃^{,} -C(=O)-NH-C₂H₅, -C(=O)-N(CH₃)₂, -C(=O)-N(C₂H₅)₂, -O-Phenyl, -O-Benzyl, Phenyl und Benzyl substituiert sein kann, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, - CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann, steht;
R⁹ für H; F; Cl; Br; I; -SF₅; -NO₂; -CF₃; -CF₂Cl; -CN; -NH₂; -OH; -SH; -C(=O)-NH₂; -S(=O)₂-NH₂; -C(=O)-NH-OH: -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; - C(=O)-OR²²; -C(=O)-R²³; -S(=O)₂-R²⁴ oder für einen Alkyl-Rest ausgewählt aus der Gruppe bestehend aus Methyl, Ethyl, n-Propyl, Isopropyl, tert-Butyl, n-Butyl, sec-Butyl, Isobutyl, n-Pentyl, n-Hexyl und n-Heptyl steht;
R¹⁰ für -SF₅; -NO₂; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³; -S(=O)₂-R²⁴;
für einen Alkyl-Rest ausgewählt aus der Gruppe bestehend aus Methyl, Ethyl, n-Propyl, Isopropyl, tert-Butyl, n-Butyl, sec-Butyl, Isobutyl, n-Pentyl, n-Hexyl und n-Heptyl, der jeweils mit ggf. 1, 2, 3, 4, 5, 6, 7, 8 oder 9 Substituenten unabhängig voneinander ausgewählt aus der Gruppe bestehend aus -CN, -NO₂, -OH, -NH₂, -SH, -O-CH₃, -O-C₂H₅, -O-CH(CH₃)₂, -O-C(CH₃)₃, -S-CH₃, -S-C₂H₅, -S-CH(CH₃)₂, -S-C(CH₃)₃, -NH-CH₃, -NH-C₂H₅, -NH-C(CH₃)₃, -N(CH₃)₂, -N(C₂H₅)₂, -N(CH₃)(C₂H₅), - OCF₃ und -SCF₃ substituiert ist;
für einen Alkenyl-Rest ausgewählt aus der Gruppe bestehend aus Ethenyl, Propenyl, Butenyl und Pentenyl;
für einen Alkinyl-Rest ausgewählt aus der Gruppe bestehend aus Ethinyl, Propinyl, Butinyl und Pentinyl;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste an das Grundgerüst gebunden ist und ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, - S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅. -NH-C(CH₃)₃, -N(CH₃)₂, -N(C₂H₅)₂, -N(CH₃)(C₂H₅), -O-Phenyl, -O-Benzyl, Phenyl und Benzyl substituiert sein kann, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CH(CH₃)₂, -O-C(CH₃)₃, -O-CF₃, -S-CF₃, Phenyl und -O-Benzyl substituiert sein kann;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzoxazolyl, Benzisoxazolyl, Thiazolyl, Oxazolyl, Isoxazolyl, Pyridazinyl, Pyrazinyl, Pyrimidinyl, Indazolyl, Chinoxalinyl. Chinolinyl und Isochinolinyl, wobei der Rest jeweils über eine -(CH₂)-, -(CH₂)₂- oder -(CH₂)₃-Gruppe gebunden sein kann und/oder jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, - S-C₂H₅, -S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-OH, -C(=O)-O-CH₃, -C(=O)-O-C₂H₅, - C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, - C(=O)-NH₂, -C(=O)-NH-CH₃, -C(=O)-NH-C₂H₅, -C(=O)-N(CH₃)₂, -C(=O)-N(C₂H₅)₂, - O-Phenyl, -O-Benzyl, Phenyl und Benzyl substituiert sein kann, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -0-Benzyl substituiert sein kann, steht;
R¹¹, R¹², R¹³, R¹⁴, R¹⁵, R¹⁶, R¹⁷, R¹⁸, R¹⁹, R²⁰, R²¹, R²², R²³ und R²⁴, unabhängig voneinander, jeweils
für einen Alkyl-Rest ausgewählt aus der Gruppe bestehend aus Methyl, Ethyl, n-Propyl, Isopropyl, tert-Butyl, n-Butyl, sec-Butyl, Isobutyl, n-Pentyl, n-Hexyl und n-Heptyl;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über eine -(CH₂)-, -(CH₂)₂- oder -(CH₂)₃-Gruppe gebunden sein kann und/oder ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, - NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-CH₃, - C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, - N(CH₃)₂, -N(C₂H₅)₂, -N(CH₃)(C₂H₅), -O-Phenyl, -O-Benzyl, Phenyl und Benzyl substituiert sein kann, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, Methyl, Ethyl, n-Propyl, Isopropyl, n-Butyl, sec-Butyl, Isobutyl, tert-Butyl, - O-CH₃, -O-C₂H₅, -O-CH(CH₃)₂, -O-C(CH₃)₃, -O-CF₃, -S-CF₃, Phenyl und -O-Benzyl substituiert sein kann;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzoxazolyl, Benzisoxazolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl, wobei der Rest jeweils über eine -(CH₂)-, -(CH₂)₂- oder -(CH₂)₃-Gruppe gebunden sein kann und/oder jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, - S-C₂H₅, -S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-OH, -C(=O)-O-CH₃, -C(=O)-O-C₂H₅, - C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃. - C(=O)-NH₂, -C(=O)-NH-CH₃, -C(=O)-NH-C₂H₅, -C(=O)-N(CH₃)₂, -C(=O)-N(C₂H₅)₂, - O-Phenyl, -O-Benzyl, Phenyl und Benzyl substituiert sein kann, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann, stehen;
oder
R¹² und R¹³ jeweils zusammen mit dem sie verbindenden Stickstoffatom als Ringglied einen Rest ausgewählt aus der Gruppe bestehend aus Pyrrolidinyl, Piperidinyl, (1,3,4,5)-Tetrahydropyrido[4,3-b]indolyl, (3,4)-Dihydro-1H-isochinolinyl, (1,3,4,9)-Tetrahydro-[b]-carbolinyl, Imidazolidinyl, (1,3)-Thiazolidinyl, Piperazinyl, Morpholinyl, Azepanyl, Diazepanyl und Thiomorpholinyl bilden, der ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, - S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -O-Phenyl, -O-Benzyl, Phenyl und Benzyl substituiert sein können, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CH(CH₃)₂, -O-C(CH₃)₃, -O-CF₃, -S-CF₃, Phenyl und -O-Benzyl substituiert sein kann;
und
R²⁵ und R²⁶, unabhängig voneinander, jeweils für einen Wasserstoff-Rest;
für einen Alkyl-Rest ausgewählt aus der Gruppe bestehend aus Methyl, Ethyl, n-Propyl, Isopropyl, tert-Butyl, n-Butyl, sec-Butyl, Isobutyl, n-Pentyl, n-Hexyl und n-Heptyl;
oder für einen Rest ausgewählt aus der Gruppe bestehend aus Cyclopropyl, Cyclobutyl, Cyclopentyl, Cyclohexyl, Cyclopentenyl und Cyclohexenyl stehen;
mit der Maßgabe, dass R²⁵ und R²⁶ nicht jeweils für einen Wasserstoff-Rest stehen;
oder
R²⁵ und R²⁶ jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl, Cyclopentenyl und Cyclohexenyl bilden;
wobei
sofern nicht anders angegeben, die vorstehend genannten Alkyl-, Alkenyl- und Alkinyl-Reste jeweils ggf. mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, -O-CH₃, -O-C₂H₅, -O-CH(CH₃)₂, -O-C(CH₃)₃, -S-CH₃, -S-C₂H₅, -S-CH(CH₃)₂, -S-C(CH₃)₃, -NH-CH₃, -NH-C₂H₅, -NH-C(CH₃)₃, -N(CH₃)₂, -N(C₂H₅)₂, - N(CH₃)(C₂H₅), -OCF₃ und -SCF₃ substituiert sein können;
jeweils ggf. in Form eines ihrer reinen Stereoisomeren, insbesondere Enantiomeren oder Diastereomeren, ihrer Racemate oder in Form einer Mischung von
Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

In einer weiteren Ausführungsform sind ebenfalls bevorzugt Verbindungen der vorstehend angegebenen allgemeinen Formel I, worin
X für O, S oder N-C≡N steht;
n für 0, 1, 2, 3 oder 4 steht;
R¹, R², R³ und R⁴, unabhängig voneinander, jeweils für H; F; Cl; Br; I; -SF₅; -NO₂; -CN; -NH₂; -OH; -SH; -C(=O)-NH₂; -S(=O)₂-NH₂; -C(=O)-NH-OH; -C(=O)-OH; - C(=O)-H; -S(=O)₂-OH; -NHR¹¹; -NR¹²R¹³; -OR¹⁴; -SR¹⁵; -S(=O)₂-R²⁴ oder für einen Rest ausgewählt aus der Gruppe bestehend aus Methyl, -CF₃, -CCl₃, -CBr₃, -CHF₂, - CH₂F, -CF₂Cl, -CCl₂F, Ethyl, -CF₂-CH₃, -CH₂-CF₃, -C₂F₅, -CH₂-CCl₃, -CH₂-CBr₃, - CHF-CF₂Cl, -CF₂-CF₂Cl, -CFCl-CF₂Cl, n-Propyl, -CF₂-CF₂-CF₃, -CF(CF₃)₂, Isopropyl, sec-Butyl, Isobutyl und tert-Butyl stehen;
R⁵ für F; Cl; Br; I; -SF₅; -OR¹⁴; -SR¹⁵; -S(=O)₂-R²⁴;
für einen Rest ausgewählt aus der Gruppe bestehend aus Methyl, -CF₃, -CCl₃, -CBr₃, -CHF₂, -CH₂F, -CF₂Cl, -CCl₂F, -CH₂-CN, -CH₂-O-CH₃, -CH₂-O-CF₃, -CH₂-SF₃, Ethyl, - CF₂-CH₃, -CH₂-CF₃, -C₂F₅, -CH₂-CCl₃, -CH₂-CBr₃, -CHF-CF₂Cl, -CF₂-CF₂Cl, -CFCI-CF₂Cl, -CH₂-CH₂-CN, n-Propyl, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), - CH(CH₃)(S-CH₃), n-Butyl, -CF₂-CF₂-CF₂-CF₃, -CH₂-CH₂-CH₂-CH₂-CN, sec-Butyl, Isobutyl und tert-Butyl;
oder für einen Rest ausgewählt aus der Gruppe bestehend aus Cyclopropyl, Cyclobutyl, Cyclopentyl, Cyclohexyl, Cyclopentenyl und Cyclohexenyl, der ggf. jeweils mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl, tert-Butyl und n-Pentyl substituiert sein kann;
T für C-R⁶ und U für C-R⁷ und V für N und W für C-R⁸
oder
T für C-R⁶ und U für N und V für C-R⁹ und W für C-R⁸
oder
T für N und U für C-R⁷ und V für C-R⁹ und W für C-R⁸
oder
T für N und U für N und V für C-R⁹ und W für C-R⁸
oder
T für N und U für C-R⁷ und V für N und W für C-R⁸
oder
T für C-R⁶ und U für N und V für N und W für C-R⁸
oder
T für C-R⁶ und U für C-R⁷ und V für C-R⁹ und W für C-R¹⁰
steht;
R⁶ und R⁷, unabhängig voneinander, jeweils für H; F; Cl; Br; I; -SF₅; -NO₂; -CN; -NH₂; -OH; -SH; -C(=O)-NH₂; -S(=O)₂-NH₂; -C(=O)-NH-OH; -C(=O)-OH; - C(=O)-H; -S(=O)₂-OH; -NHR¹¹; -NR¹²R¹³; -OR¹⁴; -SR¹⁵; -S(=O)₂-R²⁴ oder für einen Rest ausgewählt aus der Gruppe bestehend aus Methyl, -CF₃, -CCl₃, -CBr₃, -CHF₂, - CH₂F, -CF₂Cl, -CCl₂F, Ethyl, -CF₂-CH₃, -CH₂-CF₃, -C₂F₅, -CH₂-CCl₃, -CH₂-CBr₃, - CHF-CF₂Cl, -CF₂-CF₂Cl, -CFCl-CF₂Cl, n-Propyl, -CF₂-CF₂-CF₃, -CF(CF₃)₂, Isopropyl, sec-Butyl, Isobutyl und tert-Butyl stehen;
R⁸ für H; F; Cl; Br; I; -SF₅; -NO₂; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-OR²²; -S(=O)₂-R²⁴
oder für einen Rest ausgewählt aus der Gruppe bestehend aus Methyl, -CF₃, -CCl₃, - CBr₃, -CHF₂, -CH₂F, -CF₂Cl, -CCl₂F, Ethyl, -CF₂-CH₃, -CH₂-CF₃, -C₂F₅, -CH₂-CCl₃, - CH₂-CBr₃, -CHF-CF₂Cl, -CF₂-CF₂Cl, -CFCl-CF₂Cl, n-Propyl, -CF₂-CF₂-CF₃, - CF(CF₃)₂, Isopropyl, sec-Butyl, Isobutyl, tert-Butyl, n-Pentyl, sec-Pentyl, neo-Pentyl, n-Hexyl, Ethenyl, Propenyl, Butenyl, Pentenyl, Ethinyl, Propinyl, Butinyl, Pentinyl, - CF=CF₂, -CCl=Cl2, -CH₂-CF=CF₂, -CH₂-CCl=CCl₂, -C=C-I, -C≡C-F und -C≡C-Cl;
für einen Rest ausgewählt aus der Gruppe bestehend aus Cyclopropyl, Cyclobutyl, Cyclopentyl, Cyclohexyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste an das Grundgerüst gebunden ist und ggf. jeweils mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, - C(=O)-O-CH₃, -C(=O)-O-C₂H₅, -C(=O)-O-CH(CH₃)₂ und -C(=O)-O-C(CH₃)₃ substituiert sein kann;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl, der jeweils über eine -(CH₂)-, -(CH₂)₂- oder -(CH₂)₃-Gruppe gebunden sein kann und/oder jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-OH, - C(=O)-O-CH₃, -C(=O)-O-C₂H₅, -C(=O)-O-CH(CH₃)₂, -C(=O)-O-C(CH₃)₃, -NH-CH₃, - NH-C₂H₅, -NH-C(CH₃)₃, -N(CH₃)₂, -N(C₂H₅)₂, -N(CH₃)(C₂H₅), -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein kann, steht;
R⁹ für H; F; Cl; Br; I; -SF₅; -NO₂; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -S(=O)₂-R²⁴ oder für einen Rest ausgewählt aus der Gruppe bestehend aus Methyl, - CF₃, -CCl₃, -CBr₃, -CHF₂, -CH₂F, -CF₂Cl, -CCl₂F, Ethyl, -CF₂-CH₃, -CH₂-CF₃, -C₂F₅, - CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, sec-Butyl, Isobutyl und tert-Butyl steht;
R¹⁰ für -SF₅; -NO₂; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; -C(=O)-OR²²; -S(=O)₂-R²⁴
oder für einen Rest ausgewählt aus der Gruppe bestehend aus Ethenyl, Propenyl, Butenyl, Pentenyl, Ethinyl, Propinyl, Butinyl, Pentinyl, -CF=CF₂, -CCl=Cl₂, -CH₂-CF=CF₂, -CH₂-CCl=CCl₂, -C≡C-I, -C=C-F und -C≡C-Cl;
für einen Rest ausgewählt aus der Gruppe bestehend aus Cyclopropyl, Cyclobutyl, Cyclopentyl, Cyclohexyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste an das Grundgerüst gebunden ist und ggf. jeweils mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, - C(=O)-O-CH₃, -C(=O)-O-C₂H₅, -C(=O)-O-CH(CH₃)₂ und -C(=O)-O-C(CH₃)₃ substituiert sein kann;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl, der jeweils über eine -(CH₂)-, -(CH₂)₂- oder -(CH₂)₃-Gruppe gebunden sein kann und/oder jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-OH, - C(=O)-O-CH3, -C(=O)-O-C₂H₅, -C(=O)-O-CH(CH₃)₂, -C(=O)-O-C(CH₃)₃, -NH-CH₃, - NH-C₂H₅, -NH-C(CH₃)₃, -N(CH₃)₂, -N(C₂H₅)₂, -N(CH₃)(C₂H₅), -NH-C(=O)-O-CH_{3,} -NH-C(=O)-O-C₂H₅, -NH-C(=O-C(CH₃)₃, -C(=O)-H, -C(=O)-CH₃, -C(=O)-C₂H₅, -C(=O)-CH(CH₃)₂, -C(=O)-C(CH₃), -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein kann, steht;
R¹¹, R¹², R¹³, R¹⁴, R¹⁵, R²² und R²⁴, unabhängig voneinander, jeweils
für einen Rest ausgewählt aus der Gruppe bestehend aus Methyl, -CF₃, -CCl₃, -CBr₃, -CHF₂, -CH₂F, -CF₂Cl, -CCl₂F, -CH₂-CN, -CH₂-O-CH₃, -CH₂-O-CF₃, -CH₂-SF₃, Ethyl, - CF₂-CH₃, -CH₂-CF₃, -C₂F₅, -CH₂-CCl₃, -CH₂-CBr₃, -CHF-CF₂Cl, -CF₂-CF₂Cl, -CFCI-CF₂Cl, -CH₂-CH₂-CN, n-Propyl, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃. -CH(CH₃)(O-CH₃), - CH(CH₃)(S-CH₃), n-Butyl, -CF₂-CF₂-CF₂-CF₃, -CH₂-CH₂-CH₂-CH₂-CN, sec-Butyl, Isobutyl und tert-Butyl stehen;
für einen Rest ausgewählt aus der Gruppe bestehend aus Cyclopropyl, Cyclobutyl, Cyclopentyl, Cyclohexyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, -C(=O)-O-C₂H₅, - C(=O)-O-CH(CH₃)₂ und -C(=O)-O-C(CH₃)₃ substituiert sein kann;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Thiazolyl, Oxazolyl und Isoxazolyl, wobei der Rest jeweils über eine - (CH₂)-, -(CH₂)₂- oder -(CH₂)₃-Gruppe gebunden sein kann und/oder jeweils ggf. mit 1, 2, 3, 4 oder 5 Substituenten ausgewählt aus der Gruppe bestehend aus F, Cl, Br, I, - CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-OH, - C(=O)-O-CH₃, -C(=O)-O-C₂H₅, -C(=O)-O-CH(CH₃)₂, -C(=O)-O-C(CH₃)₃, -NH-CH₃, - NH-C₂H₅, -NH-C(CH₃)₃, -N(CH₃)₂, -N(C₂H₅)₂, -N(CH₃)(C₂H₅), -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein kann, stehen;
oder
R¹² und R¹³ jeweils zusammen mit dem sie verbindenden Stickstoffatom als Ringglied einen Rest ausgewählt aus der Gruppe bestehend aus Pyrrolidinyl, Piperidinyl, Piperazinyl, Morpholinyl, Azepanyl, Diazepanyl und Thiomorpholinyl bilden, der ggf. jeweils mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl, tert-Butyl, n-Pentyl, -C(=O)-CH₃, -C(=O)-C₂H₅, -C(=O)-CH(CH₃)₂. -C(=O)C(CH₃)₃ und Benzyl substituiert sein kann,
und
R²⁵ und R²⁶, unabhängig voneinander, jeweils für einen Wasserstoff-Rest;
für einen Alkyl-Rest ausgewählt aus der Gruppe bestehend aus Methyl, Ethyl und n-Propyl;
oder für einen Rest ausgewählt aus der Gruppe bestehend aus Cyclopropyl, Cyclobutyl, Cyclopentyl und Cyclohexyl stehen;
mit der Maßgabe, dass R²⁵ und R²⁶ nicht jeweils für einen Wasserstoff-Rest stehen; oder
R²⁵ und R²⁶ jeweils zusammen mit dem sie verbindenden Kohlenstoffatom als Ringglied einen Rest ausgewählt aus der Gruppe bestehend aus Cyclopropyl, Cyclobutyl, Cyclopentyl und Cyclohexyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

In einer weiteren Ausführungsform sind ebenfalls bevorzugt Verbindungen der vorstehend angegebenen allgemeinen Formel I, worin
X für O, S oder N-C≡N steht;
n für 0, 1 oder 2 steht;
R¹, R³ und R⁴, unabhängig voneinander, jeweils für H; F; Cl; Br; oder für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂CF₂Cl und -CFCl-CF₂Cl stehen;
R² für F; Cl; Br; I oder für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, sec-Butyl, Isobutyl, tert-Butyl, -O-CH₃, -O-CF₃, -O-CCl₃, -O-CBr₃, -O-CHF₂, -O-CH₂F, -O-CF₂Cl, -O-CCl₂F, -O-C₂H₅, -O-CF₂-CH₃, -O-CH₂-CF₃, -O-C₂F₅, -O-CH₂-CCl₃, -O-CH₂-CBr₃, -O-CHF-CF₂Cl, -O-CF₂-CF₂Cl, -O-CFCl-CF₂Cl, -O-CH₂-CH₂-CH₃, -O-CF₂-CF₂-CF₃, -O-CF(CF₃)₂, -O-CH(CH₃)₂, -O-C(CH₃)₃, -S-CH₃, -S-CF₃, -S-CCl₃, -S-CBr₃, -S-CHF₂, -S-CH₂F, -S-CF₂Cl, -S-CCl₂F, - S-C₂H₅, -S-CF₂-CH₃, -S-CH₂-CF₃, -S-C₂F₅, -S-CH₂-CCl₃, -S-CH₂-CBr₃, -S-CHF-CF₂Cl, -S-CF₂-CF₂Cl, -S-CFCl-CF₂Cl, -S-CH₂-CH₂-CH₃, -S-CF₂CF₂-CF₃, -S-CF(CF₃)₂, -S-CH(CH₃)₂ und -S-C(CH₃)₃ steht;
R⁵ für F; Cl; Br; I; -SF₅;
für einen Rest ausgewählt aus der Gruppe bestehend aus -CF₃, -CCl₃, -CBr₃, -CHF₂, -CH₂F, -CF₂Cl, -CCl₂F, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, -CF₂-CF₂-CF₃, -CF(CF₃)₂, sec-Butyl, Isobutyl, tert-Butyl, -O-CF₃, -O-CCl₃, -O-CBr₃, -O-CHF₂, -O-CH₂F, -O-CF₂Cl, -O-CCl₂F, -O-CF₂-CH₃, -O-CH₂-CF₃, -O-C₂F₅, -O-CH₂-CCl₃, -O-CH₂-CBr₃, -O-CHF-CF₂Cl, -O-CF₂-CF₂Cl, -O-CFCl-CF₂Cl, -O-CF₂-CF₂-CF₃, -O-CF(CF₃)₂, -O-CH(CH₃)₂, -O-C(CH₃)₃, -S-CF₃, -S-CCl₃, -S-CBr₃, -S-CHF₂, -S-CH₂F, -S-CF₂Cl, -S-CCl₂F, -S-CF₂-CH₃, -S-CH₂-CF₃, -S-C₂F₅, -S-CH₂-CCl₃, -S-CH₂-CBr₃, -S-CHF-CF₂Cl, -S-CF₂-CF₂Cl, -S-CFCI-CF₂Cl, -S-CF₂-CF₂-CF₃, -S-CF(CF₃)₂, -S-CH(CH₃)₂, -S-C(CH₃)₃, -S(=O)₂-CF₃, - S(=O)₂-CCl₃, -S(=O)₂-CBr₃, -S(=O)₂-CHF₂, -S(=O)₂-CH₂F, -S(=O)₂-CF₂Cl, -S(=O)₂-CCl₂F, -S(=O)₂-CF₂-CH₃, -S(=O)₂-CH₂-CF₃, -S(=O)₂-C₂F₅, -S(=O)₂-CH₂-CCl₃, - S(=O)₂-CH₂-CBr₃, -S(=O)₂-CHF-CF₂Cl, -S(=O)₂-CF₂-CF₂Cl, -S(=O)₂-CFCl-CF₂Cl, - S(=O)₂-CF₂-CF₂-CF₃, -S(=O)₂-CF(CF₃)₂, -S(=O)₂-CH(CH₃)₂ und -S(=O)₂-C(CH₃)₃;
oder für einen Rest ausgewählt aus der Gruppe bestehend aus Cyclopropyl, Cyclobutyl, Cyclopentyl, Cyclohexyl, Cyclopentenyl und Cyclohexenyl steht;
T für C-R⁶ und U für C-R⁷ und V für N und W für C-R⁸
oder
T für C-R⁶ und U für N und V für C-R⁹ und W für C-R⁸
oder
T für N und U für C-R⁷und V für C-R⁹ und W für C-R⁸
oder
T für N und U für N und V für C-R⁹ und W für C-R⁸
oder
T für N und U für C-R⁷ und V für N und W für C-R⁸
oder
T für C-R⁶ und U für N und V für N und W für C-R⁸ oder
T für C-R⁶ und U für C-R⁷ und V für C-R⁹ und W für C-R¹⁰
steht;
R⁶ und R⁷, unabhängig voneinander, jeweils für H; F; Cl; Br; I; -NO₂; -CN; oder für einen Rest ausgewählt aus der Gruppe bestehend aus Methyl, -CF₃, -CCl₃, -CBr₃, - CHF₂, -CH₂F, -CF₂Cl, -CCl₂F, Ethyl, n-Propyl, Isopropyl, sec-Butyl, Isobutyl und tert-Butyl stehen;
R⁸ für F; Cl; Br; I; -OH; -CN; -NH₂; -NO₂; -NHR¹¹; -NR¹²R¹³; -OR ¹⁴; -SR¹⁵; -C(=O)-OR²²;
oder für einen Rest ausgewählt aus der Gruppe bestehend aus Methyl, -CF₃, -CCl₃, - CBr₃, -CHF₂, -CH₂F, -CF₂Cl, -CCl₂F, Ethyl, -CF₂-CH₃, -CH₂-CF₃, -C₂F₅, -CH₂-CCl₃, - CH₂-CBr₃, -CHF-CF₂Cl, -CF₂-CF₂Cl, -CFCl-CF₂Cl, n-Propyl, -CF₂-CF₂-CF₃, - CF(CF₃)₂, Isopropyl, sec-Butyl, Isobutyl und tert-Butyl;
für einen Rest ausgewählt aus der Gruppe bestehend aus Cyclopropyl, Cyclobutyl, Cyclopentyl, Cyclohexyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste an das Grundgerüst gebunden ist;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, lsopropyl, n-Butyl, sec-Butyl, Isobutyl und tert-Butyl substituiert sein kann, steht;
R⁹ für H; F; Cl; Br; I; -NO₂; -CN; oder für einen Rest ausgewählt aus der Gruppe bestehend aus Methyl, -CF₃, -CCl₃, -CBr₃, -CHF₂, -CH₂F, -CF₂Cl, -CCl₂F, Ethyl n-Propyl, -CF₂-CF₂-CF₃, -CF(CF₃)₂, Isopropyl, sec-Butyl, Isobutyl und tert-Butyl steht; R¹⁰ für -CN; -OH; -NH₂; -NO₂; -NHR¹¹; -NR¹²R¹³; -OR ¹⁴; -SR¹⁵; -C(=O)-OR²²;
für einen Rest ausgewählt aus der Gruppe bestehend aus Cyclopropyl,
Cyclobutyl, Cyclopentyl, Cyclohexyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste an das Grundgerüst gebunden ist;
für einen Rest ausgewählt aus der Gruppe bestehend aus Cyclopropyl,
Cyclobutyl, Cyclopentyl, Cyclohexyl, Pyrrolidinyl, Piperidinyl, Morpholinyl, Piperazinyl, Azepanyl, Diazepanyl, Azocanyl und Thiomorpholinyl steht;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der
jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl und tert-Butyl substituiert sein kann, steht;
R¹¹, R¹² , R¹³ , R¹⁴, R¹⁵ und R²², unabhängig voneinander, jeweils
für einen Rest ausgewählt aus der Gruppe bestehend aus Methyl, Ethyl, n-Propyl, Isopropyl, n-Butyl, sec-Butyl, Isobutyl und tert-Butyl stehen;
für einen Rest ausgewählt aus der Gruppe bestehend aus Cyclopropyl, Cyclobutyl, Cyclopentyl, Cyclohexyl, Pyrrolidinyl, Piperidinyl, Morpholinyl, Piperazinyl, Azepanyl, Diazepanyl, Azocanyl und Thiomorpholinyl stehen;
oder für einen Rest ausgewählt aus der Gruppe bestehend aus Phenyl, Naphthyl, Thiophenyl, Furanyl, Pyrrolyl und Pyridinyl, wobei der Rest jeweils ggf. mit 1, 2, 3, 4 oder 5 Substituenten ausgewählt aus der Gruppe bestehend aus F, Cl, Br, I, -CN, - CF₃, -SF₅, -OH, -O-CH₃, -O-C₂H₅, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl und tert-Butyl substituiert sein kann, stehen;
oder
R¹² und R¹³ jeweils zusammen mit dem sie verbindenden Stickstoffatom als Ringglied einen Rest ausgewählt aus der Gruppe bestehend aus Pyrrolidinyl, Piperidinyl, Piperazinyl, Morpholinyl, Azepanyl, Diazepanyl und Thiomorpholinyl bilden, der ggf. jeweils mit 1, 2, 3, 4 oder 5 Substituenten unabhängig voneinander ausgewählt aus der Gruppe bestehend aus Oxo (=O), Thioxo (=S), Methyl, Ethyl, n-Propyl, Isopropyl, n-Butyl, sec-Butyl, Isobutyl, tert-Butyl, n-Pentyl, -C(=O)-CH₃, - C(=O)-C₂H₅, -C(=O)-CH(CH₃)₂, -C(=O)-C(CH₃)₃ und Benzyl substituiert sein kann,
und
R²⁵ und R²⁶, unabhängig voneinander, jeweils für einen Wasserstoff-Rest
oder für einen Alkyl-Rest ausgewählt aus der Gruppe bestehend aus Methyl, Ethyl und n-Propyl stehen;
mit der Maßgabe, dass R²⁵ und R²⁶ nicht jeweils für einen Wasserstoff-Rest stehen;
oder
R²⁵ und R²⁶ jeweils zusammen mit dem sie verbindenden Kohlenstoffatom als Ringglied einen Rest ausgewählt aus der Gruppe bestehend aus Cyclopropyl, Cyclobutyl, Cyclopentyl und Cyclohexyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

In einer weiteren Ausführungsform sind besonders bevorzugt Verbindungen der vorstehend angegebenen allgemeinen Formel I, worin
X für O oder S steht;
n für 0, 1 oder 2 steht;
R¹, R³ und R⁴ jeweils für H stehen;
R² für F; Cl; Br, I oder für einen Rest ausgewählt aus der Gruppe bestehend aus Methyl, -CF₃, -CCl₃, -CBr₃, -CHF₂, -CH₂F, -O-CH₃, -O-CF₃, -O-CCl₃, -O-CBr₃, -O-CHF₂, -O-CH₂F, -S-CH₃, -S-CF₃, -S-CCl₃, -S-CBr₃, -S-CHF₂, -S-CH₂F, -S-CF₂Cl und - S-CCl₂F steht;
R⁵ für F; Cl; Br; I; -SF₅;
für einen Rest ausgewählt aus der Gruppe bestehend aus -CF₃, -CCl₃, -CBr₃, -CHF₂, -CH₂F, -CF₂Cl, -CCl₂F, tert-Butyl, -O-CF₃, -O-CCl₃, -O-CBr₃, -O-CHF₂, -O-CH₂F, -O-CF₂Cl, -O-CCl₂F, -O-CF₂-CH₃, -S-CF₃, -S-CCl₃, -S-CBr₃, -S-CHF₂, -S-CH₂F, -S-CF₂Cl, -S-CCl₂F, -S-CF₂-CH₃, -S(=O)₂-CF₃. -S(=O)₂-CCl₃, -S(=O)₂-CBr₃, -S(=O)₂-CHF₂, -S(=O)₂-CH₂F und -S(=O)₂-CF₂Cl,
oder für einen Rest ausgewählt aus der Gruppe bestehend aus Cyclopropyl, Cyclobutyl, Cyclopentyl, Cyclohexyl, Cyclopentenyl und Cyclohexenyl steht;
T für C-R⁶ und U für C-R⁷ und V für N und W für C-R⁸
oder
T für C-R⁶ und U für N und V für C-R⁹ und W für C-R⁸
oder
T für N und U für C-R⁷ und V für C-R⁹ und W für C-R⁸
oder
T für N und U für N und V für C-R⁹ und W für C-R⁸
oder
T für N und U für C-R⁷ und V für N und W für C-R⁸
oder
T für C-R⁶ und U für N und V für N und W für C-R⁸ oder
T für C-R⁶ und U für C-R⁷ und V für C-R⁹ und W für C-R¹⁰
steht;
R⁶ und R⁷ jeweils für H; F; Cl; Br und I stehen;
R⁸ für F; Cl; Br; I; -OH; -CN; -NH₂, -NO₂; -NHR¹¹; -NR¹²R¹³; -OR¹⁴ ; -SR¹⁵; -C(=O)-OR²²;
oder für einen Rest ausgewählt aus der Gruppe bestehend aus Methyl, -CF₃, -CCl₃, - CBr₃, -CHF₂, -CH₂F, -CF₂Cl, -CCl₂F, Ethyl, -CF₂-CH₃, -CH₂-CF₃, -C₂F₅, -CH₂-CCl₃, - CH₂-CBr₃, -CHF-CF₂Cl, -CF₂-CF₂Cl, -CFCl-CF₂Cl, n-Propyl, -CF₂-CF₂-CF₃, - CF(CF₃)₂, Isopropyl, sec-Butyl, Isobutyl und tert-Butyl;
für einen Rest ausgewählt aus der Gruppe bestehend aus Cyclopropyl, Cyclobutyl, Cyclopentyl, Cyclohexyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste an das Grundgerüst gebunden ist;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl und tert-Butyl substituiert sein kann, steht;
R⁹ für H; F; Cl; Br oder I steht;
R¹⁰ für -CN; -OH; -NH₂; -NO₂; -NHR¹¹; -NR¹²R¹³; -OR¹⁴; -SR¹⁵; -C(=O)-OR²²;
für einen Rest ausgewählt aus der Gruppe bestehend aus Cyclopropyl, Cyclobutyl, Cyclopentyl, Cyclohexyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste an das Grundgerüst gebunden ist;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl und tert-Butyl substituiert sein kann, steht;
R¹¹, R¹², R¹³, R¹⁴, R¹⁵ und R²², unabhängig voneinander, jeweils
für einen Rest ausgewählt aus der Gruppe bestehend aus Methyl, Ethyl, n-Propyl, Isopropyl, n-Butyl, sec-Butyl, Isobutyl und tert-Butyl stehen;
für einen Rest ausgewählt aus der Gruppe bestehend aus Cyclopropyl, Cyclobutyl, Cyclopentyl und Cyclohexyl stehen;
oder für einen Rest ausgewählt aus der Gruppe bestehend aus Phenyl, Naphthyl, Thiophenyl, Furanyl, Pyrrolyl und Pyridinyl, wobei der Rest jeweils ggf. mit 1, 2, 3, 4 oder 5 Substituenten ausgewählt aus der Gruppe bestehend aus F, Cl, Br, -O-CH₃, - O-C₂H₅, Methyl, Ethyl, n-Propyl, Isopropyl, n-Butyl, sec-Butyl, Isobutyl und tert-Butyl substituiert sein kann, stehen;
oder
R¹² und R¹³ jeweils zusammen mit dem sie verbindenden Stickstoffatom als Ringglied einen Rest ausgewählt aus der Gruppe bestehend aus Pyrrolidinyl, Piperidinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, n-Pentyl und Benzyl substituiert sein kann;
und
R²⁵ und R²⁶, unabhängig voneinander, jeweils für einen Wasserstoff-Rest oder für einen Alkyl-Rest ausgewählt aus der Gruppe bestehend aus Methyl, Ethyl und n-Propyl stehen;
mit der Maßgabe, dass R²⁵ und R²⁶ nicht jeweils für einen Wasserstoff-Rest stehen;
oder
R²⁵ und R²⁶ jeweils zusammen mit dem sie verbindenden Kohlenstoffatom als Ringglied einen Rest ausgewählt aus der Gruppe bestehend aus Cyclopropyl, Cyclobutyl, Cyclopentyl und Cyclohexyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

In einer weiteren Ausführungsform sind ebenfalls besonders bevorzugt Verbindungen der vorstehend angegebenen allgemeinen Formel I, worin
X für O steht;
n für 1 steht;
R¹, R³ und R⁴ jeweils für H stehen;
R² für F; Cl; Br oder I steht;
R⁵ für einen Rest ausgewählt aus der Gruppe bestehend aus -CF₃, -CCl₃, -CBr₃, - CHF₂, -CH₂F, -CF₂Cl, -CCl₂F, tert-Butyl, -O-CF₃, -O-CCl₃, -O-CBr₃, -O-CHF₂, -O-CH₂F, -S-CF₃, -S-CCl₃, -S-CBr₃, -S-CHF₂ und -S-CH₂F steht;
T für CH und U für CH und V für N und W für C-R⁸
oder
T für CH und U für N und V für CH und W für C-R⁸
oder
T für N und U für CH und V für CH und W für C-R⁸
oder
T für N und U für N und V für CH und W für C-R⁸
oder
T für N und U für CH und V für N und W für C-R⁸
oder
T für CH und U für N und V für N und W für C-R³
oder
T für CH und U für CH und V für CH und W für G-R¹⁰
steht;
R⁸ für F; Cl; Br; I; -CN; -OH; -NH₂; -NO₂; -NHR¹¹; -NR¹²R¹³; -OR¹⁴; -SR¹⁵;
oder für einen Rest ausgewählt aus der Gruppe bestehend aus Methyl, -CF₃, -CCl₃, - CBr₃, -CHF₂, -CH₂F, -CF₂Cl, -CCl₂F, Ethyl, -CF₂-CH₃, -CH₂-CF₃, -C₂F₅, -CH₂-CCl₃, - CH₂-CBr₃, -CHF-CF₂Cl, -CF₂-CF₂Cl, -CFCl-CF₂Cl, n-Propyl, -CF₂-CF₂-CF₃, - CF(CF₃)₂, Isopropyl, sec-Butyl, Isobutyl und tert-Butyl;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl und tert-Butyl substituiert sein kann, steht;
R¹⁰ für -CN; -NH₂; -NO₂; -NHR¹¹; -NR¹²R¹³; -OR¹⁴; -SR¹⁵;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der
jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl und tert-Butyl substituiert sein kann, steht;
R¹¹ R¹², R¹³, R¹⁴ und R¹⁵, unabhängig voneinander, jeweils
für einen Rest ausgewählt aus der Gruppe bestehend aus Methyl, Ethyl, n-Propyl, Isopropyl, n-Butyl, sec-Butyl, Isobutyl und tert-Butyl stehen;
für einen Rest ausgewählt aus der Gruppe bestehend aus Cyclopropyl, Cyclobutyl, Cyclopentyl und Cyclohexyl stehen;
oder
R¹² und R¹³ jeweils zusammen mit dem sie verbindenden Stickstoffatom als Ringglied einen Rest ausgewählt aus der Gruppe bestehend aus Pyrrolidinyl, Piperidinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus Methyl, Ethyl und Benzyl substituiert sein kann;;
und
R²⁵ für einen Alkyl-Rest ausgewählt aus der Gruppe bestehend aus Methyl, Ethyl und n-Propyl steht;
R²⁶ für einen Wasserstoff-Rest steht;
oder
R²⁵ und R²⁶ jeweils zusammen mit dem sie verbindenden Kohlenstoffatom als
Ringglied einen Rest ausgewählt aus der Gruppe bestehend aus Cyclopropyl, Cyclobutyl, Cyclopentyl und Cyclohexyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ein weiterer Gegenstand der Erfindung sind Verbindungen der allgemeinen Formel Ia, 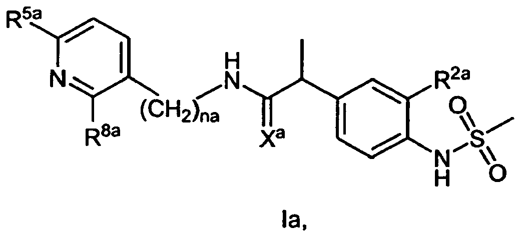 worin
X^{a} für O oder S steht;
na für 0, 1 oder 2 steht;
R^{2a} für F; Cl; Br; I oder für einen Rest ausgewählt aus der Gruppe bestehend aus Methyl, -CF₃, -CCl₃, -CBr₃, -CHF₂, -CH₂F, -O-CH₃, -O-CF₃, -O-CCl₃, -O-CBr₃, -O-CHF₂, -O-CH₂F, -S-CH₃, -S-CF₃, -S-CCl₃, -S-CBr₃, -S-CHF₂, -S-CH₂F, -S-CF₂Cl und - S-CCl₂F steht;
R^{5a} für F; Cl; Br; I; -SF₅;
für einen Rest ausgewählt aus der Gruppe bestehend aus -CF₃, -CCl₃, -CBr₃, -CHF₂, -CH₂F, -CF₂Cl, -CCl₂F, tert-Butyl, -O-CF₃, -O-CCl₃, -O-CBr₃, -O-CHF₂, -O-CH₂F, -O-CF₂Cl, -O-CCl₂F, -O-CF₂-CH₃, -S-CF₃, -S-CCl₃, -S-CBr₃, -S-CHF₂, -S-CH₂F, -S-CF₂Cl, -S-CCl₂F, -S-CF₂-CH₃, -S(=O)₂-CF₃, -S(=O)₂-CCl₃, -S(=O)₂-CBr₃, -S(=O)₂-CHF₂, -S(=O)₂-CH₂F und -S(=O)₂-CF₂Cl;
oder für einen Rest ausgewählt aus der Gruppe bestehend aus Cyclopropyl, Cyclobutyl, Cyclopentyl, Cyclohexyl, Cyclopentenyl und Cyclohexenyl steht;
R^{8a} für F; Cl; Br; I; -OH; -CN; -NH₂; -NO₂; -NHR^{11a}; -NR^{12a}R^{13a}; -OR^{14a}; -SR^{15a}; - C(=O)-OR^{22a};
oder für einen Rest ausgewählt aus der Gruppe bestehend aus Methyl, -CF₃, -CCl₃, - CBr₃, -CHF₂, -CH₂F, -CF₂Cl, -CCl₂F, Ethyl, -CF₂-CH₃, -CH₂-CF₃, -C₂F₅, -CH₂-CCl₃, - CH₂-CBr₃, -CHF-CF₂Cl, -CF₂-CF₂Cl, -CFCl-CF₂Cl, n-Propyl, -CF₂-CF₂-CF₃, - CF(CF₃)₂, Isopropyl, sec-Butyl, Isobutyl und tert-Butyl;
für einen Rest ausgewählt aus der Gruppe bestehend aus Cyclopropyl, Cyclobutyl, Cyclopentyl, Cyclohexyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste an das Grundgerüst gebunden ist;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl und tert-Butyl substituiert sein kann, steht;
R^{11a}, R^{12a}, R^{13a}, R^{14a}, R^{15a} und R^{22a}, unabhängig voneinander, jeweils
für einen Rest ausgewählt aus der Gruppe bestehend aus Methyl, Ethyl, n-Propyl, Isopropyl, n-Butyl, sec-Butyl, Isobutyl und tert-Butyl stehen;
für einen Rest ausgewählt aus der Gruppe bestehend aus Cyclopropyl,
Cyclobutyl, Cyclopentyl und Cyclohexyl stehen;
oder für einen Rest ausgewählt aus der Gruppe bestehend aus Phenyl, Naphthyl, Thiophenyl, Furanyl, Pyrrolyl und Pyridinyl, wobei der Rest jeweils ggf. mit 1, 2, 3, 4 oder 5 Substituenten ausgewählt aus der Gruppe bestehend aus F, Cl, Br, -O-CH₃, - O-C₂H₅, Methyl, Ethyl, n-Propyl, Isopropyl, n-Butyl, sec-Butyl, Isobutyl und tert-Butyl substituiert sein kann, stehen;
oder
R^{12a} und R^{13a} jeweils zusammen mit dem sie verbindenden Stickstoffatom als Ringglied einen Rest ausgewählt aus der Gruppe bestehend aus Pyrrolidinyl, Piperidinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, n-Pentyl und Benzyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ein weiterer Gegenstand der Erfindung sind Verbindungen der allgemeinen Formel Ib, 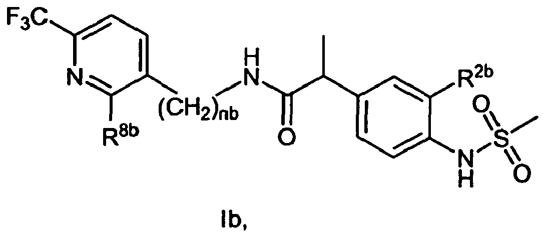 worin
nb für 0, 1 oder 2 steht;
R^{2b} für F; Cl; Br oder I steht;
R^{8b} für F; Cl; Br; I; -CN; -OH; -NH₂; -NO₂; -NHR^{11b}; -NR^{12b}R^{13b}; -OR^{14b}; SR^{15b};
für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, sec-Butyl, Isobutyl und tert-Butyl;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl und tert-Butyl substituiert sein kann, steht;
R^{11b}, R^{12b}, R^{13b}, R^{14b} und R^{15b}, unabhängig voneinander, jeweils
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Rest ausgewählt aus der Gruppe bestehend aus Cyclopropyl, Cyclobutyl, Cyclopentyl und Cyclohexyl stehen;
oder
R^{12b} und R^{13b} jeweils zusammen mit dem sie verbindenden Stickstoffatom als Ringglied einen Rest ausgewählt aus der Gruppe bestehend aus Pyrrolidinyl, Piperidinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, n-Pentyl und Benzyl substituiert sein kann; jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

In einer weiteren Ausführungsform sind Verbindungen der allgemeinen Formel lb ganz besonders bevorzugt,
worin
nb für 1 steht;
R^{2b} für F steht;
R^{8b} für F; Cl; Br; I; -CN; -OH; -NH₂; -NO₂; -NHR^{11b}; -NR^{12b}R^{13b}; -OR^{14b}; -SR^{15b};
für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, sec-Butyl, Isobutyl und tert-Butyl;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl und tert-Butyl substituiert sein kann, steht;
R^{11b}, R^{12b}, R^{13b}, R^{14b} und R^{15b}, unabhängig voneinander, jeweils für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Rest ausgewählt aus der Gruppe bestehend aus Cyclopropyl, Cyclobutyl, Cyclopentyl und Cyclohexyl stehen;
oder
R^{12b} und R^{13b} jeweils zusammen mit dem sie verbindenden Stickstoffatom als Ringglied einen Rest ausgewählt aus der Gruppe bestehend aus Pyrrolidinyl, Piperidinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus Methyl, Ethyl und Benzyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ein weiterer Gegenstand der Erfindung sind Verbindungen der allgemeinen Formel Ic, 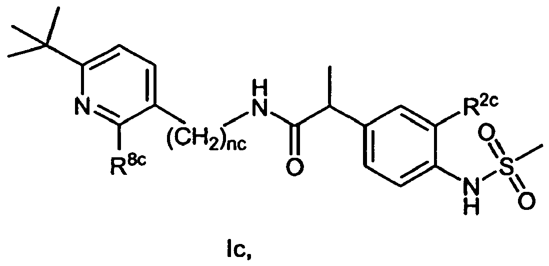 worin
nc für 0, 1 oder 2 steht;
R^{2c} für F; Cl; Br oder I steht;
R^{8c} für F; Cl; Br; I; -CN; -OH; -NH₂; -NO₂; -NHR^{11c}; -NR^{12c}R^{13c}; -OR^{14c}; -SR^{15c};
für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, sec-Butyl, Isobutyl und tert-Butyl;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl und tert-Butyl substituiert sein kann, steht;
R^{11c}, R^{12c}, R^{13c}, R^{14c} und R^{15c}, unabhängig voneinander, jeweils
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Rest ausgewählt aus der Gruppe bestehend aus Cyclopropyl, Cyclobutyl, Cyclopentyl und Cyclohexyl stehen;
oder
R^{12c} und R^{13c} jeweils zusammen mit dem sie verbindenden Stickstoffatom als Ringglied einen Rest ausgewählt aus der Gruppe bestehend aus Pyrrolidinyl, Piperidinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, n-Pentyl und Benzyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

In einer weiteren Ausführungsform sind ganz besonders bevorzugt Verbindungen der allgemeinen Formel Ic,
worin
nc für 1 steht;
R^{2c} für F steht;
R^{8c} für F; Cl; Br, I; -CN; -OH; -NH₂; -NO₂-; -NHR^{11c}; -NR^{12c}R^{13c}; -OR^{14c}; -SR^{15c}; für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, sec-Butyl, Isobutyl und tert-Butyl;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl und tert-Butyl substituiert sein kann, steht;
R^{11c}, R^{12c}, R^{13c}, R^{14c} und R^{15c}, unabhängig voneinander, jeweils für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Rest ausgewählt aus der Gruppe bestehend aus Cyclopropyl, Cyclobutyl, Cyclopentyl und Cyclohexyl stehen;
oder
R^{12c} und R^{13c} jeweils zusammen mit dem sie verbindenden Stickstoffatom als Ringglied einen Rest ausgewählt aus der Gruppe bestehend aus Pyrrolidinyl, Piperidinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus Methyl, Ethyl und Benzyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ein weiterer Gegenstand der Erfindungs sind Verbindungen der allgemeinen Formel Id, 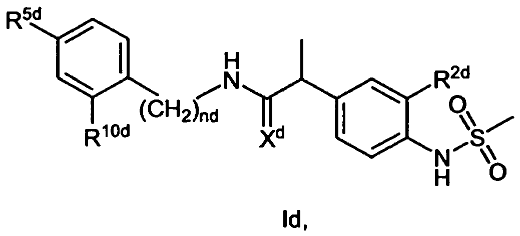 worin
X^{d} für O oder S steht;
nd für 0, 1 oder 2 steht;
R^{2d} für F; Cl; Br; I oder für einen Rest ausgewählt aus der Gruppe bestehend aus Methyl, -CF₃, -CCl₃, -CBr₃, -CHF₂, -CH₂F, -O-CH₃, -O-CF₃, -O-CCl₃, -O-CBr₃, -O-CHF₂, -O-CH₂F, -S-CH₃, -S-CF₃, -S-CCl₃, -S-CBr₃, -S-CHF₂, -S-CH₂F, -S-CF₂Cl und - S-CCl₂F steht;
R^{5d} für F; Cl; Br; I; -SF₅;
für einen Rest ausgewählt aus der Gruppe bestehend aus -CF₃, -CCl₃, -CBr₃, -CHF₂, -CH₂F, -CF₂Cl, -CCl₂F, tert-Butyl, -O-CF₃, -O-CCl₃, -O-CBr₃, -O-CHF₂, -O-CH₂F, -O-CF₂Cl, -O-CCl₂F, -O-CF₂-CH₃, -S-CF₃, -S-CCl₃, -S-CBr₃, -S-CHF₂, -S-CH₂F, -S-CF₂Cl, -S-CCl₂F, -S-CF₂-CH₃, -S(=O)₂-CF₃, -S(=O)₂-CCl₃, -S(=O)₂-CBr₃, -S(=O)₂-CHF₂, -S(=O)₂-CH₂F und -S(=O)₂-CF₂Cl;
oder für einen Rest ausgewählt aus der Gruppe bestehend aus Cyclopropyl, Cyclobutyl, Cyclopentyl, Cyclohexyl, Cyclopentenyl und Cyclohexenyl steht;
R^{10d} für -CN; -OH; -NH₂; -NO₂; -NHR^{11d}; -NR^{12d}R^{13d}; -OR^{14d}; -SR^{15d}; -C(=O)-OR^{22d}; für einen Rest ausgewählt aus der Gruppe bestehend aus Cyclopropyl, Cyclobutyl, Cyclopentyl, Cyclohexyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste an das Grundgerüst gebunden ist;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl und tert-Butyl substituiert sein kann, steht;
R^{11d}, R^{12d}, R^{13d}, R^{14d}, R^{15d} und R^{22d}, unabhängig voneinander, jeweils
für einen Rest ausgewählt aus der Gruppe bestehend aus Methyl, Ethyl, n-Propyl, Isopropyl, n-Butyl, sec-Butyl, Isobutyl und tert-Butyl stehen;
für einen Rest ausgewählt aus der Gruppe bestehend aus Cyclopropyl, Cyclobutyl, Cyclopentyl und Cyclohexyl stehen;
oder für einen Rest ausgewählt aus der Gruppe bestehend aus Phenyl, Naphthyl, Thiophenyl, Furanyl, Pyrrolyl und Pyridinyl, wobei der Rest jeweils ggf. mit 1, 2, 3, 4 oder 5 Substituenten ausgewählt aus der Gruppe bestehend aus F, Cl, Br, -O-CH₃, - O-C₂H₅, Methyl, Ethyl, n-Propyl, Isopropyl, n-Butyl, sec-Butyl, Isobutyl und tert-Butyl substituiert sein kann, stehen;
oder
R^{12d} und R^{13d} jeweils zusammen mit dem sie verbindenden Stickstoffatom als Ringglied einen Rest ausgewählt aus der Gruppe bestehend aus Pyrrolidinyl, Piperidinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, n-Pentyl und Benzyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

In einer weiteren Ausführungsform sind ganz besonders bevorzugt Verbindungen der allgemeinen Formel Ie, 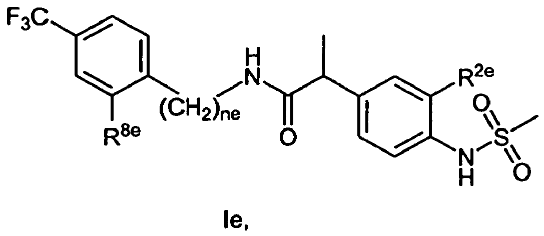 worin
ne für 0, 1 oder 2 steht;
R^{2e} für F; Cl; Br oder I steht;
R^{8e} für F; Cl; Br; I; -CN; -OH; -NH₂; -NO₂; -NHR^{11e}; -NR^{12e}R^{13e}; -OR^{14e}; -SR^{15e};
für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, sec-Butyl, Isobutyl und tert-Butyl;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl und tert-Butyl substituiert sein kann, steht;
R^{11e}, R^{12e}, R^{13e}, R^{14e} und R^{15e}, unabhängig voneinander, jeweils
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Rest ausgewählt aus der Gruppe bestehend aus Cyclopropyl, Cyclobutyl, Cyclopentyl und Cyclohexyl stehen;
oder
R^{12e} und R^{13e} jeweils zusammen mit dem sie verbindenden Stickstoffatom als Ringglied einen Rest ausgewählt aus der Gruppe bestehend aus Pyrrolidinyl, Piperidinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, n-Pentyl und Benzyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

In einer weiteren Ausführungsform sind ganz besonders bevorzugt Verbindungen der allgemeinen Formel Ie,
worin
ne für 1 steht;
R^{2e} für F steht;
R^{8e} für F; Cl; Br; I; -CN; -OH; -NH₂; -NO₂; -NHR^{11e}; -NR^{12e}R^{13e}; -OR^{14e}; -SR^{15e};
für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, sec-Butyl, Isobutyl und tert-Butyl;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl und tert-Butyl substituiert sein kann, steht;
R^{11e}, R^{12e}, R^{13e}, R^{14e} und R^{15e}, unabhängig voneinander, jeweils
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Rest ausgewählt aus der Gruppe bestehend aus Cyclopropyl, Cyclobutyl, Cyclopentyl und Cyclohexyl stehen;
oder
R^{12e} und R^{13e} jeweils zusammen mit dem sie verbindenden Stickstoffatom als Ringglied einen Rest ausgewählt aus der Gruppe bestehend aus Pyrrolidinyl, Piperidinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus Methyl, Ethyl und Benzyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ein weiterer Gegenstand der Erfindung sind Verbindungen der allgemeinen Formel If, 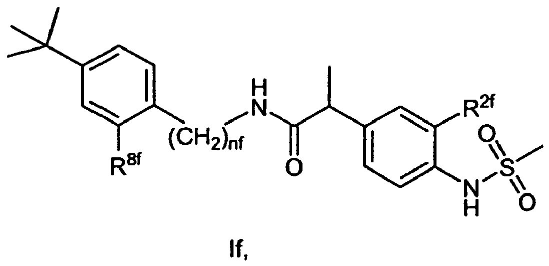 worin
nf für 0, 1 oder 2 steht;
R^{2f} für F; Cl; Br oder I steht;
R^{8f} für F; Cl; Br; I; -CN; -OH; -NH₂; -NO₂; -NHR^{11f}; -NR^{12f}R^{13f}; -OR^{14f}; -SR^{15f};
für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl_{3,} -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, sec-Butyl, Isobutyl und tert-Butyl;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl und tert-Butyl substituiert sein kann, steht;
R^{11f}, R^{12f}, R^{13f}, R^{14f} und R^{15f}, unabhängig voneinander, jeweils
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Rest ausgewählt aus der Gruppe bestehend aus Cyclopropyl, Cyclobutyl, Cyclopentyl und Cyclohexyl stehen;
oder
R^{12f} und R^{13f} jeweils zusammen mit dem sie verbindenden Stickstoffatom als Ringglied einen Rest ausgewählt aus der Gruppe bestehend aus Pyrrolidinyl, Piperidinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, n-Pentyl und Benzyl substituiert sein kann; jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

In einer weiteren Ausführungsform sind ganz besonders bevorzugt Verbindungen der allgemeinen Formel If,
worin
nf für 1 steht;
R^{2f} für F steht;
R^{8f} für F; Cl; Br; I; -CN; -OH; -NH₂; -NO₂; -NHR^{11f}; -NR^{12f}R^{13f}; -OR^{14f}; -SR^{15f};
für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, sec-Butyl, Isobutyl und tert-Butyl;
oder für einen Rest ausgewählt aus der Gruppe bestehend aus Phenyl, Naphthyl, Oxazolyl, Thiazolyl, Imidazolyl, Pyrimidinyl, Thiophenyl, Furanyl und Pyridinyl, der jeweils ggf. mit 1, 2, 3, 4 oder 5 Substituenten ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sec-Butyl, Isobutyl und tert-Butyl substituiert sein kann, steht;
R^{11f}, R^{12f}, R^{13f}, R^{14f} und R^{15f}, unabhängig voneinander, jeweils
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Rest ausgewählt aus der Gruppe bestehend aus Cyclopropyl, Cyclobutyl, Cyclopentyl und Cyclohexyl stehen;
oder
R^{12f} und R^{13f} jeweils zusammen mit dem sie verbindenden Stickstoffatom als Ringglied einen Rest ausgewählt aus der Gruppe bestehend aus Pyrrolidinyl, Piperidinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus Methyl, Ethyl und Benzyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ein weiterer Gegenstand der Erfindung sind Verbindungen der allgemeinen Formel Ig, 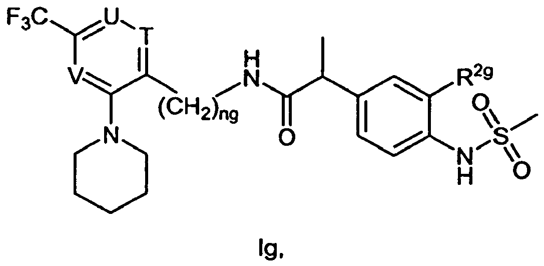 worin
ng für 0, 1 oder 2 steht;
R^{2g} für F; Cl; Br oder I steht;
T für CH und U für N und V für CH
oder
T für N und U für CH und V für CH
oder
T für N und U für N und V für CH
oder
T für N und U für CH und V für N
oder
T für CH und U für N und V für N
steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

In einer weiteren Ausführungsform sind ganz besonders bevorzugt Verbindungen der allgemeinen Formel Ig, worin
ng für 1 steht;
R^{2g} für F steht;
T für CH und U für N und V für CH
oder
T für N und U für CH und V für CH
oder
T für N und U für N und V für CH
oder
T für N und U für CH und V für N
oder
T für CH und U für N und V für N
steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ein weiterer Gegenstand der Erfindung sind Verbindungen der vorstehend angegebenen allgemeinen Formel I ausgewählt aus der Gruppe bestehend aus
[1] 2-(3-Fluor-4-(methylsulfonylamino)phenyl)-N-((2-methyl-6-(trifluormethyl)pyridin-3-yl)methyl)propanamid
[2] 2-(3-Fluor-4-(methylsulfonylamino)phenyl)-N-((2-(piperidin-1-yl)-6-(trifluormethyl)pyridin-3-yl)methyl)propanamid
[3] 2-(3-Fluor-4-(methylsulfonylamino)phenyl)-N-((2-piperidin-1-yl)-4-(trifluormethyl)benryl)propanamid
[4] 2-(3-Fluor-4-(methylsulfonylamino)phenyl)-N-((2-fluor-6-(trifluormethyl)pyridin-3-yl)methyl)propanamid
[5] N-((2-Chlor-6-(trifluormethyl)pyridin-3-yl)methyl)-2-(3-fluor-4-(methylsulfonylamino)phenyl)propanamid
[6] N-((2-Brom-6-(trifluormethyl)pyridin-3-yl)methyl)-2-(3-fluor-4-(methylsulfonylamino)phenyl)propanamid
[7] 2-(3-Fluor-4-(methylsulfonylamino)phenyl)-N-((2-iod-6-(trifluormethyl)pyridin-3-yl)methyl)propanamid
[8] N-((2-tert-Butyl-6-(trifluormethyl)pyridin-3-yl)methyl)-2-(3-fluor-4-(methylsulfonylamino)phenyl)propanamid
[9] N-((2-Cyano-6-(trifluormethyl)pyridin-3-yl)methyl)-2-(3-fluor-4-(methylsulfonylamino)phenyl)propanamid
[10] (S)-2-(3-Fluor-4-(methylsulfonylamino)phenyl)-N-((2-(piperidin-1-yl)-6-(trifluormethyl)pyridin-3-yl)methyl)propanamid
[11] (R)-2-(3-Fluor-4-(methylsulfonylamino)phenyl)-N-((2-(piperidin-1-yl)-6-(trifluormethyl)pyridin-3-yl)methyl)propanamid
[12] 2-(3-Fluor-4-(methylsulfonylamino)phenyl)-N-((2-morpholino-6-(trifluormethyl)pyridin-3-yl)methyl)propanamid
[13] 2-(3-Fluor-4-(methylsulfonylamino)phenyl)-N-((2-(pyrrolidin-1-yl)-6-(trifluormethyl)pyridin-3-yl)methyl)propanamid
[14] N-((2-(Dimethylamino)-6-(trifluormethyt)pyridin-3-yl)methyl)-2-(3-fluor-4-(methylsulfonylamino)phenyl)propanamid
[15] N-((2-(Diethylamino)-6-(trifluormethyl)pyridin-3-yl)methyl)-2-(3-fluor-4-(methylsulfonylamino)phenyl)propanamid
[16] N-((2-(Dipropylamino)-6-(trifluormethyl)pyridin-3-yl)methyl)-2-(3-fluor-4-(methylsulfonylamino)phenyl)propanamid
[17] 2-(3-Fluor-4-(methylsulfonylamino)phenyl)-N-((2-hydroxy-6-(trifluormethyl)pyridin-3-yl)methyl)propanamid
[18] 2-(3-Fluor-4-(methylsulfonylamino)phenyl)-N-((2-methoxy-6-(trifluormethyl)pyridin-3-yl)methyl)propanamid
[19] N-((2-Butoxy-6-(trifluormethyl)pyridin-3-yl)methyl)-2-(3-fluor-4-(methylsulfonylamino)phenyl)propanamid
[20] 2-(3-Fluor-4-(methylsulfonylamino)phenyl)-N-((2-isopropoxy-6-(trifluormethyl)pyridin-3-yl)methyl)propanamid
[21] N-((2-Cyclopentyloxy-6-(trifluormethyl)pyridin-3-yl)methyl)-2-(3-fluor-4-(methylsulfonylamino)phenyl)propanamid
[22] 2-(3-Fluor-4-(methylsulfonylamino)phenyl)-N-((2-phenyl-6-(trifluormethyl)pyridin-3-yl)methyl)propanamid
[23] 2-(3-Fluor-4-(methylsulfonylamino)phenyl)-N-((2-(4-fluor-phenyl)-6-(trifluormethyl)pyridin-3-yl)methyl)propanamid
[24] 2-(3-Fluor-4-(methylsulfonylamino)phenyl)-N-((6-(trifluormethyl)-2,2'-bipyridin-3-yl)methyl)propanamid
[25] 2-(3-Fluor-4-(methylsulfonylamino)phenyl)-N-((6-(trifluormethyl)-2,3'-bipyridin-3-yl)methyl)propanamid
[26] 2-(3-Fluor-4-(methylsulfonylamino)phenyl)-N-((2-(pyrimidin-2-yl)-6-(trifluormethyl)pyridin-3-yl)methyl)propanamid
[27] 2-(3-Fluor-4-(methylsulfonylamino)phenyl)-N-((2-(thiazol-2-yl)-6-(trifluormethyl)pyridin-3-yl)methyl)propanamid
[28] 2-(3-Fluor-4-(methylsulfonylamino)phenyl)-N-((2-(oxazol-2-yl)-6-(trifluormethyl)pyridin-3-yl)methyl)propanamid
[29] N-((2-(1H-Imidazol-2-yl)-6-(trifluormethyl)pyridin-3-yl)methyl)-2-(3-fluor-4-(methylsulfonylamino)phenyl)propanamid
[30] N-(2-Cyano-4-(trifluormethyl)benzyl)-2-(3-fluor-4-(methylsulfonylamino)phenyl)propanamid
[31] (S)-2-(3-Fluor-4-(methylsulfonylamino)phenyl)-N-(2-(piperidin-1-yl)-4-(trifluormethyl)benzyl)propanamid
[32] (R)-2-(3-Fluor-4-(methylsulfonylamino)phenyl)-N-(2-(piperidin-1-yl)-4-(trifluormethyl)benzyl)propanamid
[33] 2-(3-Fluor-4-(methylsulfonylamino)phenyl)-N-(2-morpholino-4-(trifluormethyl)benzyl)propanamid
[34] 2-(3-Fluor-4-(methylsulfonylamino)phenyl)-N-(2-(pyrrolidin-1-yl)-4-(trifluormethyl)benzyl)propanamid
[35] N-(2-(Dimethylamino)-4-(trifluormethyl)benzyl)-2-(3-fluor-4-(methylsulfonylamino)phenyl)propanamid
[36] N-(2-(Diethylamino)-4-(trifluormethyl)benzyl)-2-(3-fluor-4-(methylsulfonylamino)phenyl)propanamid
[37] N-(2-(Dipropylamino)-4-(trifluormethyl)benzyl)-2-(3-fluor-4-(methylsulfonylamino)phenyl)propanamid
[38] 2-(3-Fluor-4-(methylsulfonylamino)phenyl)-N-(2-hydroxy-4-(trifluormethyl)benzyl)propanamid
[39] 2-(3-Fluor-4-(methylsulfonylamino)phenyl)-N-(2-methoxy-4-(trifluormethyl)benzyl)propanamid
[40] N-(2-Butoxy-4-(trifluormethyl)benzyl)-2-(3-fluor-4-(methylsulfonylamino)phenyl)propanamid
[41] 2-(3-Fluor-4-(methylsulfonylamino)phenyl)-N-(2-isopropoxy-4-(trifluormethyl)benzyl)propanamid
[42] N-(2-(Cyclopentyloxy)-4-(trifluormethyl)benzyl)-2-(3-fluor-4-(methylsulfonylamino)phenyl)propanamid
[43] 2-(3-Fluor-4-(methylsulfonylamino)phenyl)-N-((5-(trifluormethyl)biphenyl-2-yl)methyl)propanamid
[44] 2-(3-Fluor-4-(methylsulfonylamino)phenyl)-N-((4'-fluor-5-(trifluormethyl)biphenyl-2-yl)methyl)propanamid
[45] 2-(3-Fluor-4-(methylsulfonylamino)phenyl)-N-(2-(pyridin-2-yl)-4-(trifluormethyl)benzyl)propanamid
[46] 2-(3-Fluor-4-(methylsulfonylamino)phenyl)-N-(2-(pyridin-3-yl)-4-(trifluormethyl)benzyl)propanamid
[47] 2-(3-Fluor-4-(methylsulfonylamino)phenyl)-N-(2-(pyrimidin-2-yl)-4-(trifluormethyl)benzyl)propanamid
[48] 2-(3-Fluor-4-(methylsulfonylamino)phenyl)-N-(2-(thiazol-2-yl)-4-(trifluormethyl)benzyl)propanamid
[49] 2-(3-Fluor-4-(methylsulfonylamino)phenyl)-N-(2-(oxazol-2-yl)-4-(trifluormethyl)benzyl)propanamid
[50] N-(2-(1H-Imidazol-2-yl)-4-(trifluormethyl)benzyl-(3-fluor-4-(methylsulfonylamino)phenyl)propanamid
[51] N-((6-tert-Butyl-2-(piperidin-1-yl)pyridin-3-yl)methyl)-2-(3-fluor-4-(methylsufonamido)phenyl)propanamid
[52] 2-(3-Fluor-4-(methylsulfonylamino)phenyl)-N-((4-(pipendin-1-yl)-6-(trifluormethyl)pyridin-3-yl)methyl)propanamid
[53] 2-(3-Fluor-4-(methylsulfonylamino)phenyl)-N-((3-(piperidin-1-yl)-5-(trifluormethyl)pyridin-2-yl)methyl)propanamid
[54] 2-(3-Fluor-4-(methylsulfonylamino)phenyl)-N-((4-(piperidin-1-yl)-2-(trifluormethyl)pyrimidin-5-yl)methyl)propanamid
[55] 2-(3-Fluor-4-(methylsulfonylamino)phenyl)-N-((3-(piperidin-1-yl)-5-(trifluormethyl)pyrazin-2-yl)methyl)propanamid
[56] 2-(3-Fluor-4-(methylsulfonylamino)phenyl)-N-((4-(piperidin-1-yl)-6-(trifluormethyl)pyridazin-3-yl)methyl)propanamid
[57] 2-(3-Fluor-4-(methylsulfonylamino)phenyl)-N-((2-piperidin-1-yl)-6-(trifluormethyl)pyridin-3-yl)propanamid
[58] 2-(3-Fluor-4-(methylsulfonylamino)phenyl)-N-((2-piperidin-1-yl)-4-(trifluormethyl)phenyl)propanamid
[59] 2-(3-Fluor-4-(methylsulfonylamino)phenyl)-N-(2-(2-(piperidin-1-yl)-6-(trifluormethyl)pyridin-3-yl)ethyl)propanamid
[60] 2-(3-Fluor-4-(methylsulfonylamino)phenyl)-N-(2-(piperidin-1-yl)-4-(trifluormethyl)phenethyl)propanamid
[61] N-(2-Amino-4-(trifluormethyl)benzyl)-2-(3-fluor-4-(methylsulfonylamino)phenyl)propanamid
[62] 2-(3-Fluor-4-(methylsulfonylamino)phenyl)-N-(2-nitro-4-(trifluormethyl)benzyl)propanamid
[63] N-(4-tert-Butyl-2-(piperidin-1-yl)benzyl)-2-(3-fluor-4-(methylsulfonylamino)phenyl)propanamid
[64] 2-(3-Chlor-4-(methylsulfonylamino)phenyl)-N-((2-(piperidin-1-yl)-6-(trifluormethyl)pyridin-3-yl)methyl)propanamid
[65] 2-(3-Chlor-4-(methylsulfonylamino)phenyl)-N-((2-(pyrrolidin-1-yl)-6-(trifluormethyl)pyridin-3-yl)methyl)propanamid
[66] 2-(3-Chlor-4-(methylsulfonylamino)phenyl)-N-(2-(piperidin-1-yl)-4-(trifluormethyl)benzyl)propanamid
[67] 2-(3-Brom-4-(methylsulfonylamino)phenyl)-N-((2-(piperidin-1-yl)-6-(trifluormethyl)pyridin-3-yl)methyl)propanamid
[68] 2-(3-Brom-4-(methylsulfonylamino)phenyl)-N-(2-(piperidin-1-yl)-4-(trifluormethyl)benzyl)propanamid
[69] 2-(3-Brom-4-(methylsulfonylamino)phenyl)-N-(2-(pyrrolidin-1-yl)-4-(trifluormethyl)benzyl)propanamid
[70] N-(4-tert-Butyl-2-cyanobenzyl)-2-(3-fluor-4-(methylsulfonylamino)phenyl)propanamid
[71] N-((6-(Chlordiflourmethyl)-2-(piperidin-1-yl)pyridin-3-yl)methyl)-2-(3-fluor-(4-methylsulfonylamino)phenyl)propanamid
[72] (S)-2-(3-Fluor-4-(methylsulfonylamino)phenyl)-N-((2-morpholino-6-(trifluormethyl)pyridin-3-yl)methyl)propanamid
[73] N-((2-(4-Benzylpiperazin-1-yl)-6-(trifluormethyl)pyridin-3-yl)methyl)-2-(3-fluor-4-(methylsulfonylamino)phenyl)propanamid
[74] 2-(3-Fluor-4-(methylsulfonylamino)phenyl)-N-((2-piperazin-1-yl)-6-(trifluormethyl)pyridin-3-yl)methyl)propanamid
[75] N-(2-Chlor-4-(trifluormethyl)benzyl)-2-(3-fluor-4-(methylsulfonylamino)phenyl)propanamid
[76] N-((2-(Cyclohexyloxy)-6-(trifluormethyl)pyridin-3-yl)methyl-2-(3-fluor-4-(methylsulfonylamino)phenyl)propanamid
[77] N-((3-Chlor-5-(trifluormethyl)pyridin-2-yl)methyl-2-(3-fluor-4-(methylsulfonylamino)phenyl)propanamid
[78] 2-(3-Fluor-4-(methylsulfonylamino)phenyl)-N-((3-(pyrrolidin-1-yl)-5-(trifluormethyl)pyridin-2-yl)methyl)propanamid
[79] N-((2-(3,5-Dimethylpiperidin-1-yl)-6-(trifluormethyl)pyridin-3-yl)methyl)-2-(3-fluor-4-(methylsulfonylamino)phenyl)propanamid
[80] 2-(3-Fluor-4-(methylsulfonylamino)phenyl)-N-((2-(4-methylpiperidin-1-yl)-6-(trifluormethyl)pyridin-3-yl)methyl)propanamid
[81] N-((2-(Azepan-1-yl)-6-(trifluormethyl)pyridin-3-yl)methyl)-2-(3-fluor-4-(methylsulfonylamino)phenyl)propanamid
und
[82] 2-(3-Fluor-4-(methylsulfonylamino)phenyl)-N-(2-(4-methylpiperidin-1-yl)-4-(trifiuormethyl)benzyl)propanamid;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin können erfindungsgemäße Verbindungen der allgemeinen Formeln A und I bevorzugt sein, die im FLIPR-Assay mit CHO K1-Zellen, die mit dem menschlichen VR1-Gen transfiziert wurden, in einer Konzentration kleiner 2000 nM, bevorzugt kleiner 1000 nM, besonders bevorzugt kleiner 300 nM, ganz besonders bevorzugt kleiner 100 nM, noch weiter bevorzugt kleiner 75 nM, darüber hinaus bevorzugt kleiner 50 nM, am weitesten bevorzugt kleiner 10 nM, eine 50-prozentige Verdrängung von Capsaicin bewirken, dass in einer Konzentration von 100 nM vorliegt.

Dabei wird Im FLIPR-Assay der Ca²⁺-Einstrom mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert, wie untenstehend beschrieben.

Ein weiterer Gegenstand der vorliegenden Erfindung ist ein Verfahren zur Herstellung von Verbindungen der vorstehend angegebenen allgemeinen Formel A und I gemäß dem wenigstens eine Verbindung der allgemeinen Formel II, 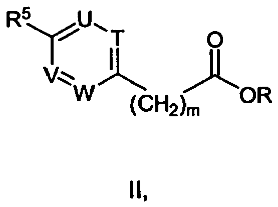 worin R⁵, U, T, V und W die vorstehend genannte Bedeutung haben, m für 0, 1, 2 oder 3 steht und R für Wasserstoff oder für einen C₁₋₆-Alkyl-Rest steht, in einem Reaktionsmedium, in Gegenwart wenigstens eines Reduktionsmittels, vorzugsweise in Gegenwart wenigstens eines Reduktionsmittels ausgewählt aus der Gruppe bestehend aus Natriumhydrid, Natrium, Kaliumhydrid, Lithiumaluminiumhydrid, Natriumborhydrid, BH₃xTHF und Di(isobutyl)aluminiumhydrid
zu wenigstens einer Verbindung der allgemeinen Formel III, 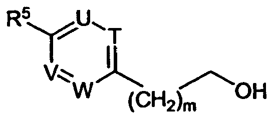 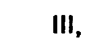 worin R⁵, U, T, V und W die vorstehend genannte Bedeutung haben und m für 0, 1, 2 oder 3 steht, umgesetzt wird und diese ggf. gereinigt und/oder isoliert wird,
und wenigstens eine Verbindung der allgemeinen Formel III in einem Reaktionsmedium in Gegenwart von Diphenylphosphorylazid oder in Gegenwart von HN₃ zu wenigstens einer Verbindung der allgemeinen Formel IV, 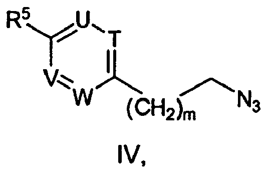 worin R⁵, U, T, V und W die vorstehend genannte Bedeutung haben und m für 0, 1, 2 oder 3 steht, umgesetzt wird und diese ggf. gereinigt und/oder isoliert wird,
und wenigstens eine Verbindung der allgemeinen Formel IV in einem Reaktionsmedium in Gegenwart wenigstens eines Reduktionsmittels, vorzugsweise in Gegenwart wenigstens eines Reduktionsmittels ausgewählt aus der Gruppe bestehend aus Natriumhydrid, Kaliumhydrid, Lithiumaluminiumhydrid, Natriumborhydrid und Di(isobutyl)aluminiumhydrid
oder in einem Reaktionsmedium in Gegenwart eines Katalysators, vorzugsweise in Gegenwart eines Katalysators basierend auf Platin oder Palladium, besonders bevorzugt in Gegenwart von Palladium auf Kohle, und in Gegenwart von Wasserstoff oder in Gegenwart von Hydrazin
oder in einem Reaktionsmedium in Gegenwart von Triphenylphosphin zu wenigstens einer Verbindung der allgemeinen Formel V, 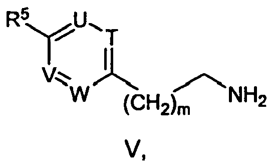 worin R⁵, U, T, V und W die vorstehend genannte Bedeutung haben und m für 0, 1, 2 oder 3 steht, umgesetzt wird und diese ggf. gereinigt und/oder isoliert wird,
oder wenigstens eine Verbindung der allgemeinen Formel VI, 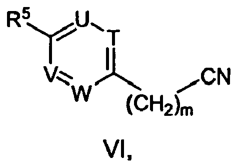 worin R⁵, U, T, V und W die vorstehend genannte Bedeutung haben und m für 0, 1, 2 oder 3 steht, in einem Reaktionsmedium in Gegenwart wenigstens eines Katalysators, vorzugsweise in Gegenwart wenigstens eines Katalysators basierend auf Palladium oder Platin, besonders bevorzugt in Gegenwart von Palladium auf Kohle, ggf. in Gegenwart wenigstens einer Säure, vorzugsweise in Gegenwart von Salzsäure, zu wenigstens einer Verbindung der allgemeinen Formel V, ggf. in Form eines entsprechenden Salzes, vorzugsweise in Form eines entsprechenden Hydrochlorids, umgesetzt wird und diese ggf. gereinigt und/oder isoliert wird,
und wenigstens eine Verbindung der allgemeinen Formel V mit wenigstens einer Verbindung der allgemeinen Formel VII, 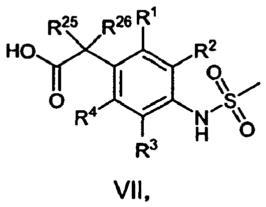 worin R¹, R², R³, R⁴, R²⁵ und R²⁶ die vorstehend genannte Bedeutung haben, in einem Reaktionsmedium, ggf. in Gegenwart wenigstens eines geeigneten Kopplungsmittels, ggf. in Gegenwart wenigstens einer Base,
oder mit wenigstens einer Verbindung der allgemeinen Formel VIII, 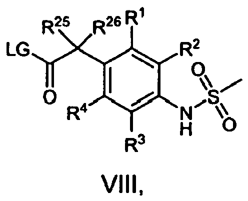 worin R¹, R², R³, R⁴, R²⁵ und R²⁶ die vorstehend genannte Bedeutung haben und LG für eine Abgangsgruppe, vorzugsweise für ein Chlor- oder Bromatom steht, in einem Reaktionsmedium, ggf. in Gegenwart wenigstens einer Base, zu wenigstens einer Verbindung der allgemeinen Formel Ih, 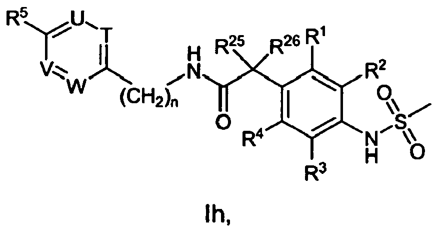 worin T, U, V, W, R¹, R², R³, R⁴, R⁵, R²⁵ und R²⁶ die vorstehend genannte Bedeutung haben und n für 1, 2, 3 oder 4 steht, umgesetzt wird und diese ggf. gereinigt und/oder isoliert wird,
und ggf. wenigstens eine Verbindung der allgemeinen Formel Ih in einem Reaktionsmedium mit wenigstens einer Verbindung der allgemeinen Formel IX, 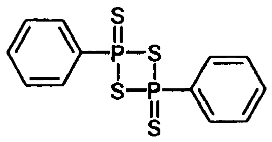 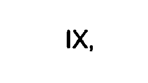 worin die Phenyl-Reste jeweils mit 1 oder 2 Substituenten unabhängig voneinander ausgewählt aus der Gruppe bestehend aus Methoxy, Phenoxy, Cl, Methyl und Br, bevorzugt jeweils mit einem Phenoxy-Rest oder Methoxy-Rest, besonders bevorzugt jeweils mit einem Methoxy-Rest in para-Position, substituiert sind, oder mit Phosphorpentasulfid, zu wenigstens einer Verbindung der allgemeinen Formel Ik, 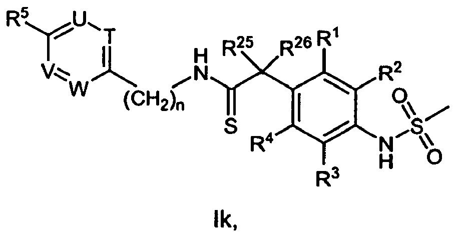 worin T, U, V, W, R¹, R², R³, R⁴, R⁵, R²⁵ und R²⁶ die vorstehend genannte Bedeutung haben und n für 1, 2, 3 oder 4 steht, umgesetzt wird und diese ggf. gereinigt und/oder isoliert wird.

Ein weiterer Gegenstand der vorliegenden Erfindung ist ein Verfahren zur Herstellung von Verbindungen der vorstehend angegebenen allgemeinen Formel I gemäß dem wenigstens eine Verbindung der allgemeinen Formel X, 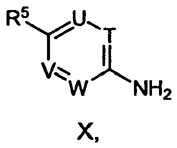 worin R⁵, U, T, V und W die vorstehend genannte Bedeutung haben, mit wenigstens einer Verbindung der allgemeinen Formel VII, 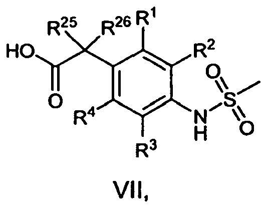 worin R¹, R², R³, R⁴, R²⁵ und R²⁶, die vorstehend genannte Bedeutung haben, in einem Reaktionsmedium, ggf. in Gegenwart wenigstens eines geeigneten Kopplungsmittels, ggf. in Gegenwart wenigstens einer Base,
oder mit wenigstens einer Verbindung der allgemeinen Formel VIII, 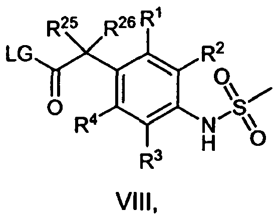 worin R¹, R², R³, R⁴, R²⁵ und R²⁶, die vorstehend genannte Bedeutung haben und LG für eine Abgangsgruppe, vorzugsweise für ein Chlor- oder Bromatom steht, in einem Reaktionsmedium, ggf. in Gegenwart wenigstens einer Base, zu wenigstens einer Verbindung der allgemeinen Formel Im, 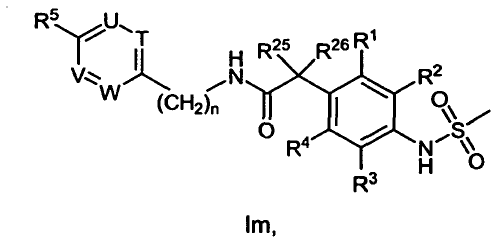 worin T, U, V, W, R¹, R², R³, R⁴, R⁵, R²⁵ und R²⁶, die vorstehend genannte Bedeutung haben, umgesetzt wird und diese ggf. gereinigt und/oder isoliert wird, und ggf. wenigstens eine Verbindung der allgemeinen Formel Im in einem Reaktionsmedium mit wenigstens einer Verbindung der allgemeinen Formel IX, 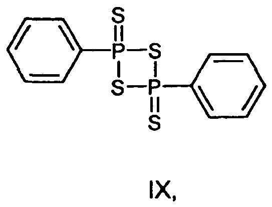 worin die Phenyl-Reste jeweils mit 1 oder 2 Substituenten unabhängig voneinander ausgewählt aus der Gruppe bestehend aus Methoxy, Phenoxy, Cl, Methyl und Br, bevorzugt jeweils mit einem Phenoxy-Rest oder Methoxy-Rest, besonders bevorzugt jeweils mit einem Methoxy-Rest in para-Position, substituiert sind, oder mit Phosphorpentasulfid, zu wenigstens einer Verbindung der allgemeinen Formel In, 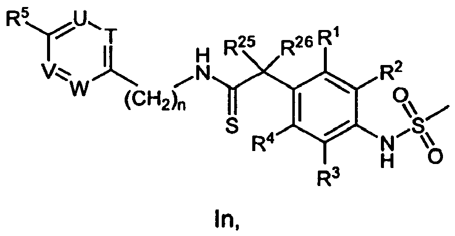 worin T, U, V, W, R¹, R², R³, R⁴, R⁵, R²⁵ und R²⁶, die vorstehend genannte Bedeutung haben, umgesetzt wird und diese ggf. gereinigt und/oder isoliert wird.

Die Umsetzung von Verbindungen der vorstehend angegebenen allgemeinen Formeln V bzw. X mit Carbonsäuren der vorstehend angegebenen allgemeinen Formel VII zu Verbindungen der vorstehend angegebenen allgemeinen Formeln Ih bzw. Im erfolgt vorzugsweise in einem Reaktionsmedium ausgewählt aus der Gruppe bestehend aus Diethylether, Tetrahydrofuran, Acetonitril, Methanol, Ethanol, (1,2)-Dichlorethan, Dimethylformamid, Dichlormethan und entsprechenden Mischungen, ggf. in Gegenwart wenigstens eines Kupplungsreagenzes, vorzugsweise ausgewählt aus der Gruppe bestehend aus 1-Benzotriazolyloxy-tris-(dimethylamino)-phosphonium hexafluorphosphat (BOP), Dicyclohexylcarbodümid (DCC), N'-(3-Dimethylaminopropyl)-N-ethylcarbodiimid (EDCI), Diisoproylcarbodiimid, 1,1'-Carbonyl-diimidazol (CDI), N-[(Dimethyamino)-1H-1, 2, 3-triazolo[4, 5-b]pyridino-1-ylmethylen]-N-methylmethanaminium hexafluorphosphat N-oxid (HATU), O-(Benzotriazol-1-yl)-N,N,N',N'-tetramethyluroniom hexafluorphosphat (HBTU), O-(Benzotriazol-1-yl)-N,N,N',N'-tetramethyluronium-tetrafluorborat (TBTU) und 1-Hydroxy-7-azabenzotriazol (HOAt), ggf. in Gegenwart wenigstens einer organischen Base, vorzugsweise ausgewählt aus der Gruppe bestehend aus Triethylamin, Pyridin, Dimethylaminopyridin, N-Methylmorpholin und Diisopropylethylamin, vorzugsweise bei Temperaturen von -70°C bis 100°C.

Alternativ erfolgt die Umsetzung von Verbindungen vorstehend angegebenen allgemeinen Formeln V bzw. X mit Carbonsäurederivaten der vorstehend angegebenen allgemeinen Formel VIII, worin LG für eine Abgangsgruppe, bevorzugt für ein Chlor- oder Bromatom, steht, zu Verbindungen der vorstehend angegebenen allgemeinen Formeln Ih bzw. Im in einem Reaktionsmedium vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart einer organischen oder anorganischen Base, vorzugsweise ausgewählt aus der Gruppe bestehend aus Triethylamin, Dimethylaminopyridin, Pyridin und Diisopropylamin, bei Temperaturen von -70°C bis 100°C.

Die Umsetzung von Verbindungen der allgemeinen Formeln Ih bzw. Im zu Verbindungen der allgemeinen Formeln Ik bzw. In erfolgt vorzugsweise in einem Reaktionsmedium ausgewählt aus der Gruppe bestehend aus Toluol, para-Xylol, ortho-Xylol, meta-Xylol, Acetonitril, Dichlormethan, Dimethylformamid und Mischungen der vorstehend genannten Reaktionsmedien, unter Zusatz eines Dithiaphosphetans, besonders bevorzugt unter Zusatz von 2,4-Bis(4-methoxyphenyl)-1,3,2,4-dithiadiphosphetan-2,4-disulfide (Lawesson-Reagenz), oder unter Zusatz von Phosphorpentasulfid, bei Temperaturen von 50 bis 150 °C.

Die Verbindungen der vorstehend angegebenen Formeln I, II, III, IV, V, VI, VIII, IX und X sind jeweils am Markt käuflich erhältlich und können auch nach üblichen, dem Fachmann bekannten Verfahren hergestellt werden.

Die Synthese von Verbindungen der allgemeinen Formel VII kann der Druckschrift "4-(Methylsulfonylamino)phenyl analogues as vanilloid antagonist showing excellent analgesic activity and the pharmaceutical compositions comprising the same" von J. W. Lee et al. [WO 2005/003084-A1] entnommen werden. Die entsprechenden Teile der Referenz gelten hiermit als Teil der Offenbarung.

Die vorstehend beschriebenen Umsetzungen können jeweils unter den üblichen dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden. Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie. Sämtliche der vorstehend beschriebenen Verfahrensschritte sowie jeweils auch die Reinigung und/oder Isolierung von Zwischen- oder Endprodukten können teilweise oder vollständig unter einer Inertgasatmosphäre, vorzugsweise unter Stickstoffatmosphäre, durchgeführt werden.

Bevorzugte Verbindungen der allgemeinen Formeln I, Ia, Ia1, Ib, Ib1, Ic, Ic1, Id, Id1, le, Ie1, If, If1, Ig, Ih, Ik, Im, In, A, B1, B2, C1 und C2 können jeweils als (S)-Enantiomer vorliegen. Beispielhaft ist im Folgenden das (S)-Enantiomer von Verbindungen der allgemeinen Formel Ia angeben.

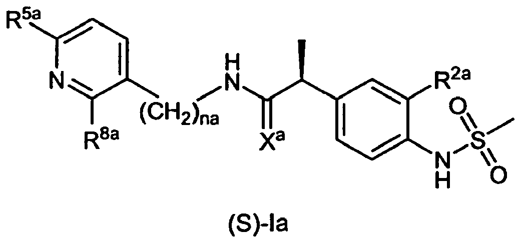

Die erfindungsgemäßen substituierten Verbindungen der vorstehend genannten allgemeinen Formeln I, Ia, Ia1, Ib, Ib1, Ic, Ic1, Id, Id1, le, Ie1, If, If1, Ig, Ih, Ik, Im, In, A, B1, B2, C1 und C2, im Folgenden nur als Verbindungen der allgemeinen Formel I bezeichnet, sowie entsprechende Stereoisomere können sowohl in Form ihrer freien Basen, ihrer freien Säuren wie auch in Form entsprechender Salze, insbesondere physiologisch verträglicher Salze, isoliert werden.

Die freien Basen der jeweiligen erfindungsgemäßen substituierten Verbindungen der vorstehend genannten allgemeinen Formel I sowie entsprechender Stereoisomere; insbesondere Verbindungen der allgemeinen Formel I, die einen Pyridin-Rest und/oder einen basischen Rest in Position des Substituenten R⁸ aufweisen, können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Zitronensäure, Glutaminsäure oder Asparaginsäure, in die entsprechenden Salze, vorzugsweise physiologisch verträglichen Salze, überführt werden. Die freien Basen der jeweiligen substituierten Verbindungen der vorstehend genannten allgemeinen Formel I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der substituierten Verbindungen der vorstehend genannten allgemeinen Formel I und entsprechender Stereoisomere durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze [NHₓR₄₋ₓ]⁺, worin x = 0, 1, 2, 3 oder 4 ist und R für einen linearen oder verzweigten C₁₋₄-Alkyl-Rest steht, genannt.

Die erfindungsgemäßen substituierten Verbindungen der vorstehend genannten allgemeinen Formel I und entsprechender Stereoisomere können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die erfindungsgemäßen substituierten Verbindungen der vorstehend genannten allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten Verbindungen der vorstehend genannten allgemeinen Formel I und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine erfindungsgemäße Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Diese erfindungsgemäßen Arzneimittel eignen sich insbesondere zur Vanilloid-Rezeptor 1-(VR1/TRPV1)-Regulation, vorzugsweise zur Vanilloid-Rezeptor 1-(VR1/TRPV1)-Hemmung und/oder zur Vanilloid-Rezeptor 1(VR1/TRPV1)-Stimulation.

Ebenfalls bevorzugt eignen sich die erfindungsgemäßen Arzneimittel zur Prophylaxe und/oder Behandlung von Störungen oder Krankheiten, die zumindest teilweise durch Vanilloid-Rezeptoren 1 vermittelt werden.

Bevorzugt eignet sich das erfindungsgemäße Arzneimittel zur Behandlung und/oder Prophylaxe von einer oder mehreren Erkrankungen ausgewählt aus der Gruppe bestehend aus Schmerz ausgewählt aus der Gruppe bestehend aus akutem Schmerz, chronischem Schmerz, neuropathischem Schmerz und visceralem Schmerz; Gelenkschmerz; Hyperalgesie; Allodynie; Kausalgie; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma, Bronchitis und Lungenentzündung; Husten; Harninkontinenz; einer überaktiven Blase (overactive bladder, OAB); Erkrankungen und/oder Verletzungen des Magen-Darm-Trakts; Zwölffingerdarmgeschwüren; Magengeschwüren; Reizdarmsyndrom; Schlaganfällen; Augenreizungen; Hautreizungen; neurotischen Hauterkrankungen; allergischen Hautkrankheiten; Psiorasis; Vitiligo; Herpes simplex; Entzündungen, vorzugsweise Entzündungen des Darmes, der Augen, der Blase, der Haut oder der Nasenschleimhaut; Diarrhöe; Pruritus; Osteoporose; Arthritis; Osteoarthritis; rheumatischen Erkrankungen; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Behandlung von Wunden und/oder Verbrennungen; zur Behandlung von durchtrennten Nerven; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Besonders bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Entzündungen, vorzugsweise Entzündungen des Darmes, der Augen, der Blase, der Haut oder der Nasenschleimhaut; Harninkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Ganz besonders bevorzugt eignet sich das erfindungsgemäße Arzneimittel zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz, und/oder Harninkontinenz.

Ein weiterer Gegenstand der vorliegenden Erfindung ist die Verwendung wenigstens einer erfindungsgemäßen Verbindung sowie ggf. eines oder mehrerer pharmazeutisch verträglicher Hilfsstoffe zur Herstellung eines Arzneimittels zur Vanilloid-Rezeptor 1-(VR1/TRPV1)-Regulation, vorzugsweise zur Vanilloid-Rezeptor 1-(VR1/TRPV1)-Hemmung und/oder zur Vanilloid-Rezeptor 1(VR1/TRPV1)-Stimulation.

Bevorzugt ist die Verwendung wenigstens einer erfindungsgemäßen substituierten Verbindung sowie ggf. eines oder mehrerer pharmazeutisch verträglicher Hilfsstoffe zur Herstellung eines Arzneimittels zur Prophylaxe und/oder Behandlung von Störungen oder Krankheiten, die zumindest teilweise durch Vanilloid-Rezeptoren 1 vermittelt werden.

Besonders bevorzugt ist die Verwendung wenigstens einer erfindungsgemäßen Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz und Gelenkschmerz.

Besonders bevorzugt ist die Verwendung wenigstens einer erfindungsgemäßen Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Hyperalgesie; Allodynie; Kausalgie; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma, Bronchitis und Lungenentzündung; Husten; Harninkontinenz; einer überaktiven Blase (overactive bladder, OAB); Erkrankungen und/oder Verletzungen des Magen-Darm-Trakts; Zwölffingerdarmgeschwüren; Magengeschwüren; Reizdarmsyndrom; Schlaganfällen; Augenreizungen; Hautreizungen; neurotischen Hauterkrankungen; allergischen Hautkrankheiten; Psiorasis; Vitiligo; Herpes simplex; Entzündungen, vorzugsweise Entzündungen des Darmes, der Augen, der Blase, der Haut oder der Nasenschleimhaut; Diarrhöe; Pruritus; Osteoporose; Arthritis; Osteoarthritis; rheumatischen Erkrankungen; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Behandlung von Wunden und/oder Verbrennungen; zur Behandlung von durchtrennten Nerven; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Ganz besonders bevorzugt ist die Verwendung wenigstens einer erfindungsgemäßen substituierten Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Entzündungen, vorzugsweise Entzündungen des Darmes, der Augen, der Blase, der Haut oder der Nasenschleimhaut; Harninkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Noch weiter bevorzugt ist die Verwendung wenigstens einer erfindungsgemäßen substituierten Verbindung sowie ggf. eines oder mehrerer pharmazeutisch verträglicher Hilfsstoffe zur Herstellung eines Arzneimittels zur Behandlung und/oder Prophylaxe von Schmerz, vorzugsweise ausgewählt aus der Gruppe bestehend aus akutem Schmerz, chronischem Schmerz, neuropathischem Schmerz und visceralem Schmerz, und/oder Harninkontinenz

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einer substituierten Verbindung der vorstehend angegebenen allgemeinen Formel I, ggf. in Form eines ihrer reinen Stereoisomeren, insbesondere Enantiomeren oder Diastereomeren, ihres Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die beispielsweise ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Die in dem erfindungsgemäßen Arzneimittel zum Einsatz kommenden erfindungsgemäßen substituierten Verbindungen in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die jeweilige erfindungsgemäße substituierte Verbindung auch verzögert freisetzen.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung. Die an den Patienten zu verabreichende Menge der jeweiligen erfindungsgemäßen substituierten Verbindungen der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,001 bis 100 mg/kg, vorzugsweise 0,05 bis 75 mg/kg, besonders bevorzugt 0,05 bis 50 mg/kg, Körpergewicht des Patienten wenigstens einer solchen erfindungsgemäßen Verbindung appliziert.

### Pharmakologische Methoden

### I. Funktionelle Untersuchung am Vanilloid-Rezeptor 1 (VRI/TRPV1-Rezeptor)

Die agonistische bzw. antagonistische Wirkung der zu untersuchenden Substanzen am Vanilloid-Rezeptor 1 (VR1/TRPV1) der Spezies Ratte kann mit folgendem Assay bestimmt werden. Gemäß diesem Assay wird der Ca²⁺-Einstrom durch den Rezeptorkanal mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert.

### Methode:

Komplett-Medium: 50 mL HAMS F12 Nutrient Mixture (Gibco Invitrogen GmbH, Karlsruhe, Deutschland) mit 10 Vol-% FCS (fetal calf serum, Gibco Invitrogen GmbH, Karlsruhe, Deutschland, hitzeinaktiviert); 2mM L-Glutamin (Sigma, München, Deutschland); 1 Gew-% AA-Lösung (Antibiotika/Antimykotika-Lösung, PAA, Pasching, Österreich) und 25 ng/ml Medium NGF (2.5 S, Gibco Invitrogen GmbH, Karlsruhe, Deutschland)

Zellkultur-Platte: Poly-D-Lysin-beschichtete, schwarze 96-Loch-Platten mit klarem Boden (96 weil black/clear plate, BD Biosciences, Heidelberg, Deutschland) werden zusätzlich mit Laminin (Gibco Invitrogen GmbH, Karlsruhe, Deutschland) beschichtet, indem Laminin auf eine Konzentration 100 µg/mL mit PBS (Ca-Mg-free PBS, Gibco Invitrogen GmbH, Karlsruhe, Deutschland) verdünnt wird. Es werden Aliquots mit einer Konzentration von 100 µg/mL an Laminin entnommen und bei -20°C gelagert. Die Aliquots werden mit PBS im Verhältnis 1:10 auf 10 µg/mL Laminin verdünnt und jeweils 50 µL der Lösung in eine Vertiefung der Zellkultur-Platte pipettiert. Die Zellkultur-Platten werden mindestens zwei Stunden bei 37°C inkubiert, die überstehende Lösung abgesaugt und die Vertiefungen werden jeweils zweimal mit PBS gewaschen. Die beschichteten Zellkultur-Platten werden mit überstehendem PBS aufbewahrt und dieses erst direkt vor der Aufgabe der Zellen entfernt.

### Präparation der Zellen:

Enthaupteten Ratten wird die Wirbelsäule entnommen und diese direkt in kalten, d. h. in einem Eisbad befindlichen, HBSS-Puffer (Hank's buffered saline solution, Gibco Invitrogen GmbH, Karlsruhe, Deutschland) versetzt mit 1 Vol-% (Volumenprozent) einer AA-Lösung (Antibiotika/Antimykotika-Lösung, PAA, Pasching, Österreich) gelegt. Die Wirbelsäule wird längs durchtrennt und zusammen mit Fascien dem Wirbelkanal entnommen. Anschließend werden die Dorsalwurzelganglien (DRGs; dorsal root ganglia) entnommen und wiederum in kaltem HBSS-Puffer versetzt mit 1 Vol-% einer AA-Lösung aufbewahrt. Die vollständig von Blutresten und Spinalnerven befreiten DRGs werden jeweils in 500 µL kalte Collagenase Typ 2 (PAA, Pasching, Österreich) überführt und 35 Minuten bei 37 °C inkubiert. Nach Zugabe von 2.5 Vol-% Trypsin (PAA, Pasching, Österreich) wird weitere 10 Minuten bei 37 °C inkubiert. Nach der vollständigen Inkubation wird die Enzymlösung vorsichtig ab pipettiert und die zurückgebliebenen DRGs werden jeweils mit 500 µL Komplett-Medium versetzt. Die DRGs werden jeweils mehrfach suspendiert, mittels einer Spritze durch Kanülen Nr. 1, Nr. 12 und Nr. 16 gezogen und in 50 mL Falcon-Röhrchen überführt und dieses mit Komplett-Medium auf 15 mL aufgefüllt. Der Inhalt jedes Falcon-Röhrchen wird jeweils durch einen 70 µm Falcon-Filtereinsatz filtriert und 10 Minuten bei 1200 Umdrehungen und Raumtemperatur zentrifugiert. Das resultierende Pellet wird jeweils in 250 µL Komplett-Medium aufgenommen und die Zellzahl ermittelt.

Die Anzahl der Zellen in der Suspension wird auf 3 mal 10⁵ pro mL eingestellt und jeweils 150 µL dieser Suspension in eine Vertiefung der wie vorstehend beschrieben beschichteten Zellkultur-Platten gegeben. Im Brutschrank werden die Platten bei 37 °C, 5 Vol-% CO₂ und 95 % relativer Luftfeuchtigkeit zwei bis drei Tage stehen gelassen.

Anschließend werden die Zellen mit 2 µM Fluo-4 und 0,01 Vol-% Pluronic F127 (Molecular Probes Europe BV, Leiden Niederlande) in HBSS-Puffer (Hank's buffered saline solution, Gibco Invitrogen GmbH, Karlsruhe, Deutschland) für 30 min bei 37 °C beladen, 3 x mit HBSS-Puffer gewaschen und nach einer weiteren Inkubation von 15 Minuten bei Raumtemperatur zur Ca²⁺-Messung im FLIPR-Assay eingesetzt. Die Ca²⁺-abhängige Fluoreszenz wird dabei vor und nach Zugabe von Substanzen gemessen (λex = 488 nm, λem = 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Verbindungen (10 µM) auf die Zellen pipettiert und der Ca²⁺-Einstrom mit der Kontrolle (Capsaicin 10 µM) verglichen. Daraus ergibt sich die Angabe in % Aktivierung bezogen auf das Ca²⁺-Signal nach Zugabe von 10 µM Capsaicin (CP). Nach 5 Minuten Inkubation werden 100 nM Capsaicin appliziert und ebenfalls der Einstrom von Ca²⁺ ermittelt.

Desensitisierende Agonisten und Antagonisten führen zu einer Unterdrückung des Ca²⁺-Einstroms. Es werden % Inhibierung im Vergleich zu der maximal erreichbaren Inhibierung mit 10 µM Capsaicin berechnet.

Es werden Dreifach-Bestimmungen (n=3) durchgeführt und diese in mindestens 3 unabhängigen Experimenten wiederholt (N=4).

Ausgehend von der prozentualen Verdrängung durch unterschiedliche Konzentrationen der zu prüfenden Verbindungen der allgemeinen Formel I wurden IC₅₀ Hemmkonzentrationen berechnet, die eine 50-prozentige Verdrängung von Capsaicin bewirken. Durch Umrechnung mittels der Cheng-Prusoff-Beziehung wurden Kᵢ-Werte für die Prüfsubstanzen erhalten (Cheng, Prusoff; Biochem. Pharmacol. 22, 3099-3108, 1973).

### II. Funktionelle Untersuchungen am Vanilloid Rezeptor (VR1)

Die agonistische bzw. antagonistische Wirkung der zu untersuchenden Substanzen auf Vanilloid Rezeptor (VR1) kann auch mit dem folgenden Assay bestimmt werden. Gemäß diesem Assay wird der Ca²⁺-Einstrom durch den Kanal mit Hilfe eines Ca²⁺-sensitiven Farbstoffs (Typ Fluo-4, Molecular Probes, Europe BV, Leiden, Niederlande) im Fluorescent Imaging Plate Reader (FLIPR, Molecular Devices, Sunnyvale, USA) quantifiziert.

### Methode:

Chinese Hamster Ovary Zellen (CHO K1-Zellen, European Collection of Cell Cultures (ECACC) Großbritannien) werden stabil mit dem VR1-Gen transfiziert. Für funktionelle Untersuchungen werden diese Zellen auf Poly-D-Lysin-beschichtete, schwarze 96-Loch-Platten mit klarem Boden (BD Biosciences, Heidelberg, Deutschland) in einer Dichte von 25.000 Zellen/Loch ausplattiert. Über Nacht werden die Zellen bei 37°C und 5 % CO₂ in einem Kulturmedium (Nutrient Mixture 'am's F12, 10 Vol-% FCS (Fetal calf serum), 18 µg/ml L-Prolin) inkubiert. Am folgenden Tag werden die Zellen mit Fluo-4 (Fluo-4 2 µM, Pluronic F127 0,01 Vol-%, Molecular Probes in HBSS (Hank's buffered saline solution), Gibco Invitrogen GmbH, Karlsruhe, Deutschland) für 30 Minuten bei 37°C inkubiert. Anschließend werden die Platten 3 mal mit HBSS-Puffer gewaschen und nach einer weiteren Inkubation von 15 Minuten bei Raumtemperatur zur Ca²⁺-Messung im FLIPR eingesetzt. Die Ca²⁺-abhängige Fluoreszenz wird dabei vor und nach Zugabe der zu untersuchenden Substanzen gemessen (Wellenlänge λₑₓ=488 nm, λₑₘ= 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Substanzen (10µM) auf die Zellen pipettiert und der Ca²⁺-Einstrom mit der Kontrolle (Capsaicin 10 µM) verglichen (% Aktivierung bezogen auf das Ca²⁺-Signal nach Zugabe von 10 µM Capsaicin). Nach 5 Minuten Inkubation werden 100 nM Capsaicin appliziert und ebenfalls der Einstrom von Ca²⁺ ermittelt.

Desensitisierende Agonisten und Antagonisten führten zu einer Unterdrückung des Ca²⁺-Einstroms. Es werden % Inhibierung im Vergleich zu der maximal erreichbaren Inhibierung mit 10 µM Capsaicin berechnet.

Ausgehend von der prozentualen Verdrängung durch unterschiedliche Konzentrationen der zu prüfenden Verbindungen der allgemeinen Formel I wurden IC₅₀ Hemmkonzentrationen berechnet, die eine 50-prozentige Verdrängung von Capsaicin bewirken. Durch Umrechnung mittels der Cheng-Prusoff-Beziehung wurden Kᵢ-Werte für die Prüfsubstanzen erhalten (Cheng, Prusoff; Biochem. Pharmacol. 22, 3099-3108, 1973).

### III. Formalin-Test an der Maus

Die Untersuchung zur Bestimmung der antinociceptiven Wirkung der erfindungsgemäßen Verbindungen wird im Formalin-Test an männlichen Mäusen (NMRI, 20 bis 30 g Körpergewicht, Iffa, Credo, Belgien) durchgeführt.

Im Formalin-Test werden gemäß D. Dubuisson et al., Pain 1977, 4, 161-174 die erste (frühe) Phase (0 bis 15 Minuten nach der Formalin-Injektion) und die zweite (späte) Phase (15 bis 60 Minuten nach der Formalin-Injektion) unterschieden. Die frühe Phase stellt als direkte Reaktion auf die Formalin-Injektion ein Modell für Akutschmerz dar, während die späte Phase als Modell für persistierenden (chronischen) Schmerz angesehen wird (T. J. Coderre et al., Pain 1993, 52, 259-285). Die entsprechenden Literaturbeschreibungen werden hiermit als Referenz eingeführt und gelten als Teil der Offenbarung.

Die erfindungsgemäßen Verbindungen werden in der zweiten Phase des Formalin-Tests untersucht, um Aussagen über Substanzwirkungen auf chronisch/entzündlichen Schmerz zu erhalten.

In Abhängigkeit von der Applikationsart der erfindungsgemäßen Verbindungen wird der Applikationszeitpunkt der erfindungsgemäßen Verbindungen vor der Formalin-Injektion gewählt. Die intravenöse Applikation von 10 mg/kg Körpergewicht der Testsubstanzen erfolgt 5 Minuten vor der Formalin-Injektion. Diese erfolgt durch eine einmalige subkutane Formalin-Injektion (20 µL, 1 %-ige wäßrige Lösung) in die dorsale Seite der rechten Hinterpfote, so dass bei frei beweglichen Versuchstieren eine nociceptive Reaktion induziert wird, die sich in deutlichem Lecken und Beißen der betreffenden Pfote äußert.

Anschließend wird für einen Untersuchungszeitraum von drei Minuten in der zweiten (späten) Phase des Formalin-Tests (21 bis 24 Minuten nach der Formalin-Injektion) das nociceptive Verhalten durch Beobachtung der Tiere kontinuierlich erfaßt. Die Quantifizierung des Schmerzverhaltens erfolgt durch Summation der Sekunden, in denen die Tiere in dem Untersuchungszeitraum ein Lecken und Beißen der betroffenen Pfote zeigen.

Der Vergleich erfolgt jeweils mit Kontrolltieren, die anstelle der erfindungsgemäßen Verbindungen Vehikel (0.9%-ige wäßrige Natriumchlorid-Lsg.) vor Formalinapplikation erhalten. Basierend auf der Quantifizierung des Schmerzverhaltens wird die Substanzwirkung im Formalin-Test als Änderung gegen die entsprechende Kontrolle in Prozent ermittelt.

Nach Injektion von Substanzen, die im Formalin-Test antinociceptiv wirksam sind, werden die beschriebenen Verhaltensweisen der Tiere, d. h. Lecken und Beißen, reduziert bzw. aufgehoben.

### IV. Untersuchung auf analgetische Wirksamkeit im Writhing-Test

Die Untersuchung der erfindungsgemäßen Verbindungen der allgemeinen Formel I auf analgetische Wirksamkeit wurde im Phenylchinon-induzierten Writhing an der Maus, modifiziert nach I.C. Hendershot und J. Forsaith (1959) J. Pharmacol. Exp. Ther. 125, 237-240 durchgeführt. Die entsprechende Literaturbeschreibung wird hiermit als Referenz eingeführt und gilt als Teil der Offenbarung.

Dazu wurden männliche NMRI-Mäuse mit einem Gewicht von 25 bis 30 g verwendet. Gruppen von 10 Tieren pro Verbindungsdosis erhielten 10 Minuten nach intravenöser Gabe der zu untersuchenden Verbindungen 0,3 ml/Maus einer 0,02%igen wäßrigen Lösung von Phenylchinon (Phenylbenzochinon, Fa. Sigma, Deisenhofen, Deutschland; Herstellung der Lösung unter Zusatz von 5 Gew.-% Ethanol und Aufbewahrung im Wasserbad bei 45°C) intraperitoneal appliziert. Die Tiere wurden einzeln in Beobachtungskäfige gesetzt. Mit Hilfe eines Drucktastenzählers wurde die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mit geführt, die nur physiologische Kochsalzlösung erhalten hatten. Alle Verbindungen wurden in der Standarddosierung von 10 mg/kg getestet.

### V. Hypothermie Assay an der Maus

Methodenbeschreibung:
Der Hypothermie Assay wird in männlichen NMRI Mäusen (Gewicht 25-35 Gramm, Züchter IFFA CREDO, Brüssel, Belgien) durchgeführt. Die Tiere wurden unter standardisierten Bedingungen gehalten: Licht/Dunkel Rhythmus (6:00 bis 18:00 Uhr Licht-; 18:00 bis 6:00 Uhr Dunkelphase), Raumtemperatur 19-22°C, relative Luftfeuchte 35-70%, 15 Raumluftwechsel pro Stunde, Luftbewegung < 0.2 m/sec. Die Tiere erhielten Standardfutter (ssniff R/M-Haltung, ssniff Spezialdiäten GmbH, Soest, Germany) und Leitungswasser. Wasser und Futter wurden während des Versuches entzogen. Alle Tiere wurden nur einmal im Versuch eingesetzt. Die Tiere hatten eine Eingewöhnungsphase von mindestens 5 Tagen.

Die akute Applikation von Capsaicin (VR-1 Agonist) führt zu einem Abfall der Körperkemtemperatur in Ratten und Mäusen über eine Stimulation von Wärmesensoren. Nur spezifisch wirkende VR-1-rezeptor Antagonisten können die Capsaicin induzierte Hypothermie antagonisieren. Eine durch Morphin induzierte Hypothermie dagegen wird nicht von VR-1 Antagonisten antagonisiert. Dieses Modell ist daher geeignet, Substanzen mit VR-1 antagonistischen Eigenschaften über die Wirkung auf die Körpertemperatur zu identifizieren.

Für die Messung der Körperkemtemperatur wurde ein digitales Thermometer (Thermalert TH-5, physitemp, Clifton NJ, USA) benutzt. Der Meßfühler wird dabei in das Rektum der Tiere eingeführt.

Als individueller Basiswert wird bei jedem Tier 2 mal im Abstand von ca. einer halben Stunde die Körpertemperatur gemessen. Anschließend erhält eine Gruppe von Tieren (n= 6 bis 10) eine intraperitoneale (i.p.) Applikation von Capsaicin 3mg/kg und Vehikel (Kontrollgruppe). Eine andere Gruppe von Tieren erhält die zu testende Substanz (i.v. oder p.o.) und zusätzlich Capsaicin (3mg/kg) i.p. Die Applikation der Testsubstanz erfolgt i.v. 10 min bzw p.o. 15 Minuten vor Capsaicin. Anschließend wird 7,5 / 15 und 30 min nach Capsaicin(i.v. + i.p.) bzw. 15 / 30 / 60 /90 /120 min (p.o. + i.p.) nach Capsaicin die Körpertemperatur gemessen. Zusätzlich wird eine Tiergruppe nur mit der Testsubstanz behandelt sowie eine Gruppe nur mit Vehikel. Die Auswertung bzw. Darstellung der Meßwerte erfolgt als Mittelwert +/- S.E.M. der absoluten Werte als Grafik. Die antagonistische Wirkung wird berechnet als Prozent Reduktion der Capsaicin-induzierten Hypothermie.

### VI. Neuropathischer Schmerz an der Maus

Die Untersuchung auf Wirksamkeit im neuropathischen Schmerz wurde im Bennett-Modell (chronic constriction injury; Bennett und Xie, 1988, Pain 33: 87-107) untersucht.

NMRI Mäuse mit einem Gewicht von 16-18g werden unter Ketavet-Rompun-Narkose mit drei losen Ligaturen des rechten nervus ischiaticus versehen. Die Tiere entwickeln an der vom geschädigten Nerv innervierten Pfote eine Überempfindlichkeit, die nach einer Erholungsphase von einer Woche über etwa drei Wochen mittels einer 4°C kalten Metallplatte quantifiziert wird (Kälte-Allodynie). Die Tiere werden für einen Zeitraum von 2 min. auf dieser Platte beobachtet und die Anzahl der Wegziehreaktionen der geschädigten Pfote wird gemessen. Bezogen auf den Vorwert vor Substanzapplikation wird die Substanzwirkung über einen bestimmten Zeitraum an verschiedenen Zeitpunkten (z.B. 15, 30, 45, 60 min. nach Applikation) bestimmt und die resultierend Fläche unter der Kurve (AUC) und/oder die Hemmung der Kälte-Allodynie zu den einzelnen Meßpunkten in Prozent Wirkung zur Vehikelkontrolle (AUC) bzw. zum Ausgangswert (Einzelmeßpunkte) ausgedrückt. Die Gruppengröße beträgt n=10, die Signifikanz einer anti-allodynischen Wirkung (*=p<0.05) wird anhand einer Varianzanalyse mit wiederholter Messung und einer post hoc Analyse nach Bonferroni bestimmt.

Im Folgenden wird die Erfindung mit Hilfe einiger Beispiele erläutert. Diese Erläuterungen sind lediglich beispielhaft und schränken den allgemeinen Erfindungsgedanken nicht ein.

### Beispiele:

Die Ausbeuten der hergestellten Verbindungen wurden nicht optimiert.

Alle Temperaturen sind unkorrigiert.

Die Angabe "Äquivalente" bedeutet Stoffmengenäquivalente, "RT" Raumtemperatur, "M" und "N" sind Konzentrationsangaben in mol/l, "aq." wäßrig, "ges." gesättigt, "Lsg." Lösung,

Weitere Abkürzungen:

| | |
|---|---|
| DMF | N,N-Dimethylformamid |
| EDCI | N-Ethyt-N'-(3-dimethylaminopropyl)-carbodiimid Hydrochlorid |
| EE | Essigsäureethylester |
| H₂O | Wasser |
| MeOH | Methanol |

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, Oakwood etc.) bezogen oder nach den üblichen dem Fachmann bekannten Methoden synthetisiert.

Als stationäre Phase für die Säulenchromathographie wurde Kieselgel 60 (0.0-0 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Lösungsmitteln, Laufmitteln oder für chromatographische Untersuchungen sind stets in Volumen/Volumen angegeben.

Die Analytik erfolgte durch Massenspektroskopie und NMR.

### 1. Allgemeine Vorschrift zur Darstellung von Aminen der allgemeinen Formel V-A

Die Darstellung von Aminen der allgemeinen Formel V-A erfolgt wie im nachfolgenden Schema 1 dargestellt.

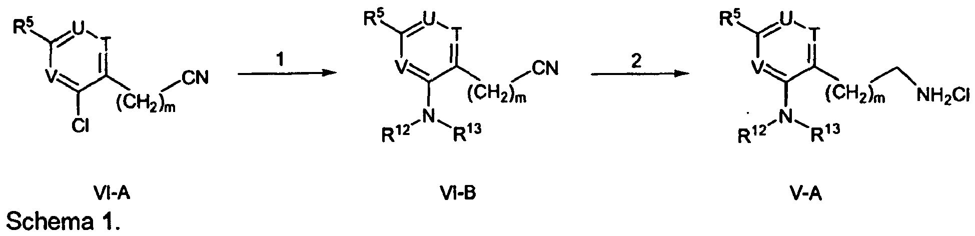

### Stufe 1: Darstellung von Nitrilen der allgemeinen Formel VI-A

### Methode A:

Verbindungen der allgemeinen Formel VI-A (1 Äquivalent), worin R⁵, U, T und V die vorstehend genannte Bedeutung haben und m für 0, 1, 2 oder 3 steht, werden mit einem Amin der allgemeinen Formel HNR¹²R¹³ (6 Äquivalente) 48 Stunden bei RT gerührt. Die Reaktionsmischung wird mit 1 N Salzsäure versetzt und mehrfach mit EE extrahiert. Die wäßrige Phase wird mit NaCl gesättigt und anschließend wiederum mit EE extrahiert. Die vereinigten organischen Phasen werden mit 1 N Salzsäure und mit ges. aq. NaCl-Lsg. gewaschen, über MgSO₄ getrocknet und das Lösungsmittel wurde im Vakuum entfernt.

Die folgenden Verbindungen A-1 bis A-6 wurden gemäß der vorstehend genannten allgemeinen Vorschrift erhalten:

### Verbindung A-1:

### 2-(Piperidin-1-yl)-6-(trifluormethyl)nicotinonitril

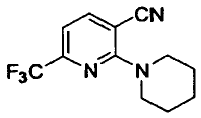

Die Verbindung wurde in einer Ausbeute von 86 % als leicht gelbes Öl erhalten.

¹H NMR (300 MHz, CDCl₃) δ 7.87 (d, 1 H, *J* = 7.8 Hz), 6.95 (d, 1 H, *J* = 7.8 Hz), 3.78 (m, 4 H), 1.71 (m, 6 H) IR (KBr) 2941, 2857, 2218, 1590, 1496, 1453, 1346, 1318, 1239, 1186 cm⁻¹ MS (FAB) *m*/*z* 256 (M+H)

### Verbindung A-2:

### 2-(Morpholin-4-yl)-6-(trifluormethyl)nicotinonitrit

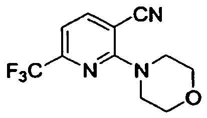

Die Verbindung wurde in einer Ausbeute von 78 % als leicht gelbes Öl erhalten.

¹H NMR (300 MHz, CDCl₃) δ 7.94 (d, 1 H, *J* = 7.8 Hz), 7.05 (d, 1 H, *J* = 7.8 Hz), 3.84 (s, 8 H)

IR (KBr) 3397, 2968,1511,1428,1337,1124cm-1

MS (FAB) *m*/*z* 258 (M+H)

### Verbindung A-3:

### 2-(Pyrrolidin-1-yl)-6-(trifluormethyl)nicotinonitril

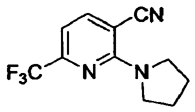

Die Verbindung wurde in einer Ausbeute von 85 % als leicht gelbes Öl erhalten.

¹H NMR (300 MHz, CDCl₃) #x3b4;#x3b4; 7.83 (d, H, *J* = 7.8 Hz), 6.86 (d, 1 H, *J* = 7.8 Hz), 3.78-3.83 (m, 4 H), 1.96-2.04 (m, 4 H) IR (KBr) 2976, 2880, 2216, 1591, 1502, 1457, 1344, 1303, 1247, 1181 cm⁻¹ MS (FAB) *m*/*z* 242 (M+H)

### Verbindung A-4:

### 2-(Piperidin-1-yl)-4-(trifluormethyl)benzonitril

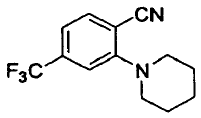

Die Verbindung wurde in einer Ausbeute von 74 % als leicht gelbes Öl erhalten.

¹H NMR (300 MHz, CDCl₃) δ 7.63 (d, 1 H, *J* = 7.8 Hz), 7.1-7.19 (m, 2 H), 3.22-3.26 (m, 4 H), 1.60-1.80 (m, 6 H)

### Verbindung A-5:

### 2-(Morpholin-4-yl)-4-(trifluormethyl)benzonitril

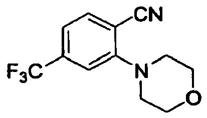

Die Verbindung wurde in einer Ausbeute von 80 % als leicht gelbes Öl erhalten.

¹H NMR (300 MHz, CDCl₃) δ 8.31 (d, 1 H, *J* =7.8 Hz), 7.85-7.88 (m, 2 H), 4.36-4.39 (m, 4 H), 3.76-3.79 (m, 4 H)

IR (KBr) 2856, 1614, 2210, 1501, 1430, 1311, 1258, 1173, 1122, 1077 cm⁻¹

### Verbindung A-6:

### 2-(Pyrrolidin-1-yl)-4-(trifluormethyl)benzonitril

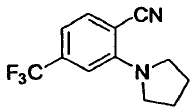

Die Verbindung wurde in einer Ausbeute von 80 % als leicht gelbes Öl erhalten.

¹H NMR (400 MHz, CDCl₃) δ 7.53 (d, 1 H, *J* = 8.8 Hz), 6.83-6.85 (m, 2 H), 3.65 (t, 4 H, *J* = 6.4 Hz), 2.04 (t, 4 H, *J* = 6.4 Hz)

IR (KBr) 2972, 2212, 1619, 1561, 1504, 1454, 1306, 1169 cm⁻¹

MS (FAB) *m*/*z* 241 (M+H)

### Methode B:

Verbindungen der allgemeinen Formel VI-A (1 Äquivalent), worin R⁵, U, T und V die vorstehend genannte Bedeutung haben und m für 0, 1, 2 oder 3 steht, werden mit einem Amin der allgemeinen Formel HNR¹²R¹³ (2 Äquivalente) und DBU [1,8-Diaza-bicyclo[5.4.0]undec-7-en] (2 Äquivalente) in Acetonitril (7 ml pro mmol der Verbindung der Formel VI-A) 12 Stunden bei RT gerührt. Die Reaktionsmischung wird mehrfach mit EE extrahiert. Die vereinigten organischen Phasen werden mit ges. aq. NaCl-Lsg. gewaschen, über MgSO₄ getrocknet und das Lösungsmittel wurde im Vakuum entfernt. Der Rückstand wird jeweils durch Säulenchromatographie (SiO₂, verschiedene Mischungen Hexan/EE) gereinigt.

Die folgenden Verbindungen A-7 bis A-102 wurden gemäß der vorstehend genannten allgemeinen Vorschrift erhalten:

### A-7: 6-(Chlorodifluoromethyl)-2-(piperidin-1-yl)pyridine-3-carbonitril

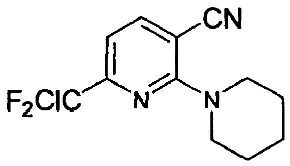

¹H NMR (300 MHz, CDCl₃) δ 7.87 (d, 1 H, *J* = 7.8 Hz), 6.94 (δ, 1 H, *J* = 7.8 Hz),_3.22-3.26 (m, 4 H), 1.60-1.80 (m, 6 H); IR (pur) 2939, 2857, 2217, 1588, 1493, 1451, 1296, 1235, 1109, 977, 917, 807 cm⁻¹; MS (FAB) *m*/*z* 272 (M+H)

### A-8: 2-(4-Benzylpiperazin-1-yl)-6-(trifluoromethyl)pyridine-3-carbonitril

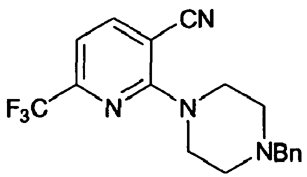

¹H NMR (300 MHz, CDCl₃) δ 7.83 (d, 1H, *J* = 7.8 Hz), 7.19 - 7.30 (m, 5H), 6.94 (d, 1 H, *J* = 7.8 Hz), 3.80-3.83 (m, 4 H), 3.52 (s, 2 H), 2.52-2.56 (m, 4 H); IR (pur) 2813, 1590, 1498, 1451, 1321, 1239, 1143, 968, 824, 742 cm⁻¹; MS (FAB) *m*/*z* 347 (M+H)

### A-9: 6-(Trifluoromethyl)-2-(4-methylpiperidin-1-yl)pyridine-3-carbonitril

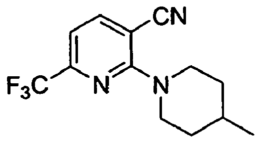

¹H NMR (300 MHz, CDCl₃) δ 7.87 (d, 1 H, *J* = 7.8 Hz), 6.95 (d, 1 H, *J* = 7.8 Hz), 4.53 (m, 2H), 3.05 (m, 2 H), 1.78 (m, 2 H), 1.64 (m, 1 H), 1.29 (m, 2 H), 1.00 (d, 3 H, *J* = 6.6 Hz); IR (pur) 2926, 2852, 2218, 1590, 1497, 1456, 1324, 1237, 1186, 1147, 1082, 963 cm⁻¹; MS (FAB) *m*/*z* 270 (M+H)

### A-10: 6-(Trifluoromethyl)-2-(3,5-dimethylpiperidin-1-yl)pyridine-3-carbonitril

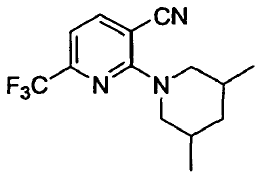

¹H NMR (300 MHz, CDCl₃) δ 7.84 (d, 1 H, *J* = 7.8 Hz), 6.91 (d, 1 H, *J* = 7.8 Hz), 4.50 (m, 2 H), 2.49 (m, 2 H), 1.67-1.89 (m, 4 H), 0.92 (d, 6 H, J = 6.6 Hz) IR (pur) 2925, 2852, 2216, 1592, 1498, 1457, 1325, 1188, 1145, 1080, 962 cm⁻¹ MS (FAB) *m*/*z* 284 (M+H)

### A-11: 2-Azocan-1-yl-6-trifluoromethyl-nicotinonitril

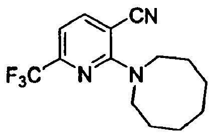

¹H NMR (300 MHz, CDCl₃) δ 7.86 (d, 1 H, J = 7.8 Hz), 6.87 (d, 1 H, J = 7.8 Hz), 3.88 (t, 4 H, J = 6.0 Hz), 1.87 (m, 4 H), 1.55 (m, 4 H); IR (KBr) 2929, 2857, 2213, 1592, 1563, 1510, 1455, 1327, 1235, 1188, 1145, 1080, 999, 816, 743 cm⁻¹; MS (FAB) *m*/*z* 284(M+H)

### A-12: 4-Phenyl-6'-trifluoromethyl-3,4,5,6-tetrahydro-2H-[1,2']bipyridinyl-3'-carbonitril

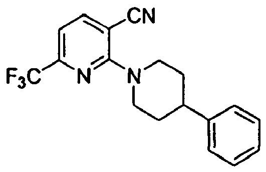

¹H NMR (300 MHz, CDCl₃) δ 7.90 (dd, 1 H, J = 7.5. 0.9 Hz), 7.20-7.35 (m, 5 H), 7.00 (d, 1 H, J = 7.5 Hz), 4.70 (dt, 2 H, J =13.5, 1.8 Hz), 3.17 (dt, 2 H, J = 13.5, 3.3 Hz), 2.82 (m, 1 H), 1.95 (m, 2 H), 1.87 (m, 2 H); IR (KBr) 2938, 2852, 2217, 1590, 1566, 1376, 1190, 1145, 1081, 1012, 958, 824, 752 cm⁻¹; MS (FAB) *m*/*z* 332(M+H)

### A-13: 4-Fluoro-6'-trifluoromethyl-3,4,5,6-tetrahydro-2H-[1,2']bipyridiny1-3'-carbonitril

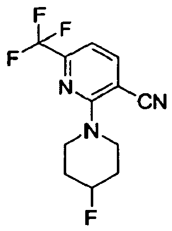

¹H NMR (300 MHz, CDCl₃) δ 7.93 (dd, 1 H, J = 7.5, 0.9 Hz), 7.04 (d, 1 H, J = 7.5 Hz), 4.94 (dm, 1 H, J = 48.3 Hz), 3.98 (m, 2 H), 3.81 (m, 1 H), 1.90-2.13 (m, 4 H) MS (FAB) *m*/*z* 274(M+H)

### A-14: 6'-(Chloro-difluoro-methyl)-4-methyl-3,4,5,6-tetrahydro-2H-[1,2']bipyridinyt-3'-carbonitril

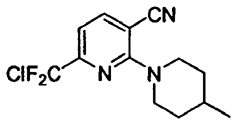

¹H NMR (300 MHz, CDCl₃) δ 7.86 (d, 1 H, *J* = 7.5 Hz), 6.93 (d, 1 H, *J* = 7.5 Hz), 4.53 (m, 2H), 3.05 (m, 2 H), 1.62-1.80 (m, 3 H), 1.23-1.27 (m, 2 H), 0.99 (d, 3 H, J = 6.6 Hz); IR (KBr) 2925, 2217, 1589, 1559, 1497, 1455, 1336, 12231, cm⁻¹ MS (FAB) *m*/*z* 286(M+H)

### A-15: 2-Azepan-1-yl-6-(chloro-difluoro-methyl)-nicotinonitril

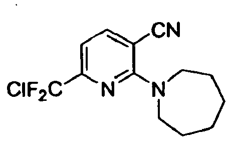

¹H NMR (300 MHz, CDCl₃) δ 7.85 (d, 1 H, *J* = 7.5 Hz), 6.85 (d, 1 H, *J* = 7.5 Hz), 3.87 (t, 4H, *J* = 6.= Hz), 1.90 (m, 4 H), 1.60 (m, 4 H); IR (KBr) 2931, 2214, 1590, 1558, 1506, 1455, 1339 cm⁻¹; MS (FAB) *m*/*z* 286(M+H)

### A-16: 6'-(4-Fluoro-phenyl)-4-methyl-3,4,5,6-tetrahydro-2H-[1,2']bipyridinyl-3'-carbonitril

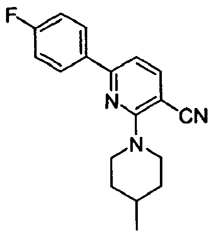

¹H NMR (300 MHz, CDCl₃) δ 8.00 (m, 2H), 7.77 (d, 1H, *J* = 7.8 Hz), 7.07-7.17 (m, 3 H), 4.51 (m, 2 H), 3.05 (m, 2 H), 1.77 (m, 2 H), 1.66 (m, 1 H), 1.35 (m, 2 H), 0.99 (d, 3 H, J = 6.6 Hz); IR (KBr) 2935, 2210, 1576, 1508, 1449, 1329, 1233, 1156, 1116, 1021, 949 cm⁻¹; MS (FAB) *m*/*z* 296(M+H)

### A-17: 2-Azepan-1-yl-6-(4-fluoro-phenyl)-nicotinonitril

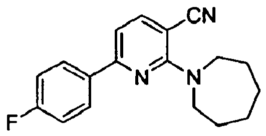

¹H NMR (300 MHz, CDCl₃) δ 8.00 (m, 2H), 7.75 (d, 1 H, *J* = 7.8 Hz), 7.13 (dd, 1 H, *J* = 8.7, 8.7 Hz), 7.01 (d, 1 H, *J* = 7.8 Hz), 3.92 (t, 4H, *J* = 6.= Hz), 1.92 (m, 4 H), 1.60 (m, 4 H); IR (KBr) 2930, 2855, 2206, 1577, 1504, 1452, 1338, 1277, 1234, 1155, 848, 805 cm⁻¹; MS (FAB) *m*/*z* 296(M+H)

### A-18: 6-(Chloro-difluoro-methyl)-2-dipropylamino-nicotinonitril

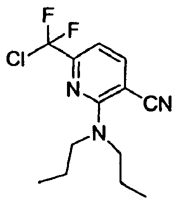

¹H NMR (300 MHz, CDCl₃) δ 7.82 (d, 1 H, *J* = 7.8 Hz), 6.83 (d, 1 H, *J* = 7.8 Hz), 3.63 (t, 4H, J = 7.5 Hz), 1.73 (m, 4 H), 0.96 (t, 6 H, J = 7.2 Hz); IR (KBr) 2968,2214,1590, 1455, 1374, 1232, 1108 cm⁻¹; MS (FAB) *m*/*z* 288(M+H)

### A-19: 2-(1,3-Dihydro-isoindol-2-yl)-6-trifluoromethyl-nicotinonitril

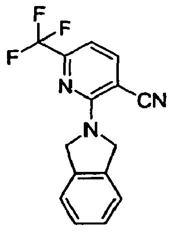

¹H NMR (300 MHz, CDCl₃) δ 7.93 (d, 1 H, *J* = 7.8 Hz), 7.30-7.37 (m, 4H), 6.97 (d, 1 H, *J* = 7.8 Hz), 5.20 (s, 4H); IR (KBr) 2966, 2213,1588, 1480, 1455, 1374, 1232, 1176 cm⁻¹; MS (FAB) *m*/*z* 290(M+H)

### A-20: 3'-Cyano-4-phenyl-6'-trifluoromethyl-3,4,5,6-tetrahydro-2H-[1,2']bipyridinyl-4-carbonsäureethylester

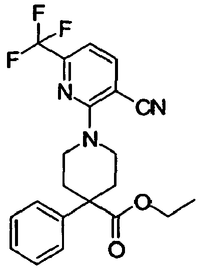

¹H NMR (300 MHz, CDCl₃) δ 8.22 (dd, 1 H, J = 8.1, 0.6 Hz), 7.90 (d, 1 H, J = 8.1 Hz), 7.24-7.42 (m, 5 H), 4.41 (m, 2 H), 4.16 (q, 2 H, J= 7.0 Hz), 3.38 (m, 2 H), 2.73 (m, 2 H), 2.08 (m, 2 H), 1.21 (t, 3 H, J = 7.0 Hz); IR (pur) 2926, 2218, 1725, 1590, 1495, 1456, 1321, 1186, 1148, 1040, 963, 824, 738, 698 cm⁻¹; MS (FAB) *m*/*z* 404 (M+H)

### A-21: 4,6'-Bis-trifluoromethyl-3,4,5,6-tetrahydro-2H-[1,2']bipyridinyl-3'-carbonitril

¹H NMR (300 MHz, CDCl₃) δ 7.94 (d, 1 H, J = 7.8 Hz), 7.07 (d, 1 H, J = 7.8 Hz), 4.62 (m, 2 H), 3.07 (m, 2 H), 2.35 (m, 1 H), 2.03 (m, 2 H), 1.70 (m, 2 H) IR (pur) 2964, 2221, 1591, 1495, 1456, 1394, 1342, 1254, 1147, 1084, 960, 827, 744, 697 cm⁻¹; MS (FAB) *m*/*z* 324 (M+H)

### A-22: 4-Methoxymethyl-6'-trifluoromethyl-3,4,5,6-tetrahydro-2H-[1,2']bipyridinyl-3'-carbonitril

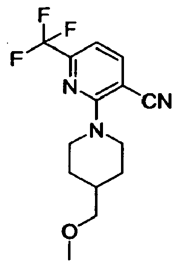

¹H NMR (300 MHz, CDCl₃) δ 7.88 (d, 1 H, J = 7.8 Hz), 6.97 (d, 1 H, J = 7.8 Hz), 4.57 (m, 2 H), 3.35 (s, 3 H), 3.27 (d, 2 H, J = 6.0 Hz), 3.07 (m, 2 H), 1.87 (m, 2 H), 1.28-1.45 (m, 3 H); IR(pur) 2951, 2237, 1590, 1465, 1431, 1349, 1269, 1188, 1150, 1117, 969, 842, 743 cm⁻¹; MS (FAB) *m*/*z* 300 (M+H)

### A-23: 2-(4-p-Tolyl-piperazin-1-yl)-6-trifluoromethyl-nicotinonitril

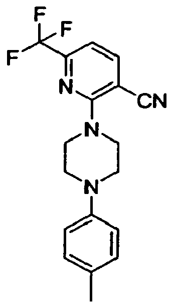

¹H NMR (300 MHz, CDCl₃) δ 7.93 (d, 1 H, J = 7.5 Hz), 7.11 (d, 2 H, J = 8.4 Hz), 7.05 (d, 1 H, J = 7.5 Hz), 6.88 (d, 2 H, J = 8.4 Hz), 4.00 (m, 4 H), 3.28 (m, 4 H), 2.28 (s, 3 H); IR (pur) 2918, 2219, 1590, 1513, 1449, 1381, 1319, 1236, 1186, 1147, 1086, 1044, 970, 815, 743, 703 cm⁻¹; MS (FAB) *m*/*z* 347 (M+H)

### A-24: 2-(4-m-Tolyl-piperazin-1-yl)-6-trifluoromethyt-nicotinonitril

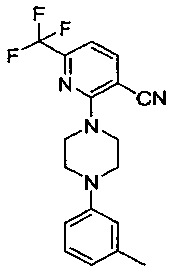

¹H NMR (300 MHz, CDCl₃) δ 7.94 (d, 1 H, J = 7.8 Hz), 7.19 (t, 1 H, J = 7.5 Hz), 7.06 (d, 1 H, J = 7.8 Hz), 6.72-6.78 (m, 3 H), 4.00 (m, 4 H), 3.33 (m, 4 H), 2.34 (s, 3 H) IR (pur) 2830, 2214, 1591, 1487, 1320, 1345, 1184, 1140, 1088, 967, 816, 770, 694 cm⁻¹; MS (FAB) *m*/*z* 347 (M+H)

### A-25: 2-[4-(4-Methoxy-phenyl)-piperazin-1-yl]-6-trifluoromethyl-nicotinonitril

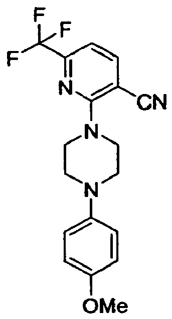

¹H NMR (300 MHz, CDCl₃) δ 7.93 (d, 1 H, J = 7.8 Hz), 7.05 (d, 1 H, J = 7.8 Hz), 6.94 (d, 2 H, J = 6.9 Hz), 6.86 (d, 2 H, J = 6.9 Hz), 4.00 (m, 4 H), 3.77 (s, 3 H), 3.21 (m, 4 H); IR (pur) 2832, 2219, 1590, 1510, 1448, 1319, 1241, 1184, 1146, 1085, 1035, 970, 825, 743, 702 cm⁻¹; MS (FAB) *m*/*z* 363 (M+H)

### A-26: 6-Trifluoromethyl-2-[4-(4-trifl uoromethyl-phenyl)-piperazin-1-yl]-nicotinoniteil

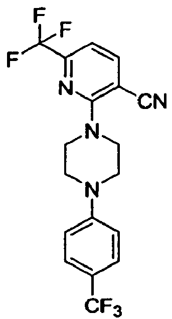

¹H NMR (300 MHz, CDCl₃) δ 7.96 (d, 1 H, J = 8.1 Hz), 7.52 (d, 2 H, J = 8.7 Hz), 7.09 (d, 1 H, J = 8.1 Hz), 6.94 (d, 2 H, J = 8.7 Hz), 4.01 (m, 4 H), 3.46 (m, 4 H); IR(pur) 2923, 2220, 1685, 1594, 1509, 1455, 1344, 1318, 1233, 1186, 1147, 1089, 965, 818 cm⁻¹; MS (FAB) *m*/*z* 401 (M+H)

### A-27: 6-Trifluoromethyl-2-[4-(3-trifluoromethyl-pyridin-2-yl)-piperazin-1-yl]-nicotinonitril

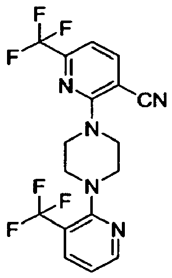

¹H NMR (300 MHz, CDCl₃) δ 8.22 (d, 1 H, J = 3.3 Hz), 7.93 (d, 1 H, J = 7.5 Hz), 7.63 (d, 1 H, J = 7.5 Hz), 7.06 (d, 1 H, J = 9.0 Hz), 6.89 (m, 1 H), 4.01 (m, 4 H), 3.53 (m, 4 H); IR (pur) 2856, 2216, 1589, 1441, 1375, 1344, 1312, 1234, 1148, 1097, 1023, 969, 832 cm⁻¹; MS (FAB) *m*/*z* 402 (M+H)

### A-28: 2-Imidazol-1-yl-6-tritluoromethyl-nicotinonitril

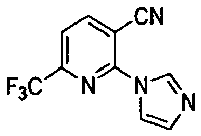

¹H NMR (300 MHz, CDCl₃) δ 8.62 (s, 1 H), 8.38 (d, 1 H, *J* = 7.9 Hz), 8.00 (s, 1 H), 7.76 (d, 1 H, *J* = 7.9 Hz), 7.29 (s, 1 H); IR (pur) 3132, 2228, 1574, 1479, 1440, 1339, 1304, 1245, 1191, 1151, 1102, 1051, 985, 845, 744, 651 cm⁻¹; MS (FAB) m/z 239(M+H)

### A-29: 2-(4-(3-Chloropyridin-2-yl)piperazin-1-yl)-6-(trifluoromethyl)nicotinonitril

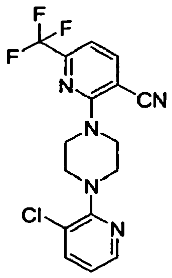

¹H NMR (300 MHz, CDC)₃) δ 8.46 (d, 1 H, J = 4.5 Hz), 7.89-7.95 (m, 2 H), 7.04-7.07 (m, 2 H), 3.99 (m, 4 H), 3.44 (m, 4 H); IR (pur) 2851, 2212, 1568, 1430, 1363, 1332, 1228, 1145, 1105, 962, 851 cm⁻¹; MS (FAB) *m*/*z* 372 (M+H)

### A-30: 2-(4-Cyclohexyl-piperazin-1-yl)-6-trifluoromethyl-nicotinonitril

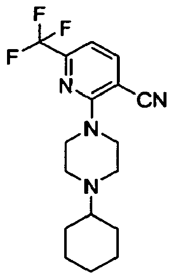

¹H NMR (300 MHz, CDCl₃) δ 7.90 (d, 1 H, J = 7.8 Hz), 6.98 (d, 1 H, J = 7.8 Hz), 3.86 (m, 4 H), 2.70 (m, 4 H), 2.31 (m, 1 H), 1.80 (m, 4 H), 1.20-1.28 (m, 6 H); MS (FAB) *m*/*z* 339 (M+H)

### A-31: 4-Phenyl-6'-trifluoromethyl-3,4,5,6-tetrahydro-2H-[1,2']bipyridinyl-4,3'-dicarbonitril

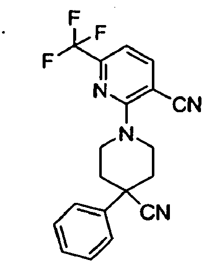

¹H NMR (300 MHz, CDCl₃) δ 7.97 (d, 1 H, J = 7.8 Hz), 7.33-7.53 (m, 5 H), 7.13 (d, 1 H, J = 7.8 Hz), 4.66 (m, 2 H), 3.55 (m, 2 H), 2.15-2.31 (m, 4 H); IR (pur) 2927, 2221, 1590, 1494, 1455, 1381, 1320, 1242, 1145, 1084, 1021, 963, 905, 829, 759, 699 cm⁻¹; MS (FAB) *m*/*z* 357 (M+H)

### A-32: 4-Phenylamino-6'-trifluoromethyl-3,4,5,6-tetrahydro-2H-[1,2']bipyridinyl-3'-carbonitril

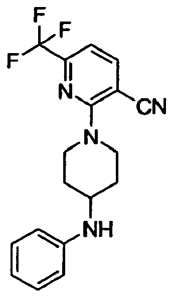

¹H NMR (300 MHz, CDCl₃) δ 7.90̃ (d, 1 H, J = 7.8 Hz), 7.19 (m, 2 H), 7.02 (d, 1 H, J = 7.8 Hz), 6.65 (m, 3 H), 4.42 (m, 2 H), 3.57 (m, 1 H), 3.30 (m, 2 H), 2.22 (m, 2 H), 1.53 (m, 2 H); MS (FAB) *m*/*z* 347 (M+H)

### A-33: 2-Azepan-1-yl-6-trifluoromethyl-nicotinonitril

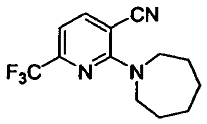

¹H NMR (300 MHz, CDCl₃) δ 7.86 (d ,1 H, *J* = 7.1 Hz), 6.88 (d, 1 H, *J* = 7.7 Hz), 3.84-3.91 (m, 4 H), 1.82-1.94 (m, 4 H), 1.54-1.64 (m, 4 H); IR (pur) 2930, 2215, 1593, 1563, 1508, 1458, 1327, 1246, 1144, 817 cm⁻¹; MS (FAB) *m*/*z* 270 (M+H)

### A-34: N-(3'-Cyano-6'-trifluoromethyl-3,4,5,6-tetrahydro-2H-[1,2']bipyridinyl-4-yl)-N-phenyl-propionamide

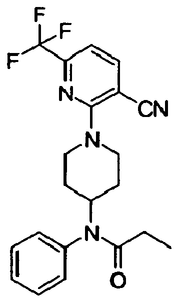

¹H NMR (300 MHz, CDCl₃) δ 7.85 (d, 1 H, J = 7.8 Hz), 7.41 (m, 3 H), 7.11 (m, 2 H), 6.95 (d, 2 H, J = 7.8 Hz), 4.96 (m, 1 H), 4.61 (m, 2 H), 3.14 (m, 2 H), 1.96 (m, 4 H), 1.46 (m, 2 H), 1.03 (t, 3 H, J = 7.5 Hz); MS (FAB) *m*/*z* 403(M+H)

### A-35: 2-(4-Dimethylamino-phenyl)-6-trifluoromethyl-nicotinonitril

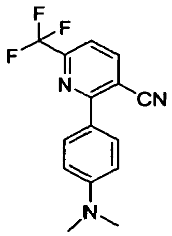

¹H NMR (300 MHz, CDCl₃) δ 8.14(d, 1 H, J = 8.1 Hz), 8.04 (d, 2 H, J = 8.7 Hz), 7.52 (d, 1 H, J = 7.8 Hz), 6.77 (d, 2 H, J = 8.7 Hz), 3.06 (s, 6 H); IR (pur) 2969, 2215, 1571, 1522, 1463, 1409, 1341, 1254, 1132, 1088, 1024, 844, 790, 763 cm⁻¹; MS (FAB) *m*/*z* 292(M+H)

### A-36: 2-(2,6-Dimethyl-morpholin-4-yl)-6-trifluoromethyl-nicotinonitril

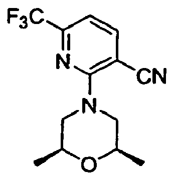

¹H NMR (300 MHz, CDCl₃) δ 7.85 (d, 1 H, J = 7.5 Hz), 6.97 (d, 1 H, J = 7.5 Hz), 4.31 (m, 2 H), 3.68 (m, 2 H), 2.74 (m, 2 H), 1.19 (d, 6 H, J = 6.3 Hz); IR (pur) 2979, 2867, 2220, 1591, 1566, 1452, 1330, 1297, 1240, 1146, 1080, 1008, 967, 828, 745 cm⁻¹; MS (FAB) *m*/*z* 286 (M+H)

### A-37: 2-(1,1-Dioxo-thiomorpholin-4-yl)-6-trifluoromethyl-nicotinonitril

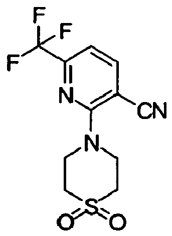

¹H NMR (300 MHz, CDCl₃) δ 8.06 (d, 1 H, J = 7.5 Hz), 7.27 (d, 1 H, J = 7.5 Hz), 4.32 (m, 4 H), 3.23 (m, 4 H); IR(pur) 2923, 2223, 1588, 1455, 1334, 1179, 1126, 1084, 865, 833 cm⁻¹; MS (FAB) *m*/*z* 306 (M+H)

### A-38: 4,6'-Dimethyl-3,4,5,6-tetrahydro-2H-[1,2']bipyridinyl-3'-carbonitril

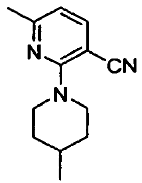

¹H NMR (300 MHz, CDCl₃) δ 7.60̃ (d, 1 H, J = 7.8 Hz), 6.53 (d, 1 H, J = 7.8 Hz), 4.39 (m, 2 H), 2.96 (m, 2 H), 2.41 (s, 3 H), 1.60-1.76 (m, 3 H), 1.35 (m, 2 H), 0.98 (d, 3 H, J = 6.3 Hz); 1 R(pur) 2922, 2847, 2211, 1585, 1556, 1453, 1375, 1331, 1245, 1105, 965, 808, 764 cm⁻¹; MS (FAB) *m*/*z* 216(M+H)

### A-39: 4-(4-Fluoro-phenyl)-6'-trifluoromethyl-3,6-dihydro-2H-[1,2']bipyridinyl-3'-carbonitril

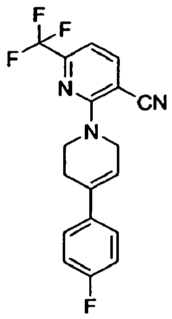

¹H NMR (300 MHz, CDCl₃) δ 7.92 (d, 1 H, J = 7.8 Hz), 7.33-7.40 (m, 2 H), 6.99-7.06 (m, 3 H), 6.07 (m, 1 H), 4.43 (q, 2 H, J = 3.0 Hz), 4.08 (t, 2 H, J = 4.8 Hz), 2.72 (q, 2 H, J = 5.7 Hz); IR(pur) 2923, 2220, 1685, 1594, 1509, 1455, 1344, 1318, 1233, 1186, 1147, 1089, 965, 818 cm⁻¹; MS (FAB) *m*/*z* 348 (M+H)

### A-40: 4-Dimethylamino-4-phenyl-6'-trifluoromethyl-3,4,5,6-tetrahydro-2H-[1,2']bipyridinyl-3'-carbonitril

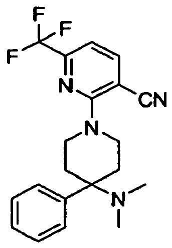

¹H NMR (300 MHz, CDCl₃) δ 7.81 (d, 1 H, J = 8.1 Hz), 7.32-7.37 (m, 2 H), 7.20-7.25 (m, 3 H), 6.89 (d, 1 H, J = 8.1 Hz), 4.05 (m, 2 H), 3.61 (m, 2 H), 2.25 (m, 4 H), 2.02 (s, 6 H); IR(pur) 2945, 2867, 2784, 2217, 1590, 1497, 1452, 1321, 1240, 1146, 1082, 954, 913, 823, 736 cm⁻¹; MS (FAB) *m*/*z* 284(M+H)

### A-41: 2-(4-Methyl-piperidin-1-yl)-4-trifluoromethyl-benzonitril

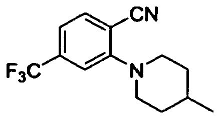

IR (pur) 2924, 2223, 1500,1433, 1319, 1175, 1134, 1080, 1134, 829 cm⁻¹; MS (FAB) *m*/*z* 269 (M+H)

### A-42: 2-Butylamino-6-trifluoromethyl-nicotinonitril

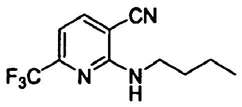

IR (pur) 3359, 2961, 2228, 1602, 1536, 1351, 1277, 1200, 1131, 821 cm⁻¹; MS (FAB) *m*/*z* 244 (M+H)

### A-43: 2,2-Dimethyl-propionsäure-3'-cyano-6'-trifluoromethyl-3,4,5,6-tetrahydro-2H-[1,2']bipyridinyl-4-yl ester

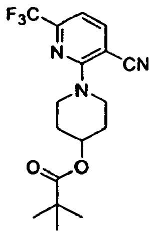

¹H NMR (CDCl₃) δ 7.92 (dd, 1 H, J = 7.9, 0.8 Hz), 7.04 (d, 1 H, J = 7.7 Hz), 5.09-5.04 (m, 1 H), 3.99-3.76 (m, 4 H), 2.06-1.80 (m, 4 H), 1.22 (s, 9 H); IR (pur) 2969, 2232, 1727, 1592, 1459, 1325, 1156, 1029 cm⁻¹

### A-44: Essigsäure 3'-cyano-6'-trifluoromethyl-3,4,5,6-tetrahydro-2H-[1,2'] bipyridinyl-4-yl ester

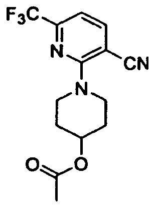

¹H NMR (CDCl₃) δ 7.92 (dd, 1 H, J = 7.9, 0.3 Hz), 7.05 (d, 1 H, J = 7.9 Hz), 5.06 (m, 1 H), 4.11-4.03 (m, 2 H), 3.70-3.62 (m, 2 H), 2.10-2.00 (m, 2 H), 2.09 (s, 3 H), 1.87-1.76 (m, 2 H); IR (pur) 2959, 2220, 1736, 1591, 1459, 1243, 1146, 1029 cm⁻¹

### A-45: 4-Methoxy-6'-trifluoromethyl-3,4,5,6-tetrahydro-2H-[1,2']bipyridinyl-3'-carbonitril

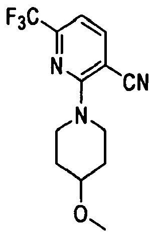

¹H NMR (CDCl₃) δ 7.90 (d, 1 H, J = 7.9 Hz), 7.00 (d, 1 H, J = 7.7 Hz), 4.12-4.05 (m, 2 H), 3.65-3.49 (m, 3 H), 3.39 (s, 3 H), 2.04-1.97 (m, 2 H), 1.80-1.73 (m, 2 H); IR (pur) 2934, 2219, 1591, 1498, 1458, 1325, 1187, 1146 cm⁻¹

### A-46: 4-Butoxy-6'-trifluoromethyl-3,4,5,6-tetrahydro-2H-[1,2']bipyridinyl-3'-carbonitril

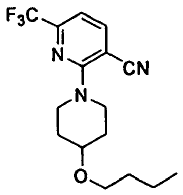

¹H NMR (CDCl₃) δ 7.89 (dd, 1 H, J = 7.7, 0.7 Hz), 6.99 (d, 1 H, J = 7.7 Hz), 4.13-4.05 (m, 2 H), 3.64-3.55 (m, 3 H), 3.48 (t, 2 H, J = 6.4 Hz), 2.02-1.93 (m, 2 H), 1.79-1.68 (m, 2 H), 1.62-1.53 (m, 2 H), 1.45-1.33 (m, 2 H), 0.93 (t, 3 H, J = 7.5 Hz); IR (pur) 2956, 2219, 1592, 1499, 1458, 1324, 1187, 1147, 959 cm⁻¹

### A-47: 4-Isopropoxy-6'-trifluoromethyl-3,4,5,6-tetrahydro-2H-[1,2']bipyridinyl-3'-carbonitril

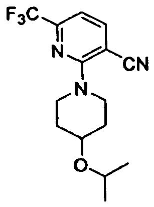

¹H NMR (CDCl₃) δ 7.89 (dd, 1 H, J = 7.7, 0.7 Hz), 6.99 (d, 1 H, J = 7.9 Hz), 4.18-4.10 (m, 2 H), 3.81-3.64 (m, 2 H), 3.60-3.52 (m, 2 H), 2.00-1.91 (m, 2 H), 1.76-1.65 (m, 2 H), 1.18 (d, 6 H, J = 6.1 Hz); IR (pur) 2971, 2220, 1592, 1499, 1458, 1324, 1236, 1185, 1147, 1039 cm⁻¹

### A-48: 4-Ethoxy-6'-trifluoromethyl-3,4,5,6-tetrahydro-2H-[1,2']bipyridinyl-3'-carbonitril

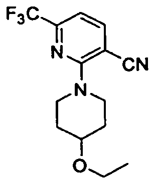

¹H NMR (CDCl₃) δ 7.89 (dd, 1 H, J = 7.9, 0.7 Hz), 6.99 (d, 1 H, J = 7.7 Hz), 4.18-4.10 (m, 2 H), 3.64-3.51 (m, 5 H), 2.04-1.95 (m, 2 H), 1.79-1.68 (m, 2 H), 1.23 (t, 3 H, J = 7.1 Hz); IR (pur) 2931, 2219, 1592, 1497, 1458, 1326, 1186, 1146, 1078 cm⁻¹

### A-49: 4-Methylen-6'-trifluoromethyl-3,4,5,6-tetrahydro-2H-[1,2']bipyridinyl-3'-carbonitril

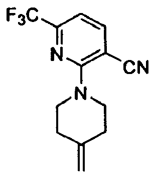

¹H NMR (CDCl₃) δ 7.91 (d, 1 H, J = 7.9Hz), 7.01 (d, 1 H, J = 7.9 Hz), 4.83 (s, 2 H), 3.85 (t, 4 H, J = 5.7 Hz), 2.39 (t, 4 H, J = 5.9Hz); IR (pur) 2946, 2220, 1591, 1495, 1458, 1333, 1238, 1191, 1147, 1088 cm⁻¹

### A-50: 2-(6-Aza-spiro[2.5]oct-6-yl)-6-trifluoromethyl-nicotinonitril

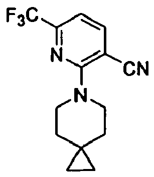

¹H NMR (CDCl₃) δ 7.88 (d, 1 H, J = 7.7 Hz), 6.97 (d, 1 H, J = 7.7 Hz), 3.87 (m, 4 H), 1.53 (m, 4 H), 0.40 (s, 4 H); IR (pur) 2925, 2219, 1591, 1496, 1457, 1332, 1237, 1189, 1147, 960 cm⁻¹

### A-51: 3-Methyl-6'-trifluoromethyl-3,4,5,6-tetrahydro-2H-[1,2']bipyridinyl-3'-carbonitril

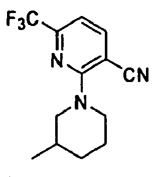

¹H NMR (CDCl₃) δ 7.87 (d, 1 H, J = 7.7 Hz), 6.95 (d, 1 H, J = 7.9 Hz), 4.47-4.36 (m, 2 H), 3.09-3.00 (m, 1 H), 2.79-2.71 (m, 1 H), 1.92-1.60 (m, 4 H), 1.27-1.14 (m, 1 H), 0.97 (d, 3 H, J = 6.6 Hz); IR (pur) 2930, 2219, 1592, 1565, 1499, 1457, 1320, 1240, 1187, 1147 cm⁻¹

### A-52: 2-Methyl-6'-trifluoromethyl-3,4,5,6-tetrahydro-2H-[1,2']bipyridinyl-3'-carbonitril

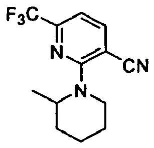

¹H NMR (CDCl₃) δ 7.87 (d, 1 H, J = 7.7 Hz), 6.93 (d, 1 H, J = 7.9 Hz), 4.85 (m, 1 H), 4.34 (m, 1 H), 3.23 (m, 1 H), 1.80-1.55 (m, 6 H), 1.33 (d, 3 H, J = 6.8 Hz); IR (pur) 2941, 2218, 1592, 1485, 1343, 1189, 1147, 1074 cm⁻¹

### A-53: 4-[(3-Cyano-6-trifluoromethyl-pyridin-2-ylamino)-methyl]-piperidin-1-carbonsäure tert-butyl ester

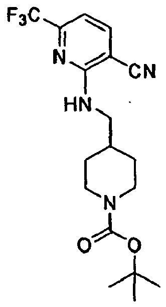

¹H NMR (CDCl₃) δ 7.80 (d, 1 H, J = 7.7 Hz), 6.95 (d, 1 H, J = 7.7 Hz), 5.45 (m, 1 H), 4.11 (m, 2 H), 3.48 (m, 2 H), 2.70 (m, 2 H), 1.80-1.65 (m, 3 H), 1.46 (s, 9 H), 1.25-1.13 (m, 2 H); IR (pur) 3369, 2926, 2223, 1685, 1599, 1533, 1424, 1281, 1178, 1146 cm⁻¹

### A-54: 4-Oxo-6'-trifluoromethyl-3,4,5,6-tetrahydro-2H-[1,2']bipyridinyl-3'-carbonitril

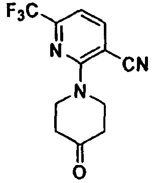

¹H NMR (CDCl₃) δ 8.00 (d, 1 H, J = 7.9 Hz), 7.15 d, 1 H, J = 7.9 Hz), 4.13 (t, 4 H, J = 6.0 Hz), 2.66 (t, 4 H, J = 6.2 Hz); IR (pur) 2976, 2221, 1713, 1567, 1460, 1338, 1236, 1187, 1143, 1099 cm⁻¹

### A-55: 6"-Trifluoromethyl-3,4,5,6,3',4',5',6'-octahydro-2H,2'H-[1,4';1',2"]terpyridine-3"-carbonitril

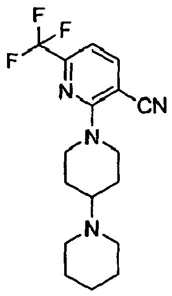

¹H NMR (300 MHz, CDCl₃) δ 7.88 (d, 1 H, *J* = 7.7 Hz), 6.98 (d, 1 H, *J* = 7.89 Hz), 4.61 (d, 2 H, *J* = 13 Hz), 3.08 (dd, 2 H, J = 13.4, 13.4 Hz), 2.58-2.51 (m, 5 H, *J* = 4.8 Hz), 1.97 (d, 2 H, *J* = 12.1 Hz), 1.72-1.56 (m, 6 H), 1.45 (d, 2 H, *J* = 5.3 Hz); IR (pur) 2854, 2218, 1336, 1240, 958, 822 cm⁻¹; MS (FAB) *m*/*z* 339 (M+H)

### A-56: 4-Pyrrolidin-1-yl-6'-trifluoromethyl-3,4,5,6-tetrahydro-2H-[1,2']bipyridinyl-3'-carbonitril

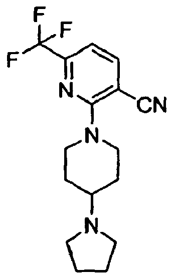

¹H NMR (300MHz, CDCl₃) δ 7.88 (d, 1 H, *J* = 7.9 Hz), 6.98 (d, 1 H, *J* = 7.9 Hz), 4.51 (d, 2 H, *J* = 13.0 Hz), 3.23-3.13 (m, 2 H), 2.60 (s, 4 H), 2.33-2.25 (m, 1 H), 2.05-2.01 (m, 2 H), 1.83-1.78 (m, 4 H), 1.71-1.59 (m, 2 H); IR(pur) 2959, 2219, 1238, 1083, 960, 824, 743 cm⁻¹; MS (FAB) *m*/*z* 325(M+H)

### A-57: 4-Morpholin-4-yl-6'-trifluoromethyl-3,4,5,6-tetrahydro-2H-[1,2']bipyridinyl-3'-carbonitril

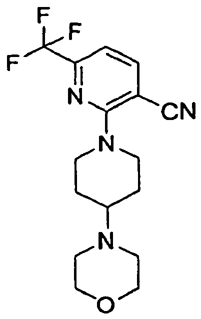

¹H NMR (300MHz, CDCl₃) δ 7.91 (d, 1 H, *J* = 7.9 Hz), 7.01 (d, 1 H, *J* = 7.7 Hz), 4.58 (d, 2 H, *J* = 13.2 Hz), 3.75-3.70 (m, 5 H), 3.15-3.06 (m, 2 H), 2.61-2.45 (m, 4 H), 2.01 (d, 2 H, *J* = 11.5 Hz), 1.69-1.56 (m, 2 H); IR (pur) 2956, 2855, 2218, 1236, 1027, 958, 876 cm⁻¹; MS (FAB) *m*/*z* 341 (M+H)

### A-58: 4-Ethyl-6'-trifluoromethyl-3,4,5,6-tetrahydro-2H-[1,2']bipyridinyl-3'-carbonitril

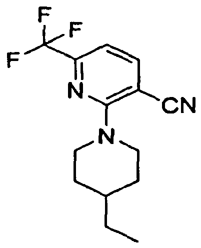

¹H NMR (400MHz, CDCl₃) δ 7.85 (d, 1 H, *J* = 7.6 Hz), 6.93 (d, 1 H, *J* = 7.6 Hz), 4.53 (d, 2 H, *J* =13.2 Hz), 3.02 (dd, 2 H, *J* = 13.2, 13.2 Hz), 1.82 (d, 2 H, *J* =12.4 Hz), 1.45-1.42 (m, 1 H), 1.33-1.28 (m, 4 H), 0.90 (t, 3 H, *J* = 7.2 Hz); IR(pur)) 2854, 2218, 1008, 911, 841, 744 cm⁻¹; MS (FAB) *m*/*z* 284(M+H)

### A-59: 4-Benzyl-6'-trifluoromethyl-3,4,5,6-tetrahydro-2H-[1,2]bipyridinyl-3'-carbonitril

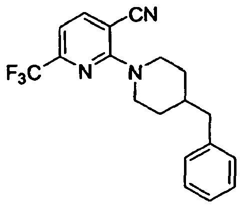

¹H NMR (300 MHz, CDCl₃) δ 7.87 (d, 1 H, *J* = 7.9 Hz), 7.35-7.22 (m, 5 H), 6.96 (d, 1 H, *J* = 7.7 Hz), 4.54 (m, 2 H), 3.00 (td, 2 H, *J* = 6.7, 2.4 Hz), 2.59 (d, 2 H, *J* = 6.8 Hz), 1.88-1.83 (m, 3 H), 1.39 (m, 2 H); IR (pur) 2921, 2230, 1590, 1498, 1455, 1320, 1240, 1145, 958, 745, 701 cm⁻¹; MS (FAB) *m*/*z* 346 (M+H)

### A-60: 2-(3,4-Dimethyl-phenylamino)-6-trifluoromethyl-nicotinonitril

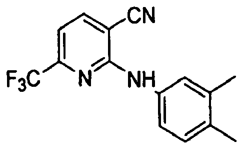

¹H NMR (300 MHz, CDCl₃), δ 7.92 (d, 1 H, *J* = 7.9 Hz), 7.43 (dd, 1 H, *J* = 8.1, 2.3 Hz), 7.38 (d, 1 H, *J* = 2.2 Hz), 7.14 (d, 1 H, *J* = 8.1 Hz), 7.10 (d, 1 H, *J* = 7.7 Hz), 2.28 (s, 3 H), 2.26 (s, 3 H); IR (pur) 3315, 2922, 2228, 1595, 1532, 1453, 1428, 1350, 1271, 1199, 1141, 968, 820 cm⁻¹; MS (FAB) *m*/*z* 292 (M+H)

### A-61: 2-(5-Chloro-2-methyl-phenylamino)-6-trifluoromethyl-nicotinonitril

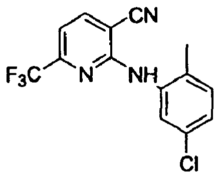

¹H NMR (300 MHz, CDCl₃) δ 8.12 (d, 1 H, *J* = 2.2 Hz), 7.98 (d, 1 H, *J* = 7.9 Hz), 7.18 (d, 2 H, *J* = 7.9 H), 7.10 (dd, 1 H, *J* = 8.1, 2.2 Hz), 2.32 (s, 3 H); IR (pur) 3424, 2231, 1589, 1536, 1452, 1349, 1273, 1189, 1136, 960, 899, 837, 802 cm⁻¹; MS (FAB) *m*/*z* 312 (M+H)

### A-62: 2-(4-Chloro-benzylamino)-6-trifluoromethyl-nicotinonitril

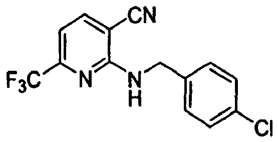

¹H NMR (300 MHz, CDCl₃) δ 7.88 (d, 1 H, *J* = 7.7 Hz), 7.33 (s, 4 H), 6.99 (d, 1 H, *J* = 7.8 Hz), 5.72 (bs, 1 H), 4.69 (d , 2 H, *J* = 5.7 Hz); IR (pur) 3372, 2221, 1598, 1531, 1404, 1349, 1278, 1136, 907, 823, 793 cm⁻¹; MS (FAB) *m*/*z* 312 (M+H)

### A-63: 2-(4-Fluoro-phenylamino)-6-trifluoromethyl-nicotinonitril

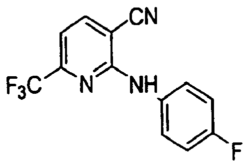

¹H NMR (300 MHz, CDCl₃) δ 7.96 (d, 1 H, *J* = 7.9 Hz), 7.65-7.57 (m, 2 H), 7.16-7.08 (m, 3 H); IR (pur) 3362, 2226, 1619, 1592, 1546, 1508, 1463, 1435, 1350, 1271, 1194, 1142, 961, 831 cm⁻¹; MS (FAB) *m*/*z* 282 (M+H)

### A-64: 2-(4-Chloro-phenylamino)-6-trifluoromethyl-nicotinonitril

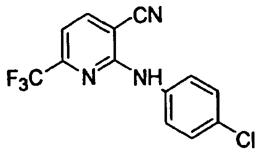

¹H NMR (300 MHz, CD₃OD) δ 7.98 (d, 1 H, *J* = 7.9 Hz), 7.61 (d, 2 H, *J* = 9.0 Hz), 7.35 (d, 2 H, *J* = 8.8 Hz), 7.18 (d, 1 H, *J* = 7.9 Hz); IR (pur) 2230, 1614, 1538, 1490, 1435, 1312, 1262, 1173, 1138, 827, 696 cm⁻¹; MS (FAB) *m*/*z* 298 (M+H)

### A-65: 2-Phenylamino-6-trifluoromethyl-nicotinonitril

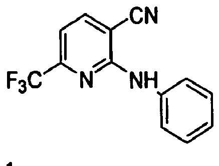

¹H NMR (300 MHz, CD₃OD) δ 7.96 (d, 1 H, *J* = 7.7 Hz), 7.66 (d, 2 H, *J* = 8.8 Hz), 7.40 (t, 2 H, *J* = 7.5 Hz), 7.16 (m, 2 H); IR (pur) 3341, 2230, 1611, 1539, 1496, 1446, 1413, 1350, 1271, 1195, 1139, 959, 828, 752, 691 cm⁻¹; MS (FAB) *m*/*z* 264 (M+H)

### A-66: 2-Azepan-1-yl-4-trifluoromethyl-benzonitril

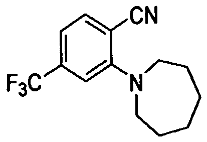

¹H NMR (300 MHz, CDCl₃) δ 7.56 (d, 1 H, *J* = 8.1 Hz), 7.03 (s, 1 H), 6.90 (d, 1 H, *J* = 8.1 Hz), 3.74-3.63 (m, 4 H), 1.98-1.83 (m, 4 H), 1.69-1.51 (m, 4 H); IR (pur) 2931, 2213, 1616, 1560, 1503, 1444, 1316, 1171, 1131, 1081, 1001, 939, 859, 810 cm⁻¹; MS (FAB) *m*/*z* 269 (M+H)

### A-67: 2-(4-Pyridin-4-yl-piperazin-1-yl)-6-trifluoromethyl-nicotinonitril

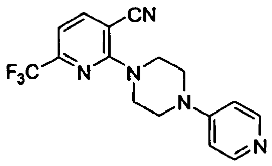

¹H NMR (300 MHz, CDCl₃) δ 8.33 (d, 2H, *J* = 6.4 Hz), 7.98 (d, 1 H, *J* = 7.9 Hz), 7.12 (d, 1 H, *J* = 7.9 Hz), 6.69 (d, 2 H, *J* = 6.6 Hz), 4.08-4.36 (m , 4 H), 3.58-3.45 (m, 4 H); IR (pur) 2917, 2230, 1592, 1481, 1445, 1390, 1321, 1236, 1139, 867, 804, 740 cm⁻¹; MS (FAB) *m*/*z* 334 (M+H)

### A-68: 2-[4-(4-Fluoro-phenyl)-piperazin-1-yl]-6-trifluoromethyl-nicotinonitril

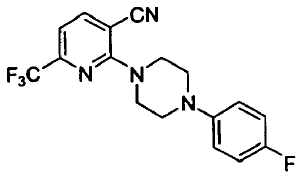

¹H NMR (300 MHz, CDCl₃) δ 7.85 (d, 1 H, *J* = 7.7 Hz), 7.08 (d , 1 H, *J* = 7.7 Hz), 7.12-6.93 (m, 4 H), 4.14-4.00 (m, 4 H), 3.32-3.21 (m, 4 H); IR (pur) 2828, 2219, 1590, 1509, 1449, 1319, 1234, 1185, 1147, 1086, 970, 824, 743, 704 cm⁻¹; MS (FAB) *m*/*z* 351 (M+H)

### A-69: 2-[4-(2-Fluoro-phenyl)-piperazin-1-yl]-6-trifluoromethyl-nicotinonitril

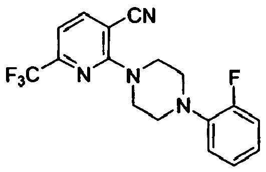

¹H NMR (300 MHz, CDCl₃) δ 7.95 (d, 1 H, *J* = 7.9 Hz), 7.07 (d, 1 H, *J* = 7.9 Hz), 7.06-6.96 (m, 4 H), 4.12-4.01 (m, 4 H), 3.34-3.22 (m, 4 H); IR (pur) 2851, 2219, 1590, 1501, 1448, 1380, 1344, 1319, 1238, 1185, 1146, 1086, 970, 820, 754 cm⁻¹; MS (FAB) *m*/*z* 351 (M+H)

### A-70: 2-(4-Phenyl-piperazin-1-yl)-6-trifluoromethyl-nicotinonitril

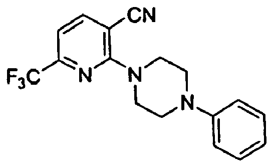

¹H NMR (300 MHz, CDCl₃) δ 7.94 (d, 1 H, *J* = 7.7Hz), 7.35-7.28 (m, 2 H), 7.06 (d, 1 H, *J* = 7.9 Hz), 6.99-6.87 (m, 3 H), 4.06-3.98 (m, 4 H), 3.42-3.37 (m, 4 H); IR (pur) 2850, 2219, 1591, 1496, 1448, 132, 1233, 1185, 1146, 1086, 970, 825, 759, 694 cm⁻¹; MS (FAB) *m*/*z* 333 (M+H)

### A-71: 2-(Methyl-phenyl-amino)-6-trifluoromethyl-nicotinonitril

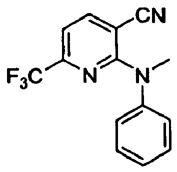

¹H NMR (300 MHz, CDCl₃) δ 7.79 (d, 1 H, *J* = 7.7 Hz), 7.47 (m, 2 H), 7.29 (m, 2 H), 7.03 (d, 1 H, *J* = 7.8 Hz), 6.78 (m, 1 H), 3.54 (s, 3 H); IR (pur) 2920, 2230, 1587, 1495, 1402, 1345, 1315, 1251, 1193, 1145, 942, 826, 745, 698 cm⁻¹; MS (FAB) *m*/*z* 278 (M+H)

### A-72: 4,4-Dimethyl-6'-trifluoromethyl-3,4,5,6-tetrahydro-2H-[1,2']bipyridinyl-3'-carbonitril

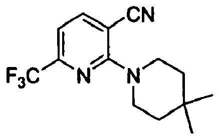

¹H NMR (300 MHz, CDCl₃) δ 7.87 (d, 1 H, *J* = 7.9 Hz), 6.96 (d, 1 H, *J* = 7.9 Hz), 3.87-3.73 (m, 4 H), 1.61-1.46 (m, 4 H), 1.03 (s, 6 H); IR (pur) 2924, 2218, 1591, 1566, 1498, 1463, 1346, 1320, 1241, 1182, 1147, 1082, 956, 823, 744 cm⁻¹; MS (FAB) *m*/*z* 284 (M+H)

### A-73: 2-(4-p-Tolyl-piperazin-1-yl)-4-trifluoromethyl-benzonitril

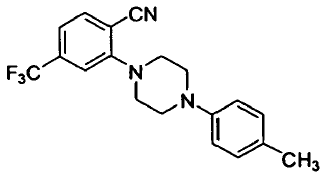

¹H NMR (300 MHz, CDCl₃) δ 7.71 (d, 1 H, *J* = 8.1 Hz), 7.26 (d, 1 H, *J* = 7.9 Hz), 7.25 (s, 1 H), 7.11 (d, 2 H, *J* = 8.4 Hz), 6.91 (d, 2 H, *J* = 8.6 Hz), 3.52-3.41 (m, 4 H), 3.43-3.37 (m, 4 H), 2.29 (s, 3 H); IR (pur) 2838, 2227, 1615, 1517, 1432, 1308, 1240, 1178, 1121, 1079, 963, 809 cm⁻¹; MS (FAB) *m*/*z* 346 (M+H)

### A-74: 2-(4-m-Tolyl-piperazin-1-yl)-4-trifluoromethyl-benzonitril

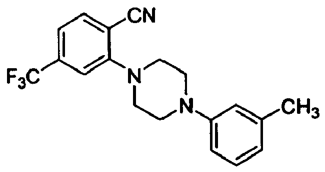

¹H NMR (300 MHz, CDCl₃) δ 7.71 (d, 1 H, *J* = 8.4 Hz), 7.26 (d, 1 H, *J* = 7.7 Hz), 7.24 (s, 1 H), 7.19 (t , 1 H, *J* = 7.9 Hz), 6.81 (s, 1 H), 6.80 (d , 1 H, *J* = 7.1 Hz), 6.74 (d, 1 H, *J* = 7.7 Hz), 3.49-3.31 (m, 8 H), 2.34 (s, 3 H); IR (pur) 2837, 2231, 1605, 1497, 1432, 1311, 1252, 1174, 1133, 1078, 964, 829, 777 cm⁻¹; MS (FAB) *m*/*z* 346 (M+H)

### A-75: 4-Trifluoromethyl-2-[4-(4-trifluoromethyl-phenyl)-piperazin-1-yl]-benzonitril

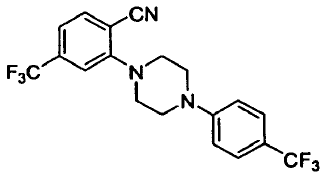

¹H NMR (300 MHz, CDCl₃) δ 7.72 (d, 1 H, *J* = 7.9 Hz), 7.53 (d, 2 H, *J* = 8.8 Hz), 7.29 (d, 1 H, *J* = 8.0 Hz), 7.25 (s, 1 H), 6.99 (d, 2 H, *J* = 8.6 Hz), 3.57-3.41 (m, 8 H); IR (pur) 2842, 2225, 1615, 1527, 1501, 1432, 1388, 1332, 1235, 1116, 1073, 962, 827, 735 cm⁻¹; MS (FAB) *m*/*z* 400 (M+H)

### A-76: 2-[4-(4-Methoxy-phenyl)-piperazin-1-yl]-4-trifluoromethyl-benzonitril

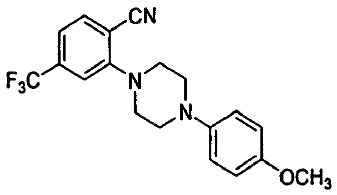

¹H NMR (300 MHz, CDCl₃) δ 7:63 (d, 1 H, *J* = 7.9 Hz), 7.19 (d, 1 H, *J* = 7.6 Hz), 7.18 (s, 1 H), 6.93-6.82 (m, 4 H), 3.72 (s, 3 H), 3.43-3.35 (m, 4 H), 3.28-3.21 (m, 4 H); IR (pur) 2962, 2837, 2228, 1515, 1432, 1306, 1261, 1176, 1117, 1036, 962, 821 cm⁻¹; MS (FAB) *m*/*z* 362 (M+H)

### A-77: 2-[4-(4-Fluoro-phenyl)-3,6-dihydro-2H-pyridin-1-yl]-4-trifluoromethyl-benzonitril

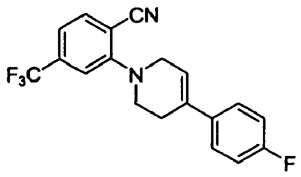

¹H NMR (300 MHz, CDCl₃) δ 7.68 (d, 1 H, *J* = 8.0 Hz), 7.40 (m, 2 H), 7.22 (s, 1 H), 7.18 (d, 1 H, *J* = 8.0 Hz), 7.05 (m, 2 H), 6.11 (m, 1 H), 3.98 (bq, 2 H, *J* = 3.1 Hz), 3.71 (t, 2 H, *J* = 5.5 Hz), 2.79 (m, 2 H); IR (pur) 2919, 1683, 1601, 1509, 1440, 1332, 1229, 1173, 1134, 838 cm⁻¹; MS (FAB) *m*/*z* 347 (M+H)

### A-78: 2-(Butyl-methyl-amino)-6-trifluoromethyl-nicotinonitril

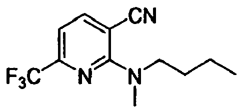

¹H NMR (300 MHz, CDCl₃) δ 7.86 (d, 1 H, *J* = 7.9 Hz), 6.89 (d, 1 H, *J* = 7.7 Hz), 3.72 (t, 2 H, *J* = 7.7 Hz), 3.33 (s, 3 H), 1.60-1.75 (m, 2 H), 1.30-1.46 (m, 2 H), 0.96 (t, 3 H, *J* = 7.4 Hz); IR (pur) 2962, 2230, 1594, 1517, 1417, 1328, 1239, 1186, 1147, 818 cm⁻¹; MS (FAB) *m*/*z* 258 (M+H)

### A-79: 2-(4-Phenyl-piperazin-1-yl)-4-trifluoromethyl-benzonitril

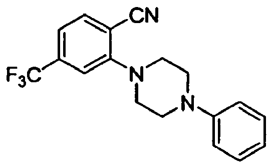

¹H NMR (300 MHz, CDCl₃) δ 7.70 (d, 1 H, *J* = 8.2 Hz), 7.23-7.33 (m, 4 H), 6.87-7.02 (m, 3 H), 3.35-3.50 (m, 8 H); IR (KBr) 2834, 2224, 1600, 1499, 1432, 1311, 1229, 1174, 1132, 1078, 962, 878, 828, 760 cm⁻¹; MS (FAB) *m*/*z* 332 (M+H)

### A-80: 2-Azocan-1-yl-4-trifluoromethyl-benzonitril

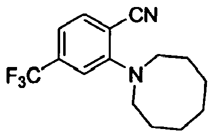

¹H NMR (300 MHz, CDCl₃) δ 7.56 (d, 1 H, *J* = 8.1 Hz), 7.00 (s , 1 H), 6.86 (dd , 1 H, *J* = 8.2, 1.3 Hz), 3.7.1-3.79 (m , 4 H), 1.79-1.91 (m, 4 H), 1.50-1.69 (m , 6 H); IR (pur) 2926, 2223, 2210, 1617, 1558, 1505, 1446, 1317, 1171, 1131, 1078, 989, 808 cm⁻¹; MS (FAB) *m*/*z* 283 (M+H)

### A-81: 2-(4,4-Dimethyl-piperidin-1-yl)-4-trifluoromethyl-benzonitril

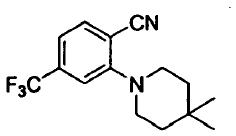

¹H NMR (300 MHz, CDCl₃) δ 7.64 (d, 1 H, *J* = 7.9 Hz), 7.21 (s, 1 H), 7.16 (d, 1 H, *J* = 7.9 Hz), 3.22-3.29 (m, 4H), 1.55-1.64 (m, 4 H), 1.03 (s, 6 H); IR (pur) 2954, 2223, 1612, 1567, 1500, 1431, 1347, 1311, 1239, 1173, 1134, 1078, 952, 874, 825 cm⁻¹; MS (FAB) *m*/*z* 283 (M+H)

### A-82: 2-(4-Ethyl-piperidin-1-yl)-4-trifluoromethyl-benzonitril

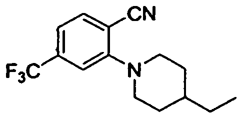

¹H NMR (300 MHz, CDCl₃) δ 7.64 (d, 1 H, *J* = 7.9 Hz), 7.13-7.21 (m, 2 H), 3.62-3.73 (m, 2 H), 2.79-2.92 (m, 2 H), 1.81-1.90 (m, 2 H), 1.25-1.55 (m, 5 H), 0.94 (t, 3 H, *J* = 7.0 Hz); IR (pur) 2930, 2224, 1612, 1567, 1500, 1433, 1312, 1247, 1216, 1174, 1133, 1078, 953, 877, 825 cm⁻¹; MS (FAB) *m*/*z* 283 (M+H)

### A-83: 2-Dipropylamino-4-trifluoromethyl-benzonitril

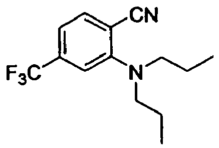

¹H NMR (300 MHz, CDCl₃) δ 7.58 (d, 1 H, *J* = 8.0 Hz), 7.04 (s, 1 H), 6.97 (d, 1 H, *J* = 8.0 Hz), 3.35-3.42 (m, 4 H), 1.58-1.72 (m, 4 H), 0.89-0.97 (m, 6 H) IR (pur) 2966, 2223, 1616, 1561, 1505, 1447, 1320, 1230, 1173, 1133, 1078, 992, 813 cm⁻¹; MS (FAB) *m*/*z* 271 (M+H)

### A-84: 4-Trifluoromethyl-2-(4-trifluoromethyl-piperidin-1-yl)-benzonitril

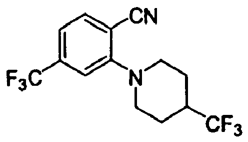

H NMR (300 MHz, CDCl₃) δ 7.69(d, 1 H, *J* = 8.0 Hz), 7.18-7.29 (m, 2 H), 3.69-3.79 (m, 2 H), 2.83-2.93 (m, 2 H), 2.22 (m , 1 H), 1.99-2.10 (m, 2 H), 1.81-1.99 (m, 2 H); IR (pur) 2963, 2230, 1613, 1500, 1435, 1391, 1336, 1311, 1256, 1139, 1080, 955, 900, 830 cm⁻¹; MS (FAB) *m*/*z* 323 (M+H)

### A-85: 2-(4-Benzyl-piperidin-1-yl)-4-trifluoromethyl-benzonitril

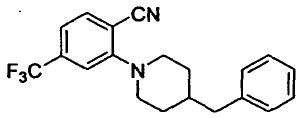

¹H NMR (400 MHz, CDCl₃) δ 7.80̃ (dd, 1 H, *J* = 7.6, 7.6 Hz), 7.64 (d, 1 H, *J* = 8.4 Hz), 7.56 (d, 1 H, *J* = 8.4 Hz), 7.52 (d, 1 H, *J* = 8.4 Hz), 7.27-7.32 (m, 2 H), 7.15-7.24 (m, 2 H), 3.61-3.66 (m, 2 H), 2.77-2.86 (m, 2 H), 2.62 (d, 2 H, *J* = 7.2 Hz), 1.78-1.85 (m, 2 H), 1.72 (m, 1H). 1.49-1.60 (m, 2 H); IR (pur) 2922, 2230, 1612, 1499, 1434, 1312, 1174, 1133, 1077, 953, 827, 746, 701 cm⁻¹; MS (FAB) *m*/*z* 345 (M+H)

### A-86: 4-Acetyl-4-phenyl-6'-trifluoromethyl-3,4,5,6-tetrahydro-2H-[1,2']bipyridinyl-3'-carbonitril

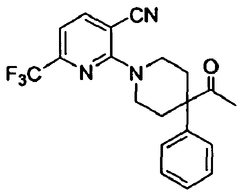

¹H NMR (300 MHz, CDCl₃) δ 7.90̃ (d, 1 H, *J* = 7.7 Hz), 7.25-7.44 (m, 5 H), 7.01 (d, 1 H, J = 7.9 Hz), 4.10-4.25 (m, 2 H), 3.51-3.63 (m, 2 H), 2.50-2.62 (m, 2 H), 2.13-2.27 (m, 2 H), 1.97 (s, 3 H); IR (pur) 2924, 2223, 1704, 1590, 1494, 1455, 1350, 1320, 1243, 1138, 959, 912, 743, 701 cm⁻¹; MS (FAB) *m*/*z* 374 (M+H)

### A-87: 6-(Chloro-difluoro-methyt)-2-(4-phenyl-piperazin-1-yl)-nicotinonitril

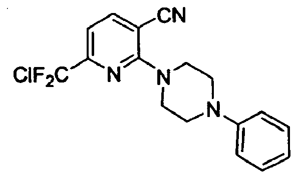

¹H NMR (300 MHz, CDCl₃) δ 7.94 (d, 1 H, *J* = 7.9 Hz), 7.24-7.34 (m, 2 H), 7.04 (d, 1 H, *J* = 7.9 Hz), 6.87-7.00 (m, 3 H), 4.00-4.06 (m, 4 H), 3.32-3.39 (m, 4 H); IR (pur) 2916, 2230, 2217, 1590, 1497, 1449, 1341, 1230, 1081, 986, 934, 812, 761, 693 cm⁻¹; MS (FAB) *m*/*z* 349 (M+H)

### A-88: 2-Dipropylamino-6-trifluoromethyl-nicotinonitril

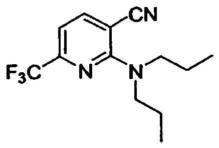

¹H NMR (300 MHz, CDCl₃) δ 7.74 (dd, 1 H, *J* = 7.8, 0.7 Hz), 6.75 (d, 1 H, *J* = 7.7 Hz), 3.53 (tt, 4 H, *J* = 7.7,1.8 Hz), 1.70-1.66 (m, 4 H), 0.86 (t, 4 H, *J* = 7.3 Hz); IR (pur) 2969, 2215, 1594, 1565, 1512, 1459, 1331 cm⁻¹; MS (FAB) *m*/*z* 272 (M+H)

### A-89: 6'-tert-Butyl-3,4,5,6-tetrahydro-2H-[1,2']bipyridinyl-3'-carbonitril

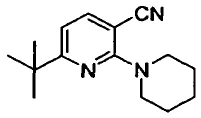

¹H NMR (300 MHz, CDCl₃) δ 7.65 (d, 1 H, *J* = 7.9 Hz), 6.70 (d, 1 H, *J* = 7.9 Hz), 3.70-3.68 (bs, 4 H), 1.65 (s, 6 H), 1.30 (s, 9 H); IR (pur) 2934, 2856, 2213, 1583, 1550, 1447, 1362 cm⁻¹; MS (FAB) *m*/*z* 244 (M+H)

### A-90: 6-tert-Butyl-2-pyrrolidin-1-yl-nicotinonitril

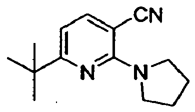

¹H NMR (300 MHz, CDCl₃) δ 7.61 (d, 1 H, *J* = 8.1 Hz), 6.59 (d, 1 H, *J* = 8.1 Hz), 3.80 (m, 4 H), 2.00 (m, 4 H), 1.28 (s, 9 H); IR (pur) 3409,2964,2785,2210,1583,1552, 1456cm⁻¹; MS (FAB) *m*/*z* 230 (M+H)

### A-91: 6'-tert-Butyl-4-methyl-3,4,5,6-tetrahydro-2H-[1,2']bipyridinyl-3'-carbonitril

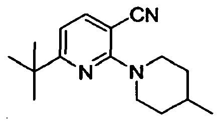

¹H NMR (300 MHz, CDCl₃) δ 7.57 (d, 1 H, *J* = 7.9 Hz), 6.63 (d, 1 H, *J* = 8 Hz), 4.37 (m, 2 H), 2.90 (td, 2 H, *J* = 12.6, 2.4 Hz), 1.68-1.16 (m , 5 H), 1.22 (s, 9 H), 0.83 (d, 3 H, *J* = 7.5 Hz); IR (pur) 2956, 2869, 2213, 1582, 1550, 1452, 1367 cm⁻¹; MS (FAB) *m*/*z* 258 (M+H)

### A-92: 6-tert-Butyl-2-dipropylamino-nicotinonitril

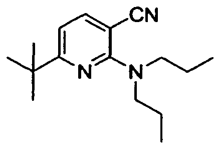

¹H NMR (300 MHz, CDCl₃) δ 7.61 (d, 1 H, *J* = 8.0 Hz), 6.59 (d, 1 H, *J* = 8.0 Hz), 3.58 (t, 4 H, *J* = 7.9 Hz), 1.68-1.64 (m, 4 H), 1.28 (s ,9 H), 0.95 (t, 6 H, *J* =7.3 Hz); IR (pur) 2964, 2873, 2208, 1585, 1550, 1495, 1456 cm⁻¹; MS (FAB) *m*/*z* 320 (M+H)

### A-93: 2-Azepan-1-yl-6-tert-butyl-nicotinonitril

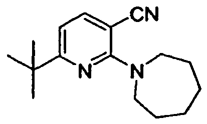

¹H NMR (300 MHz, CDCl₃) δ 7.60 (d, 1 H, *J* = 8.0 Hz), 6.60 (d, 1 H, *J* = 8.0 Hz), 3.90 (t, 4 H, *J* = 5.9 Hz), 1.89-1.85 (m, 4 H), 1.59-1.43 (m , 4 H), 1.28 (s, 9 H); IR (pur) 2930, 2859, 2208, 1584, 1549, 1487, 1453 cm⁻¹; MS (FAB) *m*/*z* 258 (M+H)

### A-94: 6'-tert-Butyl-4-phenyl-3,4,5,6-tetrahydro-2H-fl,21b1pyridinyl-3'-carbonitril

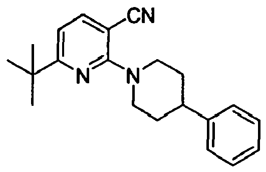

¹H NMR (300 MHz, CDCl₃) δ 7.69 (d, 1 H, *J* = 8.0 Hz), 7.38-7.19 (m, 5 H), 6.76 (d, 1 H, *J* = 7.7 Hz), 4.60 (d, 2 H, *J* = 6.2 Hz), 3.10 (td , 2 H, *J* = 12.5, 2.8 Hz), 2.79 (m, 1 H), 2.00-1.78 (m, 4 H), 1.3 (s, 9 H); IR (pur) 2959, 2213, 1583, 1550, 1452, 1368 1223 cm⁻¹, MS (FAB) *m*/*z* 320 (M+H)

### A-95: 4-Hydroxymethyl-6'-trifluoromethyl-3,4,5,6-tetrahydro-2H-[1,2']bipyridinyl-3'-carbonitril

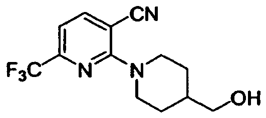

¹H NMR (300 MHz, CDCl₃) δ 7.89 (d, 1 H, *J*= 7.9 Hz), 6.99 (d, 1 H, *J* = 7.9 Hz), 4.58 (d, 1 H, *J* = 13.6 Hz), 3.57 (t, 2 H, *J* = 5.9 Hz), 3.01 (m, 2 H), 1.92-1.87 (m, 3 H), 1.41-1.34 (m, 2 H); IR (pur) 2923, 2220, 1591, 1567, 1499, 1458, 1364 cm⁻¹; MS (FAB) *m*/*z* 286 (M+H)

### A-96: 6-tert-Butyl-2-[4-(4-fluoro-phenyl)-piperazin-1-yl]-nicotinonitril

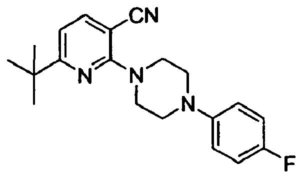

¹H NMR (300 MHz, CDCl₃) δ 7.70 (d, 1 H, *J* = 8.0 Hz), 7.02-6.89 (m, 4 H), 6.81 (d, 1 H, *J* = 8.0 Hz), 3.92-3.88 (m, 4 H), 3.26-3.23 (m , 4 H), 1.30 (s, 9 H); IR (pur) 2963, 2215, 1584, 1550, 1511, 1445, 1363 cm⁻¹; MS (FAB) *m*/*z* 339 (M+H)

### A-97: 2-Diethylamino-6-trifluoromethyl-nicotinonitril

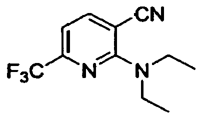

¹H NMR (300 MHz, CDCl₃) δ 7.86 (d, 1 H, *J* = 7.9 Hz), 7.07 (d, 1 H, *J* = 7.7 Hz), 3.74 (q, 4 H, *J* = 7.0 Hz), 1.30 (t, 6 H, *J* = 7.1 Hz); IR (KBr) 2983, 2216, 1594, 1566, 1514, 1459, 1330 cm⁻¹; MS (FAB) *m*/*z* 243 (M+H)

### A-98: 2-Dimethylamino-6-trifluoromethyl-nicotinonitril

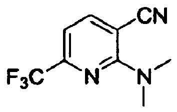

¹H NMR (300 MHz, CDCl₃) δ 7.87 (d, 1 H, *J* = 7.9 Hz), 6.92 (d, 1 H, *J* = 7.7 Hz), 3.35 (s, 4 H); IR (KBr) 2940, 2218, 1595, 1525, 1411, 1320, 1265 cm⁻¹ MS (FAB) *m*/*z* 215 (M+H)

### A-99: 2-Dibutylamino-6-trifluoromethyl-nicotinonitril

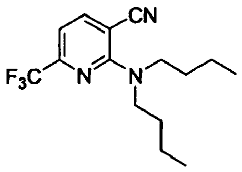

¹H NMR (300 MHz, CDCl₃) δ 7.84 (d, 1 H, *J* = 7.7 Hz), 6.85 (d, 1 H, *J* = 7.9 Hz), 3.66 (t, 4 H, *J* = 7.9 Hz), 1.72-1.60 (m, 4 H), 1.45-1.32 (m, 4 H), 0.97 (t, 6 H, *J* = 7.3 Hz); IR (KBr) 2962, 271, 2215, 1594, 1566, 1513, 1461 cm⁻¹; MS (FAB) *m*/*z* 300 (M+H)

### A-100: 2-Benzylamino-6-trifluoromethyl-nicotinonitril

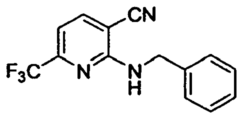

¹H NMR (300 MHz, CDCl₃) δ 7.87 (d, 1 H, *J* = 7.7 Hz), 7.40-7.26 (m, 5 H), 6.97 (d, 1 H, *J* = 7.7 Hz); IR (KBr) 3357, 2228, 1560, 1534, 1424, 1343, 1282 cm⁻¹; MS (FAB) *m*/*z* 277 (M+H)

### A-101: 4-Benzyl-4'-methyl-6'-trifluoromethyl-3,4,5,6-tetrahydro-2H-[1,2']bipyridinyl-3'-carbonitril

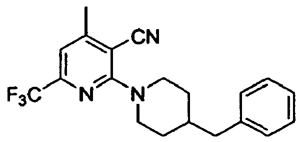

¹H NMR (300 MHz, CDCl₃) δ 7.14-7.34 (m, 5H), 6.90 (bs, 1 H), 4.39-4.49 (m, 2 H), 2.90-3.02 (m, 2 H), 2.59 (d, 2 H, *J* = 6.8 Hz), 2.52 (bs, 3 H), 1.71-1.87 (m, 2H), 1.22-1.50 (m, 3 H); IR (pur) 2922, 2850, 2214, 1577, 1494, 1452, 1391, 1243, 1182, 1143, 967, 913, 743, 701 cm⁻¹; MS (FAB) *m*/*z* 360 (M+H)

### A-102: 4,4'-Dimethyl-6'-trifluoromethyl-3,4,5,6-tetrahydro-2H-[1,2']bipyridinyl-3'-carbonitril

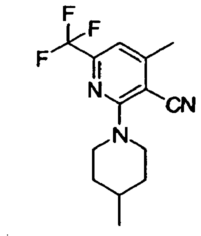

¹H NMR (300 MHz, CDCl₃) δ 6.89 (s, 1 H), 4.42 (m, 2 H), 3.02 (m, 2 H), 2.52 (s, 3 H), 1.65-1.79 (m, 3 H), 1.33 (m, 2 H), 0.99 (d, 3 H, J = 6.3 Hz); IR (pur) 2923, 2215, 1577, 1453, 1391, 1315, 1241, 1182, 1145, 1078, 969, 913, 847, 740 cm⁻¹ MS (FAB) *m*/*z* 284 (M+H)

### Stufe 2:

### Methode 1

Verbindungen der allgemeinen Formel VI-B (5 mmol), worin R⁵, R¹², R¹³, U, T und V die vorstehend genannte Bedeutung haben und m für 0, 1, 2 oder 3 steht, Palladium auf Kohle (10 %, 500 mg) und konzentrierte Salzsäure (3 mL) oder Essigsäure werden in MeOH (30 mL) gelöst und 6 Stunden bei RT einer Wasserstoffatmosphäre ausgesetzt. Die Reaktionsmischung wird über Celite filtriert und das Filtrat wird im Vakuum eingeengt. Der Rückstand wird mittels Flashchromatographie (SiO₂, EE) gereinigt.

Die folgenden Verbindungen B-1 bis B-15 wurden gemäß der vorstehend genannten allgemeinen Vorschrift erhalten:

### Verbindung B-1:

### [2-(Piperidin-1-yl)-6-(trifluormethyl)-pyridin-3-yl]methylamin

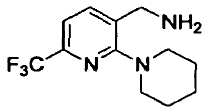

Die Verbindung wurde in einer Ausbeute von 50 % als leicht gelbes Öl erhalten. ¹H NMR (300 MHz, CDCl₃) δ 7.86 (d, 1 H, *J* = 5.7 Hz), 7.26 (d, 1 H, *J* = 5.7 Hz), 4.01 (s, 2 H), 3.11 (bs, 4 H), 1.62-1.70 (m, 6 H)

### Verbindung B-2:

### [2-(Morpholin-4-yl)-6-(trifluormethyl)-pyridin-3-yl]methylamin

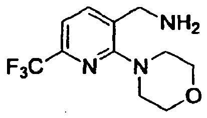

Die Verbindung wurde in einer Ausbeute von 28 % als leicht gelbes Öl erhalten.

¹H NMR (400 MHz, CDCl₃) δ 7.86 (d, 1 H, *J* = 5.7 Hz), 7.31 (d, 1 H, *J* = 5.7 Hz), 3.93 (s, 2 H), 3.85 (t, 4 H, *J* = 3.3 Hz), 3.23 (t, 4 H, *J* = 3.3 Hz)

### Verbindung B-3:

### [2-(Pyrrolidin-1-yl)-6-(trifluormethyl)-pyridin-3-yl]methylamin

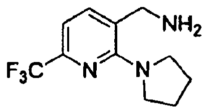

Die Verbindung wurde in einer Ausbeute von 60 % als leicht gelbes Öl erhalten.

¹H NMR (300 MHz, CDCl₃) δ 7.58 (d, 1 H, *J* = 7.5 Hz), 6.98 (d, 1 H, *J* = 7.5 Hz), 3.93 (s, 2 H), 3.55-3.60 (m, 4 H), 1.93-1.97 (m, 4 H)

### Verbindung B-4:

### 2-(Piperidin-1-yl)-4-(trifluormethyl)benzylamin

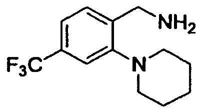

Die Verbindung wurde in einer Ausbeute von 50 % als leicht gelbes Öl erhalten.

¹H NMR (300 MHz, CD₃OD) δ 7.59 (d, 1 H, *J* = 7.8 Hz), 7.52 (s, 1 H), 7.47 (d, 1 H, *J* = 7.8 Hz), 4.28 (s, 2 H), 2.89-2.93 (m, 4 H), 1.63-1.82 (m, 6 H)

### Verbindung B-5:

### 2-(Morpholin-4-yl)-4-(trifluormethyl)benzylamin

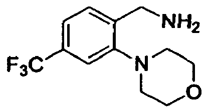

Die Verbindung wurde in einer Ausbeute von 38 % als leicht gelbes Öl erhalten.

¹H NMR (300 MHz, CD₃OD) δ 7.61 (d, 1 H, *J* = 7.8 Hz), 7.55 (s, 1 H), 7.50̃ (d, 1 H, *J* = 7.8 Hz), 4.26 (s, 2 H), 3.87 (t, 4 H, *J* = 4.5 Hz), 2.95 (t, 4 H, *J* = 4.5 Hz)

### Verbindung B-6:

### 2-(Pyrrolidin-1-yl)-4-(trifluormethyl)benzylamin

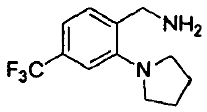

Die Verbindung wurde in einer Ausbeute von 55 % als leicht gelbes Öl erhalten.

¹H NMR (300 MHz, CD₃OD) δ 7.83 (d, 1 H, *J* = 7.8 Hz), 7.25 (s, 1 H), 7.18 (d, 1 H, *J* = 7.8 Hz), 4.21 (s, 2 H), 3.15-3.19 (m, 4 H), 1.95-1.99 (m, 4 H)

### B-7: C-[4-(4-Fluoro-phenyl)-6'-trifluoromethyl-3,4,5,6-tetrahydro-2H-[1,2']bipyridinyl-3'-yl]-methylamin

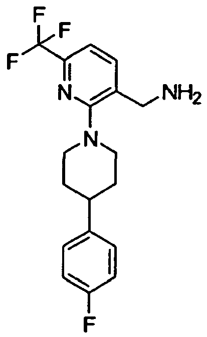

¹H NMR (300 MHz, CDCl₃) δ 8.03 (d, 1 H, *J* = 7.5 Hz), 7.41 (d, 1 H, *J* = 7.5 Hz), 7.24 (m, 2 H), 7.01 (dd, 1 H, *J* = 8.1, 8.4 Hz), 4.26 (s, 2 H), 3.45 (m, 2 H), 3.07 (m, 2 H), 2.72 (m, 1 H), 1.89-1.96 (m, 4 H); IR (pur) 2913, 2846, 1593, 1512, 1469, 1422, 1368, 1225, 1190, 1152, 950, 839 cm⁻¹; MS (FAB) *m*/*z* 354(M+H)

### B-8: 4-(2,2-dimethyl-propionyloxy)-6'-trifluoromethyl-3,4,5,6-tetrahydro-2H-[1,2']bipyridinyl-3'-ylmethyl-ammonium acetat

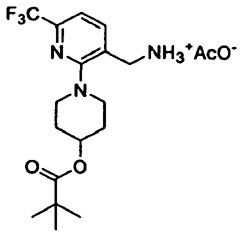

¹H NMR (CDCl₃) δ 7.81 (d, 1 H, J = 7.7 Hz), 7.31 (d, 1 H, J = 7.7 Hz), 4.98 (m, 1 H), 4.35 (s, NH3+), 3.97 (s, 2 H), 3.40-3.31 (m, 2 H), 3.18-3.10 (m, 2 H), 2.06-1.98 (m, 2 H), 2.04 (s, 3 H), 1.87-1.77 (m, 2 H), 1.22(s, 9 H); IR (pur) 2970, 1724, 1593, 1462, 1419, 1168, 1033 cm⁻¹

### B-9: 4-acetoxy-6'-trifluoromethyl-3,4,5,6-tetrahydro-2H-[1,2']bipyridinyl-3'-ylmethyl-ammonium acetat

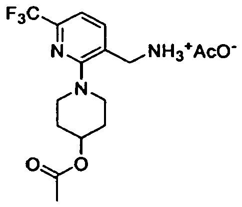

¹H NMR (CDCl₃) δ 7.81 (d, 1 H, 7.7 Hz), 7.31 (d, 1 H, 7.7 Hz), 4.97 (m, 1 H), 4.03-3.93 (m, 5 H), 3.45-3.35 (m, 2 H), 3.14-3.05 (m, 1 H), 2.08 (s, 3 H), 2.10-1.98 (m, 2 H), 1.88-1.77 (m, 2 H); IR (pur) 2957, 1734, 1419, 1247, 1138, 1034 cm⁻¹

### B-10: 4-methoxy-6'-trifluoromethyl-3,4,5,6-tetrahydro-2H-[1,2']bipyridinyl-3'-ylmethyl-ammonium acetat

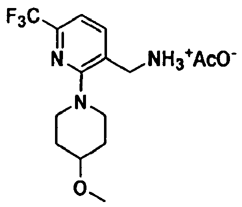

¹H NMR (CDCl₃) δ 7.80 (d, 1 H, J = 7.5Hz), 7.29 (d, 1 H, J = 7.7 Hz), 4.28 (bs, NH3), 3.97 (s, 2 H), 3.47-3.36 (m, 6 H), 3.02-2.94 (m, 2 H), 2.09-2.01 (m, 5 H), 1.77-1.65 (m, 2 H); IR (pur) 2930, 1542, 1461, 1418, 1335, 1178, 1137, 957 cm⁻¹

### B-11: 4-butoxy-6'-trifluoromethyl-3,4,5,6-tetrahydro-2H-[1,2']bipyridinyl-3'-ylmethyl-ammonium acetat

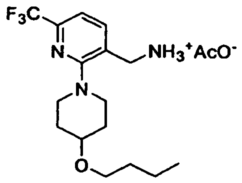

¹H NMR (CDCl₃) δ 7.80 (d, 1 H, J = 7.7 Hz), 7.29 (d, 1 H, J = 7.7 Hz), 4.03-3.98 (m, 5 H, 2 H + NH3), 3.49 (t, 2 H, J = 6.6 Hz), 3.47-3.37 (m, 3 H), 3.01-2.93 (m, 2 H), 2.07-1.97 (m, 2 H), 1.98 (s, 3 H), 1.77-1.65 (m, 2 H), 1.62-1.55 (m, 2 H), 1.45-1.33 (m, 2 H), 0.92 (t, 3 H, J = 7.3 Hz); IR (pur) 2955, 1542, 1462, 1419, 1333, 1140, 1041 cm⁻¹

### B-12: 4-Ethoxy-6'-trifluoromethyl-3,4,5,6-tetrahydro-2H-[1,2']bipyridinyl-3'-ylmethyl-ammonium acetat

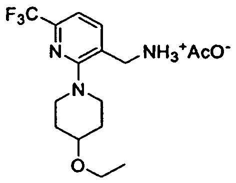

¹H NMR (CDCl₃) δ 7.80 (d, 1 H, J = 7.7 Hz), 7.29 (d, 1 H, J = 7.7 Hz), 5.30 (bs, NH3), 3.59-3.39 (m, 5 H), 3.01-2.93 (m, 2 H), 2.08-2.00 (m, 5 H), 1.78-1.66 (m, 2 H), 1.23 (t, 3 H, J = 7.0 Hz); IR (pur) 2927, 1593, 1419, 1333, 1241, 1178, 1139 cm⁻¹

### B-13: C-(5'-Trifluoromethyl-3,4,5,6-tetrahydro-2H-[1,3']bipyridinyl-2'-yl)-methylamin

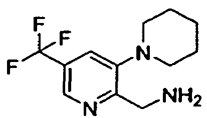

¹H NMR (300 MHz, D2O) δ 8.71 (s, 1 H), 8.21 (s, 1 H), 4.45 (s, 2 H), 3.24 (d, 4 H, J = 4.6 Hz), 1.77 (s, 4 H), 1.51 (s, 2 H); MS (FAB) m/z 260 (M+H)

### B-14: 2-(4-Ethyl-piperidin-1-yl)-4-trifluoromethyl-benzylamin

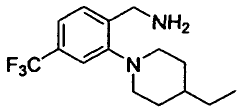

¹H NMR (400 MHz, CDCl₃) δ 7.43 (d, 1 H, *J* = 6.0 Hz), 7.27-7.33 (m, 2 H), 3.92 (s, 2 H), 3.04-3.12 (m, 2 H), 2.63-2.72 (m, 2 H), 1.78-1.85 (m, 2 H), 1.24-1.43 (m, 5 H), 0.93 (bt, 3 H); IR (pur) 2925, 1423, 1337, 1311, 1242, 1165, 1123, 1080, 949, 826 cm⁻¹; MS (FAB) *m*/*z* 287 (M+H)

### B-15: 4-isopropoxy-6'-trifluoromethyl-3,4,5,6-tetrahydro-2H-[1,2']bipyridinyl-3'-ylmethyl-ammonium acetat

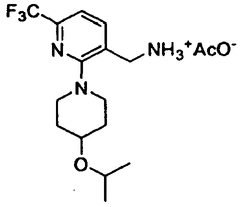

¹H NMR (CDCl₃) δ 7.78 (d, 1 H, J = 7.7 Hz), 7.28 (d, 1 H), 4.26 (bs, NH3), 3.95 (s, 2 H), 3.76 (m, 1 H), 3.57-3.40 (m, 3 H), 2.97 (m, 2 H), 2.07 (s, 3 H, AcO). 2.04-1.96 (m, 2 H), 1.76-1.65 (m, 2 H), 1.18 (d, 6 H, J = 6.2 Hz); IR (pur) 2972, 1593, 1462, 1419, 1333, 1177, 1140, 1041 cm⁻¹

### Methode 2:

Verbindungen der allgemeinen Formel VI-B (2 mmol), worin R⁵, R¹², R¹³, U, T und V die vorstehend genannte Bedeutung haben und m für 0, 1, 2 oder 3 steht, werden in THF (10 mL, 10 mL) gelöst und BH₃•S(CH₃)₂ [2.0 M in THF, 3 mL, 3 Äquivalent] wird hinzu gegeben. Die Reaktionsmischung wird 8 Stunden zum Rückfluß erhitzt, aq. HCl (2 N) wird hinzu gegeben und die Reaktionsmischung wird erneut für 30 Minuten zum Rückfluß erhitzt. Die Reaktionsmischung wird mit aq. Natriumhydroxid-Lösung (2N) versetzt und mit EE gewaschen. Die vereinigten organischen Phasen werden mit ges. aq. NaCl-Lösung gewaschen und über Magnesiumsulfat getrocknet. Das Lösungsmittel wird im Vakuum entfernt und der Rückstand säulenchromatographisch (SiO₂, verschiedene Mischung aus Dichlormethan und Methanol als Laufmittel) gereinigt.

Die folgenden Verbindungen B-16 bis B-80 wurden gemäß der vorstehend genannten allgemeinen Vorschrift erhalten:

### B-16: (6-(Clorodifluoromethyl)-2-(piperidin-1-yl)pyridin-3-yl)methanamin

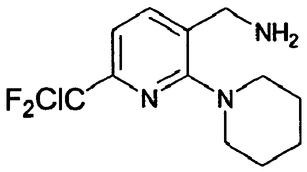

¹H NMR (300 MHz, CDCl₃) δ 7.78 (d, 1H, J = 7.8 Hz), 7.22 (d, 1 H, J = 7.8 Hz), 3.90 (s, 2H), 3.12-3.16 (m, 4 H), 1.60-1.70 (m, 6 H) IR (pur) 2935, 2851, 1590, 1417, 1373, 1300, 1091, 972, 913, 827 cm⁻¹ MS (FAB) *m*/*z* 276 (M+H)

### B-17: (2-(4-Benzylpiperazin-1-yl)-6-(trifluoromethyl)pyridin-3-yl)methanamin

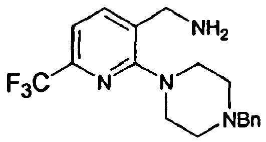

¹H NMR (300 MHz, CDCl₃) δ 7.81 (d, 1 H, J = 7.8 Hz), 7.23-7.37 (m, 6 H), 3.89 (s, 2 H), 3.58 (s, 2 H), 3.22-3.25 (m, 4 H), 2.57-2.62 (m, 4 H); IR (pur) 2814, 1592, 1417, 1324, 1176, 1135, 1005, 964, 836, 741, 700 cm⁻¹; MS (FAB) *m*/*z* 351 (M+H)

### B-18: (6-(Trifluoromethyl)-2-(4-methylpiperidin-1-yl)pyridin-3-yl)methanamin

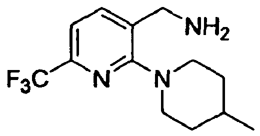

¹H NMR (300 MHz, CDCl₃) δ 7.89 (d, 1 H, J = 7.8 Hz), 7.33 (d, 1H, J = 7.8 Hz), 3.88 (s, 2H), 3.39 (m, 2 H), 2.83 (m, 2 H), 1.75 (m, 2 H), 1.55 (m, 1 H), 1.38 (m, 2 H), 1.00 (d, 3 H, J = 6.6 Hz); MS (FAB) *m*/*z* 274(M+H)

### B-19: C-(4-Fluoro-6-trifluoromethyl-3,4,5,6-tetrahydro-2H-[1,2']b1pyridinyl-3'-yl)-methylamin

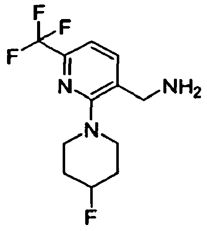

¹H NMR (300 MHz, CDCl₃) δ 7.83 (d, 1 H, J = 7.8 Hz), 7.28 (d, 1 H, J = 7.8 Hz), 4.85 (dm, 1 H, J = 48.3 Hz), 3.92 (s, 2 H), 3.39 (m, 2 H), 3.14 (m, 2 H), 2.01-2.28 (m, 4 H); MS (FAB) *m*/*z* 278(M+H)

### B-20; C-[6'-(Chloro-difluoro-methyl)-4-methyl-3,4,5,6-tetrahydro-2H-[1,2']bipyridinyl-3'-yl]-methylamin

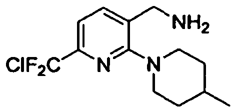

¹H NMR (300 MHz, CDCl₃) δ 7.72 (d, 1H, J = 7.5 Hz), 7.13 (d, 1 H, J = 7.5 Hz), 3.84 (s, 2H), 3.37 (m, 2 H), 2.77 (m, 2 H), 1.68 (m, 2 H), 1.48 (m, 1 H), 1.24 (m, 2 H), 0.89 (d, 3 H, J = 6.6 Hz); IR (pur) 2923, 1590, 1452, 1417, 1254, 1186cm⁻¹; MS (FAB) *m*/*z* 290(M+H)

### B-21: C-[2-Azepan-1-yl-6-(chloro-difluoro-methyl)-pyridin-3-yl]-methylamin

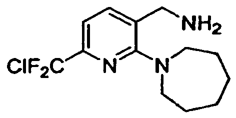

¹H NMR (300 MHz, CDCl₃) δ 7.69 (d, 1H, J = 7.5 Hz), 6.97 (d, 1H, J = 7.5 Hz), 3.98 (s, 2H), 3.37 (m, 4 H), 1.71 (m, 4 H), 1.51 (m, 4 H); IR (pur) 3432, 2928, 2857, 1593, 1452, 1421, 1371, 1257cm⁻¹; MS (FAB) *m*/*z* 290(M+H)

### B-22: C-[6'-(4-Fluoro-phenyl)-4-methyl-3,4,5,6-tetrahydro-2H-[1,2'] bipyridinyl-3'-yl]-methylamin

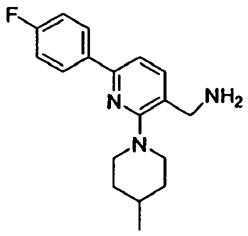

¹H NMR (300 MHz, CDCl₃) d 8.00 (m, 2H), 7.66 (d, 1 H, J = 7.8 Hz), 7.30 (d, 1 H, J = 7.8 Hz), 7.10 (dd, 2 H, J = 8.7, 8.7 Hz), 3.90 (s, 2 H), 3.43 (m, 2 H), 2.89 (m, 2 H), 1.74 (m, 2 H), 1.53 (m, 1 H), 1.38 (m, 2 H), 0.99 (d, 3 H, J = 6.3 Hz); IR (pur) 2932, 2851, 1600, 1577, 1509, 1447, 1421, 1372, 1236, 1156, 1112, 1031 cm⁻¹; MS (FAB) *m*/*z* 300(M+H)

### B-23: C-[2-Azepan-1-yl-6-(4-fluoro-phenyl)-pyridln-3-yl]-methylamin

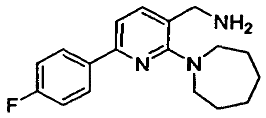

¹H NMR (300 MHz, CDCl₃) δ 7.97 (m, 2H), 7.59 (d, 1H, J = 7.8 Hz), 7.17 (d, 1 H, J = 7.8 Hz), 7.09 (dd, 2 H, J = 8.7, 8.7 Hz), 3.89 (s, 2 H), 3.49 (t, 4 H, J = 6.0 Hz), 1.81 (m, 4 H), 1.64 (m, 4 H); IR (pur) 2925, 2853, 1576, 1508, 1448, 1373, 1230, 1154, 906 cm⁻¹; MS (FAB) *m*/*z* 300(M+H)

### B-24: [3-Aminomethyl-6-(chloro-difluoro-methyl)-pyridin-2-yl]-dipropyl-amin

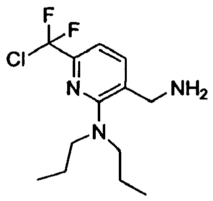

¹H NMR (300 MHz, CDCl₃) δ 7.68 (d, 1H, J = 7.8 Hz), 7.06 (d, 1H, J = 7.8 Hz), 3.84 (s, 2H), 3.08 (t, 4 H, J = 7.5 Hz), 1.47 (m, 4 H), 0.77 (t, 6 H, J = 7.2 Hz); IR (pur) 2964, 2874, 1591, 1462, 1418, 1372, 1257, 1091, 999 cm⁻¹ MS (FAB) *m*/*z* 292(M+H)

### B-25: C-[2-(1,3-Dihydro-isoindol-2-yl)-6-trifluoromethyl-pyridin-3-yl]-methylamin

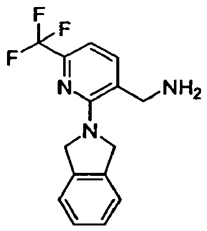

¹H NMR (300 MHz, CDCl₃) δ 7.63 (d, 1 H, J = 7.8 Hz), 7.27 - 7.31 (m, 4H), 7.03 (d, 1 H, J = 7.8 Hz), 5.06 (s, 4H), 4.08 (s, 2H); IR (pur) 3365, 2926, 2857, 1598, 1457, 1363, 1263, 1177, 1132, 1013, 820 cm⁻¹; MS (FAB) *m*/*z* 294(M+H)

### B-26: 3'-Aminomethyl-4-phenyl-6'-trifluoromethyl-3,4,5,6-tetrahydro-2H-[1,2']bipyridinyl-4-carbonsäure ethyl ester

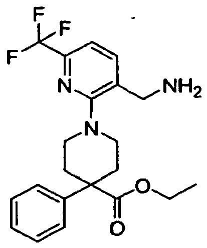

¹H NMR (300 MHz, CDCl₃) δ 7.83 (d, 1 H, J = 7.5 Hz), 7.45 (m, 2 H), 7.35 (m, 3 H), 7.26 (d, 1 H, J = 8.1 Hz), 4.15 (q, 2 H, J= 7.2 Hz), 4.03 (s, 2 H), 3.47 (m, 2 H), 3.08 (m, 2 H), 2.69 (m, 2 H), 2.10 (m, 2 H), 1.21 (t, 3 H, J = 7.2 Hz); MS (FAB) *m*/*z* 408 (M+H)

### B-27: C-(4,6'-Bis-trifluoromethyl-3,4,5,6-tetrahydro-2H-[1,2']bipyridinyl-3'-yl)-methylamin

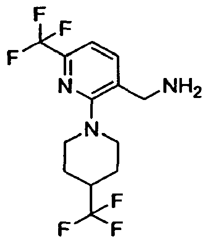

¹H NMR (300 MHz, CDCl₃) δ 7.80 (d, 1 H, J = 7.8 Hz), 7.23 (d, 1 H, J = 7.8 Hz), 3.83 (s, 2 H), 3.48 (m, 2 H), 2.79 (m, 2 H), 2.15 (m, 1 H), 1.88 (m, 2 H), 1.65 (m, 2 H); IR (pur) 2960, 1591, 121, 1378, 1337, 1255, 1141, 1084, 955, 901, 837, 698 cm⁻¹; MS (FAB) *m*/*z* 328 (M+H)

### B-28: C-(4-Methoxymethyl-6'-trifluoromethyl-3,4,5,6-tetrahydro-2H-[1,2']bipyridinyl-3'-yl)-methylamin

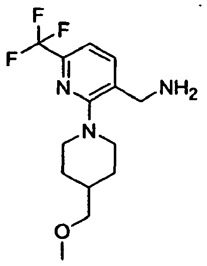

¹H NMR (300 MHz, CDCl₃) δ 7.81 (d, 1 H, J = 7.8 Hz), 7.26 (d, 1 H, J = 7.8 Hz), 3.91 (s, 2 H), 3.36 (s, 3 H), 3.29 (d, 2 H, J = 6.0 Hz), 2.87 (m, 2 H), 2.37 (s, 2 H), 1.71-1.86 (m, 4 H), 1.34-1.47 (m, 3 H); IR (pur) 2924, 1592, 1455, 1374, 1324, 1268, 1175, 1135, 950, 835 cm⁻¹; MS (FAB) *m*/*z* 304(M+H)

### B-29: C-[2-(4-p-Tolyl-piperazin-1-yl)-6-trifluoromethyl-pyridin-3-yl]-methylamin

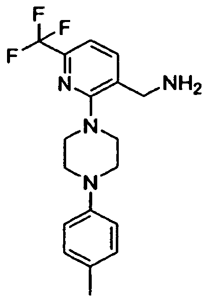

¹H NMR (300 MHz, CDCl₃) δ 7.85 (d, 1 H, J = 7.8 Hz), 7.30 (d, 1 H, J = 7.8 Hz), 7.09 (d, 2 H, J = 8.4 Hz), 6.88 (d, 2 H, J = 8.4 Hz), 3.94 (s, 2 H), 3.37 (m, 4 H), 3.26 (m, 4 H), 2.27 (s, 3 H); IR (pur) 3368, 2847, 1732, 1591, 1515, 117, 1333, 1235, 1176, 1137, 1051, 966, 916, 814, 755 cm⁻¹; MS (FAB) *m*/*z* 351(M+H)

### B-30: C-[2-(4-m-Tolyl-piperazin-1-yl)-6-trifluoromethyl-pyridin-3-yl]-methylamin

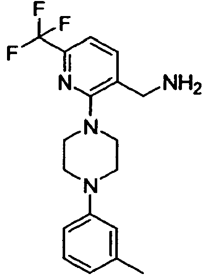

¹H NMR (300 MHz, CDCl₃) δ 7.86 (d, 1 H, J = 7.8 Hz), 7.31 (d, 1 H, J = 7.8 Hz), 7.18 (t, 1 H, J = 7.5 Hz), 6.77-6.79 (m, 2 H), 3.95 (s, 2 H), 3.31-3.38 (m, 8 H), 2.33 (s, 3 H); IR (pur) 3367, 2845, 1595, 1493, 1418, 1335, 1240, 1335, 1137, 1045, 998, 967, 836, 775, 695 cm⁻¹; MS (FAB) *m*/*z* 351 (M+H)

### B-31: C-{2-[4-(4-Methoxy-phenyl)-piperazin-1-yl]-6-trifluoromethyl-pyridin-3-yl}-methylamin

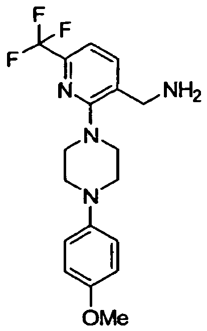

¹H NMR (300 MHz, CDCl₃) δ 7.86 (d, 1 H, J = 7.8 Hz), 7.31 (d, 1 H, J = 7.8 Hz), 6.94 (d, 2 H, J = 6.9 Hz), (d, 2 H, J = 6.9 Hz), 3.95 (s, 2 H), 3.77 (s, 3 H), 3.39 (m, 4 H), 3.22 (m, 4 H); IR (pur) 2837, 1590, 1512, 1418, 1332, 1244, 1178, 1137, 1035, 967, 826 cm⁻¹; MS (FAB) *m*/*z* 367(M+H)

### B-32: C-{6-Trifluoromethyl-2-[4-(4-trifluoromethyl-phenyl)-piperazin-1-yl]-pyridin-3-yl}-methylamin

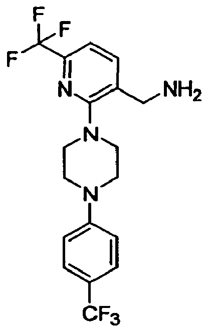

¹H NMR (300 MHz, CDCl₃) δ 7.89 (d, 1 H, J = 7.8 Hz), 7.50 (d, 2 H, J = 7.8 Hz), 7.32 (d, 1 H, J = 7.8 Hz), 6.97 (d, 2 H, J = 7.8 Hz), 4.09 (s, 2 H), 3.40 (m, 8 H), 2.27 (s, 2 H); IR (pur) 2933, 1695, 1600, 1511, 1428, 1397, 1342, 1314, 1262, 1158, 1026, 835 cm⁻¹; MS (FAB) *m*/*z* 405(M+H)

### B-33: C-{6-Trifluoromethyl-2-[4-(3-trifluoromethyl-pyridin-2-yl)-piperazin-1-yl]-pyridin-3-yl}-methylamin

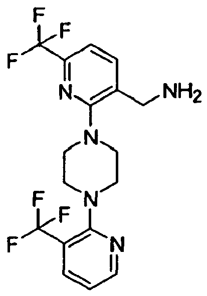

¹H NMR (300 MHz, CDCl₃) δ 8.46 (d, 1 H, J = 3.3 Hz), 7.86-7.93 (m, 2 H), 7.31 (d, 1 H, J = 7.5 Hz), 7.03 (m, 1 H), 3.97 (s, 2 H), 3.46 (m, 4 H), 3.36 (m, 4 H), 2.12 (bs, 2 H); IR (pur) 3367, 2850, 1590, 1445, 1368, 1312, 1236, 1138, 1027, 966, 837 cm⁻¹; MS (FAB) *m*/*z* 407 (M+H)

### B-34: C-[2-(4-Cyclohexyl-piperazin-1-yl)-6-trifluoromethyl-pyridin-3-yl]-methylamin

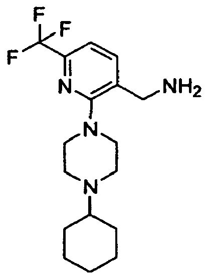

¹H NMR (300 MHz, CDCl₃) δ 7.80 (d, 1 H, J = 7.5 Hz), 7.27 (d, 1 H, J = 7.5 Hz), 3.90 (s, 2 H), 3.25 (m, 4 H), 2.73 (m, 4 H), 2.16 (m, 1 H), 1.70 (m, 4 H), 1.19-1.28 (m, 6 H); MS (FAB) *m*/*z* 343(M+H)

### B-35: 2-(4-Methyl-6'-trifluoromethyl-3,4,5,6-tetrahydro-2H-[1,2']bipyridinyl-3'-yl)-ethylamin

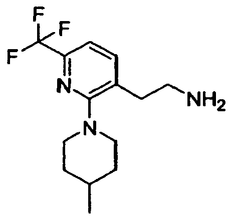

¹H NMR (300 MHz, CDCl₃) δ 7.46 (d, 1 H, J = 7.5 Hz), 7.12 (d, 1 H, J = 7.5 Hz), 3.32 (m, 2 H), 2.95 (t, 2 H, J = 6.9 Hz), 2.75 (m, 4 H), 1.55-1.63 (m, 5 H), 0.91 (d, 3 H, J = 6.3 Hz); IR (pur) 3364, 2924, 1648, 1590, 1457, 1415, 1322, 1236, 1176, 1136, 1045, 944, 834 cm⁻¹; MS (FAB) *m*/*z* 288(M+H)

### B-36: (3'-Aminomethyl-6'-trifluoromethyl-3,4,5,6-tetrahydro-2H-[1,2']bipyridinyl-4-yl)-phenyl-amin

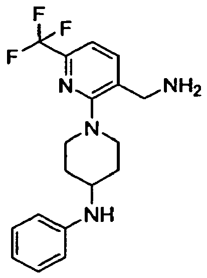

¹H NMR (300 MHz, CDCl₃) δ 7.82 (d, 1 H, J = 7.5 Hz), 7.29 (d, 1 H, J = 7.5 Hz), 7.18 (m, 2 H), 6.66 (m, 3H), 3.93 (s, 2 H), 3.47 (m, 2 H), 3.03 (m, 2 H), 2.84 (bs, 2 H), 2.18 (m, 2 H), 1.58-1.66 (m, 3 H); IR (pur) 3365, 2938, 1598, 1504, 1421, 1333, 1265, 1177, 1136, 1044, 953, 836, 752, 695 cm⁻¹; MS (FAB) *m*/**z** 351(M+H)

### B-37: C-[2-(2,6-Dimethyl-morpholin-4-yl)-6-trifluoromethyl-pyridin-3-yl]-methylamin

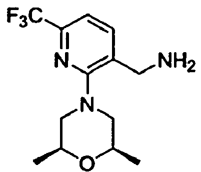

¹H NMR (300 MHz, CDCl₃) δ 7.77 (d, 1 H, J = 7.8 Hz), 7.20 (d, 1 H, J = 7.8 Hz), 3.84 (s, 2 H), 3.73 (m, 2 H), 3.25 (m, 2 H), 2.60 (m, 2 H), 1. 70 (bs, 2 H), 1.15 (d, 6 H, J = 6.3 Hz); IR (pur) 2976, 1591, 1459, 1418, 1249, 1175, 1006, 836 cm⁻¹; MS (FAB) *m*/*z* 290 (M+H)

### B-38: C-[2-(1,1-Dioxo-thiomorpholin-4-yl)-6-trifluoromethyl-pyridin-3-yl]-methylamin

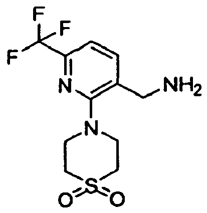

¹H NMR (300 MHz, CDCl₃) δ 7.94 (d, 1 H, J=7.5 Hz), 7.38 (d, 1 H, J = 7.5 Hz), 3.82-3.91 (m, 6 H), 3.20 (m, 4 H), 1.52 (bs, 2 H); IR (pur) 2929, 1709, 1591, 1465, 1334, 1280, 1178, 1126, 1029, 997, 864 cm⁻¹; MS (FAB) *m*/*z* 310(M+H)

### B-39: C-(2-Imidazol-1-yl-6-trifluoromethyl-pyridin-3-yl)-methylamin

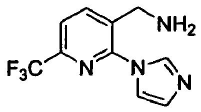

¹H NMR (300 MHz, CDCl₃) δ 8.17 (d, 1 H, *J* = 7.9 Hz), 8.11 (s, 1 H), 7.67 (d, 1 H, *J* = 7.9 Hz), 7.49 (s, 1 H), 7.14 (s, 1 H), 3.93 (s, 2 H); MS (FAB) *m*/*z* 243 (M+H)

### B-40: C-(4,6'-Dimethyl-3,4,5,6-tetrahydro-2H-[1,2']bipyridinyl-3'-yl)-methylamin

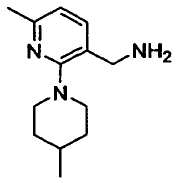

¹H NMR (300 MHz, CDCl₃) δ 7.45 (d, 1 H, J = 6.6 Hz), 6.75 (d, 1 H, J = 6.5 Hz), 3.80 (s, 2 H), 3.30 (m, 2H), 2.81 (m, 2 H), 2.42 (s, 3 H), 2.34 (bs, 2 H), 1.72 (m, 2 H), 1.51 (m, 1 H), 1.33 (m, 2 H), 0.98 (d, 3 H, J = 5.7 Hz); IR (pur) 3364, 2919, 1580, 1452, 1402, 1373, 1242, 1189, 1146, 1106, 1053, 962, 815 cm⁻¹; MS (FAB) *m*/*z* 220(M+H)

### B-41: (3-Aminomethyl-6-trifluoromethyl-pyridin-2-yl)-cyclohexyl-amin

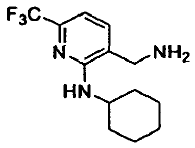

¹H NMR (CDCl₃) δ 7.24 (d, 1 H, J = 7.4 Hz), 6.78 (d, 1 H, J = 7.1 Hz), 6.69 (bs, NH), 3.99 (m, 1 H), 3.84 (s, 2 H), 2.09-2.01 (m, 2 H), 1.75-1.21 (m, 8 H)

### B-42: 3'-Aminomethyl-6'-trifluoromethyl-3,4,5,6-tetrahydro-2H-[1,2']bipyridinyl-4-ol

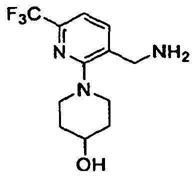

¹H NMR (CDCl₃) δ 7.90 (d, 1 H, J = 7.7 Hz), 7.35 (d, 1 H, J = 7.7 Hz), 3.90 (s, 2 H), 3.81-3.75 (m, 1 H), 3.43-3.39 (m, 2 H), 2.01-1.95 (m, 3 H), 1.72-1.61 (m, 2 H)

### B-43: 6'-Trifluoromethyl-3,4,5,6-tetrahydro-2H-[1,2']bipyridinyl-3'-ylamin

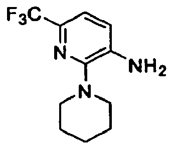

¹H NMR (CDCl₃) δ 7.17 (d, 1 H, J = 8.0 Hz), 6.92 (d, 1 H, J = 8.0 Hz), 4.02 (bs, NH), 3.07 (m, 4 H), 1.74-1.56 (m, 6 H); IR (pur) 2936, 1610, 1480, 1428, 1374, 1320, 1277, 1172, 1121 cm⁻¹

### B-44: (3-Aminomethyl-6-triftuoromethyl-pyridin-2-yl)-butyl-amin

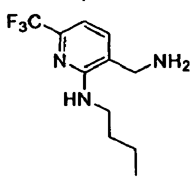

¹H NMR (CDCl₃) δ 7.24 (dd, 1 H, J = 7.3, 0.7 Hz), 6.80 (d, 1 H, J = 7.3 Hz), 6.78 (br, NH), 3.86 (s, 2 H), 3.50-3.44 (m, 2 H), 1.67-1.57 (m, 2 H), 1.49-1.37 (m, 2 H), 0.96 (t, 3 H, J = 7.1 Hz); IR (pur) 3301, 2929, 1611, 1532, 1458, 1309, 1175, 1133, 817 cm⁻¹

### B-45: C-[2-(6-Aza-spiro[2.5]oct-6-yl)-6-trifluoromethyl-pyridin-3-yl]-methylamin

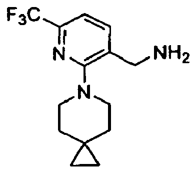

¹H NMR (CDCl₃) δ 7.82 (d, 1 H, J = 7.5 Hz), 7.26 (d, 1 H, J = 7.7 Hz), 3.93 (s, 2 H), 3.22 (m, 4 H), 1.52 (m, 4 H), 0.36 (s, 4 H); IR (pur) 2923, 1593, 1457, 1419, 1332, 1176, 1136, 956 cm⁻¹

### B-46: C-(3-Methyl-6'-trifluoromethyl-3,4,5,6-tetrahydro-2H-[1,2']bipyridiny1-3'-yl)-methylamin

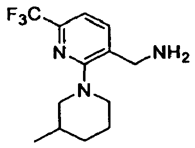

¹H NMR (CDCl₃) δ 7.80 (dd, 1 H, J = 7.7, 0.7 Hz), 7.25 (d, 1 H, J = 7.5 Hz), 3.90 (s, 2 H), 3.41-3.33 (m, 2 H), 2.84-2.75 (m, 1 H), 2.54-2.47 (m, 1 H), 1.85-1.63 (m, 4 H), 1.16-1.03 (m, 1 H), 0.94 (d, 3 H, J = 6.6 Hz); IR (pur) 2927, 1593, 1458, 1418, 1176, 1136, 1001 cm⁻¹

### B-47: C-(2-Methyl-6'-trifluoromethyl-3,4,5,6-tetrahydro-2H-[1,2']bipyridiny1-3'-yl)-methylamin

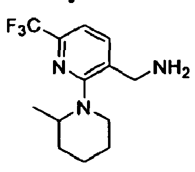

¹H NMR (CDCl₃) δ 8.05 & 7.78 (d, 1 H), 7.65 & 7.32 (d, 1 H), 4.04 & 3.78 (m, 2 H), 3.54 (m, 1 H), 3.07 (m, 1 H), 2.87 (m, 1 H), 1.84-1.42 (m, 6 H), 0.96 (d, 3 H, J = 6.2 Hz); IR (pur) 2933, 1539, 1459, 1412, 1337, 1178, 1139, 843 cm⁻¹

### B-48: 4-[(3-Aminomethyl-6-trifluoromethyl-pyridin-2-ylamino)-methyl]-piperidin-1-carbonsäure tert-butyl ester

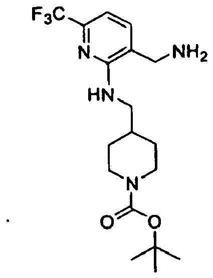

¹H NMR (CDCl₃) δ 7.26 (m, 2 H), 6.79 (d, 1 H, J = 7.0 Hz), 4.11 (m, 2 H), 3.89 (s, 2 H), 3.39 (m, 2 H), 2.69 (m, 2 H), 1.85-1.65 (m, 5 H), 1.43 (s, 9 H); IR (pur) 3376, 2925, 1680, 1610, 1533, 1427, 1366, 1173, 1137 cm⁻¹

### B-49: C-(4-Benzyl-6'-trifluoromethyl-3,4,5,6-tetrahydro-2H-[1,2']bipyridinyl-3'-yl)-methylamin

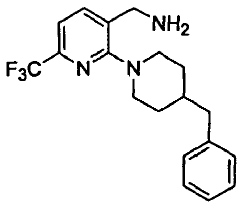

¹H NMR (300 MHz, CDCl₃) δ 7.80 (d, 1 H, *J* = 7.7 Hz), 7.35-7.21 (m, 6 H), 3.88 (s, 2 H), 3.45 (m, 2 H), 2.82 (m, 2 H), 2.60 (d, 2 H, *J* = 6.6 Hz), 1.77-1.67 (m, 3 H), 1.42 (m, 2 H); IR (pur) 3385, 2921, 2847, 1592, 1454, 1418, 1373, 1320, 1267, 1174, 1134, 953, 834, 746, 701 cm⁻¹; MS (FAB) *m*/*z* 350 (M+H)

### B-50: C-{2-[4-(4-Fluoro-phenyl)-piperazin-1-yl]-6-trifluoromethyl-pyridin-3-yl}-methylamin

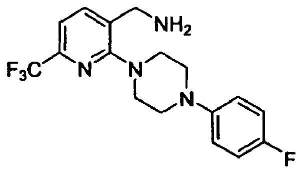

¹H NMR (300 MHz, CDCl₃) δ 7.84 (d, 1 H, *J* = 8.0 Hz), 7.31 (d, 1 H, *J* = 7.6 Hz), 7.05-6.91 (m , 4 H), 4.42 (s , 2 H), 3.44-3.35 (m, 4 H), 3.32-3.24 (m, 4 H), 1.57 (bs, 2 H); IR (pur) 2844, 1591, 1510, 1418, 1334, 1232, 1176, 1137, 1051, 966, 916, 825, 757 cm⁻¹; MS (FAB) *m*/*z* 355 (M+H)

### B-51: C-{2-[4-(2-Fluoro-phenyl)-piperazin-1-yl]-6-trifluoromethyl-pyridin-3-yl}-methylamin

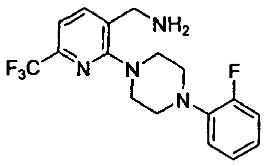

¹H NMR (300 MHz, CDCl₃) δ 7.87 (d, 1 H, *J* = 7.7 Hz), 7.31 (d, 1 H, *J* = 7.7 Hz), 7.21-7.03 (m, 4 H), 3.96 (s, 2 H), 3.48-3.35 (m, 4 H), 3.29-3.15 (m, 4 H) IR (pur) 3384m 2842m 1571 m 1501 m 1453, 1416, 1372, 1337, 1236, 1176, 1136, 1052, 966, 822, 835, 754 cm⁻¹, MS (FAB) *m*/*z* 355 (M+H)

### B-52: C-[2-(4-Phenyl-piperazin-1-yl)-6-trifluoromethyl-pyridin-3-yl]-methylamin

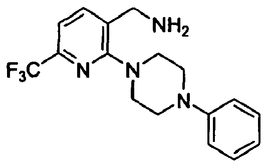

¹H NMR (300 MHz, CDCl₃) δ 7.88 (d, 1 H, *J* = 7.7Hz), 7.35-7.26 (m, 3 H), 6.98 (d, 2 H, *J* = 7.9 Hz), 6.89 (m, 1 H), 3.97 (s, 2 H), 3.44-3.32 (m, 8 H) IR (pur) 2843, 1595, 1500, 1418, 1335, 1232, 1177, 1134, 966, 836, 759, 693 cm⁻¹; MS (FAB) *m*/*z* 337 (M+H)

### B-53: (3-Aminomethyl-6-trifluoromethyl-pyridin-2-yl)-methyl-phenyl-amin

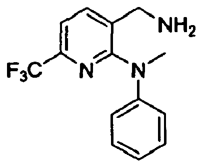

¹H NMR (300 MHz, CDCl₃) δ 7.83 (d, 1 H, *J* = 7.5 Hz), 7.34 (d, 1 H, *J* = 7.7 Hz), 7.26 (m, 2 H), 7.05 (m, 1 H), 6.91 (m, 2 H), 3.46 (s, 3 H), 3.31 (s, 2 H), 1.28 (bs, 2 H); IR (pur) 2915, 1588, 1496, 1465, 1396, 1349, 1264, 1180, 1137, 930, 835, 756, 699 cm⁻¹; MS (FAB) *m*/*z* 282 (M+H)

### B-54: C-(4,4-Dimethyl-6'-trifluoromethyl-3,4,5,6-tetrahydro-2H-[1,2']bipyridinyl-3'-yl)-methylamin

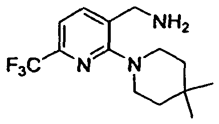

¹H NMR (300 MHz, CDCl₃) δ 7.81 (d, 1 H, *J* = 7.7 Hz), 7.25 (d, 1 H, *J* = 7.8 Hz), 3.89 (s, 2 H), 3.22-3.13 (m, 4 H), 1.59-1.46 (m, 4 H), 1.01 (s, 6 H); IR (pur) 2919, 1639, 1590, 1459, 1423, 1375, 1321, 1252, 1175, 1138, 1047, 954, 835 cm⁻¹; MS (FAB) *m*/*z* 288 (M+H)

### B-55: 2-(4-p-Tolyl-piperazin-1-yl)-4-trifluoromethyl-benzylamin

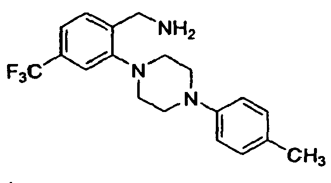

¹H NMR (300 MHz, CDCl₃) δ 7.51 (d, 1 H, *J* = 9.0 Hz), 7.37 (d, 1 H, *J* = 6.4 Hz), 7.36 (s, 1 H), 7.11 (d, 2 H, *J* = 8.4 Hz), 6.90 (d, 2 H, *J* = 8.4 Hz), 3.99 (s, 2 H), 3.10-3.02 (m, 4 H), 3.17-3.07 (m, 4 H), 2.29 (s, 3 H); IR (pur) 2826,1616, 1515, 1425, 1334, 1308, 1232, 1165, 1123, 1079, 959, 814 cm⁻¹; MS (FAB) *m*/*z* 350 (M+H)

### B-56: 2-(4-m-Tolyl-piperazin-1-yl)-4-trifluoromethyl-benzylamin

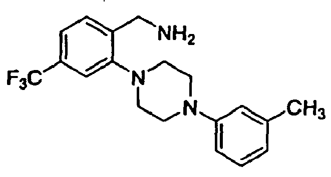

¹H NMR (300 MHz, CDCl₃) δ 7.51 (d, 1 H, *J* = 7.5 Hz), 7.37 (d, 1 H, *J* = 7.7 Hz), 7.36 (s, 1 H), 7.19 (m, 1 H), 6.81 (s, 1 H), 6.80 (d, 1 H, *J* = 7.1 Hz), 6.73 (d, 1 H, *J* = 7.5 Hz), 3.99 (s, 2 H), 3.51-3.42 (m, 4 H), 3.17-3.06 (m, 4 H), 2.34 (s, 3 H), 1.67 (bs, 2 H); IR (pur) 2828, 1604, 1498, 1425, 1336, 1310, 1250, 1166, 1123, 962, 777 cm⁻¹; MS (FAB) *m*/*z* 350 (M+H)

### B-57: 4-Trifluoromethyl-2-[4-(4-trifluoromethyl-phenyl)-piperazin-1-yl]-benzylamin

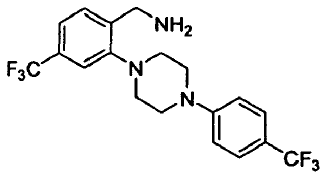

¹H NMR (300 MHz, CDCl₃) δ 7.59-7.42 (m, 3 H), 7.38 (d, 1 H, *J* = 8.4 Hz), 7.35 (s, 1 H), 6.99 (d, 2 H, *J* = 8.8 Hz), 4.00 (s, 2 H), 3.49-3.35 (m, 4 H), 3.19-3.05 (m, 4 H); IR (pur) 2838, 1616, 1527, 1425, 1332, 1238, 1163, 1116, 1073, 960, 827 cm⁻¹; MS (FAB) *m*/*z* 404 (M+H)

### B-58: 2-[4-(4-Methoxy-phenyl)-piperazin-1-yl]-4-trifluoromethyl-benzylamin

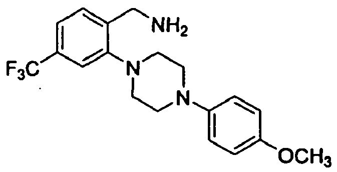

¹H NMR (300 MHz, CDCl₃) δ 7.50 (d, 1 H, *J* = 8.0 Hz), 7.38 (d, 1 H, *J* = 7.6 Hz), 7.36 (s, 1 H), 6.99-6.83 (m, 4 H), 3.98 (s, 2 H), 3.79 (s, 3 H), 3.29-3.18 (m, 4 H), 3.17-3.04 (m, 4 H) ; IR (pur) 3395, 2831, 1511, 1426, 1307, 1244, 1167, 1123, 1078, 1037, 959, 826 cm⁻¹; MS (FAB) *m*/*z* 366 (M+H)

### B-59: 2-[4-(4-Fluoro-phenyl)-piperidin-1-yl]-4-trifluoromethyl-benzylamin

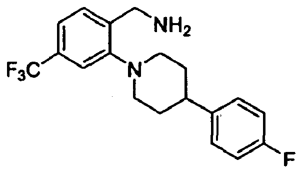

¹H NMR (300 MHz, CDCl₃) δ 7.48 (d, 1 H, *J* = 8.0 Hz), 7.35 (s, 1 H), 7.34 (d, 1 H, *J* = 7.6 Hz), 7.24 (m, 2 H), 7.02 (m, 2 H), 3.98 (s, 2 H), 3.21 (bd, 2 H, *J* = 11.5 Hz), 2.86 (td, 2 H, *J* = 11.4, 2.9 Hz), 2.65 (m, 1 H), 1.99-1.83 (m, 4 H); IR (pur) 2921, 1608, 1509, 1425, 1321, 1224, 1164, 1123, 1079, 949, 884, 833, 732 cm⁻¹; MS (FAB) *m*/*z* 353 (M+H)

### B-60: C-(6"-Trifluoromethyl-3,4,5,6,3',4',5',6'-octahydro-2H,2'H-[1,4';1',2"]terpyridin-3"-yl)-methylamin

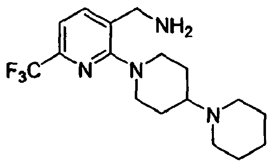

¹H NMR (300 MHz, CD₃OD) δ 7.91 (d, 1 H, *J* = 7.9 Hz), 7.35 (d, 1 H, *J* = 7.7 Hz), 3.87 (s, 2 H), 3.52-3.56 (m, 2 H), 2.82-2.90 (m, 2 H), 2.49-2.64 (m, 5 H), 1.97-2.01 (m, 2 H), 1,50-1.51 (m, 8 H); IR (pur) 2933, 2852, 1592, 1457, 14201339, 1135, 956 cm⁻¹; MS (FAB) *m*/*z* 343 (M+H)

### B-61: C-(4-Pyrrolidin-1-yl-6'-trifluoromethyl-3,4,5,6-tetrahydro-2H-[1,2']bipyridinyl-3'-yl)-methylamin

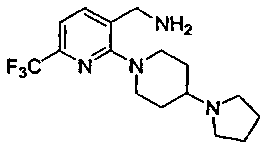

¹H NMR (300 MHz, CD₃OD) δ 7.92 (d, 1 H, *J* = 7.7 Hz), 7.36 (d, 1 H, *J* = 7.7 Hz), 3.90 (s, 2 H), 3.48-3.56 (m, 2 H), 2.87-2.95 (m, 2 H), 2.72-2.83 (m, 5 H), 2.42 (m, 1 H), 2.03-2.15 (m, 2 H), 1.79-7.92 (m, 5 H); IR (pur) 2959, 1592, 1459, 1421, 1339, 1240, 1176, 1135, 957, 834 cm⁻¹; MS (FAB) *m*/*z* 329(M+H)

### B-62: C-[6-(Chloro-difluoro-methyl)-2-(4-phenyl-piperazin-1-yl)-pyridin-3-yl]-methylamin

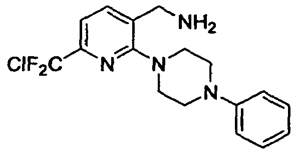

¹H NMR (300 MHz, CDCl₃) δ 7.86 (d, 1 H, *J* = 7.5 Hz), 7.26-7.34 (m, 3 H), 6.95-7.03 (m, 2 H), 6.89 (m, 1 H), 3.96 (s, 2 H), 3.30-3.46 (m, 8 H); IR (pur) 2842, 1594, 1500, 1415, 1375, 1231, 1091, 980, 932, 900, 817, 759, 682 cm⁻¹; MS (FAB) *m*/*z* 353 (M+H)

### B-63: 2-(4-Phenyl-piperazin-1-yl)-4-trifluoromethyl-benzylamin

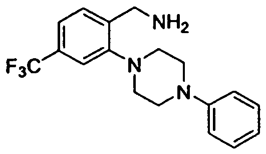

¹H NMR (300 MHz, CDCl₃) δ 7.50̃ (d, 1 H, *J* = 8.3 Hz), 7.24-7.39 (m, 4 H), 6.98 (d, 2 H, *J* = 8.1 Hz), 6.90 (dd, 1 H, *J* = 7.1, 7.1 Hz), 3.99 (s, 2 H), 3.22-3.37 (m, 4 H), 3.08-3.13 (m, 4 H); IR (pur) 2826, 1599, 1500, 1423, 1334, 1308, 1232, 1163, 1121, 1079, 959, 882, 830, 760, 693 cm⁻¹; MS (FAB) *m*/*z* 336 (M+H)

### B-64: 2-Azocan-1-yl-4-trifluoromethyl-benzylamin

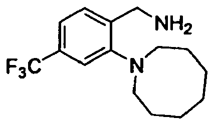

¹H NMR (300 MHz, CDCl₃) δ 7.46 (d, 1 H, *J* = 7.9Hz), 7.41 (s , 1 H), 7.30 (d , 1 H, *J* = 8.0 Hz), 4.03 (s , 2 H), 3.02-3.14 (m, 4 H), 2.44-2.56 (m , 3 H), 1.61-1.81 (m, 7 H); IR (pur) 2925, 1597, 1505, 1419, 1317, 1212, 1164, 1123, 1080, 982, 907, 827 cm⁻¹; MS (FAB) *m*/*z* 287 (M+H)

### B-65: 2-(4,4-Dimethyl-piperidin-1-yl)-4-trifluoromethyl-benzylamin

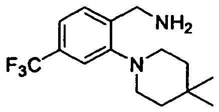

¹H NMR (300 MHz, CDCl₃) δ 7.44 (d, 1 H, *J* = 7.9 Hz), 7.27-7.36 (m, 2 H), 3.92 (s, 2 H), 2.82-2.84 (m, 4 H), 1.46-1.60 (m, 4 H), 1.01 (bs, 6 H); IR (pur) 2919, 1424, 1337, 1309, 1227, 1166, 1124, 1079, 949, 827, 734 cm⁻¹; MS (FAB) *m*/*z* 287 (M+H)

### B-66: (2-Aminomethyl-5-trifluoromethyl-phenyl)-dipropyl-amin

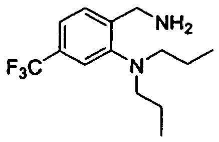

¹H NMR (300 MHz, CDCl₃) δ 7.47 (d, 1 H, *J* = 7.7 Hz), 7.31-7.37 (m, 2 H), 4.01 (s, 2 H), 2.83-2.92 (m, 4 H), 1.38-1.51 (m, 4 H), 0.81-0.92 (m, 6 H); IR (pur) 2964, 2875, 1463, 1422, 1327, 1220, 1166, 1125, 1079, 984, 891 cm⁻¹; MS (FAB) *m*/*z* 275 (M+H)

### B-67: 4-Trifluoromethyl-2-(4-trifluoromethyl-piperidin-1-yl)-benzylamin

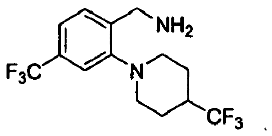

¹H NMR (300 MHz, CDCl₃) δ 7.50 (d, 1 H, *J* = 8.1 Hz), 7.36 (d, 1 H, *J* = 7.7 Hz), 7.29 (bs, 1 H), 3.95 (s, 2 H), 3.14-3.25 (m, 2 H), 2.67-2.80 (m, 2 H), 2.20 (m, 1 H), 1.93-2.05 (m, 2 H), 1.75-1.87 (m, 2 H); IR (pur) 2958, 2820, 1424, 1333, 1306, 1254, 1128, 1081, 949, 899, 829, 734 cm⁻¹; MS (FAB) *m*/*z* 327 (M+H)

### B-68: 3'-Aminomethyl-4-methyl-6'-trifluoromethyl-3,4,5,6-tetrahydro-2H-[1,2']bipyridinyl-5'-carbonsäure ethyl ester

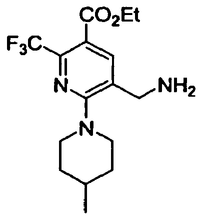

¹H NMR (300 MHz, CDCl₃) δ 8.13 (s, 1 H), 4.37 (q, 2H, J = 7.1 Hz), 3.88 (s, 2H), 3.69 (m, 2H), 2.90 (t, 2H, J = 11.5 Hz), 1.67 (m, 3H), 1.32 (m, 5H), 0.95 (d, 3H, J = 13.7 Hz); IR (pur) 3391, 2924, 1542, 1452, 1373, 1024, 971, 794 cm⁻¹; MS (FAB) *m*/*z* 346(M+H)

### B-69: C-[6'-(Chloro-difluoro-methyl)-3,5-dimethyl-3,4,5,6-tetrahydro-2H [1,2']bipyridinyl-3'-yl] -methylamin

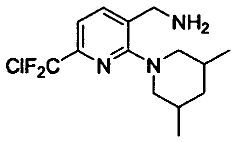

¹H NMR (300 MHz, CDCl₃) δ 7.75 (d, 1 H, *J* = 7.5 Hz), 7.18 (d, 1 H, *J* = 7.7 Hz), 3.89 (s, 2 H), 3.18 (tt, 4 H, *J* = 7.3, 2.0 Hz), 1.60-1.48 (m, 4 H), 0.86 (t, 6 H, *J* = 7.3 Hz); IR (pur) 3033, 2935, 1726, 1594, 1514, 1456, 1420 cm⁻¹; MS (FAB) *m*/*z* 304 (M+H)

### B-70: (3-Aminomethyl-6-trifluoromethyl-pyridin-2-yl)-dipropyl-amin

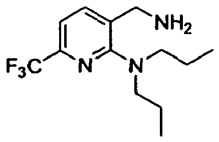

¹H NMR (300 MHz, CDCl₃) δ 7.75 (d, 1 H, *J* = 7.5 Hz), 7.18 (d, 1 H, *J* = 7.7 Hz), 3.89 (s, 2 H), 3.18 (tt, 4 H, *J* = 7.3, 2.0 Hz), 1.60-1.48 (m, 4 H), 0.86 (t, 6 H, *J* = 7.3 Hz); IR (pur) 3367, 2966, 2875, 1593, 1465, 1419, 1338 cm⁻¹; MS (FAB) *m*/*z* 261 (M+H)

### B-71: C-(6'-tert-Butyl-3,4,5,6-tetrahydro-2H-[1,2']bipyridinyl-3'-yl)-methylamin

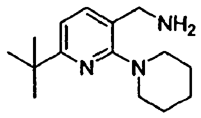

¹H NMR (300 MHz, CDCl₃) δ 7.48 (d, 1 H, *J* = 7.7 Hz), 6.90 (d, 1 H, *J* = 7.7 Hz), 3.83 (s, 2 H), 3.08 (m, 4 H), 1.70-1.50 (m, 6 H), 1.30 (s, 9 H); IR (pur) 2933, 2856, 1635, 1582, 1445, 1402, 1370 cm⁻¹; MS (FAB) *m*/*z* 248 (M+H)

### B-72: C-(6-tert-Butyl-2-pyrrolidin-1-yl-pyridin-3-yl)-methylamin

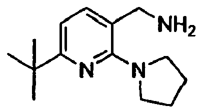

¹H NMR (300 MHz, CDCl₃) δ 7.36 (d, 1 H, *J* = 7.5 Hz), 6.70 (d, 1 H, *J* = 7.7 Hz), 3.86 (s, 2 H), 3.53(m, 4 H), 1.96-1.90 (m, 4 H), 1.30 (s, 9 H); IR (pur) 2959, 2866, 1583, 1450, 1355, 1251, 1099 cm⁻¹; MS (FAB) *m*/*z* 234 (M+H)

### B-73: C-(6'-tert-Butyl-4-methyl-3,4,5,6-tetrahydro-2H-[1,2']bipyridinyl-3'-yl)-methylamin

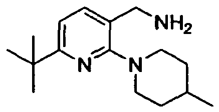

¹H NMR (300 MHz, CDCl₃) δ 7.48 (d, 1 H, *J* = 7.7 Hz), 6.90 (d, 1 H, *J* = 7.9 Hz), 3.86 (s, 2 H), 3.36 (m, 2 H), 2.82 (m, 2 H), 1.70-1.67 (m, 2 H), 1.57-1.31 (m, 3 H), 1.30 (s, 9 H), 0.98 (d, 3 H, *J* = 6.4 Hz); IR (pur) 2954, 2921, 2869, 1635, 1583, 1451, 1403 cm⁻¹; MS (FAB) *m*/*z* 262 (M+H)

### B-74: C-(2-Azepan-1-yl-6-tert-butyl-pyridin-3-yl)-methylamin

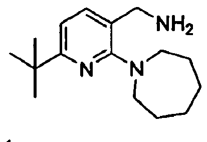

¹H NMR (300 MHz, CDCl₃) δ7.40 (d, 1 H, *J* = 7.7 Hz), 6.76 (d, 1 H, *J* = 7.7 Hz), 3.82 (s, 2 H), 3.49-3.42 (m, 4 H), 1.80 (m, 4 H), 1.62 (m, 4 H), 1.30 (s, 9 H); IR (pur) 3396, 2925, 2856, 1643, 1582, 1454, 1364 cm⁻¹; MS (FAB) *m*/*z* 262 (M+H)

### B-75: C-(6'-tert-Butyl-4-phenyl-3,4,5,6-tetrahydro-2H-[1,2']bipyridinyl-3'-yl)-methylamin

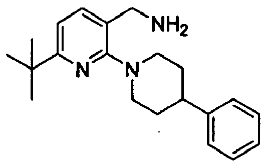

¹H NMR (300 MHz, CDCl₃) δ 7.52 (d, 1 H, *J* = 7.7 Hz), 7.36-7.19 (m, 5 H), 6.95 (d, 1 H, *J* = 7.7 Hz), 3.88 (s, 2 H), 3.55-3.51 (m, 2 H), 3.48 (s, 3 H), 3.03-2.93 (m, 2 H), 2.75-2.64 (m, 1 H), 2.05-1.54 (m, 4 H), 1.33 (s, 9 H); IR (pur) 2957, 1644, 1578, 1452, 1401, 1370, 1231 cm⁻¹; MS (FAB) *m*/*z* 324 (M+H)

### B-76: (3-Aminomethyl-6-tert-butyl-pyridin-2-yl)-dipropyl-amin

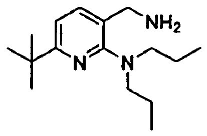

¹H NMR (300 MHz, CDCl₃) δ 7.45 (d, 1 H, *J* = 7.7 Hz), 6.94 (d,1 H, *J* = 7.7 Hz), 3.99 (s, 2 H), 3.25-3.05 (m, 4 H), 1.61-1.38 (m, 4 H), 1.33 (s, 9 H), 0.90-0.80 (m, 6 H); IR (pur) 2961, 2871, 1634, 1583, 1460, 1369, 1243 cm⁻¹, MS (FAB) *m*/*z* 264 (M+H)

### B-77: (3-Aminomethyl-6-trifluoromethyl-pyridin-2-yl)-diethyl-amin

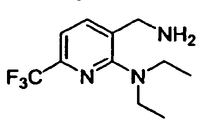

¹H NMR (300 MHz, CDCl₃) δ 7.67 (d, 1 H, *J* = 7.5 Hz), 7.07 (d; 1 H, *J* = 7.7 Hz), 3.79 (s, 2 H), 3.20-3.09 (q, 4 H, *J* = 7.0 Hz), 0.98 (t, 4 H, *J* = 7.0 Hz); IR (pur) 2924, 1588, 1429, 1332, 1219, 1170, 1129 cm⁻¹; MS (FAB) *m*/*z* 248 (M+H)

### B-78: (3-Aminomethyl-6-trifluoromethyl-pyridin-2-yl)-dimethyl-amin

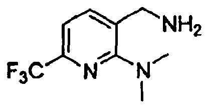

¹H NMR (300 MHz, CDCl₃) δ 7.72 (d, 1 H, *J* = 7.5 Hz), 7.12 (d, 1 H, *J* = 7.5 Hz), 4.01 (s, 2 H), 2.85 (s , 4 H); IR (pur) 2923, 1596, 1488 1394, 1350, 1272, 1175 cm⁻¹; MS (FAB) *m*/*z* 219 (M+H)

### B-79: (3-Aminomethyl-6-trifluoromethyl-pyridin-2-yl)-benzyl-amin

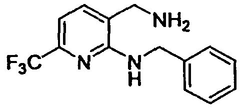

¹H NMR (300 MHz, CDCl₃) δ 7.29-7.10 (m, 6 H), 6.70 (d, 1 H, *J* = 7.4 Hz), 4.54 (d, 1 H, *J* = 2.0 Hz), 3.66 (s, 2 H), 1.41 (bs, 2 H); IR (pur) 3298, 2920, 1609, 1530, 1453, 1354 1309 cm⁻¹; MS (FAB) *m*/*z* 282 (M+H)

### B-80: C-(4,4'-Dimethyl-6'-trifluoromethyl-3,4,5,6-tetrahydro-2H-[1,2']bipyridinyl-3'-yl)-methylamin

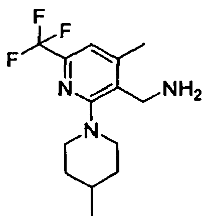

¹H NMR (300 MHz, CDCl₃) δ 7.17 (s, 1 H), 4.09 (s, 2 H), 3.31 (m, 2 H), 2.89 (m, 2 H), 2.43 (s, 3 H), 1.76 (m, 2 H), 1.53 9m, 1 H), 1.37 (m, 2 H), 1.44 (bs, 2H), 0.98 (d, 3 H, J = 6.3 Hz); IR (pur) 3380, 2952, 1598, 1567, 1465, 1373, 1311, 1276, 1176, 1138, 968, 916 cm⁻¹; MS (FAB) *m*/*z* 288(M+H)

### Methode 3:

Verbindungen der allgemeinen Formel VI-B (1.5 mmol), worin R⁵, R¹², R¹³, U, T und V die vorstehend genannte Bedeutung haben und m für 0, 1, 2 oder 3 steht, werden in Diethylether (3 mL) gelöst und bei 0 °C wird eine Suspension von Lithiumaluminiumhydrid (3 mmol) in Diethylether (5 mL) langsam zu getropft. Die Reaktionsmischung wird 4 Stunden zum Rückfluß erhitzt und bei 0 °C wird langsam Methanol und anschließend 1 N aq. NaOH-Lösung zu getropft. Die Reaktionsmischung wird mit Methanol verdünnt und über Celite filtriert. Das Lösungsmittel wird im Vakuum eritfern und der Rückstand säulenchromatographisch (SiO₂, verschiedene Mischung aus Dichlormethan und Methanol als Laufmittel) gereinigt.

Die folgende Verbindung B-81 wurde gemäß der vorstehend genannten allgemeinen Vorschrift erhalten:

### B-81: C-(4-Methylen-6'-trifluoromethyl-3,4,5,6-tetrahydro-2H-[1,2']bipyridinyl-3'-yl)-methylamin

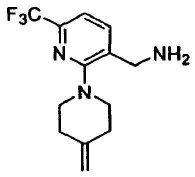

¹H NMR (CDCl₃) δ 7.84 (d, 1 H, J = 7.7 Hz), 7.27 (d, 1 H, J = 7.0 Hz), 4.76 (s, 2 H), 3.94 (s, 2 H), 3.25 (t, 4 H, J = 5.7 Hz), 2.38 (t, 4 H, J = 5.7 Hz)

### Methode 4:

Verbindungen der allgemeinen Formel VI-B (0.39 mmol), worin R⁵, R¹², R¹³, U, T und V die vorstehend genannte Bedeutung haben und m für 0, 1, 2 oder 3 steht, werden in Methanol (8 mL) gelöst. Bei 0 °C werden NiCl₂•H₂O (0.78 mmol) und Natriumborhydrid (1.56 mmol) langsam zugegeben. Die Reaktionsmischung wird 12 Stunden zum Rückfluß erhitzt. Die Reaktionsmischung wird mit Methanol verdünnt und über Celite filtriert. Das Lösungsmittel wird im Vakuum entfernt und der Rückstand säulenchromatographisch (SiO₂, verschiedene Mischung aus Dichlormethan und Methanol als Laufmittel) gereinigt.

Die folgenden Verbindungen B-82 bis B-84 wurden gemäß der vorstehend genannten allgemeinen Vorschrift erhalten:

### B-82: (3-Aminomethyl-6-trifluoromethyl-pyridin-2-yl)-butyl-methyl-amin

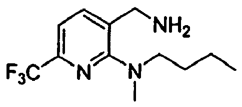

¹H NMR (300 MHz, CDCl₃) δ 7.75 (d, 1 H, *J* = 7.5 Hz), 7.18 (d, 1 H, *J* = 7.7 Hz), 3.91 (bs, 2 H), 3.19 (bt, 2 H), 2.89 (bs, 3 H), 1.52-1.65 (m, 2 H), 1.21-1.39 (m, 2 H), 0.92 (t, 3 H, *J* = 7.3 Hz); IR (pur) 2961, 2868, 1594, 1465, 1400, 1334, 1176, 1136, 831 cm⁻¹; MS (FAB) *m*/*z* 262 (M+H)

### B-83: C-(4-Phenyl-6'-trifluoromethyl-3,6-dihydro-2H-[1,2']bipyridinyl-3'-yl)-methylamin

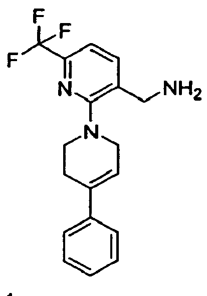

¹H NMR (300 MHz, CDCl₃) δ 7.85 (d, 1H, J = 7.5 Hz), 7.43 (d, 1H, J = 7.5 Hz), 7.22-7.35 (m, 5 H), 6.20 (m, 1 H), 3.97-4.01 (m, 4 H), 3.41-3.46 (m, 4 H), 2.74 (bs, 2 H); IR (pur) 3395, 2922, 1593, 1422, 1372, 1338, 1267, 1175, 1135, 959, 833, 750, 697 cm⁻¹; MS (FAB) *m*/*z* 334(M+H)

### B-84: C-[4-(4-Fluoro-phenyl)-6'-trifluoromethyl-3,6-dihydro-2H-[1,2']bipyridinyl-3'-yl]-methylamin

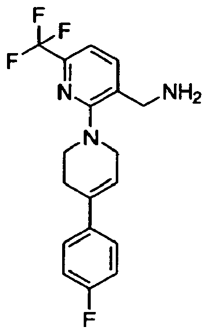

¹H NMR (300 MHz, CDCl₃) δ 7.86 (d, 1 H, J = 7.8 Hz), 7.40 (m, 3 H), 7.03 (dd, 2H, J = 9.0, 8.3 Hz), 6.14 (bs, 1 H), 3.97-4.01 (m, 4 H), 3.46 (m, 2 H), 2.70 (m, 2 H), 1.82 (bs, 2 H); IR (pur) 3365, 2922, 1600, 1510, 1425, 1340, 1230, 1174, 1135, 963, 835 cm⁻¹; MS (FAB) *m*/*z* 334(M+H)

### 3. Allgemeine Vorschrift zur Darstellung von Aminen der allgemeinen Formel V-Ba und V-Bb

Die Darstellung von Aminen der allgemeinen Formel V-Ba und V-Bb erfolgt wie im nachfolgenden Schema 2 dargestellt.

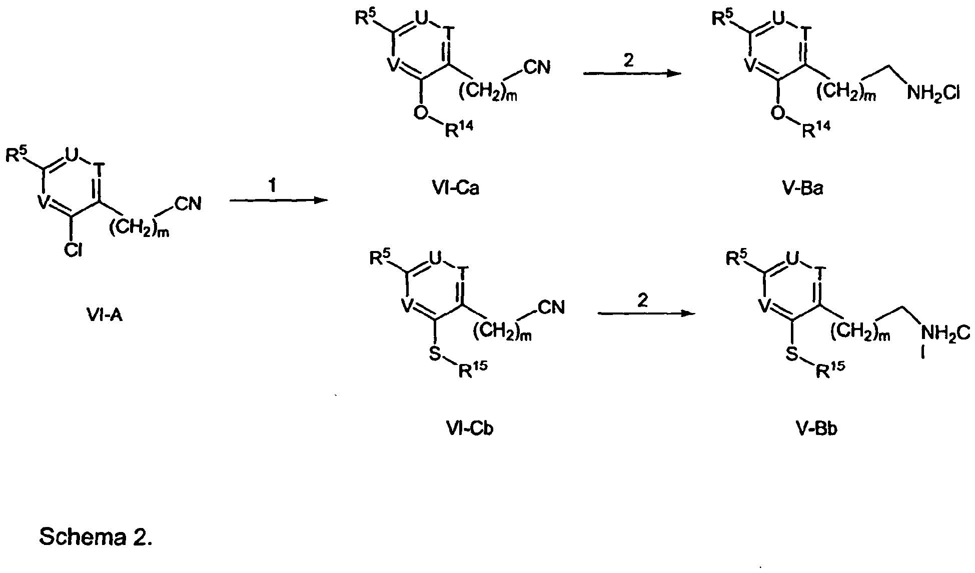

### Stufe 1: Darstellung von Nitrilen der allgemeinen Formel VI-Ca oder VI-Cb

Verbindungen der allgemeinen Formel VI-A (1 Äquivalent), worin R⁵, U, T und V die vorstehend genannte Bedeutung haben und m für 0, 1, 2 oder 3 steht, werden mit einem Alkohol der allgemeinen Formel HO-R¹⁴ (3.5 Äquivalente) und DBU [1,8-Diaza-bicyclo[5.4.0]undec-7-en] (3.5 Äquivalente) in Acetonitril (7 ml pro mmol der Verbindung der Formel VI-A) 12 Stunden bei RT gerührt. Die Reaktionsmischung wird mehrfach mit EE extrahiert. Die vereinigten organischen Phasen werden mit ges. aq. NaCl-Lsg. gewaschen, über MgSO₄ getrocknet und das Lösungsmittel wurde im Vakuum entfernt. Der Rückstand wird jeweils durch Säulenchromatographie (SiO₂, verschiedene Mischungen Hexan/EE) gereinigt.

Alternativ werden Verbindungen der allgemeinen Formel VI-Ca oder VI-Cb (1 Äquivalent), worin R⁵, U, T und V die vorstehend genannte Bedeutung haben, m für 0, 1, 2 oder 3 und R¹⁴ oder R¹⁵ für H steht, mit Verbindungen der allgemeinen Formel R¹⁴ -Br oder R¹⁵-Br (4 Äquivalente), worin R¹⁴ und R¹⁵ die vorstehend genannte Bedeutung haben und ungleich H sind, in einem Lösungsmittelgemisch aus Acetonitril und Dimethylformamid (1:2), ggf. in Gegenwart von 18-Krone-6-Ether als Katalysator, umgesetzt. Die Reaktionsmischung wird 12 h zum Rückfluß erhitzt und anschließend auf RT abgekühlt. Die Reaktionsmischung wird mit Ethylacetat extrahiert und die vereinigten organischen Phasen werden über MgSO₄ getrocknet, filtriert und das Lösungsmittel wird im Vakuum entfernt. Der Rückstand wird mittels Säulenchromatographie (SiO₂, Ethylacetat/Hexan (**1**:**1**)) gereinigt.

Die folgenden Verbindungen A-104 bis A-173 wurden gemäß der vorstehend genannten allgemeinen Vorschrift erhalten:

### A-104: 2-(3-Methyl-butoxy)-6-trifluoromethyl-nicotinonitril

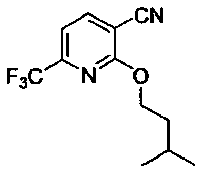

¹H NMR (300 MHz, CDCl₃) δ 8.05 (d, 1 H, *J* = 7.8 Hz), 7.33 (d, 1 H, J = 7.8 Hz), 4.53 (t, 2 H, J = 6.9 Hz), 1.65-1.96 (m, 3 H), 0.98 (d, 6 H, J = 6.3 Hz); MS (FAB) *m*/*z* 259 (M+H)

### A-105: 2-(3,3-Dimethyl-butoxy)-6-trifluoromethyl-nicotinonitril

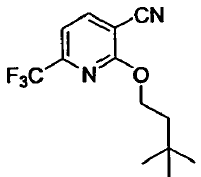

¹H NMR (300 MHz, CDCl₃) δ 8.05 (d, 1 H, *J* = 7.8 Hz), 7.33 (d, 1 H, J = 7.8 Hz), 4.56 (t, 2 H, J = 6.9 Hz), 1.77 (t, 2 H, J = 6.9 Hz), 1.01 (s, 9 H); MS (FAB) *m*/*z* 273 (M+H)

### A-106: 2-(2-Methyl-cyclopropylmethoxy)-6-trifluoromethyl-nicotinonitril

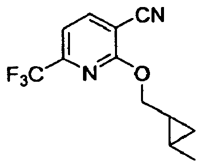

¹H NMR (300 MHz, CDCl₃) δ 8.05 (d, 1 H, J = 7.8 Hz), 7.32 (d, 1 H, J = 7.8 Hz), 4.33 (m, 2 H), 1.06 (d, 3 H, J = 6.0 Hz), 1.02 (m, 1 H), 0.85 (m, 1 H), 0.56 (m, 1 H), 0.46 (m, 1 H); MS (FAB) *m*/*z* 257 (M+H)

### A-107: 2-Butoxy-6-(chloro-difluoro-methyl)-nicotinonitril

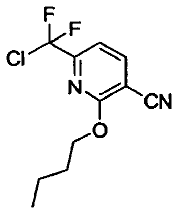

¹H NMR (300 MHz, CDCl₃) d 8.02 (d, 1H, J = 7.8 Hz), 7.28 (d, 1H, J 0 7.8 Hz), 4.59 (t, 2 H, J = 7.2 Hz), 1.84 (m, 2 H), 1.50 (m, 2 H), 0.99 (t, 3 H, J = 6.9 Hz); IR (KBr) 2964,2210,1590,1432,1373,1325,1190 cm⁻¹; MS (FAB) *m*/*z* 265 (M+H)

### A-108: 2-Phenoxy-6-trifluoromethyl-nicotinonitril

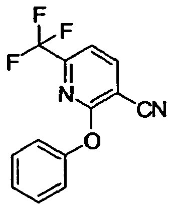

¹H NMR (300 MHz, CDCl₃) δ 8.18 (d, 1 H, J = 7.8 Hz), 7.41-7.47 (m, 2 H), 7.21-7.31 (m, 4 H); IR(pur) 3100, 2950, 2210, 1580, 1490, 1462, 1411, 1194, 1271, 1150, 947 cm⁻¹; MS (FAB) *m*/*z* 265 (M+H)

### A-109: 2-(1-Butyl-pentyloxy)-6-trifluoromethyl-nicotinonitril

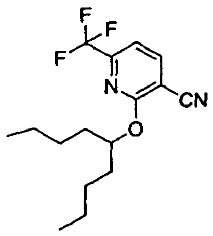

¹H NMR (300 MHz, CDCl₃) δ 8.03 (d, 1 H, J = 7.8 Hz), 7.29 (d, 1 H, J = 7.8 Hz), 5.36 (m, 1 H), 1.65-1.78(m, 4 H), 1.32-1.39 (m, 8 H), 0.90 (t, 6 H, J = 7.2 Hz) IR (pur) 2960, 2867, 2236, 1590, 1463, 1434, 1347, 1265, 1186, 1152, 1119, 966, 840, 743 cm⁻¹; MS (FAB) *m*/*z* 315 (M+H)

### A-110: 2-(1-Ethyl-propoxy)-6-trifluoromethyl-nicotinonitril

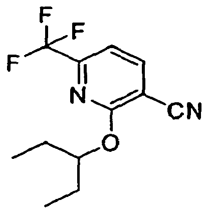

¹H NMR (300 MHz, CDCl₃) δ 7.96 (d, 1 H, J = 7.8 Hz), 7.29 (d, 1 H, J = 7.8 Hz), 5.15 (m, 1 H), 1.72 (m, 4 H), 0.89 (t, 6 H, J = 6.8 Hz); IR(pur) 2974,2236,1590,1462, 1435, 1348, 1266, 1186, 1151, 1117, 967, 840 cm⁻¹; MS (FAB) *m*/*z* 259 (M+H)

### A-111: 2-(1-Propyl-butoxy)-6-trifluoromethyl-nicotinonitril

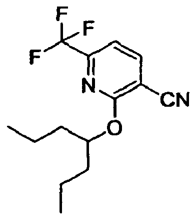

¹H NMR (300 MHz, CDCl₃) δ 8.04 (d, 1 H, J = 7.8 Hz), 7.20 (d, 1 H, J = 7.8 Hz), 5.41 (m, 1 H), 1.69 (m, 4 H), 1.43 (m, 4 H), 0.93 (t, 6 H, J = 6.9 Hz) IR(pur) 2964, 2875, 2236, 1590, 1462, 1435, 1347, 1267, 1187, 1152, 1119, 979, 839, 744cm⁻¹; MS (FAB) *m*/*z* 287 (M+H)

### A-112: 2-(1-Isobutyl-3-methyl-butoxy)-6-trifluoromethyl-nicotinonitril

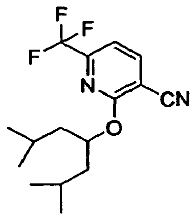

¹H NMR (300 MHz, CDCl₃) δ 7.97 (d, 1 H, J = 7.8 Hz), 7.23 (d, 1 H, J = 7.8 Hz), 5.49 (m, 1 H), 1.60-1.78 (m, 6 H), 0.84 (d, 12 H, J = 6.9 Hz); IR(pur) 3365, 2958, 2871, 2237, 1590, 1464, 1434, 1347, 1266, 1187, 1154, 964, 839cm⁻¹; MS (FAB) *m*/*z* 315 (M+H)

### A-113: 2-(4,4-Dimethyl-cyclohexyloxy)-6-trifluoromethyl-nicotinonitril

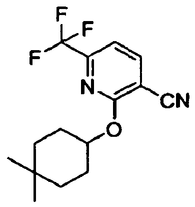

¹H NMR (300 MHz, CDCl₃) δ 8.02 (d, 1 H, J = 7.8 Hz), 7.28 (d, 1 H, J = 7.8 Hz), 5.21 (m, 1 H), 1.73-1.96 (m, 4 H), 1.55 (m, 2 H), 1.33 (m, 2 H), 0.99 (s, 3 H), 0.96 (s, 3 H); MS (FAB) *m*/*z* 299 (M+H)

### A-114: 2-(3-Methoxy-propoxy)-6-trifluoromethyl-nicotinonitril

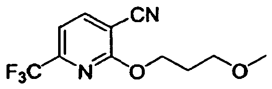

¹H NMR (300 MHz, CDCl₃) δ 8.06 (d, 1 H, *J* = 7.7 Hz), 7.35 (d, 1 H, *J* = 7.7 Hz), 4.59 (t, 2 H, *J* = 6.2 Hz), 3.59 (t, 2 H, *J* = 6.1 Hz), 3.37 (s, 3 H), 2.07-2.17 (m, 2H); IR (pur) 2929, 2223, 1591, 1463, 1375, 1347, 1312, 1267, 1188, 1150, 1119, 977, 922, 742 cm⁻¹; MS (FAB) *m*/*z* 261 (M+H)

### A-115: 2-(3-Ethoxy-propoxy)-6-trifluoromethyl-nicotinonitril

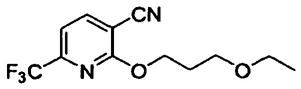

¹H NMR (300 MHz, CDCl₃) δ 8.05 (d, 1 H, *J* = 7.7 Hz), 7.34 (d, 1 H, *J* = 7.7 Hz), 4.60 (t, 2 H, *J* = 6.2 Hz), 3.62 (d, 2 H, *J* = 6.2 Hz), 3.50 (q, 2 H, *J* = 7.5 Hz), 2.04-2.16 (m, 2 H), 1.99 (t, 3 H, *J* = 7.0 Hz); IR (pur) 2976, 2870, 2237, 1590, 1470, 1436, 1375, 1347, 1269, 1187, 1150, 1118, 994, 842, 743 cm⁻¹ MS (FAB) *m*/*z* 275 (M+H)

### A-116: 2-(2-Phenoxy-ethoxy)-6-trifluoromethyl-nicotinonitril

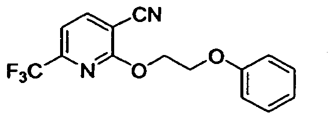

¹H NMR (300 MHz, CDCl₃) δ 8.07 (d, 1 H, *J* = 7.7 Hz), 7.38 (d, 1 H, *J* = 7.7 Hz), 7.24-7.35 (m, 2 H), 6.01-7.02 (m, 3 H), 4.86 (t, 2 H, *J* = 5.0 Hz), 4.39 (t, 2 H, *J* = 5.0 Hz); IR (pur) 2235, 1589, 1429, 1348, 1241, 1193, 1142, 1112, 963, 840, 753, 692 cm⁻¹; MS (FAB) *m*/*z* 309 (M+H)

### A-117: 2-Butoxy-6-trifluoromethyl-nicotinonitril

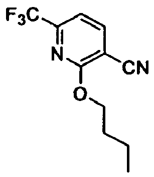

¹H NMR (CDCl₃) δ 8.04 (d, 1 H, J = 7.7 Hz), 7.33 (d, 1 H, J = 7.7 Hz), 4.50 (t, 2 H, J = 6.6 Hz), 1.87-1.78 (m, 2 H), 1.58-1.45 (m, 2 H), 0.99 (t, 3 H, J = 7.3 Hz) IR (pur) 2965, 2240, 1591, 1468, 1436, 1349, 1270, 1189, 1151 cm⁻¹

### A-118: 2-Isopropoxy-6-trifluoromethyl-nicotinonitril

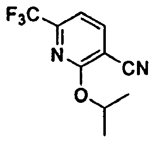

¹H NMR (CDCl₃) δ 8.04 (d, 1 H, J = 7.7 Hz), 7.30 (d, 1 H, J = 7.7 Hz), 5.46 (m, 1 H), 1.43 (d, 6H, J = 6.2Hz) IR (pur) 2988, 2237, 1591, 1435, 1343, 1269, 1187, 1150, 1115, 969 cm⁻¹

### A-119: 2-Cyclopentyloxy-6-trifluoromethyl-nicotinonitril

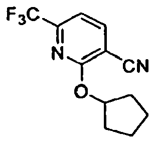

¹H NMR (CDCl₃) δ 8.02 (dd, 1 H, J = 7.7, 0.6 Hz), 7.30 (d, 1 H, J = 7.5 Hz), 5.60-5.54 (m, 1 H), 2.09-1.57 (m, 8 H) IR (pur) 2969, 2236, 1590, 1435, 1350, 1268, 1187, 1150, 974 cm⁻¹

### A-120: 2-Cyclohexyloxy-6-trifluoromethyl-nicotinonitril

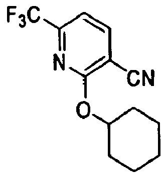

¹H NMR (CDCl₃) δ 8.02 (d, 1 H, J = 7.7 Hz), 7.29 (d, 1 H, J = 7.7 Hz), 5.24 (m, 1 H), 2.03-1.95 (m, 2 H), 1.87-1.40 (m, 8 H); IR (pur) 2940, 2862, 2236, 1591, 1436, 1348, 1269, 1188, 1150, 971 cm⁻¹

### A-121: 2-Methoxy-6-trifluoromethyl-nicotinonitril

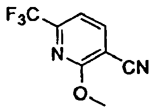

¹H NMR (CDCl₃) δ 8.06 (d, 1 H, J = 7.7 Hz), 7.37 (d, 1 H, J = 7.7 Hz), 4.13 (s, 3 H); IR (pur) 2924, 2238, 1592, 1475, 1392, 1349, 1272, 1186, 1149, 1009 cm⁻¹

### A-122: 2-Hexyloxy-6-trifluoromethyl-nicotinonitril

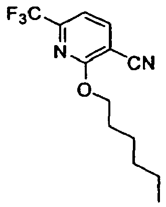

¹H NMR (CDCl₃) δ 8.04 (dd, 1 H, J = 7.7, 0.7 Hz), 7.33 (d, 1 H, J = 7.7 Hz), 4.49 (t, 2 H, J = 6.6 Hz), 1.89-1.79 (m, 2 H), 1.50-1.30 (m, 6 H), 0.91 (t, 3 H); IR (pur) 2931, 1591, 1469, 1437, 1348, 1269, 1189, 1151 cm⁻¹

### A-123: 2-Isobutoxy-6-trifluoromethyl-nicotinonitril

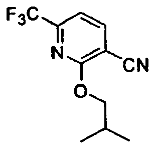

¹H NMR (CDCl₃) δ 8.04 (d, 1 H, J = 7.7 Hz), 7.33 (d, 1 H, J = 7.7 Hz), 4.26 (d, 1 H, J = 6.6 Hz), 2.17 (m, 1 H), 1.06 (d, 6 H, J = 6.8 Hz); IR (pur) 2968, 2237, 1592, 1469, 1436, 1347, 1268, 1188, 1151, 1119, 1000 cm⁻¹

### A-124: 2-Cyclopropylmethoxy-6-trifluoromethyl-nicotinonitril

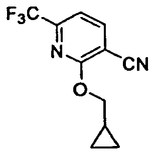

¹H NMR (CDCl₃) δ 8.05 (d, 1 H, J = 7.7 Hz), 7.33 (d, 1 H, J = 7.7 Hz), 4.35 (d, 2 H, J = 7.1 Hz), 1.40-1.30 (m, 1 H), 0.68-0.62 (m, 2 H), 0.45-0.40 (m, 2 H); IR (pur) 2960, 2240, 1592, 1468, 1438, 1391, 1355, 1266, 1187, 1149, 1119 cm⁻¹

### A-125: 2-Cyclobutylmethoxy-6-trifluoromethyl-nicotinonitril

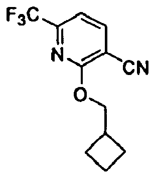

¹H NMR (CDCl₃) δ 8.04 (d, 1 H, J = 7.7 Hz), 7.33 (d, 1 H, J = 7.7 Hz), 4.46 (d, 2 H, J = 6.6 Hz), 2.88-2.78 (m, 1 H), 2.20-1.85 (m, 6 H); IR (pur) 2941, 2238, 1591, 1469, 1435, 1349, 1269, 1188, 1150, 1117, 988 cm⁻¹

### A-126: 2-Propoxy-6-trifluoromethyl-nicotinonitril

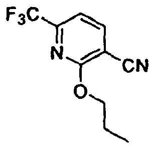

¹H NMR (CDCl₃) δ 8.05 (dd, 1 H), 7.33 (d, 1 H, J = 7.7 Hz), 4.46 (t, 2 H, J = 6.6 Hz), 1.93-1.82 (m, 2 H), 1.06 (t, 3 H, J = 7.5 Hz) IR (pur) 2974, 2238, 1592, 1437, 1346, 1271, 1190, 1151, 979 cm⁻¹

### A-127: 2-Pentyloxy-6-trifluoromethyl-nicotinonitril

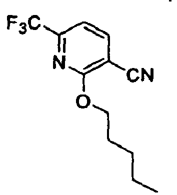

¹H NMR (CDCl₃) δ 8.04 (dd, 1 H), 7.33 (d, 1 H, J = 7.7 Hz), 4.49 (t, 2 H, J = 6.8 Hz), 1.89-1.80 (m, 2 H), 1.51-1.35 (m, 4 H), 0.94 (t, 3 H, J = 6.9 Hz); IR (pur) 2962, 2240, 1592, 1437, 1349, 1270, 1190, 1151 cm⁻¹

### A-128: 2-Cyclobutoxy-6-trifluoromethyl-nicotinonitril

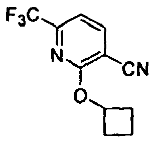

¹H NMR (CDCl₃) δ 8.03 (dd, 1 H, J = 7.7, 0.7 Hz), 7.32 (d, 1 H, J = 7.7 Hz), 5.32 (m, 1 H), 2.56-2.46 (m, 2 H), 2.32-2.19 (m, 2 H), 1.94-1.66 (m, 2 H) IR (pur) 2995, 2238, 1590, 1465, 1434, 1345, 1269, 1188, 1150 cm⁻¹

### A-129: 2-(4-Methyl-cyclohexyloxy)-6-trifluoromethyl-nicotinonitril

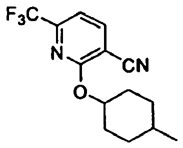

¹H NMR (CDCl₃) δ 8.02 (d, 1 H, J = 7.7 Hz), 7.29 (d, 1 H, J = 7.7 Hz), 5.11 (m, 1 H), 2.20-2.12 (m, 2H), 1.85-1.75 (m, 2 H), 1.63-1.43 (m, 3 H), 1.20-1.05 (m, 2 H), 0.94 (d, 3 H, J = 6.6 Hz); IR (pur) 2950, 2238, 1591, 1462, 1436, 1350, 1267, 1187, 1151, 993 cm⁻¹

### A-130: 2-Cyclopentylmethoxy-6-trifluoromethyl-nicotinonitril

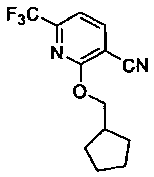

¹H NMR (CDCl₃) δ 8.04 (dd, 1 H, J = 7.7, 0.5 Hz), 7.33 (d, 1 H, J = 7.7 Hz), 4.37 (d, 2 H, J = 6.9 Hz), 2.43 (m, 1 H), 1.91-1.81 (m, 2 H), 1.71-1.56 (m, 4 H), 1.45-1.34 (m, 2 H); IR (pur) 2957, 2236, 1592, 1436, 1346, 1269, 1188, 1150, 988 cm⁻¹

### A-131: 2-Ethoxy-6-trifluoromethyl-nicotinonitril

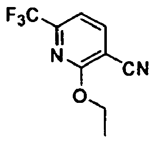

¹H NMR (CDCl₃) δ 8.05 (d, 1 H, J = 7.7 Hz), 7.33 (d, 1 H, J = 7.7 Hz), 4.57 (q, 2 H, J = 7.0 Hz), 1.47 (t, 3 H, J = 7.0 Hz); IR (pur) 2990, 2238, 1591, 1437, 1389, 1348, 1269, 1189, 1150, 1024 cm⁻¹

### A-132: 2-(4-tert-Butyl-cyclohexyloxy)-6-trifluoromethyl-nicotinonitril

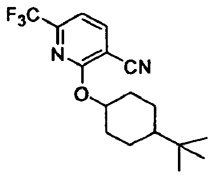

¹H NMR (CDCl₃) δ 8.02 (d, 1 H, J = 7.7 Hz), 7.29 (d, 1 H, J = 7.7 Hz), 5.07 (m, 1 H), 2.24-2.20 (m, 2 H), 1.92-1.85 (m, 2 H), 1.60-1.46 (m, 2 H), 1.27-1.04 (m, 3 H), 0.89 (s, 9 H); IR (pur) 2954, 2237, 1591, 1435, 1350, 1269, 1188, 1151, 977 cm⁻¹

### A-133: 2-(4-Ethyl-cyclohexyloxy)-6-trifluoromethyl-nicotinonitril

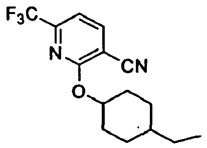

¹H NMR (CDCl₃) δ 8.02 (d, 1 H, J = 7.0 Hz), 7.29 (d, 1 H, J = 7.7 Hz), 5.11 (m, 1H), 2.21-2.13 (m, 2 H), 1.92-1.85 (m, 2 H), 1.62-1.03 (m, 7 H), 0.91 (t, 3 H, J = 7.0 Hz); IR (pur) 2934, 2237, 1591, 1435, 1350, 1269, 1188, 1151 cm⁻¹

### A-134: 6-tert-Butyl-2-cyclohexyloxy-nicotinonitril

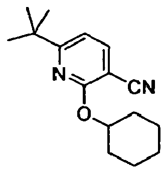

¹H NMR (CDCl₃) δ 7.76 (d, 1 H, J = 7.9 Hz), 6.91 (d, 1 H, J = 7.9 Hz), 5.16 (m, 1 H), 2.03-1.35 (m, 10 H), 1.32 (s, 9 H); IR (pur) 2938, 2230, 1592, 1564, 1451, 1415, 1365, 1261 cm⁻¹

### A-135: 6-tert-Butyl-2-cyclopentyloxy-nicotinonitril

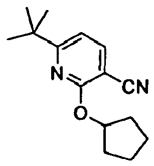

¹H NMR (CDCl₃) δ 7.75 (d, 1 H, J = 7.9 Hz), 6.91 (d, 1 H, J = 7.9 Hz), 5.50 (m, 1 H), 2.03-1.60 (m, 8 H), 1.32 (s, 9 H); IR (pur) 2964, 2230, 1592, 1564, 1451, 1414, 1353, 1262, 984 cm⁻¹

### A-136: 2-Butoxy-6-tert-butyl-nicotinonitril

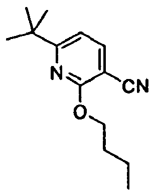

¹H NMR (CDCl₃) δ 7.77 (d, 1 H, J = 7.9 Hz), 6.94 (d, 1 H, J = 7.9 Hz), 4.44 (t, 2 H, J = 6.6 Hz), 1.80 (m, 2 H), 1.49 (m, 2 H), 1.32 (s, 9 H), 0.98 (t, 3 H, J = 7.3 Hz); IR (pur) 2961, 2230, 1593, 1565, 1455, 1418, 1369, 1261, 1112 cm⁻¹

### A-137: 6-tert-Butyl-2-hexyloxy-nicotinonitril

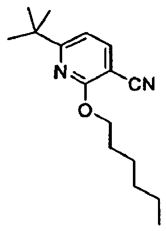

¹H NMR (CDCl₃) δ 7.77 (d, 1 H, J = 7.9 Hz), 6.94 (d, 1 H, J = 7.9 Hz), 4.43 (t, 2 H, J = 6.8 Hz), 1.81 (m, 2 H), 1.50-1.30 (m, 6 H), 1.32 (s, 9 H), 0.90 (m, 3 H); IR (pur) 2929, 2230, 1593, 1565, 1455, 1418, 1369, 1261, 1112, 1000 cm⁻¹

### A-138: 2-Benzyloxy-6-tert-butyl-nicotinonitril

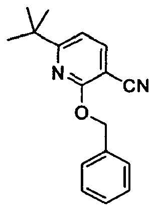

¹H NMR (CDCl₃) δ 7.79 (d, 1 H, J = 8.3 Hz), 7.50-7.30 (m, 5 H), 6.97 (d, 1 H, J = 7.9 Hz), 5.53 (s, 2 H), 1.31(s, 9 H); IR (pur) 2963, 2230, 1593, 1563, 1454, 1412, 1360, 1263, 1114, 999 cm⁻¹

### A-139: 2-Cyclohexylmethoxy-6-trifluoromethyl-nicotinonitril

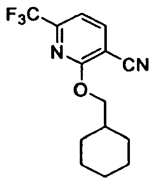

¹H NMR (CDCl₃) δ 8.04 (d, 1 H, J = 7.9 Hz), 7.32 (d, 1 H, J = 7.7 Hz), 4.28 (d, 2 H, J = 6.0 Hz), 1.90-1.70 (m, 6 H), 1.35-1.05 (m, 5 H); IR (pur) 2930, 2237, 1592, 1438, 1349, 1268, 1188, 1151, 994 cm⁻¹

### A-140: 2-(4-Methyl-cyclohexylmethoxy)-6-trifluoromethyl-nicotinonitril

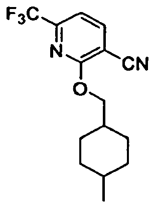

¹H NMR (CDCl₃) δ 8.04 (d, 1 H, J = 7.7 Hz), 7.32 (d, 1 H, J = 7.7 Hz), 4.40 & 4.29 (d, 2 H), 2.06-1.50 (m, 7 H), 1.37-1.28 (m, 2 H), 1.15-1.05 (m, 1 H), 0.95 & 0.90 (d, 3 H); IR (pur) 2924, 2237, 1592, 1437, 1349, 1268, 1188, 1151, 1118, 988 cm⁻¹

### A-141: 4-(3-Cyano-6-trifluoromethyl-pyridin-2-yloxymethyl)-piperidin-1-carbonsäure tert-butyl ester

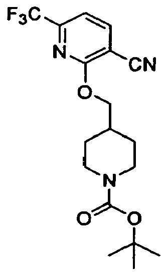

¹H NMR (CDCl₃) δ 8.06 (d, 1 H, J = 7.7 Hz), 7.36 (d, 1 H, J = 7.7 Hz), 4.34 (d, 2 H), 4.23-4.12 (m, 2 H), 2.81-2.70 (m, 2 H), 2.04 (m, 1 H), 1.87-1.81 (m, 2 H), 1.47 (s, 9 H), 1.37-1.23 (m, 2 H); IR (pur) 2926, 2236, 1690, 1591, 1434, 1363, 1268, 1181, 1149, 986 cm⁻¹

### A-142: 6-Trifluoromethyl-2-(4-trifluoromethyl-cyclohexyloxy)-nicotinonitril

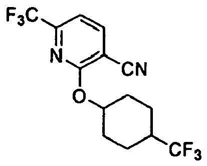

¹H NMR (CDCl₃) δ 8.05 (d, 1 H, J = 7.7 Hz), 7.34 (d, 1 H, J = 7.7 Hz), 5.51 & 5.14 (m, 1 H), 2.35-2.05 (m, 4 H), 1.87-1.50 (m, 5 H); IR (pur) 2957, 2237, 1591, 1463, 1436, 1346, 1270, 1186, 1150, 1014 cm⁻¹

### A-143: 4-(3-Cyano-6-trifluoromethyl-pyridin-2-yloxy)-piperidin-1-carbonsäure tert-butyl ester

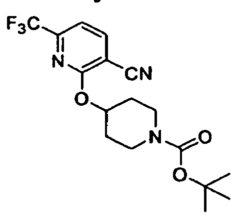

¹H NMR (CDCl₃) δ 8.07 (d, 1 H, J = 7.7 Hz), 7.35 (d, 1 H, J = 7.7 Hz), 5.42 (m, 1 H), 3.73 (m, 2 H), 3.43 (m, 2 H), 2.05-1.83 (m, 4 H), 1.48 (s, 9 H); IR (pur) 2975, 2237, 1693, 1591, 1430, 1350, 1273, 1236, 1181, 1022 cm⁻¹

### A-144: 2-(3-Methoxy-benzyloxy)-6-trifluoromethyl-nicotinonitril

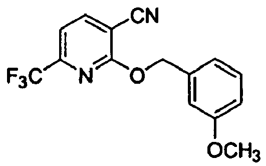

¹H NMR (300 MHz, CDCl₃) δ 8.06 (d, 1 H, *J* = 7.7 Hz), 7.36 (d, 1 H, *J* = 7.7 Hz), 7.29 (d, 1 H, *J* = 7.7 Hz), 7.10 (d, 1 H, *J* = 7.9 Hz), 7.09 (s, 1 H), 6.88 (m, 1 H), 5.54 (s, 2 H), 3.82 (s, 3 H); IR (pur) 2920, 2228, 1591, 1463, 1428, 1350, 1269, 1149, 980, 843, 781 cm⁻¹; MS (FAB) *m*/*z* 309 (M+H)

### A-145: 2-(4-Methyl-benzyloxy)-6-trifluoromethyl-nicotinonitril

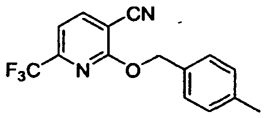

¹H NMR (300 MHz, CDCl₃) δ 8.05 (d, 1 H, *J* = 7.7 Hz), 7.42 (d, 2 H, *J* = 8.0 Hz), 7.32 (d, 1 H, *J* = 7.7 Hz), 7.19 (d, 2 H, *J* = 7.9 Hz), 5.52 (s, 2 H), 2.36 (s, 3 H) IR (pur) 2923, 2236, 1590, 1464, 1432, 1348, 1270, 1186, 1149, 1117, 977, 842, 808, 745 cm⁻¹; MS (FAB) *m*/*z* 293 (M+H)

### A-146: 2-(4-Fluoro-benzyloxy)-6-trifluoromethyl-nicotinonitril

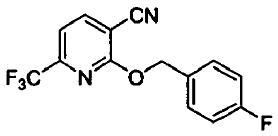

¹H NMR (300 MHz, CDCl₃) δ 8.07 H, *J* = 7.7 Hz), 7.52 (m, 2 H), 7.37 (d, 1 H, *J* = 7.7 Hz), 7.07 (m, 2 H), 5.52 (s, 2 H); IR (pur) 2230, 1590, 1512, 1465, 1434, 1348, 1270, 1228, 1187, 1151, 1116, 979, 835, 745 cm⁻¹; MS (FAB) *m*/*z* 297 (M+H)

### A-147: 2-(Pyridin-4-ylmethoxy)-6-trifluoromethyl-nicotinonitril

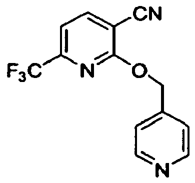

¹H NMR (300 MHz, CDCl₃) δ 8.66 (s, 2 H), 8.13 (d, 1 H, *J* = 7.7 Hz), 7.48-7.39 (m, 3 H), 5.57 (s, 2 H); IR (pur) 3028, 2218, 1591, 1466, 1427, 1358, 1275, 1176, 1145, 1021, 1145, 1021, 938, 865, 801, 772 cm⁻¹; MS (FAB) *m*/*z* 280 (M+H)

### A-148: 2-Phenethyloxy-6-trifluoromethyl-nicotinonitril

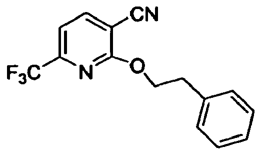

¹H NMR (300 MHz, CDCl₃) δ 8.03 (d, 1 H, *J* = 7.7 Hz), 7.41-7.26 (m, 6 H), 4.67 (t, 2 H, *J* = 6.9 Hz), 3.15 (t , 2 H, *J* = 6.9 Hz); IR (pur) 3031, 2236, 1590, 1468, 1434, 1348, 1268, 1187, 1148, 1117, 996, 955, 842, 749, 701 cm⁻¹; MS (FAB ) *m*/*z* 293 (M+H)

### A-149: 2-(Pyridin-2-ylmethoxy)-6-trifluoromethyl-nicotinonitril

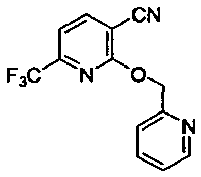

¹H NMR (300 MHz, CDCl₃) δ 8.62 (m, 1 H), 8.11 (d, 1 H, *J* = 7.7 Hz), 7.76 (td, 1 H, *J* = 7.7, 1.8 Hz), 7.57 (d, 1 H, *J* = 7.7 Hz), 7.41 (d, 1 H, *J* = 7.7 Hz), 7.27 (m, 1 H), 5.66 (s, 2 H); IR (pur) 2237, 1588, 1473, 1423, 1355, 1279, 1191, 1149, 1024, 937, 857, 768 cm⁻¹; MS (FAB) *m*/*z* 280 (M+H)

### A-150: 2-Benzyloxy-6-trifluoromethyl-nicotinonitril

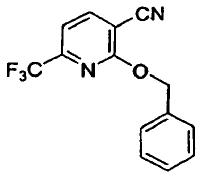

¹H NMR (300 MHz, CDCl₃) δ 8.06 (d, 1 H, *J* = 7.7 Hz), 7.58-7.50 (m, 2 H), 7.47-7.36 (m, 4 H), 5.56 (s, 2 H); IR (pur) 2237, 1590, 1464, 1429, 1350, 1271, 1180, 1149, 1115, 984, 842, 741, 699 cm⁻¹; MS (FAB) *m*/*z* 279 (M+H)

### A-151: 2-Benzyloxy-4-trifluoromethyl-benzonitril

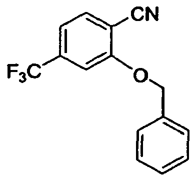

¹H NMR (300 MHz, CDCl₃) δ 7.72 (d, 1 H, *J* = 7.9 Hz), 7.48-7.34 (m, 5 H), 7.29 (d, 1 H, *J* = 8.0 Hz), 7.25 (s, 1 H), 5.26 (s, 2 H); IR (pur) 2229, 1504, 1431, 1371, 1328, 1243, 1121, 1078, 991, 877, 822, 738, 695 cm⁻¹; MS (FAB) *m*/*z* 278 (M+H)

### A-152: 2-(Pyridin-3-ylmethoxy)-6-trifluoromethyl-nicotinonitril

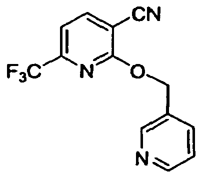

¹H NMR (300 MHz, CDCl₃) δ 8.78 (s, 1 H), 8.61 (d, 1 H, *J* = 4.8 Hz), 8.09 (d, 1 H, *J* = 7.7 Hz), 7.88 (dt, 1 H, *J* = 7.9 Hz), 7.39 (d, 1 H, *J* = 7.7 Hz), 7.34 (m, 1 H), 5.58 (s, 2 H); IR (pur) 2231, 1587, 1411, 1348, 1269, 1175, 1110, 1014, 936, 847, 790, 707 cm⁻¹; MS (FAB) *m*/*z* 280 (M+H)

### A-153: 6-Trifluoromethyl-2-(4-trifluoromethyl-benzyloxy)-nicotinonitril

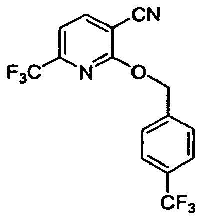

¹H NMR (300 MHz, CDCl₃) δ 8.09 (d, 1 H, *J* = 7.6 Hz), 7.76-7.61 (m, 4 H), 7.39 (d, 1 H, *J* = 7.8 Hz), 5.61 (s, 2 H); IR (pur) 2237, 1590, 1466, 1434, 1326, 1272, 1121, 1067, 1012, 845, 744 cm⁻¹; MS (FAB) *m*/*z* 347 (M+H)

### A-154: 2-(4-Ethyl-benzyloxy)-6-trifluoromethyl-nicotinonitril

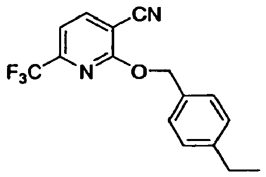

¹H NMR (300 MHz, CDCl₃) δ 8.04 (d, 1 H, *J* = 7.7 Hz), 7.45 (d, 2 H, *J* = 7.9 Hz), 7.34 (d, 1 H, *J* = 7.7 Hz), 7.22 (d, 2 H, *J* = 8.0 Hz), 5.53 (s, 2 H), 2.66 (q, 2 H, *J* = 7.7 Hz), 1.24 (t, 3 H, *J* = 7.6 Hz); IR (pur) 2967, 2231, 1590, 1464, 1432, 1348, 1271, 1187, 1150, 1117, 977, 843 cm⁻¹; MS (FAB) *m*/*z* 307 (M+H)

### A-155: 2-(4-Butyl-benzyloxy)-6-trifluoromethyl-nicotinonitril

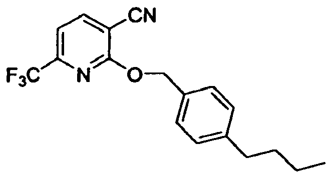

¹H NMR (300 MHz, CDCl₃) δ 8.05 (d, 1 H, *J* = 7.7 Hz), 7.43 (d, 2 H, *J* = 7.9 Hz), 7.34 (d, 1 H, *J* = 7.7 Hz), 7.20 (d, 2 H, *J* = 7.9 Hz), 5.52 (s, 2 H), 2.61 (t, 2 H, *J* = 7.7 Hz), 1.60 (m, 2 H), 1.35 (m, 2 H), 0.92 (t, 3 H, *J* = 7.3 Hz); IR (pur) 2930, 2230, 1590, 1464, 1432, 1348, 1271, 1187, 1150, 1116, 976, 840 cm⁻¹; MS (FAB) *m*/*z* 335 (M+H)

### A-156: 2-(4-tert-Butyl-benzyloxy)-6-trifluoromethyl-nicotinonitril

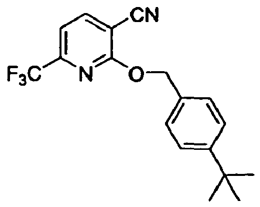

¹H NMR (300 MHz, CDCl₃) δ 8.05 (d, 1 H, *J* = 7.6 Hz), 7.57-7.40 (m, 4 H), 7.35 (d, 1 H, *J* = 7.8 Hz), 5.53 (s, 2 H), 1.33 (s, 9 H); IR (pur) 2964, 2237, 1590, 1465, 1432, 1348, 1271, 1186, 1150, 1117, 975, 840, 744 cm⁻¹; MS (FAB) *m*/*z* 335 (M+H)

### A-157: 2-(Indan-2-yloxy)-6-trifluoromethyl-nicotinonitril

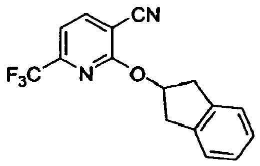

¹H NMR (300 MHz, CDCl₃) δ 8.05 (d, 1 H, *J* = 7.7 Hz), 7.36 (d, 1 H, *J* = 7.7 Hz), 7.29-7.17 (m, 4 H), 5.91 (m, 1 H), 3.52 (dd, 2 H, *J* = 16.9, 6.9 Hz), 3.14 (dd, 2 H, *J* = 16.9, 4.1 Hz); IR (pur) 2915, 2236, 1590, 1463, 1431, 1348, 1267, 1188, 1148, 1008, 972, 938, 841, 744 cm⁻¹; MS (FAB) *m*/*z* 305 (M+H)

### A-158: 2-(4-Chloro-benzyloxy)-6-trifluoromethyl-nicotinonitril

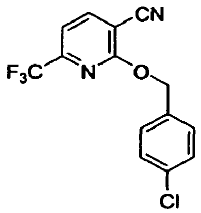

¹H NMR (300 MHz, CDCl₃) d 8.07, (d, 1 H, *J* = 7.7 Hz), 7.49-7.32 (m, 4 H), 7.37 (d, 1 H, *J* = 7.7 Hz), 5.52 (s, 2 H); IR (pur) 2237, 1591, 1492, 1464, 1432, 1402, 1348, 1269, 1187, 1149, 1116, 987, 843, 809, 745 cm⁻¹, MS (FAB) *m*/*z* 313 (M+H)

Die Verbindungen A-159 und A-161 wurde aus den entsprechenden Alkinen durch Lindlar Reduktion nach der folgenden Methode erhalten.

Triethylamin (11 mmol) und Lindlar Katalysator (7 Gew.-%, 1 mmol) wurden zu einer Lösung des entsprechenden Alkins (10 mmol) in DMF (25 mL) gegeben.

Das Reaktionsgefäß wurde evakuiert, mit Wasserstoff gespült und anschließend wurde die Reaktionsmischung 8 Stunden unter einer Wasserstoffatmosphäre gerührt. Die Reaktionsmischung wurde über Celite filtriert und mit Diethylacetat (25 mL) gewaschen. Die organische Phase wrude mit aq. NH₄Cl-Lösung (2 Gew.-%, 37 mL) und zweimal mit Wasser (2 x 25 mL) gewaschen, über MgSO₄ getrocknet, filtriert und das Lösungsmittel wurde im Vakuum entfernt. Der Rückstand wurde mittels Säulenchromatographie (Hexan/Ethylacetat 1:4) gereinigt.

### A-159: 2-But-2-enyloxy-6-trifluoromethyl-nicotinonitril

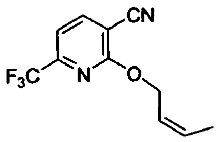

¹H NMR (300 MHz, CDCl₃) δ 8.05 (d, 1 H, *J* = 7.7 Hz), 7.34 (d, 1 H, *J* = 7.7 Hz), 5.81 (m, 1 H), 5.72 (m, 1 H), 5.09 (d, 2 H, *J* = 6.6 Hz), 1.81 (d, 3 H, *J* = 6.8 Hz) IR (pur) 2919, 2237, 1591, 1466, 1433, 1335, 1267, 1188, 1150, 1118, 969, 842, 747 cm⁻¹; MS (FAB) *m*/*z* 243 (M+H)

### A-160: 2-But-2-inyloxy-6-trifluoromethyl-nicotinonitril

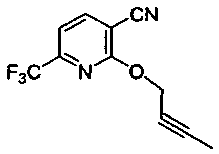

¹H NMR (300 MHz, CDCl₃) δ 8.08 (d, 1 H, *J* = 7.5 Hz), 7.39 (d, 1 H, *J* = 7.7 Hz), 5.10 (q, 2 H, *J* = 2.4 Hz), 1.87 (t, 3 H, *J* = 2.3 Hz); IR (pur) 2924, 2239, 1590, 1460, 1429, 1348, 1271, 1189, 1151, 1117, 977, 931, 844, 745 cm⁻¹; MS (FAB) *m*/*z* 241 (M+H)

### A-161: 2-Pent-2-enyloxy-6-trifluoromethyl-nicotinonitril

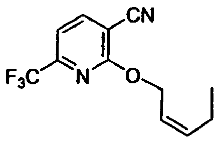

¹H NMR (300 MHz, CDCl₃) δ 8.05 (d, 1 H, *J* = 7.7 Hz), 7.34 (d, 1 H, *J* = 7.7 Hz), 5.73-5.65 (m, 2 H), 5.07 (d, 2 H, *J* = 6.0 Hz), 2.23 (m, 2 H), 1.03 (t, 3 H, *J* = 7 .6 Hz); IR (pur) 2967, 2237, 1590, 1465, 1431, 1405, 1342, 1267, 1187, 1151, 1118, 976, 842, 746 cm⁻¹; MS (FAB) *m*/*z* 257 (M+H)

### A-162: 2-Pent-2-inyloxy-6-trifluoromethyl-nicotinonitril

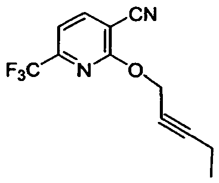

¹H NMR (300 MHz, CDCl) δ 8.08 (d, 1 H, *J* = 7.7 Hz), 7.39 (d, 1 H, *J* = 7.7 Hz), 5.11 (t, 2 H, *J* = 2.1 Hz), 2.23 (m, 2 H), 1.14 (t, 3 H, *J* = 7.5 Hz); IR (pur) 2982, 2238, 1590, 1461, 1428, 1348, 1272, 1189, 1151, 1117, 979, 844 cm⁻¹; MS (FAB) *m*/*z* 255 (M+H)

### A-163: 2-p-Tolyloxy-6-trifluoromethyl-nicotinonitril

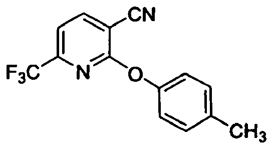

¹H NMR (300 MHz, CDCl₃) δ 8.17 (d, 1 H, *J* = 7.7 Hz), 7.44 (d, 1 H, *J* = 7.7 Hz), 7.22 (d, 2 H, *J* = 8.6 Hz), 7.10 (m, 2 H), 2.39 (s, 3 H); IR (pur) 2921, 2237, 1585, 1508, 1462, 1409, 1348, 1269, 1188, 1149, 1115, 947, 853 cm⁻¹; MS (FAB) *m*/*z* 279 (M+H)

### A-164: 2-Cyclopentyloxy-4-trifluoromethyl-benzonitril

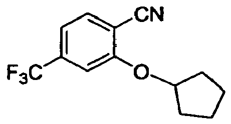

¹H NMR (300 MHz, CDCl₃) δ 7.67 (d, 1 H, *J* = 7.9 Hz), 7.23 (d, 1 H, *J* = 7.9 Hz), 7.17 (bs, 1 H), 4.92 (m , 1 H), 1.80-2.23 (m, 6 H), 1.61-1.77 (m, 2 H) IR (pur) 2959, 2232, 1506, 1435, 1328, 1245, 1163, 1122, 1079, 877, 825 cm⁻¹ MS (FAB) *m*/*z* 256 (M+H)

### A-165: 2-Cyclohexyloxy-4-trifluoromethyl-benzonitril

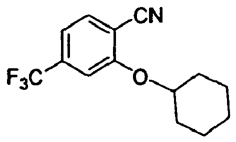

¹H NMR (300 MHz, CDCl₃) δ 7.68 (d, 1 H, *J* = 8.1 Hz), 7.23 (d, 1 H, *J* = 8.0 Hz), 7.17 (bs, 1 H), 4.49 (m, 1 H), 1.78-2.02 (m, 4 H), 1.63-1.77 (m , 2 H), 1.35-1.62 (m , 4 H); IR (pur) 2939, 2862, 2233, 1615, 1503, 1430, 1328, 1247, 1176, 1132, 1075, 1018, 970, 904, 829 cm⁻¹; MS (FAB) *m*/*z* 270 (M+H)

### A-166: 2-Butoxy-4-trifluoromethyl-benzonitril

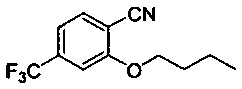

¹H NMR (300 MHz, CDCl₃) δ 7.68 (d, 1 H, *J* = 8.0 Hz), 7.14-7.30 (m, 2 H), 4.13 (t, 2 H, *J* = 6.4 Hz), 1.81-1.93 (m, 2 H), 1.49-1.62 (m, 2 H), 1.01(t, 3 H, *J* = 7.3 Hz); IR (pur) 2962, 2223, 1616, 1580, 1505, 1432, 1393, 1329, 1251, 1176, 1133, 1075, 975, 919, 865, 829 cm⁻¹; MS (FAB) *m*/*z* 244 (M+H)

### A-167: 2-Cyclopentyloxy-4-methyl-6-trifluoromethyl-nicotinonitril

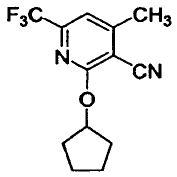

¹H NMR (300 MHz, CDCl₃) δ 7.16 (s, 1 H), 5.54 (m, 1 H), 2.58 (s, 3H), 2.02 (m, 2H), 1.85 (m, 4H), 1.64 (m, 2H); IR (pur) 2967, 2232, 1576, 1348, 1314, 1075, 913, 865 cm-¹; MS (FAB) *m*/*z* 271 (M+H)

### A-168: 2-Butoxy-4-tert-butyl-benzonitril

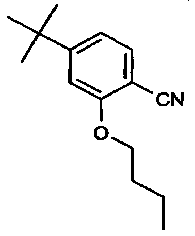

¹H NMR (300 MHz, CDCl₃) δ 7.40 (d, 1 H, *J* = 8.2 Hz), 7.0 (dd, 1 H, *J* = 1.6, 1.6 Hz), 6.90 (d, 1 H, *J* = 1.4 Hz), 4.0 (t, 2 H, *J* = 6.4 Hz), 1.88-1.74 (m, 2 H), 1.61-1.50 (m, 2 H), 1.3 (s, 9 H), 0.9 (t, 3 H, *J* = 1.8 Hz) IR (pur) 2963, 2224, 1604, 1412, 1237 cm⁻¹

### A-169: 4-tert-Butyl-2-isobutoxy-benzonitril

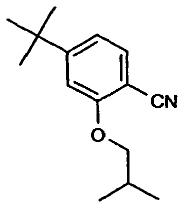

¹H NMR (300 MHz, CDCl₃) δ 7.47 (d, 1 H, *J* = 8.2 Hz), 7.0 (dd, 1 H, *J* = 1.6, 1.6 Hz), 6.92 (d, 1 H, *J* = 1.4 Hz), 3.83 (d, 2 H, *J* = 6.4 Hz), 2.24-2.10 (m, 1 H), 1.32 (s, 9 H), 1.08 (d, 6 H, *J* = 6.8 Hz) IR (pur) 2963, 2225, 1606, 1563, 1501, 1469 cm⁻¹

### A-170: 4-tert-Butyl-2-cyclohexyloxy-benzonitril

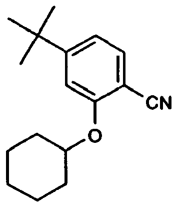

¹H NMR (300 MHz, CDCl₃) δ 7.46 (d, 1 H, *J* = 8.0 Hz), 7.0 (dd, 1 H, *J* = 1.6, 1.6 Hz), 6.95 (d, 1 H, *J* = 1.6 Hz), 4.43-4.39 (m, 1 H), 2.0-1.77 (m, 4 H), 1.77-1.60 (m, 4 H), 1.48- 1.37 (m, 2 H), 1.31 (s, 9 H) IR (pur) 2934, 2858, 2225, 1741, 1604, 1563 cm⁻¹

### A-171: 4-tert-Butyl-2-(2,2-dimethyl-propoxy)-benzonitril

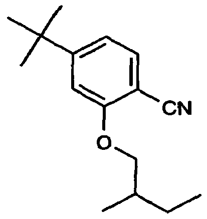

¹H NMR (300 MHz, CDCl₃) δ 7.46 (d, 1 H, *J* = 8.2 Hz), 7.0 (dd, 1 H, *J* = 1.6, 1.6 Hz), 6.91 (d, 1 H, *J* = 1.4 Hz), 3.70 (s, 2 H), 1.32 (s, 9 H), 1.09 (s, 9 H) IR (pur) 2963, 2225, 1605, 1564, 1500, 1468 cm⁻¹

### A-172: 4-tert-Butyl-2-cyclopentyloxy-benzonitril

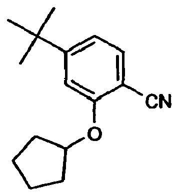

¹H NMR (300 MHz, CDCl₃) δ 7.45 (d, 1 H, *J*= 8.0 Hz), 6.99-6.92 (m, 2 H), 4.91-4.86 (m, 1 H), 1.96-1.83 (m, 6 H), 1.67-1.58 (m, 2 H), 1.31 (s, 9 H) IR (pur) 2963, 2872, 2224, 1604, 1563, 1498 cm⁻¹

### A-173: 4-tert-Butyl-2-pentoxy-benzonitril

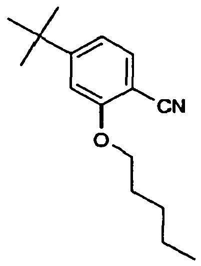

¹H NMR (300 MHz, CDCl₃) δ 7.46 (d, 1 H, *J* = 8.0 Hz), 7.0 (dd, 1 H, *J* = 1.6, 1.6 Hz), 6.93 (d, 1 H, *J* = 1.6 Hz), 4.07 (t, 2 H, J = 6.4 Hz), 1.90-1.81 (m, 2 H), 1.54 - 1.35 (m, 4 H), 1.31 (s, 9 H), 0.94 (t, 3 H, *J* = 6.9 Hz) IR (pur) 2960, 2870, 2225, 1605, 1564, 1500 cm⁻¹

### Stufe 2:

### Methode 1

Verbindungen der allgemeinen Formel VI-Ca oder VI-Cb (5 mmol), worin R⁵, R¹⁴, U, T und V die vorstehend genannte Bedeutung haben und m für 0, 1, 2 oder 3 steht, Palladium auf Kohle (10 %, 500 mg) und konzentrierte Salzsäure (3 mL) oder Essigsäure werden in MeOH (30 mL) gelöst und 6 Stunden bei RT einer Wasserstoffatmosphäre ausgesetzt. Die Reaktionsmischung wird über Celite filtriert und das Filtrat wird im Vakuum eingeengt. Der Rückstand wird mittels Flashchromatographie (SiO₂, EE) gereinigt.

Die folgenden Verbindungen B-85 bis B-88 wurden gemäß der vorstehend genannten allgemeinen Vorschrift erhalten:

### B-85: 2-Cyclopentylmethoxy-6-trifluoromethyl-pyridin-3-ylmethyl-ammonium acetat

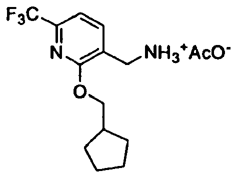

¹H NMR (CDCl₃) δ 7.68 (d, 1 H, J = 7.5 Hz), 7.23 (d, 1 H, J = 7.3 Hz), 4.95 (bs, NH3), 4.30 (d, 2 H), 2.39 (m, 1 H), 1.96 (s, 3 H, AcO-), 1.88-1.75 (m, 2 H), 1.68-1.54 (m, 4 H), 1.42-1.30 (m, 2 H); IR (pur) 2955, 2637, 2244, 1539, 1426, 1369, 1141, 997 cm⁻¹

### B-86: 2-Ethoxy-6-trifluoromethyl-pyridin-3-ylmethyl-ammonium acetat

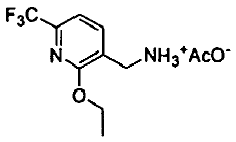

¹H NMR (CDCl₃) δ 7.66 (d, 1 H, J = 7.4 Hz), 7.23 (d, 1 H, J = 7.4 Hz), 5.66 (bs, NH₃), 4.48 (q, 2 H, J = 7.1 Hz), 3.91 (s, 2 H), 2.00 (s, 3 H, AcO), 1.42 (t, 3 H, J = 7.0 Hz); IR (pur) 2990, 1537, 1426, 1347, 1186, 1146, 1025 cm⁻¹

### B-87: 2-(4-Ethyl-cyclohexyloxy)-6-trifluoromethyl-pyridin-3-ylmethyl-ammonium acetat

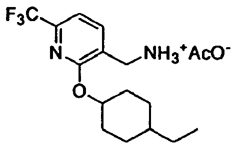

¹H NMR (CDCl₃) δ 7.62 (d, 1 H, J = 7.3 Hz), 7.19 (d, 1 H, J = 7.5 Hz), 5.45 (bs, NH3), 5.08 (m, 1 H), 3.86 (s, 2 H), 2.22-2.15 (m, 2 H), 2.03 (s, 3 H, AcO), 1.87-1.82 (m, 2 H), 1.50-1.03 (m, 7 H), 0.91 (t, 3 H, J = 6.8 Hz); IR (pur) 2926, 1572, 1421, 1355, 1275, 1186, 1141, 1010 cm⁻¹

### B-88: 2-(4-tert-Butyl-cyclohexyloxy)-6-trifluoromethyl-pyridin-3-ylmethyl-ammonium acetat

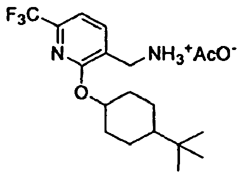

¹H NMR (CDCl₃) δ 7.62 (d, 1 H, J = 7.1 Hz), 7.19 (d, 1 H, J = 7.5 Hz), 5.04 (m, 1 H), 4.13 (bs, NH3), 3.85 (s, 2 H), 7.25-7.18 (m, 2 H), 2.05 (s, 3 H, AcO), 1.87-1.83 (m, 2 H), 1.46-1.02 (m, 5 H), 0.89 (s, 9 H); IR (pur) 2951, 1545, 1468, 1424, 1357, 1272, 1183 cm⁻¹

### Methode 2:

Verbindungen der allgemeinen Formel VI-Ca oder VI-Cb (2 mmol), worin R⁵, R¹⁴, U, T und V die vorstehend genannte Bedeutung haben und m für 0, 1, 2 oder 3 steht, werden in THF (10 mL, 10 mL) gelöst und BH₃•S(CH₃)₂ [2.0 M in THF, 3 mL, 3 Äquivalent] wird hinzu gegeben. Die Reaktionsmischung wird 8 Stunden zum Rückfluß erhitzt, aq. HCl (2 N) wird hinzu gegeben und die Reaktionsmischung wird erneut für 30 Minuten zum Rückfluß erhitzt. Die Reaktionsmischung wird mit aq. Natriumhydroxid-Lösung (2N) versetzt und mit EE gewaschen. Die vereinigten organischen Phasen werden mit ges. aq. NaCl-Lösung gewaschen und über Magnesiumsulfat getrocknet. Das Lösungsmittel wird im Vakuum entfernt und der Rückstand säulenchromatographisch (SiO₂, verschiedene Mischung aus Dichlormethan und Methanol als Laufmittel) gereinigt.

Die folgenden Verbindungen B-89 bis B-144 wurden gemäß der vorstehend genannten allgemeinen Vorschrift erhalten:

### B-89: C-[2-(3-Methyl-butoxy)-6-trifluoromethyl-pyridin-3-yl]-methylamin

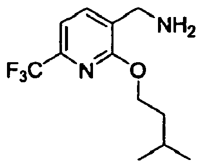

¹H NMR (300 MHz, CDCl₃) δ 7.63 (d, 1 H, *J* = 7.8 Hz), 7.21 (d, 1 H, J = 7.8 Hz), 4.43 (t, 1 H, J = 6.9 Hz), 3.84 (s, 2 H), 2.43 (bs, 2 H), 1.60-1.89(m, 3 H), 0.97 (d, 6 H, J = 6.6 Hz); MS (FAB) *m*/*z* 263 (M+H)

### B-90: C-[2-(3,3-Dimethyl-butoxy)-6-trifluoromethyl-pyridin-3-yl]-methylamin

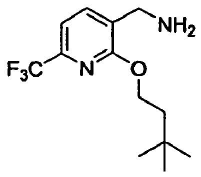

¹H NMR (300 MHz, CDCl₃) δ 7.56 (d, 1 H, *J* = 7.8 Hz), 7.13 (d, 1 H, J = 7.8 Hz), 4.38 (t, 1 H, J = 6.9 Hz), 3.74 (s, 2 H), 1.64(t, 2 H, J = 6.9 Hz), 0.92 (s, 9 H); MS (FAB) *m*/*z* 277 (M+H)

### B-91: C-[2-(2-Methyl-cyclopropylmethoxy)-6-trifluoromethyl-pyridin-3-yl]-methylamin

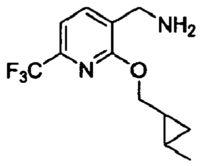

¹H NMR (300 MHz, CDCl₃) δ 7.63 (d, 1 H, J = 7.8 Hz), 7.21 (d, 1 H, J = 7.8 Hz), 4.24 (m, 2 H), 3.85 (s, 2 H), 1.08 (d, 3 H, J = 6.0 Hz), 0.98 (m, 1 H), 0.77 (m, 1 H), 0.52 (m, 1 H), 0.34 (m, 1 H); MS (FAB) *m*/*z* 261 (M+H)

### B-92: C-[2-Butoxy-6-(chloro-difluoro-methyl)-pyridin-3-yl]-methylamin

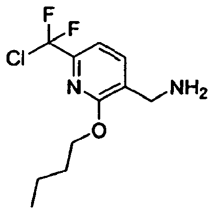

¹H NMR (300 MHz, CDCl₃) δ 7.71 (d, 1 H, J = 7.8 Hz), 6.98 (d, 1 H, J = 7.8 Hz), 3.99 (s, 2H), 3.59 (t, 2 H, J = 7.2 Hz), 1.63 (m, 2 H), 1.38 (m, 2 H), 0.95 (t, 3 H, J = 6.9 Hz); IR (pur) 2960, 1599, 1422, 1353, 1264, 1094 cm⁻¹; MS (FAB) *m*/*z* 265(M+H)

### B-93: C-(2-Phenoxy-6-trifluoromethyl-pyridin-3-yl)-methylamin

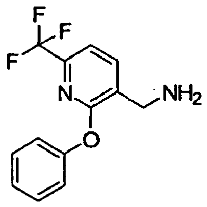

¹H NMR (300 MHz, CDCl₃) δ̇ 7.86 (dd, 1 H, J = 7.5,1.5 Hz), 7.35-7.43 (m,3 H, 7.15-7.23 (m, 3 H), 4.03 (s, 2 H); IR (pur) 2922, 1589, 1490, 1468, 1405, 1257, 1186, 1138, 941, 839, 752, 691 cm⁻¹; MS (FAB) *m*/*z* 269(M+H)

### B-94: C-(2-Butoxy-6-trifluoromethyl-pyridin-3-yl)-methylamin

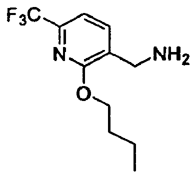

¹H NMR (CDCl₃) δ 7.65 (d, 1 H, J = 7.3 Hz), 7.21 (d, 1 H, J = 7.3 Hz), 4.41 (t, 2 H, J = 6.4 Hz), 3.84 (s, 2 H), 1.78 (m, 2 H), 1.50 (m, 2 H), 0.98 (t, 3 H, J = 7.3 Hz); IR (pur) 2963, 1607, 1470, 1425, 1357, 1193, 1132 cm⁻¹

### B-95: C-(2-Isopropoxy-6-trifluoromethyl-pyridin-3-yl)-methylamin

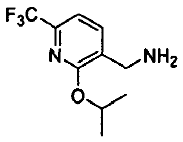

¹H NMR (CDCl₃) δ 7.63 (d, 1 H, J = 7.3 Hz), 7.19 (d, 1 H, J = 7.3 Hz), 5.42 (m, 1 H), 3.82 (s, 2 H), 1.37 (d, 6 H, J = 6.2 Hz); IR(pur) 3370, 2983, 1602, 1467, 1421, 1341, 1268, 1178, 1141, 969 cm⁻¹

### B-96: C-(2-Cyclopentyloxy-6-trifluoromethyl-pyridin-3-yl)-methylamin

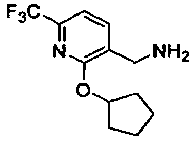

¹H NMR (CDCl₃) δ 7.62 (d, 1 H, J = 7.3 Hz), 7.19 (d, 1 H, J = 7.3 Hz), 5.53 (m, 1 H), 3.81 (s, 2 H), 2.05-1.95 (m, 2 H), 1.82-1.63 (m, 6 H)

### B-97: C-(2-Cyclohexyloxy-6-trifluoromethyl-pyridin-3-yl)-methylamin

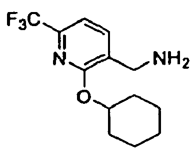

¹H NMR (CDCl₃) δ 7.63 (d, 1 H, J = 7.3 Hz), 7.18 (d, 1 H, J = 7.5 Hz), 5.20 (m, 1 H), 3.83 (s, 2 H), 1.99-1.95 (m, 2 H), 1.78-1.39 (m, 8 H); IR (pur) 2937, 2860, 1603, 1462, 1421, 1362, 1264, 1140, 972 cm⁻¹

### B-98: C-(2-Hexyloxy-6-trifluoromethyl-pyridin-3-yl)-methylamin

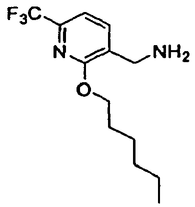

¹H NMR (CDCl₃) δ 7.65 (d, 1 H J = 7.3 Hz), 7.22 (d, 1 H, J = 7.3 Hz), 4.40 (t, 2 H, J = 6.6 Hz), 3.85 (s, 2 H), 1.84-1.74 (m, 2 H), 1.50-1.30 (m, 6 H), 0.90 (t, 3 H, J = 7.0 Hz); IR (pur) 2929, 1603, 1465, 1424, 1361, 1266, 1179, 1141 cm⁻¹

### B-99: C-(2-Isobutoxy-6-trifluoromethyl-pyridin-3-yl)-methylamin

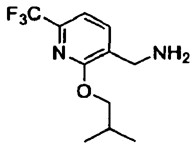

¹H NMR (CDCl₃) δ 7.66 (d, 1 H, J = 7.3 Hz), 7.22 (d, 1 H, J = 7.3 Hz), 4.18 (d, 2 H, J = 6.6 Hz), 3.86 (s, 2 H), 2.12 (m, 1 H), 1.04 (d, 6 H, J = 6.8 Hz) IR (pur) 2964, 1603, 1465, 1424, 1362, 1266, 1178, 1140, 1011 cm⁻¹

### B-100: C-(2-Cyclopropylmethoxy-6-trifluoromethyl-pyridin-3-yl)-methylamin

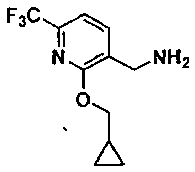

¹H NMR (CDCl₃) δ 7.65 (d, 1 H, J = 7.5 Hz), 7.21 (d, 1 H, J = 7.3 Hz), 4.25 (d, 2 H, J = 7.1 Hz), 3.87 (s, 2 H), 1.34-1.25 (m, 1 H), 0.63-0.57 (m, 2 H), 0.39-0.35 (m, 2 H); IR (pur) 2948, 1603, 1465, 1427, 1388, 1263, 1177, 1138, 990 cm⁻¹

### B-101: C-(2-Cyclobutylmethoxy-6-trifluoromethyl-pyridin-3-yl)-methylamin

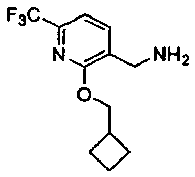

¹H NMR (CDCl₃) δ 7.64 (d, 1 H, J = 6.6 Hz), 7.22 (d, 1 H, J = 7.5 Hz), 4.37 (d, 2 H, J = 6.8 Hz), 3.85 (s, 2 H), 2.85-2.75 (m, 1 H), 2.17-1.85 (m, 6 H); IR (pur) 2933, 1602, 1464, 1422, 1365, 1265, 1178, 1140, 998 cm⁻¹

### B-102: 2-Butoxy-4-trifluoromethyl-benzylamin

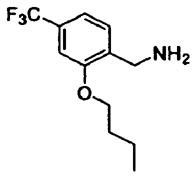

¹H NMR (CDCl₃) δ 7.33 (d, 1 H, J = 7.9 Hz), 7.18 (d, 1 H, J = 7.7 Hz), 7.05 (s, 1 H), 4.04 (t, 2 H, J = 6.4 Hz), 3.87 (s, 2 H), 1.83 (m, 2 H), 1.51 (m, 2 H), 1.00 (t, 3 H, J = 7.3 Hz); IR (pur) 3340, 2953, 1617, 1507, 1428, 1336, 1243, 1119 cm⁻¹

### B-103: C-(2-Propoxy-6-trifluoromethyl-pyridin-3-yl)-methylamin

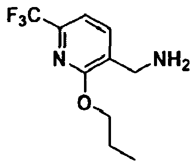

¹H NMR (CDCl₃) δ 7.65 (dd, 1 H, J = 7.3, 0.8 Hz), 7.22 (d, 1 H, J = 7.3 Hz), 4.37 (t, 2 H, J = 6.6 Hz), 3.85 (s, 2 H), 1.88-1.77 (m, 2 H), 1.04 (t, 3 H, J = 7.5 Hz); IR (pur) 2970, 1603, 1466, 1425, 1364, 1268, 1178, 1140 cm⁻¹

### B-104: C-(2-Pentyloxy-6-trifluoromethyl-pyridin-3-yl)-methylamin

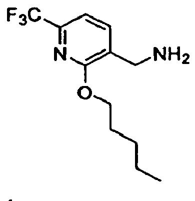

¹H NMR (CDCl₃) δ 7.65 (d, 1 H, J = 7.3 Hz), 7.22 (d, 1 H, J = 7.3 Hz), 4.40 (t, 2 H, J = 6.6 Hz), 3.85 (s, 2 H), 1.91-1.67 (m, 2 H), 1.46-1.35 (m, 4 H), 0.93 (t, 3 H, J = 7.3 Hz); IR (pur) 2957, 1465, 1424, 1361, 1267, 1179, 1140 cm⁻¹

### B-105: C-(2-Cyclobutoxy-6-trifluoromethyl-pyridin-3-yl)-methylamin

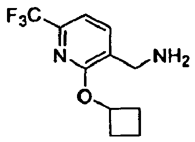

¹H NMR (CDCl₃) δ 7.64 (d, 1 H, J = 7.5 Hz), 7.20 (d, 1 H, J = 7.4 Hz), 5.28 (m, 1 H), 3.85 (s, 2 H), 2.53-2.47 (m, 2 H), 2.15-2.10 (m, 2 H), 1.88-1.69 (m, 2 H); IR (pur) 2990, 1602, 1466, 1420, 1346, 1265, 1178, 1139, 959 cm⁻¹

### B-106: C-[2-(4-Methyl-cyclohexyloxy)-6-trifluoromethyl-pyridin-3-yl]-methylamin

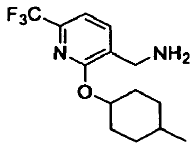

¹H NMR (CDCl₃) δ 7.62 (d, 1 H, J = 7.4 Hz), 7.18 (d, 1 H, J = 7.4 Hz), 5.07 (m, 1 H), 3.81 (s, 2 H), 2.18-2.15 (m, 2 H), 1.79-1.76 (m, 2 H), 1.51-1.39 (m, 3 H), 1.17-1.08 (m, 2 H), 0.93 (d, 3 H, J = 6.5 Hz); IR (pur) 2929, 1603, 1462, 1420, 1356, 1266, 1178,1140,1005 cm⁻¹

### B-107: C-(6-tert-Butyl-2-cyclohexyloxy-pyridin-3-yl)-methylamin

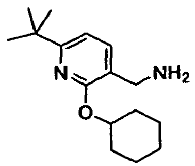

¹H NMR (CDCl₃) δ 7.37 (d, 1 H, J = 7.5 Hz), 6.77 (d, 1 H, J = 7.5 Hz), 5.15 (m, 1 H), 3.76 (bs, NH2), 3.48 (s, 2 H), 2.30-1.39 (m, 10 H), 1.30 (s, 9 H); IR (pur) 2935, 1582, 1452, 1406, 1363, 1254, 982 cm⁻¹

### B-108: C-(6-tert-Butyl-2-cyclopentyloxy-pyridin-3-yl)-methylamin

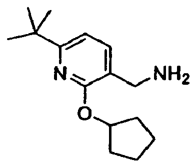

¹H NMR (CDCl₃) δ 7.36 (d, 1 H, J = 7.4 Hz), 6.78 (d, 1 H, J = 7.3 Hz), 5.50 (m, 1 H), 3.73 (s, 2 H), 2.11 (bs, NH2), 2.03-1.63 (m, 8 H), 1.31 (s, 9 H); IR (pur) 2960, 1583, 1454, 1406, 1350, 1255, 988 cm⁻¹

### B-109: C-(2-Butoxy-6-tert-butyl-pyridin-3-yl)-methylamin

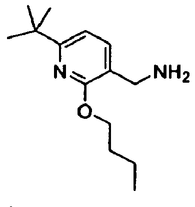

¹H NMR (CDCl₃) δ 7.38 (d, 1 H, J = 7.5 Hz), 6.80 (d, 1 H, J = 7.5 Hz), 4.39 (t, 2 H, J = 6.6 Hz), 3.77 (s, 2 H), 2.17 (bs, NH2), 1.77 (m, 2 H), 1.49 (m, 2 H), 1.31 (s, 9 H), 0.98 (t, 3 H, J = 7.4 Hz); IR (pur) 2958, 1583, 1458, 1411, 1364, 1254 cm⁻¹

### B-110: C-(6-tert-Butyl-2-hexyloxy-pyridin-3-yl)-methylamin

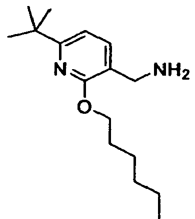

¹H NMR (CDCl₃) δ 7.37 (d, 1 H, J = 7.3 Hz), 6.79 (d, 1 H, J = 7.5 Hz), 4.37 (t, 2 H, J = 6.6 Hz), 3.74 (s, 2 H), 1.78 (m, 2 H), 1.48-1.30 (m, 6 H), 1.31 (s, 9 H), 0.90 (m, 3 H); IR (pur) 2956, 1582, 1458, 1411, 1361, 1253, 1016 cm⁻¹

### B-111: C-(2-Benzyloxy-6-tert-butyl-pyridin-3-yl)-methylamin

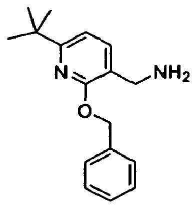

¹H NMR (CDCl₃) δ 7.47-7.29 (m, 6 H), 6.83 (d, 1 H, J = 7.5 Hz), 5.47 (s, 2 H), 3.79 (s, 2 H), 1.31 (s, 9 H); IR (pur) 2957, 1582, 1454, 1405, 1357, 1253, 1009 cm⁻¹

### B-112: C-(2-Cyclohexylmethoxy-6-trifluoromethyl-pyridin-3-yl)-methylamin

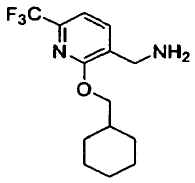

¹H NMR (CDCl₃) δ 7.64 (d, 1 H, J = 7.3 Hz), 7.21 (d, 1 H, J = 7.3 Hz), 4.20 (d, 2 H), 3.85 (s, 2 H), 1.86-1.67 (m, 5 H), 1.32-1.00 (m, 6 H)

### B-113: C-[2-(4-Methyl-cyclohexylmethoxy)-6-trifluoromethyl-pyridin-3-yl]-methylamin

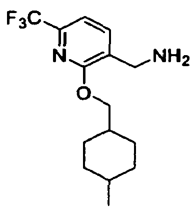

¹H NMR (CDCl₃) δ 7.78 & 7.64 (d, 1 H), 7.21 (d, 1 H, J = 7.3 Hz), 4.40 & 3.85 (s, 2 H), 4.31 & 4.20 (m, 2 H), 2.00-1.50 (m, 7 H), 1.40-1.00 (m, 3 H), 0.95-0.87 (m, 3 H); IR (pur) 2923, 1602, 1462, 1423, 1359, 1264, 1177, 1140, 1110 cm⁻¹

### B-114: C-[2-(2,2-Dimethyl-cyclopropylmethoxy)-6-trifluoromethyl-pyridin-3-yl]-methylamin

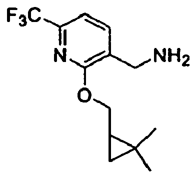

¹H NMR (CDCl₃) δ 7.64 (d, 1 H, J = 7.3 Hz), 7.21 (d, 1 H, J = 7.3 Hz), 4.63 (dd, 1 H, J = 11.5,6.6 Hz), 4.20 (dd, 1 H, J = 11.6, 8.9 Hz), 3.86 (s, 2 H), 1.14 (s, 3 H), 1.10 (s, 3 H), 0.88 (m, 1 H), 0.58 (dd, 1 H, J = 8.6, 4.4 Hz), 0.30 (dd, 1 H, J = 4.8, 4.8 Hz); IR (pur) 2951, 1603, 1464, 1426, 1396, 1344, 1264, 1178, 1141, 987 cm⁻¹

### B-115: 4-(3-Aminomethyl-6-trifluoromethyl-pyridin-2-yloxymethyl)-piperidin-1-carbonsäure tert-butyl ester

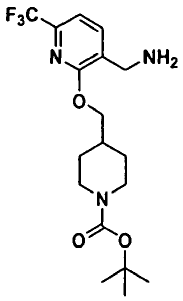

¹H NMR (CDCl₃) δ 7.69 (d, 1 H, J = 7.5 Hz), 7.24 (d, 1 H, J = 7.5 Hz), 4.27 (d, 2 H), 4.20-4.07 (m, 2 H), 3.86 (s, 2 H), 2.80-2.65 (m, 2 H), 1.83-1.50 (m, 3 H), 1.47 (s, 9 H), 1.35-1.20 (m, 2 H); IR (pur) 3392, 2926, 1688, 1424, 1361, 1268, 1174, 1142, 1017 cm⁻¹

### B-116: C-[6-Trifluoromethyl-2-(4-trifluoromethyl-cyclohexyloxy)-pyridin-3-yl]-methylamin

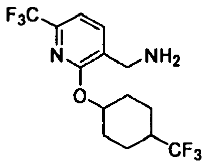

IR (pur) 2923, 1603, 1465, 1424, 1363, 1269, 1180, 1139 cm⁻¹

### B-117: 4-(3-Aminomethyl-6-trifluoromethyl-pyridin-2-yloxy)-piperidin-1-carbonsäure tert-butyl ester

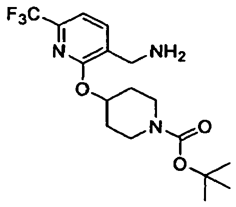

¹H NMR (CDCl₃) δ 7.69 (d, 1 H, J = 6.8 Hz), 7.24 (d, 1 H, J = 7.3 Hz), 5.36 (m, 1 H), 3.85 (s, 2 H), 3.68 (m, 2 H), 3.40 (m, 2 H), 2.05-1.60 (m, 4 H), 1.48 (s, 9 H) IR (pur) 3393, 2928, 1688, 1421, 1363, 1272, 1237, 1173, 1139, 1028 cm⁻¹

### B-118: C-[2-(Pyridin-4-ylmethoxy)-6-trifluoromethyl-pyridin-3-yl]-methylamin

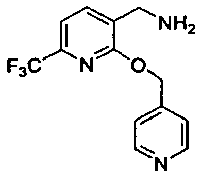

¹H NMR (300 MHz, CDCl₃) δ 8.62 (d, 2 H, *J* = 6.0 Hz), 7.78 (d, 1 H, *J* = 7.2 Hz), 7.38 (d, 2 H, *J* = 6.2 Hz), 7.31 (d, 1 H, *J* = 7.6 Hz), 5.49 (s, 2 H), 3.96 (s, 2 H) IR (pur) 3367, 1602, 1468, 1417, 1359, 1267, 1179, 1137, 1179, 1137, 1024, 936, 840, 801 cm⁻¹; MS (FAB) *m*/*z* 284 (M+H)

### B-119: C-(2-Phenethyloxy-6-trifluoromethyl-pyridin-3-yl)-methylamin

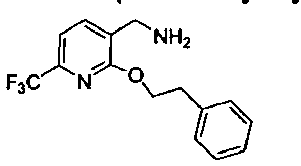

¹H NMR (300 MHz, CDCl₃) δ 7.63 (d, 1 H, *J* = 7.2 Hz), 7.38-7.21 (m, 6 H), 4.63 (t, 2 H, *J* = 6.6 Hz), 3.78 (s, 2 H), 3.11 (t, 2 H, *J* = 6.6 Hz), 1.61 (bs, 2 H) IR (pur) 3029, 2956, 1599, 1463, 1423, 1354, 1270, 1180, 1139, 1004, 951, 839, 747, 701 cm⁻¹; MS (FAB) *m*/*z* 297 (M+H)

### B-120: C-[2-(Pyridin-2-ylmethoxy)-6-trifluoromethyl-pyridin-3-yl]-methylamin

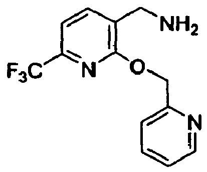

¹H NMR (300 MHz, CDCl₃) δ 8.61 (d, 1 H, *J* = 4.9 Hz), 7.75 (d, 1 H, *J* = 7.7 Hz), 7.71 (td, 1 H, *J* = 7.7, 1.7 Hz), 7.48 (d, 1 H, *J* = 7.9 Hz), 7.26 (m, 2 H), 5.61 (s, 2 H), 3.98 (s, 2 H), 2.13 (bs, 2 H); IR (pur) 3395, 2920, 1598, 1417, 1355, 1274, 1181, 1137, 1002, 936, 840, 756 cm⁻¹; MS (FAB) *m*/*z* 284 (M+H)

### B-121: C-(2-Benzyloxy-6-trifluoromethyl-pyridin-3-yl)-methylamin

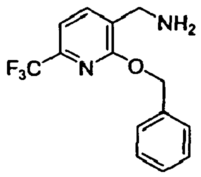

¹H NMR (300 MHz, CDCl₃) δ 7.70 (d, 1 H, *J* = 7.3 Hz), 7.48 (m, 2 H), 7.42-7.35 (m, 4 H), 5.47 (s, 2 H), 3.70 (s, 2 H), 1.76 (bs, 2 H); IR (pur) 2925, 1652, 1600, 1539, 1459, 1419, 1355, 1267, 1179, 1138, 992, 838, 741, 698cm⁻¹; MS (FAB) *m*/*z* 283 (M+H)

### B-122: 2-Benzyloxy-4-trifluoromethyl-benzylamin

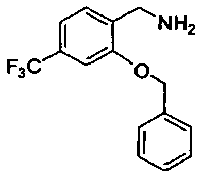

¹H NMR (300 MHz, CDCl₃) δ 7.51-7.35 (m, 6 H), 7.22 (d, 1 H, *J* = 7.7 Hz), 7.16 (s, 1 H), 5.13 (s, 2 H), 3.92 (s, 2 H), 1.60 (bs, 2 H); IR (pur) 2920, 1509, 1426, 1328, 1239, 1166, 1122, 1019, 917, 858, 740, 697 cm⁻¹; MS (FAB) *m*/*z* 282 (M+H)

### B-123: C-[2-(Pyridin-3-ylmethoxy)-6-trifluoromethyl-pyridin-3-yl]-methylamin

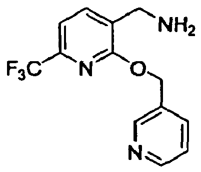

¹H NMR (300 MHz, CDCl₃) δ 8.75 (s, 1 H), 8.58 (d, 1 H, *J* = 4.4 Hz), 7.84 (d, 1 H, *J* = 7.7 Hz), 7.74 (d, 1 H, *J* = 7.5 Hz), 7.31 (d, 1 H, *J* = 4.9 Hz), 7.29 (d, 1 H, *J* = 7.3 Hz), 5.50 (s, 2 H), 3.89 (s, 2 H), 1.68 (bs, 2 H); IR (pur) 2920, 1599, 1538, 1462, 1416, 1356, 1267, 1179, 1137, 997, 840 cm⁻¹; MS (FAB) *m*/*z* 284 (M+H)

### B-124: C-[6-Trifluoromethyl-2-(4-trifluoromethyl-benzyloxy)-pyridin-3-yl]-methylamin

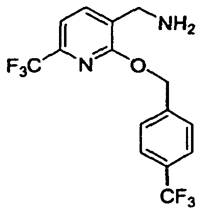

¹H NMR (300 MHz, CDCl₃) □7.74 (d, 1 H, *J* = 7.3 Hz), 7.69-7.54 (m, 4 H), 7.29 (d, 1 H, *J* = 7.5 Hz), 5.53 (s, 2 H), 3.91 (s, 2 H), 1.50 (bs, 2 H); IR (pur) 2919, 1600, 1467, 1419, 1356, 1326, 1267, 1131, 1067, 1014, 936, 826 cm⁻¹; MS (FAB) *m*/*z* 351 (M+H)

### B-125: C-[2-(4-Butyl-benzyloxy)-6-trifluoromethyl-pyridin-3-yl]-methylamin

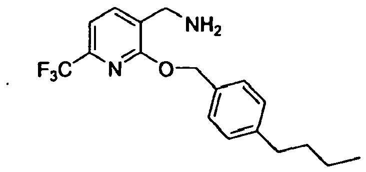

¹H NMR (300 MHz, CDCl₃) δ 7.68 (d, 1 H, *J* = 7.5 Hz), 7.39 (d, 2 H, *J* = 7.9 Hz), 7.25 (d, 1 H, *J* = 7.4 Hz), 7.19 (d, 2 H, *J* = 8.0 Hz), 5.43 (s, 2 H), 3.87 (s, 2 H), 2.61 (t, 2 H, *J* = 7.9 Hz), 1.60 (m, 2 H), 1.36 (m, 2 H), 0.93 (t , 3 H, *J* = 7.3 Hz) IR (pur) 2929, 1599, 1463, 1420, 1353, 1267, 1180, 1141, 990, 836 cm⁻¹; MS (FAB) *m*/*z* 339 (M+H)

### B-126: C-[2-(4-tert-Butyl-benzyloxy)-6-trifluoromethyl-pyridin-3-yl]-methylamin

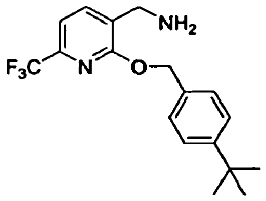

¹H NMR (300 MHz, CDCl₃) δ 7.68 (d, 1 H, *J* = 7.3 Hz), 7.49-7.34 (m, 4 H), 7.25 (d, 1 H, *J* = 7.5 Hz), 5.44 (s, 2 H), 3.87 (s, 2 H), 1.52 (bs , 2 H), 1.33 (s , 9 H) IR (pur) 2963, 1599, 1516, 1464, 1421, 1354, 1267, 1179, 1140, 990, 837 cm⁻¹; MS (FAB) *m*/*z* 339 (M+H)

### B-127: C-[2-(Indan-2-yloxy)-6-trifluoromethyl-pyridin-3-yl]-methylamin

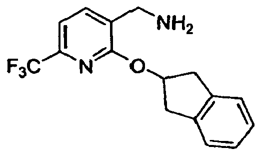

¹H NMR (300 MHz, CDCl₃) δ 7.66 (d, 1 H, *J* = 8.1 Hz), 7.25 (d, 1 H, *J* = 8.0 Hz), 7.24-7.15 (m, 4 H), 5.91 (m, 1 H), 3.76 (s, 2 H), 3.48 (dd, 2 H, *J* = 17.0, 6.6 Hz), 3.14 (dd, 2 H, *J* = 16.9, 3.7 Hz), 1.43 (bs, 2 H); IR (pur) 2953, 1676, 1596, 1464, 1418, 1348, 1266, 1186, 1139, 1012, 970, 935, 843, 743 cm⁻¹; MS (FAB) *m*/*z* 309 (M+H)

### B-128: C-[2-(4-Chloro-benzyloxy)-6-trifluoromethyl-pyridin-3-yl]-methylamin

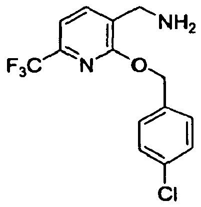

¹H NMR (300 MHz, CDCl₃) δ 7.71 (d, 1 H; *J* = 7.3 Hz), 7.44-7.32 (m, 4 H), 7.27 (d, 1 H, *J* = 7.3 Hz), 5.43 (s, 2 H), 3.88 (s, 2 H), 1.50 (bs, 2 H); IR (pur) 2919, 1600, 1493, 1465, 1423, 355, 1264, 1179, 1138, 1110, 997, 935, 839 cm⁻¹; MS (FAB) *m*/*z* 317 (M+H)

### B-129: C-[6-(Chloro-difluoro-methyl)-2-cyclopentyloxy-pyridin-3-yl]-methylamin

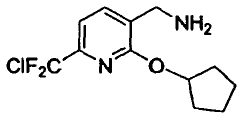

¹H NMR (300 MHz, CDCl₃) δ 7.60 (d, 1 H, *J* = 7.3 Hz), 7.16 (d, 1 H, *J* = 7.5 Hz), 5.52 (m, 1 H), 3.81 (m, 2 H), 1.95-2.10 (m, 2 H), 1.60-1.90 (m, 6 H); IR (pur) 3367, 2961, 1599, 1456, 1418, 1349, 1265, 1096, 991, 888, 827 cm⁻¹; MS (FAB) *m*/*z* 277 (M+H)

### B-130: C-[6-(Chloro-difluoro-methyl)-2-cyclohexyloxy-pyridin-3-yl]-methylamin

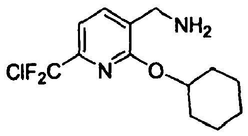

¹H NMR (400 MHz, CDCl₃) □7.61 (d, 1 H, *J* = 7.6 Hz), 7.15 (d, 1 H, *J* = 7.6 Hz), 5.19 (m, 1 H), 3.83 (s, 2 H), 1.93-2.04 (m, 2 H), 1.70-1.82 (m, 2 H), 1.52-1.66 (m, 6 H); IR (pur) 2936, 2858, 1600, 1455, 1419, 1364, 1263, 1096, 989, 881 cm⁻¹; MS (FAB) *m*/*z* 291 (M+H)

### B-131: C-[6-(Chloro-difluoro-methyl)-2-(pyridin-3-ylmethoxy)-pyridin-3-yl]-methylamin

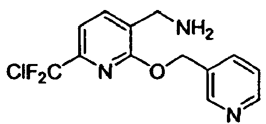

¹H NMR (300 MHz, CDCl₃) δ 8.76 (s, 1 H), 8.57 (m, 1 H), 7.85 (m, 1 H), 7.72 (d, 1 H, *J* = 7.1 Hz), 7.31 (m , 1 H), 7.25 (d, 1 H, *J* = 7.5 Hz), 5.51 (bs, 2 H), 3.88 (s, 2H); IR (pur) 2922, 1598, 1456, 1414, 1357, 1096, 1005, 884, 829, 712 cm⁻¹; MS (FAB) *m*/*z* 300 (M+H)

### B-132: C-[6-(Chloro-difl uoro-methyl)-2-(pyridin-2-yl methoxy)-pyridin-3-yl]-methylamin

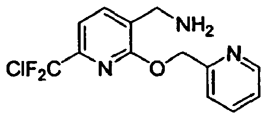

¹H NMR (300 MHz, CDCl₃) δ 8.59 (m, 1 H), 7.68-7.84 (m , 2 H), 7.47 (m , 1 H), 7.21-7.26 (m , 2 H), 5.66 (s, 2 H), 4.09 (s, 2 H); IR (pur) 2921, 1597, 1416, 1350, 1272, 1097, 1011, 969, 832, 763 cm⁻¹; MS (FAB) *m*/*z* 300 (M+H)

### B-133: C-[6-(Chloro-difluoro-methyl)-2-isobutoxy-pyridin-3-yl]-methylamin

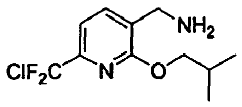

¹H NMR (300 MHz, CDCl₃) δ 7.63 (d, 1 H, *J* = 7.4 Hz), 7.19 (d , 1 H, *J* = 7.5 Hz), 4.15-4.23 (m , 2 H), 3.86 (bs , 2 H), 2.12 (m, 1 H), 1.04 (d , 6 H, *J* = 6.7 Hz); IR (pur) 2963, 1599, 1460, 1422, 1361, 1264, 1184, 1095, 1012, 970, 880, 826 cm⁻¹; MS (FAB) *m*/*z* 265 (M+H)

### B-134: 2-Cyclopentyloxy-4-trifluoromethyl-benzylamin

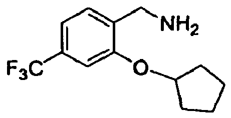

¹H NMR (300 MHz, CDCl₃) δ 7.32 (d, 1 H, *J* = 7.5 Hz), 7.15 (d, 1 H, *J* = 7.7 Hz), 7.04 (bs, 1 H), 4.86 (m, 1 H), 3.82 (s, 2 H), 1.60-2.02 (m, 8 H); IR (pur) 2962, 1590, 1507, 1427, 1331, 1238, 1167, 1122, 989, 916, 862 cm⁻¹; MS (FAB) *m*/*z* 260 (M+H)

### B-135: 2-Cyclohexyloxy-4-trifluoromethyl-benzylamin

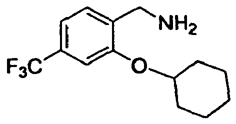

¹H NMR (300 MHz, CDCl₃) δ 7.34 (d, 1 H, *J* = 7.9 Hz), 7.15 (d, 1 H, *J* = 7.7 Hz), 7.05 (bs, 1 H), 4.39 (m, 1 H), 3.86 (s, 2 H), 1.90-2.00 (m, 4 H), 1.70-1.89 (m, 2 H), 1.51-1.69 (m, 2 H), 1.32-1.51 (m, 2 H); IR (pur) 2938, 2860, 1589, 1507, 1426, 1329, 1234, 1164, 1122, 1078, 1044, 973, 906, 862 cm⁻¹; MS (FAB) *m*/*z* 274 (M+H)

### B-136: 2-Butoxy-4-trifluoromethyl-benzylamin

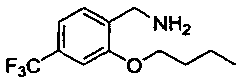

¹H NMR (300 MHz, CDCl₃) δ 7.34 (d, 1 H, *J* = 7.7 Hz), 7.18 (d, 1 H, *J* = 7.7 Hz), 7.05 (bs, 1 H), 4.04 (t, 2 H, *J* = 6.2 Hz), 3.87 (s, 2 H), 1.72-1.85 (m, 2 H), 1.41-1.60 (m , 2 H), 1.00 (t , 3 H, *J* = 7.3 Hz); IR (pur) 3304, 2957, 1507, 1427, 1382, 1330, 1239, 1159, 1114, 919, 861, 822 cm⁻¹; MS (FAB) *m*/*z* 248 (M+H)

### B-137: C-(2-Cyclopentyloxy-4-methyl-6-trifluoromethyl-pyridin-3-yl)-methylamin

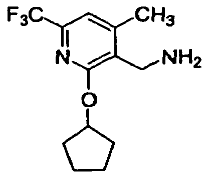

¹H NMR (300 MHz, CDCl₃) δ 7.04 (s, 1 H), 5.51 (m, 1H), 3.82 (s, 2H), 2.38 (s, 3H), 2.01 (m, 2H), 1.75 (m, 6H); IR (pur) 2964, 1574, 1288, 1061, 993, 917, 865, 723 cm⁻¹; MS (FAB) *m*/*z* 275(M+H)

### B-138: C-[2-(1-Butyl-pentyloxy)-6-trifluoromethyl-pyridin-3-yl]-methylamin

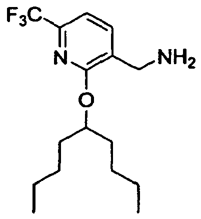

¹H NMR (300 MHz, CDCl₃) δ 7.62 (d, 1 H, J = 7.3 Hz), 7.18 (d, 1 H, J = 7.5 Hz), 5.32 (m, 1 H), 3.82 (s, 2H), 1.67~1.75 (m, 2H), 1.33 (m, 9H), 0.90 (m, 7H); IR (pur) 2932, 2865, 1601, 1464, 975, 835, 744, 701 cm⁻¹; MS (FAB) *m*/*z* 319(M+H)

### B-139: C-(2-p-Tolyloxy-6-trifluoromethyl-pyridin-3-yl)-methylamin

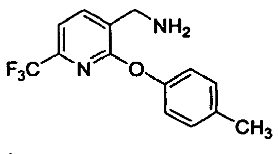

¹H NMR (300 MHz, CDCl₃) δ 7.84 (d, 1 H, *J* = 7.3 Hz), 7.35 (d, 1 H, *J* = 7.5 Hz), 7.19 (d, 2 H, *J* = 8.8 Hz), 7.06 (m, 2 H), 4.02 (s, 2 H), 2.37 (s, 3 H); IR (pur) 2923, 1596, 1511, 1463, 1403, 1262, 1142, 943, 816 cm⁻¹; MS (FAB) *m*/*z* 283 (M+H)

### B-140: C-(4-Methyl-2-pentyloxy-6-trifluoromethyl-pyridin-3-yl)-methylamin

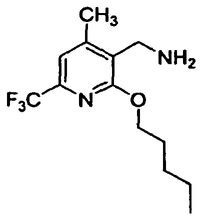

¹H NMR (300 MHz, CDCl₃) δ 7.06 (s,1 H), 4.37 (t, 2 H, *J* = 6.6 Hz), 3.85 (s, 2 H), 2.40 (s, 3 H), 1.80 (m, 2 H), 1.45 (m, 2 H), 1.39-1.31 (m, 4 H), 0.90 (m, 3H) IR (pur) 2931, 1610, 1575, 1463, 1409, 1348, 1290, 1246, 1177, 1138, 1073, 917, 866, 721 cm⁻¹; MS (FAB) *m*/*z* 277 (M+H)

### B-141: C-(2-Methoxy-6-trifluoromethyl-pyridin-3-yl)-methylamin

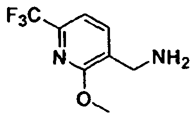

¹H NMR (CDCl₃) δ 7.67 (d, 1 H, J = 7.3 Hz), 7.25 (d, 1 H), 4.03 (s, 3 H), 3.85 (s, 2 H); IR (pur) 3400, 2923, 1738, 1468, 1370, 1268, 1137 cm⁻¹

### B-142: C-[2-(4-Ethyl-benzyloxy)-6-trifluoromethyl-pyridin-3-yl]-methylamin

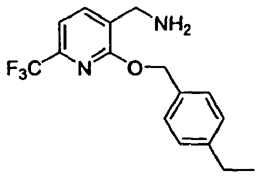

¹H NMR (300 MHz, CDCl₃) δ 7.68 (d, 1 H, *J* = 7.3 Hz), 7.41 (d, 2 H, *J* = 7.9 Hz), 7.25 (d, 1 H, *J* = 7.2 Hz), 7.21 (d , 2 H, *J* = 7.9 Hz), 5.44 (s, 2 H), 3.87 (s, 2 H), 2.66 (q, 2 H, *J* = 7.5 Hz), 1.63 (bs, 2 H), 1.24 (t , 3 H, *J* = 7.6 Hz); IR (pur) 2965, 1600, 1463, 1419, 1354, 1265, 1178, 1138, 1111, 990, 825cm⁻¹; MS (FAB) *m*/*z* 311 (M+H)

### B-143: C-[2-Benryloxy-6-(chloro-difluoro-methyl)-pyridin-3-yl]-methylamin

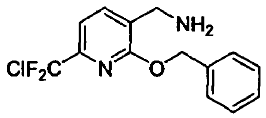

¹H NMR (400 MHz, CDCl₃) δ 7.67 (d, 1 H, *J* = 7.6 Hz), 7.45-7.52 (m, 2 H), 7.28-7.40 (m, 3 H), 7.22 (d, 1 H, *J* = 7.6 Hz), 5.48 (s, 2 H), 3.88 (s, 2 H); IR (pur) 2923, 1599, 1456, 1416, 1356, 1256, 1096, 1000, 883, 872, 698 cm⁻¹; MS (FAB) *m*/*z* 299 (M+H)

### B-144: C-[6-(Chloro-difluoro-methyl)-2-hexyloxy-pyridin-3-yl]-methylamin

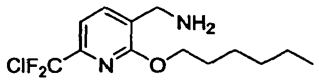

¹H NMR (300 MHz, CDCl₃) δ 7.62 (d, 1 H, *J* = 7.3 Hz), 7.18 (d, 1 H, *J* = 7.5 Hz), 4.34-4.44 (m, 2 H), 3.80-3.85 (m, 2 H), 1.70-1.84 (m, 2 H), 1.21-1.52 (m, 6 H), 0.85-0.95 (m, 3 H); IR (pur) 2930, 1600, 1460, 1423, 1364, 1264, 1096, 1002, 879, 827 cm⁻¹; MS (FAB) *m*/*z* 293 (M+H)

### Methode 3:

Verbindungen der allgemeinen Formel VI-Ca oder VI-CB (1.5 mmol), worin R⁵, R¹⁴, U, T und V die vorstehend genannte Bedeutung haben und m für 0, 1, 2 oder 3 steht, werden in Diethylether (3 mL) gelöst und bei 0 °C wird eine Suspension von Lithiumaluminiumhydrid (3 mmol) in Ether (5 mL) langsam zu getropft. Die Reaktionsmischung wird 4 Stunden zum Rückfluß erhitzt und bei 0 °C wird langsam Methanol und anschließend 1 N aq. NaOH-Lösung zu getropft. Die Reaktionsmischung wird mit Methanol verdünnt und über Celite filtriert. Das Lösungsmittel wird im Vakuum entfernt und der Rückstand säulenchromatographisch (SiO₂, verschiedene Mischung aus Dichlormethan und Methanol als Laufmittel) gereinigt.

Die folgenden Verbindungen B-145 bis B-150 wurden gemäß der vorstehend genannten allgemeinen Vorschrift erhalten:

### B-145: 2-Butoxy-4-tert-butyl-benzylamin

¹H NMR (300 MHz, CDCl₃) δ 7.17 (d, 1 H, *J* = 7.8 Hz), 6.92 (dd, 1 H, *J* = 1.6, 1.6 Hz), 6.88 (d, 1 H, *J* = 1.6 Hz), 4.02 (t, 2 H, *J* = 6.4 Hz), 3.85 (bs, 2 H), 1.85-1.76 (m, 2 H), 1.57-1.44 (m, 2 H), 1.3 (s, 9 H), 0.98 (t, 3 H, *J* = 7.3 Hz) IR (pur) 2959, 2869, 1612, 1576, 1507, 1468 cm⁻¹

### B-146: 4-tert-Butyl-2-Isobutoxy-benzylamin

¹H NMR (300 MHz, CDCl₃) δ 7.18 (d, 1 H, *J* = 7.7 Hz), 6.92 (dd, 1 H, *J* = 1.6, 1.6 Hz), 6.86 (d, 1 H, *J* = 1.6 Hz), 4.15 (bs, 2 H), 3.80 (s, 2 H), 3.78 (d, 2 H, *J* = 6.4 Hz), 2.18-2.10 (m, 1 H), 1.30 (s, 9 H), 1.05 (d, 6 H, *J* = 6.6 Hz) IR (pur) 2958, 1614, 1513, 1409, 1269, 1232 cm⁻¹

### B-147: 4-tert-Butyl-2-cyclohexyloxy-benzylamin

¹H NMR (300 MHz, CDCl₃) δ 7.18 (d, 1 H, *J*= 7.5 Hz), 6.91-6.89 (m, 2 H), 5.29 (bs, 2 H), 4.43-4.30 (m, 1 H), 3.88 (s, 2 H), 2.03-1.25 (m, 10 H), 1.29 (s, 9 H) IR (pur) 2932, 2875, 1611, 1504, 1455, 1412 cm⁻¹

### B-148: 4-tert-Butyl-2-(2,2-dimethyl-propoxy)-benzylamin

¹H NMR (300 MHz, CDCl₃) δ 7.22 (d, 1 H, *J* = 7.8 Hz), 6.94 (dd, 1 H, *J* = 1.6, 1.6 Hz), 6.86 (s, 1 H), 5.41 (bs, 2 H), 3.95 (s, 2 H), 3.60 (s, 2 H), 1.30 (s, 9 H), 1.06 (s, 9 H)

IR (pur) 2958, 2867, 1613, 1577, 1475, 1410 cm⁻¹

### B-149: 4-tert-Butyl-2-cyclopentyloxy-benzylamin

¹H NMR (300 MHz, CDCl₃) δ 7.12 (d, 1 H, *J* = 8.2 Hz), 6.90-6.88 (m, 2 H), 4.86-4.80 (m, 1 H), 3.75 (s, 2 H), 2.94 (bs, 2 H), 1.95-1.61 (m, 8 H), 1.30 (s, 9 H) IR (pur) 2959, 1611, 1576, 1503, 1412, 1269 cm⁻¹

### B-150: 4-tert-Butyl-2-pentyloxy-benzylamin

¹H NMR (300 MHz, CDCl₃) δ 7.18 (d, 1 H, *J* = 7.8 Hz), 6.94-6.87 (m, 2 H), 4.73 (bs, 2 H), 4.01 (t, 2 H, *J* = 6.4 Hz), 3.88 (s, 2 H), 1.86-1.78 (m, 2 H), 1.51-1.35 (m, 4 H), 1.30 (s, 9 H), 0.93 (t, 3 H, *J* = 6.9 Hz) IR (pur) 2958, 2866, 1614, 1511, 1463, 1415 cm⁻¹

### 4. Allgemeine Vorschrift zur Darstellung von Aminen der allgemeinen Formel V-C

Die Darstellung von Aminen der allgemeinen Formel V-C erfolgt wie im nachfolgenden Schema 3 dargestellt.

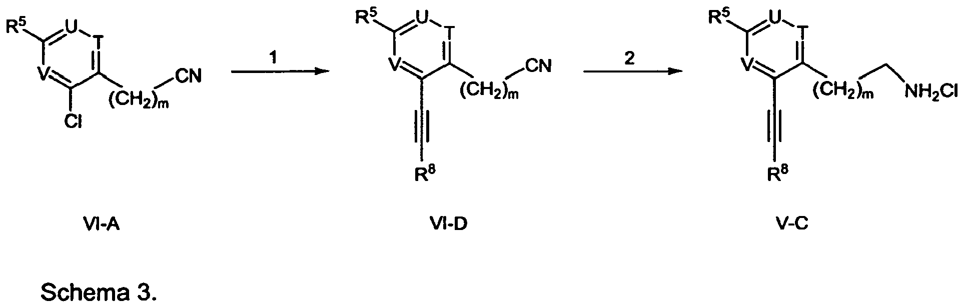

### Stufe 1: Darstellung von Nitrilen der allgemeinen Formel VI-D

Verbindungen der allgemeinen Formel VI-A (1 Äquivalent), worin R⁵, U, T und V die vorstehend genannte Bedeutung haben und m für 0, 1, 2 oder 3 steht, werden mit Bis(triphenylphosphin)palladium-dichlorid (7 Mol-%) und Kupfer(I)iodid (14 Mol-%) in 1-Methyl-2-pyrrolidinon (7 mL pro mmol Verbindung der allgemeinen Formel VI-A) gelöst. Nach 10 Minuten werden das Alkin der allgemeinen Formel HC≡C-R⁸ (3.5

Äquivalente) und N,N-Diisopropylethylamin (2 Äquivalente) zugegeben und die Reaktionsmischung wird 12 h bei einer Temperatur zwischen 90 und 110 °C gerührt.. Die Reaktionsmischung wird über Celite filtriert und mehrfach mit EE extrahiert. Die vereinigten organischen Phasen werden mit ges. aq. NaCl-Lsg. gewaschen, über MgSO₄ getrocknet und das Lösungsmittel wurde im Vakuum entfernt. Der Rückstand wird jeweils durch Säulenchromatographie (SiO₂, verschiedene Mischungen Hexan/EE) gereinigt.

Die folgenden Verbindungen A-174 bis A-180 wurden gemäß der vorstehend genannten allgemeinen Vorschrift erhalten:

### A-174: 2-Pent-1-inyl-6-trifluoromethyl-nicotinonitril

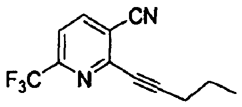

¹H NMR (300 MHz, CDCl₃) δ 8.13 (d, 1 H, *J* = 8.3 Hz), 7.68 (d, 1 H, 8.3 Hz), 2.55 (t, 2 H, *J* = 7.1 Hz), 1.68-1.80 (m, 2 H), 1.11 (t, 3 H, *J* = 7.3 Hz); IR (pur) 9969, 2230, 1569, 1406, 1341, 1197, 1153, 1086, 851 cm⁻¹; MS (FAB) *m*/*z* 239 (M+H)

### A-175: 2-(3,3-Dimethyl-but-1-inyl)-6-trifluoromethyl-nicotinonitril

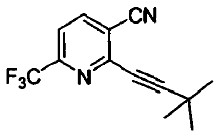

¹H NMR (300 MHz, CDCl₃) δ 8.11 (d, 1 H, *J* = 8.3 Hz), 7.66 (d , 1 H, *J* = 8.3 Hz), 1.41 (bs, 9 H); IR (pur) 2975, 2240, 2216, 1568, 1450, 1403, 1342, 1277, 1191, 1153, 1121, 1085, 850 cm⁻¹; MS (FAB) *m*/*z* 253 (M+H)

### A-176: 2-p-Tolylethinyl-6-trifluoromethyl-nicotinonitril

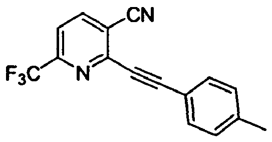

¹H NMR (300 MHz, CDCl₃) δ 8.17 (d, 1 H, *J* = 8.1 Hz), 7.69 (d , 1 H, *J* = 8.1 Hz), 7.57-7.65 (m, 2 H), 7.24-7.26 (m , 2 H), 2.41 (s, 3 H); IR (pur) 3079, 2216, 1567, 1413, 1343, 1286, 1184, 1143, 1112, 851, 819 cm⁻¹; MS (FAB) *m*/*z* 287 (M+H)

### A-177: 2-Hex-1-inyl-6-trifluoromethyl-nicotinonitril

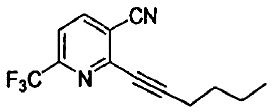

¹H NMR (300 MHz, CDCl₃) δ 8.12 (d, 1 H, *J* = 7.7 Hz), 7.67 (d, 1 H, *J* = 8.1 Hz), 2.57 (d, 2 H, *J* = 7.0 Hz), 1.61-1.76 (m, 2 H), 1.47-1.61 (m, 2 H), 0.97 (t, 3 H, *J* = 7.3 Hz); IR (pur) 2962, 2234, 1570, 1449, 1406, 1342, 1198, 1153, 1124, 1085, 849, 742 cm⁻¹; MS (FAB) *m*/*z* 253 (M+H)

### A-178: 2-(4-Methyl-pent-1-inyl)-6-trifluoromethyl-nicotinonitril

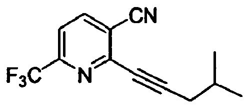

¹H NMR (300 MHz, CDCl₃) δ 8.13 (d, 1 H, *J* = 8.1 Hz), 7.68 (d, 1 H, *J* = 8.1 Hz), 2.47 (d, 2 H, *J* = 6.4 Hz), 2.04 (m, 1 H), 1.11 (d, 6 H, *J* = 6.6 Hz); IR (pur) 2964, 2233, 1571, 1450, 1405, 1341, 1198, 1153, 1086, 1017, 850, 743 cm⁻¹; MS (FAB) *m*/*z* 253 (M+H)

### A-179: 2-(3-Cyclohexyl-prop-1-inyl)-6-trifluoromethyl-nicotinonitril

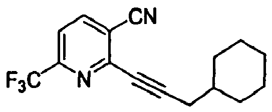

¹H NMR (300 MHz, CDCl₃) δ 8.12 (d, 1 H, *J* = 8.2 Hz), 7.66 (d, 1 H, *J* = 8.1 Hz), 2.47 (d, 2 H, *J* = 6.6 Hz), 1.39-1.94 (m, 5H), 0.88-1.40 (m, 6 H); IR (pur) 2925, 2852, 2231, 1569, 1448, 1405, 1341, 1194, 1154, 1124, 1085, 847 cm⁻¹ MS (FAB) *m*/*z* 293 (M+H)

### A-180: 2-(4-Fluoro-phenylethinyl)-6-trifluoromethyl-nicotinonitril

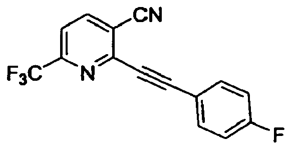

¹H NMR (300 MHz, CDCl₃) δ 8.19 (d, 1 H, *J* = 8.0 Hz), 7.67-7.70 (m, 3 H), 7.06-7.18 (m, 2 H); IR (pur) 3077, 2233, 1562, 1507, 1445, 1409, 1341, 1288, 1233, 1157, 1111, 840 cm⁻¹; MS (FAB) *m*/*z* 291 (M+H)

### 5. Allgemeine Vorschrift zur Darstellung von Aminen der allgemeinen (Formel V-D

Die Darstellung von Aminen der allgemeinen Formel V-D erfolgt wie im nachfolgenden Schema 4 dargestellt.

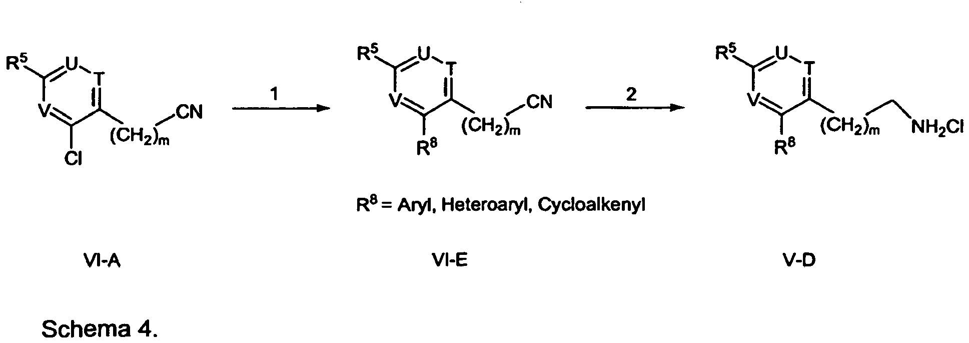

### Stufe 1: Darstellung von Nitrilen der allgemeinen Formel VI-E

Verbindungen der allgemeinen Formel VI-A (1 Äquivalent), worin R⁵, U, T und V die vorstehend genannte Bedeutung haben und m für 0, 1, 2 oder 3 steht, werden mit Palladiumdichlorid (5 mol-%) und einer Verbindung der allgemeinen Formel R⁸-B(OH)2 (2 Äquivalente) in einem Lösungsmittelgemisch aus Toluol/Dioxan/ 2 N aq. Natriumcarbonat-Lösung (20 ml pro 1 mmol Verbindungen der allgemeinen Formel VI-A) gerührt. Die Reaktionsmischung wird 12 h unter Rückfluß erhitzt und über Celite filtriert. Die vereinigten organischen Phasen werden über Magnesiumsulfat getrocknet und das Lösungsmittel im Vakuum entfernt. Der Rückstand wird säulenchromatographisch (SiO₂, verschiedene Lösungsmittelgemische von Hexan und EE) gereinigt.

Die folgenden Verbindungen A-181 bis A-201 wurden gemäß der vorstehend genannten allgemeinen Vorschrift erhalten:

### A-181: 2-(4-Chloro-phenyl)-6-trifluoromethyl-nicotinonitril

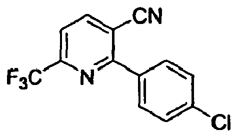

¹H NMR (300 MHz, CDCl₃) δ 8.29 (d, 1 H, *J* = 8.0 Hz), 7.98 (d, 2 H, *J* = 9.2 Hz), 7.76 (d, 1 H, *J* = 8.1 Hz), 7.54 (d, 2 H, *J* = 8.8 Hz); IR (pur) 2220, 1593, 1493, 1454, 1404, 1340, 1186, 1151, 1091, 1045, 1013, 841 cm⁻¹; MS (FAB) *m*/*z* 283 (M+H)

### A-182: 6-(Trifluoromethyl)-2-phenylpyridine-3-carbonitril

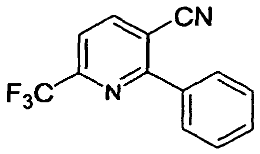

¹H NMR (300 MHz, CDCl₃) δ 7.50-7.55 (m, 3 H), 7.72 (d, 1 H, *J* = 7.8 Hz), 7.95-8.01 (m, 2 H), 8.24 (d, 1 H, *J* = 7.8 Hz); IR (pur) 2923, 2250, 1515, 1461, 1400, 1339, 1186, 1148 cm⁻¹; MS (FAB) *m*/*z* 249 (M+H)

### A-183: 2-Thiophen-2-yl-6-trifluoromethyl-nicotinonitril

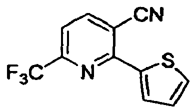

¹H NMR (400 MHz, CDCl₃) δ 8.33 (d, 1 H, *J* = 2.7 Hz), 8.17 (d, 1 H, *J* = 6.0 Hz), 7.61 (dd, 1 H, J = 7.8, 0.6 Hz), 7.58 (d, 1 H, J = 6.0 Hz), 7.20 (t, 1 H, J = 2.7 Hz); MS (FAB) *m*/*z* 255 (M+H)

### A-184: 2-(4-Fluoro-phenyl)-6-trifluoromethyl-nicotinonitril

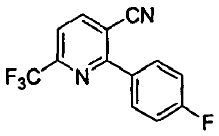

¹H NMR (300 MHz, CDCl₃) δ 8.27 (d, 1 H, *J* = 8.1 Hz), 8.04 (m, 2 H), 7.74 (d, 1 H, J = 8.1 Hz), 7.24 (m, 2 H); IR (pur) 3363, 2958, 1716, 1614, 1515, 1457, 1344, 1247, 1143, 1050, 833 cm⁻¹; MS (FAB) *m*/*z* 267 (M+H)

### A-185: 2-(4-tert-Butyl-phenyl)-&trifluoromethyl-nicotinonitril

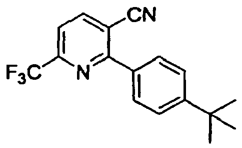

¹H NMR (300 MHz, CDCl₃) δ 8.26 (d, 1 H, *J* = 8.1 Hz), 7.96 (d, 2 H, J = 9.0 Hz), 7.70 (d, 1 H, J = 8.1 Hz), 7.54 (d, 2 H, J = 9.0 Hz), 1.37 (s, 9 H); IR (pur) 3267, 2920, 1731, 1604, 1510, 1413, 1345, 1229, 1141, 1094, 1049, 839, 749 cm⁻¹; MS (FAB) *m*/*z* 306 (M+H)

### A-186: 2-(3-Chloro-4-fluoro-phenyl)-6-trifluoromethyl-nicotinonitril

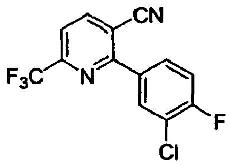

¹H NMR (300 MHz, CDCl₃) δ 8.29 (d, 1 H, *J* = 7.8 Hz), 8.02 (dd, 1 H, J = 6.9, 2.1 Hz), 7.95 (m, 1 H), 7.78 (d, 1 H, J = 7.8 Hz), 7.33 (t, 1 H, J= 8.4 Hz); MS (FAB) *m*/*z* 301 (M+H)

### A-187: 2-(3-Fluoro-phenyl)-6-trifluoromethyl-nicotinonitril

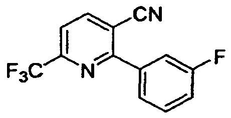

¹H NMR (300 MHz, CDCl₃) δ 8.29 (d, 1 H, *J* = 8.0 Hz), 7.82 (m, 1 H), 7.78 (d, 1 H, *J* = 8.0 Hz), 7.71 (m, 1 H), 7.53 (m, 1 H), 7.26 (m, 1 H); IR (pur) 3424, 2235, 1584, 1463, 1398, 1340, 1278, 1189, 1153, 1093, 1051, 918, 850, 781, 707 cm⁻¹; MS (FAB) *mlz* 267 (M+H)

### A-188: 2-Cyclohex-1-enyl-6-trifluoromethyl-nicotinonitril

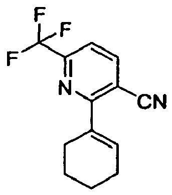

¹H NMR (300 MHz, CDCl₃) δ 8.13 (d, 1 H, J = 8.1 Hz), 7.19 (d, 1 H, J = 8.1 Hz), 6.65 (m, 1 H), 2.57 (m, 2 H), 2.33 (m, 2 H), 1.66-1.86 (m, 4 H); MS (FAB) *m*/*z* 253(M+H)

### A-189: 4'-tert-Butyl-5-trifluoromethyl-biphenyl-2-carbonitril

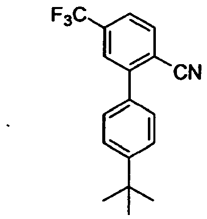

¹H NMR (300 MHz, CDCl₃) δ 7.89 (d, 1 H, J = 7.7 Hz), 7.78 (s, 1 H), 7.68 (d, 1H, J = 7.9 Hz), 7.54 (d, 4H, J = 0.9 Hz), 1.38 (s, 9H); IR (pur) 2964,2240,1538,1420,1335, 1260, 1175, 1075, 838 cm⁻¹; MS (FAB) *m*/*z* 304(M+H)

### A-190: 4'-Methoxy-5-trifluoromethyl-biphenyl-2-carbonitril

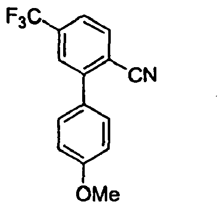

¹H NMR (300 MHz, CDCl₃) δ 7.88 (d, 1 H, J = 8.2 Hz), 7.75 (s, 1 H), 7.66 (d, 1 H, J = 8.0 Hz), 7.53 (dd, 2H, J = 6.8 Hz, J = 1.8 Hz), 7.05 (dd, 2H, J = 6.6 Hz, J = 2.0 Hz), 3.88 (s, 3H); IR (pur) 2958, 2240, 1610, 1517, 1294, 1076, 1040, 909, 831 cm⁻¹; MS (FAB) m/z 277(M+H)

### A-191: 3'-Chloro-5-trifluoromethyl-biphenyl-2-carbonitril

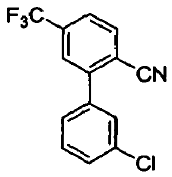

¹H NMR (300 MHz, CDCl₃) δ 7.93 (d, 1H, J = 8.3 Hz), 7.75 (d, 2H, J = 7.7 Hz), 7.53 (m, 1 H), 7.45 (m, 3H); IR (pur) 3068, 2232, 1567, 1411, 1252, 1041, 839, 698 cm⁻¹; MS (FAB) m/z 282(M+H)

### A-192: 3'-Fluoro-5-trifluoromethyl-biphenyl-2-carbonitril

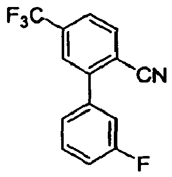

¹H NMR (300 MHz, CDCl₃) δ 7.92 (dd, 1H, J = 7.9 Hz, J = 0.6 Hz), 7.75 (m, 2H), 7.52 (m, 1 H), 7.37 (d, 1H, J = 6.0 Hz), 7.20 (m, 2H); IR (pur) 2238, 1588, 1489, 1450, 1292, 907, 841, 791, 701 cm⁻¹; MS (FAB) *m*/*z* 265(M+H)

### A-193: 3'-Chloro-4'-fluoro-5-trifluoromethyl-biphenyl-2-carbonitril

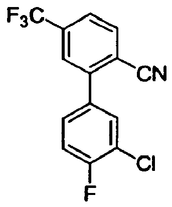

¹H NMR (300 MHz, CDCl₃) δ 7.93 (d, 1 H, J = 8.2 Hz), 7.76 (d, 2H, J = 7.1 Hz), 7.61 (dd, 1 H, J = 6.8 Hz, J = 2.4 Hz), 7.48 (m, 1 H), 7.32 (m, 1 H); IR (pur) 2238, 1490, 1416, 1333, 1263, 1177, 1075, 888, 835 cm⁻¹; MS (FAB) *m*/*z* 299(M+H)

### A-194: 3',4'-Dimethoxy-5-trifluoromethyl-biphenyl-2-carbonitril

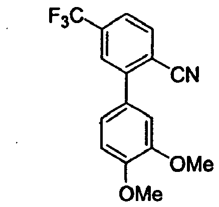

¹H NMR (300 MHz, CDCl₃) δ 7.88 (d, 1 H, J = 8.3 Hz), 7.78 (s, 1 H), 7.67(d, 1 H, J = 8.0 Hz), 7.14 (m, 2H), 7.01 (d, 1 H, J = 8.3 Hz), 3.97 (s, 3H), 3.96 (s, 3H), IR (pur) 2940, 2238, 1604, 1521, 1420, 1217, 1075, 1025, 838 cm⁻¹, MS (FAB) *m*/*z* 308(M+H)

### A-195: 2-Pyridin-3-yl-4-triftuoromethyl-benzonitril

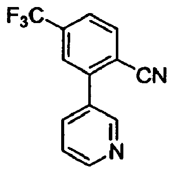

¹H NMR (300 MHz, CDCl₃) δ 8.81 (d, 1H, J = 2.2 Hz), 8.77 (dd, 1 H, J = 5.0 Hz, J = 1.7 Hz), 7.96 (m, 2H), 7.80 (d, 2H, J = 5.7 Hz), 7.50 (m, 1 H); IR (pur) 3031, 2238, 2229, 1569, 1415, 1015, 929, 839, 808 cm⁻¹; MS (FAB) *m*/*z* 249(M+H)

### A-196: 2-(3, 4-Dimethoxy-phenyl)-6-trifluoromethyl-nicotinonitril

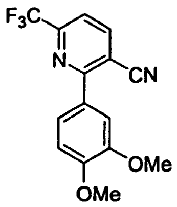

¹H NMR (300 MHz, CDCl₃) δ 8.19 (d, 1H, J = 7.9 Hz), 7.62 (m, 3H), 6.98 (d, 1 H, J = 8.4 Hz), 3.95 (s, 3H), 3.92 (s, 3H); IR (pur) 2969, 2238, 1569, 1462, 1340, 1088, 1024, 845, 762 cm⁻¹; MS (FAB) *m*/*z* 309(M+H)

### A-197: 2-(3,5-Dimethoxy-phenyl)-6-trifluoromethyl-nicotinonitril

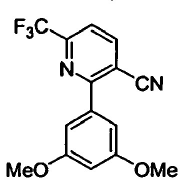

¹H NMR (300 MHz, CDCl₃) δ 8.23 (d, 1H, J = 8.1 Hz), 7.71 (d, 1H, J = 8.1 Hz), 7.10 (s, 2H), 6.61 (s, 1H), 3.85 (s, 6H); IR (pur) 2233, 1598, 1458, 1400, 920, 859, 831, 790 cm⁻¹; MS (FAB) *m*/*z* 309(M+H)

### A-198: 3',5'-Dimethoxy-5-trifluoromethyl-biphenyl-2-carbonitril

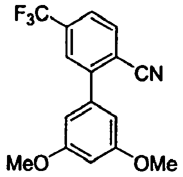

¹H NMR (300 MHz, CDCl₃) δ 7.80 (m, 2H), 6.95 (d, 1 H, J = 2.4 Hz), 6.73 (m, 2H), 6.57 (m, 1H), 3.86 (s, 3H), 3.85 (s, 3H); IR (pur) 2940, 2240, 1457, 1422, 1067, 905, 843, 701 cm⁻¹; MS (FAB) *m*/*z* 308(M+H)

### A-199: 2-(3-Chloro-phenyl)-6-trifluoromethyl-nicotinonitril

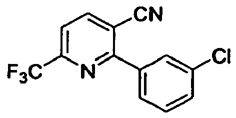

¹H NMR (300 MHz, CDCl₃) δ 8.29 (d, 1 H, *J* = 8.1 Hz), 7.96 (m, 1 H), 7.90 (m, 1 H), 7.78 (d, 1 H, *J* = 8.0 Hz), 7.46-7.55(m, 2 H); IR (pur) 3394, 2231, 1566, 1337, 1194, 1134, 1089, 850 cm⁻¹; MS (FAB) *m*/*z* 283 (M+H)

### A-200: 2-(2-Fluoro-phenyl)-6-trifluoromethyl-nicotinonitril

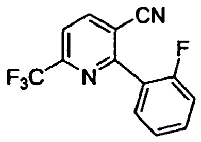

¹H NMR (300 MHz, CDCl₃) δ 8.29 (d, 1 H, *J* = 8.1 Hz), 7.83 (d, 1 H, *J* = 8.1 Hz), 7.65 (m, 1 H), 7.57 (m, 1 H), 7.36 (dd , 1 H, *J* = 7.5, 1.1 Hz), 7.30 (m, 1 H); IR (pur) 2230, 1617, 1463, 1401, 1340, 1186, 1149, 852, 762 cm⁻¹; MS (FAB) *m*/*z* 267 (M+H)

### A-201: 2-(4-Methoxy-phenyl)-6-trifluoromethyl-nicotinonitril

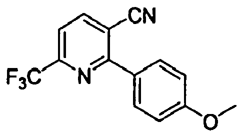

¹H NMR (300 MHz, CDCl₃) δ 8.231 H, *J* = 8.0 Hz), 8.04 (d, 2 H, *J* = 9.0 Hz), 7.67 (d, 1 H, *J* = 8.0 Hz), 7.06(d, 2 H, , *J* = 9.0 Hz), 3.90(s, 3 H); IR (pur) 2239, 1608, 1398, 1340, 1259, 1181, 1148, 1087, 841 cm⁻¹; MS (FAB) *m*/*z* 279 (M+H)

### Stufe 2:

### Methode 1

Verbindungen der allgemeinen Formel VI-E (5 mmol), worin R⁵, R⁸, U, T und V die vorstehend genannte Bedeutung haben und m für 0, 1, 2 oder 3 steht, Palladium auf Kohle (10 %, 500 mg) und konzentrierte Salzsäure (3 mL) oder Essigsäure werden in MeOH (30 mL) gelöst und 6 Stunden bei RT einer Wasserstoffatmosphäre ausgesetzt. Die Reaktionsmischung wird über Celite filtriert und das Filtrat wird im Vakuum eingeengt. Der Rückstand wird mittels Flashchromatographie (SiO₂, EE) gereinigt.

Die folgenden Verbindungen B-151 bis B-152 wurden gemäß der vorstehend genannten allgemeinen Vorschrift erhalten:

### B-151: C-(2-Cyclohexyl-6-trifluoromethyl-pyridin-3-yl)-methylamin

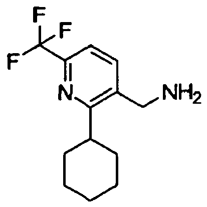

¹H NMR (300 MHz, CDCl₃) δ 7.81 (d, 1 H, J = 7.8 Hz), 7.45 (d, 1 H, J = 7.8 Hz), 3.99 (s, 2 H), 2.87 (m, 1 H), 1.72-1.88 (m, 6 H), 1.44 (bs, 2 H), 1.34-1.37 (m, 4 H); IR (pur) 2928, 2855, 1588, 1453, 1405, 1343, 1257, 1179, 1137, 1011, 917, 841 cm⁻¹; MS (FAB) *m*/*z* 259(M+H)

### B-152: C-(4-Phenyl-6-trifluoromethyl-pyridin-3-yl)-methylamin

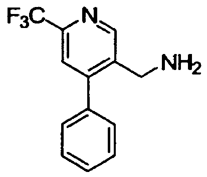

¹H NMR (300 MHz, CD₃OD) δ 7.76 (s, 1 H), 7.18-7.55 (m, 6 H), 4.27 (s, 2 H) IR (pur) 398, 2948, 1595 ,1491, 1404, 1332, 1220, 1140, 1084, 919, 769, 701 cm⁻¹; MS (FAB) *m*/*z* 253(M+H)

### Methode 2:

Verbindungen der allgemeinen Formel VI-E (2 mmol), worin R⁵, R³, U, T und V die vorstehend genannte Bedeutung haben und m für 0, 1, 2 oder 3 steht, werden in THF (10 mL, 10 mL) gelöst und BH₃•S(CH₃)₂ [2.0 M in THF, 3 mL, 3 Äquivalent] wird hinzu gegeben. Die Reaktionsmischung wird 8 Stunden zum Rückfluß erhitzt, aq. HCl (2 N) wird hinzu gegeben und die Reaktionsmischung wird erneut für 30 Minuten zum Rückfluß erhitzt. Die Reaktionsmischung wird mit aq. Natriumhydroxid-Lösung (2N) versetzt und mit EE gewaschen. Die vereinigten organischen Phasen werden mit ges. aq. NaCl-Lösung gewaschen und über Magnesiumsulfat getrocknet. Das Lösungsmittel wird im Vakuum entfernt und der Rückstand säulenchromatographisch (SiO₂, verschiedene Mischung aus Dichlormethan und Methanol als Laufmittel) gereinigt.

Die folgenden Verbindungen B-153 bis B-171 wurden gemäß der vorstehend genannten allgemeinen Vorschrift erhalten:

### B-153: (6-(Trifluoromethyl)-2-phenylpyridin-3-yl)methanamin

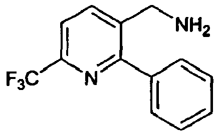

¹H NMR (300 MHz, CDCl₃) d 8.07 (d, 1 H, , *J* = 7.8 Hz), 7.67 (d, 1 H, , *J* = 7.8 Hz), 7.43 - 7.55 (m, 5H), 3.97 ((s, 2 H); IR (pur) 2924, 1402, 1344, 1179, 1136, 844, 768, 702 cm⁻¹; MS (FAB) *m*/*z* 253(M+H)

### B-154: (2-Bromo-6-(trifluoromethyl)pyridin-3-yl)methanamin

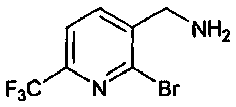

¹H NMR (300 MHz, CDCl₃) δ 7.91 (d, 1 H, J = 7.8 Hz), 7.61 (d, 1 H, J = 7.8 Hz), 3.95 (s, 2H); MS (FAB) *m*/*z* 256 (M+H)

### B-155: C-[2-(4-tert-Butyl-phenyl)-6-trifluoromethyl-pyridin-3-yl]-methylamin

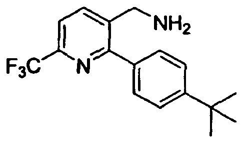

¹H NMR (300 MHz, CDCl₃ + CD₃OD) d 8.26 (d, 1H, J = 7.8 Hz), 7.63 (d, 1 H, J = 7.8 Hz), 7.40 (d, 2 H, J = 8.1 Hz), 7.29 (d, 2 H, J = 8.1 Hz), 4. 51(s, 2 H), 1.25 (s, 9 H); MS (FAB) *m*/*z* 309 (M+H)

### B-156: C-[2-(3-Chloro-4-fluoro-phenyl)-6-trifluoromethyl-pyridin-3-yl]-methylamin

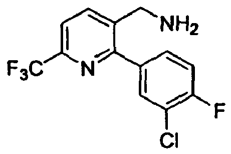

¹H NMR (300 MHz, CDCl₃) δ 8.14 (d, 1 H, *J* = 7.8 Hz), 7.65-7.71 (m, 2H), 7.46 (m, 1 H), 7.23 (t, 1 H, J= 8.4 Hz), 3.96 (s, 2 H); MS (FAB) *m*/*z* 305 (M+H)

### B-157: [4-(3-Aminomethyl-6-trifluoromethyl-pyridin-2-yl)-phenyl]-dimethyl-amin

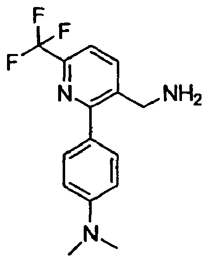

¹H NMR (300 MHz, CDCl₃) δ 8.03 (d, 1 H, J = 7.5 Hz), 7.56 (d, 1 H, J = 7.5 Hz), 7.47 (d, 2 H, J = 9.0 Hz), 6.77 (d, 2 H, J = 9.0 Hz), 4.06 (s, 2 H), 3.01 (s, 6 H), 2.36 (bs, 2 H); IR (pur) 3396, 2921, 1610, 1518, 1401, 1344, 1176, 944, 824 cm⁻¹; MS (FAB) *m*/*z* 296(M+H)

### B-158: C-[2-(4-Chloro-phenyl)-6-trifluoromethyl-pyridin-3-yl]-methylamin

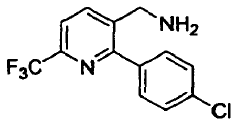

¹H NMR (300 MHz, CDCl₃) δ 8.11 (d, 1 H, *J* = 8.0 Hz), 7.66 (m, 1 H), 7.52(d, 2 H, *J* = 8.2 Hz), 7.44 (d, 2 H, *J* = 8.4 Hz), 3.96 (s, 2 H); IR (pur) 2921, 1595, 1460, 1407, 1344, 1178, 1138, 1093, 835 cm⁻¹; MS (FAB) *m*/*z* 287 (M+H)

### B-159: C-[2-(3-Fluoro-phenyl)-6-trifluoromethyl-pyridin-3-yl]-methylamin

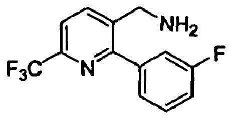

¹H NMR (300 MHz, CDCl₃) δ 8.12 (d, 1 H, *J* = 8.1 Hz), 7.70 (d, 1 H, 8.1 Hz), 7.45 (m, 1 H), 7.27-7.35 (m, 2 H), 7.09 (m, 1 H), 3.98 (s, 2H); IR (pur) 2922,1587,1463,1400, 1344, 1272, 1183, 1136, 845, 792, 708 cm⁻¹; MS (FAB) *m*/*z* 271 (M+H)

### B-160: C-[2-(3-Chloro-phenyl)-6-trifluoromethyl-pyridin-3-yl]-methylamin

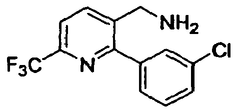

¹H NMR (300 MHz, CDCl₃) δ 8.11 (d, 1 H, *J* = 8.0 Hz), 7.70 (d, 1 H, *J* = 8.0 Hz), 7.56(m, 1 H), 7.37-7.46 (m, 3 H), 3.97 (s, 2 H); IR (pur) 2922, 1586, 1344, 1179, 1138, 1099, 888, 845 cm⁻¹; MS (FAB) *m*/*z* 287 (M+H)

### B-161: C-[2-(2-Fluoro-phenyl)-6-trifluoromethyl-pyridin-3-yl]-methylamin

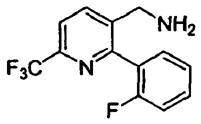

¹H NMR (300 MHz, CDCl₃) δ 8.14 (d, 1 H, *J* = 8.0 Hz), 7.74 (d, 1 H, *J* = 8.1 Hz), 7.42-7.48(m, 2 H), 7.30 (m, 1 H), 7.17 (m, 1 H), 3.86 (s, 2 H); IR (pur) 2924, 1617, 1456, 1345, 1179, 1138, 762 cm⁻¹; MS (FAB) *m*/*z* 271 (M+H)

### B-162: C-[2-(4-Methoxy-phenyl)-6-trifluoromethyl-pyridin-3-yl]-methylamin

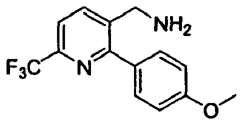

¹H NMR (300 MHz, CDCl₃) δ 8.06 (d, 1 H, *J* = 7.9 Hz), 7.63 (d, 1 H, *J* = 7.9 Hz), 7.52(d, 2 H, *J =* 8.8 Hz), 7.00 (d, 2 H, *J* = 8.8 Hz), 4.01 (s, 2 H), 3.87 (bs, 3 H); IR (pur) 2926, 1611, 1515, 1345, 1251, 1178, 1135, 837 cm⁻¹; MS (FAB) *m*/*z* 283 (M+H)

### B-163: C-(4'-tert-Butyl-5-trifluoromethyl-biphenyl-2-yl)-methytamin

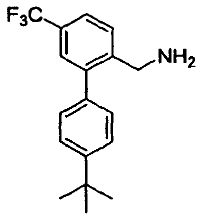

¹H NMR (300 MHz, CDCl₃) δ 7.60 (s, 2H), 7.47 (m, 3H), 7.26 (m, 2H), 3.87 (s, 2H), 1.37 (s, 9H); IR (pur) 2963, 1514, 1419, 1259, 1167, 1078, 1036, 836 cm⁻¹; MS (FAB) *m*/*z* 308(M+H)

### B-164: C-(4'-Methoxy-5-trifluoromethyl-biphenyl-2-yl)-methylamin

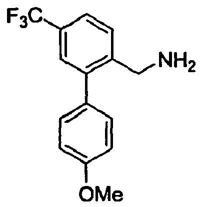

¹H NMR (300 MHz, CDCl₃) δ 7.59 (s, 2H), 7.48 (s, 1 H), 7.25 (m, 2H), 6.98 (dd, 2H, J = 8.6 Hz, J = 2.0 Hz), 3.86(s, 3H); IR (pur) 3328, 2914, 1610, 1516, 1464, 1418, 1042, 904 cm⁻¹; MS (FAB) *m*/*z* 282(M+H)

### B-165: C-(3'-Chloro-5-trifluoromethyl-biphenyl-2-yl)-methylamin

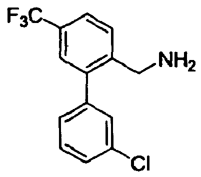

¹H NMR (300 MHz, CDCl₃) δ 7.65 (s, 2H), 7.47 (s, 1 H), 7.39 (m, 2H), 7.35 (m, 1 H), 7.22 (m, 1H), 3.84 (s, 2H); IR (pur) 2921, 1565, 1419, 1256, 1040, 835, 791, 701 cm⁻¹; MS (FAB) *m*/*z* 286(M+H)

### B-166: C-(3'-Fluoro-5-trifluoromethyl-biphenyl-2-yl)-methylamin

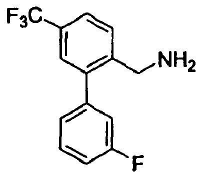

¹H NMR (300 MHz, CDCl₃) δ 7.64 (d, 2H, J = 1.3 Hz), 7.48 (s, 1 H), 7.41 (m, 1 H), 7.09 (m, 3H), 3.85 (s, 2H); IR (pur) 2920, 1615, 1485, 1444, 1274, 902, 791, 705 cm⁻¹; MS (FAB) *m*/*z* 270(M+H)

### B-167: C-(3'-Chloro-4'-fluoro-5-trifluoromethyl-biphenyl-2-yl)-methylamin

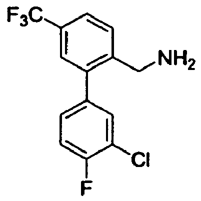

¹H NMR (300 MHz, CDCl₃) δ 7.65 (s, 2H), 7.43 (m, 2H), 7.24 (m, 2H), 3.83 (s, 2H); IR (pur) 2921, 1494, 1419, 1168, 1078, 886, 828 cm⁻¹; MS (FAB) *m*/*z* 304(M+H)

### B-168: C-(3',4'-Dimethoxy-5-trifluoromethyl-biphenyl-2-yl)-methylamin

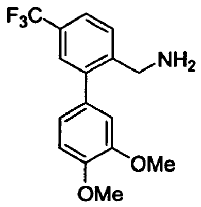

¹H NMR (300 MHz, CDCl₃) δ 7.60 (d, 2H, J = 1.3 Hz), 7.50 (s, 1 H), 6.94 (m, 1 H), 6.88 (m, 2H), 3.94 (s, 3H), 3.90 (s, 3H), 3.69 (m, 2H); IR (pur) 3367,2938,1518,1421, 1170, 1078,1026,816 cm⁻¹; MS (FAB) *m*/*z* 312(M+H)

### B-169: C-[2-(3,4-Dimethoxy-phenyl)-6-trifluoromethyl-pyridin-3-yl]-methylamin

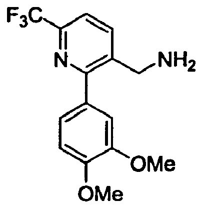

¹H NMR (300 MHz, CDCl₃) δ 8.06 (d, 1 H, J = 7.9 Hz), 7.64 (d, 1 H, J = 7.9 Hz), 7.16 (d, 1 H, J = 2.0 Hz), 7.12 (dd, 1 H, J = 8.1 Hz, J = 2.0 Hz), 6.96 (d, 1H, J = 8.3 Hz), 4.01 (s, 2H), 3.94 (s, 3H), 3.93 (s, 3H); IR (pur) 2937, 1604, 1463, 1415, 1253, 1175, 1026, 819 cm⁻¹; MS (FAB) *m*/*z* 313(M+H)

### B-170: C-[2-(3,5-Dimethoxy-phenyl)-6-trifluoromethyl-pyridin-3-yl]-methylamin

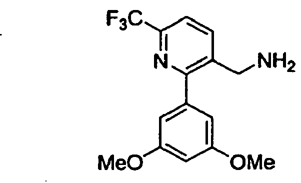

¹H NMR (300 MHz, CDCl₃) δ 8.07 (d, 1 H, J = 8.0 Hz), 7.67 (d, 1 H, J = 8.0 Hz), 6.65 (d, 2H, J = 1.8 Hz), 6.53 (t, 1 H, J = 2.2 Hz), 3.96 (s, 2H), 3.82 (s, 6H); IR (pur) 2942, 1597, 1460, 1401, 1345, 1098, 1040, 841 cm⁻¹; MS (FAB) m/z 313(M+H)

### B-171: C-(3',5'-Dimethoxy-5-trifluoromethyl-biphenyl-2-yl)-methylamin

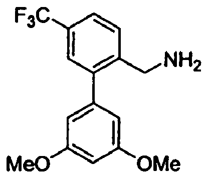

¹H NMR (300 MHz, CDCl₃) δ 7.60 (s, 2H), 7.50 (s, 1 H), 6.50 (t, 1 H, J = 2.4 Hz), 6.46 (d, 2H, J = 2.4 Hz), 3.87 (s, 2H), 3.82 (s, 6H); IR (pur) 2940, 1458, 1417, 1332, 1207, 1077, 903, 837 cm⁻¹; MS (FAB) m/z 312(M+H)

### 6. Allgemeine Vorschrift zur Darstellung von Carbonsäuren der allgemeinen Formel VIIa

Die Darstellung von Carbonsäuren der allgemeinen Formel Vlla erfolgt wie im nachfolgenden Schema 5 dargestellt.

### Schema 5.

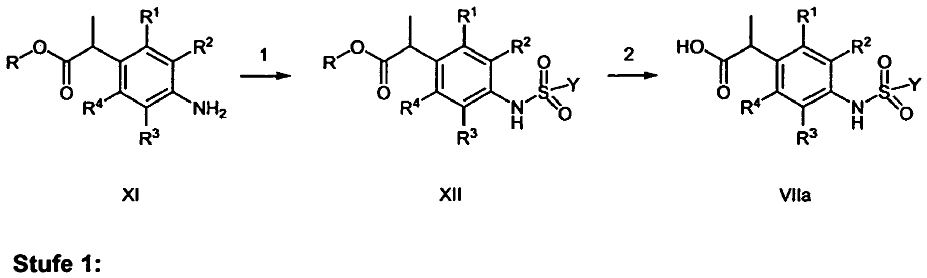

Verbindungen der allgemeinen Formel XI (7 mmol), worin R¹, R², R³ und R⁴ die vorstehend genannte Bedeutung haben und R für einen linearen oder verzweigten C₁₋₆-Alkyl-Rest steht, werden mit Verbindungen der allgemeinen Formel Cl-S(=O)₂-Y (8 mmol), worin Y die vorstehend genannte Bedeutung hat, 10 min bei 0 °C und anschließend 3 Stunden bei Raumtemperatur in Pyridin (10 mL) gerührt. Die Reaktionsmischung wird in Dichlormethan und aq. HCl (1 N) aufgenommen, die organische Phase wird abgetrennt und das Lösungmittel im Vakuum entfernt. Der Rückstand wird jeweils aus Dichlormethan/Hexan-Mischungen kristallisiert.

### Stufe 2:

Verbindungen der allgemeinen Formel XII (5 mmol), worin worin R¹, R², R³, R⁴ und Y die vorstehend genannte Bedeutung haben und R für einen linearen oder verzweigten C₁₋₆-Alkyl-Rest steht, werden mit Lithiumhydroxid Monohydrat (15 mmol) in einem Lösungsmittel-Gemisch aus Wasser und Tetrahydrofuran (1:2, 24 mL) 4 Stunden bei 40 °C gerührt. Die Reaktionsmischung wird in Dichlormethan und Wasser aufgenommen, mit aq. Salzsäure (1 N) versetzt und mehrfach mit Dichlormethan extrahiert. Die vereinigten organischen Phasen werden mit ges. aq. NaCl-Lösung gewaschen und über Natriumsulfat getrocknet. Das Lösungsmittel wird im Vakuum entfernt und der Rückstand aus Ethylacetat/Hexan-Mischungen kristallisiert.

### D-1: 2-(4-Dimethylaminosulfonylamino-3-fluoro-phenyl)-propionsäureethylester

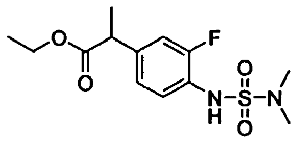

¹H NMR (300 MHz, CDCl₃) δ 7.44 (dd, 1 H, J = 8.1, 8.1 Hz), 6.94-7.05 (m, 2 H), 6.78 (bs, 1 H), 4.07 (m, 2 H), 3.62 (q, 1 H, J = 6.9 Hz), 2.76 (s, 6 H), 1.39 (d, 3 H, J = 6.9 Hz), 1.39 (t, 3 H, J = 7.2 Hz), 1.17(t, 3 H, J = 7.5 Hz) MS (FAB) *m*/*z* 319(M+H)

### D-2: 2-(4-Dimethylaminosulfonylamino-3-fluoro-phenyl)-propionsäure

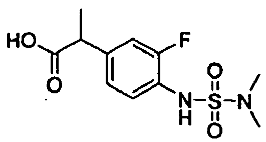

¹H NMR (300 MHz, CDCl₃) δ 750 (dd, 1 H, J = 8.1, 8.1 Hz), 7.05-7.12 (m, 2 H), 6.69 (bs, 1 H), 3.71 (q, 1 H, J = 6.9 Hz), 2.82 (s, 6 H), 1.49 (d, 3 H, J = 6.9 Hz) MS (FAB) *m*/*z* 291 (M+H)

### D-3: 2-[3-Fluoro-4-(2,2,2-trifluoro-ethansulfonylamino)-phenyl]-propionsäureethyl ester

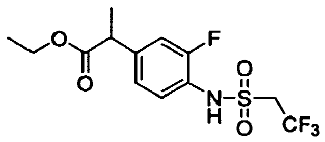

¹H NMR (300 MHz, CDCl₃) δ 7.50 (dd, 1 H, J = 8.1, 8.1 Hz), 7.11-7.18 (m, 2 H), 7.00 (bs, 1 H), 4.14 (m, 2 H), 3.87 (q, 2 H, J = 9.0 Hz), 3.70 (q, 1 H, J = 6.9 Hz), 1.49 (d, 3 H, J = 6.9 Hz), 1.23 (t, 3 H, J = 6.9 Hz) MS (FAB) *m*/*z* 358 (M+H)

### D-4: 2-[3-Fluoro-4-(2,2,2-trifluoro-ethansulfonylamino)-phenyl]-propionsäure

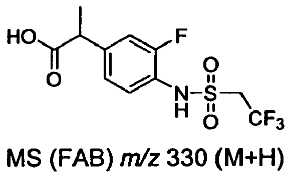

### D-5: 2-(3-Fluoro-4-trifluoromethansulfonylamino-phenyl)-propionsäureethyl ester

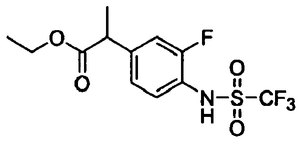

¹H NMR (300 MHz, CDCl₃) δ 7.45 (dd, 1 H, J = 8.1, 8.1 Hz), 7.09-7.16 (m, 2 H), 7.00 (bs, 1 H), 4.14 (m, 2 H), 3.70 (q, 1 H, J = 6.9 Hz), 1.49 (d, 3 H, J = 6.9 Hz), 1.22 (t, 3 H, J = 7.2 Hz); MS (FAB) *m*/*z* 344 (M+H)

### D-6: 2-(4-Aminosulfonylamino-3-fluoro-phenyl)-propionsäure

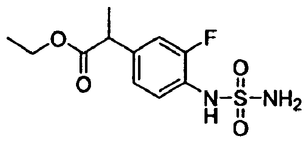

¹H NMR (300 MHz, CDCl₃) δ 7.49 (dd, 1 H, J = 8.1. 8.1 Hz), 7.04-7.12 (m, 2 H), 6.68 (bs, 1 H), 5.05 (bs, 2 H), 4.14 (m, 2 H), 3.68 (q, 1 H, J = 6.9 Hz), 1.46 (d, 3 H, J = 6.9 Hz), 1.23 (t, 3 H, J = 7.2 Hz) MS (FAB) *m*/*z* 291(M+H)

### D-7: 2-[3-Fluoro-4-(2,2,2-trifluoro-ethansulfonylamino)-phenyl]-propionsäure

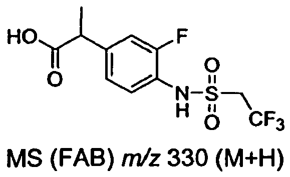

### D-8: 2-[3-Fluoro-4-(propan-2-sulfonylamino)-phenyl]-propionsäureethylester

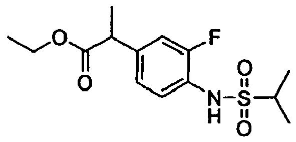

¹H NMR (300 MHz, CDCl₃) δ 7.55 (dd, 1 H, J = 8.1, 8.1 Hz), 7.05-7.12 (m, 2 H), 6.71 (bs, 1 H), 4.15 (m, 2 H), 3.67 (q, 1 H, J = 6.9 Hz), 3.07 (m, 1 H), 1.47 (d, 3 H, J = 6.9 Hz), 1.40 (d, 6 H, J = 6.9 Hz), 1.22 (t, 3 H, J = 7.2 Hz) MS (FAB) *m*/*z* 318(M+H)

### D-9: 2-(4-Ethansulfonylam ino-3-fluoro-phenyl)-propionsäureethylester

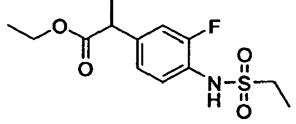

¹H NMR (300 MHz, CDCl₃) δ 7.52 (dd, 1 H, J = 8.1, 8.1 Hz), 7.06-7.14 (m, 2 H), 6.62 (bs, 1 H), 4.13 (m, 2 H), 3.66 (q, 1 H, J = 6.9 Hz), 3.12 (q, 2 H, J = 7.2 Hz), 1.47 (d, 3 H, J = 6.9 Hz), 1.39 (t, 3 H, J = 7.2 Hz), 1.2 5(t, 3 H, J = 7.2 Hz) MS (FAB) *m*/*z* 304(M+H)

### D-10: 2-(4-Ethansulfonylamino-3-fluoro-phenyl)-propionsäure

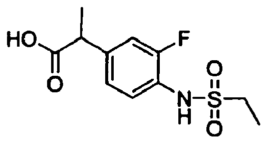

¹H NMR (300 MHz, CDCl₃) δ 7.53 (dd, 1 H, J = 8.1, 8.1 Hz), 7.08-7.15 (m, 2 H), 6.76 (bs, 1 H), 3.71 (q, 1 H, J = 6.9 Hz), 3.12 (q, 2 H, J = 7.5 Hz), 1.50 (d, 3 H, J = 6.9 Hz), 1.39 (t, 3 H, J = 7.5 Hz) MS (FAB) *m*/*z* 276(M+H)

Verbindungen der allgemeinen Formel VIIa, worin R² für Methyl steht, können gemäß dem nachfolgend angegebenen Verfahren hergestellt werden.

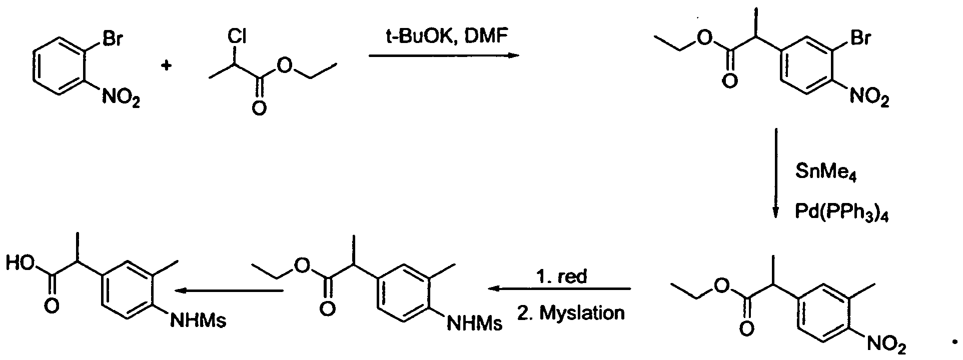

Zu einer Lösung von Kalium-tert-butoxide (125.7 g, 1.12 mol) in DMF (600 mL) wird bei -30 °C innerhalb von 3 Minuten eine Mischung aus 1-Brom-2-nitro-benzol (56.5 g, 0.28 mol) und Ethyl-2-chloropropionat (38.7 g, 0.28 mol) gegeben.

Die Reaktionsmischung wird 2 min bei -30 °C gerührt und weiteres Ethyl-2-chloropropionat (3.87 g, 0.028 mol) wird hinzugegeben. Nach 5 Minuten wird die Reaktionsmischung in eine kühle 10-%ige aq. HCl-Lösung gegeben, mit Wasser verdünnt und mehrfach mit EtOAc extrahiert. Die vereinigten organischen Phasen werden mit Wasser und ges. aq. NaCl-Lösung gewaschen, über MgSO₄ getrocknet und das Lösungsmittel wird im Vakuum entfernt. Der Rückstand wird mittels Säulenchromatographie (SiO₂, EtOAc:hexanes (1:10)) gereinigt.

Zu einer Lösung von Ethyl-2-(3-bromo-4-nitrophenyl)propanoat (3.76 g, 0.012 mol) in DMF (20 mL) wird unter Stickstoffatmosphäre Pd(PPh₃)₄ (0.77 g, 5 mol %) und Tetramethylzinn (6.68 g, 0.037 mol) gegeben. Die Reaktionsmischung wird 8 h bei 120 °C gerührt und über Celite filtriert. Das Filtrat wird mit Wasser verdünnt und mehrfach mit EtOAc extrahiert. The vereinigten organischen Phasen werden mit Wasser und ges. aq. NaCl-Lösung gewaschen, über MgSO₄ getrocknet und das Lösungsmittel wird im Vakuum entfernt. Der Rückstand wird mittels Säulenchromatographie (SiO₂, EtOAc:hexanes (1:10)) gereinigt.

Eine Lösung von Ethyl-2-(3-methyl-4-nitrophenyl)propanoat (1.76 g, 0.007 mol) und 10% Pd auf Kohle (200 mg) in MeOH (30 mL) wird 6 Stunden einer Wasserstoffatmosphäre ausgesetzt. Die Reaktionsmischung wird filtriert und das Lösungsmittel im Vakkum entfernt. Der Rückstand wird mittels Säulenchromatographie (SiO₂, EtOAc:hexanes (1:4)) gereinigt. Das so gewonnene Produkt (1.43 g, 0.007 mol) und Methansulfonylchlorid (0.95 g, 0.008 mol) in pyridine (10 mL) werden 10 Minuten bei 0°C und 3 h bei RT gerührt. Pyridin wird durch Aufarbeitung mit 1 N aq. HCl/Dichlormethan entfernt. Das Lösungsmittel wird im Vakuum entfernt und der Rückstand wird durch Kristallisation aus Dichlormethan und n-Hexan gereinigt.

### 7. Allgemeine Vorschrift zur Umsetzung von Aminen der allgemeinen Formeln V oder X mit Carbonsäuren der allgemeinen Formel VII

Die Säure der allgemeinen Formel VII (1 Äquivalent), das Amin der allgemeinen Formeln V oder X (1.2 Äquivalente) und EDCI (1.2 Äquivalente) werden in DMF (10 mmol Säure in 20 mL) 12 Stunden bei RT gerührt und anschließend Wasser dazugegeben. Die Reaktionsmischung wird mehrfach mit EE extrahiert, die wäßrige Phase wird mit NaCl gesättigt und anschließend wieder mit EE extrahiert. Die vereinigten organischen Phasen werden mit 1 N Salzsäure und ges. aq. NaCl-Lsg. gewaschen, über MgSO₄ getrocknet und das Lösungsmittel wird im Vakuum entfernt. Der Rückstand wird mittels Flashchromatographie (SiO₂, EE/Hexan 1:2) gereinigt.

Die folgenden Beispielverbindungen 1, 2, 3, 10, 12, 13, 33 und 34 wurden gemäß der vorstehend genannten allgemeinen Vorschrift erhalten:

### Beispiel 2:

### 2-(3-Fluor-4-(methylsulfonylamino)phenyl)-N-((2-(piperidin-1-yl)-6-(trifluormethyl)pyridin-3-yl)methyl)propanamid

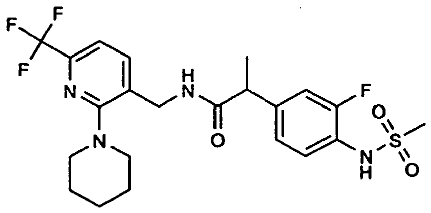

Die Verbindung wurde in einer Ausbeute von 88 % als weißer Feststoff mit einem Schmelzpunkt von 75 - 79 °C erhalten.

¹H NMR (300 MHz, CDCl₃) δ 7.47-7.55 (m, 2H, Ar), 7.07-7.22 (m, 3 H, Ar), 6.33 (bt, 1 H, NHCO), 4.47 (d, 2 H, *J* = 5.7 Hz, ArC*H*₂NH), 3.54 (q, 1 H, J = 6.9 Hz, C*H*CH₃), 3.00-3.05 (m, 7 H, Piperidin, SO₂CH₃), 1.61 (m, 6 H, Piperidin), 1.52 (d, 3 H, *J* = 6.9 Hz, CHC*H*₃) IR (KBr) 3741, 3281,2935, 1652, 1512, 1419, 1334,1248 cm⁻¹ MS (FAB) *m*/*z* 503 (M+H)

### Beispiel 10:

### (S)-2-(3-Fluor-4-(methylsulfonylamino)phenyl)-N-((2-(piperidin-1-yl)-6-(trifluormethyl)pyridin-3-yl)methyl)propanamid

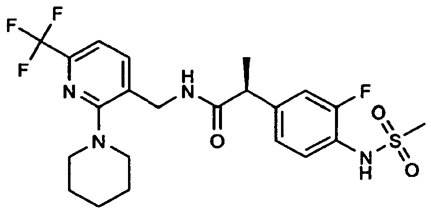

Die Verbindung wurde in einer Ausbeute von 65 % als weißer Feststoff mit einem Schmelzpunkt von 75 - 79°C erhalten.

¹H NMR (300 MHz, CDCl₃) δ 7.47-7.55 (m, 2H, Ar), 7.07-7.22 (m, 3 H, Ar), 6.33 (bt, 1 H, NHCO), 4.47 (d, 2 H, *J* = 5.7 Hz, ArCH₂NH), 3.54 (q, 1 H, *J* = 6.9 Hz, C*H*CH₃), 3.00-3.05 (m, 7 H, Piperidin, SO₂CH₃), 1.52 (d, 3 H, *J* = 6.9 Hz, CHC*H*₃), 1.61 (m, 6 H, Piperidin) IR (KBr) 3289, 2935, 2853, 1655, 1591, 1512, 1419, 1335 cm⁻¹ MS (FAB) m/z 503 (M+H)

### Beispiel 12:

### 2-(3-Fluor-4-(methylsulfonylamino)phenyl)-N-((2-morpholino-6-(trifluormethyl)pyridin-3-yl)methyl)propanamid

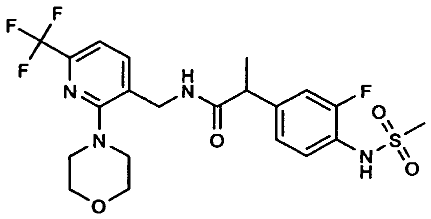

Die Verbindung wurde in einer Ausbeute von 60 % als weißer Feststoff erhalten.

¹H NMR (300 MHz, CDCl₃) δ 7.49-7.56 (m, 2 H, Ar), 7.26 (d, 1 H, *J* = 7.5 Hz, Ar), 7.08-7.17 (m, 2 H, Ar), 6.53 (bs, 1 H, NHSO₂), 6.06 (bt, 1 H, NHCO), 4.48 (d, 2 H, *J* = 5.7 Hz, ArCH₂NH), 3.76 (m, 4 H, Morpholin), 3.57 (q, 1 H, *J* = 6.9 Hz, C*H*CH₃), 3.13 (m, 4 H, Morpholin), 3.04 (s, 3 H, SO₂CH₃), 1.55 (d, 3 H, *J* = 6.9 Hz, CHC*H*₃) IR (KBr) 3741, 1645, 1512, 1416, 1334, 1157 cm⁻¹ MS (FAB) *m*/*z* 505 (M+H)

### Beispiel 13:

### 2-(3-Fluor-4-(methylsulfonylamino)phenyl)-N-((-(pyrrolidin-1-yl)-6-(trifluormethyl)pyridin-3-yl)methyl)propanamid

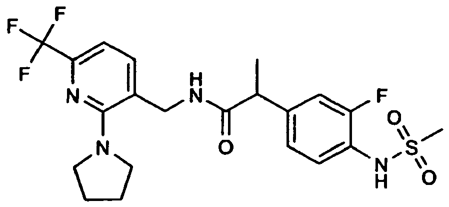

Die Verbindung wurde in einer Ausbeute von 80 % erhalten.

¹H NMR (300 MHz, CDCl₃) δ 7.51 (t, 1 H, *J* = 8.3 Hz, H-5), 7.38 (d, 2 H, *J* = 7.5 Hz Ar), 7.13 (dd, 1 H, *J* = 11.1, 2.0 Hz, Ar), 7.07 (dd, 1 H, *J* = 7.8, 1.8 Hz, Ar), 6.94 (d, 1 H, *J* = 7.5 Hz, Ar), 5.72 (bt, 1 H, NHCO), 4.47 (d, 2 H, *J* = 5.3 Hz, ArCH₂NH), 3.52 (q, 1 H, *J* = 6.9 Hz, C*H*CH₃), 3.42-3.46 (m, 4 H, Pyrrolidin), 3.02 (s, 3 H, SO₂CH₃), 1.82-1.89 (m, 4 H, Pyrrolidin), 1.50 (d, 3 H, *J* = 6.9 Hz, CHCH₃)

### Beispiel 1:

### 2-(3-Fluor-4-(methylsulfonylamino)phenyl)-N-((2-methyl-6-(trifluormethyl)pyridin-3-yl)methyl)propanamid

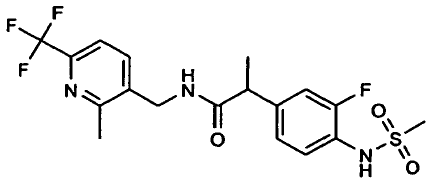

Die Verbindung wurde in einer Ausbeute von 78 % als weißer Feststoff mit einem Schmelzpunkt von 149 -152°C erhalten.

¹H NMR (300 MHz, CDCl₃) δ 7.54 (d, 1 H, *J* = 7.8 Hz, Ar), 7.41-7.46 (m, 2 H, Ar), 7.14 (dd, 1 H, *J* = 11.1, 2.0 Hz, Ar), 7.07 (dd, 1 H, *J* = 7.8, 1.8 Hz, Ar), 6.87 (bs, 1 H, NHSO₂), 6.16 (bt, 1 H, NHCO), 4.43 (d, 2 H, *J* = 5.1 Hz, ArCH₂NH), 3.58 (q, 1 H, *J* = 6.9 Hz, C*H*CH₃), 3.01 (s, 3 H, SO₂CH₃), 2.50 (s, 3 H, ArCH₃), 1.50 (d, 3 H, *J* = 6.9 Hz, CHC*H*₃)

IR (KBr) 3741, 1649, 1513, 1338, 1153, 756 cm⁻¹ MS (FAB) *m*/*z* 434 (M+H)

### Beispiel 3:

### 2-(3-Fluor-4-(methylsulfonylamino)phenyl)-N-((2-piperidin-1-yl)-4-(trifluormethyl)benzyl)propanamid

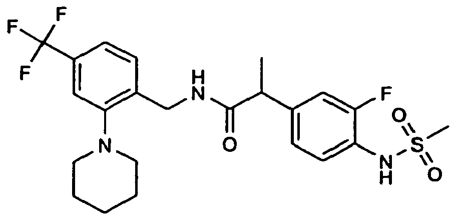

Die Verbindung wurde in einer Ausbeute von 78 % als leicht gelber Feststoff mit einem Schmelzpunkt von 126 - 127 °C erhalten.

¹H NMR (300 MHz, CDCl₃) δ 7.48 (t, 1 H, *J* = 8.3 Hz, H-5), 7.32 (bd, 2 H, Ar), 7.05-7.15 (m, 4 H, Ar), 6.81 (bs, 1 H, NHSO₂), 6.66 (bt, 1 H, NHCO), 4.52 (d, 2 H, *J* = 5.1 Hz, ArCH₂NH), 3.55 (q, 1 H, *J* = 6.9 Hz, C*H*CH₃), 3.00 (s, 3 H, SO₂CH₃), 2.79 (bs, 4 H, Piperidin), 1.49-1.64 (m, 7 H, Piperidin, CHC*H*₃), 1.25 (m, 2 H, Piperidin) IR (KBr) 3289,2934, 1652, 1511, 1423, 1337, 1220, 1160 cm⁻¹

MS (FAB) *m*/*z* 502 (M+H)

### Beispiel 33:

### 2-(3-Fluor-4-(methylsulfonylamino)phenyl)-N-(2-morpholino-4-(trifluormethyl)benzyl)propanamid

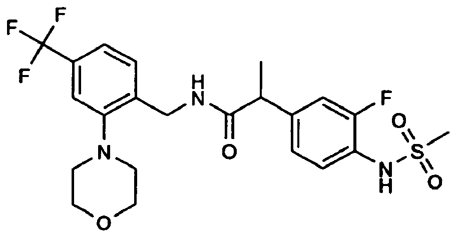

Die Verbindung wurde in einer Ausbeute von 60 % als weißer Feststoff erhalten.

¹H NMR (300 MHz, CDCl₃) δ 7.43 (t, 1 H, *J* = 8.3 Hz, Ar), 7.16-7.24 (m, 3 H, Ar), 7.09 (dd, 1 H, *J* = 11.1, 2.0 Hz, Ar), 7.01 (dd, 1 H, *J* = 7.8, 1.8 Hz, Ar), 6.70 (bs, 1 H, NHSO₂), 6.15 (bt, 1 H, NHCO), 4.47 (d, 2 H, *J* = 5.4 Hz, ArCH₂NH), 3.70 (t, 4 H, *J* = 4.2 Hz, Morpholin), 3.50 (q, 1 H, *J* = 7.2 Hz, CHCH₃), 2.95 (s, 3 H, SO₂CH₃), 2.78 (t, 4 H, *J* = 4.2 Hz, Morpholin), 1.45 (d, 3 H, *J* = 7.2 Hz, CHC*H*₃)

IR (KBr) 2921, 1650, 1512, 1423, 1336, 1159 cm⁻¹

MS (FAB) *m*/*z* 504 (M+H)

### Beispiel 34:

### 2-(3-Fluor-4-(methylsulfonylamino)phenyl)-N-(2-(pyrrolidin-1-yl)-4-(trifluormethyl)benzyl)propanamid

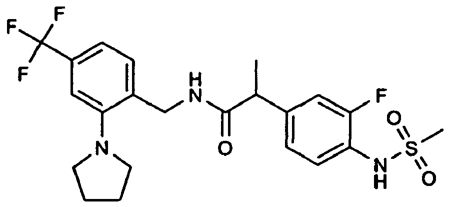

Die Verbindung wurde in einer Ausbeute von 70 % erhalten.

¹H NMR (300 MHz, CDCl₃) δ 7.49 (t, 1 H, *J*= 8.1 Hz, Ar), 7.05-7.19 (m, 5 H, Ar), 6.79 (bs, 1 H, NHSO₂), 6.26 (bt, 1 H, NHCO), 4.49 (d, 2 H, *J* = 4.8 Hz, ArCH₂NH), 3.54 (q, 1 H, *J* = 7.2 Hz, C*H*CH₃), 3.08-3.12 (m, 4 H, Pyrrolidin), 3.01 (s, 3 H, SO₂CH₃), 1.86-1.90 (m, 4 H, Pyrrolidin), 1.50 (d, 3 H, *J* = 7.2 Hz, CHC*H*₃)

Die in der Tabelle 1 aufgeführten Verbindungen können ebenfalls wie vorstehend beschrieben erhalten werden. Dem Fachmann sind die dafür benötigten Ausgangsverbindungen bekannt.

**Tabelle 1:**

| | |
|---|---|
| [4] | 2-(3-Fluor-4-(methylsulfonylamino)phenyl)-N-((2-fluor-6-(trifluormethyl)pyridin-3-yl)methyl)propanamid |
| [5] | N-((2-Chlor-6-(trifluormethyl)pyridin-3-yl)methyl)-2-(3-fluor-4-(methylsulfonylamino)phenyl)propanamid |
| [6] | N-((2-Brom-6-(trifluormethyl)pyridin-3-yl)methyl)-2-(3-fluor-4-(methylsulfonylamino)phenyl)propanamid |
| [7] | 2-(3-Fluor-4-(methylsulfonylamino)phenyl)-N-((2-iod-6-(trifluormethyl)pyridin-3-yl)methyl)propanamid |
| [8] | N-((2-tert-Butyl-((trifluormethyl)pyridin-3-yl)methyl)-2-(3-fluor-4-(methylsulfonylamino)phenyl)propanamid |
| [9] | N-((2-Cyano-6-(trifluormethyl)pyridin-3-yl)methyl)-2-(3-fluor-4-(methylsulfonylamino)phenyl)propanamid |
| [11] | (R)-2-(3-Fluor-4-(methylsulfonylamino)phenyl)-N-((2-(piperidin-1-yl)-6-(trifluormethyl)pyridin-3-yl)methyl)propanamid |
| [14] | N-((2-(Dimethylamino)-6-(trifluormethyl)pyridin-3-yl)methyl)-2-(3-fluor-4-(methylsulfonylamino)phenyl)propanamid |
| [15] | N-((2-(Diethylamino)-6-(trifluormethyl)pyridin-3-yl)methyl)-2-(3-fluor-4-(methylsulfonylamino)phenyl)propanamid |
| [16] | N-((2-(Dipropylamino)-6-(trifluormethyl)pyridin-3-yl)methyl)-2-(3-fluor-4-(methylsulfonylamino)phenyl)propanamid |
| [17] | 2-(3-Fluor-4-(methylsulfonylamino)phenyl)-N-((2-hydroxy-6-(trifluormethyl)pyridin-3-yl)methyl)propanamid |
| [18] | 2-(3-Fluor-4-(methylsulfonylamino)phenyl)-N-((2-methoxy-6-(trifluormethyl)pyridin-3-yl)methyl)propanamid |
| [19] | N-((2-Butoxy-6-(trifluormethyl)pyridin-3-yl)methyl)-2-(3-fluor-4-(methylsulfonylamino)phenyl)propanamid |
| [20] | 2-(3-Fluor-4-(methylsulfonylamino)phenyl)-N-((2-isopropoxy-6-(trifluormethyl)pyridin-3-yl)methyl)propanamid |
| [21] | N-((2-Cyclopentyloxy-6-(trifluormethyl)pyridin-3-yl)methyl)-2-(3-fluor-4-(methylsulfonylamino)phenyl)propanamid |
| [22] | 2-(3-Fluor-4-(methylsulfonylamino)phenyl)-N-((2-phenyl-6-(trifluormethyl)pyridin-3-yl)methyl)propanamid |
| [23] | 2-(3-Fluor-4-(methylsulfonylamino)phenyl)-N-((2-(4-fluor-phenyl)-6-(trifluormethyl)pyridin-3-yl)methyl)propanamid |
| [24] | 2-(3-Fluor-4-(methylsulfonylamino)phenyl)-N-((6-(trifluormethyl)-2,2'-bipyridin-3-yl)methyl)propanamid |
| [25] | 2-(3-Fluor-4-(methylsulfonylamino)phenyl)-N-((6-(trifluormethyl)-2,3'-bipyridin3-yl)methyl)propanamid |
| [26] | 2-(3-Fluor-4-(methylsulfonylamino)phenyl)-N-((2-(pyrimidin-2-yl)-6-(trifluormethyl)pyridin-3-yl)methyl)propanamid |
| [27] | 2-(3-Fluor-4-(methylsulfonylamino)phenylrN-((2-(thiazol-2-yl)-6-(trifluormethyl)pyridin-3-yl)methyl)propanamid |
| [28] | 2-(3-Fluor-4-(methylsulfonylamino)phenyl)-N-((2-(oxazol-2-yl)-6-(trifluormethyl)pyridin-3-yl)methyl)propanamid |
| [29] | N-((2-(1H-Imidazol-2-yl)-6-(trifluormethyl)pyridin-3-yl)methyl)-2-(3-fluor-4-(methylsulfonylamino)phenyl)propanamid |
| [30] | N-(2-Cyano-4-(trifluormethyl)benzyl-(3-fluor-4-(methylsulfonylamino)phenyl)propanamid |
| [31] | (S)-2-(3-Fluor-4-(methylsulfonylamino)phenyl)-N-(2-(piperidin-1-yl)-4-(trifluormethyl)benzyl)propanamid |
| [32] | (R)-2-(3-Fluor-4-(methylsulfonylamino)phenyl)-N-(2-(piperidin-1-yl)-4-(trifluormethyl)benzyl)propanamid |
| [35] | N-(2-(Dimethylamino)-4-(trifluormethyl)benzyl)-2-(3-fluor-4-(methylsulfonylamino)phenyl)propanamid |
| [36] | N-(2-(Diethylamino)-4-(trifluormethyl)benzyl)-2-(3-fluor-4-(methylsulfonylamino)phenyl)propanamid |
| [37] | N-(2-(Dipropylamino)-4-(trifluormethyl)benzyl)-2-(3-fluor-4-(methylsulfonylamino)phenyl)propanamid |
| [38] | 2-(3-Fluor-4-(methylsulfonylamino)phenyl)-N-(2-hydroxy-4-(trifluormethyl)benzyl)propanamid |
| [39] | 2-(3-Fluor-4-(methylsulfonylamino)phenyl)-N-(2-methoxy-4-(trifluormethyl)benzyl)propanamid |
| [40] | N-(2-Butoxy-4-(trifluormethyl)benzyl)-2-(3-fluor-4-(methylsulfonylamino)phenyl)propanamid |
| [41] | 2-(3-Fluor-4-(methylsulfonylamino)phenyl)-N-(2-isopropoxy-4-(trifluormethyl)benzyl)propanamid |
| [42] | N-(2-(Cyclopentyloxy)-4-(trifluormethyl)benzyl)-2-(3-fluor-4-(methylsulfonylamino)phenyl)propanamid |
| [43] | 2-(3-Fluor-4-(methylsulfonylamino)phenyl)-N-((5-(trifluormethyl)biphenyl-2-yl)methyl)propanamid |
| [44] | 2-(3-Fluor-4-(methylsulfonylamino)phenyl)-N-((4'-fluor-5-(trifluormethyl)biphenyl-2-yl)methyl)propanamid |
| [45] | 2-(3-Fluor-4-(methylsulfonylamino)phenyl)-N-(2-(pyridin-2-yl)-4-(trifluormethyl)benzyl)propanamid |
| [46] | 2-(3-Fluor-4-(methylsulfonylamino)phenyl)-N-(2-(pyridin-3-yl)-4-(trifluormethyl)benzyl)propanamid |
| [47] | 2-(3-Fluor-4-(methylsulfonylamino)phenyl)-N-(2-(pyrimidin-2-yl)-4-(trifluormethyl)benzyl)propanamid |
| [48] | 2-(3-Fluor-4-(methylsulfonylamino)phenyl)-N-(2-(thiazol-2-yl)-4-(trifluormethyl)benzyl)propanamid |
| [49] | 2-(3-Fluor-4-(methylsulfonylamino)phenyl)-N-(2-(oxazol-2-yl)-4-(trifluormethyl)benzyl)propanamid |
| [50] | N-(2-(1H-Imidazol-2-yl)4-(trifluormethyl)benzyl)-2-(3-fluor4-(methylsulfonylamino)phenyl)propanamid |
| [51] | N-((6-tert-Butyl-2-(piperidin-1-yl)pyridin-3-yl)methyl)-2-(3-fluor-4-(methylsufonamido)phenyl)propanamid |
| [52] | 2-(3-Fluor-4-(methylsulfonylamino)phenyl)-N-((4-(piperidin-1-yl)-6-(trifluormethyl)pyridin-3-yl)methyl)propanamid |
| [53] | 2-(3-Fluor-4-(methylsulfonylamino)phenyl)-N-((3-(piperidin-1-yl)-5-(trifluormethyl)pyridin-2-yl)methyl)propanamid |
| [54] | 2-(3-Fluor-4-(methylsulfonylamino)phenyl)-N-((4-(piperidin-1-yl)-2-(trifluormethyl)pyrimidin-5-yl)methyl)propanamid |
| [55] | 2-(3-Fluor-4-(methylsulfonylamino)phenyl)-N-((3-(piperidin-1-yl)-5-(trifluormethyl)pyrazin-2-yl)methyl)propanamid |
| [56] | 2-(3-Fluor-4-(methylsulfonylamino)phenyl)-N-((4-(piperidin-1-yl)-6-(trifluormethylpyridazin-3-yl)methyl)propanamid |
| [57] | 2-(3-Fluor-4-(methylsulfonylamino)phenyl)-N-((2-piperidin-1-yl)-6-(trifluormethyl)pyridin-3-yl)propanamid |
| [58] | 2-(3-Fluor-4-(methylsulfonylamino)phenyl)-N-((2-piperidin-1-yl)-4-(trifluormethyl)phenyl)propanamid |
| [59] | 2-(3-Fluor-4-(methylsulfonylamino)phenyl)-N-(2-(2-(piperidin-1-yl)-6-(trifluormethyl)pyridin-3-yl)ethyl)propanamid |
| [60] | 2-(3-Fluor-4-(methylsulfonylamino)phenyl)-N-(2-(piperidin-1-yl)-4-(trifluormethyl)phenethyl)propanamid |
| [61] | N-(2-Amino-4-(trifluormethyl)benzyl)-2-(3-fluor-4-(methylsulfonylamino)phenyl)propanamid |
| [62] | 2-(3-Fluor-4-(methylsulfonylamino)phenyl)-N-(2-nitro-4-(trifluormethyl)benzyl)propanamid |
| [63] | N-(4-tert-Butyl-2-(piperidin-1-yl)benzyl)-2-(3-fluor-4-(methylsu)fonylamino)phenyl)propanamid |
| [64] | 2-(3-Chlor-4-(methylsulfonylamino)phenyl)-N-((2-(piperidin-1-yl)-6-(trifluormethyl)pyridin-3-yl)methyl)propanamid |
| [65] | 2-(3-Chlor-4-(methylsulfonylamino)phenyl)-N-((2-(pyrrolidin-1-yl)-6-(trifluormethyl)pyridin-3-yl)methyl)propanamid |
| [66] | 2-(3-Chlor-4-(methylsulfonylamino)phenyl)-N-(2-(piperidin-1-yl)-4-(trifluormethyl)benzyl)propanamid |
| [67] | 2-(3-Brom-4-(methylsulfonylamino)phenyl)-N-((2-(piperidin-1-yl)-6-(trifluormethyl)pyridin-3-yl)methyl)propanamid |
| [68] | 2-(3-Brom-4-(methylsulfonylamino)phenyl)-N-(2-(piperidin-1-yl)-4-(trifluormethyl)benzyl)propanamid |
| [69] | 2-(3-Brom-4-(methylsulfonylamino)phenyl)-N-(2-(pyrrolidin-1-yl)-4-(trifluormethyl)benzyl)propanamid |
| [70] | N-(4-tert-Butyl-2-cyanobenzyl)-2-(3-fluor-4-(methylsulfonylamino)phenyl)propanamid |
| [71] | N-((6-(Chlordiflourmethyl)-2-(piperidin-1-yl)pyridin-3-yl)methyl)-2-(3-fluor-(4-methylsulfonylamino)phenyl)propanamid |
| [72] | (S)-2-(3-Fluor-4-(methylsulfonylamino)phenyl)-N-((2-morpholino-6-(trifluormethyl)pyridin-3-yl)methyl)propanamid |
| [73] | N-((2-(4-Benzylpiperazin-1-yl)-6-(trifluormethyl)pyridin-3-yl)methyl)-2-(3-fluor-4-(methylsulfonylamino)phenyl)propanamid |
| [74] | 2-(3-Fluor-4-(methylsulfonylamino)phenyl)-N-((2-piperazin-1-yl)-6-(trifluormethyl)pyridin-3-yl)methyl)propanamid |
| [75] | N-(2-Chlor-4-(trifluormethyl)benzyl)-2-(3-fluor-4-(methylsulfonylamino)phenyl)propanamid |
| [76] | N-((2-(Cyclohexyloxy)-6-(trifluormethyl)pyridin-3-yl)methyl-2-(3-fluor-4-(methylsulfonylamino)phenyl)propanamid |
| [77] | N-((3-Chlor-5-(trifluormethyl)pyridin-2-yl)methyl-2-(3-fluor-4-(methylsulfonylamino)phenyl)propanamid |
| [78] | 2-(3-Fluor-4-(methylsulfonylamino)phenyl)-N-((3-(pyrrolidin-1-yl)-5-(trifluormethyl)pyridin-2-yl)methyl)propanamid |
| [79] | N-((2-(3,5-Dimethylpiperidin-1-yl)-6-(trifluormethyl)pyridin-3-yl)methyl)-2-(3-fluor-4-(methylsulfonylamino)phenyl)propanamid |
| [80] | 2-(3-Fluor-4-(methylsulfonylamino)phenyl)-N-((2-(4-methylpiperidin-1-yl)-6-(trifluormethyl)pyridin-3-yl)methyl)propanamid |
| [81] | N-((2-(Azepan-1-yl)-6-(trifluormethyl)pyridin-3-yl)methyl)-2-(3-fluor-4-(methylsulfonylamino)phenyl)propanamid |
| [82] | 2-(3-Fluor-4-(methylsulfonylamino)phenyl)-N-(2-(4-methylpiperidin-1-yl)-4-(trifluormethyl)benzyl)propanamid; |

Die Beispielverbindungen 8, 9, 23, 24, 25, 26, 27, 28, 29, 30, 35, 36, 38, 39, 47, 48, 49, 50, 52, 54 55, 56, 59 und 60 können durch die vorstehend beschriebenen Methoden hergestellt werden.

### Beispiel 81: N-(2-Azepan-1-yl-6-trifluoromethyl-pyridin-3-ylmethyl)-2-(3-fluoro-4-methansulfonylamino-phenyl)-propionamid

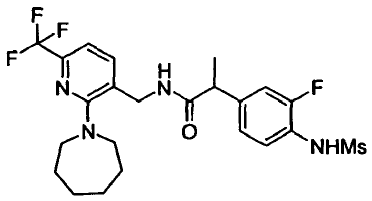

¹H NMR (300 MHz, CDCl₃) δ 7.52 (dd, 1 H, J = 8.1, 8.1 Hz), 7.40 (d, 1 H, J = 7.5 Hz), 7.14 (dd, 1 H, J = 8.1, 1.8 Hz), 7.08 (d, 1 H, J = 8.1 Hz), 7.03 (d, 1 H, J= 7.5 Hz), 5.86 (bt, 1 H), 4.43 (d, 2 H, J = 5.7 Hz), 3.54 (q, 1 H, *J* = 6.9 Hz), 3.38 (m, 4 H), 3.03 (s, 3 H), 1.75 (m, 4 H), 1.57 (m, 4 H), 1.52 (d, 3 H, *J* = 6.9 Hz); IR (KBr) 3291, 2928, 1652, 1593, 1511, 1422, 1333, 1275, 1214, 1159, 972, 822, 759 cm⁻¹; MS (FAB) *m*/*z* 531 (M+H)

### Beispiel 80: 2-(3-Fluoro-4-methansulfonylamino-phenyl)-N-(2-4-methylpiperidin-1-yl-6-trifluoromethyl - pyridin-3-ylmethyl)-propionamid

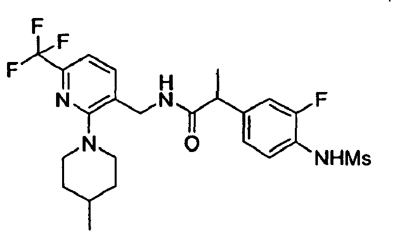

¹H NMR (300 MHz, CDCl₃) δ 7.47-7.55 (m, 2H), 7.07-7.22 (m, 3 H), 6.29 (bt, 1 H), 4.47 (d, 2 H, *J* = 5.7 Hz), 3.54 (q, 1 H, *J* = 6.9 Hz), 3.30 (m, 2 H), 3.03 (s, 3 H), 2.82 (m,2 H), 1.71 (m, 2 H), 1.52 (d, 3 H, *J* = 6.9 Hz), 1.24 (m, 3 H), 0.97 (d, 3 H, J = 6.6 Hz); IR (KBr) 3290, 2924, 1655, 1592, 1512, 1456, 1419, 1334, 1157, 970, 834, 758cm⁻¹; MS (FAB) *m*/*z* 517 (M+H)

### Beispiel 79: 2-(3-Fluoro-4-methansulfonylamino-phenyl)-N-(2- 3, 5 dimethylpiperidin-1-yl-6-trifluoromethyl - pyridin-3-ylmethyl)-propionamid

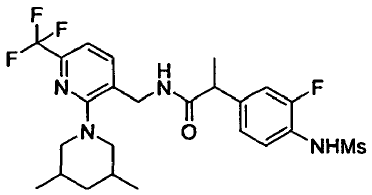

¹H NMR (300 MHz, CDCl₃) δ 7.47-7.54 (m, 2 H),. 7.21 (d, 1 H, J = 7.8 Hz), 7.13 (dd, 1 H, J = 8.1, 1.8 Hz), 7.07 (d, 1 H, J = 8.1 Hz), 6.48 (bs, 1 H), 6.28 (bt, 1 H), 4.47 (d, 2 H, *J* = 5.7 Hz), 3.54 (q, 1 H, *J* = 6.9 Hz), 3.23 (m, 2 H), 3.03 (s, 3 H), 2.35 (m, 2 H), 1.54-1.76 (m, 2 H), 1.52 (d, 3 H, *J* = 6.9 Hz), 0.90 (d, 3 H, J = 5.7 Hz), 0.88 (d, 3 H, J = 5.7 Hz); IR (KBr) 3289, 2957, 1655, 1591, 1512, 1458, 1419, 1336, 1247, 1158, 1013, 970, 831, 757cm⁻¹; MS (FAB) *m*/*z* 531 (M+H)

Die nachfolgenden Verbindungen wurden ebenfalls nach dem oben beschriebenen Verfahren erhalten.

### Beispiel 85: N-(2-Dimethylamino-6-trifluoromethyl-pyridin-3-ylmethyl)-2-(3-fluoro-4-methansulfonylamino-phenyl)-propionamid

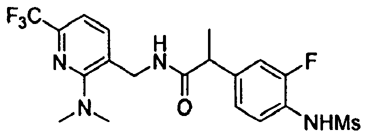

¹H NMR (300 MHz, CDCl₃) δ 7.46-7.40 (m , 2 H), 7.20-7.00 (m, 3 H), 6.60 (bt, 1 H), 4.50 (bd, 2 H), 3.60 (m, 1 H), 3.00 (s, 3 H), 2.80 (s, 6 H), 1.49 (d, 3 H, *J* = 7.0 Hz); IR (KBr) 3284, 2936, 1656, 1594, 1511, 1395, 1335 cm⁻¹; MS (FAB) *m*/*z* 463 (M+H)

### Beispiel 87: 2-(3-Fluoro-4-methansulfonylamino-phenyl)-N-((6-(trifluoromethyl)-2-(1H-imidazol-1-yl)pyridin-3-yl)methyl)-propionamid

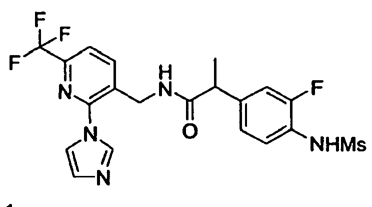

¹H NMR (300 MHz, CDCl₃) δ 7.98 (d, 1 H, *J* = 8.4 Hz), 7.82 (s, 1 H), 7.70 (d, 1 H, J = 8.4 Hz), 7.49 (dd, 1 H, J = 8.1, 8.1 Hz), 7.30 (s, 1 H), 7.18 (s, 1 H), 7.04-7.08 (m, 2 H), 6.15 (bt, 1 H), 4.45 (d , 2 H, *J* = 5.1 Hz), 3.53 (q, 1 H, *J* = 6.9 Hz), 3.04 (s, 3 H), 1.47 (d, 3 H, J = 6.9 Hz); IR (KBr) 2923, 1665, 1511, 1424, 1337, 1154, 973, 760 cm⁻¹; MS (FAB) *m*/*z* 487 (M+H)

### Beispiel 88: 2-(3-Fluoro-4-methansulfonylamino-phenyl)-N-((6-(trifluoromethyl)-2-(thiophen-2-yl)pyridin-3-yl)methyl)-propionamid

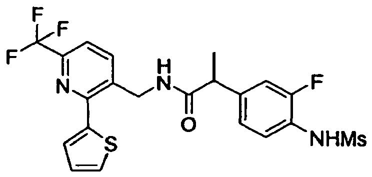

¹H NMR (300 MHz, CDCl₃) δ 7.74 (d, 1 H, J = 8.7 Hz), 7.47-7.5 (m, 3 H), 7.35 (dd, 1 H, 3.6, 1.2 Hz), 7.02-7.11 (m, 3 H), 6.51 (bs, 1 H), 5.79 (bt, 1 H), 4.70 (d, 2 H, *J* = 5.1 Hz), 3.53 (q, 1 H, *J* = 6.9 Hz), 3.02 (s, 3H ), 1.51 (d, 3 H, J = 6.9 Hz); IR (KBr) 2920, 1737, 1644, 1509, 1428, 1328, 1148, 979, 768 cm⁻¹, MS (FAB) *m*/*z* 502 (M+H)

### Beispiel 89: 2-(3-Fluoro-4-methansulfonylamino-phenyl)-N-((2-(trifluoromethyl)-6-phenylpyridin-4-yl)methyl)-propionamid

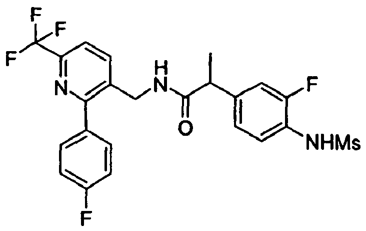

¹H NMR (300 MHz, CDCl₃) δ 7.77 (d, 1 H, J = 8.1 Hz), 7.61 (d, 1 H, J= 8.1 Hz), 7.50 (dd, 1 H, J = 8.1, 8.1 Hz), 7.40-7.45 (m, 2 H), 6.99-7.16 (m, 4 H), 6.57 (bs, 1 H), 5.63 (bt, 1 H), 4.49 (d, 2 H, *J* = 5.7 Hz), 3.47 (q, 1 H, *J* = 6.9 Hz), 3.02 (s, 3 H), 1.49 (d, 3 H, J = 6.9 Hz); IR (KBr) 3296, 1657, 1513, 1457, 1411, 1340, 1156, 1048, 973, 842, 757 cm⁻¹; MS (FAB) *m*/*z* 514 (M+H)

### Beispiel 90: N-(2-Cyclohexylamino-6-trifluoromethyl-pyridin-3-ylmethyl)-2-(3-fluoro-4-methansulfonylamino-phenyl)-propionamid

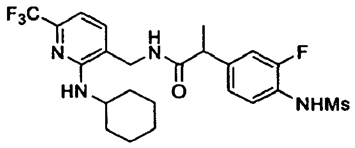

¹H NMR (CDCl₃) δ 7.51 (dd, 1 H, J = 6.6, 6.6 Hz), 7.22 (d, 1 H, J = 5.8 Hz), 7.18 (dd, 1 H, J = 8.9, 1.5 Hz), 7.08 (d, 2 H, J = 6.6 Hz), 6.74 (d, 1 H, J = 5.8 Hz), 6.47 (bs, NH), 5.84 (bd, NH), 5.67 (bt, NH), 4.32 (m, 2 H), 3.91 (m, 1 H), 3.48 (q, 1 H, J = 5.7 ' Hz), 3.03 (s, 3 H), 1.98-1.61 (m, 5 H), 1.52 (d, 3 H, J = 5.7 Hz), 1.42-1.07 (m, 5 H); IR (pur) 3337, 2930, 2854, 1647, 1514, 1453, 1334, 1159, 909 cm⁻¹; MS (FAB) m/z 517 (M+H)

### Beispiel 91: 2-(3-Fluoro-4-methansulfonylamino-phenyl)-N-(2-hexyloxy-6-trifluoromethyl-pyridin-3-ylmethyl)-propionamid

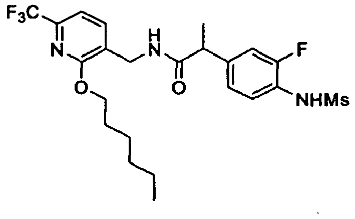

¹H NMR (CDCl₃) δ 7.57 (d, 1 H, J = 7.1 Hz), 7.52 (dd, 1 H, J = 8.3, 8.3 Hz), 7.19 (d, 1 H, J = 7.3 Hz), 7.12-7.05 (m, 2 H), 6.48 (bs, NH), 5.99 (bt, NH), 4.38-4.29 (m, 4 H), 3.51 (q, 1 H, J = 7.1 Hz), 3.03 (s, 3 H), 1.75-1.68 (m, 2 H), 1.49 (d, 3 H, J = 7.1 Hz), 1.38-1.30 (m, 6 H), 0.91 (t, 3 H); IR (pur) 3292, 2930, 1654, 1514, 1463, 1425, 1338, 1269, 1155, 973 cm⁻¹; MS (FAB) m/z 520 (M+H)

### Beispiel 95: 2-(3-Fluoro-4-methansulfonylamino-phenyl)-N-(2-isobutoxy-6-trifluoromethyl-pyridin-3-ylmethyl)-propionamid

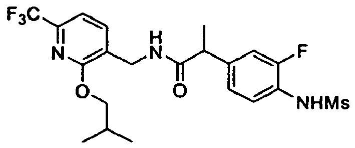

¹H NMR (CDCl₃) δ 7.58 (d, 1 H, J = 7.3 Hz), 7.51 (dd, 1 H, J = 8.4, 8.4 Hz), 7.19 (d, 1 H, J = 7.5 Hz), 7.12-7.05 (m, 2 H), 6.50 (bs, NH), 5.95 (bt, NH), 4.41-4.37 (m, 2 H), 4.17-4.06 (m, 2 H), 3.51 (q, 1 H, J = 7.1 Hz), 3.03 (s, 3 H), 2.05 (m, 1 H), 1.49 (d, 3 H, J = 7.1 Hz), 0.99 (d, 6 H, J = 6.8Hz); IR (pur) 3295, 2966, 1655, 1514, 1463, 1425, 1336, 1157 cm⁻¹; MS (FAB) m/z 492 (M+H)

### Beispiel 100: N-(2-Cyclopropylmethoxy-6-trifluoromethyl-pyridin-3-ylmethyl)-2-(3-fluoro-4-methansulfonylamino-phenyl)-propionamid

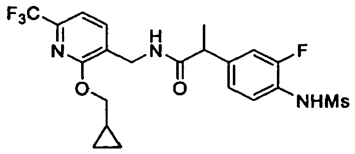

¹H NMR (CDCl₃) δ 7.59 (d, 1 H, J = 7.3 Hz), 7.51 (dd, 1 H, J = 8.3, 8.3 Hz), 7.19 (d, 1 H, J = 7.5 Hz), 7.13-7.06 (m, 2 H), 6.49 (bs, NH), 6.08 (bt, NH), 4.42-4.39 (m, 2 H), 4.24-4.11 (m, 2 H), 3.52 (q, 1 H, J = 7.1 Hz), 3.03 (s, 3 H), 1.59 (d, 3 H, J = 7.0 Hz), 1.25-1.15 (m, 1 H), 0.62-0.56 (m, 2 H), 0.36-0.33 (m, 2 H); IR (pur) 3288, 1655, 1513, 1427, 1376, 1335, 1158, 984 cm⁻¹; MS (FAB) m/z 490 (M+H)

### Beispiel 101: N-(2-Cyclobutylmethoxy-6-trifluoromethyl-pyridin-3-ylmethyl)-2-(3-fluoro-4-methansulfonylamino-phenyl)-propionamid

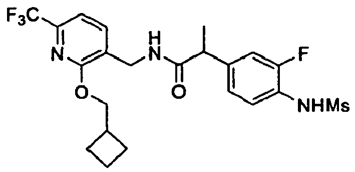

¹H NMR (CDCl₃) δ 7.57 (d, 1 H, J = 7.5 Hz), 7.50 (dd, 1 H, J = 8.2, 8.2 Hz), 7.19 (d, 1 H, J = 7.3 Hz), 7.12-7.04 (m, 2 H), 6.64 (bs, NH), 6.02 (bt, NH), 4.45-4.26 (m, 4 H), 3.51 (q, 1 H, J = 7.1 Hz), 3.03 (s, 3 H), 2.71 (m, 1 H), 2.13-1.79 (m, 6 H), 1.48 (d, 3 H, J = 7.1 Hz); IR (pur) 3289, 2940, 1656, 1513, 1424, 1335, 1157, 993 cm⁻¹; MS (FAB) m/z 504 (M+H)

### Beispiel 102: 2-(3-Chloro-4-methansulfonylamino-phenyl)-N-(2-pyrrolidin-1-yl-6 trifluoromethyl-pyridin-3-ylmethyl)-propionamid

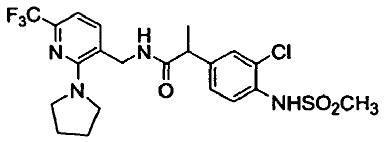

¹H NMR (300 MHz, CDCl₃) δ 7.60 (d, 1 H, *J* = 8.4 Hz), 7.41 (s, 1 H) 7.39 (d, 1 H, *J* = 7.9 Hz), 7.22 (d, 1 H, *J* = 8.4 Hz), 6.95 (d, 1 H, *J* = 7.5 Hz), 6.79 (bs, 1 H), 5.74 (bt, 1 H), 4.47 (d, 2 H, *J* = 5.1 Hz), 3.52 (q, 1 H, *J* = 7.1 Hz), 3.48-3.41 (m, 4 H), 3.01 (s, 3 H), 1.89-1.82 (m, 4 H), 1.51 (d , 3 H, *J* = 7.0 Hz); IR (pur) 3291, 2974, 1651, 1598, 1497, 1431, 1333, 1159, 971, 913, 733cm⁻¹; MS (FAB) *m*/*z* 505 (M+H)

### Beispiel 103: 2-(3-Bromo-4-methansulfonylamino-phenyl)-N-(2-pyrrolidin-1-yl-6-trifluoromethyl-pyridin-3-ylmethyl)-propionamid

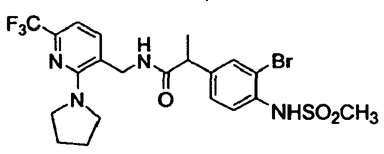

¹H NMR (300 MHz, CDCl₃) δ 7.60 (d, 1 H, *J* = 8.4 Hz), 7.56 (d, 1 H, *J* = 2.0 Hz), 7.39 (d, 1 H, *J* = 7.5 Hz), 7.27 (dd, 1 H, *J* = 8.3. 2.2 Hz), 6.95 (d, 1 H, *J* = 7.5 Hz), 6.77 (bs, 1 H), 5.77 (bt, 1 H), 4.47 (d, 2 H, *J* = 5.1 Hz), 3.51 (q, 1 H, *J* = 7.1 Hz), 3.47-3.41 (m, 4 H), 3.01 (s, 3 H), 1.89-1.83 (m, 4 H), 1.51 (d, 3 H, *J* = 7.1 Hz); IR (pur) 3294, 2973, 1651, 1598, 1494, 1431, 1333, 1159, 971, 912, 733 cm⁻¹; MS (FAB) *m*/*z* 549 (M+H)

### Beispiel 104: N-(4-Benzyl-6'-trifluoromethyl-3,4,5,6-tetrahydro-2H-[1,2']bipyridinyl-3'-ylmethyl)-2-(3-fluoro-4-methansulfonylamino-phenyl)-propionamid

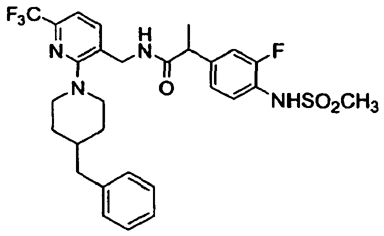

¹H NMR (300 MHz, CDCl₃) δ 7.53 (dd, 1 H, *J* = 8.2, 8.2 Hz), 7.48 (d, 1 H, *J* = 7.9 Hz), 7.29-7.14 (m, 7 H), 7.07 (d, 1 H, *J* = 8.1 Hz), 6.49 (bs, 1 H), 6.23 (bt, 1 H), 4.46 (d, 2 H, *J* = 5.7 Hz), 3.54 (q, 1 H, *J* = 7.0 Hz), 3.31 (m, 2 H), 3.02 (s, 3 H), 2.78 (m, 2 H), 2.59 (d, 2 H, *J* = 6.6 Hz), 1.78-1.71 (m, 3 H), 1.52 (d , 3 H, *J* = 7.1 Hz), 1.30 (m, 2 H); IR (pur) 3292, 2923, 1655, 1592, 1512, 1420, 1335, 1158, 968, 939, 734 cm⁻¹; MS (FAB) *m*/*z* 593 (M+H)

### Beispiel 106: N-(2-Benzyloxy-6-trifluoromethyl-pyridin-3-ylmethyl)-2-(3-fluoro-4-methansulfonylamino-phenyl)-propionamid

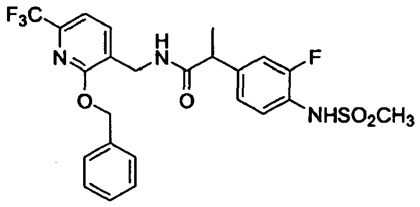

¹H NMR (300 MHz, CDCl₃) δ 7.62 (d, 1 H, *J* = 7.1 Hz), 7.47 (dd, 1 H, *J* = 8.4, 8.4 Hz), 7.44-7.36 (m, 5 H), 7.24 (d, 1 H, *J* = 7.5 Hz), 7.04 (dd, 1 H, *J* =11.2, 1.8 Hz), 6.97 (d, 1 H, *J* = 8.4 Hz), 6.42 (bs, 1 H), 5.96 (bt, 1 H), 5.41 (m, 2 H), 4.39 (m, 2 H), 3.41 (q, 1 H, *J* = 7.1 Hz), 3.01 (s, 3 H), 1.42 (d , 3 H, *J* = 7.1 Hz); IR (pur) 3295, 1655, 1512, 1419, 1353, 1267, 1156, 977, 907, 737 cm⁻¹; MS (FAB) *m*/*z* 526 (M+H)

### Beispiel 107: 2-(3-Fluoro-4-methansulfonylamino-phenyl)-N-[2-(3-methoxy-benzyloxy)-6-trifluoromethyl-pyridin-3-ylmethyl]-propionamid

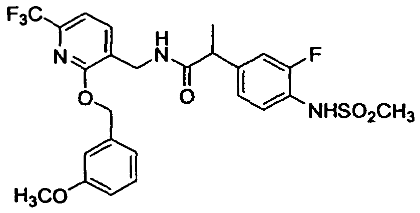

¹H NMR (300 MHz, CDCl₃) δ 7.62 (d, 1 H, *J* = 7.7 Hz), 7.46 (dd, 1 H, *J* = 8.3, 8.3 Hz), 7.31 (dd, 1 H, *J* = 8.1 Hz), 7.23 (d, 1 H, *J* = 7.4 Hz), 7.06-6.88 (m, 4 H), 6.90 (m, 1 H), 6.49 (bs, 1 H), 5.99 (bt, 1 H), 5.39 (m, 2 H), 4.39 (m, 2 H), 3.83 (s, 3H), 3.42 (q, 1 H, *J* = 7.1 Hz), 3.01 (s, 3 H), 1.42 (d , 3 H, *J* = 7.1 Hz); IR (pur) 3294, 1656, 1600, 1512, 1417, 1349, 1267, 1157, 976, 910, 735 cm⁻¹; MS (FAB) *m*/*z* 556 (M+H)

### Beispiel 108: N-(2-Butoxy-4-tert-butyl-benzyl)-2-(3-fluoro-4-methansulfonylamino-phenyl)-propionamid

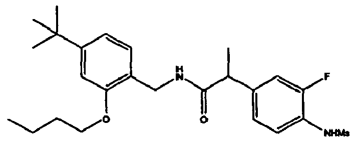

¹H NMR (300 MHz, CDCl₃) δ 7.48 (t, 1 H, *J* = 8.2 Hz), 7.16-7.04 (m, 3 H), 6.92-6.85 (m, 2 H), 6.59 (bs, 1 H), 5.98 (bt, 1 H), 4.45-4.29 (m, 2 H), 3.98-3.90 (m, 2 H), 3.46 (q, 1 H, *J* = 6.9 Hz), 3.01 (s, 3 H), 1.75-1.65 (m, 2 H), 1.48 (d, 3 H, *J* = 7.1 Hz), 1.30 (s, 9 H), 0.97 (t, 3 H, *J* = 7.3 Hz); IR (KBr) 3289, 2961, 1650, 1510, 1413, 1334 cm⁻¹; MS (FAB) *m*/*z* 479 (M+H)

### Beispiel 109: 2-(3-Fluoro-4-methansulfonylamino-phenyl)-N-[2-(4-phenyl-piperazin-1-yl)-6-trifluoromethyl-pyridin-3-ylmethyl]-propionamid

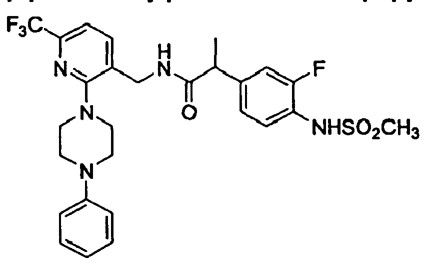

¹H NMR (300 MHz, CDCl₃) δ 7.54 (d, 1 H, *J* = 7.7Hz), 7.48 (dd, 1 H, *J* = 8.2, 8.2 Hz), 7.31 (m, 3 H), 7.13 (dd, 1 H, *J* = 11.0, 1.8 Hz), 7.08 (d, 1 H, *J* = 8.8 Hz), 6.96-6.89 (m, 3 H), 6.33 (bs, 1 H), 6.20 (bt, 1 H), 4.54 (d, 2 H, *J* = 6.0 Hz), 3.57 (q, 1 H, *J* = 7.0 Hz), 3.32-3.29 (m, 8 H), 2.99 (s, 3 H), 1.53 (d, 3 H, *J* = 7.1 Hz); IR (pur) 3292, 1658, 1594, 1508, 1418, 1374, 1335, 1231, 1155, 968, 909, 834, 758, 694 cm⁻¹; MS (FAB) *m*/*z* 580 (M+H)

### Beispiel 110: 2-(3-Fluoro-4-methansulfonylamino-phenyl)-N-(2-phenylamino-6-trifluoromethyl-pyridin-3-ylmethyl)-propionamid

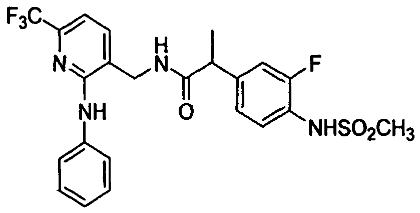

¹H NMR (300 MHz, CD₃OD) δ 7.67 (m, 2 H), 7.59 (d, 1 H, *J* = 8.2 Hz), 7.34 (dd, 1 H, *J* = 8.2, 8.2 Hz), 7.19 (dd, 1 H, *J* = 10.9, 1.9 Hz), 7.11 (d, 1 H, *J* = 8.4 Hz), 7.06 (d, 1 H, *J* = 7.7 Hz), 7.02-6.95 (m, 3 H), 4.45 (m, 2 H), 3.67 (q, 1 H, *J* =7.1 Hz), 2.89 (s, 3 H), 1.47 (d, 3 H, *J* = 7.1 Hz); IR (pur) 3306, 2926, 1706, 1645, 1509, 1428, 1328, 1156, 968, 833 cm⁻¹; MS (FAB) *m*/*z* 511 (M+H)

### Beispiel 111: 2-(3-Fluoro-4-methansulfonylamino-phenyl)-N-(2-propoxy-6-trifluoromethyl-pyridin-3-ylmethyl)-propionamid

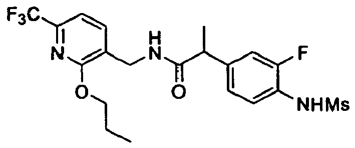

¹H NMR (CDCl₃) δ 7.58 (d, 1 H, J = 7.5 Hz), 7.52 (dd, 1 H, J = 8.2, 8.2 Hz), 7.19 (d, 1 H, J = 7.3 Hz), 7.12-7.05 (m, 2 H), 6.50 (bs, NH), 5.97 (bt, NH), 4.39-4.23 (m, 4 H), 3.52 (q, 1 H, J = 7.1 Hz), 3.03 (s, 3 H), 1.74 (m, 2 H), 0.99 (t, 3 H, J = 7.3 Hz); IR (pur) 3287, 2972, 1655, 1513, 1426, 1336, 1256, 976 cm⁻¹; MS (FAB) m/z 478 (M+H)

### Beispiel 112: 2-(3-Fluoro-4-methansulfonylamino-phenyl)-N-[2-(4-fluoro-phenylamino)-6-trifluoromethyl-pyridin-3-ylmethyl]-propionamid

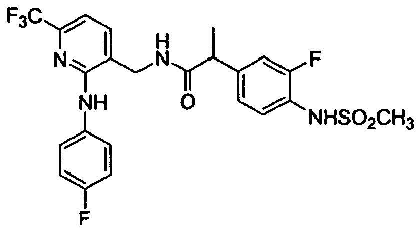

¹H NMR (300 MHz, CD₃OD) δ 7.90 (m, 2 H), 7.59 (d, 1 H, *J* = 7.5 Hz), 7.33 (dd, 1 H, *J* = 8.3, 8.3 Hz), 7.24 (m, 1 H), 7.21 (dd, 1 H, *J* = 11.4, 1.8 Hz), 7.11 (d, 1 H, *J* = 8.4 Hz), 7.06 (d, 1 H, *J* = 7.5 Hz), 6.59 (m, 1 H), 4.46 (m, 2 H), 3.68 (q, 1 H, *J* =7.1 Hz), 2.87 (s, 3 H), 1.47 (d , 3 H, *J* = 7.1 Hz); IR (pur) 3267, 2928, 1707, 1644, 1593, 1502, 1433, 1329, 1157, 969, 817, 755, 694 cm⁻¹; MS (FAB) *m*/*z* 529 (M+H)

### Beispiel 113: N-[2-(4-Chloro-phenylamino)-6-trifluoromethyl-pyridin-3-ylmethyl]-2-(3-fluoro-4-methansulfonylamino-phenyl)-propionamid

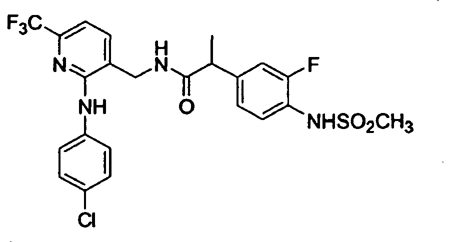

¹H NMR (300 MHz, CD₃OD) δ 7.10 (d, 2 H, *J* = 9.0 Hz,) 7.62 (d, 1 H, *J* = 7.5 Hz), 7.34 (dd, 1 H, *J* = 8.3, 8.3 Hz), 7.23 (d, 2 H, *J* = 9.0 Hz), 7.19 (dd, 1 H, *J* = 11.7, 2.0 Hz), 7.10 (d, 1 H, *J* = 8.3 Hz), 7.09 (d, 1 H, *J* = 7.5 Hz), 4.45 (m, 2 H), 3.67 (q, 1 H, *J* = 7.0 Hz), 2.88 (s, 3 H), 1.47 (d, 3 H, *J* = 7.1 Hz); IR (pur) 2922, 1645, 1496, 1466, 1334, 1151, 971, 819 cm⁻¹; MS (FAB) *m*/*z* 545 (M+H)

### Beispiel 114: 2-(3-Fluoro-4-methansulfonylamino-phenyl)-N-(2-fluoro-4-trifluoromethyl-benzyl)-propionamid

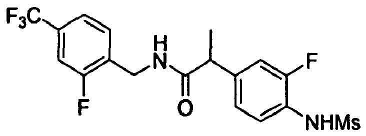

¹H NMR (300 MHz, CDCl₃) δ 7.50 (dd ,1 H, *J* = 8.0, 8.0 Hz), 7.41-7.26 (m, 3 H), 7.13 (dd, 1 H, *J* = 11.0, 2.0 Hz), 7.07 (bd, 1 H), 6.60 (bs, 1 H), 6.00 (bt, 1 H), 4.48 (m, 2 H), 3.03 (s, 3 H), 1.49 (d, 3 H *J* = 7.1 Hz); IR (KBr) 3288, 1657, 1588, 1512, 1430, 1332, 1220 cm⁻¹; MS (FAB) *m*/*z* 437 (M+H)

### Beispiel 115: N-(2-Benzylamino-6-trifluoromethyl-pyridin-3-ylmethyl)-2-(3-fluoro-4-methansulfonylamino-phenyl)-propionamid

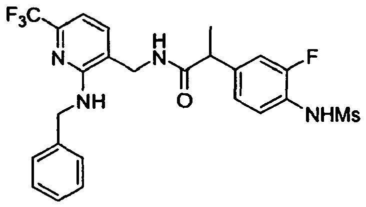

¹H NMR (300 MHz, CD₃OD) δ 7.42-7.14 (m, 8 H), 6.82 (d, 1 H, *J* = 7.2 Hz), 6.69 (bt, 1 H), 4.68-4.44 (m, 2 H), 4.25 (m, 2 H), 3.62 (q, 1 H, *J* = 7.1 Hz), 2.94 (s, 3 H), 1.37 (d, 3 H, *J* = 7.3 Hz); IR (KBr) 3269, 2928, 2493, 1706, 1644, 1513, 1452 cm⁻¹; MS (FAB) *m*/*z* 525 (M+H)

### Beispiel 117: 2-(3-Fluoro-4-methansulfonylamino-phenyl)-N-((2-(4-tert-butylphenyl)-6-(trifluoromethyl)pyridin-3-ylmethyl)-propionamid

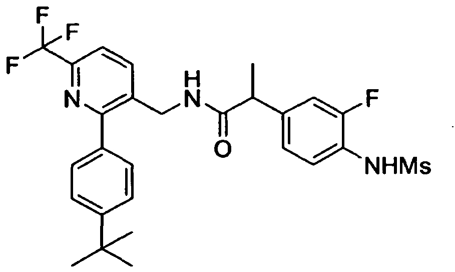

¹H NMR (300 MHz, CD₃OD) δ 7.77 (d, 1 H, J = 8.1 Hz), 7.66 (d, 1 H, J = 8.1 Hz), 7.50 (d, 2 H, J = 6.6 Hz), 7.40-7.45 (m, 3 H), 7.06-7.19 (m, 3 H), 4.40 (s, 2 H), 3.65 (q, 1 H, J = 6.6 Hz), 2.96 (s, 3 H), 1.40 (d, 3 H, J = 6.6 Hz), 1.35 (s, 9 H); IR (KBr) 2927, 2856, 1619, 1511, 1455, 1339, 1274, 1158 cm⁻¹; MS (FAB) *m*/*z* 552 (M+H)

### Beispiel 118: 2-(3-Fluoro-4-methansulfonylamino-phenyl)-(N(2-(3-chloro-4-fluorophenyl)-6-(trifluoromethyl))pyridin-3-ylmethyl)-propionamid

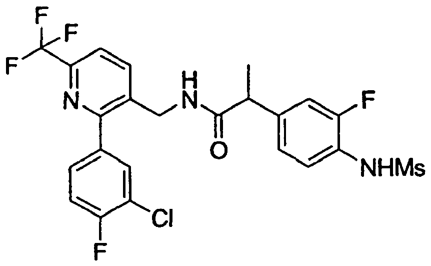

¹H NMR (300 MHz, CD₃OD) δ 7.75 (d, 1 H, J = 7.5 Hz), 7.63 (d, 1 H, J = 7.5 Hz), 7.56 (dd, 1 H, J = 2.1 Hz, 6.9 Hz), 7.34-7.49 (m, 3 H), 7.23 (t, 1 H, J = 7.5 Hz), 7.05-7.14 (m, 2 H), 4.41(s, 2 H), 3.56 (q, 1 H, J = 6.9 Hz), 2.98 (s, 3 H), 1.40 (d, 3 H, J = 6.9 Hz); IR (KBr) 2919, 1651, 1508, 1409, 1338, 1150, 971, 829 cm⁻¹; MS (FAB) *m*/*z* 548 (M+H)

### Beispiel 122: 2-(3-Fluoro-4-methansulfonylamino-phenyl)-(N(2-(butylthio)-6-(trifluoromethyl)pyridin-3-ylmethyl)-propionamid

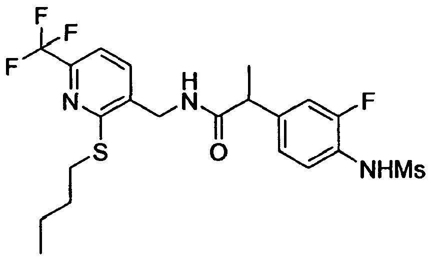

¹H NMR (300 MHz, CDCl₃) δ 7.42-7.50 (m, 2 H), 7.27 (s, 1 H), 7.13 (dd, 1 H, J = 8.1, 1.8 Hz), 7.07 (d, 1 H, J = 8.1 Hz), 6.98 (bs 1 H), 6.33 (bt, 1 H), 4.36 (m, 2 H), 3.56 (q, 1 H, J = 6.9 Hz), 3.22 (t, 2 H, J = 7.5 Hz), 3.01 (s, 3 H), 1.66 (m, 2 H), 1.38-1.50 (m, 5 H), 0.93 (t, 3 H, J= 7.2 Hz); IR (KBr) 3291, 2930, 2856, 1707, 1587, 1513, 1337, 1272, 1154, 1108, 898, 815 cm⁻¹; MS (FAB) *m*/*z* 508 (M+H)

### Beispiel 124: 2-(3-Fluoro-4-methansulfonylamino-phenyl)-N-[2-(2-methyl-cyclopropyl methoxy)-6-trifluoromethyl-pyridin-3-ylmethyl]-propionamid

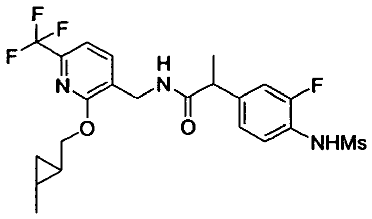

¹H NMR (300 MHz, CD₃OD) δ 7.48 (d, 1 H, J = 7.5 Hz), 7.43 (dd, 1 H, *J* = 8.1, 8.1 Hz ), 7.15-7.23 (m, 3 H), 4.34 (d, 2 H, J = 5.1 Hz), 4.20 (d, 2 H, J = 7.1 Hz), 3.73 (q, 1 H, J = 6.9 Hz), 2.98 (s, 3 H), 1.46 (d, 3 H, J = 7.1 Hz), 1.04 (d, 3 H, J = 6.0 Hz), 0.95 (m, 1 H), 0.78 (m, 1 H), 0.51 (m, 1 H), 0.31 (m, 1 H); IR (KBr) 3280, 2928, 1654, 1512, 1450, 1427, 1339, 1267, 1158, 980 cm⁻¹; MS (FAB) *m*/*z* 504 (M+H)

### Beispiel 125: 2-(3-Fluoro-4-methansulfonylamino-phenyl)-(N(2-(3,3-dimethylbutoxy)-6-(trifluoromethyl)pyridin-3-ylmethyl)-propionamid

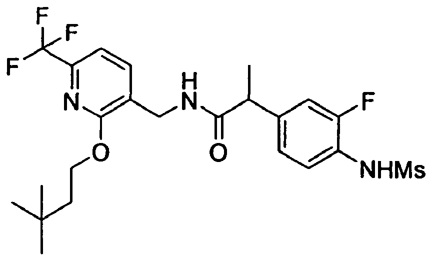

¹H NMR (300 MHz, CD₃OD) δ 7.40-7.48 (m, 2 H), 7.13-7.22(m, 3 H), 4.42 (t, 2 H, J = 7.5 Hz), 4.31 (d, 2 H, J = 7.2 Hz), 3.72 (q, 1 H, J = 6.9 Hz), 2.97 (s, 3 H), 1.68 (t, 2 H, J = 7.2 Hz), 1.45 (d, 3 H, J = 6.9 Hz), 0.97 (s, 9 H); IR (KBr) 3352, 3077, 2950, 1655, 1545, 1510, 1427, 1366, 1331, 1150 cm⁻¹; MS (FAB) *m*/*z* 520 (M+H)

### Beispiel 126: 2-(3-Fluoro-4-methansulfonylamino-phenyl)-(N-(2-(cyclohexylthio)-6-(trifluoromethyl)pyridin-3-ylmethyl)-propionamid

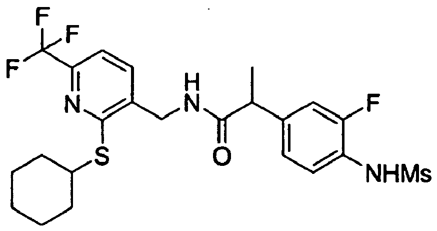

¹H NMR (300 MHz, CD₃OD) δ 7.35-7.48 (m, 3 H), 7.26 (d, 1 H, J = 7.8 Hz), 7.16 (dd, 1 H, J = 1.8, 11.1 Hz), 7.10 (d, 1 H, J = 8.4 Hz), 6.13 (bs, 1 H), 4.35 (d, 2 H, J = 5.7 Hz), 3.82 (m, 1 H), 3.56 (q, 1 H, J = 7.2 Hz), 3.02 (s, 3 H), 2.06 (m, 2 H), 1.75 (m, 2 H), 1.49 (d, 3 H, J = 7.2 Hz), 1.26-1.33 (m, 6 H); IR (KBr) 3284, 2932, 2854, 1654, 1586, 1512, 1449, 1336, 1267 cm⁻¹; MS (FAB) *m*/*z* 534 (M+H)

### Beispiel 127: 2-(4-Methansulfonylamino-3-methyl-phenyl)-N-(6'-trifluoromethyl-3,4,5,6-tetrahydro-2H-[1,2']bipyridinyl-3'-ylmethyl)-propionamid

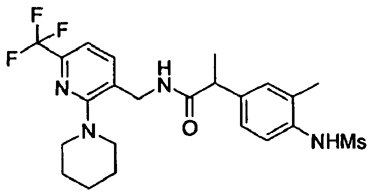

¹H NMR (300 MHz, CDCl₃) δ 7.46 (d, 1 H, J = 8.1 Hz), 7.39 (d, 1 H, J = 8.1 Hz), 7.11-7.20 (m, 3 H), 6.33 (s, 1 H), 6.25 (bs, 1 H), 4.46 (d, 2 H, J = 5.7 Hz), 3.56 (q, 1 H, J = 7.5 Hz), 2.96-3.02 (m, 7 H), 2.38 (s, 3 H), 1.54-1.63 (m, 6 H), 1.52 (d, 3 H, J = 7.5 Hz); IR (KBr) 3288, 2928, 2853, 1652, 1538, 1457, 1246, 970 cm⁻¹; MS (FAB) *m*/*z* 499 (M+H)

### Beispiel 128: N-(2-Azocan-1-yl-6-trifluoromethyl-pyridin-3-ylmethyl)-2-(3-fluoro-4-methansulfonylamino-phenyl)-propionamid

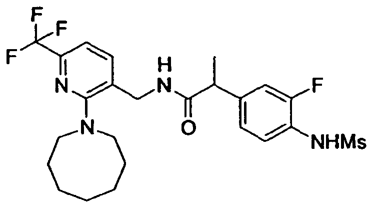

¹H NMR (300 MHz, CDCl₃) δ 7.48 (t, 1 H, J = 8.1 Hz), 7.36 (d, 1 H, J = 7.8 Hz), 7.14 (dd, 1 H, J = 2.1, 11.1 Hz), 7.07 (d, 1 H, J = 8.1 Hz), 6.96 (d, 1 H, J = 7.8 Hz), 6.94 (bs, 1 H), 5.97 (bs, 1 H), 4.39 (d, 2 H, J = 5.1 Hz), 3.59 (q, 1 H, J = 7.2 Hz), 3.46 (m, 4 H), 3.01 (s, 3 H), 1.68 (m, 4 H), 1.51 (m, 6 H); IR (KBr) 3275, 2926, 1652, 1594, 1509, 1454, 1421, 1334 cm⁻¹; MS (FAB) *m*/*z* 531 (M+H)

### Beispiel 129: 2-(3-Fluoro-4-methansulfonylamino-phenyl)-N-(2-pyrrolidin-1-yl-6-trifluoromethyl-pyridin-3-ylmethyl)-thiopropionamid

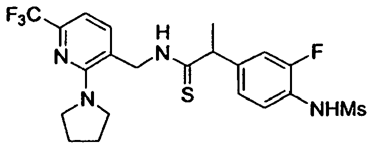

¹H NMR (300 MHz, CDCl₃) δ 7.97 (bs, 1 H), 7.50 (dd, 1 H, J = 8.1, 8.1 Hz), 7.08-7.29 (m, 4 H), 6.54 (bs, 1 H), 4.85 (d, 2 H, J = 5.7 Hz), 4.01 (q, 1 H, J = 6.9 Hz), 3.09 (m, 4 H), 3.01 (s, 3 H), 1.87 (m, 4 H), 1.62 (d, 3 H, J = 6.9 Hz); IR (KBr)) 3296, 2923, 1509, 1428, 1334, 1159, 1121, 978, 907, 733 cm⁻¹; MS(FAB) *m*/*z* 505 (M+H)

### Beispiel 130: 2-(3-Fluoro-4-methansulfonylamino-phenyl)-N-[2-(4-fluoro-phenyl)-6-trifluoromethyl-pyridin-3-ylmethyl]-thiopropionamid

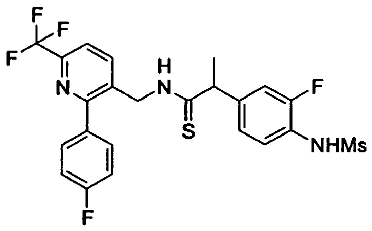

¹H NMR (300 MHz, CDCl₃) δ 7.76 (d, 1 H, J = 8.1 Hz), 7.60 (d, 1 H, J = 8.1 Hz), 7.42-7.47 (m, 3 H), 7.00-7.19 (m, 5 H), 6.54 (bs, 1 H), 4.95 (d, 2 H, J = 5.7 Hz), 3.93 (q, 1 H, J = 6.9 Hz), 3.03 (s, 3 H), 1.59 (d, 3 H, J = 6.9 Hz); IR (KBr)) 3300, 1512, 1409, 1340, 1155, 1047 cm⁻¹; MS (FAB) *m*/*z* 530 (M+H)

### Beispiel 131: 2-(3-Fluoro-4-methansulfonylamino-phenyl)-N-(4-methylpiperidin-1-yl-6-chlroro difluoromethyl - pyridin-3-ylmethyl)-propionamid

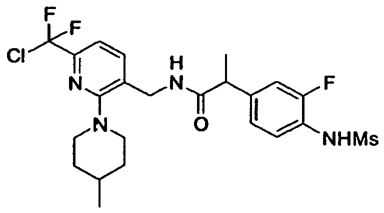

¹H NMR (300 MHz, CDCl₃) δ 7.45-7.53 (m, 2 H), 7.07-7.18 (m, 3 H), 6.72 (bs, 1 H), 6.37 (bt, 1 H), 4.46 (d, 2 H, J = 5.7 Hz), 3.56 (q, 1 H, J = 6.9 Hz), 3.32 (m, 2 H), 3.02 (s, 3 H), 2.82 (m, 2 H), 1.71 (m, 2 H), 1.53 (d, 3 H, J = 7.5 Hz), 1.23 (m, 3 H), 0.97 (d, 3 H, J = 6.9 Hz); IR (KBr) 2924, 1653, 1590, 1512, 1453, 1334, 1157 cm⁻¹; MS (FAB) *m*/*z* 534 (M+H)

### Beispiel 132: N-[2-Azepan-1-yl-6-(chloro-difluoro-methyl)-pyridin-3-ylmethyl]-2-(3-fluoro-4-methansulfonylamino-phenyl)-propionamid

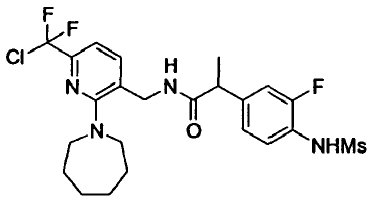

¹H NMR (300 MHz, CDCl₃) δ 7.52 (dd, 1 H, J = 8.1, 8.1 Hz), 7.38 (d, 1 H, J = 7.5 Hz), 7.17 (dd, 1 H, J = 1.8, 11.1 Hz), 7.08 (d, 1 H, J = 8.1 Hz), 6.99 (d, 1 H, J = 7.5 Hz), 6.57 (bs, 1 H), 5.87 (bt, 1 H), 4.42 (d, 2 H, J = 5.7 Hz), 3.56 (q, 1 H, J = 6.9 Hz), 3.39 (t, 4 H, J = 6.0 Hz), 3.02 (s, 3 H), 1.75 (m, 4 H), 1.56 (m, 4 H), 1.52 (d, 3 H, J = 6.9 Hz); IR (KBr) 3286, 2929, 1652, 1592, 1511, 1452, 1421, 1333,1159 cm⁻¹; MS (FAB) *m*/*z* 534 (M+H)

### Beispiel 134: 2-(3-Fluoro-4-methansulfonylamino-phenyl)-N-[2-(4-methyl-piperazin-1-yl)-6-trifluoromethyl-pyridin-3-ylmethyl]-propionamid

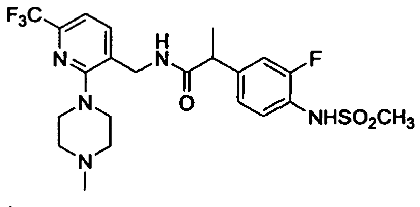

¹H NMR (300 MHz, CDCl₃) δ 7.54 (dd, 1 H, *J* = 8.4, 8.4 Hz), 7.50 (d, 1 H, *J* = 8.1 Hz), 7.23 (d, 1 H, *J* = 7.7 Hz), 7.14 (dd, 1 H, *J =* 11.2, 1.9 Hz), 7.09 (d, 1 H, *J* = 8.2 Hz), 6.21 (bt, 1 H), 4.47 (m, 2 H), 3.57 (q, 1 H, *J* = 7.1 Hz), 3.19-3.15 (m, 4 H), 3.04 (s, 3 H), 2.53-2.49 (m, 4 H), 2.34 (s, 3 H), 1.54 (d , 3 H, *J* = 7.1 Hz); IR (pur) 2935, 1655, 1591, 1511, 1457, 1417, 1334, 1149, 966, 757 cm⁻¹; MS (FAB) *m*/*z* 518 (M+H)

### Beispiel 135: N-[2-(3,4-Dimethyl-phenylamino)-6-trifluoromethyl-pyridin-3-ylmethyl]-2-(3-fluoro-4-methansulfonylamino-phenyl)-propionamid

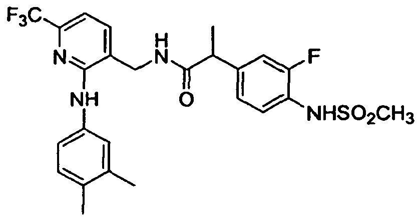

¹H NMR (300 MHz, CDCl₃) δ 7.56 (m, 2 H), 7.43 (dd, 1 H, *J* = 8.4, 8.4 Hz), 7.39 (d, 1 H, *J* = 7.8 Hz), 7.14 (dd, 1 H, *J* = 11.0, 2.2 Hz), 7.06 (d, 1 H, *J* = 8.7 Hz), 7.03 (d, 1 H, *J* = 7.7 Hz), 6.95 (d, 1 H, *J* = 7.5 Hz), 6.41 (bs, 1 H), 5.85 (bt, 1 H), 4.47 (d, 2 H, *J* = 6.4 Hz), 3.52 (q, 1 H, *J* =7.1 Hz), 2.96 (s, 3 H), 2.27 (s, 3 H), 2.23 (s, 3 H), 1.52 (d , 3 H, *J* = 7.1 Hz); IR (pur) 3363, 2922, 1646, 1538, 1509, 1428, 1328, 1156, 970, 814 cm⁻¹; MS (FAB) *m*/*z* 539 (M+H)

### Beispiel 136: N-[2-(5-Chloro-2-methyl-phenylamino)-6-trifluoromethyf-pyridin-3-ylmethyl]-2-(3-fluoro-4-methansulfonylamino-phenyl)-propionamid

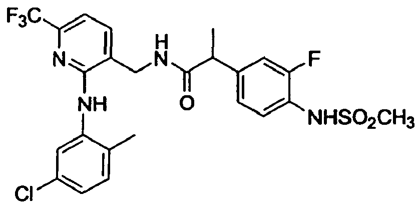

¹H NMR (300 MHz, CDCl₃) δ 7.68 (d, 1 H, *J* = 2.2 Hz), 7.45 (d, 1 H, *J* = 7.5 Hz), 7.42 (dd, 1H, *J* = 8.3, 8.3 Hz), 7.11 (d, 1 H, *J* = 7.7 H), 7.08 (dd, 1 H, *J* = 9.0, 2.2 Hz), 7.03-7.00 (m, 3 H), 6.43 (bs, 1 H), 5.87 (bt, 1 H), 4.49 (m, 2 H), 3.51 (q, 1 H, *J* =7.1 Hz), 3.02 (s, 3 H), 2.25 (s, 3 H), 1.48 (d, 3 H, *J* = 7.1 Hz); IR (pur) 3293, 1706, 1651, 1595, 1517, 1423, 1334, 1156, 969, 904, 819 cm⁻¹; MS (FAB) *m*/*z* 559 (M+H)

### Beispiel 137: N-(2-Azocan-1-yl-6-trifluoromethyl-pyridin-3-ylmethyl)-2-(3-fluoro-4-methansulfonylamino-phenyl)-propionamid

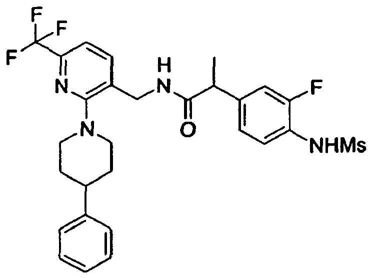

¹H NMR (300 MHz, CDCl₃) δ 7.48-7.51 (m, 2 H), 7.08-7.36 (m, 8 H), 6.52 (s, 1 H), 6.23 (bs, 1 H), 4.53 (d, 2 H, J = 5.1 Hz), 3.56 (q, 1 H, J = 7.2 Hz), 3.46 (m, 2 H), 2.95-3.00 (m, 5 H), 2.03 (m, 2 H), 1.82 (m, 2 H), 1.54 (d, 3 H, J = 7.2 Hz); IR (KBr) 2933, 1655, 1592, 1512, 1419, 1374, 1336, 1224, 1158, 957, 834, 758, 701 cm⁻¹; MS (FAB) *m*/*z* 579 (M+H)

### Beispiel 138: 2-(3-Fluoro-4-methansulfonylamino-phenyl)-N-(4-fluoro-6'-trifluoromethyl-3,4,5,6-tetrahydro-2H-[1,2']bipyridinyl-3'-ylmethyl)-propionamid

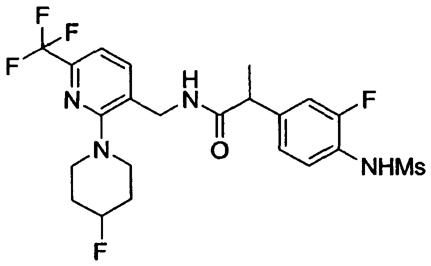

¹H NMR (300 MHz, CD₃OD) δ 7.50 (d, 1 H, J = 8.1 Hz), 7.43 (t, 1 H, J = 8.1 Hz), 7.14-7.26 (m, 3 H), 4.75 (dm, 1 H, J = 50 Hz), 4.38 (d, 2 H, J = 5.7 Hz) 3.71 (q, 1 H, J = 7.2 Hz), 3.30 (m, 2 H), 3.03 (m, 2 H), 2.96 (s, 3 H), 1.88 (m, 4 H), 1.46 (d, 3 H, J = 7.2 Hz); IR (KBr) 2926, 2854, 1656, 1591, 1512, 1418 cm⁻¹; MS (FAB) *m*/*z* 521 (M+H)

### Beispiel 139: 2-(3-Fluoro-4-methansulfonylamino-phenyl)-N-(6'-trifluoromethyl-3,6-dihydro-2H-[1,2']bipyridinyl-3'-ylmethyl)-propionamid

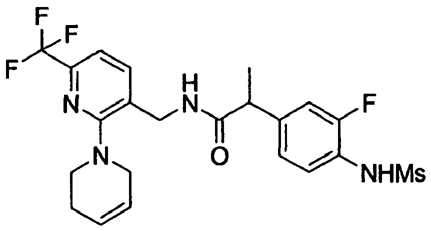

¹H NMR (300 MHz, CDCl₃) δ 7.47-7.52 (m, 2 H), 7.06-7.22 (m, 4 H), 6.68 (bs, 1 H), 6.40 (bt, 1 H), 5.79-5.83 (m, 2 H), 4.49 (d, 2 H, J = 5.7 Hz), 3.69 (m, 2 H), 3.56 (q, 1 H, J = 7.2 Hz), 3.21 (m, 2 H), 3.02 (s, 3 H), 2.27 (m, 2 H), 1.52 (d, 3 H, J = 7.2 Hz); IR (KBr) 3286, 2924, 1654, 1592, 1512, 1423, 1337, 1271, 1158, 972, 833, 737 cm⁻¹; MS (FAB) *m*/*z* 501 (M+H)

### Beispiel 142: 2-(3-Fluoro-4-methansulfonylamino-phenyl)-N-(2-pentyl-6-trifluoromethyl-pyridin-3-ylmethyl)-propionamid

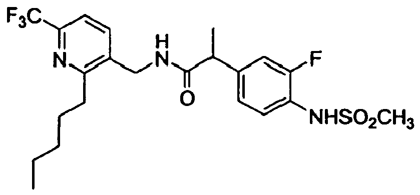

¹H NMR (300 MHz, CDCl₃) δ 7.54 (d, 1 H, *J* = 7.7 Hz), 7.43 (dd, 1 H, *J* = 8.0, 8.0 Hz), 7.41 (d, 1 H, 7.9 Hz), 7.15 (dd, 1 H, *J* = 11.2, 1.8 Hz), 7.06 (d, 1 H, *J* = 1.4 Hz), 6.20 (bt, 1 H), 4.41-4.55 (m, 2 H), 3.60 (q, 1 H, *J* = 7.0 Hz), 3.01 (s, 3 H), 2.75 (t, 2 H, *J* = 7.9 Hz), 1.61-1.71 (m, 2 H), 1.51 (d, 3 H, *J* = 7.1 Hz), 1.18-1.35 (m, 4 H), 0.85-0.90 (m, 3 H); IR (KBr) 3289, 2930, 1655, 1521, 1459, 1340, 1157, 973, 911, 732 cm⁻¹; MS (FAB) *m*/*z* 490 (M+H)

### Beispiel 147: N-[2-(4-Chloro-phenyl)-6-trifluoromethyl-pyridin-3-ylmethyl]-2-(3-fluoro-4-methansulfonylamino-phenyl)-propionamid

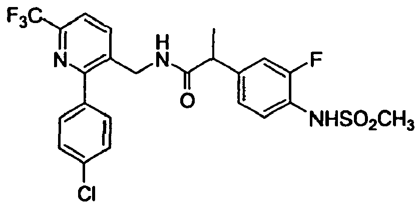

¹H NMR (300 MHz, CDCl₃) δ 7.79 (d, 1 H, *J* = 8.2 Hz), 7.62 (d, 1 H, *J* = 8.1 Hz), 7.53 (m, 1 H), 7.37-7.45 (m, 4 H), 7.06 (m, 1 H), 7.02 (d, 1 H, *J* = 7.9 Hz), 5.59 (bt, 1 H), 4.50 (d, 2 H, *J* = 6.0 Hz), 3.48 (q, 1 H, *J* = 7.3 Hz), 3.04 (s, 3 H), 1.47 (d, 3 H, *J* = 7.1 Hz); IR (KBr) 3290, 1657, 1512, 1456, 1409, 1339, 1154, 972, 910, 835, 732 cm⁻¹; MS (FAB) *m*/*z* 530 (M+H)

### Beispiel 148: 2-(3-Fluoro-4-methansulfonylamino-phenyl)-N-[2-(3-fluoro-phenyl)-6-trifluoromethyl-pyridin-3-ylmethyl]-propionamid

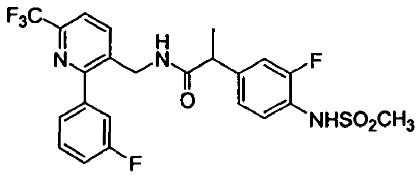

¹H NMR (300 MHz, CDCl₃) δ 7.79 (d, 1 H, *J* = 8.1 Hz), 7.62 (d, 1 H, *J* = 8.1 Hz), 7.36-7.44 (m, 2 H), 6.97-7.19 (m, 5 H), 6.90 (bs, 1 H), 6.01 (bt, 1 H), 4.37-4.51 (m, 2 H), 3.50 (q, 1 H, *J* = 7.1 Hz), 3.00 (s, 3 H), 1.45 (d, 3 H, *J* = 7.1 Hz); IR (KBr) 3239, 1655, 1586, 1512, 1448, 1340, 1154, 972, 912 cm⁻¹; MS (FAB) *m*/*z* 514 (M+H)

### Beispiel 149: N-[2-(3-Chloro-phenyl)-6-trifluoromethyl-pyridin-3-ylmethyl]-2-(3-fluoro-4-methansulfonylamino-phenyl)-propionamid

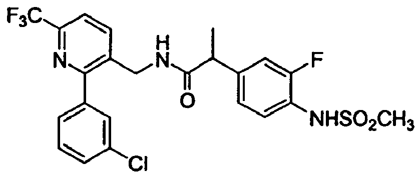

¹H NMR (300 MHz, CDCl₃) δ 7.82 (d, 1 H, *J* = 8.3 Hz), 7.62 (d, 1 H, *J* = 8.0 Hz), 7.52 (dd, 1 H, *J* = 8.3, 8.3 Hz), 7.40-7.42 (m, 3 H), 7.31 (m, 1 H), 7.07 (m, 1 H), 7.01 (m, 1 H), 4.48 (d, 2 H, *J* = 6.6 Hz), 3.48 (q, 1 H, *J* = 7.0 Hz), 3.04 (s, 3 H), 1.47 (d, 3 H, *J* = 7.1 Hz); IR (KBr) 3293, 2927, 1655, 1512, 1340, 1153, 732 cm⁻¹; MS (FAB) *m*/*z* 530 **(M+H)**

### Beispiel 150: 2-(3-Fluoro-4-methansulfonylamino-phenyl)-N-[2-(2-fluoro-phenyl)-6-trifluoromethyl-pyridin-3-ylmethyl]-propionamid

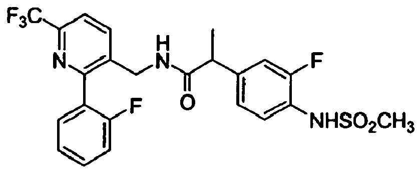

¹H NMR (300 MHz, CDCl₃) δ 7.86 (d, 1 H, *J* = 8.1 Hz), 7.66 (d, 1 H, *J* = 8.0 Hz), 7.36-7.51 (m, 3 H), 7.28 (m, 1 H), 7.01-7.16 (m, 3 H), 6.68 (bs, 1 H), 5.84 (bt, 1 H), 4.29-4.44 (m, 2 H), 3.49 (q, 1 H, *J* = 7.0 Hz), 3.02 (s, 3 H), 1.47 (d, 3 H, *J* = 7.1 Hz); IR (KBr) 3292, 1658, 1512, 1340, 1156, 973, 732 cm⁻¹; MS (FAB) *m*/*z* 514 (M+H)

### Beispiel 151: 2-(3-Fluoro-4-methansulfonylamino-phenyl)-N-[2-(4-methoxy-phenyl)-6-trifluoromethyl-pyridin-3-ylmethyl]-propionamid

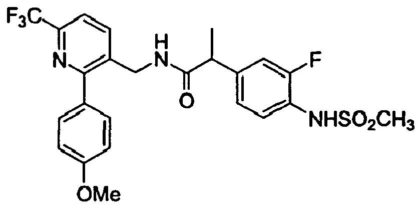

¹H NMR (300 MHz, CDCl₃) δ 7.77 (d, 1 H, *J* = 7.9 Hz), 7.57 (d, 1 H, *J* = 8.1 Hz), 7.50 (dd, 1 H, *J* = 8.3, 8.3 Hz), 7.38 (d, 2 H, *J* = 8.8 Hz), 7.01-7.06 (m, 2 H), 6.96 (d, 2 H, *J* = 8.9 Hz), 6.50 (bs, 1 H), 5.57 (bs, 1 H), 4.53 (d, 2 H, *J* = 5.3 Hz), 3.86 (s, 3 H), 3.46 (q, 1 H, *J* = 7.0 Hz), 3.03 (s, 3 H), 1.46 (d , 3 H, *J* = 7.1 Hz); IR (KBr) 2928, 1655, 1514, 1340, 1251, 1155, 973, 837, 732 cm⁻¹; MS (FAB) *m*/*z* 526 (M+H)

### Beispiel 152: N-[4-tert-Butyl-2-(2,2-dimethyl-propoxy)-benzyl]-2-(3-fluoro-4-methansulfonylamino-phenyl)-propionamid

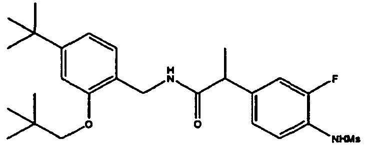

¹H NMR (300 MHz, CDCl₃) δ 7.47 (t, 1 H, *J* = 8.2 Hz), 7.16-7.11 (m, 2 H), 7.04 (d, 1 H, *J* = 8.2 Hz), 6.93-6.84 (m, 2 H), 6.52 (bs, 1 H), 5.90 (bt, 1 H), 4.50-4.31 (m, 2 H), 3.63-3.57 (m, 2 H), 3.44 (q, 1 H, *J* = 6.9 Hz), 3.01 (s, 3 H), 1.47 (d, 3 H, *J* = 6.9 Hz), 1.31 (s, 9 H), 1.0 (s, 9 H); IR (KBr) 3292, 2960, 1649, 1511, 1457, 1408 cm⁻¹; MS (FAB) *m*/*z* 493 (M+H)

### Beispiel 153: N-(4-tert-Butyl-2-pentyloxy-benzyl)-2-(3-fluoro-4-methansulfonylamino-phenyl)-propionamid

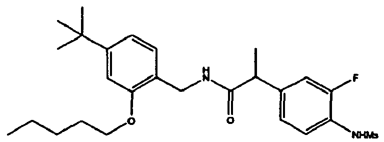

¹H NMR (300 MHz, CDCl₃) δ 7.49 (t, 1 H, *J* = 8.2 Hz), 7.16-7.04 (m, 3 H), 6.92-6.85 (m, 2 H) 6.52 (bs, 1 H), 5.99 (bt, 1 H), 4.45-4.29 (m, 2 H), 4.01-3.89 (m, 2 H), 3.46 (q, 1 H, *J* = 7.1 Hz), 3.01 (s, 3 H), 1.77-1.68 (m, 2 H), 1.48 (d, 3 H, *J* = 7.1 Hz), 1.43-1.39 (m, 4 H), 1.30 (s, 9 H), 0.93 (t, 3 H, *J* = 7.1 Hz); IR (KBr) 3288, 2959, 2868, 1650, 1510, 1455 cm⁻¹; MS (FAB) *m*/*z* 493 (M+H)

### Beispiel 154: N-(4-tert-Butyl-2-cyclohexyloxy-benzyl)-2-(3-fluoro-4-methansulfonylamino-phenyl)-propionamid

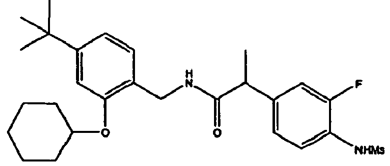

¹H NMR (300 MHz, CDCl₃) δ 7.50 (t, 1 H, *J* = 8.4 Hz), 7.17-6.86 (m, 5 H), 6.44 (bs, 1 H), 6.01 (bt, 1 H), 4.45-4.30 (m, 3 H), 3.47 (q, 1 H, *J* = 6.9 Hz), 3.01 (s, 3 H), 1.95-1.25 (m, 10 H), 1.48 (d, 3 H, *J* = 7.1 Hz), 1.29 (s, 9 H); IR (KBr) 3292, 2935, 2859, 1650, 1509, 1454 cm⁻¹; MS (FAB) *m*/*z* 505 (M+H)

### Beispiel 155: N-(4-tert-Butyl-2-cyclopentyloxy-benzyl)-2-(3-fluoro-4-methansulfonylamino-phenyl)-propionamid

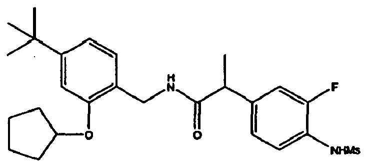

¹H NMR (300 MHz, CDCl₃) δ 7.49 (t, 1 H, *J* = 8.2 Hz), 7.16-7.04 (m, 3 H), 6.90-6.86 (m, 2 H), 6.51 (bs, 1 H), 5.94 (bt, 1 H), 4.78-4.76 (m, 1 H), 4.41-4.25 (m, 2 H), 3.46 (q, 1 H, *J* = 7.1 Hz), 3.01 (s, 3 H), 1.90-1.61 (m, 8 H), 1.48 (d, 3 H, *J* = 7.1 Hz), 1.29 (s, 9 H); IR (KBr) 3289, 2962, 2870, 1650, 1509, 1411 cm⁻¹; MS (FAB) *m*/*z* 491 (M+H)

### Beispiel 156: N-(2-Cyclobutoxy-6-trifluoromethyl-pyridin-3-ylmethyl)-2-(3-fluoro-4-methansulfonylamino-phenyl)-propionamid

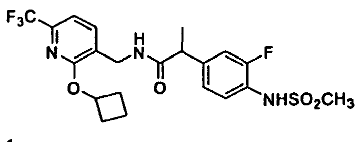

¹H NMR (CDCl₃) δ 7.58-7.51 (m, 2 H), 7.18 (d, 1 H, J = 7.3 Hz), 7.13-7.07 (m, 2 H), 6.50 (bs, NH), 6.00 (bt, NH), 5.20 (m, 1 H), 4.37 (d, 2 H, J = 6.2 Hz), 3.56 (q, 1 H, J = 7.0 Hz), 3.03 (s, 3 H), 2.50-2.40 (m, 2 H), 2.05-1.65 (m, 4 H), 1.50 (d, 3 H, J = 7.1 1 Hz); IR (pur) 3290, 2987, 1655, 1513, 1421, 1340, 1275, 1157, 1071, 962 cm⁻¹; MS (FAB) m/z 490 (M+H)

### Beispiel 157: 2-(3-Fluoro-4-methansulfonylamino-phenyl)-N-[2-(4-methyl-cyclohexyloxy)-6-trifluoromethyl-pyridin-3-ylmethyl]-propionamid

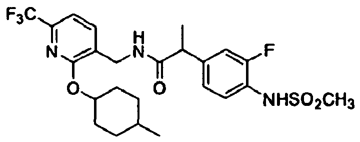

¹H NMR (CDCl₃) δ 7.57-7.49 (m, 2 H), 7.16 (d, 1 H, J = 7.3 Hz), 7.12-7.05 (m, 2 H), 6.48 (bs, NH), 5.99 (bt, NH), 5.00 (m, 1 H), 4.34 (d, 2 H, J = 5.8 Hz), 3.51 (q, 1 H, J = 6.8 Hz), 3.03 (s, 3 H), 2.12-2.00 (m, 2 H), 1.80-1.72 (m, 2 H), 1.50-1.10 (m, 5 H), 1.48 (d, 3 H, J = 7.1 Hz), 0.94 (d, 3 H, J = 6.6 Hz); IR (pur) 3287, 2931, 1655, 1513, 1422, 1336, 1271, 1158, 914, 734 cm⁻¹; MS (FAB) m/z 532 (M+H)

### Beispiel 158: Essigsäure 3'-{[2-(3-fluoro-4-methansulfonylamino-phenyl)-propionylamino]-methyl}-6'-trifluoromethyl-3,4,5,6-tetrahydro-2H-[1,2']bipyridinyl-4-yl ester

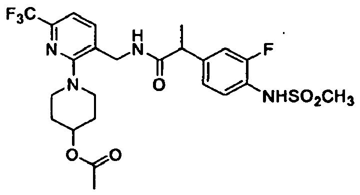

¹H NMR (CDCl₃) δ 7.57-7.48 (m, 2 H), 7.24 (d, 1 H, J = 8.1 Hz), 7.17-7.09 (m, 2 H), 6.47 (bs, NH), 6.05 (bt, NH), 4.93 (m, 1 H), 4.47 (d, 2 H, J = 5.7 Hz), 3.57 (q, 1 H, J = 7.0 Hz), 3.35-3.25 (m, 2 H), 3.07-2.97 (m, 2 H), 3.04 (s, 3 H), 2.08 (s, 3 H), 2.02-1.92 (m, 2 H), 1.80-1.70 (m, 2 H), 1.54 (d, 3 H, J = 7.3 Hz);IR (pur) 3362, 2910, 1726, 1657, 1512, 1419, 1335, 1260, 1157, 1033, 758 cm⁻¹; MS (FAB) m/z 561 (M+H)

### Beispiel 159: 2-(3-Fluoro-4-methansulfonylamino-phenyl)-N-(4-methoxy-6'-trifluoromethyl-3,4,5,6-tetrahydro-2H-[1,2]bipyridinyl-3'-ylmethyl)-propionamid

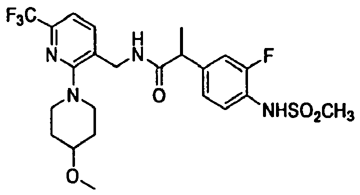

¹H NMR (CDCl₃) δ 7.53-7.47 (m, 2 H), 7.22 (d, 1 H, J = 7.7 Hz), 7.15-7.07 (m, 2 H), 6.77 (bs, N H), 6.32 (bt, NH), 4.47 (d, 2 H, J = 5.7 Hz), 3.58 (q, 1 H, J = 7.1 Hz), 3.40-3.25 (m, 3 H), 3.37 (s, 3 H), 3.03 (s, 3 H), 2.95-2.86 (m, 2 H), 2.04-1.95 (m, 2 H), 1.63-1.50 (m, 2 H), 1.53 (d, 3 H, J = 7.0 Hz); IR (pur) 3289, 2932, 1656, 1592, 1512, 1457, 1418, 1335, 1275, 1158, 733 cm⁻¹; MS (FAB) m/z 533 (M+H)

### Beispiel 160: N-(4-Butoxy-6'-trifluoromethyl-3,4,5,6-tetrahydro-2H-[1,2']bipyridinyl-3'-ylmethyl)-2-(3-fluoro-4-methansulfonylamino-phenyl)-propionamid

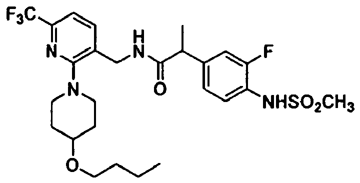

¹H NMR (CDCl₃) δ 7.54-7.48 (m, 2 H), 7.21 (d, 1 H, J = 7.5 Hz), 7.14-7.07 (m, 2H), 6.64 (bs, NH), 6.26 (bt, NH), 4.47 (d, 2 H, J = 5.7 Hz), 3.57 (q, 1 H, J = 7.1 Hz), 3.50-3.26 (m, 5 H), 3.03 (s, 3 H), 2.94-2.86 (m, 2 H), 2.02-1.95 (m, 2 H), 1.62-1.50 (m, 7 H), 1.45-1.33 (m, 2 H), 0.93 (t, 3 H, J = 7.3 Hz); IR (pur) 3295, 2931, 1654, 1513, 1458, 1420, 1335, 1157 cm⁻¹; MS (FAB) m/z 575 (M+H)

### Beispiel 161: N-(2-Cyclopentylmethoxy-6-trifluoromethyl-pyridin-3-ylmethyl)-2-(3-fluoro-4-methansulfonylamino-phenyl)-propionamid

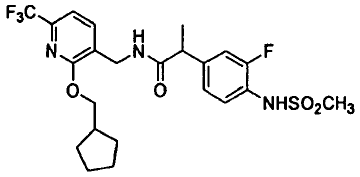

¹H NMR (CDCl₃) δ 7.58 (d, 1 H, J = 7.3 Hz), 7.51 (dd, 1 H, J = 8.4, 8.4 Hz), 7.19 (d, 1 H, J = 7.3 Hz), 7.11-7.04 (m, 2 H), 6.54 (bs, NH), 6.00 (bt, NH), 4.38 (m, 2 H), 4.20 (m, 2 H), 3.50 (q, 1 H, J = 7.1 Hz), 3.03 (s, 3 H), 2.29 (m, 1 H), 1.80-1.70 (m, 2 H), 1.70-1.55 (m, 4 H), 1.48 (d, 3 H, J = 7.1 Hz), 1.37-1.27 (m, 2 H); IR (pur) 3293, 2952, 1655, 1513, 1424, 1338, 1158 cm⁻¹; MS (FAB) m/z 518 (M+H)

### Beispiel 162: 2-(3-Fluoro-4-methansulfonylamino-phenyl)-N-(4-isopropoxy-6'-trifluoromethyl-3,4,5,6-tetrahydro-2H-[1,2']bipyridinyl-3'-ylmethyl)-propionamid

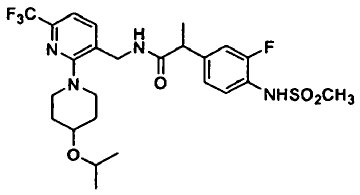

¹H NMR (CDCl₃) δ 7.55-7.48 (m, 2 H), 7.22 (d, 1 H, J = 7.7 Hz), 7.15-7.08 (m, 2 H), 6.56 (bs, NH), 6.23 (bt, NH), 4.47 (d, 2 H, J = 5.9 Hz), 3.74 (m, 1 H), 3.60-3.45 (m, 2 H), 3.37-3.33 (m, 2 H), 3.04 (s, 3 H), 2.94-2.85 (m, 2 H), 1.98-1.90 (m, 2 H), 1.62-1.50 (m, 2 H), 1.53 (d, 3 H, J = 7.0 Hz), 1.18 (d, 6 H, J = 6.1 Hz); IR (pur) 3289, 2925, 1655, 1593, 1513, 1335, 1155 cm⁻¹; MS (FAB) m/z 561 (M+H)

### Beispiel 163: N-(2-Ethoxy-6-trifluoromethyl-pyridin-3-ylmethyl)-2-(3-fluoro-4-methansulfonylamino-phenyl)-propionamid

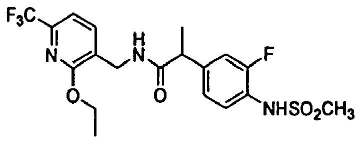

¹H NMR (CDCl₃) δ 7.58 (d, 1 H, J = 7.3 Hz), 7.51 (dd, 1 H, J = 8.2, 8.2 Hz), 7.19 (d, 1 H, J = 7.3 Hz), 7.12-7.05 (m, 2 H), 6.58 (bs, NH), 6.02 (bt, NH), 4.44-4.36 (m, 4 H), 3.53 (q, 1 H, J = 7.0 Hz), 3.03 (s, 3 H), 1.49 (d, 3 H, J = 7.1 Hz), 1.34 (t, 3 H, J = 7.1 Hz); IR (pur) 3294, 1654, 1513, 1425, 1342, 1156 cm⁻¹; MS (FAB) m/z 464 (M+H)

### Beispiel 164: 2-(3-Fluoro-4-methansulfonylamino-phenyl)-N-(6"-trifluoromethyl-3,4,5,6,3',4',5',6'-octahydro-2H,2'H-[1,4';1',2"]terpyridin-3"-ylmethyl)-propionamid

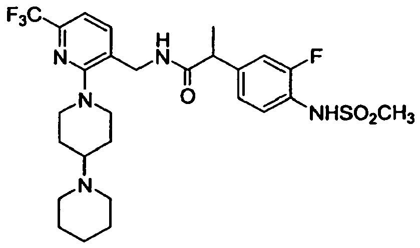

¹H NMR (300 MHz, CD₃OD) δ 7.51 (d, 1 H, *J* = 7.7 Hz), 7.42 (dd, 1 H, *J* = 8.2, 8.2 Hz), 7.53 (d, 1 H, *J* = 7.7 Hz), 7.13-7.21 (m, 2 H), 4.30-4.47 (m, 2 H), 3.71 (q, 1 H, *J* = 7.0 Hz), 3.48-3.52 (m, 2 H), 2.97 (s, 3 H), 2.80-2.84 (m, 2 H), 2.55-2.75 (m, 5 H), 1.88-2.00 (m, 2 H), 1.60-1.75 (m, 6 H), 1.50-1.55 (m, 2 H), 1.46 (d , 3 H, *J* = 7.0 Hz); IR (KBr) 2924, 1649, 1509, 1456, 1419, 1334, 1124, 961 cm⁻¹; MS (FAB) *m*/*z* 586 (M+H)

### Beispiel 165; 2-(3-Fluoro-4-methansulfonylamino-phenyl)-N-(4-pyrrolidin-1-yl-6'-trifluoromethyl-3,4,5,6-tetrahydro-2H-[1,2']bipyridinyl-3'-ylmethyl)-propionamid

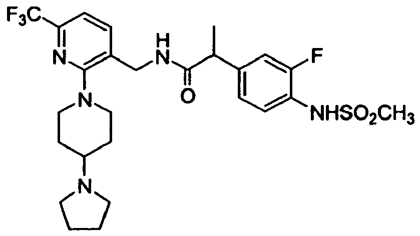

¹H NMR (300 MHz, CD₃OD) δ 7.50 (d, 1 H, *J* = 7.7 Hz), 7.42 (dd, 1 H, *J* = 8.3, 8.3 Hz), 7.25 (d, 1 H, *J* = 7.7 Hz), 7.10-7.22 (m, 2 H), 4.29-4.45 (m, 2 H), 3.72 (q, 1 H, *J* = 7.1 Hz), 3.40-3.50 (m, 2 H), 2.70-2.92 (m, 6 H), 2.40 (m, 1 H), 1.95-2.10 (m, 2 H), 1.81-2.10 (m, 4 H), 1.57-1.74 (m, 2 H), 1.46 (d, 3 H, *J* = 7.0 Hz) IR (KBr) 3296, 2926, 1651, 1580, 1420, 1333, 1126, 980, 832 cm⁻¹; MS (FAB) *m*/*z* 572 (M+H)

### Beispiel 166: N-[6-(Chloro-difluoro-methyl)-2-cyclopentyloxy-pyridin-3-ylmethyl]-2-(3-fluoro-4-methansulfonylamino-phenyl)-propionamid

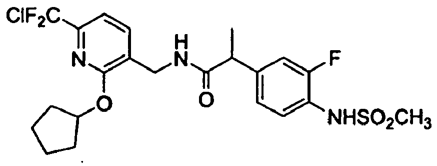

¹H NMR (300 MHz, CDCl₃) δ 7.48-7.57 (m, 2 H), 7.03-7.15 (m, 3 H), 6.56 (bs, 1 H), 5.96 (bt, 1 H), 5.46 (m, 1 H), 4.27-4.42 (m, 2 H), 3.52 (q, 1 H, *J* = 7.1 Hz), 3.03 (s, 3 H), 1.19-2.08 (m, 2 H), 1.56-1.78 (m, 6 H), 1.49 (d, 3 H, *J* = 7.1 Hz); IR (KBr) 3288, 2967, 1655, 1512, 1419, 1339, 1159, 1112, 989, 889 cm⁻¹; MS (FAB) *m*/*z* 520 (M+H)

### Beispiel 167: N-[2-(Butyl-methyl-amino)-6-trifluoromethyl-pyridin-3-ylmethyl]-2-(3-fluoro-4-methansulfonylamino-phenyl)-propionamid

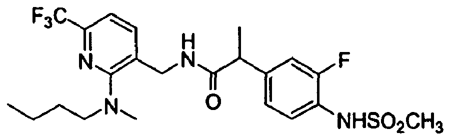

¹H NMR (300 MHz, CDCl₃) δ 7.53 (dd, 1 H, *J* = 8.3, 8.3 Hz), 7.45 (d, 1 H, *J* = 7.9 Hz), 7.05-7.19 (m, 3 H), 6.52 (bs, 1 H), 6.13 (bt, 1 H), 4.46 (d, 2 H, *J* = 5.9 Hz), 3.56 (q, 1 H, *J* = 7.1 Hz), 3.05-3.12 (m, 2 H), 3.04 (s, 3 H), 2.80 (s, 3 H), 1.42-1.58 (m, 5 H), 1.20-1.38 (m, 2 H), 0.90 (t, 3 H, *J* = 7.3 Hz); IR (KBr) 3280, 2932, 1653, 1511, 1460, 1400, 1335, 1159, 971 cm⁻¹; MS (FAB) *m*/*z* 505 (M+H)

### Beispiel 168: N-[6-(Chloro-difluoro-methyl)-2-cyclohexyloxy-pyridin-3-ylmethyl]-2-(3-fluoro-4-methansulfonylamino-phenyl)-propionamid

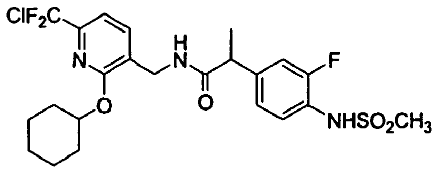

¹H NMR (300 MHz, CDCl₃) δ □7.48-7.59 (m, 2 H), 7.02-7.14 (m, 3 H), 6.49 (bs, 1 H), 6.01 (bt, 1 H), 5.13 (m, 1 H), 4.29-4.47 (m, 2 H), 3.52 (q, 1 H, *J* = 7.3 Hz), 3.03 (s, 3 H), 1.85-1.99 (m, 2 H), 1.62-1.77 (m, 2 H), 1.38-1.52 (m, 9 H); IR (KBr) 3288, 2935, 2857, 1653, 1512, 1420, 1335, 1266, 1158, 1114, 987, 882 cm⁻¹; MS (FAB) *m*/*z* 534 (M+H)

### Beispiel 169: N-[2-Benzyloxy-6-(chloro-difluoro-methyl)-pyridin-3-ylmethyl]-2-(3-fluoro-4-methansulfonylamino-phenyl)-propionamid

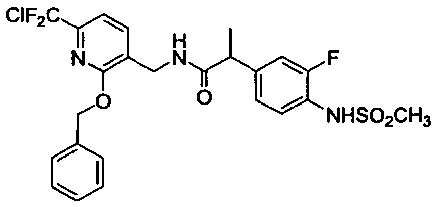

¹H NMR (300 MHz, CDCl₃) δ 7.59 (d, 1 H, *J* = 7.3 Hz), 7.30-7.50 (m, 6 H), 7.20 (d, 1 H, *J* = 7.8 Hz), 7.05 (dd, 1 H, *J* = 11.2, 2.0 Hz), 6.97 (d, 1 H, *J* = 7.9 Hz), 6.52 (bs, 1 H), 6.00 (bt, 1 H), 5.36-5.49 (m, 2 H), 4.30-4.46 (m, 2 H), 3.42 (q, 1 H, *J* = 7.1 Hz), 3.00 (s, 3 H), 1.43 (d , 3 H, *J* = 7.1 Hz); IR (KBr) 3286, 1653, 1511, 1417, 1334, 1267, 1157, 1114, 971, 883, 756 cm⁻¹; MS (FAB) *m*/*z* 542 (M+H)

### Beispiel 170: N-[2-(4-tert-Butyl-cyclohexyloxy)-6-trifluoromethyl-pyridin-3-ylmethyl]-2-(3-fluoro-4-methansulfonylamino-phenyl)-propionamid

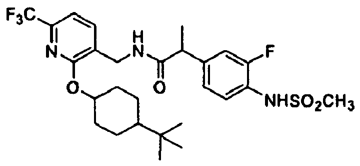

¹H NMR (CDCl₃) δ 7.57-7.50 (m, 2 H), 7.16 (d, 1 H, J = 7.3 Hz), 7.12-7.05 (m, 2 H), 6.46 (bs, NH), 5.98 (bt, NH), 4.96 (m, 1 H), 4.34 (m, 2 H), 3.51 (q, 1 H, J = 7.1 Hz), 3.03 (s, 3 H), 2.20-2.10 (m, 2 H), 1.88-1.80 (m, 2 H), 1.49 (d, 3 H, J = 7.1 Hz), 1.30-1.00 (m, 5 H), 0.89 (s, 9 H); IR (pur) 3291, 2950, 2866, 1654, 1513, 1422, 1338, 1268,1158 cm⁻¹; MS (FAB) m/z 574 (M+H)

### Beispiel 171: N-[2-(4-Ethyl-cyclohexyloxy)-6-trifluoromethyl-pyridin-3-ylmethyl]-2-(3-fluoro-4-methansulfonylamino-phenyl)-propionamid

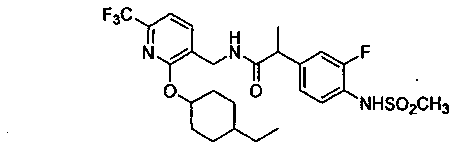

¹H NMR (CDCl₃) δ 7.57-7.50 (m, 2 H), 7.16 (d, 1 H, J = 7.3 Hz), 7.12-7.05 (m, 2 H), 6.47 (bs, NH), 5.99 (bt, NH), 5.00 (m, 1 H), 4.34 (m, 2 H), 3.52 (q, 1 H, J = 7.5 Hz), 3.03 (s, 3 H), 2.13-2.03 (m, 2 H), 1.87-1.80 (m, 2 H), 1.49 (d, 3 H, J = 7.1 Hz), 1.32-1.04 (m, 7 H), 0.91 (t, 3 H, J = 7.1 Hz); IR (pur) 3287, 2935, 2858, 1655, 1513, 1421, 1337, 1269, 1159 cm⁻¹; MS (FAB) m/z 546 (M+H)

### Beispiel 172: 2-(3-Fluoro-4-methansulfonylamino-phenyl)-N-[2-(4-methyl-benzyloxy)-6-trifluoromethyl-pyridin-3-ylmethyl]-propionamid

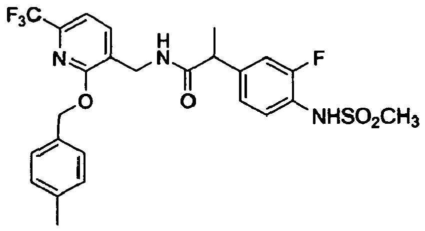

¹H NMR (300 MHz, CDCl₃) δ 7.61 (d, 1 H, *J* = 7.4 Hz), 7.47 (dd, 1 H, *J* = 8.1, 8.1 Hz), 7.31 (d, 2 H, *J* = 8.0 Hz), 7.22 (d, 1 H, *J* = 7.2 Hz), 7.20 (d, 2 H, *J* = 7.9 Hz), 7.03 (dd, 1 H, *J* = 11.5, 1.9 Hz), 6.96 (d, 1 H, *J* = 8.6 Hz), 6.46 (bs, 1 H), 5.98 (bt, 1 H), 5.36 (m, 2 H), 4.37 (m, 2 H), 3.40 (q, 1 H, *J* =7.1 Hz), 3.01 (s, 3 H), 2.28 (s, 3 H), 1.42 (d , 3 H, *J* = 7.0 Hz); IR (pur) 3289, 2925, 1654, 1513, 1458, 1422, 1137, 1267, 1158, 976, 933, 808 cm⁻¹; MS (FAB) *m*/*z* 540 (M+H)

### Beispiel 173: N-[2-(4-Chloro-benzylamino)-6-trifluoromethyl-pyridin-3-ylmethyl]-2-(3-fluoro-4-methansulfonylamino-phenyl)-propionamid

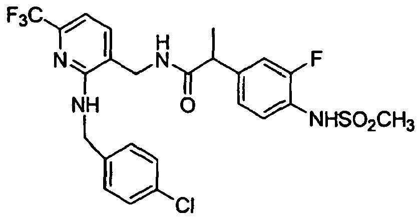

¹H NMR (300 MHz, CDCl₃) δ 7.48 (dd, 1 H, *J* = 8.3, 8.3 Hz), 7.33 (d, 2 H, *J* = 8.6 Hz), 7.25 (d, 2 H, *J* = 8.6 Hz), 7.24 (d, 1 H, *J* = 7.5 Hz), 7.07 (dd, 1 H, *J* = 11.2, 2.0 Hz), 6.99 (d, 1 H, *J* = 8.4 Hz), 6.81 (d, 1 H, *J* = 7.5 Hz), 6.71 (bt, 1 H), 6.47 (bs, 1 H), 5.72 (bs, 1 H), 4.58 (m, 2 H), 4.32 (m, 2 H), 3.44 (q, 1 H, *J* =7.1 Hz), 3.03 (s, 3 H), 1.42 (d, 3 H, *J* = 7.1 Hz); IR (pur) 3343, 2929, 1706, 16347, 1610, 1514, 1454, 1334, 1158, 1016, 973, 909, 833, 760 cm⁻¹; MS (FAB) *m*/*z* 559 (M+H)

### Beispiel 174: N-(2-Azepan-1-yl-4-trifluoromethyl-benzyl)-2-(3-fluoro-4-methansulfonylamino-phenyl)-propionamid

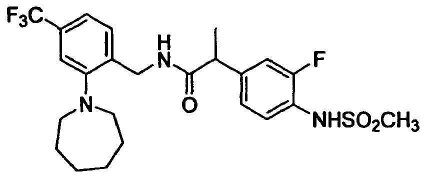

¹H NMR (300 MHz, CDCl₃) δ 7.51 (dd, 1 H, *J* = 8.2, 8.2 Hz), 7.32 (s, 1 H), 7.23 (s, 2 H), 7.14 (dd, 1 H, *J* = 11.3, 1.9 Hz), 7.08 (d, 1 H, *J* = 8.2 Hz), 6.52 (bs, 1 H), 6.43 (bt, 1 H), 4.53 (m, 2 H), 3.54 (q, 1 H, *J* =7.0 Hz), 3.04-3.00 (m, 7 H), 1.72-1.64 (m, 8 H), 1.52 (d , 3 H, *J* = 7.0 Hz); IR (pur) 3273. 2930, 2854, 1650, 1510, 1424, 1335, 1159, 1121, 972, 901, 737 cm⁻¹; MS (FAB) *m*/*z* 516 (M+H)

### Beispiel 175: N-[2-(4-Fluoro-benzyloxy)-6-trifluoromethyl-pyridin-3-ylmethyl]-2-(3-fluoro-4-methansulfonylamino-phenyl)-propionamid

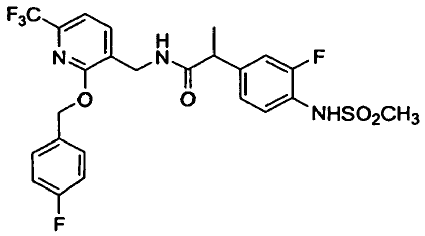

¹H NMR (300 MHz, CDCl₃) δ 7.59 (d, 1 H, *J* = 7.3 Hz), 7.48 (dd, 1 H, *J* = 8.3, 8.3 Hz) 7.41 (m, 2 H), 7.23 (d, 1 H, *J* = 7.5 Hz), 7.06 (m, 3 H), 6.99 (d, 1 H, *J* = 8.0 Hz), 6.51 (bs, 1 H), 5.93 (bt, 1 H), 5.37 (m, 2 H), 4.38 (m, 2 H), 3.45 (q, 1 H, *J* = 7.1 Hz), 3.02 (s, 3 H), 1.44 (d, 3 H, *J* = 7.1 Hz); IR (pur) 2925, 1654, 1603, 1512, 1423, 1337, 1268, 1225, 1158, 975, 931, 759 cm⁻¹; MS (FAB) *m*/*z* 544 (M+H)

### Beispiel 176: 2-(3-Fluoro-4-methansulfonylamino-phenyl)-N-[2-(4-pyridin-4-yl-piperazin-1-yl)-6-trifluoromethyl-pyridin-3-ylmethyl]-propionamid

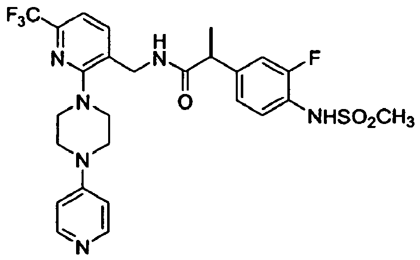

¹H NMR (300 MHz, CDCl₃) δ 8.28 (d, 2H, *J* = 6.4 Hz), 7.55 (d, 1 H, *J* = 7.9 Hz), 7.50 (dd, 1 H, *J* = 8.2, 8.2 Hz), 7.29 (d, 1 H, *J* = 7.9 Hz), 7.14 (dd, 1 H, *J* = 11.4, 2.0 Hz), 7.09 (d, 1 H, *J* = 8.3 Hz), 6.69 (d, 2 H, *J* = 6.6 Hz), 6.26 (bt, 1 H), 4.52 (d, 2 H, *J* = 5.7 Hz), 3.60 (q, 1 H, *J* = 7.0 Hz), 3.43-3.38 (m, 4 H), 3.29-3.25 (m, 4 H), 3.02 (s, 3 H), 1.54 (d, 3 H, *J* = 7_{.}1 Hz); IR (pur) 2848, 1650, 1597, 1512, 1454, 1416, 1333, 1238, 1152, 994, 966, 808, 735 cm⁻¹; MS (FAB) *m*/*z* 581 (M+H)

### Beispiel 177: 2-(3-Fluoro-4-methansulfonylamino-phenyl)-N-[2-(pyridin-4-ylmethoxy)-6-trifluoromethyl-pyridin-3-ylmethyl]-propionamid

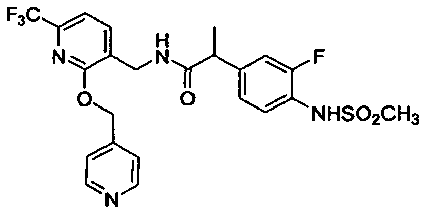

¹H NMR (300 MHz, CDCl₃) δ 8.49 (d, 2 H, *J* = 6.2 Hz), 7.61 (d, 1 H, *J* = 7.4 Hz), 7.49 (d, 2 H, *J* = 6.2 Hz), 7.40 (dd, 1 H, *J* = 8.3, 8.3 Hz), 7.32 (d, 1 H, *J* = 7.4 Hz), 7.19 (dd, 1 H, *J* =11.5, 2.0 Hz), 7.14 (d, 1 H, *J* = 8.4 Hz), 5.49 (s, 2 H), 4.35 (m, 2 H), 3.72 (q, 1 H, *J* = 6.9 Hz), 2.96 (s, 3 H), 1.45 (d , 3 H, *J* = 7.0 Hz); IR (pur) 3735, 3264, 1640, 1514, 1462, 1419, 1335, 1270 1154, 970, 827 cm⁻¹; MS (FAB) *m*/*z* 527 (M+H)

### Beispiel 178: 2-(3-Fluoro-4-methansulfonylamino-phenyl)-N-(2-phenethyloxy-6-trifluoromethyl-pyridin-3-ylmethyl)-propionamid

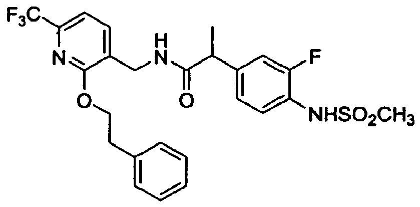

¹H NMR (300 MHz, CDCl₃) δ 7.60 (d, 1 H, *J* = 7.4 Hz), 7.46 (dd, 1 H, *J* = 8.3, 8.3 Hz), 7.33 (m, 5 H), 7.19 (d, 1 H, *J* = 7.3 Hz), 6.97 (dd, 1 H, *J* = 11.3, 1.8 Hz), 6.89 (d, 1 H, *J* = 8.9 Hz), 6.43 (bs, 1 H), 5.70 (bt, 1 H), 4.66 (m, 1 H), 4.50 (m, 1 H), 4.28 (d, 2 H, *J* = 6.2 Hz), 3.14-3.05 (m, 3 H), 2.99 (s, 3 H), 1.36 (d , 3 H, *J* = 7.1 Hz); IR (pur) 3296, 2925, 1659, 1602, 1511, 1424, 1337, 1269, 1158, 973, 755, 701 cm⁻¹; MS (FAB) *m*/*z* 539 (M+H)

### Beispiel 179: 2-(3-Fluoro-4-methansulfonylamino-phenyl)-N-{2-[4-(4-fluoro-phenyl)-piperazin-1-yl]-6-trifluoromethyl-pyridin-3-ylmethyl}-propionamid

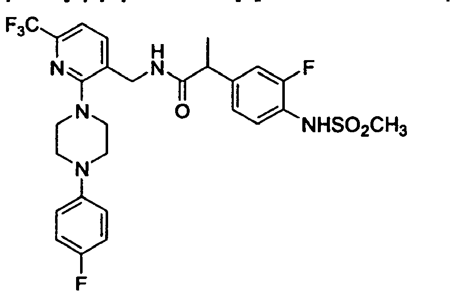

¹H NMR (300 MHz, CDCl₃) δ 7.53 (d, 1 H, *J* = 7.5Hz), 7.49 (dd, 1 H, *J* = 7.9, 7.9 Hz), 7.27 (d , 1 H, *J* = 7.5 Hz), 7.14 (d, 1 H, *J* = 11.0 Hz), 7.08 (d, 1 H, *J* = 8.4 Hz), 6.99 (m , 2 H), 6.90 (m, 2 H), 6.58 (bs, 1 H), 6.17 (bt, 1 H), 4.52 (d, 2 H, *J* = 5.7 Hz), 3.58 (q, 1 H, *J* = 6.8 Hz), 3.29-3.25 (m, 4 H), 3.22-3.18 (m, 4 H), 3.01 (s, 3 H), 1.53 (d, 3 H, *J* = 7.1 Hz); IR (pur) 3296, 2925, 2851, 1658, 1591, 1510, 1418, 1335, 1232, 1156, 968, 828, 758 cm⁻¹; MS (FAB) *m*/*z* 598 (M+H)

### Beispiel 180: N-[6-(Chloro-difluoro-methyl)-2-hexyloxy-pyridin-3-ylmethyl]-2-(3-fluoro-4-methansulfonylamino-phenyl)-propionamid

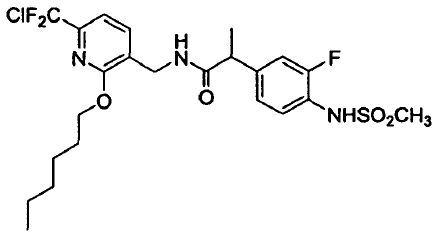

¹H NMR (400 MHz, CDCl₃) δ 7.47-7.58 (m, 2 H), 7.03-7.17 (m, 3 H), 6.50 (bs, 1 H), 5.98 (bt, 1 H), 4.25-4.43 (m, 4 H), 3.51 (q, 1 H, *J* = 6.8 Hz), 3.03 (s, 3 H), 1.67-1.78 (m, 2 H), 1.49 (d, 3 H, *J* = 6.8 Hz), 1.27-1.46 (m, 6 H), 0.87-0.94 (m , 3 H); IR (KBr) 3291, 2930, 1654, 1512, 1424, 1337, 1267, 1158, 1113, 974, 880 cm⁻¹; MS (FAB) *m*/*z* 536 (M+H)

### Beispiel 181: N-[6-(Chloro-difluoro-methyl)-2-(pyridin-3-ylmethoxy)-pyridin-3-ylmethyl]-2-(3-fluoro-4-methansulfonylamino-phenyl)-propionamid

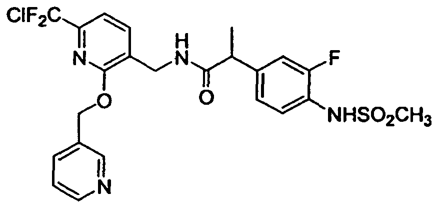

¹H NMR (400 MHz, CDCl₃) δ 8.56-8.61 (m, 2 H), 7.77 (m, 1 H), 7.62 (d, 1 H, *J* = 7.6 Hz), 7.48 (dd, 1 H, *J* = 8.0, 8.0 Hz), 7.31 (m, 1 H), 7.21 (d, 1 H, *J* = 7.6 Hz), 6.92-7.09 (m, 2 H), 5.88 (bt, 1 H), 5.37-5.47 (m, 2 H), 4.30-4.43 (m, 2 H), 3.49 (q, 1 H *J* = 6.8 Hz), 3.03 (s , 3 H), 1.28 (d, 3 H, *J* = 6.8 Hz); IR (KBr) 2964, 1656, 1597, 1511, 1414, 1332, 1262, 1155, 1094, 1020, 800, 732 cm⁻¹; MS (FAB) *m*/*z* 543 (M+H)

### Beispiel 182: N-[6-(Chloro-difluoro-methyl)-2-(pyridin-2-ylmethoxy)-pyridin-3-ylmethyl]-2-(3-fluoro-4-methansulfonylamino-phenyl)-propionamid

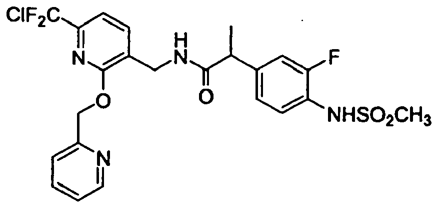

¹H NMR (400 MHz, CDCl₃) δ 8.62 (d, 1 H, *J* = 4.4 Hz), 7.75 (dd, 1 H, *J* = 7.6, 7.6 Hz), 7.67 (d, 1 H, *J* = 7.2 Hz), 7.40-7.51 (m, 2 H), 7.19-7.27 (m, 2 H), 7.13 (dd, 1 H, *J* = 11.2, 1.6 Hz), 7.04 (d, 1 H, *J* = 8.4 Hz), 6.50 (bs, 1 H), 5.48-5.63 (m, 2 H), 4.40-4.61 (m, 2 H), 3.60 (q, 1 H, *J* = 7.2 Hz), 3.05 (s, 3 H), 1.49 (d, 3 H, *J* = 7.2 Hz); IR (KBr) 3287, 1659, 1596, 1511, 1454, 1415, 1334, 1270, 1157, 1117, 971, 882, 828, 758 cm⁻¹; MS (FAB) *m*/*z* 543 (M+H)

### Beispiel 183: N-(2-Dibutylamino-6-trifluoromethyl-pyridin-3-ylmethyl)-2-(3-fluoro-4-methansulfonylamino-phenyl)-propionamid

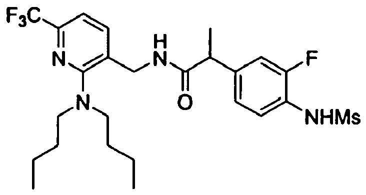

¹H NMR (300 MHz, CDCl₃) δ 7.51-7.43 (m, 2 H), 7.19-7.07 (m, 3 H), 6.96 (bs, 1 H), 6.40 (bt, 1 H), 4.50 (m, 2 H), 3.56 (q, 1 H, *J* = 7.1 Hz), 3.13 (m, 4 H), 3.02 (s, 3 H), 1.52 (d, 3 H, *J* = 7.1 Hz) 1.50 (m, 4 H), 1.31-1.10 (m, 4 H), 0.87 (t, 6 H, *J* = 7.1 Hz); IR (KBr) 3294, 2960, 1655, 1593, 1513, 1462, 1419 cm⁻¹; MS (FAB) *m*/*z* 547 (M+H)

### Beispiel 184: 2-(3-Fluoro-4-methansulfonylamino-phenyl)-N-(6'-(4-fluoro-phenyl)-4-methyl-3,4,5,6-tetrahydro-2H-[1,2']bipyridinyl-3'-ylmethyl]-propionamid

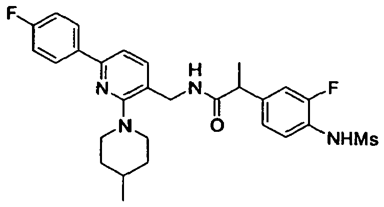

¹H NMR (300 MHz, CDCl₃) δ 7.49-7.54 (m, 2 H), 7.51 (dd, 1 H, J = 8.1, 8.1 Hz), 7.43 (d, 1 H, 7.8 Hz), 7.29 (d, 1 H, J = 7.8 Hz), 7.09-7.17 (m, 4 H), 6.64 (bt, 1 H), 4.48 (d, 2 H, J = 5.7 Hz), 3.52 (q, 1 H, J = 6.9 Hz), 3.30 (m, 2 H), 3.03 (s, 3 H), 2.88 (m, 2 H), 1.76 (m, 2 H), 1.51 (d, 3 H, J = 6.9 Hz), 1.24 (m, 3 H), 0.99 (d, 3 H, J = 6.6 Hz); IR (KBr) 3292, 2962, 1653, 1512, 1457, 1423, 1335, 1267, 1158, 1113, 977, 889, 824 cm⁻¹; MS (FAB) *m*/*z* 508 (M+H)

### Beispiel 185: 2-(3-Fluoro-4-methansulfonylamino-phenyl)-N-[6'-(4-fluoro-phenyl)-4-methyl-3,4,5,6-tetrahydro-2H-[1,2']bipyridinyl-3'-ylmethyl]-propionamid

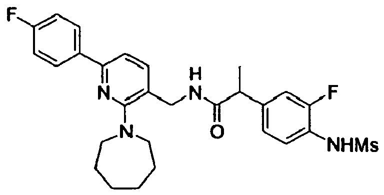

¹H NMR (300 MHz, CDCl₃) δ 7.95-8.00 (m, 2 H), 7.52 (dd, 1 H, J = 8.1, 8.1 Hz), 7.37 (d, 1H J = 7.5 Hz), 7.08-7.18 (m, 5 H), 6.43 (bs, 1 H), 6.07 (bt, 1 H), 4.44 (d, 2 H, J = 5.7 Hz), 3.54 (q, 1 H, J = 7.2 Hz), 3.40 (m, 2 H), 3.00 (s, 3 H), 1.78 (m, 4 H), 1.61 (m, 4 H), 1.52 (d, 3 H, J = 7.2 Hz); IR (KBr) 3287, 2927, 1649, 1509, 1448, 1333, 1228, 1157, 972, 909, 813, 732cm⁻¹; MS (FAB) *m*/*z* 508 (M+H)

### Beispiel 186: N-[6-(Chloro-difluoro-methyl)-2-dipropylamino-pyridin-3-ylmethyl]-2-(3-fluoro-4-methansulfonylamino-phenyl)-propionamid

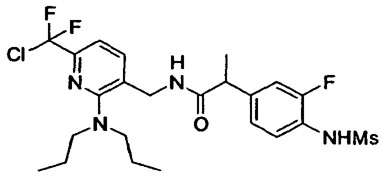

¹H NMR (300 MHz, CDCl₃) δ 7.53 (dd, 1 H, J = 8.1, 8.1 Hz), 7.43 (d, 1 H, J = 7.8 Hz), 7.16 (dd, 1 H, J = 2.1, 10.8 Hz), 7.08 -7.12 (m, 2 H), 6.46 (bs, 1 H), 6.15 (bt, 1 H), 4.44 (d, 2 H, J = 5.7 Hz), 3.53 (q, 1 H, J = 6.9 Hz), 3.10 (m, 4 H), 3.02 (s, 3 H), 1.44-1.54 (m, 4 H), 0.83 (t, 6 H, J = 7.2 Hz); IR (KBr) 3288, 2965, 1652, 1591, 1511, 1456, 1419, 1334, 1158, 1110, 974, 938, 820, 734 cm⁻¹; MS (FAB) *m*/*z* 535 (M+H)

### Beispiel 187: N-[6'-(Chloro-difluoro-methyl)-3,5-dimethyl-3,4,5,6-tetrahydro-2H-[1,2']bipyridinyl-3'-ylmethyl]-2-(3-fluoro-4-methansulfonylamino-phenyl)-propionamid

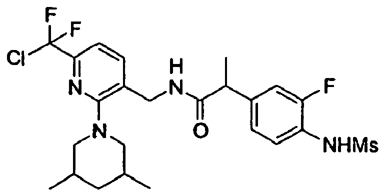

¹H NMR (300 MHz, CDCl₃) δ 7.45-7.54 (m, 2 H), 7.06-7.15 (m, 3 H), 6.62 (bs, 1 H), 6.31 (bt, 1 H), 4.46 (d, 2 H, J = 5.7 Hz), 3.54 (q, 1 H, J = 7.2 Hz), 3.25 (m, 2 H), 3.02 (s, 3 H), 2.36 (m, 2 H), 2.03 (m, 1 H), 1.53-1.65 (m, 3 H), 1.52 (d, 3 H, J = 7.2 Hz) 0.92 (d, 3 H, J = 6.6 Hz), 0.88 (d, 3 H, J = 6.6 Hz); IR (pur) 2926, 1653, 1591, 1511, 145, 1334, 1253, 1017, 967, 733 cm⁻¹; MS (FAB) *m*/*z* 548 (M+H)

### Beispiel 188: N-[2-(1,3-Dihydro-isoindol-2-yl)-6-trifluoromethyl-pyridin-3-ylmethyl]-2-(3-fluoro-4-methansulfonylamino-phenyl)-propionamid

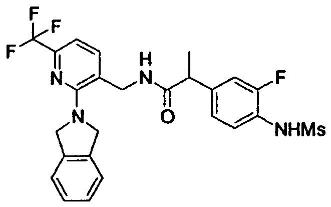

¹H NMR (300 MHz, CDCl₃) δ 7.46 (d, 1 H, J = 7. 5 Hz), 7.25-7.37 (m, 5 H), 7.01-7.10 (m, 3 H), 6.24 (bs, 1 H), 5.75 (bt, 1 H), 4. 84 (s, 4 H), 4.59 (d, 2 H, J = 5.7 Hz), 3.52 (q, 1 H, J = 7.2 Hz), 2.94 (s, 3 H), 1.49 (d, 3 H, J = 7.2 Hz); IR (KBr) 3298, 2922, 1650, 1512, 1457, 1425, 1334, 1155, 747 cm⁻¹; MS (FAB) *m*/*z* 537 (M+H)

### Beispiel 189: 3'-{[2-(3-Fluoro-4-methansulfonylamino-phenyl)-propionylamino]-methyl}-4-phenyl-6'-trifluoromethyl-3,4,5,6-tetrahydro-2H-[1,2']bipyridinyl-4-carbonsäure ethyl ester

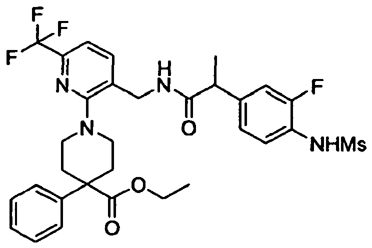

¹H NMR (300 MHz, CDCl₃) δ 7.28-7.50 (m, 7 H), 7.21 (d, 1 H, 7.8 Hz), 7.12 (dd, 1 H, J = 7.8, 2.1 Hz), 7.04 (d, 1 H, J = 8.1 Hz), 6.44 (bs, 1 H), 6.13 (bt, 1 H), 4.47 (d, 2 H, J = 5.7 Hz), 4.14 (q, 2 H, J = 7.2 Hz), 3.52 (q, 1 H, J = 6.9 Hz), 3.37 (m, 2 H), 2.98-3.05 (m, 5 H), 2.66 (m, 2 H), 1.99 (m, 2 H), 1.52 (d, 3 H, J = 7.2 Hz), 1.18 (t, 3 H, 6.9 Hz); IR (pur) 2927, 1721, 1654, 1512, 1455, 1336, 1159, 968 cm⁻¹; MS (FAB) *m*/*z* 651 (M+H)

### Beispiel 190: N-(4,6'-Bis-trifluoromethyl-3,4,5,6-tetrahydro-2H-[1,2']bipyridinyl-3'-ylmethyl)-2-(3-fluoro-4-methansulfonylamino-phenyl)-propionamid

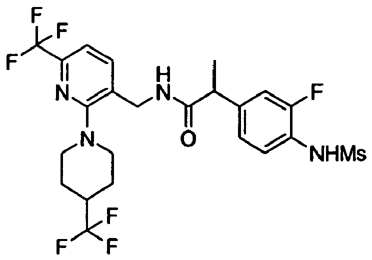

¹H NMR (300 MHz, CDCl₃) δ 7.47-7.51 (m, 2 H), 7.25 (d, 1 H, *J* = 7.8 Hz), 7.08-7.15 (m, 2 H), 6.34 (bs 1 H), 6.04 (bt, 1 H), 4.47 (d, 2 H, J = 5.7 Hz), 3.61 (q, 1 H, J = 6.9 Hz), 3.43 (m, 2 H), 3.01 (s, 3 H), 2.84 (t, 2 H, J = 11.1 Hz), 1.95 (m, 2 H), 1.66 (m, 1 H), 1.53 (d, 3 H, J = 6.9 Hz); IR (KBr) 2934, 1655, 1591, 1512, 1420, 1337, 1255, 1146, 1083, 960, 908 cm⁻¹; MS (FAB) *m*/*z* 571 (M+H)

### Beispiel 195: N-(4,6'-Bis-trifluoromethyl-3,4,5,6-tetrahydro-2H-[1,2']bipyridinyl-3'-ylmethyl)-2-(4-methansulfonylamino-3-methyl-phenyl)-propionamid

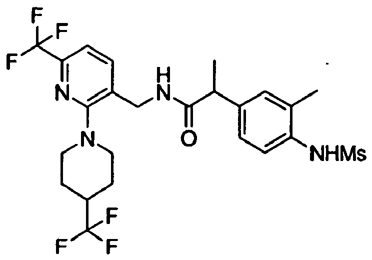

¹H NMR (300 MHz, CDCl₃) δ 7.48 (d, 1 H, J = 7.8 Hz), 7.38 (d, 1 H, J = 8.1 Hz), 7.23 (d, 1 H, J = 7.8 Hz), 7.11-7.15 (m, 2 H), 6.60 (bs, 1 H), 6.12 (bt, 1 H), 4.45 (d, 2 H, J = 5.7 Hz), 3.58 (q, 1 H, J = 6.9 Hz), 3.40 (m, 2 H), 3.01 (s, 3 H), 2.80 (m, 2 H), 2.30 (s, 3 H), 2.19 (m 1 H), 1.94 (m, 2 H), 1.62 (m, 2 H), 1.52 (d, 3 H, J = 6.9 Hz); IR (KBr) 2929, 1655, 1504, 1420, 1335, 1254, 1147, 1083, 959 cm⁻¹; MS (FAB) *m*/*z* 567 (M+H)

### Beispiel 196: 2-(4-Methansulfonylamino-3-methyl-phenyl)-N-(4-methyl-6'trifluoromethyl-3,4,5,6-tetrahydro-2H-[1,2']bipyridinyl-3'-ylmethyl)-propionamid

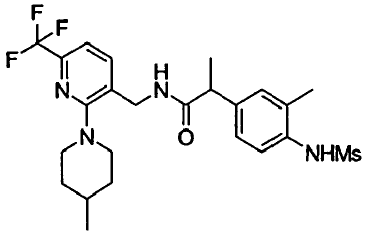

¹H NMR (300 MHz, CDCl₃) δ 7.46 (d, 1 H, J = 7.5 Hz), 7.39 (d, 1 H, J = 7.8 Hz), 7.18 (d, 1 H, J = 7.8 Hz), 7.11-7.14 (m, 2 H), 6.37 (bs, 1 H), 6.21 (bt, 1 H), 4.45 (d, 2 H, J = 5.7 Hz), 3.56 (q, 1 H, J = 6.9 Hz), 3.29 (m, 2 H), 3.01 (s, 3 H), 2.79 (m, 2 H), 2.29 (s, 3 H), 2.19 (m 1 H), 2.05 (m, 2 H), 1.69 (m, 2 H), 1.52 (d, 3 H, J= 6.9 Hz); IR (KBr) 3290, 2926, 1654, 1593, 1503, 1457, 1419, 1374, 1328, 1152, 970, 732 cm⁻¹; MS (FAB) *m*/*z* 513 (M+H)

### Beispiel 197: N-(4-Ethyl-6'-trifluoromethyl-3,4,5,6-tetrahydro-2H-[1,2']bipyridinyl-3'-ylmethyl)-2-(3-fluoro-4-methansulfonylamino-phenyl)-propionamid

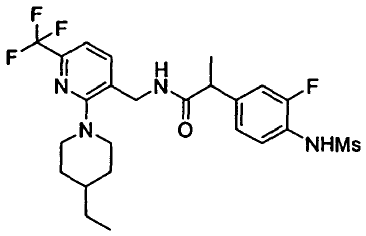

¹H NMR (300 MHz, CD₃OD) δ 7.47-7.52 (m, 2 H), 7.19 (d, 1 H, J = 7.8 Hz), 7.06-7.14 (m, 2 H), 6.69 (bs, 1 H), 6.37 (bt, 1 H), 4.47 (d, 2 H, J = 5.7 Hz), 3.56 (q, 1 H, J = 6.9 Hz), 3.33 (m, 2 H), 3.02 (s, 3 H), 2.80 (m, 2 H), 1.76 (m, 2 H), 1.52 (d, 3 H, J= 6.9 Hz), 1.21-1.32(m, 5 H), 0.91 (t, 3 H, J = 7.2 Hz); IR (KBr) 3288, 2929, 1655, 1591, 1512, 1419, 1336, 1275, 1158, 956, 910, 733 cm⁻¹; MS (FAB) *m*/*z* 531 (M+H)

### Beispiel 198: 2-(3-Fluoro-4-methansulfonylamino-phenyl)-N-(2-phenoxy-6-trifluoromethyl-pyridin-3-ylmethyl)-propionamid

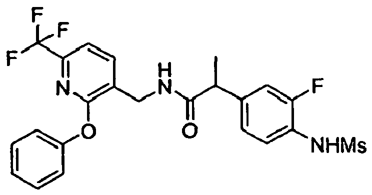

¹H NMR (300 MHz, CD₃OD) δ 7.75 (d, 1 H, J = 7.2 Hz), 7.00-7.49 (m, 9 H, J = 7.8 Hz), 6.26 (bt, 1 H), 4.51 (d, 2 H, J = 5.7 Hz), 3.56 (q, 1 H, J = 6.9 Hz), 3.01 (s, 3 H), 1.48 (d, 3 H, J = 6.9 Hz); IR (KBr) 3291, 2927, 1659, 1589, 1513, 1406, 1335, 1260, 1156, 972, 940, 835, 757 cm⁻¹; MS (FAB) *m*/*z* 512 (M+H)

### Beispiel 199: 2-(3-Fluoro-4-methansulfonylamino-phenyl)-N-(4-methoxymethyl-6'-trifluoromethyl-3,4,5,6-tetrahydro-2H-[1,2']bipyridinyl-3'-ylmethyl)-propionamid

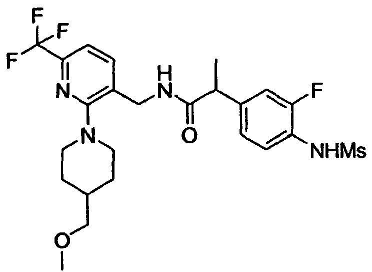

¹H NMR (300 MHz, CDCl₃) δ 7.46-7.52 (m, 2 H), 7.20 (d, 1 H, J = 7.8 Hz), 7.07-7.15 (m, 2 H), 6.82 (bs, 1 H), 6.37 (bt, 1 H), 4.46 (d, 2 H, J = 5.7 Hz), 3.58 (q, 1 H, J = 6.9 Hz), 3.26-3.38 (m, 5 H), 3.02 (s, 3 H), 2.82 (m, 2 H), 1.79 (m, 3 H), 1.51 (d, 3 H, J = 6.9 Hz), 1.25-1.30 (m, 4 H); IR (KBr) 3289, 2927, 1657, 1592, 1512, 1455, 1420, 1375, 1335, 1275, 1157, 971, 832, 753 cm⁻¹; MS (FAB) *m*/*z* 547 (M+H)

### Beispiel 200: 2-(3-Fluoro-4-methansulfonylamino-phenyl)-N-[4-(4-fluoro-phenyl)-6'-trifluoromethyl-3,4,5,6-tetrahydro-2H-[1,2']bipyridinyl-3'-ylmethyl]-propionamid

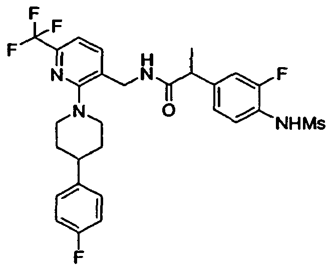

¹H NMR (300 MHz, CDCl₃) δ 7.46-7.51 (m, 2 H), 6.97-7.25 (m, 7 H), 6.72 (bs 1 H), 6.24 (bt, 1 H), 4.50 (d, 2 H, J = 5.7 Hz), 3.59 (q, 1 H, J = 6.9 Hz), 3.45 (m, 2 H), 3.00 (s, 3 H), 2.93 (m, 2 H), 1.92 (m, 2 H), 1.76 (m, 3 H), 1.51 (d, 3 H, J = 6.9 Hz); IR (KBr) 2927, 1653, 1511, 1455, 1420, 1336, 1224, 1159, 959, 833, 732 cm⁻¹; MS (FAB) *m*/*z* 597 (M+H)

### Beispiel 201: 2-(3-Fluoro-4-methansulfonylamino-phenyl)-N-{2-[4-(2-fluoro-phenyl)-piperazin-1-yl]-6-trifluoromethyl-pyridin-3-ylmethyl}-propionamid

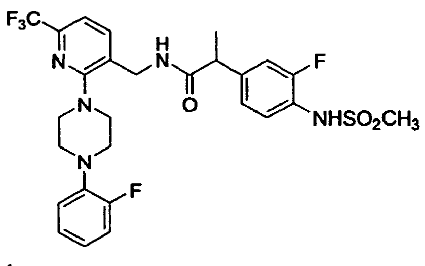

¹H NMR (300 MHz, CDCl₃) δ 7.53 (d, 1 H, *J* = 8.1 Hz), 7.52 (dd, 1 H, *J* = 8.3, 8.3 Hz), 7.27 (d, 1 H, *J* = 8.0 Hz), 7.11 (m, 4 H), 6.98 (m, 2 H), 6.40 (bs, 1H), 6.16 (bt, 1 H), 4.53 (d, 2 H, *J* = 4.6 Hz), 3.58 (q, 1 H, *J* = 7.3 Hz), 3.32-3.28 (m , 4 H), 3.18-3.15 (m, 4 H), 3.01 (s, 3 H), 1.54 (d, 3 H, *J* = 7.0 Hz); IR (pur) 2391, 2846, 1707, 1657, 1591, 1504, 1453, 1417, 1336, 1235, 1157, 968, 835, 757 cm⁻¹; MS (FAB) *m*/*z* 598 (M+H)

### Beispiel 202: 2-(3-Fluoro-4-methansulfonylamino-phenyl)-N-(2-(pyridin-2-ylmethoxy)-6-trifluoromethyl-pyridin-3-ylmethyl]-propionamid

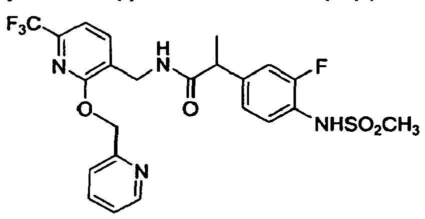

¹H NMR (300 MHz, CDCl₃) δ 8.61 (d, 1 H, *J* = 4.7 Hz), 7.74 (dd, 1 H, *J* = 7.6, 1.7 Hz), 7.67 (d, 1 H, *J* = 7.3 Hz), 7.42 (dd, 1 H, *J* = 8.2, 8.2 Hz), 7.43 (d, 1 H, *J* = 7.7 Hz), 7.33 (m, 1 H), 7.24 (d, 1 H, *J* = 7.5 Hz), 7.09 (dd, 1 H, *J* = 11.4, 2.0 Hz), 7.01 (d, 1 H, *J* = 8.2 Hz), 6.54 (bs, 1 H), 5.51 (m, 2 H), 4.45 (d, 2 H, *J* = 5.7 Hz), 3.58 (q, 1 H, *J* = 7.0 Hz), 3.01 (s, 3 H), 1.46 (d, 3 H, *J* = 7.0 Hz); IR (pur) 3271, 1656, 1598, 1512, 1417, 1335, 1273, 1155, 973, 935, 835, 761 cm⁻¹; MS (FAB) *m*/*z* 527 (M+H)

### Beispiel 203: 2-(4-Methansulfonylamino-3-methyl-phenyl)-N-[2-(4-phenyl-piperazin-1-yl)-6-trifluoromethyl-pyridin-3-ylmethyl]-propionamid

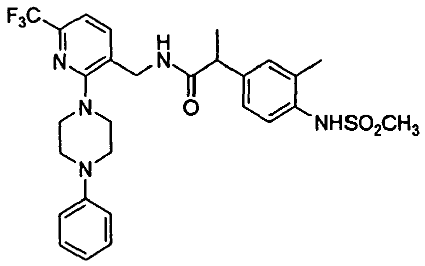

¹H NMR (300 MHz, CDCl₃) δ 7.53 (d, 1 H, *J* = 7.7 Hz), 7.32 (m, 4 H), 7.14 (s, 1 H), 7.13 (d, 1 H, *J* = 7.0 Hz), 6.92 (m, 3 H), 6.18 (bt, 1 H), 5.89 (bs, 1 H), 4.53 (d, 2 H, *J* = 5.7 Hz), 3.56 (q, 1 H, *J* = 7.1 Hz), 3.27-3.15 (m, 8 H), 2.98 (s, 3 H), 2.23 (s, 3 H), 1.51 (d, 3 H, *J* = 7.1 Hz); IR (pur) 2920, 1652, 1596, 1503, 1418, 1331, 1231, 1150, 967, 761 cm⁻¹; MS (FAB) *m*/*z* 576 (M+H)

### Beispiel 204: N-(2-Benryloxy-6-trifluormethyl-pyridin-3-ylmethyl)-2-(4-methansulfonylamino-3-methyl-phenyl)-propionamid

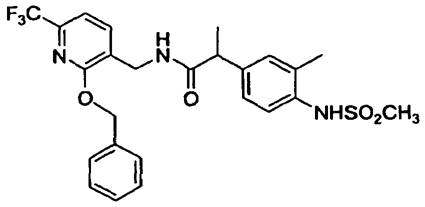

¹H NMR (300 MHz, CDCl₃) δ 7.60 (d, 1 H, *J* = 7.5 Hz), 7.39-7.33 (m, 6 H), 7.22 (d, 1 H, *J* = 7.3 Hz), 7.05 (s, 1 H), 7.04 (d, 1 H, *J* = 7.5 Hz), 6.10 (bs, 1 H), 5.91 (bt, 1 H), 5.37 (q, 2 H, *J* = 12.5 Hz), 4.37 (d , 2 H, *J* = 6.1 Hz), 3.43 (q, 1 H, *J* = 7.1 Hz), 3.00 (s, 3 H), 2.24 (s, 3 H), 1.43 (d, 3 H, *J* = 7.1 Hz); IR (pur) 3295, 2925, 1655, 1505, 1459, 1420, 1356, 1326, 1151, 977, 756 cm⁻¹; MS (FAB) *m*/*z* 522 (M+H)

### Beispiel 205: 2-(3-Fluoro-4-methansulfonylamino-phenyl)-N-[2-(methyl-phenyl-amino)-6-trifluoromethyl-pyridin-3-ylmethyl]-propionamid

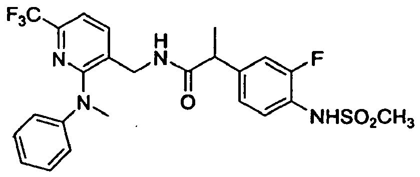

¹H NMR (300 MHz, CDCl₃) δ 7.55 (d, 1 H, *J* = 7.9 Hz), 7.53 (dd, 1 H, *J* = 8.2, 8.2 Hz) 7.29-7.23 (m, 3 H), 7.10-7.01 (m, 3 H), 6.83 (m, 2 H), 6.48(bs, 1 H), 5.42 (bt, 1 H), 3.88 (d, 2 H, *J* = 6.0 Hz), 3.38 (s, 3 H), 3.37 (q, 1 H, *J* = 7.1 Hz), 3.04 (s, 3 H), 1.43 (d, 3 H, *J* = 7.1 Hz); IR (pur) 2923, 1654, 1590, 1509, 1462, 1398, 1338, 1270, 1156, 973, 929, 758 cm⁻¹; MS (FAB) *m*/*z* 525 (M+H)

### Beispiel 206: 2-(3-Fluoro-4-methansulfonylamino-phenyl)-N-[6-trifluoromethyl-2-(4-trifluorornethyl-benzyloxy)-pyridin-3-ylmethyl]-propionamid

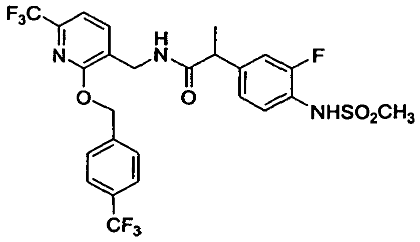

¹H NMR (300 MHz, CDCl₃) δ 7.64 (d, 1 H, *J* = 8.4 Hz), 7.61 (d, 2 H, *J* = 8.6 Hz), 7.55 (d, 2 H, *J* = 8.1 Hz), 7.49 (dd, 1 H, *J* = 8.2, 8.2 Hz), 7.25 (d, 1 H, *J* = 8.3 Hz), 7.07 (dd, 1 H, *J* = 11.2, 2.0 Hz), 7.01 (d , 1 H, *J* = 7.9 Hz), 6.43 (bs, 1 H), 5.89 (bt, 1 H), 5.46 (m, 2 H), 4.42 (d, 2H, *J* = 6.0 Hz), 3.48 (q, 1 H, *J* = 7.1 Hz), 3.02 (s, 3 H), 1.45 (d, 3 H, *J*= 7.1 Hz); IR (pur) 3369, 1657, 1511, 1419, 1326, 1267, 1159, 1119, 1067, 975, 934, 826 cm⁻¹, MS (FAB) *m*/*z* 594 (M+H)

### Beispiel 207: N-{6-(Chloro-difluoro-methyl)-2-[4-(1-vinyl-propenyl)-piperazin-1-yl]-pyridin-3-ylmethyl)-2-(3-fluoro-4-methansulfonylamino-phenyl)-propionamid

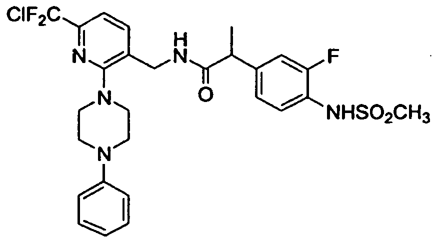

¹H NMR (300 MHz, CD₃OD) δ 8.60 (m, 1 H), 7.58 (d, 1 H, *J* = 7.5 Hz), 7.25-7.47 (m, 4 H), 7.00-7.25 (m, 4 H), 4.35-4.57 (m, 2 H), 3.73 (m, 1 H, *J* = 7.1 Hz), 3.32-3.45 (m, 8 H), 2.95 (s, 3 H), 1.47 (d, 3 H, *J* = 7.1 Hz); IR (KBr) 2919, 1646, 1592, 1506, 1446, 1332 cm⁻¹; MS (FAB) *m*/*z* 596 (M+H)

### Beispiel 208: N-[6-(Chloro-difluoro-methyl)-2-isobutoxy-pyridin-3-ylmethyl]-2-(3-fluoro-4-methansulfonylamino-phenyl)-propionamid

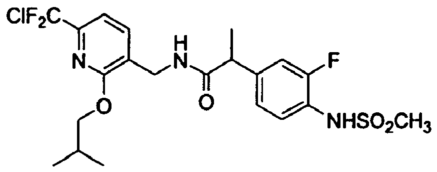

¹H NMR (300 MHz, CDCl₃) δ 7.48-7.58 (m, 2 H), 7.16 (d, 1 H, *J* = 7.5 Hz), 7.11 (m, 1 H), 7.06 (m, 1 H), 6.46 (m, 1 H), 5.95 (bt, 1 H), 4.32-4.44 (m, 2 H), 4.06-4.19 (m, 2 H), 3.51 (q, 1 H, *J* = 7.1 Hz), 3.04 (s, 3 H), 2.05 (m, 1 H), 1.49 (d, 3 H, *J* = 7.1 Hz), 0.99 (d, 6 H, *J* = 6.8 Hz); IR (KBr) 3291, 2964, 1654, 1601, 1512, 1424, 1335, 1267, 1159, 1114, 1012, 971, 881, 824 cm⁻¹; MS (FAB) *m*/*z* 508 (M+H)

### Beispiel 209: N-(2-Benzyloxy-4-trifluoromethyl-benzyl)-2-(3-fluoro-4-methansulfonylamino-phenyl)-propionamid

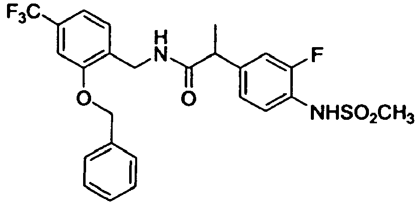

¹H NMR (300 MHz, CDCl₃) δ 7.44 (dd, 1 H, *J* = 8.4, 8.4 Hz), 7.41-7.34 (m, 5 H), 7.33 (d, 1 H, *J* = 8.6 Hz), 7.19 (d, 1 H, *J =* 7.9 Hz), 7.14 (s, 1 H), 7.05 (dd, 1 H, *J* = 11.3, 2.0 Hz), 6.95 (d , 1 H, *J* = 6.4 Hz), 6.41 (bs, 1 H), 5.94 (bt, 1 H), 5.08 (s, 2 H), 4.46 (m, 2 H), 3.41 (q, 1 H, *J* = 7.0 Hz), 2.99 (s, 3 H), 1.43 (d, 3 H, *J* = 7.1 Hz); IR (pur) 3292, 1652, 1510, 1426, 1329, 1240, 1159, 1122, 907, 742 cm⁻¹; MS (FAB) *m*/*z* 525 (M+H)

### Beispiel 210: N-(4,4-Dimethyl-6'-trifluoromethyl-3,4,5,6-tetrahydro-2H-[1,2']bipyridinyl-3'-ylmethyl)-2-(3-fluoro-4-methansulfonylamino-phenyl)-propionamid

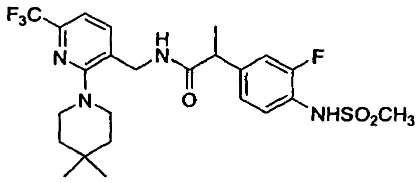

¹H NMR (300 MHz, CDCl₃) δ 7.53 (dd, 1 H, *J* = 8.4, 8.4 Hz), 7.48 (d, 1 H, *J* = 7.6 Hz), 7.21 (d, 1 H, *J* = 7.5 Hz), 7.14 (dd, 1 H, *J* =11.4, 1.9 Hz), 7.09 (d, 1 H, *J* = 8.8 Hz), 6.47 (bs, 1 H), 6.26 (bt, 1 H), 4.47 (d, 2 H, *J* = 5.0 Hz), 3.56 (q, 1 H, *J* = 7.1 Hz), 3.08-3.04 (m, 4 H), 3.03 (s, 3 H), 1.53 (d, 3 H, *J* = 7.1 Hz), 1.48-1.43 (m, 4 H), 0.99 (s, 6 H); IR (pur) 3289, 2922, 1709, 1655, 1591, 1512, 1457, 1420, 1336, 1159, 957, 834, 763 cm⁻¹; MS (FAB) *m*/*z* 531 (M+H)

### Beispiel 211: 2-(3-Fluoro-4-methansulfonylamino-phenyl)-N-[2-(pyridin-3-ylmethoxy)-6-trifluoromethyl-pyridin-3-ylmethyl]-propionamid

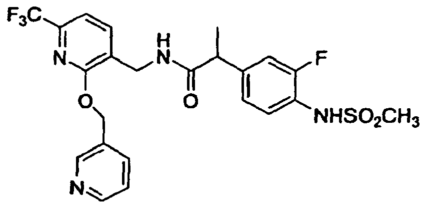

¹H NMR (300 MHz, CDCl₃) δ 8.69 (m, 2 H), 7.79(d, 1 H, *J* = 7.7 Hz), 7.64 (d, 1 H, *J* = 7.5 Hz), 7.48 (dd, 1 H, *J* = 8.1 Hz), 7.34 (m, 1 H), 7.25 (d, 1 H, *J* = 7.5 Hz), 7.05 (dd, 1 H, *J* = 11.4, 1.9 Hz), 7.02 (d , 1 H, *J* = 8.4 Hz), 5.87 (bt, 1 H), 5.41 (s, 2 H), 4.38 (d, 2 H, *J* = 6.2 Hz), 3.49 (q, 1 H, *J* = 7.3 Hz), 3.04 (s, 3 H), 1.46 (d, 3 H, *J* = 7.1 Hz); IR (pur) 3299, 1658, 1601, 1511, 1416, 1335, 1267, 1156, 974, 740 cm⁻¹; MS (FAB) *m*/*z* 527 (M+H)

### Beispiel 212: 2-(3-Fluoro-4-methansulfonylamino-phenyl)-N-{6-trifluoromethyl-2-[4-(3-trifluoromethyl-pyridin-2-yl)-piperazin-1-yl]-pyridin-3-ylmethyl}-propionamid

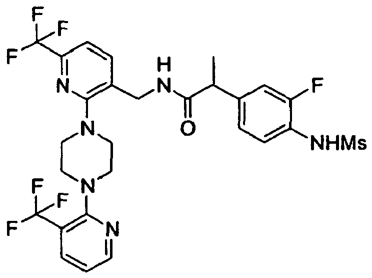

¹H NMR (300 MHz, CDCl₃) δ 8.50 (d, 1 H, J = 3.6 Hz), 7.91 (dd, 1 H, J = 7.8, 1.8 Hz), 7. 54 (d, 1 H, J = 6.9 Hz), 7.49 (dd, 1 H, J = 8.1, 8.1 Hz), 7.05-7.17 (m, 4 H), 6.40 (bt, 1 H), 4.52 (d, 2 H, J = 5.7 Hz), 3.61 (q, 1 H, J = 6. 9 Hz), 3.35 (m, 8 H), 3.02 (s, 3 H), 1.53 (d, 3 H, J = 6.9 Hz); IR (KBr) 3290, 2851, 1657, 1590,

### Beispiel 216: 2-(3-Fluoro-4-methansulfonylamino-phenyl)-N-[2-(4-phenyl-piperazin-1-yl)-4-trifluoromethyl-benzyl]-propionamid)

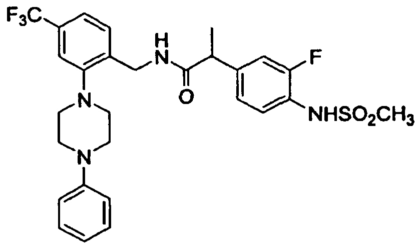

¹H NMR (300 MHz, CDCl₃) δ 7.46 (dd, 1 H, *J* = 8.4, 8.4 Hz), 7.28-7.35 (m, 5 H), 7.13 (m, 1 H), 7.07 (d , 1 H, *J* = 8.4 Hz), 6.89-6.99 (m, 3 H), 6.32 (bt, 1 H), 4.53-4.67 (m, 2 H), 3.55 (q, 1 H, *J* = 7.1 Hz), 3.20-3.28 (m, 4 H), 3.00-3.08 (m, 4 H), 2.98 (s , 3 H), 1.51 (d, 3 H, *J* = 6.9 Hz); IR (KBr) 2829, 1652, 1598, 1506, 1425, 1335, 1230, 1159, 1122, 962, 911, 733cm⁻¹; MS (FAB) *m*/*z* 579 (M+H)

### Beispiel 217: N-(2-Azocan-1-yl-4-trifluoromethyl-benzyl)-2-(3-fluoro-4-methansulfonylamino-phenyl)-propionamid

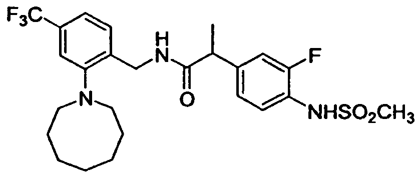

¹H NMR (300 MHz, CDCl₃) δ 7.52 (dd, 1 H, *J* = 8.3, 8.3 Hz), 7.38 (s, 1 H), 7.22-7.25 (m, 2 H), 7.15 (dd, 1 H, *J* = 11.2, 2.0 Hz), 7.11 (d, 1 H, *J* = 8.1 Hz), 6.51 (bs, 1 H), 6.01 (bt , 1 H), 4.55 (d, 2 H, *J* = 5.7 Hz), 3.55 (q, 1 H, *J* = 7.5 Hz), 3.00-3.05 (m, 7 H), 1.62-1.72 (m, 10 H), 1.53 (d , 3 H, *J* = 7.1 Hz); IR (KBr) 3289, 2926, 1651, 1510, 1420, 1334, 1214, 1160, 1122, 975, 908, 732 cm⁻¹; MS (FAB) *m*/*z* 530 (M+H)

### Beispiel 218: N-[2-(4,4-Dimethyl-piperidin-1-yl)-4-trifluoromethyl-benzyl]-2-(3-fluoro-4-methansulfonylamino-phenyl)-propionamid

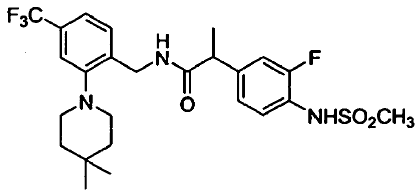

¹H NMR (300 MHz, CDCl₃) δ 7.51 (dd, 1 H, *J* = 8.3, 8.3 Hz), 7.32 (s, 1 H), 7.21-7.30 (m, 2 H), 7.14 (dd, 1 H, *J* = 11.2,1.8 Hz), 7.08 (d, 1 H, *J* = 8.6 Hz), 6.52 (bs, 2 H), 4.45-4.60 (m, 2 H), 3.54 (q, 1 H, *J* = 7.1 Hz), 3.02 (s, 3 H), 2.75-2.85 (m, 4 H), 1.52 (d, 3 H, *J* = 7.1 Hz), 1.42-1.50 (m, 4 H), 1.00 (s, 6 H); IR (KBr) 3284, 2922, 1652, 1509, 1424, 1337, 1224, 1160, 1122, 1078, 973, 894, 827, 758 cm⁻¹; MS (FAB) *m*/*z* 530 (M+H)

### Beispiel 219: 2-(3-Fluoro-4-methansulfonylamino-phenyl)-N-[2-(4-p-tolyl-piperazin-1-yl)-6-trifluoromethyl-pyridin-3-ylmethyl]-propionamid

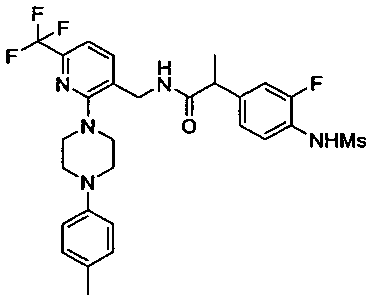

¹H NMR (300 MHz, CDCl₃) δ 7.55 (m, 1 H), 7.41 (d, 1 H, J = 7.8 Hz), 7.24-7.32 (m, 2 H), 6.96-7.12 (m, 4 H), 6. 78-6.81 (m, 3 H), 4.40 (d, 2 H, J = 5.7 Hz), 3.56 (q, 1 H, J = 6.9 Hz), 3.12 (m, 8 H), 2.86 (s, 3 H), 2.18 (s, 3 H), 1.40 (d, 3 H, J= 6.9 Hz); IR (KBr) 3292, 2923, 1659, 1591, 1514, 1418, 1374, 1335, 1235, 1155, 968, 817, 757 cm⁻¹, MS (FAB) *m*/*z* 594(M+H)

### Beispiel 220: 2-(3-Fluoro-4-methansulfonylamino-phenyl)-N-[2-(4-m-tolyl-piperazin-1-yl)-6-trifluoromethyl-pyridin-3-ylmethyl]-propionamid

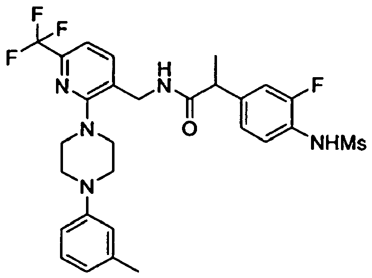

¹H NMR (300 MHz, CDCl₃) δ 7.45-7.54 (m, 2 H), 7.06-7.28 (m, 4 H), 6.73-6.76 (m, 3 H), 6.28(bs, 1 H), 6.20 (bt, 1 H), 4.52 (d, 2 H, J = 5.7 Hz), 3.56 (q, 1 H, J = 6.9 Hz), 3.24 (m, 8 H), 2.98 (s, 3 H), 2.35 (s, 3 H), 1.52 (d, 3 H, J= 6.9 Hz); IR (KBr) 3292, 2923, 1659, 1591, 1514, 1418, 1374, 1335, 1235, 1155, 968, 817, 757 cm⁻¹; MS (FAB) *m*/*z* 594 (M+H)

### Beispiel 221: 2-(3-Fluoro-4-methansulfonylamino-phenyl)-N-{2-[4-(4-methoxy-phenyl)-piperazin-1-yl]-6-trifluoromethyl-pyridin-3-ylmethyl}-propionamid

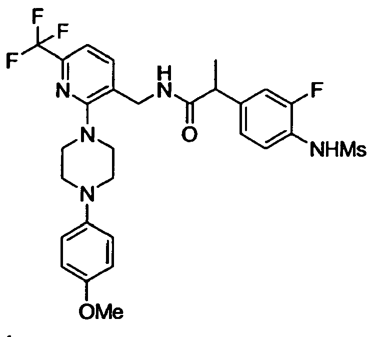

¹H NMR (300 MHz, CDCl₃) δ 7.45-7.54 (m, 2 H), 7.26 (d, 1 H, J = 7.5 Hz), 7.06-7.14 (m, 2 H), 6.85-6.93 (m, 4 H), 6.41 (bs, 1 H), 6.23 (bt, 1 H), 4.53 (d, 2 H, J = 5.7 Hz), 3.79 (s, 3 H), 3.56 (q, 1 H, J = 6.9 Hz), 3.79 (m, 4 H), 3,12 (m, 4 H), 2.99 (s, 3 H), 1.51 (d, 3 H, J = 6.9 Hz); IR (KBr) 3294, 2839, 1659, 1591, 1511, 1418, 1335, 1242, 1155, 1034, 968, 828, 757 cm⁻¹; MS (FAB) *m*/*z* 610 (M+H)

### Beispiel 222: 2-(3-Fluoro-4-methansulfonylamino-phenyl)-N-{6-trifluoromethyl-2-[4-(4-trifluoromethyl-phenyl)-piperazin-1-yl]-pyridin-3-ylmethyl}propionamid

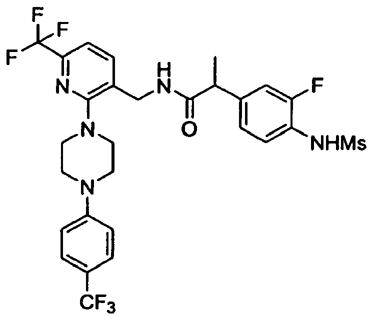

¹H NMR (300 MHz, CD₃OD δ 7.39-7.52 (m, 5 H), 7.27 (d, 1 H, J = 7.8 Hz), 7.11-7.20 (m, 2 H), 4.46 (d, 2 H, J = 5.7 Hz), 3.67 (q, 1 H, J = 6.9 Hz), 3.33-3.38 (m, 8 H), 3.00 (s, 3 H), 1.53 (d, 3 H, J = 6.9 Hz); IR (KBr) 3295, 2922, 1647, 1616, 1514, 1416, 1331, 1234, 1156, 1115, 968, 829, 757 cm⁻¹ ; MS (FAB) *m*/*z* 648 (M+H)

### Beispiel 223: N-(2-Benzyloxy-6-tert-butyl-4-hydroxymethyl-pyridin-3-ylmethyl)-2-(3-fluoro-4-methansulfonylamino-phenyl)-propionamid

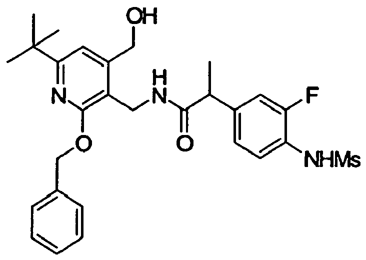

¹H NMR (300 MHz, CDCl₃) δ 7.35-7.52 (m, 6 H), 6.88-6.99 (m, 3 H), 6.54 (bs 1 H), 6.32 (bt, 1 H), 5. 46 (d, 1 H, J = 16.3 Hz), 5.34 (d, 1 H, J = 16.3 Hz), 4.65 (m, 2 H), 4.35 (d, 2 H, J = 5.7 Hz), 3.32 (q, 1 H, J = 6.9 Hz), 2.98 (s, 3 H), 1.35 (d, 3 H, J = 6.9 Hz), 1.30 (s, 9 H); IR (KBr) 3368, 2960, 1648, 1592, 1512, 1449, 1398, 1333, 1157, 1027, 973, 756cm⁻¹; MS (FAB) *m*/*z* 544 (M+H)

### Beispiel 225: 2-(3-Fluoro-4-methansulfonylamino-phenyl)-N-(2-pentyloxy-6-trifluoromethyl-pyridin-3-ylmethylrpropionamid

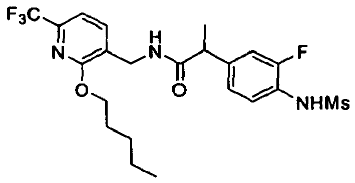

¹H NMR (CDCl₃) δ 7.57(d, 1 H, J=7.5Hz), 7.52(dd, 1 H, J=8.2, 8.2Hz), 7.19(d, 1 H, J=7.4Hz), 7.12-7.05(m, 2H), 6.45(bs, NH), 5.98(bt, NH), 4.38-4.29(m, 4H), 3.51(q, 1 H, J=7.0Hz), 3.03(s, 3H), 1.77-1.67(m, 2H), 1.49(d, 3H, J=7.1 Hz), 1.43-1.35(m, 4H), 0.93(t, 3H, J=7.1 Hz); IR(pur) 3287, 2959, 1656, 1604, 1513, 1464, 1425, 1337, 1269, 1157, 977 cm⁻¹ ; Mass (FAB) m/z 506[M+H]

### Beispiel 226: 2,2-Dimethyl-propionsäure 3'-{[2-(3-ftuoro-4-methansulfonylamino-phenyl)-propionylamino]-methyl}-6'-trifluoromethyl-3,4,5,6-tetrahydro-2H-[1,2']bipyridinyl-4-yl ester

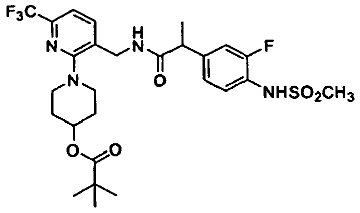

¹H NMR (CDCl₃) δ 7.54-7.47 (m, 2 H), 7.22 (d, 1 H, J = 7.7 Hz), 7.15 (dd, 1 H, J = 11.0, 1.8 Hz), 7.10 (m, 1 H), 6.49 (bs, NH), 6.01 (bt, NH), 4.94 (m, 1 H), 4.47 (d, 2 H, J = 6.0 Hz), 3.58 (q, 1 H, J = 7.0 Hz), 3.32-3.22 (m, 2 H), 3.13-3.03 (m, 2 H), 3.04 (s, 3 H), 2.00-1.90 (m, 2 H), 1.82-1.70 (m, 2 H), 1.55 (d, 3 H, J = 7.1 Hz), 1.21 (s, 9 H); IR (pur) 3300, 2973, 1715, 1656, 1513, 1420, 1336, 1284, 1163, 759 cm⁻¹; MS (FAB) m/z 603 (M+H)

### Beispiel 227: 2-(3-Fluoro-4-methansulfonylamino-phenyl)-N-(4-oxo-6'-trifluoromethyl-3,4,5,6-tetrahydro-2H-[1,2]bipyridinyl-3'-ylmethyl)-propionamid 3'-ylmethyl)-propionamid

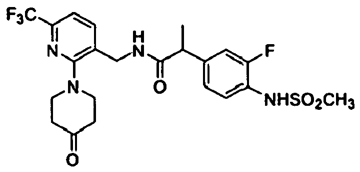

¹H NMR (CDCl₃) δ 7.55-7.49 (m, 2 H), 7.29 (d, 1 H, J = 7.9 Hz), 7.17 (dd, 1 H, J = 11.2, 2.0 Hz), 7.11 (d, 1 H, J = 8.6 Hz), 6.70 (bs, NH), 6.04 (bt, NH), 4.54 (d, 2 H, J = 5.7 Hz), 3.61 (q, 1 H, J = 7.0 Hz), 3.49 (t, 4 H, J = 6.0 Hz), 3.04 (s, 3 H), 2.55 (t, 4 H, J = 6.1 Hz), 1.55 (d, 3 H, J = 7.1 Hz); IR (pur) 3294, 1712, 1658, 1592, 1513, 1418, 1335, 1156, 733 cm⁻¹; MS (FAB) m/z 517 (M+H)

### Beispiel 228: N-(4-Ethoxy-6'-trifluoromethyl-3,4,5,6-tetrahydro-2H-[1,2']bipyridinyl-3'-ylmethyl)-2-(3-fluoro-4-methansulfonylamino-phenyl)-propionamid

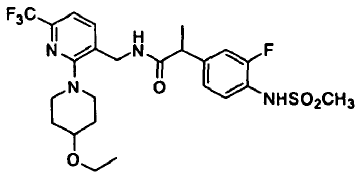

¹H NMR (CDCl₃) δ 7.55-7.48 (m, 2 H), 7.23 (d, 1 H, J = 7.7 Hz), 7.15-7.08 (m, 2 H), 6.54 (bs, NH), 6.23 (bt, NH), 4.48 (d, 2 H), 3.58-3.23 (m, 6 H), 3.04 (s, 3 H), 2.94-2.86 (m, 2 H), 2.05-1.95 (m , 2 H), 1.63-1.50 (m, 2 H), 1.53 (d, 3 H, J = 7.1 Hz), 1.24 (t, 3 H, J = 7.0 Hz); IR (pur) 3290, 2929, 1655, 1513, 1419, 1335, 1158 cm⁻¹; MS (FAB) m/z 547 (M+H)

### Beispiel 229: N-[2-(4-Ethyl-piperidin-1-yl)-4-trifluoromethyl-benzyl]-2-(3-fluoro-4-methansulfonylamino-phenyl)-propionamid

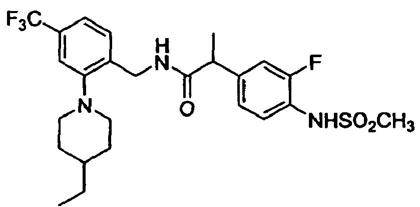

¹H NMR (300 MHz, CDCl₃) δ 7.51 (dd, 1 H, *J* = 8.3, 8.3 Hz), 7.29 (s, 1 H), 7.21-7.27 (m , 2 H), 7.14 (m, 1 H), 7.08 (d, 1 H, *J* = 8.4 Hz), 6.48-6.59 (m , 2 H), 4.46-4.60 (m , 2 H), 3.53 (q, 1 H, *J* = 6.9 Hz), 2.91-3.07 (m, 5 H), 2.58-2.61 (m, 2 H), 1.75-1.86 (m, 2 H), 1.52 (d, 3 H, *J* = 7.1 Hz), 1.10-1.37 (m, 5 H), 0.93 (t, 3 H, *J* = 7.0 Hz); IR (KBr) 3285, 2930, 1652, 1509, 1423, 1336, 1160, 1122, 973, 910, 733 cm⁻¹; MS (FAB) *m*/*z* 530 (M+H)

### Beispiel 230: 2-(3-Fluoro-4-methansulfonylamino-phenyl)-N-[4-trifluoromethyl-2-(4-trifluoromethyl-piperidin-1-yl)-benzyl]-propionamid

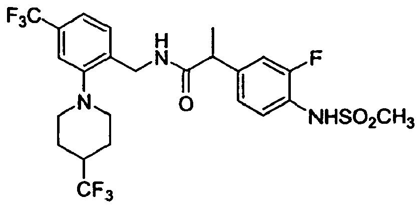

¹H NMR (300 MHz, CDCl₃) δ 7.52 (dd, 1 H, *J* = 8.2, 8.2 Hz), 7.22-7.32 (m, 3 H), 7.12-7.19 (m, 1 H), 7.09 (d, 1 H, *J* = 8.3 Hz), 6.52 (bs, 1 H), 6.12 (bt, 1 H), 4.52 (d, 2 H, *J* = 5.9 Hz), 3.56 (q, 1 H, *J* = 7.1 Hz), 3.05-3.15 (m, 2 H), 3.03 (bs, 3 H), 2.62-2.77 (m, 2 H), 2.08-2.24 (m, 1 H), 1.93-2.02 (m, 2 H), 1.61-1.74 (m, 2 H), 1.54 (d , 3 H, *J* = 7.1 Hz); IR (KBr) 3289, 2936, 1653, 1510, 1425, 1334, 1254, 1158, 1081, 972, 907, 825, 733 cm⁻¹; MS (FAB) *m*/*z* 570 (M+H)

### Beispiel 231: N-[2-(4-Benzyl-piperidin-1-yl)-4-trifluoromethyl-benzyl]-2-(3-fluoro-4-methansulfonylamino-phenyl)-propionamid

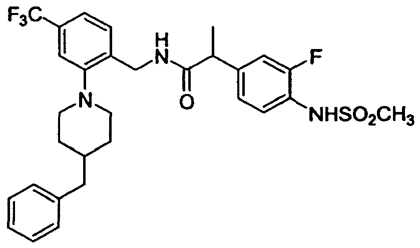

¹H NMR (300 MHz, CDCl₃) δ 7.52 (dd, 1 H, *J* = 8.2, 8.2 Hz), 7.27-7.35 (m , 3 H), 7.20-7.26 (m , 2 H), 7.11-7.19 (m , 4 H), 7.08 (d , 1 H, *J* = 8.2 Hz), 6.38-6.46 (m, 2 H), 4.51 (d, 2 H, *J* = 5.7 Hz), 3.52 (q, 1 H, *J* = 7.1 Hz), 2.85-3.05 (m, 5 H), 2.55-2.70 (m , 4 H), 1.60-1.80 (m, 3 H), 1.52 (d, 3 H, *J* = 7.1 Hz), 1.21-1.38 (m, 2 H); IR (KBr) 3292, 2923, 1652, 1509, 1424, 1336, 1160, 1121, 973, 909, 734, 701 cm⁻¹; MS (FAB ) *m*/*z* 592 (M+H)

### Beispiel 233: N-(6-tert-Butyl-2-cyclohexyloxy-pyridin-3-ylmethyl)-2-(3-fluoro-4-methansulfonylamino-phenyl)-propionamid

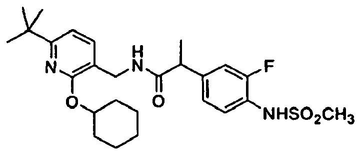

¹H NMR (CDCl₃) δ 7.50 (dd, 1 H, J = 8.3, 8.3 Hz), 7.36 (d, 1 H, J = 7.3 Hz), 7.14-7.05 (m, 2 H), 6.77 (d, 1 H, J = 7.5 Hz), 6.70 (bs, NH), 6.14 (bt, NH), 5.10 (m, 1 H), 4.39-4.23 (m, 2 H), 3.49 (q, 1 H, J = 7.1 Hz), 3.02 (s, 3 H), 1.92 (m, 2 H), 1.71 (m, 2 H), 1.60-1.25 (m, 6 H), 1.48 (d, 1 H, J = 7.1 Hz), 1.28 (s, 9 H); IR (pur) 3288, 2935, 2859, 1652, 1585, 1513, 1451, 1406, 1335, 1254, 1159, 733 cm⁻¹; MS (FAB) m/z 506 (M+H)

### Beispiel 234: N-(6-tert-Butyl-2-cyclopentyloxy-pyridin-3-ylmethyl)-2-(3-fluoro-4-methansulfonylamino-phenyl)-propionamid

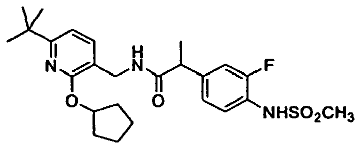

¹H NMR (CDCl₃) δ 7.50 (dd, 1 H, J = 8.3, 8.3 Hz), 7.35 (d, 1 H, J = 7.7 Hz), 7.14-7.05 (m, 2 H), 6.77 (d, 1 H, J = 7.5 Hz), 6.59 (bs, NH), 6.03 (bt, NH), 5.44 (m, 1 H), 4.36-4.21 (m, 1 H), 3.48 (q, 1 H, J = 6.8 Hz), 3.02 (s, 3 H), 1.96 (m, 2 H), 1.75-1.60 (m, 6 H), 1.48 (d, 3 H, J = 7.1 Hz), 1.29 (s, 9 H); IR (pur) 3291, 2962, 1652, 1513, 1452, 1406, 1337, 1255, 1159, 982 cm⁻¹ MS (FAB) m/z 492 (M+H)

### Beispiel 235: N-(2-Butoxy-6-tert-butyl-pyridin-3-ylmethyl)-2-(3-fluoro-4-methansulfonylamino-phenyl)-propionamid

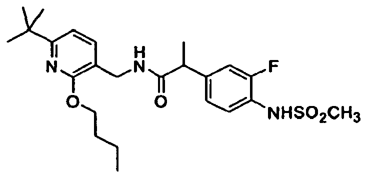

¹H NMR (CDCl₃) δ 7.50 (dd, 1 H, J = 8.4, 8.4 Hz), 7.36 (d, 1 H, J = 7.5 Hz), 7.15-7.05 (m, 2 H), 6.79 (d, 1 H, J = 7.5 Hz), 6.59 (bs, NH), 6.06 (bt, NH), 4.39-4.23 (m, 4 H), 3.48 (q, 1 H, J = 7.3 Hz), 3.02 (s, 3 H), 1.69 (m, 2 H), 1.48 (d, 3 H, J = 7.1 Hz), 1.43 (m, 2 H), 1.29 (s, 9 H), 0.97 (t, 3 H, J = 7.3 Hz); IR (pur) 3289, 2959, 1651, 1585, 1513, 1455, 1410, 1337, 1254, 1159 cm⁻¹; MS (FAB) m/z 480 (M+H)

### Beispiel 236: N-(6-tert-Butyl-2-hexyloxy-pyridin-3-ylmethyl)-2-(3-fluoro-4-methansulfonylamino-phenyl)-propionamid

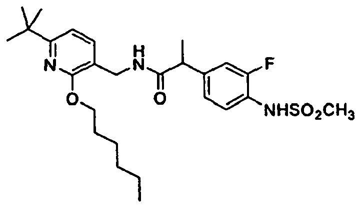

¹H NMR (CDCl₃) δ 7.50 (dd, 1 H, J = 8.4, 8.4 Hz), 7.36 (d, 1 H, J = 7.5 Hz), 7.15-7.05 (m, 2 H), 6.79 (d, 1 H, J = 7.5 Hz), 6.58 (bs, NH), 6.07 (bt, NH), 4.39-4.24 (m, 4 H), 3.48 (q, 1 H, J = 7.0 Hz), 3.02 (s, 3 H), 1.71 (m, 2 H), 1.48 (d, 3 H, J = 7.1 Hz), 1.45-1.25 (m, 6 H), 1.29 (s, 9 H), 0.91 (m, 3 H); IR (pur) 3289, 2957, 1651, 1585, 1513, 1455, 1411, 1338, 1254, 1159, 973 cm⁻¹; MS (FAB) m/z 508 (M+H)

### Beispiel 237: N-(2-Benzyloxy-6-tert-butyl-pyridin-3-ylmethyl)-2-(3-fluoro-4-methansulfonylamino-phenyl)-propionamid

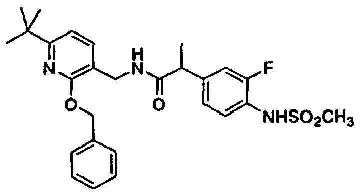

¹H NMR (CDCl₃) δ 7.46-7.30 (m, 7 H), 7.06 (dd, 1 H, J = 11.2, 1.8 Hz), 6.95 (d, 1 H, J = 8.4 Hz), 6.84 (d, 1 H, J = 7.5 Hz), 6.53 (bs, NH), 6.06 (bt, NH), 5.42 (m, 2 H), 4.42-4.26 (m, 2 H), 3.38 (q, 1 H, J = 7.1 Hz), 2.98 (s, 3 H), 1.41 (d, 3 H, J= 7.1 Hz), 1.30 (s, 9 H); IR (pur) 3291, 2959, 1652, 1512, 1452, 1405, 1338, 1254, 1158 cm⁻¹; MS (FAB) m/z 514 (M+H)

### Beispiel 238: N-(2-Cyclohexyloxy-4-trifluoromethyl-benzyl)-2-(3-fluoro-4-methansulfonylamino-phenyl)-propionamid

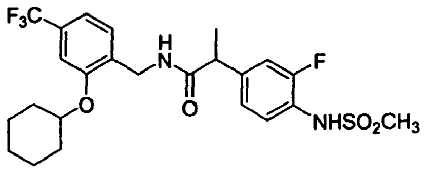

¹H NMR (300 MHz, CDCl₃) δ 7.51 (dd, 1 H, *J* = 8.4, 8.4 Hz), 7.30 (m, 1 H), 7.09-7.10 (m, 2 H), 7.06 (d, 1 H, *J* = 8.3 Hz), 7.02 (bs, 1 H), 6.47 (bs, 1 H), 5.53 (m, 1 H), 4.27-4.50 (m, 3 H), 3.50 (q, 1 H, *J* = 7.0 Hz), 3.02 (s, 3 H), 1.84-1.96 (m, 2 H), 1.64-1.78 (m , 2 H), 1.25-1.63 (m, 9 H); IR (KBr) 3289, 2937, 2859, 1653, 1510, 1427, 1330, 1236, 1160, 1124, 1043, 973, 906, 733 cm⁻¹; MS (FAB ) *m*/*z* 517 (M+H)

### Beispiel 240: N-(6-tert-Butyl-2-pyrrolidin-1-yl-pyridin-3-ylmethyl)-2-(3-fluoro-4-methan sulfonylamino-phenyl)-propionamid

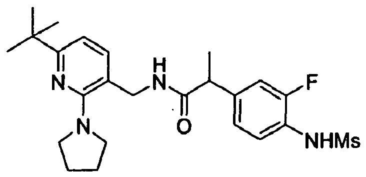

¹H NMR (300 MHz, CDCl₃) δ 7.51 (dd, 1 H, *J* = 8.3, 8.3 Hz), 7.24 (d, 1 H, *J* = 7.7 Hz), 7.20 (dd, 1 H, *J* =11.0, 2.0 Hz), 7.08 (d, 1 H, *J* = 8.8 Hz), 6.67 (d, 1 H, *J* = 7.7 Hz), 6.50 (bs, 1 H), 5.90 (bs, 1 H), 4.4 (d, 2 H, *J* = 4.6 Hz), 3.5 (q, 1 H, *J* = 7.0 Hz), 3.41 - 3.36 (m, 4 H), 3.00 (s, 3 H), 1.85 -1.80 (m, 4 H), 1.50 (d, 3 H, *J* = 7.1 Hz), 1.30 (s, 9 H); IR (KBr) 3289, 2962, 2868, 1650, 1513, 1450, 1411 cm⁻¹; MS (FAB) *m*/*z* 477 (M+H)

### Beispiel 241: N-(6'-tert-Butyl-4-methyl-3,4,5,6-tetrahydro-2H-[1,2']bipyridinyl-3'-ylmethyl)-2-(3-fluoro-4-methansulfonylamino-phenyl)-propionamid

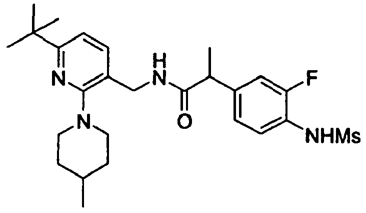

¹H NMR (300 MHz, CDCl₃) δ 7.51 (dd, 1 H, *J* = 8.3, 8.3 Hz), 7.30 (d, 1 H, *J* = 7.7 Hz), 7.20-7.00 (m, 2 H), 6.90 (d, 1 H, *J* = 7.7 Hz), 4.45 (m, 2 H), 3.52 (q, 1 H, *J* = 7.0 Hz), 3.30 (m, 2 H), 3.00 (s, 3 H), 2.78 (m, 2 H), 1.70-1.50 (m, 5 H), 1.50 (d, 3 H , *J* = 7.1 Hz), 1.30 (s, 9 H), 0.97 (d, 3 H, *J* = 6.6 Hz); IR (KBr) 3291, 2922, 1651, 1513, 1452, 1400, 1335 cm⁻¹; MS (FAB) *m*/*z* 505 (M+H)

### Beispiel 243: N-[2-(4-Butyl-benzyloxy)-6-trifluoromethyl-pyridin-3-ylmethyl]-2-(3-fluoro-4-methansulfonylamino-phenyl)-propionamid

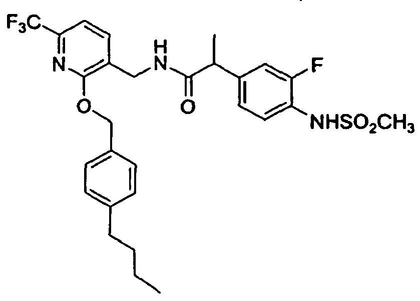

¹H NMR (300 MHz, CDCl₃) δ 7.61 (d, 1 H, *J* = 7.5 Hz), 7.48 (dd, 1 H, *J* = 8.2, 8.2 Hz), 7.33 (d, 2 H, *J* = 8.1 Hz), 7.23 (d, 1 H, *J* = 7.3 Hz), 7.10 (d, 2 H, *J* = 7.9Hz), 7.04 (dd, 1 H, *J* = 11.2, 2.0 Hz), 6.97 (d, 1 H, J = 8.6 Hz), 6.41 (bs, 1 H), 5.94 (bt, 1 H), 5.38 (m, 2 H), 4.37 (m, 2 H), 3.39 (q, 1 H, *J* = 7.0 Hz), 3.01 (s, 3 H), 2.63 (t , 2 H, *J* = 7.8 Hz), 1.61 (m , 2 H), 1.41 (d, 3 H, *J* = 7.1 Hz), 1.38 (m, 2 H), 0.93 (t, 3 H, *J* = 7.3 Hz); IR (pur) 3289, 2930, 1655, 1602, 1512, 1463, 1420, 1352, 1267, 1158, 975, 933, 831, 761 cm⁻¹; MS (FAB) *m*/*z* 582 (M+H)

### Beispiel 244: N-[2-(4-tert-Butyl-benzyloxy)-6-trifluoromethyl-pyridin-3-ylmethyl]-2-(3-fluoro-4-methansulfonylamino-phenyl)-propionamid

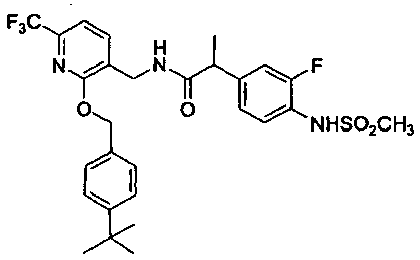

¹H NMR (300 MHz, CDCl₃) δ 7.61 (d, 1 H, *J* = 7.1 Hz), 7.48 (dd, 1 H, *J* = 8.3, 8.3 Hz), 7.45-7.37 (m, 4 H), 7.23 (d, 1 H, *J* = 7.3 Hz), 7.04 (dd, 1 H, *J* = 11.2, 1.9 Hz), 6.97 (d, 1 H, *J* = 8.1 Hz), 6.42 (bs, 1 H), 5.98 (bt, 1 H), 5.39 (m, 2 H), 4.38 (m, 2 H), 3.40 (q, 1 H, *J* = 7.3 Hz), 3.00 (s, 3 H), 1.41 (d, 3 H, *J* = 7.1 Hz), 1.34 (s, 9 H); IR (pur) 3293, 2964, 1656, 1601, 1513, 1462, 1422, 1348, 1267, 1156, 976, 833, 758 cm⁻¹; MS (FAB ) *m*/*z* 582 (M+H)

### Beispiel 245: 2-(3-Fluoro-4-methansulfonylamino-phenyl)-N-[2-(indan-2-yloxy)-6-trifluoromethyl-pyridin-3-ylmethyl]-propionamid

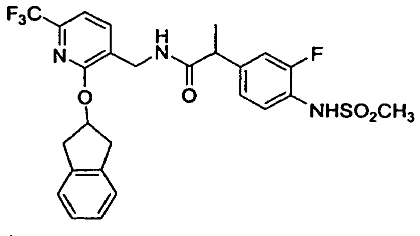

¹H NMR (300 MHz, CDCl₃) δ 7.59 (d, 1 H, J = 7.3 Hz), 7.46 (dd, 1 H, *J* = 8.2, 8.2 Hz), 7.29-7.24 (m, 5 H), 6.99 (dd, 1 H, *J* = 11.2, 2.0 Hz), 6.91 (d, 1 H, *J* = 8.8 Hz), 6.35 (bs, 1 H), 5.89 (m, 1 H), 5.79 (bt , 1 H), 4.27 (m, 2 H), 3.43 (dd, 2 H, *J* = 17.2, 5.5 Hz), 3.08-3.04 (m, 3 H), 3.00 (s, 3 H), 1.31 (d, 3 H, *J* = 7.1 Hz) IR (pur) 3291, 2927, 1658, 1600, 1511, 1418, 1339, 1268, 1157, 1015, 970, 933, 747 cm⁻¹; MS (FAB) *m*/*z* 552 (M+H)

### Beispiel 246: 2-(3-Fluoro-4-methansulfonylamino-phenyl)-N-[2-(4-p-tolyl-piperazin-1-yl)-4-trifluoromethyl-benzyl]-propionamid

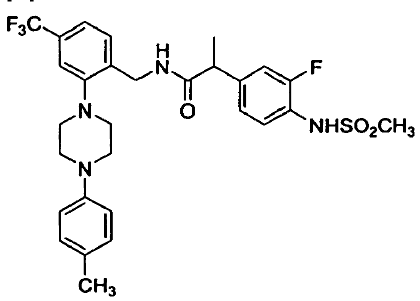

¹H NMR (300 MHz, CDCl₃) δ 7.46 (dd, 1 H, *J* = 8.2, 8.2 Hz), 7.34 (s, 1 H), 7.33 (d, 1 H, *J* = 7.5 Hz), 7.31 (d, 1 H, *J* = 7.0 Hz), 7.13 (d, 2 H, *J* = 8.1 Hz), 7.14 (dd, 1 H, *J* = 11.2, 2.0 Hz), 7.08 (d, 1 H, *J* = 10.0 Hz), 6.86 (d, 2 H, *J* = 8.6 Hz), 6.35 (bt, 2H), 6.22 (bs, 1 H), 4.57 (m, 2 H), 3.54 (q, 1 H, *J* = 7.0 Hz), 3.18-3.12 (m, 4 H), 3.05-3.01 (m, 4 H), 2.97 (s, 3 H), 2.31 (s, 3 H), 1.51 (d, 3 H, *J* = 7.1 Hz); IR (pur) 2923, 1655, 1513, 1425, 1334, 1221, 1159, 1123, 963, 816, 757 cm⁻¹; MS (FAB) *m*/*z* 593 (M+H)

### Beispiel 247: 2-(3-Fluoro-4-methansulfonylamino-phenyl)-N-[2-(4-m-tolyl-piperazin-1-yl)-4-trifluoromethyl-benzyl]-propionamid

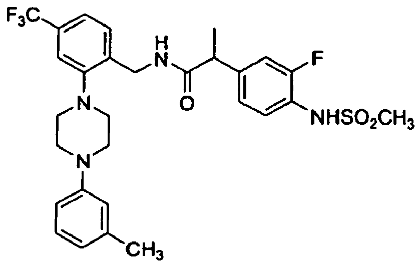

¹H NMR (300 MHz, CDCl₃) δ 7.46 (dd, 1 H, *J* = 8.2 Hz), 7.36-7.26 (m, 3 H), 7.21 (m, 1 H), 7.13 (dd, 1 H, *J* = 11.2, 2.0 Hz), 7.07 (d, 1 H, *J* = 8.1, 8.1 Hz), 6.79-6.74 (m, 3 H), 6.33 (bt, 2 H), 6.25 (bs, 1 H), 4.57 (m, 2 H), 3.54 (q, 1 H, *J* = 7.1 Hz), 3.24-3.18 (m, 4 H), 3.08-3.01 (m, 4 H), 2.97 (s, 3 H), 2.36 (s, 3 H), 1.51 (d, 3 H, *J* = 7.1 Hz); IR (pur) 3294, 2921, 1653, 1603, 1509, 1425, 1335, 1249, 1159, 1122, 965, 775 cm⁻¹; MS (FAB) *m*/*z* 593 (M+H)

### Beispiel 248: 2-(3-Fluoro-4-methansulfonylamino-phenyl)-N-{4-trifluoromethyl-2-[4-(4-trifluoromethyl-phenyl)-piperazin-1-yl]-benzyl}-propionamid

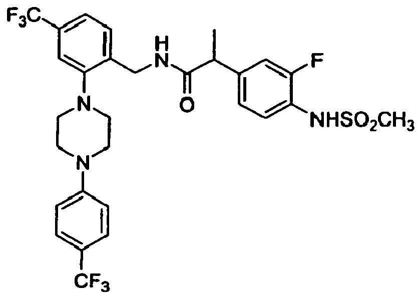

¹H NMR (300 MHz, CDCl₃) δ 7.53 (d, 2 H, *J* = 8.6 Hz), 7.50 (dd, 1 H, *J* = 8.3, 8.3 Hz), 7.33 (s, 1 H), 7.30 (d, 2 H, *J* = 8.2 Hz), 7.14 (dd, 1 H, *J* = 11.2, 2.0 Hz), 7.08 (d, 1 H, *J* = 10.0 Hz), 6.97 (d, 2 H, *J* = 8.9Hz), 6.23 (bt, 1 H), 4.58 (d, 2 H, *J* = 6.4 Hz), 3.54 (q, 1 H, *J* = 7.1 Hz), 3.39-3.31 (m, 4 H), 3.04-2.98 (m, 4 H) ,3.01 (s, 3 H), 1.53 (d, 3 H, *J* =7.1 Hz); IR (pur) 2923, 1652, 1615, 1511, 1423, 1331, 1237, 1159, 1117, 961, 827 cm⁻¹; MS (FAB) *m*/*z* 647 (M+H)

### Beispiel 249: 2-(3-Fluoro-4-methansulfonylamino-phenyl)-N-{2-[4-(4-methoxy-phenyl)-piperazin-1-yl]4-trifluoromethyl-benzyl}-propionamid

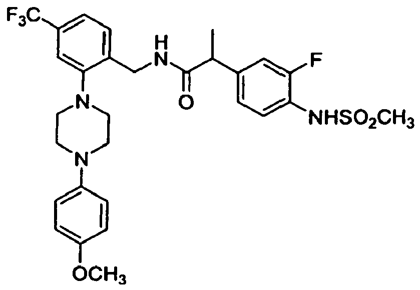

¹H NMR (300 MHz, CDCl₃) δ 7.47 (dd, 1 H, *J* = 8.2, 8.2 Hz), 7.33 (d, 2 H, *J* = 10.1 Hz), 7.29 (s, 1 H), 7.13 (dd, 1 H, *J* = 11.2, 2.0 Hz), 7.07 (d, 1 H, *J* = 8.3 Hz), 6.98-6.85 (m, 4 H), 6.35 (bs, 1 H), 6.33 (bt, 1 H), 4.59 (m, 2 H), 3.80 (s, 3 H), 3.54 (q, 1 H, *J* = 7.1 Hz), 3.15-3.08 (m, 4 H), 3.05-2.98 (m, 4 H), 2.98 (s, 3 H ), 1.51 (d, 3 H, *J* = 7.1 Hz); IR (pur) 2929, 1657, 1511, 1425, 1335, 1244, 1159, 1122, 1036, 963, 827, 757 cm⁻¹; MS (FAB) *m*/*z* 609 (M+H)

### Beispiel 255: 2-(3-Fluoro-4-methansulfonylamino-phenyl)-N-{2-[4-(4-fluoro-phenyl)-piperidin-1-yl]-4-trifluoromethyl-benzyl}-propionamid

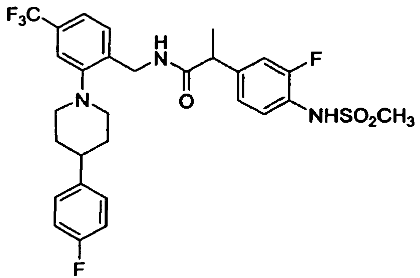

¹H NMR (300 MHz, CDCl₃) δ 7.50 (dd, 1 H, *J* = 8.3 Hz), 7.30-7.26 (m, 3 H), 7.23-7.18 (m, 2 H), 7.15 (dd, 1 H, *J* = 11.8, 2.0 Hz), 7.08 (d, 1 H, *J* = 10.0Hz), 7.03 (m, 2 H), 6.46 (bs, 1 H), 6.24 (bt, 1 H), 4.56 (d, 2 H, *J* = 5.7 Hz), 3.56 (q, 1 H, *J* = 7.1 Hz), 3.09 (m, 2 H), 3.00 (s, 3 H), 2.83 (m, 2 H), 2.64 (m, 1 H), 1.94 (m, 2 H), 1.78 (m, 2 H), 1.54 (d, 3 H, *J* = 6.9 Hz); IR (pur) 3320, 2922, 1652, 1509, 1423, 1332, 1222, 1159, 1120, 971, 888, 834, 763 cm⁻¹; MS (FAB) *m*/*z* 596 (M+H)

### Beispiel 256: N-(2-Butoxy-6-trifluoromethyl-pyridin-3-ylmethyl)-2-(4-methansulfonylamino-3-methyl-phenyl)-propionamid

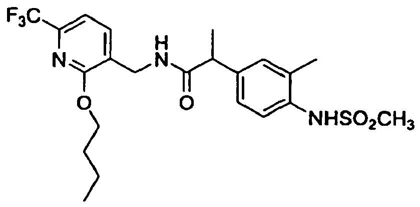

¹H NMR (300 MHz, CDCl₃) δ 7.55 (d, 1 H, *J* = 7.5 Hz), 7.40 (d, 1 H, *J* = 8.8 Hz), 7.17 (d, 1 H, *J* = 7.3 Hz), 7.11 (d, 1 H, *J* = 6.9 Hz), 7.10 (s, 1 H), 6.21 (bs, 1 H), 5.93 (bt, 1 H), 4.36 (d, 2 H, *J* = 6.4 Hz), 4.31 (m, 2 H), 3.51 (q, 1 H, *J* = 7.1 Hz), 3.03 (s, 3 H), 2.28 (s, 3 H), 1.69 (m, 2 H), 1.48 (d, 3 H, *J* = 7.1 Hz), 1.41 (m, 2 H), 0.96 (t, 3 H, *J* = 7.3 Hz); IR (pur) 3291, 2963, 1654, 1605, 1537, 1463, 1425, 1326, 1151, 972, 932, 834 cm⁻¹; MS (FAB) *m*/*z* 488 (M+H)

### Beispiel 257: N-(2-Hexyloxy-6-trifluoromethyl-pyridin-3-ylmethyl)-2-(4-methansulfonylamino-3-methyl-phenyl)-propionamid

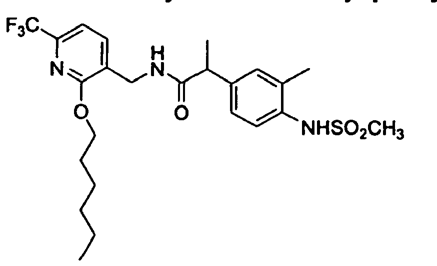

¹H NMR (300 MHz, CDCl₃) δ 7.54 (d, 1 H, *J* = 7.5 Hz), 7.39 (d, 1 H, *J* = 9.0 Hz), 7.17 (d, 1 H, *J* = 7.3 Hz), 7.11 (d, 1 H, *J* = 7.0 Hz), 7.09 (s, 1 H), 6.21 (bs, 1 H), 5.94 (bt, 1 H), 4.36 (d, 2H, *J* = 6.2 Hz), 4.30 (m, 2 H), 3.51 (q, 1 H, *J* = 7.1 Hz), 3.03 (s, 3 H), 2.28 (s, 3 H), 1.69 (m, 2 H), 1.48 (d, 3 H, *J* = 7.1 Hz), 1.39-1.26 (m, 6 H), 0.91 (t, 3 H, *J*=6.6 Hz); IR (pur) 3290, 2931, 1655, 1604, 1504, 1464

### Beispiel 258: N-[2-(4-Chloro-benzyloxy)-6-trifluoromethyl-pyridin-3-ylmethyl]-2-(3-fluoro-4-methansulfonylamino-phenyl)-propionamid

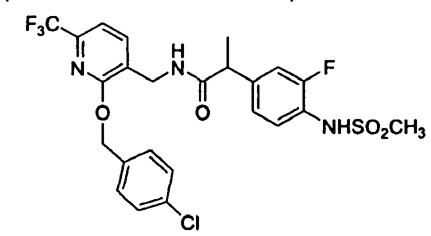

¹H NMR (300 MHz, CDCl₃) δ 7.60 (d, 1 H, *J* = 7.5 Hz), 7.48 (dd, 1 H, *J* = 8.3, 8.3 Hz), 7.39-7.28 (m, 4 H), 7.24 (d, 1 H, *J* = 7.5 Hz), 7.05 (dd, 1 H, *J* = 11.2, 2.0Hz), 7.00 (d, 1 H, *J* = 8.3 Hz), 6.48 (bs, 1 H), 5.91 (bt, 1 H), 5.37 (d, 2 H, *J* = 4.7 Hz), 4.39 (m, 2 H), 3.45 (q, 1 H, *J* = 7.1 Hz), 3.02 (s, 3 H), 1.44 (d, 3 H, *J* = 7.0 Hz); IR (pur) 3299, 2929, 1658, 1601, 1512, 1461, 1423, 1350, 1267, 7756, 975, 934, 808 cm⁻¹; MS (FAB ) *m*/*z* 560 (M+H)

### Beispiel 259: N-(4-Dimethylaminomethyl-4-phenyl-6'-trifluoromethyl-3,4,5,6-tetrahydro-2H-[1,2']bipyridinyl-3'-ylmethyl)-2-(3-fluoro-4-methansulfonylamino-phenyl)-propionamid

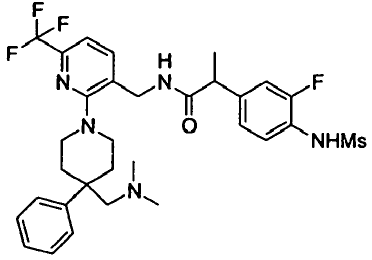

¹H NMR (300 MHz, CDCl₃) δ 7.45-7.50 (m, 2 H), 7.36 (m, 4 H), 7.21 (m, 1 H), 7.18 (d, 1 H, J = 8.1 Hz), 7.13 (dd, 1 H, J = 11.1, 1.8 Hz), 7.06 (d, 1 H, J = 8.1 Hz), 6.37 (bt, 1 H), 4.46 (d, 2 H, J = 5.7 Hz), 3.58 (q, 1 H, J = 6.9 Hz), 3.17 (m, 2 H), 3.01 (s, 3 H), 2.98 (m, 2 H), 2.52 (s, 2 H), 2.18 (m, 2 H), 1.98 (s, 6 H), 1.51 (d, 3 H, J = 6.9 Hz); IR (KBr) 2937, 1657, 1592, 1509, 1456, 1420, 1335, 1252, 1157, 1041, 972, 758, 702 cm⁻¹; MS (FAB) *m*/*z* 636 (M+H)

### Beispiel 260: N-[2-(4-Cyclohexyl-piperazin-1-yl)-6-trifluoromethyl-pyridin-3-ylmethyl]-2-(3-fluoro-4-methansulfonylamino-phenyl)-propionamid

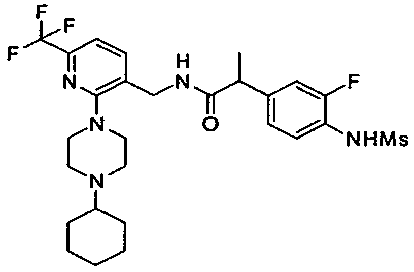

¹H NMR (300 MHz, CDCl₃) δ 7.47-7.53 (m, 2 H), 7.23 (d, 1 H, J = 7.8 Hz), 7.07-7.15 (m, 2 H), 6.26 (bt, 1 H), 4.44 (d, 2 H, J = 5.7 Hz), 3.58 (q, 1 H, J = 6.9 Hz), 3.27 (m, 4 H), 3.03 (s, 3 H), 2.84 (m, 4 H), 2.50 (m, 1 H), 1.94 (m, 2 H), 1.85 (m, 2 H), 1.51 (d, 3 H, J = 6.9 Hz), 1.25-1.30 (m, 6 H); IR (KBr)) 2934, 2857, 1657, 1591, 1502, 1459, 1418, 1334, 1271, 1152, 979, 757 cm⁻¹; MS (FAB) *m*/*z* 586 (M+H)

### Beispiel 261: N-(6-tert-Butyl-2-cyclopentyloxy-4-hydroxymethyl-pyridin-3-ylmethyl)-2-(3-fluoro-4-methansulfonylamino-phenyl)-propionamid

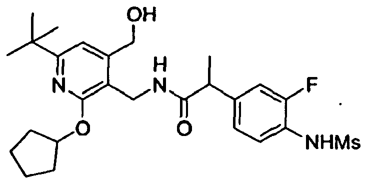

¹H NMR (300 MHz, CDCl₃) δ 7.48 (dd, 1 H, J = 8.1, 8.1 Hz), 6.99- 7.05 (m, 2 H), 6.82 (s, 1 H), 6.32 (bt, 1 H), 5.40 (m, 1 H), 4.63 (d, 2 H, J = 5.7 Hz), 4.37 (m, 2 H), 3.47 (q, 1 H, J = 6.9 Hz), 3.03 (s, 3 H), 1.94 (m, 2 H), 1.63 (m, 6 H), 1.45 (d, 3 H, J = 6.9 Hz), 1.29 (s, 9 H); IR (KBr) 3369, 2962, 1651, 1592, 1513, 1452, 1397, 1336, 1158, 1026, 974, 758 cm⁻¹; MS (FAB) *m*/*z* 522 (M+H)

### Beispiel 262: 2-(4-Methansulfonylamino-phenyl)-N-(4-methyl-6'-trifluoromethyl-3,4,5,6-tetrahydro-2H-[1,2']bipyridinyl-3'-ylmethyl)-propionamid

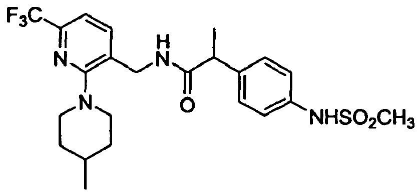

¹H NMR (300 MHz, CDCl₃) δ 7.46 (d, 1 H, *J* = 7.5 Hz), 7.23-7.31 (m, 2 H), 7.14-7.22(m, 3 H), 6.60 (bs, 1 H), 6.20 (bt , 1 H), 4.46 (d, 2 H, *J* = 5.9 Hz), 3.59 (q, 1 H, *J* = 7.1 Hz), 3.19-3.38 (m, 2 H), 3.01 (s, 3 H), 2.75-2.87 (m, 2 H), 1.65-1.79 (m, 2 H), 1.48-1.56 (m , 4 H), 1.14-1.32 (m, 2 H), 0.97 (d, 3 H, , *J* = 6.6 Hz); IR (KBr) 3287, 2921, 1646, 1512, 1458, 1423, 1335, 1233, 1145, 970, 840 cm⁻¹; MS (FAB) *m*/*z* 499 (M+H)

### Beispiel 263: N-[2-(3,3-Dimethyl-butyl)-6-trifluoromethyl-pyridin-3-ylmethyl]-2-(3-fluoro-4-methansulfonylamino-phenyl)-propionamid

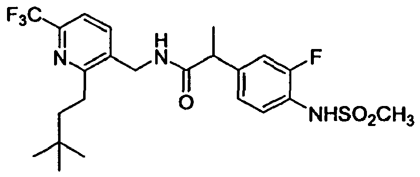

¹H NMR (300 MHz, CDCl₃) δ 7.49-7.58 (m, 2 H), 7.43(d, 1 H, *J* = 7.9 Hz), 7.17 (dd, 1 H, *J* = 11.2, 1.8 Hz), 7.11 (d, 1 H, *J* = 8.6 Hz), 6.48 (bs, 1 H), 5.70 (bt, 1 H), 4.41-4.56 (m, 2 H), 3.58 (q , 1 H, *J* = 7.3 Hz), 3.04 (s, 3 H), 2.71-2.79 (m, 2 H), 1.51-1.60 (m, 5 H), 0.96 (s, 9 H); IR (KBr) 3292, 2957, 1656, 1512, 1463, 1408, 1340, 1277, 1157, 972, 906, 758 cm⁻¹; MS (FAB) *m*/*z* 504 (M+H)

### Beispiel 264: 2-(3-Fluoro-4-methansulfonylamino-phenyl)-N-[2-(2-p-tolyl-ethyl)-6-trifluoromethyl-pyridin-3-ylmethyl]-propionamid

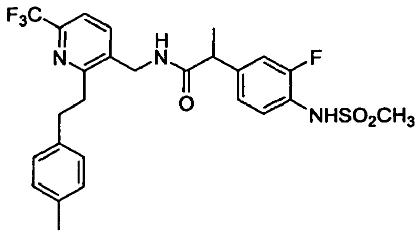

¹H NMR (300 MHz, CDCl₃) δ 7.48-7.59 (m, 2 H), 7.46 (m, 1 H), 7.12 (dd, 1 H, *J* = 11.2, 2.0 Hz), 6.96-7.10 (m, 5 H), 6.41 (bs, 1 H), 5.26 (bt, 1 H), 4.20-4.39 (m , 2 H), 3.35 (q, 1 H, *J* = 7.1 Hz), 3.05-3.15 (m, 4 H), 3.05 (s, 3 H), 2.31 (s, 3 H), 1.47 (d, 3 H, *J*=7.1 Hz); IR (KBr) 3298, 2925, 1658, 1589, 1513, 1408, 1339, 1279, 1157, 973, 912, 813, 733 cm⁻¹; MS (FAB) *m*/*z* 538 (M+H)

### Beispiel 267: 2-(3-Fluoro-4-methansulfonylamino-phenyl)-N-(2-hexyl-6-trifluoromethyl-pyridin-3-ylmethyl)-propionamid

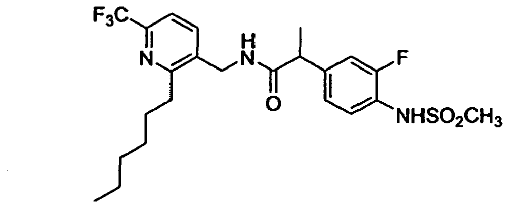

¹H NMR (300 MHz, CDCl₃) δ 7.50-7.59 (m, 2 H), 7.44 (d, 1 H, *J* = 8.1 Hz), 7.16 (dd, 1 H, *J* = 11.2, 2.0 Hz), 7.10 (d, 1 H, *J* = 8.4 Hz), 6.43 (bs, 1 H), 5.70 (bt , 1 H), 4.41-4.58 (m, 2 H), 3.57 (q, 1 H, *J* = 7.1 Hz), 3.04 (bs, 3 H), 2.78 (t, 2 H, *J* = 7.9 Hz), 1.61-1.74 (m, 2 H), 1.53 (d, 3H, *J*=7.1 Hz), 1.21-1.43 (m , 6 H), 0.88 (m , 3 H); IR (KBr) 3289, 2929, 2857, 1658, 1589, 1512, 1462, 1408, 1341, 1277, 1158, 973, 908 cm⁻¹; MS (FAB) *m*/*z* 504 (M+H)

### Beispiel 268: 2-(3-Fluoro-4-methansulfonylamino-phenyl)-N-[2-(4-methyl-pentyl)-6-trifluoromethyl-pyridin-3-ylmethyl]-propionamid

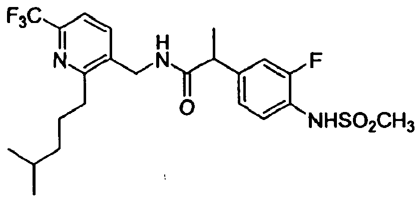

¹H NMR (300 MHz, CDCl₃) δ 7.51-7.58 (m, 2 H), 7.44 (d, 1 H, *J* = 8.0 Hz), 7.17 (dd, 1 H, *J* = 11.2, 2.0 Hz), 7.10 (d, 1 H, *J* = 8.4 Hz), 6.48 (bs, 1 H), 5.70 (bt, 1 H), 4.41-4.57 (m , 2 H), 3.57 (q, 1 H, *J* = 7.0 Hz), 3.04 (s, 3 H), 2.76 (t, 2 H, *J* = 8.0 Hz), 1.61-1.76 (m, 2 H), 1.58 (m, 1 H), 1.54 (d, 3 H, *J* = 7.1 Hz), 1.19-1.27 (m , 2 H), 0.87 (d , 6 H, *J* = 6.6 Hz); IR (KBr) 3290, 2956, 1656, 1589, 1512, 1462, 1408, 1339, 1279, 1158, 972, 906 cm⁻¹; MS (FAB) *m*/*z* 504 (M+H)

### Beispiel 269: 2-(3-Fluoro-4-methansulfonylamino-phenyl)-N-(4-hydroxy-6'-trifluoromethyl-3,4,5,6-tetrahydro-2H-[1,2']bipyridinyl-3'-ylmethyl)-propionamid

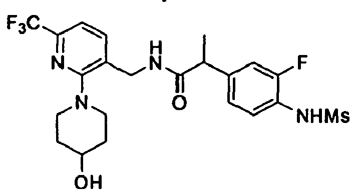

¹H NMR (CDCl₃) δ 7.54-7.49 (m, 2 H), 7.23 (d, 1 H, J = 7.7 Hz), 7.16-7.09 (m, 2 H), 6.69 (bs, NH), 6.25 (bt, NH), 4.48 (m, 2 H), 3.84 (m, 1 H), 3.58 (q, 1 H, J = 7.3 Hz), 3.38-3.26 (m, 2 H), 3.04 (s, 3 H), 2.97-2.88 (m, 2 H), 2.02-1.92 (m, 2 H), 1.75 (s, OH), 1.53 (d, 3 H, J = 7.1 Hz); IR (pur) 3294, 2934, 1658, 1592, 1512, 1418, 1334, 1155, 732 cm⁻¹; Mass (FAB) m/z 519[M+H]

### Beispiel 270: N-(2-Cyclohexylmethoxy-6-trifluoromethyl-pyridin-3-ylmethyl)-2-(3-fluoro-4-methansulfonylamino-phenyl)-propionamid

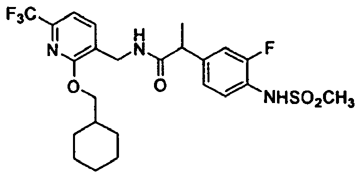

¹H NMR (CDCl₃) δ 7.56 (d, 1 H, J = 7.3 Hz), 7.51 (dd, 1 H, J = 8.3, 8.3 Hz), 7.18 (d, 1 H, J = 7.3 Hz), 7.12-7.04 (m, 2 H), 6.57 (bs, NH), 5.99 (bt, NH), 4.38 (m, H), 4.16 (m, 2 H), 3.51 (q, 1 H, J = 7.1 Hz), 3.03 (s, 3 H), 1.82-1.67 (m, 5 H), 1.49 (d, 3 H, J = 7.1 Hz), 1.32-1.00 (m, 6 H); IR (pur) 3292, 2928, 2854, 1656, 1513, 1425, 1338, 1269, 1158 cm⁻¹; MS (FAB) m/z 532 (M+H)

### Beispiel 271: 2-(3-Fluoro-4-methansulfonylamino-phenyl)-N-[2-(4-methyl-cyclohexylmethoxy)-6-trifluoromethyl-pyridin-3-ylmethyl]-propionamid

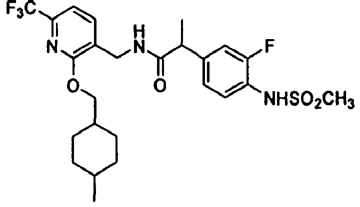

¹H NMR (CDCl₃) δ 7.56 (d, 1 H, J = 7.3 Hz), 7.50 (dd, 1 H, J = 8.2, 8.2 Hz), 7.18 (d, 1 H, J = 7.5 Hz), 7.12-7.04 (m, 2 H), 6.59 (bs, NH), 6.00 (bt, NH), 4.45-4.11 (m, 4 H), 3.51 (q, 1 H, J = 7.1 Hz), 3.03 (s, 3 H), 1.95-1.25 (m, 12 H), 1.10-0.90 (m, 4 H); IR (pur) 3295, 2924, 1655, 1513, 1425, 1337, 1268, 1158 cm⁻¹; MS (FAB) m/z 546 (M+H)

### Beispiel 279: N-[2-(2,2-Dimethyl-cyclopropylmethoxy)-6-trifluoromethyl-pyridin-3-ylmethyl]-2-(3-fluoro-4-methansulfonylamino-phenyl)-propionamid

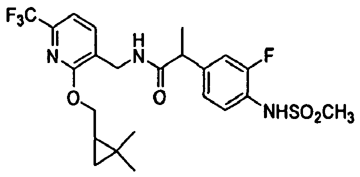

¹H NMR (CDCl₃) δ 7.58 (d, 1 H, J = 7.3 Hz), 7.51 (dd, 1 H, J = 8.4, 8.4 Hz), 7.19 (d, 1 H, J = 7.3 Hz), 7.14-7.05 (m, 2 H), 6.54 (bs, NH), 6.07 (bt, NH), 4.57-4.33 (m, 3 H), 4.24-4.15 (m, 1 H), 3.51 (q, 1 H, J = 7.1 Hz), 3.03 (s, 3 H), 1.49 (dd, 3 H, J = 7.0, 1.7 Hz), 1.13 (d, 3 H, J = 1.5 Hz), 1.09 (s, 3 H), 1.06-0.95 (m, 1 H), 0.57 (dd, 1 H, J = 8.4, 4.4 Hz), 0.28 (m, 1 H); IR (pur) 3293, 2928, 1655, 1514, 1427, 1339, 1266, 1158, 980 cm⁻¹; MS (FAB) m/z 518(M+H)

### Beispiel 282: 2-(3-Fluoro-4-methansulfonylamino-phenyl)-N-(3-methyl-6'-trifluoromethyl-3,4,5,6-tetrahydro-2H-[1,2']bipyridinyl-3'-ylmethyl)-propionamid

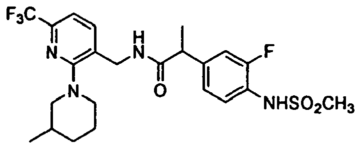

¹H NMR (CDCl₃) δ 7.54-7.47 (m, 2 H), 7.21 (d, 1 H, J = 7.7 Hz), 7.15-7.07 (m, 2 H), 6.64 (bs, NH), 6.34 (bt, NH), 4.48 (d, 2 H, J = 5.9 Hz), 3.56 (q, 1 H, J = 7.0 Hz), 3.32-3.17 (m, 2 H), 3.03 (s, 3 H), 2.74 (m, 1 H), 2.46 (m, 1 H), 1.82-1.61 (m, 4 H), 1.53 (d, 3 H, J = 7.1 Hz), 1.13-1.01 (m, 1 H), 0.91 (m, 3 H); IR (pur) 3295, 2927, 1655, 1593, 1513, 1458, 1419, 1336, 1158 cm⁻¹; MS (FAB) m/z 517 (M+H)

### Beispiel 283: 2-(3-Fluoro-4-methansulfonylamino-phenyl)-N-(2-methyl-6'-trifluoromethyl-3,4,5,6-tetrahydro-2H-[1,2']bipyridinyl-3'-ylmethyl)-propionamid

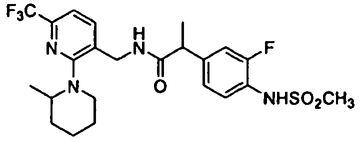

¹H NMR (CDCl₃) δ 7.62-7.48 (m, 2 H), 7.30 (m, 1 H), 7.18-7.07 (m, 2 H), 6.71 (bt, NH), 6.58 (bs, NH), 4.67-4.57 (m, 1 H), 4.35 (m, 1 H), 3.56-3.46 (m, 2 H), 3.03 & 3.02 (s, 3H), 3.01-2.95 (m, 1 H), 2.79 (m, 1 H), 1.80-1.50 (m, 9 H), 0.90 & 0.85 (d, 3 H); IR (pur) 3289, 2933, 2853, 1655, 1512, 1456, 1411, 1335, 1158 cm⁻¹; MS (FAB) m/z 517 (M+H)

### Beispiel 284: 2-(3-Fluoro-4-methansulfonylamino-phenyl)-N-[2-(4-methyl-6'-trifluoromethyl-3,4,5,6-tetrahydro-2H-[1,2]bipyridinyl-3'-yl)-ethyl]-propionamid

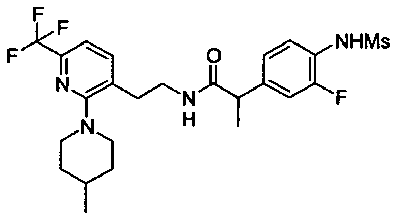

¹H NMR (300 MHz, CDCl₃) δ 7.44-7.50 (m, 2 H), 7.19 (d, 1 H, J = 7.8 Hz), 6.96-7.06 (m, 2 H), 5.79 (bt, 1 H), 3. 54 (q, 2 H, J = 6.3 Hz), 3.25-3.40 (m, 3 H), 3.03 (s, 3 H), 2.78-2.87 (m, 4 H), 1.76 (m, 2 H), 1.60 (m, 3 H), 1.42 (d, 3 H, J= 6.9 Hz), 0.99 (d, 3 H, J = 6.6 Hz); IR (KBr) 2920, 1646, 1537, 1455, 1415, 1325, 1153, 832 cm⁻¹; MS (FAB) *m*/*z* 531 (M+H)

### Beispiel 285: N-(4-Cyano-4-phenyl-6'-trifluoromethyl-3,4,5,6-tetrahydro-2H-[1,2']bipyridinyl-3'-ylmethyl)-2-(3-fluoro-4-methansulfonylamino-phenyl)-propionamid

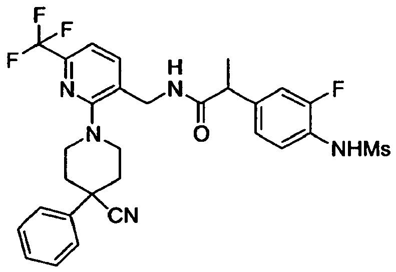

¹H NMR (300 MHz, CDCl₃) δ 7.28-7.52 (m, 8 H), 7.08-7.17 (m, 2 H), 6.04 (bt, 1 H), 4.49 (d, 2 H, J = 5.7 Hz), 3.58 (q, 1 H, J = 6.9 Hz), 3.36-3.60 (m, 5 H), 3.02 (s, 3 H), 2.17 (m, 4 H), 1.54 (d, 3 H, J = 6.9 Hz); IR (KBr) 2931, 1657, 1590, 1509, 1455, 1324, 1241, 1154, 966, 758, 702 cm⁻¹; MS (FAB) *m*/*z* 604(M+H)

### Beispiel 287: 2-(4-Ethansulfonylamino-3-fluoro-phenyl)-N-(4-methyl-6'-trifluoromethyl-3,4,5,6-tetrahydro-2H-[1,2']bipyridinyl-3'-ylmethyl)-propionamid

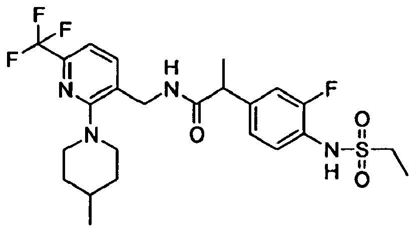

¹H NMR (300 MHz, CDCl₃) δ 7.53 (dd, 1 H, J = 8.1, 8.1 Hz), 7.47 (d, 1 H, J = 7.5 Hz), 7.19 (d, 1 H, J = 7.5 Hz), 7.11 (d, 1 H, J = 11.4, 1.8 Hz), 7.06 (d, 1 H, 8.4 Hz), 6.55 (bs, 1 H), 6.30 (bt, 1 H), 4.46 (d, 2 H, J = 5.7 Hz), 3.55(q, 1 H, J = 6. 9 Hz), 3.29 (m, 2 H), 3.11 (q, 2 H, J = 7.5 Hz), 2.80 (m, 2 H), 1.67-1.70 (m, 3 H), 1.52 (d, 3 H, J = 6.9 Hz), 1.38 (t, 3 H, J = 7.5 Hz), 1.22 (m, 2 H), 0.97 (d, 3 H, J = 6.3 Hz); IR (KBr) 3290, 2926, 2658, 1592, 1511, 1456, 1418, 1374, 1335, 1275, 1148, 942, 832, 757 cm⁻¹; MS (FAB) *m*/*z* 531 (M+H)

### Beispiel 288: 2-(4-dimethylaminsulfonylamino-3-fluoro-phenyl)-N-(4-methyl-6'-trifluoromethyl-3,4,5,6-tetrahydro-2H-[1,2']bipyridinyl-3'-ylmethyl)-propionamid

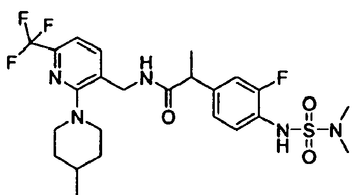

¹H NMR (300 MHz, CDCl₃) δ 7.45-7.52 (m, 2 H), 7.18 (d, 1 H, J = 7.5 Hz), 7.02-7.10 (m, 2 H), 6.26 (bs, 1 H), 4.46 (d, 2 H, J = 5.7 Hz), 4.55 (q, 1 H, J = 6.9 Hz), 3.30 (m, 2 H), 2.77-2.83 (m, 9 H), 1.72 (m, 2 H), 1.52 (d, 3 H, J = 6.9 Hz), 1.19-1.26 (m, 3 H), 0.97 (d, 3 H, J = 6.0 Hz); IR (KBr) 3293, 2923, 1658, 1592, 1512, 1456, 1420, 1339, 1272, 1152, 959, 758 cm⁻¹; MS (FAB) *m*/*z* 546(M+H)

### Beispiel 289: 2-(4-Methansulfonylamino-3-methoxy-phenyl)-N-(4-methyl-6'-trifluoromethyl-3,4,5,6-tetrahydro-2H-[1,2']bipyridinyl-3'-ylmethyl)-propionamid

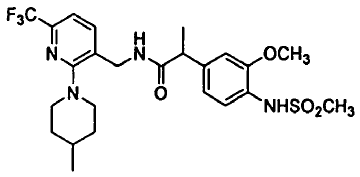

¹H NMR (CDCl₃) δ 7.48-7.45 (m, 2 H), 7.18 (d, 1 H, J = 7.7 Hz), 6.89-6.83 (m, 2 H), 6.75 (bs, NH), 6.25 (bt, NH), 4.46 (d, 2 H, J = 5.7 Hz), 3.83 (s, 3 H), 3.57 (q, 1 H, J = 7.0 Hz), 3.33-3.21 (m, 2 H), 2.95 (s, 3 H), 2.84-2.76 (m, 2 H), 1.75-1.63 (m, 3 H), 1.54 (d, 3 H, J = 7.1 Hz), 1.30-1.13 (m, 2 H), 0.97 (d, 3 H, J = 6.4 Hz); IR (pur) 3297, 2925, 1656, 1594, 1512, 1459, 1419, 1336, 1130 cm⁻¹; MS (FAB) m/z 529 (M+H)

### Beispiel 290: 2-(3-Fluoro-4-methansulfonylamino-phenyl)-N-(4-phenylamino-6'-trifluoromethyl-3,4,5,6-tetrahydro-2H-[1,2']bipyridinyl-3'-ylmethyl)-propionamid

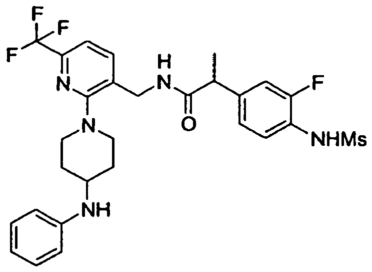

¹H NMR (300 MHz, CDCl₃) δ 7.49-7.54 (m, 2 H), 7.08-7.25 (m, 6 H), 6. 72 (t, 1 H, J = 7.2 Hz), 6.63 (d, 2 H, J = 8.1 Hz), 6.21 (bt, 1 H), 4.48 (d, 2 H, J = 5.7 Hz), 3.57 (q, 1 H, J = 6.9 Hz), 3.35-3.46 (m, 3 H), 3.01-3.04 (m, 5 H), 2.60 (m, 2 H), 2.17 (m, 2 H), 1.52 (d, 3 H, J = 6.9 Hz); IR (KBr) 2927. 1655. 1597. 1511. 1456. 1420. 1375. 1334. 1155. 972. 756 cm⁻¹; MS (FAB) *m*/*z* 594(M+H)

### Beispiel 291: N-(2-Cyclohexyl-6-trifluoromethyl-pyridin-3-ylmethyl)-2-(3-fluoro-4-methansulfonylam ino-phenyl)-propionamid

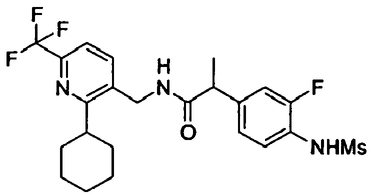

¹H NMR (300 MHz, CDCl₃) δ 7.48-7.54 (m, 2 H), 7.38 (d, 1 H, J = 8.1 Hz), 7.14 (dd, 1 H, J = 11.1, 1.8 Hz) 7.08 (d, 1 H, J = 8.1 Hz), 5.78 (bt, 1 H), 4.49 (d, 2 H, J = 5.7 Hz), 3.57 (q, 1 H, J = 6.9 Hz), 3.02 (s, 3 H), 2.76 (m, 1 H), 1.62-1.81 (m, 6 H), 1.52 (d, 3 H, J = 6.9 Hz), 1.25-1.31 (m, 4 H); IR (KBr) 3300, 2927, 2855, 1643, 1512, 1453, 1336, 1151, 973, 753 cm⁻¹; MS (FAB) *m*/*z* 502(M+H)

### Beispiel 292: 2-(3-Fluoro-4-methansulfonylamino-phenyl)-N-(4-phenyl-6-trifluoromethyl-pyridin-3-ylmethyl)-propionamid

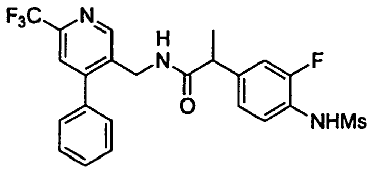

¹H NMR (300 MHz, CDCl₃) δ 8.55 (s, 1 H), 7.46-7.53 (m, 5 H), 7.24-7.27 (m, 2 H), 7.05 (dd, 1 H, J = 11.1, 1.8 Hz), 6.99 (d, 1 H, J = 8.1 Hz), 6.62 (bs 1 H), 5.67 (bt, 1 H), 4.49 (d, 2 H, J = 5.7 Hz), 3.45 (q, 1 H, J = 6.9 Hz), 3.04 (s, 3 H), 1.45 (d, 3 H, J = 6.9 Hz); IR (pur) 3296, 2937, 1715, 1646, 1592, 1505, 1457, 1416, 1361, 1277, 1159, 963, 758 cm⁻¹; MS (FAB) *m*/*z* 496(M+H)

### Beispiel 293: 2-(3-Fluoro-4-methansulfonylamino-phenyl)-N-(4-methyl-6'-trifluoromethyl-3,4,5,6-tetrahydro-2H-[1,2']bipyridinyl-3'-ylmethyl)-thiopropionamid

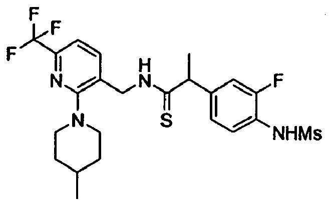

¹H NMR (300 MHz, CDCl₃) δ 8.25 (bs, 1 H), 7.48-7.54 (m, 2 H), 7.10-7.25 (m, 3 H), 4.93 (d, 2 H, J = 5.7 Hz), 4.02 (q, 1 H, J = 6.9 Hz), 3.29 (m, 2 H), 3.02 (s, 3 H), 2.84 (m, 2 H), 1.70 (m, 2 H), 1.67 (d, 3 H, J = 6.9 Hz), 1.50 (m, 1 H), 1.24 (m, 2 H), 0.98 (d, 3 H, J = 6.6 Hz); IR (KBr) 3268, 2924,1592, 1512, 1418, 1333,1157, 1045, 970, 833, 758 cm⁻¹; MS (FAB) *m*/*z* 533(M+H)

### Beispiel 296: N-(2-Azepan-1-yl-6-tert-butyl-pyridin-3-ylmethyl)-2-(3-fluoro-4-methansulfonyl amino-phenyl)-propionamid

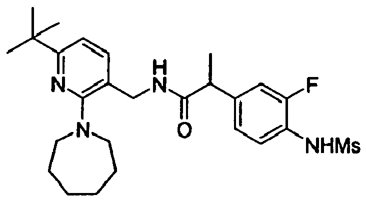

¹H NMR (300 MHz, CDCl₃) δ 7.52 (dd, 1 H, *J* = 8.4, 8.4 Hz), 7.24 (d, 1 H, *J* = 7.7 Hz), 7.16 (dd, 1 H, *J* = 11.3, 2.0 Hz), 7.08 (d, 1 H, *J* = 8.8 Hz), 6.74 (d, 1H, *J* = 7.7 Hz), 6.16 (bs, 1 H), 4.37 (m, 2 H), 3.51 (q, 1 H, *J* = 7.1 Hz), 3.34-3.30 (m, 4 H), 3.02 (s, 3 H), 1.80-1.60 (m, 4 H), 1.58-1.49 (m, 4 H), 1.51 (d, 3 H, *J* = 7.1 Hz), 1.29 (s, 9 H); IR (KBr) 3275, 2926, 1650, 1588, 1513, 1448, 1335 cm⁻¹; MS (FAB) *m*/*z* 505 (M+H)

### Beispiel 297: N-(6-tert-Butyl-2-dipropylamino-pyridin-3-ylmethyl)-2-(3-fluor-4-methansulfonylamino-phenyl)-propionamid

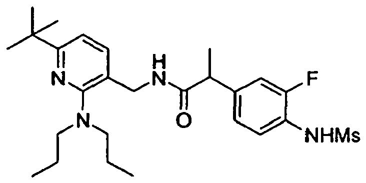

¹H NMR (300 MHz, CDCl₃) δ 7.51 (dd, 1 H, J = 8.3, 8.3 Hz), 7.29 (d, 1 H, J = 7.7 Hz), 7.17 (dd, 1 H, J = 11.3 Hz, 1.8 Hz), 7.08 (d, 1 H, *J* = 8.4 Hz), 6.85 (d, 1 H, J = 7.7 Hz), 6.74 (bs, 1 H), 6.47 (bs, 1 H), 4.47-4.33 (m, 2 H), 3.49 (q, 1 H, J = 7.0 Hz), 3.07-2.96 (m, 7 H), 1.52-1.34 (m, 7 H), 1.29 (s, 9 H), 0.82 (t, 6 H, J = 7.4 Hz); IR (KBr) 3290. 2961, 2871, 1650, 1513, 1456, 1335 cm⁻¹; MS (FAB) m/z 507 (M+H)

### Beispiel 298: N-(2-But-2-enyloxy-6-trifluoromethyl-pyridin-3-ylmethyl)2-(3-fluoro-4-methansulfonylamino-phenyl)-propionamid

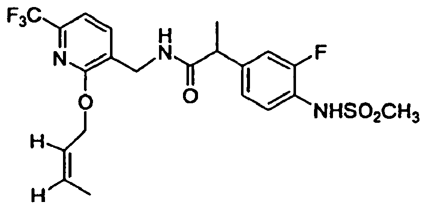

¹H NMR (300 MHz, CDCl₃) δ 7.59 (d, 1 H, J = 8.2 Hz), 7.52 (dd, 1 H, J = 8.3, 8.3 Hz), 7.20 (d, 1 H, J = 7.5 Hz), 7.09 (dd, 1 H, J = 11.5, 1.8 Hz), 7.06 (d, 1 H, J = 9.1 Hz), 6.48 (bs, 1 H), 6.01 (bt, 1 H), 5.76 (m, 1 H), 5.58 (m, 1 H), 4.94 (d, 2 H, J = 6.8 Hz), 4.37 (d, 2 H, *J* = 6.0 Hz), 3.50 (q, 1 H, *J* = 7.0 Hz), 3.02 (s, 3 H), 1.77 (d, 3 H, *J* = 7.0 Hz), 1.48 (d, 3 H, J = 7 .1 Hz); IR (pur) 3289, 2928, 1655, 1602, 1512, 1462, 1422, 1373, 1334, 1265, 1156, 973, 904, 833 cm⁻¹; MS (FAB) *m*/*z* 490 (M+H)

### Beispiel 299: 2-(3-Fluoro-4-methansulfonylamino-phenyl)-N-(2-pent-2-enyloxy-6-trifluoromethyl-pyridin-3-ylmethyl)-propionamid

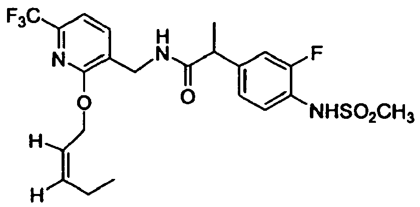

¹H NMR (300 MHz, CDCl₃) δ 7.59 (d, 1 H, *J* = 7.4 Hz), 7.51 (dd, 1 H, *J* = 8.4, 8.4 Hz), 7.20 (d, 1 H, *J* = 8.4 Hz), 7.09 (dd, 1 H, *J* = 11.5, 1.8 Hz), 7.06 (d, 1 H, *J* = 9.2 Hz), 6.49 (s, 1 H), 6.01 (bt, 1 H), 5.67 (m, 1 H), 5.52 (m, 1 H), 4.92 (d, 2 H, *J* = 4.7 Hz), 4.37 (d, 2 H, *J* = 6.4 Hz), 3.50 (q, 1 H, *J* = 6.8 Hz), 3.03 (s, 3 H ), 2.20 (m, 2 H), 1.48 (d, 3 H, *J* = 7.1 Hz), 1.02 (t, 3 H, *J* = 7.5 Hz); IR (pur) 3292, 2971, 1656, 1601, 1512, 1462, 1422, 1338, 1266, 1157, 977, 903, 759 cm⁻¹; MS (FAB ) *m*/*z* 504 (M+H)

### Beispiel 300:_2-(3-Fluoro-4-methansulfonylamino-phenyl)-N-(2-pent-1-enyl-6-trifluoromethyl-pyridin-3-ylmethyl)-propionamid

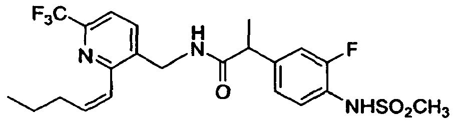

¹H NMR (300 MHz, CDCl₃) δ 7.62 (d, 1 H, *J* = 8.1 Hz), 7.53 (dd, 1 H, *J* = 8.3, 8.3 Hz), 7.45 (d, 1 H, *J* = 7.9 Hz), 7.14 (dd, 1 H, *J* = 11.2, 2.0 Hz), 7.08 (d, 1 H, *J* = 8.4 Hz), 6.52 (bs, 1 H), 5.73 (bt, 1 H), 6.41 (dt, 1 H, *J* = 11.6 Hz), 6.05 (m, 1 H), 5.73 (bt, 1 H), 4.47 (m, 2 H), 3.53 (q, 1 H, *J* = 7.1 Hz), 3.03 (s, 3 H), 2.44 (m, 2 H), 1.51 (d, 3 H, *J* = 7.1 Hz), 1.47 (m, 2 H), 0.92 (t, 3 H, J = 7.3 Hz); IR (pur) 3296, 2927, 1652, 1513, 1458, 1339, 1280, 1156, 973 cm⁻¹; MS (FAB) *m*/*z* 488 (M+H)

### Beispiel 301: 2-(3-Fluoro-4-methansulfonylamino-phenyl)-N-(2-pent-1-enyl-6-trifluoromethyl-pyridin-3-ylmethyl)-propionamid

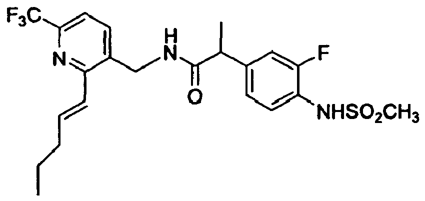

¹H NMR (300 MHz, CDCl₃) δ 7.58 (d, 1 H, *J* = 7.9 Hz), 7.52 (dd, 1 H, *J* = 8.3, 8.3 Hz), 7.41 (d, 1 H, *J* = 7.9 Hz), 7.15 (dd, 1 H, *J* = 11.2, 2.0 Hz), 7.08 (d, 1 H, *J* = 7.9 Hz), 6.51 (dt, 2 H, *J* =15.0 Hz), 5.64 (bt, 1 H), 4.52 (m, 1 H), 3.53 (q, 1 H, *J* = 7.4 Hz), 3.03 (s, 3 H), 2.24 (m, 2 H), 1.59-1.45 (m, 5 H), 0.97 (t, 3 H, *J* = 7.3 Hz); IR (pur) 3291, 2930, 1652, 1587, 1513, 1456, 1412, 1339, 1278, 1156, 973, 936, 838 cm⁻¹; MS (FAB) *m*/*z* 488 (M+H)

### Beispiel 302: 2-(3-Fluoro-4-methansulfonylamino-phenyl)-N-(4-methyl-2-pentyloxy-6-trifluoromethyl-pyridin-3-ylmethyl)-propionamid

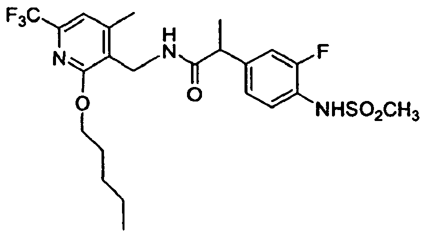

¹H NMR (300 MHz, CDCl₃) δ 7.50 (dd, 1 H, *J* = 8.3, 8.3 Hz), 7.13-7.01 (m, 3 H), 6.03 (bt, 1 H), 4.42 (m, 2 H), 4.29 (m, 2 H), 3.45 (q, 1 H, *J* = 7.0 Hz), 3.02 (s, 3 H), 2.49 (s, 3 H), 1.69 (m, 2 H), 1.45 (d, 3 H, *J* = 7.1 Hz), 1.35 (m, 2 H), 1.25 (m, 2 H), 0.98-0.87 (m, 5 H); IR (pur) 3291, 2926, 1649, 1512, 1459, 1409, 1341, 1291, 1245, 1158, 972, 912, 766 cm⁻¹; MS (FAB) *m*/*z* 520 (M+H)

### Beispiel 303: 2-(3-Fluoro-4-methansulfonylamino-phenyl)-N-{2-[2-(4-fluoro-phenyl)-ethyl]-6-trifluoromethyl-pyridin-3-ylmethyl}-propionamid

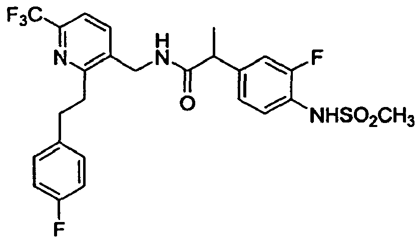

¹H NMR (300 MHz, CDCl₃ δ 7.35-7.56 (m, 3 H), 7.02-7.17 (m, 4 H), 6.88-6.97 (m, 2 H), 6.54 (bs , 1 H), 5.49 (bt , 1 H), 4.24-4.40 (m, 2 H), 3.46 (q , 1 H, *J* = 7.0 Hz), 3.00-3.12 (m , 7 H), 1.49 (d, 3 H, *J* = 7.1 Hz); IR (KBr) 3296, 1652, 1511, 1456, 1338, 1157, 972, 911, 832, 734 cm⁻¹; MS (FAB) *m*/*z* 542 (M+H)

### Beispiel 304: N-(4-Acetyl-4-phenyl-6'-trifluoromethyl-3,4,5,6-tetrahydro-2H-[1,2']bipyridinyl-3'-ylmethyl)-2-(3-fluoro-4-methansulfonylamino-phenyl)-propionamid

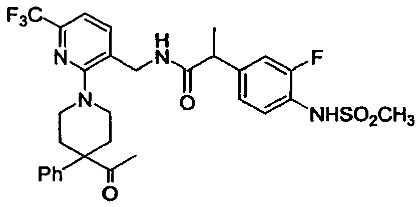

¹H NMR (300 MHz, CDCl₃) δ 7.44-7.53 (m, 2 H), 7.27-7.43 (m, 5 H), 7.21 (d, 1 H, *J* = 7.7 Hz), 7.11 (m , 1 H), 7.05 (d , 1 H, *J* = 8.6 Hz), 6.50 (bs, 1 H), 6.12 (bt , 1 H), 4.46 (d , 2 H, *J* = 5.7 Hz), 3.55 (q, 1 H, *J* = 7.0 Hz), 3.19-3.32 (m, 2 H), 2.97-3.10 (m, 5 H), 2.41-2.54 (m, 2 H), 2.05-2.20 (m, 2 H), 1.95 (s, 3 H), 1.52 (d, 3 H, *J* = 7.0 Hz); IR (KBr) 2928, 1699, 1652, 1592, 1512, 1455, 1420, 1336, 1159, 965, 910, 733 cm⁻¹; MS (FAB) *m*/*z* 621 (M+H)

### Beispiel 307: 2-(3-Fluoro-4-methansulfonylamino-phenyl)-N-[4-(phenyl-propionyl-amino)-6'-trifluoromethyl-3,4,5,6-tetrahydro-2H-[1,2']bipyridinyl-3'-ylmethyl]-propionamid

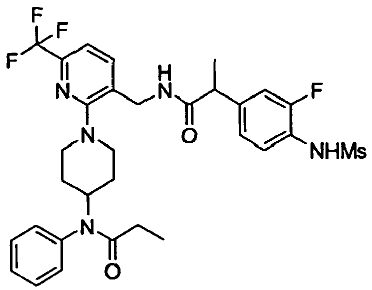

¹H NMR (300 MHz, CDCl₃ δ 7.42-7.52 (m, 6 H), 7.05-7.19 (m, 4 H), 6.99 (d, 1 H, J = 8.1 Hz), 6.16 (bt, 1 H), 4.75 (m, 1 H), 4.36 (d, 2 H, J = 5.7 Hz), 4.12 (q, 2 H, J = 7.2 Hz), 3.48 (q, 1 H, J = 6.9 Hz), 3.34 (m, 2 H), 3.02 (s, 3 H), 2.92 (m, 2 H), 1.90 (m, 2 H), 1.46 (d, 3 H, J = 6.9 Hz), 1.25 (t, 3 H, 7.2 Hz); IR (KBr) 2927, 1639, 1592, 1509, 1456, 1414, 1373, 1337, 1275, 1158, 959, 705 cm⁻¹; MS (FAB) *m*/*z* 650(M+H)

### Beispiel 308: N-[2-(4-Dimethylamino-phenyl)-6-trifluoromethyl-pyridin-3-ylmethyl]-2-(3-fluoro-4-methansulfonylamino-phenyl)-propionamid

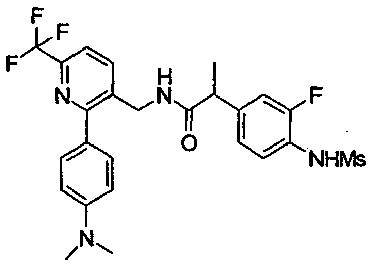

¹H NMR (300 MHz, CDCl₃) δ 7.74 (d, 1 H, J = 7.8 Hz), 7.53 (dd, 1 H, J = 8.1, 8.1 Hz), 7.48 (d, 2 H, J = 8.7 Hz), 7.31 (d, 1 H, J = 7.8 Hz), 7.02 (dd, 1 H, J = 11.1, 1.8 Hz), 6.97 (d, 1 H, J = 8.1 Hz), 6.72 (d, 1 H, 8.7 Hz) (m, 6 H), 6.58 (bs, 1 H), 5.58 (bt, 1 H), 4.57 (d, 2 H, J = 5.7 Hz), 3.44 (q, 1 H, J = 6.9 Hz), 3.01 (s, 6 H), 2.96 (s, 3 H), 1.44 (d, 3 H, J = 6.9 Hz); IR (KBr) 3291, 2926, 1658, 1611, 1514, 1454, 1403, 1339, 1265, 1157, 972, 825, 736 cm⁻¹; MS (FAB) *m*/*z* 539(M+H)

### Beispiel 309: 2-[3-Fluoro-4-(propan-2-sulfonylamino)-phenyl]-N-(4-methyl-6'-trifluoromethyl-3,4,5,6-tetrahydro-2H-[1,2']bipyridinyl-3'-ylmethyl)-propionamid

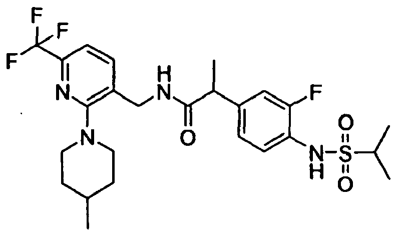

¹H NMR (300 MHz, CDCl₃) δ 7.56 (dd, 1 H, J = 8.1,8.1 Hz), 7.6 (d, 1 H, J = 7.5 Hz), 7.18 (d, 1 H, J = 7.5 Hz), 7.07 (dd, 1 H, J = 11.1, 1.8 Hz), 7.05 (d, 1 H, J = 8.1 Hz), 6.56 (bs, 1 H), 6.33 (bt, 1 H), 4.46 (d, 1 H, J = 5.7 Hz), 3.55 (q, 1 H, J = 6.9 Hz), 3.20-3.34 (m, 3 H), 2.81 (m, 2 H), 1.71 (m, 3 H), 1.50 (d, 3 H, J = 6.9 Hz), 1.39 (d, 6 H, J = 6.9 Hz), 0.97 (d, 3 H, J = 6.3 Hz); IR (KBr) 3273, 2923, 1657, 1592, 1511, 1458, 1419, 1332, 1270, 1142, 909, 732 cm⁻¹; MS (FAB) *m*/*z* 545 (M+H)

### Beispiel 310: 2-[3-Fluoro-4-(2,2,2-trifluoro-ethansulfonylamino)-phenyl]-N-(4-methyl-6'-trifluoromethyl-3,4,5,6-tetrahydro-2H-[1,2']bipyridinyl-3'-ylmethyl)-propionamid

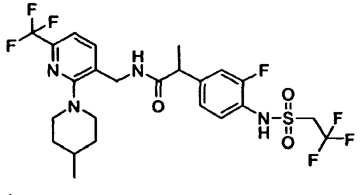

¹H NMR (300 MHz, CDCl₃) δ 7.48-7.53 (m, 2 H), 7.21 (d, 1 H, J = 7.5 Hz), 7.16 (d, 1 H, J = 11.1 Hz), 7.09 (d, 1 H, J = 8.1 Hz), 6.41 (bt, 1 H), 4.48 (d, 2 H, J = 5.7 Hz), 3.84 (m, 2 H), 3.67 (q, 1 H, J = 6.9 Hz), 3.30 (m, 2 H), 2.82 (m, 2 H), 1.72 (m, 3 H), 1.53 (d, 3 H, J = 6.9 Hz), 1.25 (m, 2 H), 0.98 (d, 3 H, J = 6.6 Hz); IR (pur) 2924,1657, 1592, 1512, 1456, 1418, 1358, 1253, 1169, 1134, 1086, 944 cm⁻¹; MS (FAB) *m*/*z* 585 (M+H)

### Beispiel 311: N-[2-(2,6-Dimethyl-morpholin-4-yl)-6-trifluoromethyl-pyridin-3-ylmethyl]-2-(3-fluoro-4-methansulfonylamino-phenyl)-propionamid

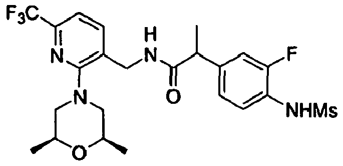

¹H NMR (300 MHz, CDCl₃) δ 7.48-7.52 (m, 2 H), 7.10-7.25 (m, 3 H), 6.52 (bs, 1 H), 6.06 (bt, 1 H), 4.47 (d, 2 H, J = 5.7 Hz), 3.70 (m, 2 H), 3.58 (q, 1 H, J = 6.9 Hz), 3.16 (m, 2 H), 3.04 (s, 3 H), 2.64 (m, 2 H), 1.55 (d, 3 H, J = 6.9 Hz), 1.19 (d, 6 H, J = 6.3 Hz); IR (KBr) 3295, 2977, 1657, 1592, 1512, 1458, 1417, 1334, 1154, 1006, 972, 912, 733 cm⁻¹; MS (FAB) *m*/*z* 533 (M+H)

### Beispiel 312: 2-(3-Fluoro-4-trifluoromethansulfonylamino-phenyl)-N-(4-methyl-6'-trifluoromethyl-3,4,5,6-tetrahydro-2H-[1,2]bipyridinyl-3'-ylmethyl)-propionamid

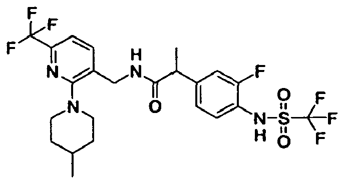

¹H NMR (300 MHz, CDCl₃) δ 7.28-7.51 (m, 2 H), 7.10-7.23 (m, 3 H), 6.56 (bs, 1 H), 6.06 (bt, 1 H), 4.50 (d, 2 H, J = 5.7 Hz), 3.57 (q, 1 H, J = 6.9 Hz), 3.33 (m, 2 H), 2.84 (m, 2 H), 1.73 (m, 2 H), 1.53 (d, 3 H, J = 6.9 Hz), 1.25 (m, 3 H), 0.98 (d, 3 H, J = 6.0 Hz); IR (pur) 2924, 1656, 1593, 1512, 1456, 1423, 1377, 1338, 1232, 1203, 1142, 956, 738 cm⁻¹; MS (FAB) *m*/*z* 571 (M+H)

### Beispiel 313: 2-(3-Fluoro-4-aminosulfonylamino-phenyl)-N-(4-methyl-6'-trifluoromethyl-3,4,5,6-tetrahydro-2H-[1,2']bipyridinyl-3'-ylmethyl)-propionamid

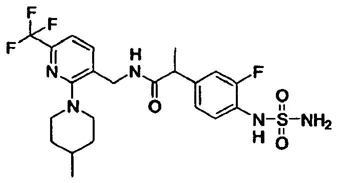

¹H NMR (300 MHz, CDCl₃) δ 7.46-7.51 (m, 2 H), 7.19 (d, 1 H, J = 7.2 Hz), 7.05-7.10 (m, 2 H), 6.83 (bs, 1 H), 6.50 (bt, 1 H), 5.04 (s, 2 H), 4.46 (d, 2 H, J = 5.7 Hz), 3.57 (q, 1 H, J = 6.9 Hz), 3.27 (m, 2 H), 2.81 (m, 2 H), 1.78 (m, 2 H), 1.51 (d, 3 H, J = 6.9 Hz), 1.25 (m, 3 H), 0.97 (d, 3 H, J = 6.0 Hz); IR (KBr) 3292, 2924, 1653, 1592, 1514, 1455, 1418, 1339, 1169, 944, 833, 736 cm⁻¹; MS (FAB) *m*/*z* 571 (M+H)

### Beispiel 314: N-[2-(1,1-Dioxo-1l6-thiomorpholin-4-yl)-6-trifluoromethyl-pyridin-3-ylmethyl]-2-(3-fluoro-4-methansulfonylamino-phenyl)-propionamid

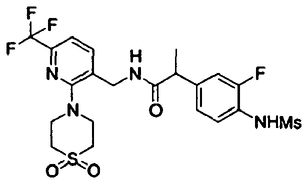

¹H NMR (300 MHz, CDCl₃) δ 7.50-7.56 (m, 2 H), 7.33 (d, 1 H, J = 7.5 Hz), 7.09-7.17 (m, 2 H), 5.94 (bt, 1 H), 4.46 (d, 2 H, J = 5.7 Hz), 3.72 (m, 4 H), 3.60 (q, 1 H, J = 6.9 Hz), 3.16 (m, 4 H), 3.02 (s, 3 H), 1.54 (d, 3 H, J = 6.9 Hz); IR (KBr) 3369, 2933, 1659, 1590, 1514, 1462, 1415, 1333, 1278, 1124, 974, 912, 732 cm⁻¹; MS (FAB) *m*/*z* 553 (M+H)

### Beispiel 315: N-(6'-Difluoromethyl-4-methyl-3,4,5,6-tetrahydro-2H-[1,2']bipyridinyl-3'-ylmethyl)-2-(3-fluoro-4-methansulfonylamino-phenyl)-propionamid

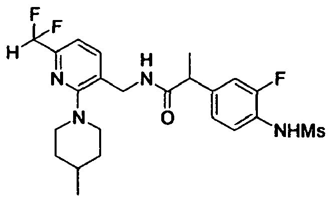

¹H NMR (300 MHz, CDCl₃) δ 7.47-7.54 (m, 2 H), 7.06-7.19 (m, 3 H), 6.48 (t, 1 H, J = 55.0 Hz), 6.41 (bs, 1 H), 6.06 (bt, 1 H), 4.46 (d, 2 H, J = 5.7 Hz), 3.55 (q, 1 H, J = 6.9 Hz), 3.25 (m, 2 H), 3.02 (s, 3 H), 2.79 (m, 2 H), 1.71 (m, 2 H), 1.52 (d, 3 H, J = 6.9 Hz), 1.25 (m, 3 H), 0.97 (d, 6 H, J = 6.0 Hz); IR (KBr) 3291, 2922, 1652, 1589, 1512, 1421, 1334, 1158, 1087, 1041, 970, 795 cm⁻¹; MS (FAB) *m*/*z 499* (M+H)

### Beispiel 316: N-(4,6'-Dimethyl-3,4,5,6-tetrahydro-2H-[1,2]bipyridinyl-3'-ylmethyl)-2-(3-fluoro-4-methansulfonylamino-phenyl)-propionamid

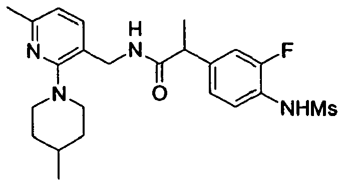

¹H NMR (300 MHz, CDCl₃) δ 7.50 (dd, 1 H, J = 8.1, 8.1 Hz), 7.28 (d, 1 H, J = 7.5 Hz), 7.13 (dd, 1 H, J = 8.1, 1.8 Hz), 7.07 (d, 1 H, J= 8.1 Hz), 6.75 (d, 1 H, J= 7.5 Hz), 6.72 (bs, 1 H), 6.46 (bt, 1 H), 4.40 (d, 2 H, J = 5.7 Hz), 3.51 (q, 1 H, J = 6.9 Hz), 3.18 (m, 2 H), 3.02 (s, 3 H), 2.77 (m, 2 H), 2.42 (s, 3 H), 1.72 (m, 2 H), 1.50 (d, 3 H, J = 6.9 Hz), 1.26 (m, 3 H), 0.97 (d, 6 H, J= 6.0 Hz); IR (KBr) 3289, 2922, 1651, 1584, 1511, 1453, 1374, 1333, 1157, 1115, 971, 735 cm⁻¹; MS (FAB) m/z 463(M+H)

### Beispiel 317: 2-(3-Fluoro-4-methansulfonylamino-phenyl)-N-(4-phenyl-6'-trifluoromethyl-3,6-dihydro-2H-[1,2']bipyridinyl-3'-ylmethyl)-propionamid

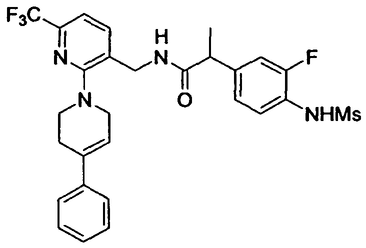

¹H NMR (300 MHz, CDCl₃) δ 7.21-7.54 (m, 7 H), 7.02-7.16 (m, 3 H), 6.63 (bs, 1 H), 6.35 (bt, 1 H), 6.18 (m, 1 H), 4.52 (d, 2 H, J = 5.7 Hz), 3.87 (d, 2 H, J = 2.7 Hz), 3.36-3.59 (m, 3 H), 2.99 (s, 3 H), 2.67 (m, 2 H), 1.52 (d, 3 H, J = 6.9 Hz) IR (KBr) 3293, 2930, 1656, 1592, 1512, 1421, 1336, 1274, 1229, 1157, 970, 833, 755, 697 cm⁻¹; MS (FAB) *m*/*z* 577(M+H)

### Beispiel 318: N-(4,4'-Dimethyl-6'-trifluoromethyl-3,4,5,6-tetrahydro-2H-[1,2]bipyridinyl-3'-ylmethyl)-2-(3-fluoro-4-methansulfonylamino-phenyl)-propionamid

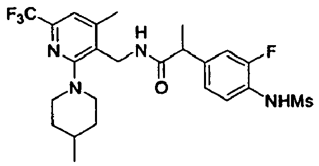

¹H NMR (300 MHz, CDCl₃) δ 7.48 (dd, 1 H, J = 8.1,8.1 Hz), 7.14 (s, 1 H), 7.02-7.07 (m, 2 H), 6.80 (bs, 1 H), 4.52 (d, 2 H, J = 5.7 Hz), 3.48 (q, 1 H, J = 6.9 Hz), 3.17 (m, 1 H), 2.01-3.04 (m, 4 H), 2.79 (m, 2 H), 2.38 (m, 2 H), 1.70 (m, 2 H), 1.47 (d, 3 H, J = 6.9 Hz), 1.13-1.25 (m, 3 H), 0.97 (d, 3 H, J = 6.3 Hz); IR (KBr) 3302, 2923, 1644, 1512, 1451, 1408, 1333, 1280, 1159, 975, 759 cm⁻¹; MS (FAB) *m*/*z 531*(M+H)

### Beispiel 319: N-[2-(4-Cyclohexyl-piperazin-1-yl)-4-trifluoromethyl-benzyl]-2-(3-fluoro-4- methansulfonylamino-phenyl)-propionamid

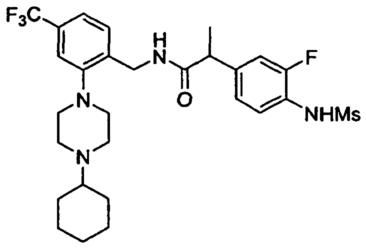

¹H NMR (300 MHz, CDCl₃) δ 7.52 (t, 1 H, J = 8.2 Hz), 7.31 (s, 1 H), 7.26~7.28 (m, 2 H), 7.08~7.16 (m, 2 H), 6.42 (bs, 1 H), 4.52 (d, 2 H, J = 5.9), 3.54 (q, 1 H, J = 7.1 Hz), 3.0 (s, 3 H), 2.88~2.95 (m, 4 H), 2.67 (s, 3 H), 1.81~1.90 (m, 3 H), 1.64 (m, 2 H), 1.52 (d, 3 H, J = 7.0 Hz), 1.20~1.30 (m, 5 H), 0.89~0.92 (m, 2 H); IR (KBr) 3294, 2855, 1654, 1509, 1424, 975, 910, 734 cm⁻¹; MS (FAB) *m*/*z* 585 (M+H)

### Beispiel 320: N-(4'-tert-Butyl-5-trifluoromethyl-biphenyl-2-ylmethyl)-2-(3-fluoro-4-methansulfonylamino-phenyl)-propionamid

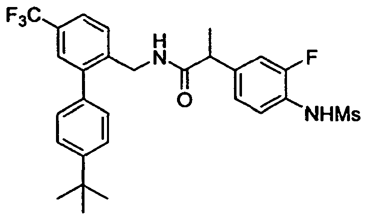

¹H NMR (300 MHz, CDCl₃) δ 7.28∼7.55 (m, 6 H), 7.16 (d, 2 H, J = 7.4 Hz), 7.07 (dd, 1 H, J = 11.2, 1.8 Hz), 6.97~7.02 (m, 1 H), 5.51 (bt, 1 H), 4.41~4.51 (m, 2 H), 3.43 (q, 1H, J = 7.1 Hz), 3.0 (s, 3 H), 1.44 (d, 3 H, J = 7.1 Hz), 1.36 (s, 9 H); IR (KBr) 2965, 1460, 1259, 1078, 979, 908, 836, 734 cm⁻¹; MS (FAB) *m*/*z* 551 (M+H)

### Beispiel 321: 2-(3-Fluoro-4-methansulfonylamino-phenyl)-N-(4'-methoxy-5-trifluoromethyl-biphenyl-2-ylmethyl)-propionamid

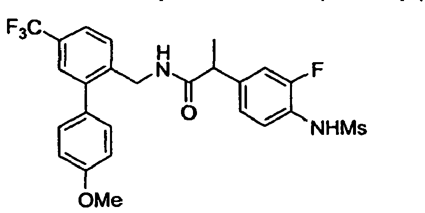

¹H NMR (300 MHz, CDCl₃) δ 7.46∼7.54 (m, 3 H), 7.39 (d, 1 H, J = 8.0 Hz), 7.14 (dd, 2 H, J = 6.4, 2.0 Hz), 7.05 (dd, 1 H, J = 11.0, 1.8 Hz), 6.99 (d, 1 H, J = 8.3 Hz), 6.93 (dd, 2 H, J = 6.8, 2.2 Hz), 5.46 (bt, 1 H), 4.43 (t, 2 H, J = 3.7 Hz), 3.86 (s, 3 H), 3.43 (q, 1 H, J = 7.5Hz), 3.02 (s, 3 H), 1.44 (d, 3 H, J = 7.0 Hz); IR (KBr) 3295,1422, 1252, 1042, 973, 907, 835, 732 cm⁻¹; MS (FAB) *m*/*z* 525(M+H)

### Beispiel 322: N-(3'-Chloro-5-trifluoromethyl-biphenyl-2-ylmethyl)-2-(3-fluoro-4 methansulfonylamino-phenyl)-propionamid

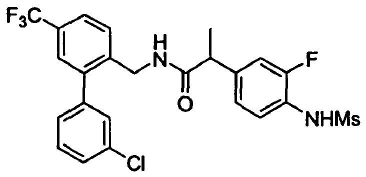

¹H NMR (300 MHz, CDCl₃) δ 7.58∼7.32 (m, 6 H), 7.23 (m, 1 H), 7.14∼7.00 (m, 3 H), 5.61 (b t, 1 H), 4.39 (t, 2 H, J = 5.5 Hz), 3.46 (q, 1 H, J = 7.1 Hz), 3.0 (s, 3H), 1.45 (d, 3H, J = 7.1 Hz); IR (KBr) 3290, 1651, 1421, 1078, 1041, 974, 908, 732 cm⁻¹; MS (FAB) *m*/*z* 528 (M+H)

### Beispiel 323: 2-(3-Fluoro-4-methansulfonylamino-phenyl)-N-(3'-fluoro-5-trifluoromethyl-biphenyl-2-ylmethyl)-propionamid

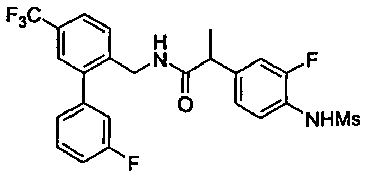

¹H NMR (300 MHz, CDCl₃) δ 7.38~7.59 (m, 5 H), 7.00∼7.09 (m, 4 H), 6.93 (d, 1 H, J =10.4 Hz) 4.39 (m, 2 H), 3.45 (q, 1 H, J = 7.3Hz), 3.03 (s, 3 H), 1.46 (d, 3 H, J = 7.1 Hz); IR (KBr) 3289, 1586, 1446, 1277, 1078, 973, 907, 733 cm⁻¹; MS (FAB) *m*/*z* 513 (M+H)

### Beispiel 324: N-(3'-Chloro-4'-fluoro-5-trifluoromethyl-biphenyl-2-ylmethyl)-2-(3-fluoro-4-methansulfonylamino-phenyl)-propionamid

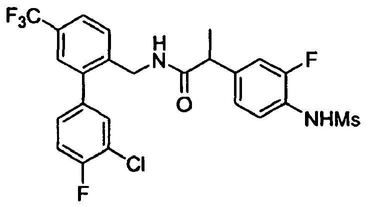

¹H NMR (300 MHz, CDCl₃) δ 7.49∼7.59 (m, 2 H), 7.37~7.44 (m, 2 H), 7.02~7.22 (m, 5 H), 5.54 (bt, 1 H), 4.38 (d, 2 H, J = 6.0Hz), 3.49 (q, 1 H, J = 7.0Hz), 3.04 (s, 3 H), 1.47 (d, 3 H, J = 7.1 Hz); IR (KBr) 3246, 1420, 1265, 1077, 973, 908, 828, 732 cm⁻¹; MS (FAB) *m*/*z* 548 (M+H)

### Beispiel 325: N-(3',4'-Dimethoxy-5-trifluoromethyl-biphenyl-2-ylmethyl)-2-(3-fluoro-4-methansulfonylamino-phenyl)-propionamid

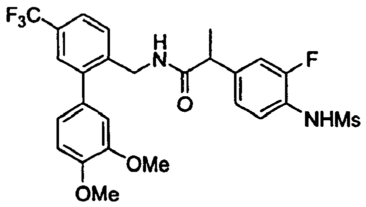

¹H NMR (300 MHz, CDCl₃) δ 7.43~7.53 (m, 3 H), 7.38 (d, 1 H, J = 8.1 Hz), 7.07 (dd, 1 H, J = 11.3, 2.0 Hz), 7.0 (d, 1 H, J = 8.2 Hz), 6.90 (d, 1 H, J = 8.2 Hz), 6.74∼6.77 (m, 2 H), 5.72 (bs, 1 H), 4.44 (m, 2 H), 3.92 (s, 3 H), 3.86 (s, 3 H), 3.46 (q, 1 H, J = 7.1 Hz), 3.01 (s, 3 H), 1.44 (d, 3 H, J = 7.1 Hz); IR (KBr) 2936, 1423, 1078, 1025, 974, 908, 765, 732 cm⁻¹; MS (FAB) *m*/*z* 555 (M+H)

### Beispiel 326: N-[2-(3,4-Dimethoxy-phenyl)-6-trifluoromethyl-pyridin-3-ylmethyl]-2-(3-fluoro-4-methansulfonylamino-phenyl)-propionamid

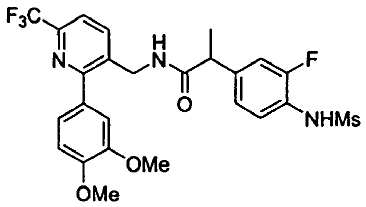

¹H NMR (300 MHz, CDCl₃) δ 7.76 (d, 1 H, J = 8.1 Hz), 7.57 (d, 1 H, J = 8.0 Hz), 7.45 (t, 1 H, J = 8.3 Hz), 6.79-7.08 (m, 5 H), 5.90 (bt, 1 H), 4.53 (d, 2 H, J = 5.5 Hz), 3.91 (s, 3 H), 3.88 (s, 3 H), 3.49 (q, 1 H, J = 6.9 Hz), 3.02 (s, 3 H), 1.43 (d, 3 H, J = 7.2 Hz); IR (KBr) 3271, 2937, 1587, 1416, 1025, 972, 913 cm⁻¹; MS (FAB) *m*/*z* 556 (M+H)

### Beispiel 327: 4-(3-{[2-(3-Fluoro-4-methansulfonylamino-phenyl)-propionylamino]-methyl}-6-trifluoromethyl-pyridin-2-yloxymethyl)-piperidin-1-carbonsäure tert-butyl ester

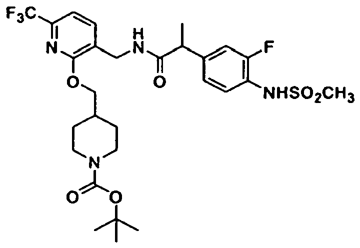

¹H NMR (CDCl₃) δ 7.60 d, 1 H, J = 7.5 Hz), 7.52 (dd, 1 H, J = 8.4, 8.4 Hz), 7.21 (d, 1 H, J = 7.5 Hz), 7.12-7.05 (m, 2 H), 5.83 (bs, N H), 4.37(d, 2 H, J = 5.9 Hz), 4.25-4.07 (m, 4 H), 3.53 (q, 1 H, J = 6.4 Hz), 3.04 (s, 3 H), 2.78-2.63 (m, 2 H), 1.90 (m, 1 H), 1.68-1.55 (m, 2 H), 1.48 (s, 9 H), 1.25-1.05 (m, 2 H); IR (pur) 3303, 2935, 1665, 1426, 1359, 1271, 1157, 757 cm⁻¹; MS (FAB) m/z 633 (M+H)

### Beispiel 331: N-(2-Dipropylamino-6-trifluoromethyl-pyridin-3-ylmethyl)-2-(4-methansulfonylamino-3-methyl-phenyl)-propionamid

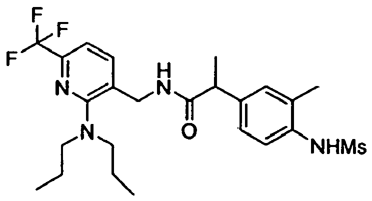

¹H NMR (300 MHz, CDCl₃) δ 7.36-7.41 (m, 2 H), 7.08-7.18 (m, 4 H), 6.29 (bt, 1 H), 4.42 (d, 2 H, J = 5.7 Hz), 3.56 (q, 1 H, J = 6.9 Hz), 3.09 (t, 4 H, J = 7.5 Hz), 2.99 (s, 3 H), 2.32 (s, 3 H), 2.82 (m, 2 H), 1.52 (d, 3 H, J = 6.9 Hz), 1.43 (m, 4 H), 0.82 (t, 6 H, J = 7.5 Hz); IR (pur) 3272, 2965, 1655, 1594, 1503, 1460, 1419, 1331, 1152, 1027, 895, 825, 762 cm⁻¹; MS (FAB) m/z 563 (M+H)

### Beispiel 339: 2-(3-Fluoro-4-methansulfonylamino-phenyl)-N-[6-trifluoromethyl-2-(4-trifluoromethyl-cyclohexyloxy)-pyridin-3-ylmethyl]-propionamid

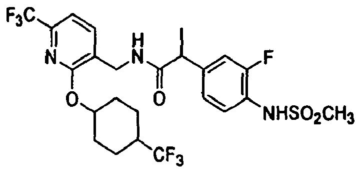

¹H NMR (CDCl₃) δ 7.59 (d, 1 H, J = 7.0 Hz), 7.51 (dd, 1 H, J=8.2, 8.2 Hz), 7.20 (d, 1 H, J = 7.5 Hz), 7.13-7.05 (m, 2 H), 6.50 (bs, NH), 5.91 (bt, NH), 5.43 (m, 1 H), 4.39 (m, 2 H), 3.51 (q, 1 H, J = 6.6 Hz), 3.03 (s, 3 H), 2.20-2.08 (m, 3 H), 1.85-1.77 (m, 2 H), 1.63-1.50 (m, 4 H), 1.49 (d, 3 H, J = 7.1 Hz); IR (pur) 3293, 2953, 1658, 1513, 1422, 1343, 1264, 1141, 970 cm⁻¹; MS (FAB) m/z 586 (M+H)

### Beispiel 340: 2-(3-Fluoro-4-methansulfonylamino-phenyl)-N-[6-trifluoromethyl-2-(4-trifluoromethyl-cyclohexyloxy)-pyridin-3-ylmethyl]-propionamid

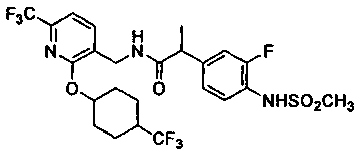

¹H NMR (CDCl₃) δ 7.57-7.50 (m, 2 H), 7.19 (d, 1 H, J = 7.3 Hz), 7.13-7.06 (m, 2 H), 5.90 (bt, NH), 5.03 (m, 1 H), 4.36 (m, 2 H), 3.53 (q, 1 H, J = 7.4 Hz), 3.05 (s, 3 H), 2.28-2.00 (m, 4 H), 1.62-1.25 (m, 5 H), 1.50(d, 3 H, J = 7.1 Hz); IR (pur) 3288, 2952, 1658, 1512, 1422, 1365, 1338, 1275, 1156, 975 cm⁻¹; MS (FAB) m/z 586 (M+H)

### Beispiel 341: 4-(3-{[2-(3-Fluoro-4-methansulfonylamino-phenyl)-propionylamino]-methyl}-6-trifluoromethyl-pyridin-2-yloxy)-piperidin-1-carbonsäure tert-butyl ester

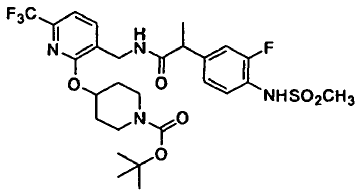

¹H NMR (CDCl₃) δ 7.60 (d, 1 H, J = 7.3 Hz), 7.50 (dd, 1 H, J = 8.2, 8.2 Hz), 7.20 (d, 1 H, J = 7.3 Hz), 7.13-7.04 (m, 2 H), 5.87 (bt, NH), 5.24 (m, 1 H), 4.36 (d, 2 H), 3.70-3.62 (m, 2 H), 3.54 (q, 1 H, J = 7.7 Hz), 3.28-3.17 (m, 2 H), 3.04 (s, 3 H), 1.98-1.88 (m, 2 H), 1.54-1.40 (m, 2 H), 1.51 (d, 3 H), 1.50 (s, 9 H); IR (pur) 3301, 2977, 1665, 1420, 1337, 1276, 1163, 1027 cm⁻¹; MS (FAB) m/z 619 (M+H)

### Beispiel 342: 4-[(3-{[2-(3-Fluoro-4-methansulfonylamino-phenyl)-propionylamino]-methyl}-6-trifluoromethyl-pyridin-2-ylamino)-methyl]-piperidin-1-carbonsäure tert-butyl ester

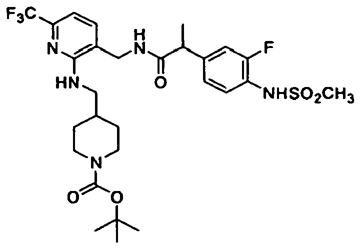

¹H NMR (CDCl₃) δ 7.48(dd, 1 H, J = 8.2, 8.2 Hz), 7.25 (d, 1 H), 7.16 (d, 1 H), 7.06 (d, 1 H), 6.77 (d, 1 H, J = 7.3 Hz), 6.21 (bs, NH), 5.93 (bs, NH), 4.32 (m, 2 H), 4.06 (m, 2 H), 3.49 (q, 1 H, J = 7.3 Hz), 3.32 (m, 2 H), 2.66 (m, 2 H), 1.76 (m, 2 H), 1.51 (d, 3 H, J = 7.0 Hz), 1.46 (s, 9 H); IR (pur) 3303, 2927, 1658, 1611, 1515, 1428, 1335, 1161, 734 cm⁻¹; MS (FAB) m/z 632 (M+H)

### Beispiel 343: 2-(3-Fluoro-4-methansulfonylamino-phenyl)-N-[2-(piperidin-4-ylmethoxy)-6-trifluoromethyl-pyridin-3-ylmethyl]-propionamid

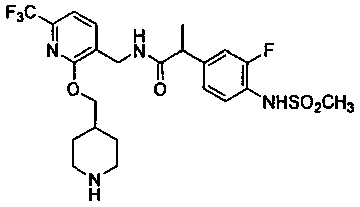

¹H NMR (CDCl₃) δ 7.58 (d, 1 H, J = 7.9 Hz), 7.43 (dd, 1 H, J= 8.3, 8.3 Hz), 7.29 (d, 1 H, J = 7.4 Hz), 7.22-7.15 (m, 2 H), 4.47-4.23 (m, 4 H), 3.73(q, 1 H, J = 7.1 Hz), 3.43-3.36 (m, 2 H), 3.05-2.93 (m, 2 H), 3.00 (s, 3 H), 2.04-1.96 (m, 3 H), 1.53-1.45 (m, 2H), 1.46 (d, 3 H, J = 7.1 Hz); IR (pur) 3405, 2923, 1674, 1512, 1425, 1334, 1270, 1153 cm⁻¹ ; MS (FAB) m/z 533 (M+H)

### Beispiel 344: 2-(3-Fluoro-4-methansulfonylamino-phenyl)-N-[2-(piperidin-4-yloxy)-6-trifluoromethyl-pyridin-3-ylmethyl]-propionamid

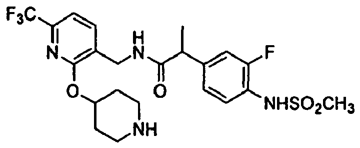

¹H NMR (CDCl₃) δ 7.58 (d, 1 H, J = 7.5 Hz), 7.41 (dd, 1 H, J = 8.3, 8.3 Hz), 7.27 (d, 1 H, J = 7.5 Hz), 7.19-7.11 (m, 2 H), 5.29 (m, 1 H), 4.36 (m, 2 H), 3.71 (q, 1 H, J = 7.0 Hz), 3.20 (m, 2 H), 3.01-2.90 (m, 2 H), 2.97 (s, 3 H), 2.06 (m, 2 H), 1.81 (m, 2 H), 1.45 (d, 3 H, J = 7.1 Hz); IR (pur) 3397, 2923, 1657, 1505, 1421, 1292, 1115, 987 cm⁻¹; MS (FAB) m/z 519 (M+H)

### Beispiel 345: 2-(3-Fluoro-4-methansulfonylamino-phenyl)-N-(2-p-tolyloxy-6-trifluoromethyl-pyridin-3-ylmethyl)-propionamid

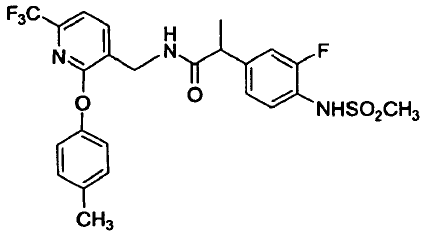

¹H NMR (300 MHz, CDCl₃) δ 7.74 (J = 7.5 Hz),7.47 (dd, 1 H, *J* = 8.4, 8.4 Hz), 7.31 (d, 1 H, *J* = 7.9 Hz), 7.18 (d, 2 H, *J* = 8.8 Hz), 7.01 (m, 2 H), 6.91 (m, 2 H), 4.49 (m, 2 H), 3.58 (q, 1 H, *J* = 7.0 Hz), 2.94 (s, 3 H), 1.49 (d, 3 H, *J* = 7.1 Hz); IR (pur) 3292, 1655, 1593, 1509, 1465, 1406, 1336, 1260, 1156, 972, 940, 831 cm⁻¹; MS (FAB) *m*/*z* 526 (M+H)

### Beispiel 346: N-[2-(2-Cyclohexyl-vinyl)-6-trifluoromethyl-pyridin-3-ylmethyl]-2-(3-fluoro-4-methansulfonylamino-phenyl)-propionamid

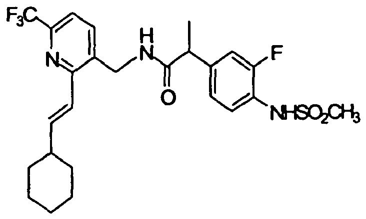

¹H NMR (300 MHz, CDCl₃) δ 7.57 (d, 1 H, *J* = 7.9 Hz), 7.52 (dd, 1 H, *J* = 8.3, 8.3 Hz), 7.41 (d, 1 H, *J* = 7.9 Hz), 7.15 (dd, 1 H, *J* = 11.3, 2.0 Hz), 7.08 (d, 1 H, *J* = 7.0 Hz), 6.49 (m, 2 H), 5.64 (bt, 1 H), 4.52 (m, 2 H), 3.53 (q, 1 H, *J* = 7.0 Hz), 3.03 (s, 3 H), 2.17 (m, 1 H), 1.85-1.73 (m, 4 H), 1.52 (d, 3 H, *J* = 7.1 Hz), 1.34-1.23 (m, 6 H); IR (pur) 3292, 2927, 2853, 1651, 1588, 1513, 1452, 1412, 1340, 1157, 973, 843 cm⁻¹; MS (FAB ) *m*/*z* 528 (M+H)

### Beispiel 347: 2-(3-Fluoro-4-methansulfonylamino-phenyl)-N-(4-methyl-6'-trifluoromethyl-3,4,5,6-tetrahydro-2H-[1,2']bipyridinyl-3'-ylmethyl)-butyramid

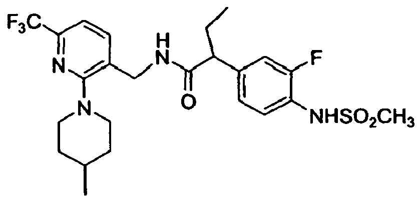

¹H NMR (300 MHz, CDCl₃) δ 7.51 (dd, 1 H, *J* = 8.3, 8.3 Hz), 7.49 (d, 1 H, *J* = 8.3 Hz), 7.18 (m, 2 H), 7.08 (d, 1 H, *J* = 8.3 Hz), 6.51 (bs, 1 H), 6.34 (bt, 1 H), 4.47 (m, 2 H), 3.31 (m, 2 H), 3.21 (t, 1 H, *J* = 7.7 Hz), 3.03 (s, 3 H), 2.83 (m, 2 H), 2.16 (m, 1 H), 1.80 (m, 1 H), 1.73 (m, 2 H), 1.55 (m, 1 H), 1.26 (m, 2 H), 0.98 (d, 3 H, *J* = 6.6 Hz), 0.91 (t, 3 H, *J* = 7.5 Hz); IR (pur) 3291, 2925, 1652, 1592, 1512, 1456, 1419, 1335, 1272, 1157, 969, 832 cm⁻¹; MS (FAB) *m*/*z* 531 (M+H)

### Beispiel 348: N-[2-(3,5-Dimethoxy-phenyl)-6-trifluoromethyl-pyridin-3-ylmethyl]-2-(3-fluoro-4-methansulfonylamino-phenyl)-propionamid

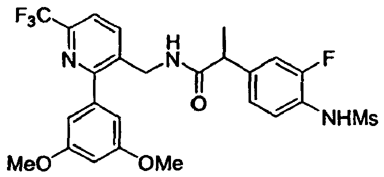

¹H NMR (300 MHz, CDCl₃) δ 7.81 (d, 1 H, J = 8.0 Hz), 7.61 (d, 1 H, J = 8.0 Hz), 7.49 (t, 1 H, J = 8.4 Hz), 6.98~7.07 (m, 2 H), 6.51 (s, 3 H), 5.64 (bt, 1 H), 4.49 (d, 2 H, J = 3.8 Hz), 3.81 (s, 6 H), 3.46 (q, 1 H, J = 7.1 Hz), 3.03 (s, 3 H), 1.45 (d, 3 H, J = 7.1 Hz); IR (KBr) 3293, 2931, 1655, 1458, 1402, 973, 911, 732 cm⁻¹; MS (FAB) *m*/*z* 556 (M+H)

### Beispiel 349: N-(2-Cyclopentyloxy-4-methyl-6-trifluoromethyl-pyridin-3-ylmethyl)-2-(3-fluoro-4-methansulfonylamino-phenyl)-propionamid)

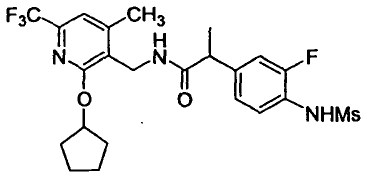

¹H NMR (300 MHz, CDCl₃) δ 7.49 (m, 1 H), 7.00~7.07 (m, 3 H), 6.05 (bt, 1 H), 5.43 (m, 1 H), 4.39 (m, 2 H), 3.47 (q, 1 H, J = 7.1 Hz), 3.02 (s, 3 H), 2.47 (s, 3 H), 1.96 (m, 2 H), 1.58~1.65 (m, 6 H), 1.45 (d, 3 H, J = 7.1 Hz); IR (KBr) 3271, 2967, 1290, 1246. 1093, 973, 911, 731 cm⁻¹; MS (FAB) *m*/*z* 518 (M+H)

### Beispiel 350: N-(3',5'-Dimethoxy-5-trifluoromethyl-biphenyl-2-ylmethyl)-2-(3-fluoro-4-methansulfonylamino-phenyl)-propionamid

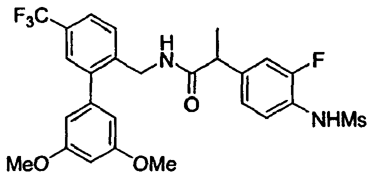

¹H NMR (300 MHz, CDCl₃) δ 7.40~7.56 (m, 4 H), 6.98~7.08 (m, 2 H), 6.48 (t, 1 H, J = 2.4 Hz), 6.35 (d, 2 H, J = 2.2 Hz), 5.56 (bt, 1 H), 4.43 (t, 2 H, J = 5.5 Hz), 3.81 (s, 6 H), 3.43 (q, 1 H, J = 7.2 Hz), 3.02 (s, 3 H), 1.44 (d, 3 H, J = 7.1 Hz); IR (KBr) 3298, 1651, 1512, 1455, 1207, 1078, 907 cm⁻¹; MS (FAB) *m*/*z* 555 (M+H)

### Beispiel 351: 5-{[2-(3-Fluoro-4-methansulfonylamino-phenyl)-propionylamino]-methyl}-4-(4-methyl-piperidin-1-yl)-2-trifluoromethyl-benzoesäure ethyl ester

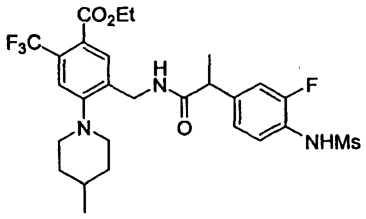

¹H NMR (300 MHz, CDCl₃) δ 7.74 (s, 1 H), 7.48 (t, 1 H, J = 8.4 Hz), 7.11~7.20 (m, 2 H), 4.38~4.31 (m, 4 H), 3.68~3.59 (m, 3 H), 3.02 (s, 3 H), 2.83~2.92 (m, 2 H), 1.74∼1.52 (m, 3 H), 1.53 (d, 3 H, J = 7.1 Hz), 1.36 (t, 3 H, J = 7.1 Hz), 1.29~1.26 (m, 2 H), 0.97 (d, 3 H, J = 6.4 Hz); IR (KBr) 3364, 2927, 1725, 1373, 1031, 916, 796, 732 cm⁻¹; MS (FAB) *m*/*z* 589 (M+H)

### Beispiel 352: N-[2-(1-Butyl-pentyloxy)-6-trifluoromethyl-pyridin-3-ylmethyl]-2-(3-fluoro-4-methansulfonylamino-phenyl)-propionamid

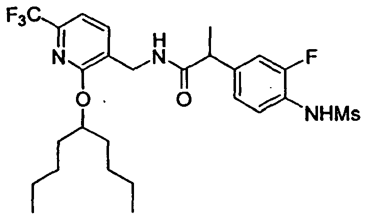

¹H NMR (300 MHz, CDCl₃) δ 7.49~7.56 (m, 2 H), 7.15 (d, 1 H, J = 7.7 Hz), 7.08 (t, 1 H, J = 5.9 Hz), 6.47 (bs, 1 H), 5.98 (bt, 1 H), 5.29 (m, 1 H), 4.37 (m, 2 H), 3.49 (q, 1 H, J = 7.0 Hz), 3.03 (s, 3H), 1.57 (m, 2 H), 1.49 (d, 3 H, J = 7.0 Hz), 1.24~1.31 (m, 8 H), 0.88~0.90 (m, 6 H); IR (pur) 3295, 2933, 2865, 1601, 1513, 1463, 1269, 974 cm⁻¹; MS (FAB) *m*/*z* 562(M+H)

### Beispiel 353: N-(6-tert-Butyl-2-isobutoxy-pyridin-3-ylmethyl)-2-(3-fluoro-4-methansulfonylamino-phenyl)-propionamid

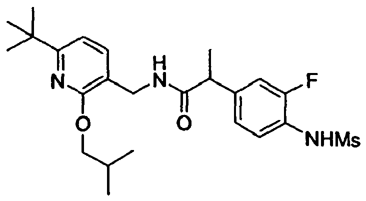

¹H NMR (300 MHz, CDCl₃) δ 7.49 (t, 1 H, J = 8.4 Hz), 7.36 (d, 1 H, J = 7.5 Hz), 7.04∼7.15 (m, 2 H), 6.79 (d, 1 H, J = 7.5 Hz), 6.06 (bt, 1 H), 4.32 (m, 2 H), 4.05∼4.16 (m, 3 H), 3.48 (q, 1 H, J = 7.1 Hz), 3.02 (s, 3 H), 1.48 (d, 3 H, J = 7.1 Hz), 1.29 (s, 9 H), 0.97 (d, 6 H, J = 6.6 Hz); IR (KBr) 3291, 1585, 1410, 1254, 1119, 1019, 972, 732 cm⁻¹; MS (FAB) *m*/*z* 480 (M+H)

### Beispiel 354: 2-(3-Fluoro-4-methansulfonylamino-phenyl)-N-(2-phenylethinyl-6-trifluoromethyl-pyridin-3-ylmethyl)-propionamid

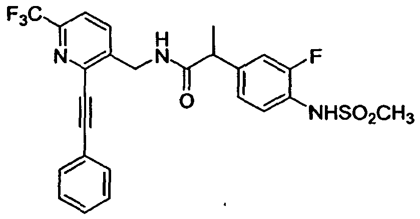

¹H NMR (300 MHz, CDCl₃) δ 7.80 (d , 1 H, *J* = 8.0 Hz), 7.58 (d, 1 H, *J* = 8.1 Hz), 7.49-7.55 (m, 2 H), 7.35-7.48 (m, 4 H), 7.00-7.11 (m , 2 H), 6.08 (bt, 1 H), 4.65 (d, 2 H, *J* = 6.0 Hz), 3.56 (q , 1 H, *J* = 7.0 Hz), 3.00 (s , 3 H), 1.49 (d, 3 H, *J* = 7.1 Hz); IR (KBr) 3297, 2220, 1657, 1513, 1454, 1405, 1340, 1153, 1115, 972, 912, 759, 731 cm⁻¹; MS (FAB) *m*/*z* 520 (M+H)

### Beispiel 355: 2-(3-Fluoro-4-methansulfonylamino-phenyl)-N-[2-(3-methoxy-propoxy)-6-trifluoromethyl-pyridin-3-ylmethyl]-propionamid (SJS-284)

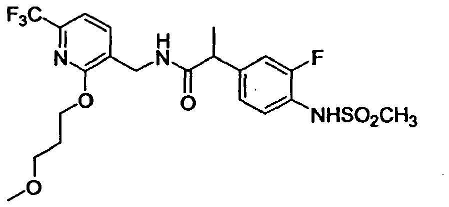

¹H NMR (300 MHz, CDCl₃) δ 7.61 (d, 1 H, *J* = 7.3 Hz), 7.49 (dd, 1 H, *J* = 8.1, 8.1 Hz), 7.20 (d, 1 H, *J* = 7.3 Hz), 7.02-7.11 (m, 2 H), 6.44 (bt, 1 H), 4.47-4.50 (m, 2 H), 4.34 (d, 2 H, *J* = 6.0 Hz), 3.42-3.61 (m, 3 H), 3.36 (s, 3 H), 3.03 (s, 3 H), 1.89-2.01 (m, 2 H), 1.47 (d, 3 H, *J* = 7.1 Hz); IR (KBr) 3296, 2924, 1656, 1603, 1513, 1425, 1338, 1269, 1157, 975, 908 cm⁻¹; MS (FAB) *m*/*z* (M+H)

### Beispiel 356: N-(4-Benzyl-4'-methyl-6'-trifluoromethyl-3,4,5,6-tetrahydro-2H-[1,2']bipyridinyl-3'-ylmethyl)-2-(3-fluoro-4-methansulfonylamino-phenyl)-propionamid

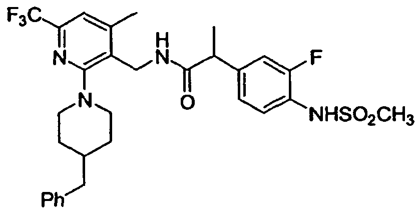

¹H NMR (300 MHz, CDCl₃) δ 7.49 (dd, 1 H, *J* = 8.3, 8.3 Hz), 7.27-7.35 (m, 2 H), 7.11-7.25 (m, 4 H), 6.89-7.10 (m, 2 H), 6.70 (bt, 1 H), 4.42-4.58 (m, 2 H), 3.45 (q, 1 H, *J* = 7.1 Hz), 3.02-3.21 (m, 2 H), 2.99 (s, 3 H), 2.68-2.83 (m, 2 H), 2.58 (d, 2 H, *J* = 6.6 Hz), 2.37 (s, 3 H), 1.64-1.80 (m, 3 H), 1.47 (d, 3 H, *J* = 7.1 Hz), 1.18-1.32 (m, 2 H)

### Beispiel 357: 2-(3-Fluoro-4-methansulfonylamino-phenyl)-N-(4-methylen-6'-trifluoromethyl-3,4,5,6-tetrahydro-2H-[1,2']bipyridinyl-3'-ylmethyl)-propionamid

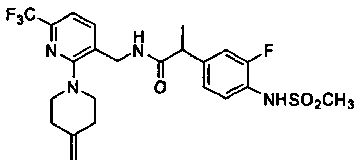

¹H NMR (CDCl₃) δ 7.53-7.48 (m, 2 H), 7.22 (d, 1 H, J = 7.7 Hz), 7.16-7.08 (m, 2 H), 6.52 (bs, NH), 6.19 (bt, NH), 4.76 (s, 2 H), 4.50 (d, 2 H, J = 5.7 Hz), 3.57 (q, 1 H, J = 7.0 Hz), 3.13 (m, 4 H), 3.03 (s, 3 H), 2.30 (m, 4 H), 1.54 (d, 3 H, J = 7.1 Hz); IR (pur) 3293, 2931, 1720, 1657, 1593, 1513, 1458, 1419, 1335, 1158 cm⁻¹; MS (FAB) m/z 515 (M+H)

### Beispiel 358: N-[2-(6-Aza-spiro[2.5]oct-6-yl)-6-trifluoromethyl-pyridin-3-ylmethyl]-2-(3-fluoro-4-methansulfonylamino-phenyl)-propionamid

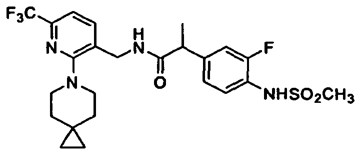

¹H NMR (CDCl₃) δ 7.55-7.49 (m, 2 H), 7.22 (d, 1 H, J = 7.7 Hz), 7.17-7.08 (m, 2 H), 6.52 (bs, NH), 6.35 (bt, NH), 4.50 (d, 2 H, J = 5.7 Hz), 3.56 (q, 1 H), 3.12 (m, 4 H), 3.03 (s, 3 H), 1.53 (d, 3 H, J = 7.1 Hz), 1.45 (m, 4 H), 0.35 (s, 4 H); IR (pur) 3292, 2926, 1656, 1593, 1513, 1420, 1335, 1158, 734 cm⁻¹; MS (FAB) m/z 529 (M+H)

### Beispiel 359: 2-(3-Fluoro-4-methansulfonylamino-phenyl)-N-[2-(3-methyl-but-2-enyloxy)-6-trifluoromethyl-pyridin-3-ylmethyl]-propionamid

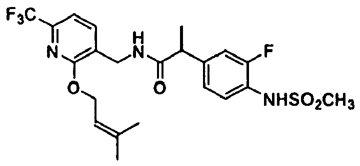

¹H NMR (CDCl₃) δ 7.58 (d, 1 H, J = 7.3 Hz), 7.51 (m, 1 H), 7.18 (d, 1 H, J = 7.5 Hz), 7.12-7.05 (m, 2 H), 6.07 (bt, NH), 5.38 (m, 1 H), 4.87 (m, 2 H), 4.37 (m, 2 H), 3.51 (q, 1 H, J = 7.1 Hz), 3.03 (s, 3 H), 1.78 (s, 6 H), 1.48 (d, 3 H, J = 7.1 Hz); IR (pur) 3289, 2935, 1656, 1603, 1513, 1420, 1333, 1262, 1158, 977 cm⁻¹; MS (FAB) m/z 503 (M+H)

### Beispiel 360: N-[2-(3-Cyclohexyl-propyl)-6-trifluoromethyl-pyridin-3-ylmethyl]-2-(3-fluoro-4-methansulfonylamino-phenyl)-propionamid

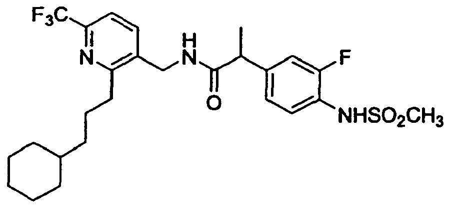

¹H NMR (300 MHz, CDCl₃) δ 7.50-7.57 (m, 2 H), 7.44 (d, 1 H, *J* = 7.9 Hz), 7.17 (dd, 1 H, *J* = 11.0, 2.0 Hz), 7.10 (d, 1 H, *J* = 8.3 Hz), 6.47 (bs, 1 H), 5.69 (bt , 1 H), 4.40-4.57 (m, 2 H), 3.57 (q, 1 H, *J* = 7.1 Hz), 3.05 (bs, 3 H), 2.75 (t, 2 H, *J* = 7.7 Hz), 1.60-1.74 (m, 8 H), 1.53 (d, 3 H, *J* = 7.1 Hz), 1.09-1.30 (m , 5 H), 0.79-0.91 (m , 2 H); IR (KBr) 3292, 2924, 2851, 1654, 1512, 1454, 1408, 1340, 1278, 1158, 972, 909, 733 cm⁻¹; MS (FAB) *m*/*z* 544 (M+H)

### Beispiel 361: N-[2-(3-Ethoxy-propoxy)-6-trifluoromethyl-pyridin-3-ylmethyl]-2-(3-fluoro-4-methansulfonylamino-phenyl)-propionamid

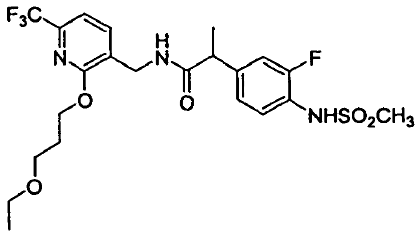

¹H NMR (300 MHz, CDCl₃) δ 7.61 (d, 1 H, *J* = 7.5 Hz) 7.50 (dd, 1 H, *J* = 8.3, 8.3 Hz), 7.20 (d, 1 H, *J* = 7.5 Hz), 7.01-7.12 (m, 2 H), 6.35 (bt, 1 H), 4.37-4.50 (m, 2 H), 4.35 (d, 2 H, *J* = 6.0 Hz), 3.47-3.60 (m, 5 H), 3.03 (s, 3 H), 1.90-2.01 (m, 2 H), 1.47 (d, 3 H, *J* = 7.0 Hz), 1.20 (t, 3 H, *J* = 7.1 Hz); IR (KBr) 3296, 2923, 1657, 1512, 1425, 1338, 1269, 1157, 972 cm⁻¹; MS (FAB) *m*/*z* (M+H)

### Beispiel 362: 2-(3-Fluoro-4-methansulfonylamino-phenyl)-N-[2-(2-phenoxy-ethoxy)-6-trifluoromethyl-pyridin-3-ylmethyl]-propionamid

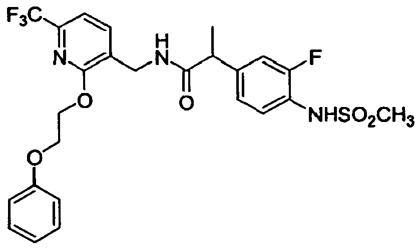

¹H NMR (300 MHz, CDCl₃) δ 7.66 (d, 1 H, *J* = 7.3 Hz), 7.41 (dd, 1 H, *J* = 8.4, 8.4 Hz), 7.22-7.35 (m, 3 H), 6.88-7.05 (m, 5 H), 6.42 (bs, 1 H), 6.21 (bt, 1 H), 4.63-4.82 (m, 2 H), 4.27-4.42 (m, 4 H), 3.34 (q, 1 H, *J* = 7.1 Hz), 2.99 (s, 3 H), 1.38 (d, 3 H, *J* = 7.0 Hz); IR (KBr) 3295, 2924, 1657, 1598, 1510, 1423, 1339, 1244, 1157, 967, 910, 756 cm⁻¹; MS (FAB) *m*/*z* (M+H)

### Beispiel 363: N-[2-(3,5-Dimethoxy-benzyloxy)-6-trifluoromethyl-pyridin-3-ylmethyl]-2-(3-fluoro-4-methansulfonylamino-phenyl)-propionamid

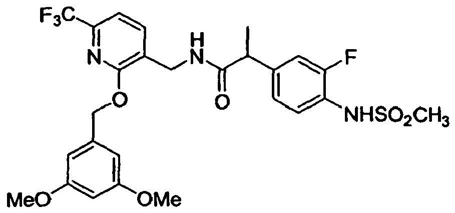

¹H NMR (300 MHz, CDCl₃) δ 7.63 (d, 1 H, *J* = 7.3 Hz) 7.45 (dd, 1 H, *J* = 8.3, 8.3 Hz), 7.24 (d, 1 H, *J* = 7.5 Hz), 7.04 (dd, 1 H, *J* = 11.2, 2.0 Hz), 6.98 (d, 1 H, *J* = 8.8 Hz), 6.59 (d, 2 H, *J* = 2.2 Hz), 6.45 (t, 1 H, , *J* = 2.4 Hz), 6.00 (bt, 1 H), 5.26-5.41 (m, 2 H), 4.30-4.48 (m, 2 H), 3.81 (s, 6 H), 3.43 (q, 1 H, *J* = 7.3 Hz), 3.01 (s, 3 H), 1.43 (d, 3 H, *J*= 7.1 Hz); IR (KBr) 1656, 1601, 1512, 1463, 1419, 1353, 1156, 1068, 976, 835 cm⁻¹; MS (FAB) *m*/*z* (M+H)

### Beispiel 364: 2-(3-Fluoro-4-methansulfonylamino-phenyl)-N-(4-hydroxymethyl-6'-trifluoro methyl-3,4,5,6-tetrahydro-2H-[1,2']bipyridinyl-3'-ylmethyl)-propionamid

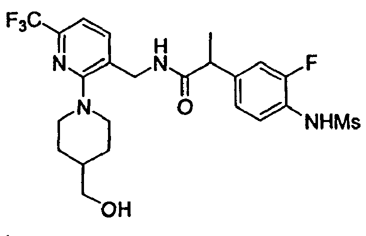

¹H NMR (300 MHz, CDCl₃) δ 7.54 (d, 1 H, *J* = 8.3 Hz), 7.51 (d, 1 H, *J* = 8.3 Hz), 7.23 (d, 1H, *J* = 7.7 Hz), 7.16-7.08 (m, 2 H), 6.24 (bs, 1 H), 4.48 (m, 2 H), 3.57-3.54 (m, 3 H), 3.28 (m, 2 H), 3.05 (s, 3 H), 2.85 (m, 2 H), 1.80 (m, 1 H), 1.57-1.51 (m, 5 H), 1.29 (m, 2 H); IR (KBr) 3294, 2925, 1655, 1593, 1513, 1419, 1334 cm⁻¹; MS (FAB) *m*/*z* 533 (M+H)

### Beispiel 365: N-(6'-tert-Butyl-4-phenyl-3,4,5,6-tetrahydro-2H-[1,2']bipyridiny1-3'-ylmethyl)-2-(3-fluoro-4-methansulfonylamino-phenyl)-propionamid

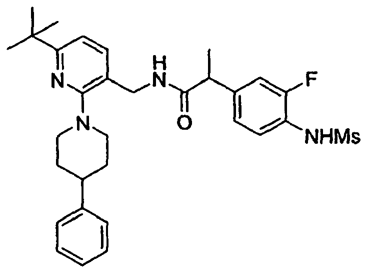

¹H NMR (300 MHz, CDCl₃) δ 7.47 (dd, 1 H, *J* = 8.3, 8.3 Hz), 7.37-7.14 (m, 7 H), 7.09 (d, 1 H, *J* = 8.6 Hz), 6.92 (d, 1 H, *J* = 7.7 Hz), 6.72 (bs, 1 H), 4.47 (m, 2 H), 3.55 (q, 1 H, *J* = 7.1 Hz), 3.40 (m, 2 H), 3.01-2.89 (m, 5 H), 2.68 (m, 1 H), 1.93-1.68 (m, 4 H), 1.52 (d, 3 H, *J* = 7.1 Hz), 1.32 (s, 9 H); IR (KBr) 3289, 2958, 1651, 1512, 1449, 1401, 1335 cm⁻¹; MS (FAB) *m*/*z* 567 (M+H)

### Beispiel 366: N-{6-tert-Butyl-2-[4-(4-fluoro-phenyl)-piperazin-1-yl]-pyridin-3-ylmethyl}-2-(3-fluoro-4-methansulfonylamino-phenyl)-propionamid

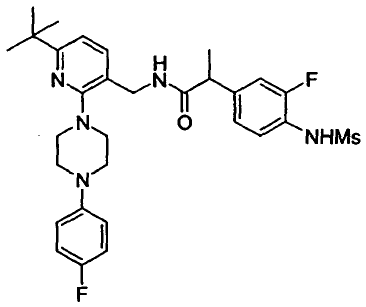

¹H NMR (300 MHz, CDCl₃) δ 7.47 (dd, 1 H, *J* = 8.2, 8.2 Hz), 7.34 (d, 1 H, *J* = 7.9 Hz), 7.17-6.87 (m, 7 H), 6.51 (bs, 1 H), 4.47 (m, 2 H), 3.53 (q, 1 H, *J* = 6.9 Hz), 3.20-3.10 (m, 8 H), 2.98 (s, 3 H), 1.51 (d, 3 H, *J* = 6.9 Hz), 1.30 (s, 9 H); IR (KBr) 3291, 2961, 1562, 1510, 1449, 1400, 1335 cm⁻¹; MS (FAB) *m*/*z* 586 (M+H)

### Beispiel 367: 2-(4-Methansulfonylamino-3-methyl-phenyl)-N-(2-pyrrolidin-1-yl-6-trifluoromethyl-pyridin-3-ylmethyl)-propionamid

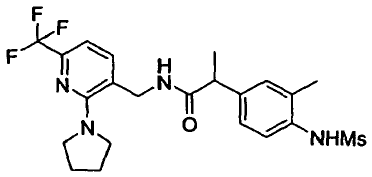

¹H NMR (300 MHz, CDCl₃) δ 7.74 (d, 1 H, J = 8.1 Hz), 7.39 (d, 1 H, J = 7.8 Hz), 7.35 (d, 1 H, J = 7.8 Hz), 7.32 (d, 1 H, J = 7.8 Hz), 7.12-7.14 (m, 2 H), 6.92 (d, 1 H, J = 7.5 Hz), 6.26 (s, 1 H), 5.68 (bs, 1 H), 4.45 (d, 2 H, J = 5.7 Hz), 3.53 (q, 1 H, J = 7.2 Hz), 3.41 (m, 4 H), 3.05 (s, 3 H), 2.32 (s, 3 H), 1.85 (m, 4 H), 1.50 (d, 3 H, J = 7.2 Hz); IR (KBr) 3292, 2926, 1651, 1599, 1537, 1458, 1330, 1153 cm⁻¹; MS (FAB) *m*/*z* 485 (M+H)

| | |
|---|---|
| 83 | (S)-2-(3-Fluor-4-methansulfonylamino-phenyl)-N-[2-(4-methyl-piperidin-1-yl)-4-trifluormethyl-benzyl]-propionamid |
| 84 | (R)-2-(3-Fluor-4-methansulfonylamino-phenyl)-N-[2-(4-methyl-piperidin-1-yl)-4-trifluormethyl-benzyl]-propionamid |
| 93 | (S)-2-(3-Fluor-4-methansulfonylamino-phenyl)-N-(2-hexyloxy-6-trifluormethyl-pyridin-3-ylmethyl)-propionamid |
| 94 | (R)-2-(3-Fluor-4-methansuffonylamino-phenyl)-N-(2-hexyloxy-6-trifluormethyl-pyridin-3-ylmethyl)propionamid |
| 96 | (S)2-(3-Fluor-4-methansulfonylamino-phenyl)-N-(2-isobutoxy-6-trifluormethyl-pyridin-3-ylmethyl)-propionamid |
| 97 | (R)-2-(3-Fluor-4-methansulfonylamino-phenyl)-N-(2-isobutoxy-6-trifluormethyl-pyridin-3-ylmethyl)propionamid |
| 98 | 2-(3-Fluor-4-methansulfonylamino-phenyl)-N-(2-isobutoxy-4-trifluormethyl-benzyl)-propionamid |
| 99 | (R)-2-(3-Fluor-4-methansulfonylamino-phenyl)-N-(2-isobutoxy-4-trifluormethyl-benzyl)-propionamid |
| 111 | 2-(3-Fluor-4-methansulfonylamino-phenyl)-N-(2-propoxy-6-trifluormethyl-pyridin-3-ylmethyl)-propionamid |
| 116 | N-(2-Butylamino-6-trifluormethyl-pyridin-3-ylmethyl)-2-(3-fluor-4-methansulfonylamino-phenyl)-propionamid |
| 120 | (S)-N-[2-(3-Chloro-fluor-phenyl)-6-trifluormethyl-pyridin-3-ylmethyl]-2-(3-fluor-4-methansulfonylamino-phenyl)-propionamid |
| 121 | (R)-N-[2-(3-Chlor-4-fluor-phenyl)-6-trifluormethyl-pyridin-3-ylmethyl]-2-(3-fluor-4methansulfonylamino-phenyl)-propionamid |
| 123 | 2-(3-Fluor-4-methansulfonylamino-phenyl)-N-[2-(3-methyl-butoxy)-6-trifluormethyl-pyridin-3-ylmethyl]-propionamid |
| 133 | N-(4-tert-Butyl-2-isobutoxy-benezyl)-2-(3-fluor-4-methansulfonylamino-phenyl)-propionamid |
| 134 | 2-(3-Fluor-4-methansulfonylamino-phenyl)-N-[2-(4-methyl-piperazin-1-yl)-6-trifluormethyl-pyridin-3-ymethyl]-propionamid |
| 140 | N-[2-Butoxy-6-(chlor-difluor-methyl)pyridin-3-ylmethyl]-2-(3-fluor-4-methansulfonylamino-phenyl)-propionamid |
| 144 | (S)-2-(3-Fluor-4-rnethansulfonylamino-phenyl)-N-(2-pentyl-6-tfdluormethyl-pyridin-3-ylmethyl)-propionamid |
| 145 | (R)-2-(3-Fluor-4-methansulfonylamino-phenyl)-N-(2-pentyl-6-trifluormethyl-pyridin-3-ylmethyl)-propionamid |
| 191 | 2-(3-Fluoro-4-(methylsulfonylamino)phenyl)-N-((2-styryl-6-(trifluoromethyl)pyridin-3-yl)methyl)propanamid |
| 192 | 2-(3-Fluor-4-methansulfonylamino-phenyl)-N-(2-phenethyl-6-trifluormethyl-pyridin-3-ylmethyl)-propionamid |
| 193 | N-{2-[4-(3-Chlor-pyridin-2-yl)-piperazin-1-yl]-6-trifluormethyl-pyridin-3-ylmethyl}-2-(3-fluor-4-methansulfonylamino-phenyl)-propionamid |
| 194 | N-{2-[4-(3-Chlor-pyridin-2-yl)-2-methyl-piperazin-1-yl]-6-tritluormethyl-pyridin-3-ylmethyl}-2-(3-fluor-4-methansulfonylamino-phenyl)-propionamid |
| 213 | 2-(3-Fluor-4-methansulfonylamino-phenyl)-N-{6-trifluormethyl-2-[4-(3-trifluormethyl-pyridin-2-yl)-piperazin-1-yl]-pyridin-3-ylmethyl}-propionamid |
| 214 | 2-(3-Fluor-4-methansulfonylamino-phenyl)-N-[2-(4-pyridin-2-yl-piperazin-1-yl)-6-trifluormethyl-pyridin-3-ylmethyl]-propionamid |
| 215 | 2-(3-Fluor-4-methansulfonylamino-phenyl)-N-(6-trifluormethyl-pyridin-3-ylmethyl)-propionamid |
| 239 | (R)-N-(2-Cyclohexyloxy-4-trifluormethyl-benzyl)-2-(3-fluor-4-methansulfonylamino-phenyl)-propionamid |
| 242 | N-[2-(4-Ethyl-benzyloxy)-6-trifluormethyl-pyridin-3-ylmethyl]-2-(3-fluor-4-methansulfonylamino-phenyl)-propionamid |
| 250 | N-[2-(3,4-Dichlor-phenyl)-6-trifluormethyl-pyridin-3-ylmethyl]-2-(3-fluor-4-methansulfonylamino-phenyl)-propionamid |
| 251 | N-[2-(3-tert-Butyl-1-oxa-2,8-diaza-spiro[4.5]dec-2-en-8-yl)-6-trifluormethyl-pyndin-3-ylmethyl]-2-(3-fluor-4-methansulfonylamino-phenyl)-propionamid |
| 252 | 2-(3-Fluor-4-methansulfonylamino-phenyl)-N-[2-(3-phenyl-1-oxa-2,8-diaza-spiro[4.5]dec-2-en-8-yl)-6-trifluormethyl-pyridin-3-ylmethyl]-propionamid |
| 253 | 2-(3-Fluor-4-(pentafluorsulfanylsulfonamid)phenyl)-N-p-tolylpropanamid |
| 254 | 2-(3-Fluor-4-methansulfonylamino-phenyl)-N-[2-(3-fluor-4-methoxy-phenyl)-6-trifluormethyl-pyridin-3-ylmethyl]-propionamid |
| 265 | N-[2-(2,3-Dihydro-benzo[1,4]dioxin-6-yl)-6-trifluormethyl-pyridin-3-ylmethyl]-2-(3-fluor-4-methansulfonylamino-phenyl)-propionamid |
| 266 | N-(2-Benzo[1,3]dioxol-5-yl-6-trifluormethyl-pyridin-3-ylmethyl)-2-(3-fluor-4-methansulfonylamino-phenyl)-propionamid |
| 272 | 2-(3-Fluor-4-methansulfonylamino-phenyl)-N-[2-(3-methansulfonylamino-phenyl)-6-trifluormethyl-pyridin-3-ylmethyl]-propionamid |
| 273 | 2-(3-Fluor-4-methansulfonylamino-phenyl)-N-[2-(2-methyl-propenyl)-6-trifluormethyl-pyridin-3-ylmethyl]-propionamid |
| 274 | N-[2-(3,3-Dimethyl-but-1-enyl)-6-trifluormethyl-pyridin-3-ylmethyl]-2-(3-fluor-4-methansulfonylamino-phenyl)-propionamid |
| 275 | 2-(3-Fluor-4-methansulfonylamino-phenyl)-N-[2-(1H-indo)-6-trifluormthyl-6-trifluormethyl-pyridin-3-ylmethyl]-propionamid |
| 276 | 2-(3-Fluor-4-methansulfonylamino-phenyl)-N-[2-(1H-indol-5-yl)-6-trifluormethyl-pyridin-3-ylmethyl]-propionamid |
| 277 | N-[2-(4-Chlor-3-fluor-phenyl)-6-trifluormethyl-pyridin-3-ylmethyl]-2-(3-fluor-4-methansulfonylamino-phenyl)propionamid |
| 278 | 2-(3-Fluor-4-methansulfonylamino-phenyl)-N-[2-(4-fluor-3-methyl-phenyl)-6-triftuormethyl-pyridin-3-ylmethyl]-propionamid |
| 294 | N-(2-Cyclohexylsulfanyl-6-trifluormethyl-pyridin-3-ylmethyl)-2-(3-fluor-4-methansulfonylamino-phenyl)-propionamid |
| 295 | 2-(3-Fluor-4-methansulfonylamino-phenyl)-N-(4-phenyl-6'-trifluormethyl-3,4,5,6-tetrahydro-2H-[1,2']bipyridinyl-3'-ylmethyl)-propionamid |
| 328 | N-(6-tert-Butyl-2-pentyloxy-pyridin-3-ylmethyl)-2-(3-fluor-4-methansulfonylamino-phenyl)-propionamid |
| 329 | N-[6-tert-Butyl-2-(3-methyl-butoxy)pyridin-3-ylmethyl]-2-(3-fluor-4-methansulfonylamino-phenyl)-propionamid |
| 330 | N-(4-Dimethylamino4-phenyl-6'-trifluormethyl-3,4,5,6-tetrahydro-2H-[1,2']bipyridinyl-3'-ylmethyl)-2-(3-fluor-4-methansulfonylamino-phenyl)-propionamid |
| 332 | 2-(3-Fluor-4-methansulfonylamino-phenyl)-N-[4-(4-fluor-phenyl)-6'-trifluormethyl-3,6-dihydro-2H-[1,2']bipyridinyl-3'-ylmethyl]-propionamid |
| 334 | N-(2-Cyclohex-1-enyl-6-trifluormethyl-pyridin-3-ylmethyl)-2-(3-fluor-4-methansufonylamino-phenyl)-propionamid |
| 335 | N-[2-(1-Ethyl-propoxy)-6-trifluormethyl-pyridin-3-ylmethyl]-2-(3-fluor-4-methansulfonylamino-phenyl)propionamid |
| 336 | 2-(3-Fluor-4-methansulfonylamino-phenyl)-N-[2-(1-propyl-butoxy)-6-trifluormethyl-pyridin-3-ylmethyl]-propionamid |
| 337 | 2-(3-Fluor-4-methansulfonylamino-phenyl)-N-[2-(1-isobutyl-3-methyl-butoxy)-6-trifluormethyl-pyridin-3-ylmethyl]-propionamid |
| 338 | N-[2-(4,4-Dimethyl-cyclohexyloxy)-6-trifluormethyl-pyridin-3-ylmethyl]-2-(3-fluor-4-methansulfonylamino-phenyl)-propionamid |
| [368] | N-((2-(1H-indol-4-yl)-6-(trifluoromethyl)pyridin-3-yl)methyl)-2-(3-fluor-4-(methylsulfonylamino)phenyl)propanamid, |
| [369] | N-((6-tert-Butyl-2-propoxypyridin-3-yl)methyl)-2-(3-fluor-4-(methylsulfonylamino)phenyl)propanamid, |
| [370] | N-((6-tert-Butyl-2-(3-methoxypropoxy)pyridin-3-yl)methyl)-2-(3-fluor-4-(methylsulfonylamino)phenyl)propanamid, |
| [371] | N-((6-tert-Butyl-2-(4-(dimethylamino)-4-phenylpiperidin-1-yl)pyridin-3-yl)methyl)-2-(3-fluor-4-(methylsulfonylamino)phenyl)propanamid, |
| [372] | N-((6-tert-Butyl-2-methoxypyridin-3-yl)methyl)-2-(3-fluor-4-(methylsulfonylamino)phenyl)propanamide, |
| [373] | N-((6-tert-Butyl-2-ethoxypyridin-3-yl)methyl)-2-(3-fluor-4-(methylsulfonylamino)phenyl)propanamid, |
| [374] | N-((6-tert-Butyl-2-isopropoxypyridin-3-yl)methyl)-2-(3-fluor-4-(methylsulfonylamino)phenyl)propanamid, |
| [375] | 2-(3-Fluor-4-(methylsulfonylamino)phenyl)-N-((2-(pentyloxy)-6-(trifluoromethyl)pyridin-3-yl)methyl)propanamid, |
| [376] | 2-(3-Fluor-4-(methylsulfonylamino)phenyl)-N-((2-(hexyloxy)-6-(trifluoromethyl)pyridin-3-yl)methyl)propanamid, |
| [377] | N-((2-(3,5-Dimethylcyclohexyloxy)-6-(trifluoromethyl)pyridin-3-yl)methyl)-2-(3-fluor-4-(methylsulfonylamino)phenyl)propanamid, |
| [378] | N-((6-tert-Butyl-2-(2-ethoxyethoxy)pyridin-3-yl)methyl)-2-(fluor-4-(methylsulfonylamino)phenyl)propanamid, |

### Pharmakologische Daten

Die Affinität der erfindungsgemäßen Verbindungen für den Vanilloid-Rezeptor 1 (VR1/TRPV1-Rezeptor) wurde wie vorstehend beschrieben bestimmt (Pharmakologische Methoden I bzw. II).

Die erfindungsgemäßen Verbindungen der vorstehend angegebenen Formeln A undI weisen eine ausgezeichnet Affinität zum VR1/TRPV1-Rezeptor auf (Tabelle 2.).

**Tabelle 1.**

| **Verbindung gemäß Beispiel** | **Kᵢ (Ratte) Capsaicin [nM]** | **Kᵢ (mensch) Capsaicin [nM]** | **IC₅₀ (Mensch) [nM] nach pH-Stimulus** |
|---|---|---|---|
| 1 | 684 | 387 | |
| 2 | 3.5 | 0.4 | 218 |
| 3 | 2.6 | 1.7 | 135 |
| 6 | 95 | 169 | 2613 |
| 10 | 1.1 | 0.3 | 64 |
| 11 | 1.1 | 0.3 | 46.7 |
| 12 | 4.3 | 1.3 | 169 |
| 13 | 3.8 | 0.6 | 211 |
| 18 | 47 | 68.9 | 2711 |
| 19 | 1.0 | 0.3 | 31 |
| 20 | 5.4 | 2.1 | 93 |
| 21 | 1.3 | 0.9 | 29 |
| 22 | 4.9 | 6.4 | 62 |
| 31 | 0.5 | 0.6 | 50 |
| 33 | 4.8 | 7.2 | 1376 |
| 34 | 2.5 | 2.3 | 121 |
| 64 | 6.3 | 2.3 | |
| 65 | 36.5 | 4.2 | |
| 66 | 3.7 | 2.9 | |
| 67 | 5.1 | 3.2 | |
| 69 | 2.4 | 1.7 | |
| 70 | 9 | 15.5 | 1490 |
| 71 | 0.8 | 1.3 | 22.4 |
| 72 | 124 | 32.6 | 1660 |
| 73 | 6.6 | 8.8 | 634 |
| 75 | 12.9 | 4.0 | 1212 |
| 76 | 1.5 | 1.3 | 43.4 |

Die efindungsgemäßen Verbindungen der allgemeinen Formel I eignen sich sehr gut zur Hemmung der durch Capsaicin induzierten Hypothermie (Tabelle 3.).

**Tabelle 3.**

| **Verbindung gemäß Beispiel** | **Hemmung der Capsaicin induzierten Hypothermie¹⁾** |
|---|---|
| 2 | 73 |
| 3 | 55 |
| 10 | 49 |
| 12 | 36 |
| 13 | 7 |
| 19 | 71 |
| 20 | 31 |
| 21 | 32 |
| 31 | 88 |
| 34 | 84 |
| 71 | 79 |
| 76 | 48 |

| | |
|---|---|
| ¹⁾ im Vergleich zur Vehikelkontrolle im Wirkmaximum 15 min nach Applikation von Capsaicin; n = 5 (Anzahl der Bestimmungen) | |

Die pharmakologischen Daten für die Beispielverbindungen 14 bis 356 sind in der nachstehenden Tabelle 4. angegeben.

| **Beispiel** | **Kⱼ(Ratte) Cap. [nM]** | **Kⱼ (Mensch) Cap. [nM]** | **IC₅₀ (Mensch) [nM] nach pH-Stimulus** | **Hemmung Hypothermie (n = 5)** | **Hemmung Formalin-Test (n =10)** | **Hemmung Bennet Model (n = 10)** |
|---|---|---|---|---|---|---|
| 14 | 67.9 | 19.1 | 2386.0 | | | |
| 15 | 12.0 | 3.5 | 148.0 | | | |
| 16 | 0.8 | 0.2 | 14.7 | 0.3 po 12 % | | |
| 32 | 2.6 | 1.7 | 135.4 | 0.3 po 55% | | |
| 34 | | | | | 0.1 po 6 % | |
| | | | | | 0.3 po 51 % | |
| 37 | 1.8 | 0.3 | 26.2 | | | |
| 40 | 0.4 | 1.0 | 48 | | | |
| 42 | 0.5 | 0.6 | 26 | | | |
| 46 | | 59.4 | 40 % @ 5 µM | | | |
| | | | 5 % @ 1 µM | | | |
| 61 | ne @ 1 µM | ne @ 1 µM | 47 % @ 10 µM | | | |
| 62 | ne @ 1 µM | ne @ 1 µM | ne @ 1 µM | | | |
| 63 | 23.9 | 28.6 | 70 % @ 1 µM | | | |
| 71 | | | | | 0.3 po 0 % | |
| | | | | | 1 po 3 % | |
| 74 | 70 % 10 µM | 71 % @ 10 µM | 35 % @ 10 µM | | | |
| 76 | | | | | 0.3 po 12 % | |
| 77 | 57 % @ 10µM | 21 % @ 10 µM | ne @ 1 µM | | | |
| 78 | 19 % @ 1 µM | 15 % @ 1 µM | 36 % @ 10 µM | | | |
| 79 | 0.8 | 0.7 | 25.6 | | | |
| 80 | 0.6 | 0.3 | 5.4 | 0.3 po 66 % | 0.3 po 58% | |
| 81 | 2.4 | 0.8 | 25.1 | 0.3 po 21 % | | |
| 82 | 0.7 | 0.9 | 16.2 | 0.3 po 53 % | 0.3 po 39 % | |
| | | | | | 1 po 43 % | |
| 85 | 67.9 | 19.1 | 2386 | | | |
| 87 | 143 | 36.1 | 2574 | 0.3 po 0% | | |
| 88 | 5 | 0.3 | 57.1 | 0.3 po 38 % | 0.3 po 4% | |
| | | | | | 1 po 16% | |
| 89 | 1.2 | 0.4 | 130.8 | 0.3 po 10 % | | |
| 90 | 47 | 20.4 | 260 | 0.3 po 81 % | 0.3 po 46 % | |
| | | | | | 1 po 37 % | |
| 91 | 0.5 | 0.5 | 29.9 | | | 0.1 po 30 % |
| | | | | | | 1 po 46 % |
| | | | | | | 10 po 43% |
| 93 | 0.2 | 0.4 | 12.3 | 0.3 po 4% | | |
| 94 | | | | 0.3 po % | | |
| 95 | 0.8 | 0.5 | 52.6 | | | |
| 96 | | 0.5 | 30 | | | |
| 97 | | 44.5 | 2155 | | | |
| 98 | | 1.5 | 41 | | | |
| 99 | | 5.8 | 419 | 0.3 po 1 % | | |
| 100 | 1.2 | 1.1 | 115.2 | 0.3 po 26 % | | |
| 101 | 1 | 0.3 | 40.5 | | | |
| 102 | 7.4 | 4.2 | 18% @ 10 µM | | | |
| 103 | 4.9 | 2.8 | 16 @ 10 µM | 0.3 po 39 % | 0.3 po 36 % | |
| | | | | | 1 po 50 % | |
| 104 | 0.2 | 0.2 | 40 | | | 0.0001 po 20 % |
| | | | | | | 0.001 po 45 % |
| | | | | | | 0.01 po 49 % |
| | | | | | | 0.1 po 35 % |
| 106 | 0.5 | 0.5 | 18.6 | 0.1 po 24 % | | |
| | | | | 0.3 po 21 % | | |
| | | | | 1 po 22% | | |
| | | | | 10 po 84% | | |
| 107 | 1 | 0.8 | 43.7 | 0.3 po 21 % | | |
| 108 | 0.6 | 1.4 | 114.1 | 0.3 po 38 % | 0.3 po 15% | |
| | | | | | 1 po 24 % | |
| 109 | 0.6 | 0.7 | 30.7 | | | |
| 110 | 63.1 | 59.6 | 1395 | 0.3 pro 13 % | | |
| 111 | 3.5 | 0.9 | 90.1 | | | |
| 112 | 31.8 | 39.2 | 64 % @ 10 µM | | | |
| | | | 26 % @ 1 µM | | | |
| 112 | 236.4 | 15 % @ 1 µM | 67 % @ 25 µM | | | |
| | | | 39% @ 10 µM | | | |
| 114 | 28.1 | 68.7 | 2357 | | | |
| 115 | 10.9 | 15.8 | 203 | | | |
| 116 | 6.6 | 9.1 | 293 | 0.3 po 21 % | 0.1 po 39 % | |
| | | | | | 0.3 po 57 % | |
| 117 | 0.5 | 0.5 | 23.5 | 0.3 po 70 % | 0.3 po 5 % | |
| | | | | | 1 po 41 % | |
| 118 | 0.2 | 0.2 | 7.6 | | | |
| 120 | 0.4 | 0.7 | 51 % @ 1 µM | | | |
| | | | 41 % @ 0.1 µM | | | |
| 121 | 5.8 | 13.2 | 56 % @ 10 µM | 0.3 po 12 % | | |
| | | | 32 % @ 1 µM | | | |
| 122 | 0.6 | 0.7 | 40.4 | 0.3 po 33 % | | |
| 123 | 0.6 | 0.8 | 15.1 | 0.3 po 47 % | | |
| 124 | 0.4 | 0.3 | 5.1 | 0.3 po 39 % | | |
| 125 | 1 | 0.9 | 23,8 | 0.1 po 5 % | 0.3 po 24 % | 0.01 po 17 % |
| | | | | 0.3 po 37 % | 1 po 19 % | 0.1 po 30 % |
| | | | | 1 po 32 % | | 1 po 46 % |
| | | | | 3 po 65 % | | 3 po 40 % |
| | | | | | | 10 po 50 % |
| 126 | 1.2 | 0.9 | 101 | 0.3 po 0 % | 0.1 po 20 % | |
| | | | | | 0.3 po 69 % | |
| 127 | 0.6 | 0.5 | 15.4 | 0.3 po 6 % | 0.3 po 22 % | |
| | | | | | 1 po 25 % | |
| 128 | 1 | 0.7 | 27.8 | | | |
| 129 | 1.6 | 1.8 | 77.2 | 0.3 po 10 % | 0.3 po 5 % | |
| | | | | | 1 po 3 % | |
| 130 | 4.5 | 2.1 | 34.3 | 0.3 po 58 % | 0.3 po 41 % | |
| | | | | | 1 po 55 % | |
| 131 | 0.4 | 0.3 | 5.9 | 0.3 po 26 % | | |
| 132 | 0.7 | 0.5 | 14.7 | 0.3 po 0 % | | |
| 133 | 0.7 | 2.1 | 62 % @ 1 µM | | | |
| | | | 38 % @. 0.1 µM | | | |
| 134 | 335 | 146 | 69 % @ 25 µM | | | |
| | | | 15 % @ 1 µM | | | |
| 135 | 48.9 | 39.3 | 1160 | | | |
| 136 | 8.3 | 14 | 140 | 0.3 po 43 % | | 0.01 po 13 % |
| | | | | | | 0.1 po 33 % |
| | | | | | | 1 po 47 % |
| | | | | | | 10 po 63 % |
| 137 | 1.9 | 0.7 | 12,5 | 0.3 po 9 % | | |
| 138 | 1.2 | 0.5 | 19.8 | 0.3 po 0 % | | |
| 139 | 0.9 | 0.3 | 12.1 | 0.3 po 43 % | | |
| 140 | 0.4 | 0.7 | 9.2 | | 0.1 po 56 % | |
| | | | | | 0.3 po 73 % | |
| 142 | 1.2 | 1 | 8.9 | | | |
| 144 | 1 | 1 | 146 | | | |
| 145 | | 78 | 49 % @ 10 µM | | | |
| | | | 39 % @ 5 µM | | | |
| | | | 2% @ 1 µM | | | |
| 147 | 1 | 0.7 | 10.4 | 0.3 po 3 % | | |
| 148 | 1.1 | 1.3 | 63.6 | 0.3 po 45 % | 0.3 po 44 % | |
| | | | | | 1 po 46 % | |
| 149 | 0.7 | 0.5 | 9.7 | | | |
| 150 | 22.4 | 63.3 | 1320 | | 0.3 po 17 % | |
| 151 | 1.4 | 1 | 92.3 | | | |
| 152 | 2.2 | 3.8 | | 63 % @ 10 µM | 0.3 po 33 % | |
| | | | | 45 % @ 1 µM | 1 po 24 % | |
| 153 | 0.7 | 2.2 | 92.4 | | | |
| 154 | 1.7 | 8.1 | 534 | 0.3 po 12 % | | |
| 155 | 0.8 | 1.8 | 145 | | | |
| 156 | 1.7 | 1.4 | 69.8 | | | |
| 157 | 1.3 | 2.9 | 43.1 | | | |
| 158 | 12.6 | 4 | 139 | | | |
| 159 | 21.4 | 2.4 | 280 | 0.3 po 28 % | 0.3 po 10 % | |
| | | | | | 1 po 14 % | |
| 160 | 3 | 3.5 | 28.2 | 0.3 po 28 % | 0.3 po 10 % | |
| | | | | | 1 po 14 % | |
| 161 | 0.7 | 0.4 | 11.6 | | | |
| 162 | 1.6 | 3 | 58 % @ 1 µM | | | |
| | | | 31 % @ 0.1 µM | | | |
| 163 | 8.5 | 12.7 | 277 | | | |
| 164 | 85% @ 10µM; 1.5% @ µM | 84% @10 µM; 20%@ 1µM | 28% @ 25 µM; 30%@10µM | | | |
| 165 | 82%@ 25µM; 4%@ 10µM | 76%@ 25µM; 0.5%@ 10µM | 40%@25µM; 28%@10µM | 0.3 po 41 % | 0.3 po 29 % | |
| | | | | | 1 po 15 % | |
| 166 | 1.1 | 0.5 | 18.5 | 0.3 po 28 % | 0.3 po 48 % | |
| | | | | | 1 po 72 % | |
| 167 | 2.8 | 0.8 | 21.3 | 0.3 po 19 % | 0.1 po 62 % | |
| | | | | | 0.3 po 56 % | |
| 168 | 1.4 | 0.9 | 17.6 | 0,3 po 21 % | 0.3 po 1 % | |
| 169 | 0.6 | 0.9 | 40.5 | | | |
| 170 | 5.1 | 7.7 | 242 | | | |
| 171 | 2.4 | 4.8 | 125 | 0.3 po 32 % | 0.3 po 45 % | |
| | | | | | 1 po 46 % | |
| 172 | 0.4 | 1.5 | 53 | | | |
| 173 | 78.4 | 65.7 | 414 | 0.3 po 36 % | 0.3 po 0 % | |
| 174 | 1.4 | 1.6 | 64.5 | 0.3 po 39 % | 0.3 po 5 % | |
| | | | | | 1po 49 % | |
| 175 | 1.2 | 2.5 | 25 | | | |
| 176 | 60%@10µM8% | 63%@ 10µM | 43% @ 10 µM. 13%@ 1µM | | | |
| | @1µM | 7.2 % @ 1 µM | | | | |
| 177 | 34%@ 1 µM | 51 | 1290 | 0.3 po 14 % | 0.3 po 31 % | |
| | 9%@ 0.1µM | | | | 1 po 24 % | |
| 178 | 1.9 | 0.5 | 27.6 | 0.3 pro 42 % | 0.3 po 16 % | |
| | | | | | 1 po 28 % | |
| 179 | 1 | 1.1 | 23.1 | 0.3 po 57 % | 0.3 po 5 % | |
| | | | | | 1 po 22 % | |
| 180 | 0.3 | 0.8 | 22.8 | | | |
| 181 | 5.6 | 14.6 | 1074 | | | |
| 182 | 7.3 | 4.2 | 637 | 0.3 po 45 % | 0.3 po 1 % | |
| | | | | | 1 po 8% | |
| 183 | 0.7 | 0.6 | 10.5 | | | |
| 184 | 77 | 59 | ne @ 1 µM | | | |
| 185 | 49.1 | 425 1 | ne @ 1µM | | | |
| 186 | 0.8 | 0.6 | 37.6 | | | |
| 187 | 0.5 | 1 | 39.6 | | | |
| 188 | 5 | 5 | 192 | 0.3 po 0 % | | |
| 189 | 3 | 4.7 | 166 | | | |
| 190 | 0.4 | 0.6 | 31 | | | |
| 191 | 2 | 2.2 | 85 | | | |
| 192 | 2.4 | 1.4 | 47 | | | |
| 193 | 3.2 | 2.1 | 69% @ 1 µM | | | |
| | | | 58% @ 0.1 µM 8%@ 0.01µM | | | |
| 194 | 3.8 | 4.1 | 21 % @ 1 µM | 0.3 po 14 % | | |
| | | | 0 % 0.1 µM | | | |
| 195 | 0.4 | 1.1 | 61 | | | |
| 196 | 0.4 | 0.6 | 41% @ 1µM | | | |
| | | | 48%@ 0.1µM6%@.0.01µM | | | |
| 197 | 0.4 | 1.5 | 97.3 | | | |
| 198 | 0.9 | 6.4 | 31%@1µM 11% 0.1µM | | | |
| 199 | 1.6 | 1.4 | 31 | | | |
| 200 | 3.4 | 4.2 | 81 | | | |
| 201 | 1.2 | 2.4 | 34 | | | |
| 202 | 24.8 | 29.5 | 30%@ µM 16%0.1µM | | | |
| 203 | 3.3 | 3 | 24% @ 1µM 0%@ 0.1 µM | | | |
| 204 | 1.6 | 4 | 148.1 | | | |
| 205 | 11.4 | 0.9 | 57.4 | | | |
| 206 | 0.7 | 3.2 | 66.1 | | | |
| 207 | 0.8 | 0.8 | 38.6 | | | |
| 208 | 0.5 | 0.6 | 38.6 | | | |
| 209 | 0.2 | 0.9 | 34.9 | 3 po 46 % | | |
| 210 | 0.2 | 0.3 | 8 | | | |
| 211 | 30.3 | 142 | 3441 | | | |
| 212 | 3.6 | 4.2 | 33%@ 1µM 11 %@0.1 µM | | | |
| 213 | 2.0 | 2.8 | 49%@ 10µM 37%@ 1µM | | | |
| 214 | 1.5 | 1.4 | 108.2 | | | |
| 215 | 48% @10µM | 37% @ 10µM | 15%@25µM | | | |
| | | 4%@1 µM | | | | |
| 216 | 0.5 | 2.0 | 64.7 | 0.3 po 4 % | | |
| 217 | 1.1 | 2.8 | 36.5 | 0.1 po 40 % | 0.3 po 9 % | |
| | | | | 0.3 po 97 % | 1 po 37 % | |
| 218 | 0.4 | 1.3 | 13.8 | | | |
| 219 | 2.4 | 3.3 | 38.3 | | | |
| 220 | 2.4 | 3.4 | 39.2 | | | |
| 221 | 1.9 | 2.5 | 44.5 | 0.3 po 18 % | | |
| 222 | 2.4 | 3.4 | 39.2 | | | |
| 223 | 53%@1µM | 38%@1µM | ne @ 1µM | | | |
| | | 4%@0.1µM | | | | |
| 225 | 0.5 | 0.7 | 27.3 | 0.3 po 20 % | | |
| 226 | 1.5 | 2 | 45.2 | | | |
| 227 | 348 | 91 | 51% @ 10µM 7%1µm | | | |
| 228 | 2.5 | 1.3 | 612 | | | |
| 229 | 0.7 | 0.7 | 17.7 | | | |
| 230 | 0.6 | 0.4 | 20.1 | | | |
| 231 | 0.8 | 1.4 | 37.6 | | | |
| 233 | 1.4 | 2.5 | 63%@ 10 µM 50% @1µ | | | |
| 234 | 1.0 | 1.5 | 85.3 | 0.1 po 19 % | 0.03 po 5 % | 0.001 po 26 % |
| | | | | 0.3 po 31 % | 0.1 po 28 % | 0.01 po 48 % |
| | | | | | 0.3 po 41 % | 0.1 po 53 % |
| | | | | | 1 po 44 % | 1 po 65 % |
| 235 | 0.6 | 0.9 | 55 | | | |
| 236 | 1.4 | 2.7 | 69%@ 10µM 76%@1µM | | | |
| | | | 2%@0.1µM | | | |
| 237 | 2.0 | 4.4 | 70% @ 1 µM | | | |
| 238 | 0.6 | 0.7 | 55%@1µM; 39%@0.1µM | | | |
| | | | 3%@ 0.01µM | | | |
| 240 | 5.3 | 1.2 | 43%@ 1µM 12%@0.1µM | | | |
| 241 | 0.5 | 1.9 | 79%@1µM 34%@0.1µM | | | |
| 242 | 1.2 | 3.7 | 165 | | | |
| 243 | 5.1 | 16 | 337 | | | |
| 244 | 1.1 | 2.1 | 182 | | | |
| 245 | 1.7 | 2.7 | 74%@1µM 21% 0.1µM | | | |
| 246 | 0.5 | 0.7 | 87 | | | |
| 247 | 0.5 | 1.8 | 69%@1µM 36% 0.1µM | | | |
| 248 | 0.9 | 3.9 | 74%@25µM 66%@10µM | | | |
| | | | 63%@1µM | | | |
| 249 | 0.2 | 1.6 | 70%@ 1µM 31%@.0.1µM | | | |
| 250 | 0.9 | 0.9 | 68%@10µM51%@1µM | | | |
| | | | 24% @ 0.1 µM | | | |
| 251 | 8.7 | 3.8 | 281 | | | |
| 252 | 17.9 | 6.1 | 289 | | | |
| 254 | 1.3 | 4.2 | 63%@1µM 19%@0.1µM | | | |
| 255 | 1.3 | 0.9 | 101 | | | |
| 256 | 0.3 | 0.6 (1.5) | 59%@1µM | | | |
| | | | 43% 01 µM 15%@0.01 µM | | | |
| 257 | 0.3 | 0.6 | 67%@ µM | | | |
| | | | 63%0.1µM11%µM0.01µM | | | |
| 258 | 1.3 | 2.5 | 132 | | | |
| 259 | 22.8 | 15.4 | 47%@10µM 51%@1µM | | | |
| | | | 0%@ 0.1µM | | | |
| 260 | 25.3 | 21.6 | 66% 10µM 51%@1µM | | | |
| | | | 8.5%@ 0.1 µM | | | |
| 261 | | | ne @ 1µM | | | |
| 262 | 1.7 | 1.2 | 58%@.1µM 6%@,0.1 µM | | | |
| 263 | 0.7 | 0.6 | 63%@1µM | | | |
| | | | 52%@0.1µM4%@0.01µM | | | |
| 264 | 0.9 | 0.6 | 15.9 | | | |
| 265 | 2.5 | 5 | 33%@1µM17%@0.1µM | | | |
| 266 | | 3.4 | *ne* @ 1 µM | | | |
| 267 | 1.7 | 1.6 | 24 | | | |
| 268 | 0.8 | 0.9 | 17 | | | |
| 269 | ne @ 1µM | ne@ 1µM | ne @ 1µM | | | |
| 270 | 0.5 | 0.7 | 19.7 | | | |
| 271 | 1.1 | 1.0 | 44.4 | | | |
| 273 | | 11.5 | 1605 | | | |
| 274 | | 0.7 | 63 | | | |
| 275 | | 20.3 | 1189 | | | |
| 276 | | 78%@ 5µM | 43%@5µM 11 %@1 µM 11 %@ | | | |
| | | 35%@ 1 µM | 0,1 µM | | | |
| | | 14%@ 0.1µM | | | | |
| 278 | | 0.4 | 76 | | | |
| 279 | 1.7 | 1.1 | 161 | | | |
| 282 | 1.2 | 0.8 | 62% @ 10 µM 57%@1µM | | | |
| | | | 55%@0.1 µM12%@0.01 µM | | | |
| 283 | 4.5 | 2.9 | 51%@10µM 51%@µM | | | |
| | | | 43%@0.1µM11%@0.01µM | | | |
| 284 | 48 | 54.9 | ne @ 1µM | | | |
| 285 | 4.5 | 3.5 | 377 | | | |
| 287 | 3.5 | 4.4 | 189 | | | |
| 288 | 41 | 42 | ne @ 1 µM | | | |
| 289 | 3.3 | 2.5 | ne @ 1 µM | | | |
| 290 | | 4.4 | 480 | 0.1 po 24 % | | |
| | | | | 0.3 po 78 % | | |
| | | | | 1 po 68 % | | |
| 291 | | 0.6 | 43.4 | | | |
| 292 | | 32.7 | 63%@ 5µM; 6% @ 1µM | | | |
| 293 | | 1.4 | 129 | | | |
| 294 | | 1.9 | 304 | | | |
| 295 | | 0.6 | 25 | | | |
| 296 | | 0.9 | 154 | | | |
| 297 | | 2.5 | 179 | | | |
| 298 | | 1.6 | 183 | | | |
| 299 | | 1 | 43 | | | |
| 300 | | 2.1 | 59.5 | | | |
| 301 | | 0.9 | 102 | | | |
| 302 | | 100.1 | 1182 | | | |
| 303 | | 1.8 | 76.2 | | | |
| 304 | | 0.71 | 35.4 | | | |
| 307 | | 5.0 | 456 | | | |
| 308 | | 3.2 | 414.5 | | | |
| 309 | | 8.7 | 20% bei 5µM | | | |
| 310 | | 43.7 | 27% bei 5µM | | | |
| 311 | | 1.43 | 1092.0 | | | |
| 312 | | 68 % @ 5 µM | ne @ 1µM | | | |
| | | 7 % @ 1 µM | | | | |
| 313 | | 2.7 | ne @ 1µM | | | |
| 314 | | 46% @ 5 µM | 31%@10µM14%@5µM | | | |
| | | 12%@1µM | | | | |
| 315 | | 1 | 295 | | | |
| 316 | | 72.1 | ne @ 1µM | | | |
| 317 | | 1.7 | 43 | | | |
| 318 | | 17.5 | 32%@10µM 4%@5µM | | | |
| 319 | | 13.8 | 54%@10µM38%@5µM | | | |
| | | | 4%@1µM | | | |
| 320 | | 1.1 | 36 | | | |
| 321 | | 0.8 | 38 | | | |
| 322 | | 1 | 54% @ 1 µM 25% @ 0.1 µM | | | |
| 323 | | 1.9 | 41% @ 1µM15%@0.1µM | | | |
| 324 | | 0.6 | 33 | | | |
| 325 | | 8.6 | 716 | | | |
| 326 | | 25.6 | 66% @ 10µM | | | |
| | | | 32% @ 5µM8%@1µM | | | |
| 327 | | 3.4 | 342 | | | |
| 328 | | 0.8 | 128 | | | |
| 329 | | 0.8 | 179 | 1 po 7% | 1 po 54 % | |
| 330 | | 31.3 | 806 | 1 po 7 % 1 iv 7 % | 1 po 54 % | |
| 331 | | 0.7 | 31 % @ 10µM | | | |
| | | | 22% @ 5µM 26% @1µM18% | | | |
| | | | @ 0.1µM | | | |
| 332 | | 2.4 | 159 | | | |
| 334 | | 0.7 | 99 | | | |
| 335 | | 1.1 | ne @ 1µM | | | |
| 336 | | 0.7 | 50%@ 1 µM 35%@ 0.1 µM | | | |
| | | | 28%@ 0.05µM | | | |
| 337 | | 0.7 | 30% @ 10µM 6% @ 5 µM | | | |
| 338 | | 0.9 | 140 | | | |
| 339 | | 0.3 | 14 | | | |
| 340 | | 1.2 | 279 | | | |
| 341 | | 1.3 | 293 | | | |
| 342 | | 19.3 | 1139 | | | |
| 343 | | 55 % @ 5 µM | nu @ 1µM | | | |
| | | 8%@ 1 µM | | | | |
| 344 | | 34%@ 5 µM | ne @ 1 µM | | | |
| | | 3 % @ 1µM | | | | |
| 345 | | 2.8 | 45% @ 5µM 38% @ 1 µM 3% | | | |
| | | | @ 0.1µM | | | |
| 346 | | 0.7 | 66 | | | |
| 347 | | 1.6 | | | | |
| 349 | | 44 | ne @ 1µM | | | |
| 350 | | 5.8 | 52%@ 1µM 4%@0.1µM | | | |
| 351 | | 44 | ne @ 1µM | | | |
| 352 | | 0.5 | 61 % @ 10µM 34% @ 5µM | | | |
| | | | 27% @ 1µM | | | |
| 353 | | 0.6 | 25% @ 1µM 28% @ 0.1 µM | | | |
| | | | 18% @ 0.05µM | | | |
| 354 | | 0.8 | 85 | | | |
| 355 | | 15.3 | 2205 | | | |
| 356 | | 9.2 | ne @ 1µM | | | |
| 361 | | 9.7 | 462 | | | |
| 362 | | 1.5 | 161 | | | |
| 363 | | 0.7 | 76 | | | |
| 369 | | 1.7 | 182 | | | |
| 370 | | 5.6 | 250 | | | |
| 371 | | 3.7 | 368 | | | |
| 372 | | 14.3 | 52 % @ 1 µM | | | |
| 373 | | 14.8 | 492 | | | |
| 374 | | 2.3 | 314 | | | |
| 375 | | 0.7 | 30 | | | |
| 376 | | 0.8 | 20 | | | |
| 377 | | 3 | | | | |
| 378 | | 23.9 | 325 | | | |

Die jeweilige Dosis ist in mg/kg Körpergewicht angegeben, dabei bedeutet po, dass die Menge jeweils peroral verabreicht wurde, und iv, dass die Menge jeweils intravenös verabreicht wurde.

ne bedeutet jeweils "no effect", d. h. es wurde keine Reaktion beobachtet.

Der Wert nach dem Zeichen "@" gibt die Konzentration an, bei der die Hemmung (in Prozent) jeweils bestimmt wurde.

## Patentansprüche

1. Substituierte Verbindungen der allgemeinen Formel A, 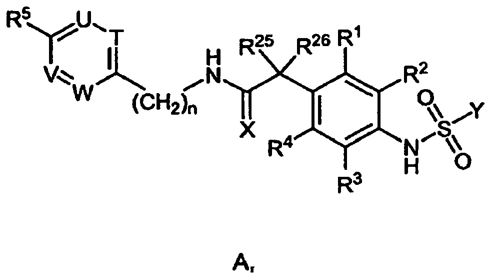 worin
X für O
steht;
Y
für einen linearen oder verzweigten, gesättigten oder ungesättigten, unsubstituierten oder wenigstens einfach substituierten aliphatischen C₁₋₁₀ Rest steht;
n für 0, 1, 2, 3 oder 4 steht;
R¹, R², R³ und R⁴, unabhängig voneinander, jeweils für H; F; Cl; Br; I; -SF₅; -NO₂; -CF₃; -CN; -NH₂; -OH; -SH; -C(=O)-NH₂-; -S(=O)₂-NH₂; -C(=O)-NH-OH; -C(=O)-OH; -C(=O)-H; -S(=O)₂-OH; -NHR¹¹; -NR¹²R¹³; -OR¹⁴;-SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³; -S(=O)-R²⁴; -S(=O)₂-R²⁴ oder für einen linearen oder verzweigten, gesättigten oder ungesättigten, unsubstituierten oder wenigstens einfach substituierten aliphatischen C₁₋₁₀ Rest stehen;
R⁵ für H; F; Cl; Br, I; -SF₅; -NO₂; -CF₃; -CF₂Cl; -CN; -NH₂; -OH; -SH; -
C(=O)-NH₂; -S(=O)₂-NH₂; -C(=O)-NH-OH; -C(=O)-OH; -C(=O)-H; - S(=O)₂-OH; -NHR¹¹; -NR¹²R¹³; -OR¹⁴, -SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³, - S(=O)₂-R²⁴; -S(=O)-R²⁴;
für einen linearen oder verzweigten, gesättigten oder ungesättigten, unsubstituierten oder wenigstens einfach substituierten aliphatischen C₁₋₁₀ Rest;
oder für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest steht, der jeweils über ein Kohlenstoffatom im Ring des cycloaliphatischen Restes an das Grundgerüst gebunden ist;
T für C-R⁶ und U für C-R⁷ und V für C-R⁹ und W für C-R¹⁰ steht;
R⁶ und R⁷, unabhängig voneinander, jeweils für H; F; Cl; Br; I; -SF₅; -NO₂; -CF₃; -CF₂Cl; -CN; -NH₂; -OH; -SH; -C(=O)-NH₂; -S(=O)₂-NH₂;-C(=O)-NH-OH; -C(=O)-OH; -C(=O)-H; -S(=O)₂-OH; -NHR¹¹; -NR¹²R¹³; - OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³, -S(=O)-R²⁴; -S(=O)₂-R²⁴; für einen linearen oder verzweigten, gesättigten oder ungesättigten, unsubstituierten oder wenigstens einfach substituierten aliphatischen C₁₋₁₀ Rest ;
oder für einen unsubstituierten oder wenigstens einfach substituierten 6- oder 10-gliedrigen Aryl-Rest, der über eine lineare oder verzweigte, unsubstituierte oder wenigstens einfach substituierte C₁₀-AlkylenGruppe oder C-₂₋₆-Alkenylen-Gruppe oder C₂₋₆-Alkinylen-Gruppe gebunden sein kann, stehen;
R⁹ für H; F; Cl; Bn I; -SF₅, -NO₂; -CF₃; -CF₂Cl; -CN; -NH₂; -OH; -SH; -
C(=O)-NH₂; -S(=O)₂-NH₂; -C(=O)-NH-OH; -C(=O)-OH; -C(=O)-H; - S(=O)₂-OH; -NHR¹¹; -NR¹²R¹³; -OR¹⁴; -SR⁵; -C(=O)-NHR¹⁶; -C(=O)-NR¹⁷R¹⁸; -S(=O₂-NHR¹⁹; -S(=O)₂-NR²⁰R²¹; -C(=O)-OR²²; -C(=O)-R²³; - S(=O)-R²⁴; -S(=O)₂R²⁴ oder für einen linearen oder verzweigten, gesättigten oder ungesättigten, unsubstituierten oder wenigstens einfach substituierten aliphatischen C₁₋₁₀ Rest steht;
R¹⁰ für -SF₅; -NO₂; -CF₃; -CF₂Cl, -CN; -NH₂; -OH; -SH; -C(=O)-NH₂;-S(=O)₂-NH₂; -C(=O)-NH-OH; -C(=O)-OH; -C(=O)-H; -S(=O)₂-OH;-NHR¹¹; -NR¹²R¹³; -OR¹⁴; -SR¹⁵; -C(=O)-NHR¹⁶; -C(=O)-NR¹⁷R¹⁸; - S(=O)₂-NHR¹⁹; -S(=O)₂-NR²⁰R²¹; -C(=O)-OR²²; -C(=O)-R²³; -S(=O)-R²⁴; -S(=O)₂-R²⁴; -C(=NH)-NH₂; -C(=NH)-NH-R²⁷; -N=C(NH₂)₂; - N=C(NHR²⁸)(NHR²⁹)_{;}
für einen linearen oder verzweigten, gesättigten oder ungesättigten aliphatischen C₁₋₁₀ Rest, der jeweils mit ggf. 1, 2, 3, 4, 5, 6, 7, 8 oder 9 Substituenten unabhängig voneinander ausgewählt aus der Gruppe bestehend aus -CN, -NO₂, -OH, -NH₂, -SH, -O(C₁₋₅-Alkyl), -S(C₁₋₅-Alkyl), -NH(C₁₋₅-Alkyl), -N(C₁₋₅Alkyl)(C₁₋₅-Alkyl), -OCF₃ und -SCF₃ substituiert ist;
für einen unsubstituierten C₂₋₁₀ Alkenyl-Rest oder einen unsubstituierten C₂₋₁₀-Akinyl-Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-AlkylenGruppe gebunden sein kann;
oder für einen unsubstituierten oder wenigstens einfach substituierten 5- bis 14-gliedrigen Aryl- oder Heteroaryl-Rest; der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe gebunden sein kann, steht;
R¹¹, R¹², R¹³, R¹⁴, R¹⁵, R¹⁶, R¹⁷, R¹⁸, R¹⁹, R²⁰ R²¹, R²², R²³, R²⁴ R²⁷, R²⁸ und R²⁹, unabhängig voneinander, jeweils
für einen linearen oder verzweigten, gesättigten oder ungesättigten, unsubstituierten oder wenigstens einfach substituierten aliphatischen C₁₋₁₀ Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder 2- bis 6-gliedrige Heteroalkylen-Gruppe gebunden sein kann;
oder für einen unsubstituierten oder wenigstens einfach substituierten 5- bis 14-gliedrigen Aryl- oder Heteroaryl-Rest; der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder 2- bis 6-gliedrige Heteroalkylen-Gruppe gebunden sein kann, stehen;
oder
R¹² und R¹³ jeweils zusammen mit dem sie verbindenden Stickstoffatom als Ringglied einen gesättigten oder ungesättigten, unsubstituierten oder wenigstens einfach substituierten 4-, 5-, 6-, 7-, 8- oder 9-gliedrigen, ggf. wenigstens ein weiteres Heteroatom als Ringglied aufweisenden heterocycloaliphatischen Rest bilden, der mit einem gesättigten oder ungesättigten, unsubstituierten oder wenigstens einfach substituierten mono-oder polyzyklischen Ringsystem kondensiert sein kann;
und
R²⁵ und R²⁶, unabhängig voneinander, jeweils für einen Wasserstoff-Rest;
für einen linearen oder verzweigten, gesättigten oder ungesättigten, unsubstituierten oder wenigstens einfach substituierten aliphatischen C₁₋₁₀ Rest;
für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder C-₂₋₆-Alkenylen-Gruppe oder C₂₋₆-Alkinylen-Gruppe gebunden sein kann;
oder für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest stehen;
mit der Maßgabe, dass R²⁵ und R²⁶ nicht jeweils für einen Wasserstoff-Rest stehen;
oder
R²⁵ und R²⁶ jeweils zusammen mit dem sie verbindenden Kohlenstoffatom als Ringglied einen gesättigten oder ungesättigten, unsubstituierten oder wenigstens einfach substituierten 3-, 4-, 5- oder 6-gliedrigen cycloaliphatischen Rest bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

2. Substituierte Verbindungen der allgemeinen Formel I gemäß Anspruch 1, 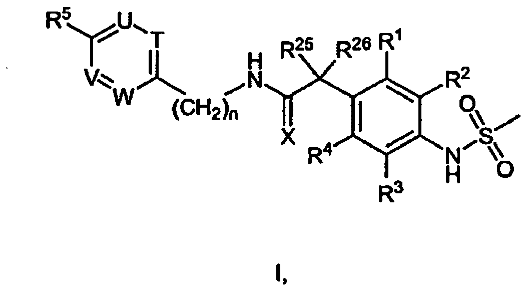 worin
X für O steht;
n für 0, 1, 2, 3 oder 4 steht;
R¹, R², R³ und R⁴, unabhängig voneinander, jeweils für H; F; Cl; Br; I; -SF₅; -NO₂; -CF₃; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴;-SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³; -S(=O)-R²⁴, -S(=O)₂-R²⁴ oder für einen linearen oder verzweigten, gesättigten oder ungesättigten, unsubstituierten oder wenigstens einfach substituierten aliphatischen C₁₋₁₀ Rest stehen;
R⁵ für H; F; Cl; Br, I; -SCF₅; -NO₂; -CF₃; -CF₂Cl; -CN; -NH₂; -OH; -SH; -
C(=O)-NH₂; -S(=O)₂-NH₂; -C(=O)-NH-OH; -C(=O)-OH; -C(=O)-H; - S(=O)₂-OH; -NHR¹¹; -NR¹²R¹³; -OR¹⁴; -SR¹⁵, -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³, - S(=O)-R²⁴; -S(=O)₂-R²⁴
für einen linearen oder verzweigten, gesättigten oder ungesättigten, unsubstituierten oder wenigstens einfach substituierten aliphatischen C₁₋₁₀ Rest;
oder für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest steht, der jeweils über ein Kohlenstoffatom im Ring des cycloaliphatischen Restes an das Grundgerüst gebunden ist;
T für C-R⁶ und U für C-R⁷ und V für C-R⁹ und W für C-R¹⁰ steht;
R⁶ und R⁷, unabhängig voneinander, jeweils für H; F; Cl; Br, **I;** -SF₅; -NO₂; -CF₃; -CF₂Cl; -CN; -NH₂; -OH; -SH; -C(=O)-NH₂; -S(=O)₂NH₂; - C(=O)-NH-OH; -C(=O)-OH; -C(=O)-H; -S(=O)₂-OH; -NHR¹¹; -NR¹²R¹³; - OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²_{;} -C(=O)-R²³, -S(=O)-R²⁴; -S(=O)₂-R²⁴; für einen linearen oder verzweigten, gesättigten oder ungesättigten, unsubstituierten oder wenigstens einfach substituierten aliphatischen C₁₋₁₀ Rest;
oder für einen unsubstituierten oder wenigstens einfach substituierten 6- oder 10-gliedrigen Aryl-Rest, der über eine lineare oder verzweigte, unsubstituierte oder wenigstens einfach substituierte C₁₋₆-AlkylenGruppe oder C-₂₋₆-Alkenylen-Gruppe oder C₂₋₆-Alkinylen-Gruppe gebunden sein kann, stehen;
R⁹ für H; F; Cl; Br, I; -SCF₅; -NO₂; -CF₃; -CF₂Cl; -CN; -NH₂; -OH; -SH; -
C(=O)-NH₂; -S(=O)₂-NH -C(=O)-NH-OH; -C(=O)-OH; -C(=O)-H; - S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³; - S(=O)-R²⁴; -S(=O)₂-R²⁴ oder für einen linearen oder verzweigten, gesättigten oder ungesättigten, unsubstituierten oder wenigstens einfach substituierten aliphatischen C₁₋₁₀ Rest steht;
R¹⁰ für -SF₅; -NO₂; -CF₃; -CF₂Cl; -CN; -NH₂; -OH; -SH; -C(=O)-NH₂; -
S(=O)₂-NH₂; -C(=O)-NH-OH; -C(=O)-OH; -C(=O)-H; -S(=O)₂-OH; - NHR¹¹; -NR¹²R¹³; -OR¹⁴; -SR¹⁵; -C(=O)-NHR¹⁶; -C(=O)-NR¹⁷R¹⁸; - S(=O)₂-NHR¹⁹_{;} -S(=O)₂NR²⁰R²¹; -C(=O)-OR²²; -C(=O)-R²³; -S(=O)-R²⁴; -S(=O)₂-R²⁴; -C(=NH)-NH₂; -C(=NH)-NH-R²⁷; -N=C(NH₂)₂; - N=C(NHR²⁸)(NHR₂₉)_{;}
für einen linearen oder verzweigten, gesättigten oder ungesättigten aliphatischen C₁₋₁₀ Rest, der jeweils mit ggf.1, 2, 3, 4, 5, 6, 7, 8 oder 9 Substituenten unabhängig voneinander ausgewählt aus der Gruppe bestehend aus -CN, -NO₂, -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), -OCF₃ und -SCF₃ substituiert ist;
für einen unsubstituierten C₂₋₁₀ Alkenyl-Rest oder einen unsubstituierten C₂₋₁₀-Akinyl-Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-AlkylenGruppe gebunden sein kann;
oder für einen unsubstituierten oder wenigstens einfach substituierten 5- bis 14-gliedrigen Aryl- oder Heteroaryl-Rest; der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe gebunden sein kann, steht;
R¹¹, R¹², R¹³, R¹⁴, R¹⁵, R¹⁶, R¹⁷, R¹⁸, R¹⁹, R²⁰, R²¹, R²² R²³ R²⁴, R²⁷ R²⁸ und R²⁹, unabhängig voneinander, jeweils
für einen linearen oder verzweigten, gesättigten oder ungesättigten, unsubstituierten oder wenigstens einfach substituierten aliphatischen C₁₋₁₀ Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder 2- bis 6-gliedrige Heteroalkylen-Gruppe gebunden sein kann;
oder für einen unsubstituierten oder wenigstens einfach substituierten 5- bis 14-gliedrigen Aryl- oder Heteroaryl-Rest; der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder 2- bis 6-gliedrige Heteroalkylen-Gruppe gebunden sein kann, stehen;
oder
R¹² und R¹³ jeweils zusammen mit dem sie verbindenden Stickstoffatom als Ringglied einen gesättigten oder ungesättigten, unsubstituierten oder wenigstens einfach substituierten 4-, 5-, 6-, 7-, 8- oder 9-gliedrigen, ggf. wenigstens ein weiteres Heteroatom als Ringglied aufweisenden heterocycloaliphatischen Rest bilden, der mit einem gesättigten oder ungesättigten, unsubstituierten oder wenigstens einfach substituierten mono-oder polyzyklischen Ringsystem kondensiert sein kann;
und
R²⁵ und R²⁶, unabhängig voneinander, jeweils für einen Wasserstoff-Rest;
für einen linearen oder verzweigten, gesättigten oder ungesättigten, unsubstituierten oder wenigstens einfach substituierten aliphatischen C₁₋₁₀ Rest;
für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder C-₂₋₆-Alkenylen-Gruppe oder C₂₋₆-Alkinylen-Gruppe gebunden sein kann;
oder für einen ungesättigten oder gesättigten, unsubstituierten oder wenigstens einfach substituierten, ggf. wenigstens ein Heteroatom als Ringgüed aufweisenden 3-, 4-, 5-, 6-, 7-, 8- oder 9-gliedrigen cycloaliphatischen Rest stehen;
mit der Maßgabe, dass R²⁵ und R²⁶ nicht jeweils für einen Wasserstoff-Rest stehen;
oder
R²⁵ und R²⁶ jeweils zusammen mit dem sie verbindenden Kohlenstoffatom als Ringglied einen gesättigten oder ungesättigten, unsubstituierten oder wenigstens einfach substituierten 3-, 4-, 5- oder 6-gliedrigen cycloaliphatischen Rest bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

3. Verbindungen gemäß Anspruch 1 oder 2, **dadurch gekennzeichnet, dass** X für O steht;
n für 0, 1, 2, 3 oder 4 steht;
R¹, R², R³ und R⁴, unabhängig voneinander, jeweils für H; F; Cl; Br, I; -SF₅-, -NO₂; -CF₃; -CN; -NH₂; -OH; -SH; -C(=O)-NH₂; -S(=O)₂-NH₂; -C(=O)-NH-OH; -C(=O)-OH_{;} -C(=O)-H; -S(=O)₂-OH; -NHR¹¹; -NR12R¹³; -OR¹⁴; SR¹⁵; -C(=O)-NHR¹⁶; -C(=O)-NR¹⁷R¹⁸; -S(=O)₂-NHR¹⁹, -S(=O)₂-NR²⁰R²¹; -C(=O)-OR²²; -C(=O)-R²³; -S(=O)-R²⁴; -S(=O)₂-R²⁴ oder für einen linearen oder verzweigten, gesättigten oder ungesättigten aliphatischen C₁₋₁₀ Rest stehen;
R⁵ für H; F; Cl; Bn I; -SF₅; -NO₂; -CF₃; -CF₂Cl; -CN; -NH₂, -OH; -SH; -
C(=O)-NH₂; -S(=O)₂-NH₂; -C(=O)-NH-OH; -C(=O)-OH; -C(=O)-H; - S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²_{;} -C(=O)-R²³, - S(=O)-R²⁴; -S(=O)₂-R²⁴ oder für einen linearen oder verzweigten, gesättigten oder ungesättigten aliphatischen C₁₋₁₀ Rest;
oder für einen ungesättigten oder gesättigten, unsubstituierten oder wenigstens einfach substituierten ggf. wenigstens ein Heteroatom als Ringglied aufweisenden 3-, 4-, 5-, 6-, 7-, 8- oder 9-gliedrigen cycloaliphatischen Rest steht, der jeweils über ein Kohlenstoffatom im Ring des cycloaliphatischen Restes an das Grundgerüst gebunden ist;
T für C-R⁶ und U für C-R⁷ und V für G-R⁹ und W für C-R¹⁰ steht;
R⁶ und R⁷, unabhängig voneinander, jeweils für H; F; Cl; Br, I; -SF₅; -NO₂, -CF₃; -CF₂Cl; -CN; -NH₂; -OH; -SH; -C(=O)-NH₂; -S(=O)₂-NH₂; - C(=O)-NH-OH, -C(=O)-OH; -C(=O)-H; -S(=O)₂-OH; -NHR¹¹; -NR¹²R¹³; - OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³, -S(=O)-R²⁴; -S(=O)₂-R²⁴; für einen linearen oder verzweigten, gesättlgten oder ungesättigten aliphatischen C₁₋₁₀ Rest;
oder für einen unsubstituierten oder wenigstens einfach substituierten 6- oder 10-gliedrigen Aryl-Rest, der über eine linearen oder verzweigte, unsubstituierte oder wenigstens einfach substituierte C₁₋₆-AlkylenGruppe oder C-₂₋₆-Alkenylen-Gruppe oder C₂₋₆-Alkinylen-Gruppe gebunden sein kann, stehen;
R⁹ für H; F; Cl; Br; I; -SF₅; -NO₂; -CF₃; -CF₂Cl; -CN; -NH₂; -OH; -SH; -
C(=O)-NH₂; -S(=O)₂-NH₂; -C(=O)-NH-OH: -C(=O)-OH; -C(=O)-H; - S(=O)₂-OH; -NHR¹¹; -NR¹²R¹³; -OR¹⁴; -SR¹⁵, -C(=O)-NHR¹⁶; -C(=O)-NR¹⁷R¹⁸; -S(=O)₂-NHR¹⁹, -S(=O)₂-NR²⁰R²¹; -C(=O)-OR²²; -C(=O)-R²³; - S(=O)-R²⁴; -S(=O)₂-R²⁴ oder für einen linearen oder verzweigten, gesättigten oder ungesättigten aliphatischen C₁₋₁₀ Rest steht;
R¹⁰ für -SF₅; -NO₂; -CF₃; -CF₂Cl; -CN; -NH₂; -OH; -SH; -C(=O)-NH₂; -
S(=O)₂-NH₂; -C(=O)-NH-OH; -C(=O)-OH; -C(=O)-H; -S(=O)₂-OH; - NHR¹¹; -NR¹²R¹³; -OR¹⁴; -SR¹⁵; -C(=O)-NHR¹⁶; -C(=O)-NR¹⁷R¹⁸; - S(=O)₂-NHR¹⁹, -S(=O)₂-NR²⁰R²¹; -C(=O)-OR²²_{;} -C(=O)-R²³; -S(=O)-R²⁴; -S(=O)₂-R²⁴; -C(=NH)-NH_{2;} -C(=NH)-NH-R²⁷; -N=C(NH₂)₂; - N=C(NHR²⁸)(NH²⁹);
für einen linearen oder verzweigten, gesättigten oder ungesättigten aliphatischen C₁₋₁₀ Rest, der jeweils mit ggf. 1, 2, 3, 4, 5, 6, 7, 8 oder 9 Substituenten unabhängig voneinander ausgewählt aus der Gruppe bestehend aus -CN, -NO₂, -OH, -NH₂, -SH, -O(C₁₋₅-Alkyl), -S(C₁₋₅-Alkyl), -NH(C₁₋₅-Alkyl), -N(C₁₋₅-Alkyl)(₁₋₅-Alkyl), -OCF₃ und -SCF₃ substituiert ist;
für einen unsubstituierten C₂₋₁₀ Alkenyl-Rest oder einen unsubstituierten C₂₋₁₀-Akinyl-Rest;
für einen ungesättigten oder gesättigten, unsubstituierten oder wenigstens einfach substituierten 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆₋AlkylenGruppe gebunden sein kann;
oder für einen unsubstituierten oder wenigstens einfach substituierten 5- bis 14-gliedrigen Aryl- oder Heteroaryt-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe gebunden sein kann, steht;
R¹¹, R¹², R¹³, R¹⁴, R¹⁵, R¹⁶, R¹⁷, R¹⁸, R¹⁹, R²⁰, R²¹, R²², R2³, R²⁴, R²⁷, R²⁸ und R²⁹, unabhängig voneinander, jeweils
für einen linearen oder verzweigten, gesättigten oder ungesättigten aliphatischen C₁₋₁₀ Rest;
für einen ungesättigten oder gesättigten, unsubstituierten oder wenigstens einfach substituierten 3-, 4-, 5-, 6-, 7-, 8- oder 9-gliedrigen cycloaliphatischen Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder 2- bis 6-gliedrige Heteroalkylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder 2- bis 6-gliedrige Heteroalkylen-Gruppe gebunden sein kann, stehen;
oder
R¹² und R¹³ jeweils zusammen mit dem sie verbindenden Stickstoffatom als Ringglied einen gesättigten oder ungesättigten, unsubstituierten oder wenigstens einfach substituierten 4-, 5-, 6-, 7-, 8- oder 9-gliedrigen, ggf. wenigstens ein weiteres Heteroatom als Ringglied aufweisenden heterocycloaliphatischen Rest bilden, der mit einem gesättigten oder ungesättigten, unsubstituierten oder wenigstens einfach substituierten mono-oder polyzyklischen Ringsystem kondensiert sein kann;
und
R²⁵ und R²⁶, unabhängig voneinander, jeweils für einen Wasserstoff-Rest;
für einen linearen oder verzweigten, gesättigten oder ungesättigten aliphatischen C₁₋₁₀ Rest;
für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder C-₂₋₆-Alkenylen-Gruppe oder C₂₋₆-Alkinylen-Gruppe gebunden sein kann;
oder für einen ungesättigten oder gesättigten 3-, 4-, 5-, 6-, 7-, 8- oder 9-gliedrigen cycloaliphatischen Rest stehen;
mit der Maßgabe, dass R²⁵ und R²⁶ nicht jeweils für einen Wasserstoff-Rest stehen;
oder
R²⁵ und R²⁶ jeweils zusammen mit dem sie verbindenden Kohlenstoffatom als Ringglied einen gesättigten oder ungesättigten 3-, 4-, 5- oder 6-gliedrigen
cycloaliphatischen Rest bilden;
wobei
sofern nicht anders angegeben, die vorstehend genannten aliphatischen C₁₋₁₀ Reste ggf. jeweils mit 1, 2, 3, 4, 5, 6, 7, 8 oder 9 Substituenten unabhängig voneinander ausgewählt aus der Gruppe bestehend aus F, Cl, Br, I, -CN, - NO₂, -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), -C(=O)-O-C₁₋₅-Alkyl, -OC(=O)-C₁₋₅-Alkyl, -O-Phenyl, Phenyl, -OCF₃ und -SCF₃ substituierte sein können;
die vorstehend genannten 2- bis 6-gliedrigen Heteroalkylen-Gruppen, C₁₋₆-Alkylen-Gruppen und C₂₋₆-Alkenylen-Gruppen und C₂₋₆-Alkinylen-Gruppen ggf. jeweils mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, -O(C₁₋₅-Alkyl), -S(C₁₋₆-Alkyl), -NH(C₁₋₅-Alkyl), -N(C₁₋₅-Alkyl)(C₁₋₅-Alkyl), - OCF₃ und -SCF₃ substituiert sein können;
die vorstehend genannten Heteroalkylen-Gruppen jeweils ggf. 1, 2 oder 3 Heteroatom(e) unabhängig voneinander ausgewählt aus der Gruppe bestehend aus Sauerstoff, Schwefel und Stickstoff (NH) als Kettenglied(er) aufweisen;
die vorstehend genannten (hetero)cycloaliphatischen Reste ggf. jeweils mit 1, 2, 3, 4 oder 5 Substituenten unabhängig voneinander ausgewählt aus der Gruppe bestehend aus -C₁₋₆-Alkylen-OH, =CH₂, -O-C₁₋₅-Alkylen-Oxetanyl, -C₁₋₅-Alkylen-O-C₁₋₅-Alkylen-Oxetanyl,-CH₂-NH-C₁₋₅-Alkyl, -CH₂-N(C₁₋₅-Alkyl, - N[C(=O)-C₁₋₅-Alkyl)-Phenyl, -CH₂-O-C₁₋₅-Alkyl, Oxo (=O), Thioxo (=S), F, Cl, Br, I, -CN, -CF₃, -SF₅, -OH, -O-C₁₋₅-Alkyl, -O-C(=O)-C₁₋₅-Alkyl, -NH₂, -NO₂, -O-CF₃, -S-CF₃, -SH, -S-C₁₋₅-Alkyl -C₁₋₅-Alkyl, -C(=O)-C₁₋₅-Alkyl, -C(=O)-OH, - C(=O)-O-C₁₋₅-Alkyl, -NH-C₁₋₅-Alkyl, -N(C₁₋₅-Alkyl)₂, -NH-Phenyl, -N(C₁₋₅-Alkyl)-Phenyl, Cyclohexyl, Cyclopentyl, (4,5)-Dihydroisoxazolyl, Thiazol, (1,2,5)-Thiadiazolyl, Thiophenyl, Phenethyl, Piperidinyl, Pyrrolidinyl, -(CH₂)-Pyridinyl, Pyridinyl, -O-Phenyl, -O-Benzyl, Phenyl und Benzyl substituiert sein, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N[C(=O)-C₁₋₅-Alkyl]-Phenyl, -NH-Phenyl, -N(C₁₋₅-Alkyl)-Phenyl, -(CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,-und die vorstehend genannten (hetero)cycloaliphatischen Reste jeweils ggf. 1, 2 oder 3 (weitere) Heteroatom(e) unabhängig voneinander ausgewählt aus der Gruppe bestehend aus Sauerstoff, Stickstoff und Schwefel aufweisen können;
die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-C₁₋₅-Alkyl, -C(=O)-OH, -C(=O)-O-C₁₋₅-Alkyl, -NH-C₁₋₅-Alkyl, -N(C₁₋₅-Alkyl)₂, -O-Phenyl, -O-Benzyl, Phenyl und Benzyl substituiert sein können, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, - NO₂, -C₁₋₅-Alkyl, -O-C₁₋₅-Alkyl, -O-CF₃, -S-CF₃, Phenyl und -O-Benzyl substituiert sein kann,
und die Ringe der vorstehend genannten mono- oder polyzyklisdhen Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
und die vorstehend genannten Aryl- oder Heteroaryl-Reste ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂, - NO₂, -O-CF₃, -S-CF₃, -SH, -S-C₁₋₅-Alkyl, -C₁₋₅-Alkyl, -C(=O)-OH, -C(=O)-O-C₁-₅-Alkyl, -NH-C₁₋₅-Alkyl, -N(C₁₋₅-Alkyl)₂, -NH-S(=O)₂-C₁₋₅-Alkyl, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, - C(=O)-N-(C₁₋₅-Alkyl)₂, -O-Phenyl, -O-Benzyl, Phenyl und Benzyl substituiert sein können, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, - NO₂, -C₁₋₅-Alkyl, -O-C₁₋₅-Alkyl, -O-CF₃, -S-CF₃, Phenyl und -O-Benzyl substituiert sein kann,
und
die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

4. Verbindungen gemäß einem oder mehreren der Ansprüche 1 bis 3, **dadurch**
**gekennzeichnet, dass**
n, X, Y, T, U, V, W, R¹ bis R⁷, R⁹ und R¹¹ bis R²⁹ wie in Anspruch 1 definiert sind und
R¹⁰ für -SF₅; -NO₂; -CF₃; -CF₂Cl; -CN; -NH₂; -OH; -SH; -C(=O)-NH₂; -
S(=O)₂-NH₂; -C(=O)-NH-OH; -C(=O)-OH; -C(=O)-H; -S(=O)₂-OH; - C(=O)-NHR¹⁶; -C(=O)-NR¹⁷R¹⁸; -S(=O)₂-NHR¹⁹; -S(=O)₂-NR²⁰R²¹; - C(=O)-OR²²_{;} -C(=O)-R²³; -S(=O)-R²⁴; -S(=O)₂-R²⁴; -C(=NH)-NH₂; - C(=NH)-NH-R²⁷; -N=C(NH₂)₂; -N=C(NHR²⁸)(NHR²⁹);
für eine ungesättigte oder gesättigte, unsubstituierte oder wenigstens einfach substituierte Kette, die 1 bis 7 Kohlenstoffatome als Kettenglieder aufweist, wobei 1, 2 oder 3 Kohlenstoffatome durch Heteroatome ausgewählt aus der Gruppe bestehend aus Sauerstoff, Schwefel und Stickstoff (NH) ersetzt werden können und die in Abwesenheit von Heteroatomen als Kettengliedern jeweils mit ggf. 1, 2, 3, 4, 5, 6, 7, 8 oder 9 Substituenten unabhängig voneinander ausgewählt aus der Gruppe bestehend aus -CN, -NO₂, -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), - OCF₃ und -SCF₃ substituiert ist;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-AlkylenGruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe gebunden sein kann, steht.

5. Verbindungen gemäß Anspruch 4, **dadurch gekennzeichnet, dass** n, X, Y, T, U, V, W, R¹ bis R⁷, R⁹ und R¹¹ bis R²⁹ wie in Anspruch 4 definiert
sind und
R¹⁰ für -SF₅; -NO₂: -CF₃; -CF₂Cl; -CN; -NH₂; -OH; -SH; -C(=O)-NH₂; -
S(=O)₂-NH₂; -C(=O)-NH-OH; -C(=O)-OH; -C(=O)-H; -S(=O)₂-OH; - C(=O)-NHR¹⁶; -C(=O)-NR¹⁷R¹⁸; -S(=O)₂-NHR¹⁹; -S(=O)₂-NR²⁰R²¹; - C(=O)-OR²²; -C(=O)-R²³; -S(=O)-R²⁴; -S(=O)₂-R²⁴; -C(=NH)-NH₂; - C(=NH)-NH-R²⁷; -N=C(NH₂)₂; -N=C(NHR²⁸)(NHR²⁹);
für eine ungesättigte oder gesättigte, unsubstituierte oder wenigstens einfach substituierte Kette, die 5 bis 7 Kohlenstoffatome als Kettenglieder aufweist, wobei 1, 2 oder 3 Kohlenstoffatome. durch Heteroatome ausgewählt aus der Gruppe bestehend aus Sauerstoff und Schwefel ersetzt werden können und die in der Abwesenheit von Heteroatomen als Kettengliedern jeweils mit ggf. 1, 2, 3, 4, 5, 6, 7, 8 oder 9 Substituenten unabhängig voneinander ausgewählt aus der Gruppe bestehend aus -CN, -NO₂, -OH, -NH₂, -SH, -O(C₁₋₅-Alkyl), - S(C₁₋₅-Alkyl), -NH(C₁₋₅-Alkyl), -N(C₁₋₅-Alkyl)(C₁₋₅-Alkyl), -OCF₃ und - SCF₃ substituiert ist;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₅-AlkylenGruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe gebunden sein kann, steht.

6. Verbindungen der allgemeinen Formel B1 gemäß einem oder mehreren der
Ansprüche 1 bis 5, 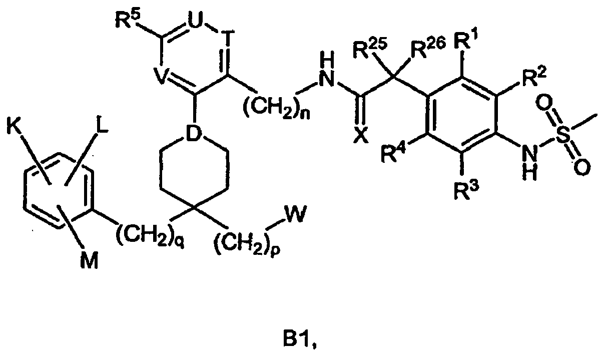 worin
U, T, V, X, n, R¹, R², R³, R⁴, R⁵, R²⁵ und R²⁶ wie in Anspruch 3 definiert sind;
D ist CH oder N;
p ist 0, 1, 2 oder 3;
q ist 0, 1, 2 oder 3;
K, L und M, unabhängig voneinander, jeweils für H, F, Cl, Br, I, -CN, - CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -NH-C₁₋₅-Alkyl, -N(C₁₋₅-Alkyl)₂, -NH-S(=O)₂-C₁₋₅-Alkyl, -NH-C(=O)-O-C₁₋₅-Alkyl, -C(=O)-H, -
C(=O)-C₁₋₅-Alkyl, -C(=O)-NH₂, -C(=O)-NH-C₁₋₅-Alkyl, -C(=O)-N-(C₁₋₅-Alkyl)₂, -O-Phenyl, -O-Benzyl, Phenyl und Benzyl stehen, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, - C₁₋₅-Alkyl, -O-C₁₋₅-Alkyl, -O-CF₃, -S-CF₃, Phenyl und -O-Benzyl substituiert sein kann;
W für -CN, -NR³⁴R³⁵, -C(=O)-R³⁶ oder -C(=O)-OR³⁷ steht;
und R³⁴, R³⁵, R³⁶ und R³⁷, unabhängig voneinander, jeweils für Wasserstoff, oder für einen linearen oder verzweigten, gesättigten oder ungesättigten aliphatischen C₁₋₁₀ Rest
für einen ungesättigten oder gesättigten, unsubstituierten oder wenigstens einfach substituierten 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder C-₂₋₆-Alkenylen-Gruppe oder C₂₋₆-Alkinylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder C-₂₋₆-Alkenylen-Gruppe oder C₂₋₆-Alkinylen-Gruppe gebunden sein kann, stehen.

7. Verbindungen der allgemeinen Formel B2 gemäß Anspruch 6, 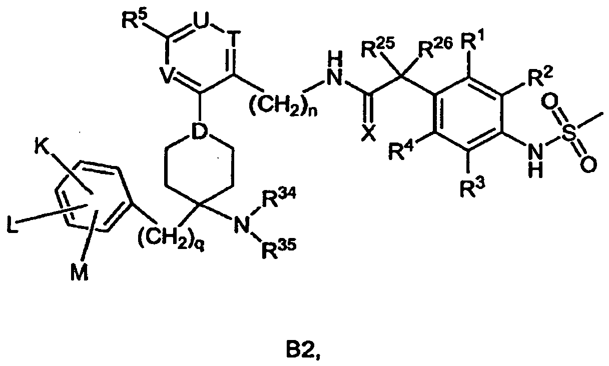 worin
U, T, V, X, n, R¹, R², R³, R⁴, R⁵, R²⁵ und R²⁶ wie in Anspruch 3 definiert sind;
D ist CH oder N;
q ist 0, 1, 2 oder 3;
K, L und M, unabhängig voneinander, jeweils für H, F, Cl, Br, I, -CN, - CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -NH-C₁₋₅-Alkyl, -N(C₁₋₅-Alkyl)₂, -NH-S(=O)₂-C₁₋₅-Alkyl, -NH-C(=O)-O-C₁₋₅-Alkyl, -C(=O)-H, - C(=O)-C₁₋₆-Alkyl, -C(=O)-NH₂, -C(=O)-NH-C₁₋₆-Alkyl, -C(=O)-N-(C₁₋₅-Alkyl)₂, -O-Phenyl, -O-Benzyl, Phenyl und Benzyl stehen, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, - C₁₋₅-Alkyl, -O-C₁₋₅-Alkyl, -O-CF₃, -S-CF₃, Phenyl und -O-Benzyl substituiert sein kann;
und R³⁴ und R³⁵, unabhängig voneinander, jeweils für Wasserstoff, oder für einen linearen oder verzweigten, gesättigten oder ungesättigten aliphatischen C₁₋₁₀ Rest;
für einen ungesättigten oder gesättigten, unsubstituierten oder wenigstens einfach substituierten 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder C-₂₋₆-Alkenylen-Gruppe oder C₂₋₆-Alkinylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder C-₂₋₆-Alkenylen-Gruppe oder C₂₋₆-Alkinylen-Gruppe gebunden sein kann, stehen.

8. Verbindungen gemäß einem oder mehreren der Ansprüche 1 bis 7, **dadurch gekennzeichnet, dass**
X für O steht;
n für 0, 1, 2, 3 oder 4 steht;
R¹, R², R³ und R⁴, unabhängig voneinander, jeweils für H; F; Cl; Br; I; -SF₅; -NO₂; -CN; -CF₃; -NH₂, -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; - SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³; -S(=O)-R²⁴; -S(=O)₂-R²⁴ oder für einen Alkyl-Rest ausgewählt aus der Gruppe bestehend aus Methyl, Ethyl, n-Propyl, Isopropyl, tert-Butyl, n-Butyl, sek-Butyl, Isobutyl, n-Pentyl, n-Hexyl und n-Heptyl stehen;
R⁵ für H; F; Cl; Br; I; -SF₅; -NO₂; -CF₃; -CF₂Cl; -CN; -NH₂; -OH; -SH; -
C(=O)-NH₂; -S(=O)₂-NH₂; -C(=O)-NH-OH; -C(=O)-OH; -C(=O)-H; - S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³; - S(=O)-R²⁴; -S(=O)₂-R²⁴;
für einen Alkyl-Rest ausgewählt aus der Gruppe bestehend aus Methyl, Ethyl, n-Propyl, Isopropyl, tert-Butyl, n-Butyl, sek-Butyl, Isobutyl, n-Pentyl, n-Hexyl und n-Heptyl;
oder für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl Piperazinyl, Azepanyl, Diazepanyl, Azocanyl und Thiomorpholinyl, der jeweils über ein Kohlenstoffatom der Ringe der vorstehend genannten Reste an das Grundgerüst gebunden ist und ggf. jeweils mit 1, 2, 3, 4 oder 5 Substituenten unabhängig voneinander ausgewählt aus der Gruppe bestehend aus -CH₂-O-CH₃, -CH₂-O-CH₂-CH₃, Oxo (=O), Thioxo (=S), F, Cl, Br, I, -CN, -CF₃, -SF₅, -OH, -O-CH₃, - O-C₂H₅, -O-CH(CH₃)₂, -O-C(CH₃)₃, -NH₂, -NO₂, -O-CF₃, -S-CF₃, -SH, - S-CH₃, -S-C₂H₅, -S-CH(CH₃)₂, -S-C(CH₃)₃, Methyl, Ethyl, n-Propyl, isopropyl, n-Butyl, sek-Butyl, Isobutyl, tert-Butyl, n-Pentyl, -C(=O)-CH₃, - C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -NH-Phenyl, -N(CH₃)-Phenyl, -N(C₂H₅)-Phenyl, -N(C₂H₅)₋Phenyl, -O-CH₂-CH₂-CH₂-CH₃, Piperidinyl, Pyrrolidinyl, -O-C(=O)-CH₃, -O-C(=O)-C₂H₅, - O-C(=O)-C(CH₃)₃, -(CH₂)-Pyridinyl, Pyridinyl, -O-Phenyl, -O-Benzyl, Phenyl und Benzyl substituiert sein, wobei jeweils der zyklische Teil der Reste -NH-Phenyl, -N(CH₃)-Phenyl, -N(C₂H₅)-Phenyl, -(CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sek-Butyl, Isobutyl, tert-Butyl, -O-CH₃, -O-C₂H₅, -O-CH(CH₃)₂, -O-C(CH₃)₃, -O-CF₃, -S-CF₃, Phenyl und -O-Benzyl substituiert sein kann, steht;
T für C-R⁶ und U für C-R⁷ und V für C-R⁹ und W für C-R¹⁰ steht;
R⁶ und R⁷, unabhängig voneinander, jeweils für H; F; Cl; Br; I; -SF₅; -NO₂; -CF₃; -CF₂Cl; -CN; -NH₂; -OH; -SH; -C(=O)-NH₂; -S(=O)₂-NH₂; - C(=O)-OH; -C(=O)-H; -S(=O)₂-OH; -NHR¹¹; -NR¹²R¹³; -OR¹⁴; -SR¹⁵; - C(=O)-NHR¹⁶_{;} -C(=O)-NR¹⁷R¹⁸; -S(=O)₂-NHR¹⁹; -S(=O)₂-NR²⁰R²¹; - C(=O)-OR²²; -C(=O)-R²³, -S(=O)-R²⁴; -S(=O)₂-R²⁴; für einen Alkyl-Rest ausgewählt aus der Gruppe bestehend aus Methyl, Ethyl, n-Propyl, Isopropyl, tert-Butyl, n-Butyl, sek-Butyl, Isobutyl, n-Pentyl, n-Hexyl und n-Heptyl oder für einen Phenyl-Rest stehen, der über eine -(CH=CH)-, - C≡C-, -(CH₂)-, -(CH₂)₂- oder -(CH₂)₃-Gruppe gebunden sein kann und/oder ggf. mit 1, 2, 3, 4 oder 5 Substituenten unabhängig voneinander ausgewählt aus der Gruppe bestehend aus F, Cl, Br, I, - CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH. -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl und n-Pentyl substituiert sein kann;
R⁹ für H; F; Cl; Br, I; -SF₅; -NO₂; -CF₃; -CF₂Cl; -CN; -NH₂; -OH; -SH; -
C(=O)-NH₂; -S(=O)₂-NH₂; -C(=O)-NH-OH; -C(=O)-OH; -C(=O)-H; - S(=O)₂-OH; -NHR¹¹; -NR¹²R¹³; -OR¹⁴; -SR¹⁵; -C(=O)-NHR¹⁶; -C(=O)-NR¹⁷R¹⁸; -S(=O)₂-NHR¹⁹; -S(=O)₂-NR²⁰R²¹; -C(=O)-OR²²; -C(=O)-R²³; - S(=O)-R²⁴; -S(=O)₂-R²⁴ oder für einen Alkyl-Rest ausgewählt aus der Gruppe bestehend aus Methyl, Ethyl, n-Propyl, Isopropyl, tert-Butyl, n-Butyl, sek-Butyl, Isobutyl, n-Pentyl, n-Hexyl und n-Heptyl steht;
R¹⁰ für -SF₅; -NO₂; -CF₃; -CF₂Cl; -CN; -NH₂; -OH; -SH; -C(=O)-NH₂; -
S(=O)₂-NH₂; -C(=O)-NH-OH; -C(=O)-OH; -C(=O)-H; -S(=O)₂-OH; - NHR¹¹; -NR¹²R¹³; -OR¹⁴; -SR¹⁵; -C(=O)-NHR¹⁶; -C(=O)-NR¹⁷R¹⁸; - S(=O)₂-NHR¹⁹; -S(=O)₂-NR²⁰R²¹; -C(=O)-OR²²; -C(=O)-R²³; -S(=O)-R²⁴; -S(=O)₂-R²⁴; -C(=NH)-NH₂; -C(=NH)-NH-R²⁷; -N=C(NH₂)₂; - N=C(NHR²⁸)(NHR²⁹);
für einen Alkyl-Rest ausgewählt aus der Gruppe bestehend aus Methyl, Ethyl, n-Propyl, Isopropyl, tert-Butyl, n-Butyl, sek-Butyl, Isobutyl, n-Pentyl, n-Hexyl und n-Heptyl, der jeweils mit ggf. 1, 2, 3, 4, 5, 6, 7, 8 oder 9 Substituenten unabhängig voneinander ausgewählt aus der Gruppe bestehend aus -CN, -NO₂, -OH, -NH₂, -SH, -O-CH₃, -O-C₂H₅, - O-CH(CH₃)₂, -O-C(CH₃)₃, -S-CH₃, -S-C₂H₅, -S-CH(CH₃)₂, -S-C(CH₃)₃, - NH-CH₃, -NH-C₂H₅, -NH-C(CH₃)₃, -N(CH₃)₂, -N(C₂H₅)₂, -N(CH₃)(C₂H₅),-OCF₃ und -SCF₃ substituiert ist;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, Oxo (=O), Thioxo (=S), F, Cl, Br, I, -CN, -CF₃, - SF₅, -OH, -O-CH₃, -O-C₂H₅, -O-CH(CH₃)₂, -O-C(CH₃)₃, -NH₂, -NO₂, -0-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃. -C(=O)-C₂H₅, -C(=O)-CH(CH₃)₂, -C(=O)-C(CH₃)₃, - C(=O)-OH, -C(=O)-O-CH₃, -C(=O)-O-C₂H₅, -C(=O)-O-CH(CH₃)₂, - C(=O)-O-C(CH₃)₃, -NH-CH₃, -NH-C₂H₅, -NH-C(CH₃)₃, -N(CH₃)₂, - N(C₂H₅)₂, -N(CH₃)C₂H₅), -NH-Phenyl, -N(CH₃)-Phenyl, -N(C₂H₅)-Phenyl, -N(C₂H₅)-Phenyl, -O-CH₂-CH₂-CH₂-CH₃, Piperidinyl, Pyrrolidinyl, -O-C(=O)-CH₃, -O-C(=O)-C₂H₅, -O-C(=O)-C(CH₃)₃, -(CH₂)-Pyridinyl, Pyridinyl, -O-Phenyl, -O-Benzyl, Phenyl und Benzyl substituiert sein, wobei jeweils der zyklische Teil der Reste -NH-Phenyl, -N(CH₃)-Phenyl, -N(C₂H₅)-Phenyl, -(CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sek Butyl, Isobutyl, tert-Butyl, -O-CH₃, -O-C₂H₅, -O-CH(CH₃)₂, -O-C(CH₃)₃, -O-CF₃, -S-CF₃, Phenyl und -O-Benzyl substituiert sein kann, steht;
oder für einen Rest ausgewählt aus der Gruppe bestehend aus Tetrazolyl, Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, (2,3)-Dihydrothieno[3,4-b][1,4]dioxinyl, Benzo[b]thiophenyl, Benzoxazolyl, Benzisoxazolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl, der jeweils über eine -(CH=CH)-. -C≡C-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -NH-C(=O)-O-CH₃, -NH-S(=O)₂-CH₃, -NH-S(=O₂)-C₂H₅, -NH-S(=O)₂-CH(CH₃)₂, -NH-C(=O)-O-C₂H₅, -NH-C(=O)-O-C(CH₃)₃, -C(=O)-H, - C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein kann, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl
und -O-Benzyl substituiert sein kann, steht;
R¹¹, R¹², R¹³, R¹⁴, R¹⁵, R¹⁶, R¹⁷, R¹⁸, R¹⁹, R²⁰, R²¹, R²², R²³, R²⁴, R²⁷, R²⁸ und R²⁹, unabhängig voneinander, jeweils
für einen Rest ausgewählt aus der Gruppe bestehend aus Methyl, Ethyl, n-Propyl, Isopropyl, tert-Butyl, n-Butyl, sek-Butyl, Isobutyl, n-Pentyl, n-Hexyl, n-Heptyl, 3-Pentyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, Ethenyl, Propenyl, 2-Butenyl, 3-Butenyl, 2-Pentenyl und 3-Pentenyl;
für einen Rest ausgewählt aus der Gruppe bestehend aus 2,3-Dihydro-1H-indenyl, Cyclopropyl, Oxetanyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über eine -CH₂-O-, -CH₂-CH₂-O-, - CH₂-CH₂-O-CH₂-, -CH₂-CH(CH₃)-O-CH₂, -(CH₂)-, -(CH₂)₂- oder -(CH₂)₃-Gruppe gebunden sein kann und/oder ggf. mit 1, 2, 3, 4 oder 5 Substituenten unabhängig voneinander ausgewählt aus der Gruppe bestehend aus -CH₂-O-CH₃, -CH₂-O-CH₂-CH₃, Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, -C(=O)-C(CH₃}₃, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -NH-Phenyl, -N(CH₃)-Phenyl, -N(C₂H₅)-Phenyl, -N(C₂H₅)-Phenyl, -O-CH₂-CH₂-CH₂-CH₃, Piperidinyl, Pyrrolidinyl, -O-C(=O)-CH₃, -O-C(=O)-C₂H₅, -O-C(=O)-C(CH₃)₃, -(CH₂)-Pyridinyl, Pyridinyl, -O-Phenyl, -O-Benzyl, Phenyl und Benzyl substituiert sein, wobei jeweils der zyklische Teil der Reste -NH-Phenyl, -N(CH₃)-Phenyl, -N(C₂H₅)-Phenyl, -(CH₂)-Pyridinyl, Pyridinyl, - O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sek-Butyl, Isobutyl, tert-Butyl, -O-CH₃, -O-C₂H₅, -O-CH(CH₃)₂, -O-C(CH₃)₃, -O-CF₃, -S-CF₃, Phenyl und -O-Benzyl substituiert sein kann, steht;
oder für einen Rest ausgewählt aus der Gruppe bestehend aus Phenyl, Naphthyl, (1,3)-Benzodioxolyl, (1,4)-Benzodioxanyl, Thiophenyl,
Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzoxazolyl, Benzisoxazolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl, wobei der Rest jeweils über eine -(CH₂)-, -(CH₂)₂-oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, - C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂. -N(CH₃)(C₂H₅), -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, - C(=O)-NH₂, -C(=O)-NH-CH₃, -C(=O)-NH-C₂H₅, -C(=O)-N(CH₃)₂, - C(=O)-N(C₂H₅)₂, -O-Phenyl, -O-Benzyl, Phenyl und Benzyl substituiert sein kann, wobei jeweils der zyklische Teil der Reste -O-Phenyl, -0-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂. -C₁₋₅-Alkyl, -O-C₁₋₅-Alkyl, -O-CF₃, -S-CF₃, Phenyl und -O-Benzyl substituiert sein kann, stehen; oder
R¹² und R¹³ jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, (1,3,4,5)-Tetrahydropyrido[4,3-b]indolyl, (3,4)-Dihydro-1H-isochinolinyl, (1,3,4,9)-Tetrahydro-[b]-carbolinyl, Imidazolidinyl, (1,3)-Thiazolidinyl, Piperazinyl, Morpholinyl, Azepanyl, Diazepanyl und Thiomorpholinyl bilden, der ggf. jeweils mit 1, 2, 3, 4 oder 5 Substituenten unabhängig voneinander ausgewählt aus der Gruppe bestehend aus -CH₂-O-CH₂-Oxetanyl, -CH2-OH, -CH₂-CH₂-OH, =CH₂, -O-CH₂-Oxetanyl, (4,5)-Dihydroisoxazolyl, Thiazolyl, (1,2,5)-Thiadiazolyl, Thiophenyl, Phenethyl, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃. -CH₂-O-CH₂-CH₃, Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, - NO₂, -O-CF₃, -S-CF₃. -SH, -S-CH₃, -S-C₂H₅, -S-CH(CH₃)₂, -S-C(CH₃)₃,
Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂, - C(=O)-O-C(CH₃)₃, -NH-CH₃, -NH-C₂H₅, -NH-C(CH₃)₃, -N(CH₃)₂, - N(C₂H₅)₂, -N(CH₃)(C₂H₅), -NH-Phenyl, -N(CH₃)-Phenyl, -N(C₂H₅)-Phenyl, -N(C₂H₅)-Phenyl, -O-CH₂-CH₂-CH₂-CH₃, Piperidinyl, Pyrrolidinyl, Cyclohexyl, Cyclopentyl, -O-C(=O)-CH₃, -O-C(=O)-C₂H₅, - O-C(=O)-C(CH₃)₃, -(CH₂)-Pyridinyl, Pyridinyl, -O-Phenyl, -O-Benzyl, Phenyl und Benzyl substituiert sein, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N-[C(=O)-C₂H₅]-Phenyl, -N-[C(=O)-CH₃]-Phenyl, -NH-Phenyl, -N(CH₃)-Phenyl, -N(C₂H₅)-Phenyl, -(CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sek-Butyl, Isobutyl, tert-Butyl, -O-CH₃, -O-C₂H₅, -O-CH(CH₃)₂, -O-C(CH₃)₃, -O-CF₃, -S-CF₃, Phenyl und -O-Benzyl substituiert sein kann;
und
R²⁵ und R²⁶, unabhängig voneinander, jeweils für einen Wasserstoff-Rest;
für einen Alkyl-Rest ausgewählt aus der Gruppe bestehend aus Methyl, Ethyl, n-Propyl, Isopropyl, tert-Butyl, n-Butyl, sek-Butyl, Isobutyl, n-Pentyl, n-Hexyl und n-Heptyl;
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzoxazolyl, Benzisoxazolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl, wobei der Rest jeweils über eine -(CH₂)-, -(CH₂)₂-oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Methyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Cyclopropyl, Cyclobutyl, Cyclopentyl, Cyclohexyl, Cyclopentenyl und Cyclohexenyl stehen;
mit der Maßgabe, dass R²⁵ und R²⁶ nicht jeweils für einen Wasserstoff-Rest stehen;
oder
R²⁵ und R²⁶ jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl, Cyclopentenyl und Cyclohexenyl bilden;
wobei
sofern nicht anders angegeben, die vorstehend genannten Alkyl-, Alkenyl- und Alkinyl-Reste jeweils ggf. mit 1, 2, 3, 4, 5, 6, 7, 8 oder 9 Substituenten unabhängig voneinander ausgewählt aus der Gruppe bestehend aus -C(=O)-O-CH₃, -C(=O)-O-C₂H₅, -C(=O)-O-C(CH₃)₃, -O-C(=O)-CH₃, -O-C(=O)-C₂H₅, - O-C(=O)-CH(CH₃)₂, -O-C(=O)-C(CH₃)₃, -O-Phenyl, Phenyl, F, Cl, Br, I, -CN, - NO₂, -OH, -NH₂, -SH, -O-CH₃, -O-C₂H₅, -O-CH(CH₃)₂, -O-C(CH₃)₃, -S-CH₃, -S-C₂H₅, -S-CH(CH₃)₂, -S-C(CH₃)₃, -NH-CH₃, -NH-C₂H₅, -NH-C(CH₃)₃, -N(CH₃)₂, - N(C₂H₅)₂, -N(CH₃)(C₂H₅), -OCF₃ und -SCF₃ substituiert sein können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

9. Verbindungen gemäß einem oder mehreren der Ansprüche 1 bis 8, **dadurch gekennzeichnet, dass**
X für O steht;
Y für einen Alkyl-Rest ausgewählt aus der
Gruppe bestehend aus -CF₃, -CH₂-CF₃, Methyl, Ethyl, n-Propyl, Isopropyl, tert-Butyl, n-Butyl, sek-Butyl und Isobutyl steht;
n für 0, 1, 2, 3 oder 4 steht;
R¹, R², R³ und R⁴, unabhängig voneinander, jeweils für H; F; Cl; Br; I; -SF₅; -NO₂; -CN; -NH₂; -OH; -SH; -C(=O)-NH₂; -S(=O)₂-NH₂-, -C(=O)-NH-OH; -C(=O)-OH; -C(=O)-H; -S(=O)₂-OH; -NHR¹¹; -NR¹²R¹³; -OR¹⁴; -SR¹⁵; - S(=O)-R²⁴; -S(=O)₂-R²⁴ oder für einen Rest ausgewählt aus der Gruppe bestehend aus Methyl, -CF₃, -CCl₃, -CBr₃, -CHF₂, -CH₂F, -CF₂Cl, - CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, n-Butyl, sek-Butyl, Isobutyl und tert-Butyl stehen;
R⁵ für F; Cl; Br; I; -SF₅; -OR¹⁴; -SR¹⁵; -S(=O)-R^{2a}; -S(=O)₂-R²⁴;
für einen Rest ausgewählt aus der Gruppe bestehend aus Methyl, - CF₃, -CCl₃, -CBr₃, -CHF₂, -CH₂F, -CF₂Cl, -CCl₂F, -CH₂-CN, -CH₂-O-CH₃, -CH₂-O-CF₃, -CH₂-SF₃, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, -CH₂-CH₂-CN, n-Propyl, -CF₂-CF₂-CF₃, -CF(CF₃)₂, Isopropyl, -CH₂-CH₂₋CH₂-CN, -CH₂-O-CH₂-CH₃, -CH₂-CH₂-SF₃, -CH₂-CH₂-OCF₃, -CH(CH₃)(O-CH₃), - CH(CH₃)(S-CH₃), n-Butyl, -CF₂-CF₂-CF₂-CF₃, -CH₂-CH₂-CH₂-CH₂-CN, n-Butyl, sek-Butyl, Isobutyl, -C(CH₃)₂(CH₂OH), und tert-Butyl;
oder für einen Rest ausgewählt aus der Gruppe bestehend aus Cyclopropyl, Cyclobutyl, Cyclopentyl, Cyclohexyl, Cyclopentenyl und Cyctohexenyl, der ggf. jeweils mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl und n-Pentyl substituiert sein kann;
T für C-R⁶ und U für C-R⁷ und V für C-R⁹ und W für C-R¹⁰ steht;
R⁶ und R⁷, unabhängig voneinander, jeweils für H; F; Cl; Br; I; -SF₅; -NO₂; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; -SR¹⁵; - C(=O)-OR²²; -S(=O)-R²⁴; -S(=O)₂-R²⁴; für einen Rest ausgewählt aus der Gruppe bestehend aus -CH₂-OH, Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, - CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, n-Butyl, sek-Butyl, Isobutyl und tert-Butyl oder für einen Phenyl-Rest stehen, der ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl und n-Pentyl substituiert sein kann;
R⁹ für H; F; Cl; Br; I; -SF₅; -NO₂; -CN; -NH₂; -OH; -SH; -C(=O)-NH₂; -
S(=O)₂-NH₂; -C(=O)-NH-OH, -C(=O)-OH; -C(=O)-H; -S(=O)₂-OH; - NHR¹¹; -NR¹²R¹³; OR¹⁴; -SR¹⁵; -S(=O)-R²⁴; -S(=O)₂-R²⁴ oder für einen Rest ausgewählt aus der Gruppe bestehend aus Methyl, -CF₃, -CCl₃, - CBr₃, -CHF₂, -CH₂F, -CF₂Cl, -CCl₂F, Ethyl, -CF₂-CH₃, -CH₂-CF₃, -C₂F₅, - CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, n-Butyl, sek-Butyl, Isobutyl und tert-Butyl steht;
R¹⁰ für -SF₅; -NO₂; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹¹; -NR¹²R¹³; -OR¹⁴; SR¹⁵; -C(=O)-OR²²; -S(=O)-R²⁴; -S(=O)₂-R²⁴; -C(=NH)-NH₂; -C(=NH)-NH-R²⁷, -N=C(NH₂)₂; -N=C(NHR²⁸)(NHR²⁹);
für einen Rest ausgewählt aus der Gruppe bestehend aus Cyclopropyl, Cyclobutyl, Cyclopentyl, Cyclohexyl, Cyclohexenyl, Irnidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder - C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus-CN, -CH₂-N(CH₃)₂, -CH₂-N(C₂H₅)₂, - CH₂-NH-CH₃, -CH₂-NH-C₂H₅, -N-[C(-O)-C₂H₅]-Phenyl, -N-[C(=O)-CH₃]-Phenyl, Oxo (=O), Thioxo (=S), -OH, -O-CH₃, -O-C₂H₅, -O-CH(CH₃)₂, - O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, - C(=O)-C(CH₃)₃, -C(=O)-OH, -C(=O)-O-CH₃, -C(=O)-O-C₂H₅, -C(=O)-O-CH(CH₃)₂ und -C(=O)-O-C(CH₃)₃ substituiert sein kann;
oder für einen Rest ausgewählt aus der Gruppe bestehend aus (1,3)-Benzodioxolyl, (1,4)-Benzodioxanyl, Tetrazolyl, (2,3)-Dihydrothieno[3,4-b][1,4]dioxinyl, Benzo[b]furanyl, Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl, der jeweils über eine - (CH=CH)-, -C≡C-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl,- Br, I, -CN, - CF₃, -SF₅, -OH, -O-CH₃, -O-C₂H₅, -O-CH(CH₃)₂, -O-C(CH₃)₃, -NH₂, -NO₂, -O-CF₃, -S-CF₅, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -NH-C(=O)-O-CH₃, -NH-S(=O)₂-CH₃, -NH-S(=O₂)-C₂H₅, -NH-S(=O)₂-CH(CH₃)₂, - NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, - C(=O)-NH-C₂H₅, -C(=O)-N(CH₃)₂, -C(=O)-N(C₂H₅)₂, -O-Phenyl, -O-Benzyl, Phenyl und Benzyl substituiert sein kann, steht;
R¹¹, R¹², R¹³, R¹⁴, R¹⁵, R²², R²⁴, R²⁷, R²⁸ und R²⁹, unabhängig voneinander, jeweils
für einen Rest ausgewählt aus der Gruppe bestehend aus Methyl, -CF₃, -CCl₃, -CBr₃, -CHF₂, -CH₂F, -CF₂Cl, -CCl₂F, -CH₂-CN, -CH₂-O-CH₃, - CH₂-O-CF₃, -CH₂-SF₃, Ethyl, -CF₂-CH₃, -CH₂-CF₃, -C₂F₅, -CH₂-CCl₃, - CH₂-CBr₃, -CHF-CF₂Cl, -CF₂-CF₂Cl, -CFCl-CF₂Cl, -CH₂-CH₂-CN, n-Propyl, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), - CH(CH₃)(S-CH₃), n-Butyl, -CF₂-CF₂-CF₂-CF₃, -CH₂-CH₂-CH₂-CH₂-CN, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethyibutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus 2,3-Dihydro-1H-indenyl, Cyclopropyl, Oxetanyl, Cyclobutyl, Cyclopentyl, Cyclohexyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über eine -CH₂-O-, -CH₂-CH₂-O-, - CH₂-CH₂-O-CH₂-, -CH₂-CH(CH₃)-O-CH₂-, -(CH₂)-, -(CH₂)₂- oder-(CH₂)₃-Gruppe gebunden sein kann und/oder jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, - O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂ und -C(=O)-O-C(CH₃)₃ substituiert sein kann;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Thiazol Oxazolyl und Isoxazolyl, wobei der Rest jeweils über eine -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -NH-C(=O)-O-CH₃, -NH-C(=O)-O-C₂H₅, -NH-C(=O)-O-C(CH₃)₃, -C(=O)-H, - C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein kann, stehen;
oder
R¹² und R¹³ jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Azabicyclo[3.2.1]octyl, 6-Aza-bicyclo[3.3.1]heptyl, 8-Aza-blcyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, Piperazinyl, Morpholinyl, Azepanyl, Diazepanyl und Thiomorpholinyl bilden, der ggf. jeweils mit 1, 2, 3, 4 oder 5 Substituenten unabhängig voneinander ausgewählt aus der Gruppe bestehend aus -CH₂-O-CH₂-Oxetanyl. -CH₂-OH, -CH₂-CH₂-OH, =CH₂, -O-CH₂-Oxetanyl, (4,5)-Dihydroisoxazolyl, Thiazolyl, (1,2,5)-Thladiazolyl, Thiophenyl, Phenethyl, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, - NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂, - C(=O)-O-C(CH₃)₃, -NH-CH₃, -NH-C₂H₅, -NH-C(CH₃)₃, -N(CH₃)₂, - N(C₂H₅)₂, -N(CH₃)(C₂H₅), -NH-Phenyl, -N(CH₃)-Phenyl, -N(C₂H₅)-Phenyl, -N(C₂H₅)-Phenyl, -O-CH₂-CH₂-CH₂-CH₃, Cyclohexyl, Cyclopentyl, Piperidinyl, Pyrrolidinyl, -O-C(=O)-CH₃, -O-C(=O)-C₂H₅, - O-C(=O)-C(CH₃)₃, -(CH₂)-Pyridinyl, Pyridinyl, -O-Phenyl, -O-Benzyl, Phenyl und Benzyl substituiert sein, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N-[C(=O)-C₂H₅]-Phenyl, -N-[C(=O)-CH₃]-Phenyl, -NH-Phenyl, -N(CH₃)-Phenyl, -N(C₂H₅)-Phenyl, -(CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus -CF₃, F, Cl, Br, -OH, -CF₃, -SF₅, -CN, -NO₂, Methyl, Ethyl, n-Propyl, Isopropyl, n-Butyl, sek-Butyl, Isobutyl, tert-Butyl, -0-CH₃, -O-C₂H₅, -O-CH(CH₃)₂, -O-C(CH₃)₃, -O-CF₃, -S-CF₃, Phenyl und - O-Benzyl substituiert sein kann,
und
R²⁵ und R²⁶, unabhängig voneinander, jeweils für einen Wasserstoff-Rest;
für einen Alkyl-Rest ausgewählt aus der Gruppe bestehend aus -CH₂-OH, -CH₂-CH₂-OH, -CH₂-CH₂-CH₂-OH, -CH₂-CH₂-CH₂-CH₂-OH, Isopropyl, n-Butyl, sek-Butyl, Isobutyl, Methyl, Ethyl und n-Propyl;
für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl, der jeweils über eine -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl und n-Pentyl substituiert sein kann; oder für einen Rest ausgewählt aus der Gruppe bestehend aus Cyclopropyl, Cyclobutyl, Cyclopentyl und Cyclohexyl stehen;
mit der Maßgabe, dass R²⁵ und R²⁶ nicht jeweils für einen Wasserstoff-Rest stehen;
oder
R²⁵ und R²⁶ jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweits in Form entsprechender Salze, oder jeweils in Form entsprechender Solvate.

10. Verbindungen gemäß einem oder mehreren der Ansprüche 1 bis 9, **dadurch gekennzeichnet, dass**
X für O steht;
Y für einen Alkyl-Rest ausgewählt aus der
Gruppe bestehend aus -CF₃, -CH₂-CF₃, Methyl, Ethyl, n-Propyl, Isopropyl, tert-Butyl, n-Butyl, sek-Butyl und Isobutyl steht;
n für 0, 1 oder 2 steht;
R¹, R³ und R⁴, unabhängig voneinander, jeweils für H; F; Cl; Br; oder für einen
Rest ausgewählt aus der Gruppe bestehend aus Methyl, -CF₃, -CCl₃, -CBr₃, - CHF₂, -CH₂F, -CF₂Cl, -CCl₂F, Ethyl, -CF₂-CH₃, -CH₂-CF₃, -C₂F₅, -CH₂-CCl₃, - CH₂-CBr₃, -CHF-CF₂Cl, -CF₂-CF₂Cl und -CFCl-CF₂Cl stehen;
R² für F; Cl; Br; I oder für einen Rest ausgewählt aus der Gruppe
bestehend aus Methyl, -CF₃, -CCl₃, -CBr₃, -CHF₂, -CH₂F, -CF₂Cl, - CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, sek-Butyl, Isobutyl, tert-Butyl, -O-CH₃, -O-CF₃, -O-CCl₃, -O-CBr₃, -O-CHF₂, -O-CH₂F, -O-CF₂Cl, -O-CCl₂F, -O-C₂H₅, -O-CF₂-CH₃, - O-CH₂-CF₃, -O-C₂F₅, -O-CH₂-CCl₃, -O-CH₂-CBr₃, -O-CHF-CF₂Cl, -O-CF₂-CF₂Cl, -O-CFCl-CF₂Cl, -O-CH₂-CH₂-CH₃, -O-CF₂-CF₂-CF₃, -O-CF(CF₃)₂, -O-CH(CH₃)₂, -O-C(CH₃)₃, -S-CH₃, -S-CF₃, -S-CCl₃, -S-CBr₃, -S-CHF₂, -S-CH₂F, -S-CF₂Cl, -S-CCl₂F, -S-C₂H₅, -S-CF₂-CH₃, -S-CH₂-CF₃, -S-C₂F₅, -S-CH₂-CCl₃, -S-CH₂-CBr₃, -S-CHF-CF₂Cl, -S-CF₂-CF₂Cl, -S-CFCl-CF₂Cl, -S-CH₂-CH₂-CH₃, -S-CF₂-CF₂-CF₃, -S-CF(CF₃)₂, -S-CH(CH₃)₂ und -S-C(CH₃)₃ steht;
R⁵ für F; Cl; Br; I; -SF₅;
für einen Rest ausgewählt aus der Gruppe bestehend aus Methyl, Ethyl, -CF₃, -CCl₃, -CBr₃, -CHF₂, -CH₂F, -CF₂Cl, -CCl₂F, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, - CF₂-CF₂-CF₃, -CF(CF₃)₂, sek-Butyl, Isobutyl, -C(CH₃)₂(CH₂OH), tert-Butyl, -O-CF₃, -O-CCl₃, -O-CBr₃, -O-CHF₂, -O-CH₂F, -O-CF₂Cl, -O-CCl₂F, -O-CF₂-CH₃, -O-CH₂-CF₃, -O-C₂F₅, -O-CH₂-CCl₃, -O-CH₂-CBr₃, - O-CHF-CF₂Cl, -O-CF₂-CF₂Cl, -O-CFCl-CF₂Cl, -O-CF₂-CF₂-CF₃, -O-CF(CF₃)₂, -O-CH(CH₃)₂, -O-C(CH₃)₃, -S-CF₃, -S-CCl₃, -S-CBr₃, -S-CHF₂, -S-CH₂F, -S-CF₂Cl, -S-CCl₂F, -S-CF₂-CH₃, -S-CH₂-CF₃, -S-C₂F₅, -S-CH₂-CCl₃, -S-CH₂-CBr₃, -S-CHF-CF₂Cl, -S-CF₂-CF₂Cl, -S-CFCl-CF₂Cl, -S-CF₂-CF₂-CF₃, -S-CF(CF₃)₂, -S-CH(CH₃)₂, -S-C(CH₃)₃,-S(=O)₂-CF₃, -S(=O)₂-CCl₃, -S(=O)₂-CBr₃, -S(=O)₂-CHF₂, -S(=O)₂-CH₂F, -S(=O)₂-CF₂Cl, -S(=O)₂-CC)₂F, -S(=O)₂-CF₂-CH₃, -S(=O)₂-CH₂-CF₃, - S(=O)₂-C₂F₅, -S(=O)₂-CH₂-CCl₃, -S(=O)₂-CH₂-CBr₃, -S(=O)₂-CHF-CF₂Cl, -S(=O)₂-CF₂-CF₂Cl, -S(=O)₂-CFCl-CF₂Cl, -S(=O)₂-CF₂-CF₂-CF₃, -S(=O)₂-CF(CF₃)₂, -S(=O)₂-CH(CH₃)₂ und -S(=O)₂-C(CH₃)_{3;}
oder für einen Rest ausgewählt aus der Gruppe bestehend aus Cyclopropyl, Cyclobutyl, Cyclopentyl, Cyclohexyl, Cyclopentenyl und Cyclohexenyl steht;
T für C-R⁶ und U für C-R⁷ und V für C-R⁸ und W für C-R¹⁰ steht;
R⁶ und R⁷, unabhängig voneinander, jeweils für H; F; Cl; Br; I; -NO₂; -CN; - C(=O)-OCH₃; -C(=O)-OC₂H₅; für einen Rest ausgewählt aus der Gruppe bestehend aus -CH₂-OH, Methyl, -CF_{3,} -CCl₃, -CBr₃, -CHF₂, -CH₂F, -CF₂Cl, - CCl₂F, Ethyl, n-Propyl, Isopropyl, sek-Butyl, Isobutyl und tert-Butyl oder für einen Phenyl-Rest stehen, der ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, - NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl und n-Pentyl substituiert sein kann;
R⁹ für H; F; Cl; Br, I; -NO₂; -CN; oder für einen Rest ausgewählt aus der
Gruppe bestehend aus Methyl, -CF₃, -CCl₃, -CBr₃, -CHF₂, -CH₂F, - CF₂Cl, -CCl₂F, Ethyl n-Propyl, -CF₂-CF₂-CF₃, -CF(CF₃)₂, Isopropyl, sek-Butyl, Isobutyl und tert-Butyl steht;
R¹⁰ für -CN; -OH; -NH₂; -NO₂; -NHR¹¹; -NR¹² R¹³; -OR¹⁴; -SR¹⁵; -C(=O)-OR²²;
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C=C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)_{3,} -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo **(=O),** Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus (1,3)-Benzodioxolyl, (1,4)-Benzodioxanyl, Indolyl, Tetrazolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H_{5,} -NH₂, -N(CH₃)₂, -N(C₂H₅)₂, -NH-S(=O)₂-CH3, -NH-S(=O₂)-C₂H₅, - NH-S(=O)₂-CH(CH₃)₂, -NO_{2,} -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R¹¹, R¹², R¹³, R¹⁴, R¹⁵ und R²², unabhängig voneinander, jeweils
für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂₋O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus 2,3-Dihydro-1H-indenyl, Cyclopropyl, Oxetanyl, Cyclobutyl, Cyclopentyl, Cyclohexyl, Pyrrolidinyl, Piperidinyl, Morpholinyl, Piperazinyl, Azepanyl, Diazepanyl, Azocanyl und Thiomorpholinyl stehen, der jeweils über eine -CH₂-O-, - CH₂-CH₂-O-, -CH₂-CH₂-O-CH₂-, -CH₂-CH(CH₃)-O-CH₂-, -(CH₂)-,-(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Methyl, n-Propyl, Isopropyl, n-Butyl, sek-Butyl, -C(=O)-O-CH₃, -C(=O)-O-C₂H₅, -C(=O)-O-CH(CH₃)_{2,} -C(=O)-O-C(CH₃)₃ substituiert sein kann;
oder für einen Rest ausgewählt aus der Gruppe bestehend aus -(CH₂)-Pyridinyl, -(CH₂)₂-Pyridinyl, Benzyl, Phenethyl, Phenyl, Naphthyl, Oxazolyl, Triazolyl, Imidazolyl, Pyrimidinyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, stehen;
oder
R¹² und R¹³ jeweils zusammen mit dem sie verbindenden Stickstoffatom als Ringglied einen Rest ausgewählt aus der Gruppe bestehend aus 3-Aza-bicyclo[3.1.1]heptyl, &Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, Piperazinyl, Morpholinyl, Azepanyl, Diazepanyl und Thiomorpholinyl bilden, der ggf. jeweils mit 1, 2, 3, 4 oder 5 Substituenten unabhängig voneinander ausgewählt aus der Gruppe bestehend aus -CH₂-O-CH₂-Oxetanyl, -O-CH₂-Oxetanyl, -CH₂-OH, -CH₂-CH₂-OH, =CH₂, (4,5)-Dihydroisoxazolyl, Thiazolyl, (1,2,5)-Thiadiazolyl, Thiophenyl, Phenethyl, -CN, -CH₂-N(CH₃)₂, -CH₂-N(C₂H₅)₂, -CH₂-NH-CH₃, -CH₂-NH-C₂H₅, -N-[C(=O)C₂H₅]-Phenyl, -N-[C(=O)-CH₃]-Phenyl, -CH₂-O-CH₃, CH₂-O-CH₂-CH₃, Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek Butyl, Isobutyl, tert-Butyl, n-Pentyl, -C(=O)-CH₃, -C(=O)-C₂H₅, -C(=O)-CH(CH₃)_{2,} -C(=O)-C(CH₃)₃, -C(=O)-OH, -C(=O)-O-CH₃, -C(=O)-O-C₂H₅, -C(=O)-O-CH(CH₃)_{2,} -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -NH-Phenyl, -N(CH₃)-Phenyl, -N(C₂H₅)-Phenyl, -N(C₂H₅)-Phenyl, -O-CH₂-CH₂-CH₂-CH₃, Cyclohexyl, Cyclopentyl, Piperidinyl, Pyrrolidinyl, -O-C(=O)-CH_{3,} -0-C(=O)-C₂H₅, -O-C(=O)-C(CH₃)₃, -(CH₂)-Pyridinyl, Pyridinyl, -O-Phenyl, - O-Benzyl, Phenyl und Benzyl substituiert sein, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N-[C(=O)-C₂H₅]-Phenyl, - N-[C(=O)-CH₃]-Phenyl, -NH-Phenyl, -N(CH₃)-Phenyl, -N(C₂H₅)-Phenyl, - (CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus -CF₃, F, Cl, Br, -OH, -CF₃, -SF₅, -CN, - NO₂, Methyl, Ethyl, n-Propyl, Isopropyl, n-Butyl, sek-Butyl, Isobutyl, tert-Butyl, -O-CH₃, -O-C₂H₅, -O-CH(CH₃)₂, -O-C(CH₃)₃, -O-CF₃, -S-CF₃, Phenyl und -O-Benzyl substituiert sein kann;
und
R²⁵ und R²⁶, unabhängig voneinander, jeweils für einen Wasserstoff-Rest;
für einen Alkyl-Rest ausgewählt aus der Gruppe bestehend aus -CH₂-OH, -CH₂-CH₂-OH, -CH₂-CH₂-CH₂-OH, -CH₂-CH₂-CH₂-CH₂-OH, Isopropyl n-Butyl, sek-Butyl, Isobutyl, Methyl, Ethyl und n-Propyl oder für einen Rest ausgewählt aus der Gruppe bestehend aus Phenyl, Benzyl, Phenethyl, Cyclopropyl, Cyclobutyl, Cyclopentyl und Cyclohexyl stehen;
mit der Maßgabe, dass R²⁵ und R²⁶ nicht jeweils für einen Wasserstoff-Rest stehen;
oder
R²⁵ und R²⁶ jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
jeweils ggf. in Form eines ihrer reinen Stereoisomeren, insbesondere Enantiomeren oder Diastereomeren, ihrer Racemate oder in Form einer
Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

11. Verbindungen gemäß einem oder mehreren der Ansprüche 1 bis 10, **dadurch gekennzeichnet, dass**
X für O steht;
Y für einen Alkyl-Rest ausgewählt aus der
Gruppe bestehend aus -CF₃, -CH₂-CF₃, Methyl, Ethyl, n-Propyl, Isopropyl, tert-Butyl, n-Butyl, sek-Butyl und Isobutyl steht;
n für 1 steht;
R¹, R³ und R⁴ jeweils für H stehen;
R² für Methyl; -O-CH₃; F; Cl; Br oder I steht;
R⁵ für einen Rest ausgewählt aus der Gruppe bestehend aus Methyl, Ethyl, -CF₃, -CCl₃, -CBr₃, -CHF₂, -CH₂F, -CF₂Cl, -CCl₂F, -C(CH₃)₂(CH₂OH), tert-Butyl, -O-CF₃, -O-CCl₃, -O-CBr₃, -O-CHF₂, -O-CH₂F, -S-CF₃, -S-CCl₃, -S-CBr₃, -S-CHF₂ und -S-CH₂F steht;
T für CH und U für CH und V für CH und W für C-R¹⁰ steht;
R¹⁰ für-CN; -NH₂; -NO₂; -NHR¹¹; -NR¹²R¹³; -OR¹⁴; -SR¹⁵;
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine über eine -(CH=CH)-, - C≡C- oder -C≡CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(≡O)-CH₃, -C(=O)-C₂H_{5,} -C(≡O)-C(CH₃)_{3,} - CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Tetrazolyl, (1,3)-Benzodioxolyl, (1,4)-Benzodioxanyl, (2,3)-Dihydrothieno[3,4-b][1,4]dioxinyl, Benzo[b]furanyl, Indolyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂)-, -(CH₂)₂- oder-(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, -N(C₂H₅)₂, -NH-S(=O)₂-CH3, -NH-S(=O₂)-C₂H_{5,} -NH-S(=O)₂-CH(CH₃)₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R¹¹, R¹², R¹³, R¹⁴ und R¹⁵, unabhängig voneinander, jeweils
für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H_{5,} -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus 2,3-Dihydro-1H-indenyl, Oxetang Piperidinyl, Pyrrolidinyl, Cyclopropyl, Cyclobutyl, Cyclopentyl und Cyclohexyl stehen, der jeweils über eine -CH₂-O-, - CH₂-CH₂-O-, -CH₂-CH₂-O-CH₂-, -CH₂-CH(CH₃)-O-CH₂, -(CH₂)-, -(CH₂)₂-oder-(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, -(CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF3, F, Cl, Br, -O-CH₃, -O-C₂H₅, Methyl, Ethyl, n-Propyl, isopropyl, n-Butyl, sek-Butyl, Isobutyl und tert-Butyl substituiert sein kann, stehen;
oder
R¹² und R¹³ jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.6]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl,-CH₂-OH, -CH₂CH₂-OH, =CH₂, -C(=O)-O-CH₃, -C(=O)-O-C₂H₅, -C(=O)-O-C(CH₃)₃, -C(=O)-CH₃, -C(=O)-C₂H₅, - C(=O)-C(CH₃)₃, -CN, -CH₂-N(CH₃)₂, -CH₂-N(C₂H₅)₂, -CH₂-NH-CH₃, - CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, -NH₂, -NH-CH₃, -NH-C₂H₅, -N(CH₃)₂, - N(C₂H₅)₂, -NH-Phenyl, -N(CH₃)-Phenyl, -N(C₂H₅)-Phenyl, Oxo (=O), Thioxo (=S), -OH, F, Cl, Br, -CF₃, -O-CH₃, -O-C₂H₅, -O-C(CH₃)₃, -0-CH(CH₃)₂, -O-CH₂-CH₂₋CH₂-CH₃, Methyl, Ethyl, Isopropyl, n-Propyl, n-Butyl, tert-Butyl, sek-Butyl, Cyclohexyl, Cyclopentyl, Piperidinyl, Pyrrolidinyl, -O-Phenyl, -O-C(=O)-CH₃, -O-C(=O)-C₂H₅, -O-C(=O)-C(CH₃)₃, -(CH₂)-Pyridinyl, Pyridinyl, Phenyl und Benzyl substituiert sein kann, wobei jeweils der zyklische Teil der Reste Oxetanyl, -N-[C(=O)-C₂H₅]-Phenyl, -N-[C(=O)-CH₃]-Phenyl, -NH-Phenyl, -N(CH₃)-Phenyl, - N(C₂H₅)-Phenyl, -(CH₂)-Pyridinyl, Pyridinyl, Phenyl, -O-Phenyl und
Benzyl ggf. mit 1, 2, 3, 4 oder 5 Substituenten unabhängig voneinander ausgewählt aus der Gruppe bestehend aus -CF₃, -OH, -O-CH₃, -O-C₂H₅, F, Cl, Br, Methyl, Ethyl, Isopropyl, n-Propyl, n-Butyl, tert-Butyl und sek-Butyl substituiert sein kann;
und
R²⁵ für einen Alkyl-Rest ausgewählt aus der Gruppe bestehend aus -CH₂-OH, -CH₂-CH₂-OH, -CH₂-CH₂-CH₂-OH, -CH₂-CH₂-CH₂-CH₂-OH, Isopropyl, n-Butyl, sek-Butyl, Isobutyl, Methyl, Ethyl und n-Propyl oder für einen Rest ausgewählt aus der Gruppe bestehend aus Phenyl, Benzyl, Phenethyl, Cyclopropyl, Cyclobutyl, Cyclopentyl und Cyclohexyl steht;
R²⁶ für einen Wasserstoff-Rest oder für einen Rest ausgewählt aus der Gruppe bestehend aus Methyl, Ethyl und n-Propyl steht;
oder
R²⁵ und R²⁶ jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

12. Verbindungen gemäß einem oder mehreren der Ansprüche 1 bis 10, **dadurch gekennzeichnet, dass**
X für O steht;
n für 0, 1 oder 2 steht;
R¹, R³ und R⁴ jeweils für H stehen;
R² für F; Cl; Br; I oder für einen Rest ausgewählt aus der Gruppe
bestehend aus Methyl, -CF₃, -CCl₃, -CBr₃, -CHF₂, -CH₂F, -O-CH₃, -O-CF₃, -O-CCl₃, -O-CBr₃, -O-CHF₂, -O-CH₂F, -S-CH₃, -S-CF₃, -S-CCl₃, -S-CBr₃, -S-CHF₂, -S-CH₂F, -S-CF₂Cl und -S-CCl₂F steht;
R⁵ für F; Cl; Br; I; -SF₅;
für einen Rest ausgewählt aus der Gruppe bestehend aus Methyl, Ethyl, -CF₃, -CCl₃, -CBr₃, -CHF₂, -CH₂F, -CF₂Cl, -CCl₂F, -C(CH₃)₂(CH₂OH), tert-Butyl, -O-CF₃, -O-CCl₃, -O-CBr₃, -O-CHF₂, -O-CH₂F, -O-CF₂Cl, -O-CCl₂F, -O-CF₂-CH₃, -S-CF₃, -S-CCl₃, -S-CBr₃, -S-CHF₂, -S-CH₂F, -S-CF₂Cl, -S-CCl₂F, -S-CF₂-CH₃, -S(=O)₂-CF₃, -S(=O)₂-CCl₃, -S(=O)₂-CBr₃, -S(=O)₂-CHF₂, -S(=O)₂-CH₂F und -S(=O)₂-CF₂Cl:
oder für einen Rest ausgewählt aus der Gruppe bestehend aus Cyclopropyl, Cyclobutyl, Cyclopentyl, Cyclohexyl, Cyclopentenyl und Cyclohexenyl steht;
T für C-R⁶ und U für C-R⁷ und V für C-R⁹ und W für C-R¹⁰ steht;
R⁶ und R⁷ jeweils für-CF₃; Phenyl; -C(=O)-OCH₃; -C(=O)-OC₂H₅; Methyl; - CH₂-OH; H; F; Cl; Br und I stehen;
N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus (1,3)-Benzodioxolyl, (1,4)-Benzodioxanyl, Indolyl, Tetrazolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, -N(C₂H₅)₂, -NH-S(=O)₂-CH3, -NH-S(=O₂)-C₂H_{5,} - NH-S(=O)₂-CH(CH₃)₂, -NO₂, -O-CF₃, -S-CF_{3,} -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R⁹ für -CF₃; H; F; Cl; Br oder I steht;
R¹⁰ für -CN; -NH₂; -NO₂; -NHR¹¹; -NR¹²R¹³; -OR¹⁴; -SR¹⁵; -C(=O)-OR²²;
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus (1,3)-Benzodioxolyl, (1,4)-Benzodioxanyl, Indolyl, Tetrazolyl, (2,3)-Dihydrothieno[3,4-b][1,4]dioxinyl, Benzo[b]furanyl, Phenyl, Naphthyl, Oxazotyl, Thiazolyl, Imidazolyl, Pyrimidinyl, Thiophenyl, Furanyl und Pyridinyl, der jeweils über eine -(CH=CH)-, -C≡C-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃. -O-C₂H₅, -NH₂, -N(CH₃)₂, -N(C₂H₅)₂, -NH-S(=O)₂-CH₃, -NH-S(=O₂)-C₂H₅, - NH-S(=O)₂-CH(CH₃)₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R¹¹, R¹², R¹³, R¹⁴, R¹⁵ und R²², unabhängig voneinander, jeweils
für einen Rest ausgewählt aus der Gruppe bestehend aus Methyl, Ethyl, n-Propyl, Isopropyl, n-Butyl, sek-Butyl, Isobutyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl, 3-Pentenyl und tert-Butyl stehen;
für einen Rest ausgewählt aus der Gruppe bestehend aus 2,3-Dihydro-1H-indenyl, Piperidinyl, Pyrrolidinyl, Cyclopropyl, Oxetanyl, Cyclobutyl, Cyclopentyl und Cyclohexyl stehen, der jeweils über eine -CH₂-O-, - CH₂-CH₂-O-, -CH₂-CH₂-O-CH₂-, -CH₂-CH(CH₃)-O-CH₂-, -(CH₂)-, - (CH₂)₂- oder -(CH₂)₃-Gruppe gebunden sein kann undloder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=OFO-CH(CH₃)₂, - C(=O)-O-C(CH₃)₃ substituiert sein kann;
oder für einen Rest ausgewählt aus der Gruppe bestehend aus -(CH₂)-Pyridinyl, -(CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF3, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R¹² und R¹³ jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5.4-c]pyridinyl, Pyrrolidinyl, Piperidinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl, -CH₂-OH, - CH₂-CH₂-OH. =CH₂, (4,5)-Dihydroisoxazolyl, Thiazolyl, (1,2,5)-Thiadiazolyl, Thiophenyl, Phenethyl, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, - NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂, - C(=O)-O-C(CH₃)₃, -NH-CH₃, -NH-C₂H₅, -NH-C(CH₃)₃, -N(CH₃)₂, - N(C₂H₅)₂, -N(CH₃)(C₂H₅), -NH-Phenyl, -N(CH₃)-Phenyl, -N(C₂H₅)-Phenyl, -N(C₂H₅)-Phenyl, -O-CH₂-CH₂-CH₂-CH₃, Cyclohexyl, Cyclopentyl, Piperidinyl, Pyrrolidinyl, -O-C(=O)CH₃, -O-C(=O)-C₂H₅, - O-C(=O)-C(CH₃)₃, -(CH₂)-Pyridinyl, Pyridinyl, -O-Phenyl, -O-Benzyl, Phenyl und Benzyl substituiert sein, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N-[C(=O)-C₂H₅]-Pheny), -N-[C(=O)-CH₃]-Phenyl, -NH-Phenyl, -N(CH₃)-Phenyl, -N(C₂H₅)-Phenyl, -(CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus -CF₃, F, Cl, Br, -OH, -CF₃, -SF₅, -CN, -NO₂, Methyl, Ethyl, n-Propyl, Isopropyl, n-Butyl, sek-Butyl, Isobutyl, tert-Butyl, -O-CH₃, -O-C₂H₅, -O-CH(CH₃)₂, -O-C(CH₃)₃, -O-CF₃, -S-CF₃, Phenyl und - O-Benzyl substituiert sein kann;
und
R²⁵ und R²⁶, unabhängig voneinander, jeweils für einen Wasserstoff Rest;
für einen Alkyl-Rest ausgewählt aus der Gruppe bestehend aus -CH₂-OH, -CH₂-CH₂-OH, -CH₂-CH₂-CH₂-OH, -CH₂-CH₂-CH₂-CH₂-OH, Isopropyl, n-Butyl, sek-Butyl, Isobutyl, Methyl, Ethyl und n-Propyl oder für einen Rest ausgewählt aus der Gruppe bestehend aus Phenyl, Benzyl, Phenethyl, Cyclopropyl, Cyclobutyl, Cyclopentyl und Cyclohexyl stehen;
mit der Maßgabe, dass R²⁵ und R²⁶ nicht jeweils für einen Wasserstoff-Rest stehen;
oder
R²⁵ und R²⁶ jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

13. Verbindungen gemäß einem oder mehreren der Ansprüche 1 bis 12, **dadurch gekennzeichnet, dass**
X für O steht;
n für 1 steht;
R¹, R³ und R⁴ jeweils für H stehen;
R² für Methyl; -O-CH₃; F; Cl; Br oder I steht;
R⁵ für einen Rest ausgewählt aus der Gruppe bestehend aus Methyl, Ethyl, -CF₃, -CCl₃, -CBr₃, -CHF₂, -CH₂F, -CF₂Cl, -CCl₂F, -C(CH₃)₂(CH₂OH), tert-Butyl, -O-CF₃, -O-CCl₃, -O-CBr₃, -O-CHF₂, -O-CH₂F, -S-CF₃, -S-CCl₃, -S-CBr₃, -S-CHF₂ und -S-CH₂F steht; oder für einen Rest ausgewählt aus der Gruppe bestehend aus Cyclopropyl, Cyctobutyl, Cyclopentyl, Cyclohexyl, Cyclopentenyl und Cyclohexenyl steht;
T für CH und U für CH und V für CH und W für C-R¹⁰ steht;
R¹⁰ für-CN; -NH₂; -NO₂; -NHR¹¹; -NR¹²R¹³; -OR¹⁴; -SR¹⁵;
für einen Rest ausgewählt aus der Gruppe bestehend aus Cyclopropyl, Cyclobutyl, Cyclopentyl, Cyclohexyl, Cyclohexenyl, Pyrrolidin Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl,
Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Tetrazolyl, (1,3)-Benzodioxolyl, (1,4)-Benzodioxanyl, Indolyl, (2,3)-Dihydrothieno[3,4-b][1,4]dloxinyl, Benzo[b]furanyl, Phenyl, Naphthyl, Oxazol Thiazolyl, Imidazolyi, Pyrimidinyl, Thiophenyl, Furanyl und Pyridinyl, der jeweils über eine -(CH=CH)-, -C≡C-, -(CH₂)-. -(CH₂)₂**-** oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, -N(C₂H₅)₂, -NH-S(=O)₂-CH₃, -NH-S(=O₂)-C₂H₅, - NH-S(=O)₂-CH(CH₃)₂, -NO₂, -O-CF₃, -S-CF₃, -SH, -S-CH₃. -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R¹¹, R¹², R¹³, R¹⁴ und R¹⁵, unabhängig voneinander, jeweils
für einen Rest ausgewählt aus der Gruppe bestehend aus Methyl, Ethyl, n-Propyi, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbuty), -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus 2,3-Dihydro-1 H-indenyl, Pipecidinyl, Pyrrolidinyl, Cyclopropyl, Oxetanyl, Cyclobutyl, Cyclopentyl und Cyclohexyl stehen, der jeweils über eine -CH₂-O-, - CH₂-CH₂-O-, -CH₂-CH₂-O-CH₂-, -CH₂-CH(CH₃)-O-CH₂-, -(CH₂)-, - (CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, - C(=O)-O-C(CH₃)₃ substituiert sein kann;
oder für einen Rest ausgewählt aus der Gruppe bestehend aus -(CH₂)-Pyridinyl, -(CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF3, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R¹² und R¹³ jeweils zusammen mit dem sie verbindenden Stickstoffatom als Ringglied einen Rest ausgewählt aus der Gruppe bestehend aus 3-Aza-bicyclo[3.1.i]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)- Tetra hydropyrid i nyl , (4,5,6.7)-Tetrahydfoisoxazo!o[5,4-c]pyridinyl, Pyrrolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH2-O-CHr Oxetan -O-CH₂-0xetanyl, -CH₂-OH, -CH₂-CH₂-OH, =CH₂, -C(=O)-O-Cl-l3, -C(=O)-O-C₂H₅, -C(=O)-O-C(CH₃)₃, -C(=O)-CH₃, -C(=O)-C₂Hs, - C(=O)-C(CH₃)₃, -CN, -CH₂-N(CH₃)₂, -CH₂-N(C₂H₅)₂, -CH₂-NH-CH₃, - CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, -NH₂, -NH-CH₃, -NH-C₂H₅, -N(CH₃)₂, - N(C₂H₅)₂, -NH-Phenyl, -N(CH₃)-Phenyl, -N(C₂H₅)-Phenyl, Oxo (=O), Thioxo (=S), -OH, F, Cl, Br, -CF₃, -O-CH₃, -O-C₂H₅, -O-C(CH₃)₃, -O-CH(CH₃)₂, -O-CH₂-CH₂-CH₂-CH₃, Methyl, Ethyl, Isopropyl, n-Propyl, n-Butyl, tert-Butyl, sek-Butyl, Cyclohexyl, Cyclopentyl, Piperidinyl, Pyrrolidinyl, -O-Phenyl, -O-C(=O)-CH₃, -O-C(=O)-C₂H₅, -O-C(=O)-C(CH₃)₃, -(CH₂)-Pyridinyl, (4,5)-Dihydroisoxazolyl, Thiazolyl, (1,2,5)-Thiadiazolyl, Thiophenyl, Phenethyl, Pyridine Phenyl und Benzyl substituiert sein kann, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N-[C(=O)-C₂H₅]-Phenyl, -N-[C(=O)-CH₃]-Phenyl, -NH-Phenyl, -N(CH₃)-Phenyl, -N(C₂H₅)-Phenyl, -(CH₂)-Pyridinyl, Pyridinyl, Phenyl, -O-Phenyl und Benzyl ggf. mit 1, 2, 3, 4 oder 5 Substituenten unabhängig voneinander ausgewählt aus der Gruppe bestehend aus -CF₃, -OH, -O-CH₃, -O-C₂H₅, F, Cl, Br, Methyl, Ethyl, Isopropyl, n-Propyl, n-Butyl, tert-Butyl und sek-Butyl substituiert sein kann;
und
R²⁵ für einen Alkyl-Rest ausgewählt aus der Gruppe bestehend aus -CH₂-OH, -CH₂-CH₂-OH. Methyl, Ethyl und n-Propyl oder für einen Rest ausgewählt aus der Gruppe bestehend aus Benzyl, Phenyl, Phenethyl, Cyclopropyl, Cyclobutyl, Cyclopentyl und Cyclohexyl steht; R²⁶ für einen Wasserstoff-Rest oder für einen Rest ausgewählt aus der Gruppe bestehend aus Methyl, Ethyl und n-Propyl steht;
oder
R²⁵ und R²⁶ jeweils zusammen mit dem sie verbindenden Kohlenstoffatom als Ringglied einen Rest ausgewählt aus der Gruppe bestehend aus Cyclopropyl, Cyclobutyl, Cyclopentyl und Cyclohexyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

14. Verbindungen der allgemeinen Formel Id1 gemäß einem oder mehreren der
Ansprüche 1 bis 12, 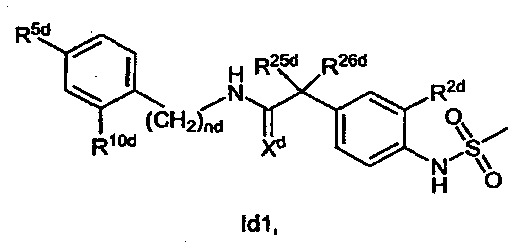 worin
X^{d} für O steht;
nd für 0, 1 oder 2 steht;
R^{2d} für F; Cl; Br, I oder für einen Rest ausgewählt aus der Gruppe
bestehend aus Methyl, -CF₃, -CCl₃, -CBr₃, -CHF₂, -CH₂F, -O-CH₃, -O-CF₃. -O-CCl₃, -O-CBr₃, -O-CHF₂, -O-CH₂F, -S-CH₃, -S-CF₃, -S-CCl₃, -S-CBr₃, -S-CHF₂, -S-CH₂F, -S-CF₂Cl und -S-CCl₂F steht;
R^{5d} für F; Cl, Br, I; -SF₅;
für einen Rest ausgewählt aus der Gruppe bestehend aus Methyl, Ethyl, -CF₃, -CCl₃, -CBr₃, -CHF₂, -CH₂F, -CF₂Cl, -CCl₂F, -C(CH₃)₂-(CH₂OH), tert-Butyl, -O-CF₃, -O-CCl₃, -O-CBr₃, -O-CHF₂, -O-CH₂F, -O-CF₂Cl, -O-CCl₂F, -O-CF₂-CH₃, -S-CF₃, -S-CCl₃, -S-CBr₃, -S-CHF₂, -S-CH₂F, -S-CF₂Cl, -S-CCl₂F, -S-CF₂-CH₃, -S(=O)₂-CF₃, -S(=O)₂-CCl₃, -S(=O)₂-CBr₃, -S(=O)₂-CHF₂, -S(=O)₂-CH₂F und -S(=O)₂-CF₂Cl;
oder für einen Rest ausgewählt aus der Gruppe bestehend aus Cyclopropyl, Cyclobutyl, Cyclopentyl, Cyclohexyl, Cyclopentenyl und Cyclohexenyl steht;
R^{10d} für -CN; -OH; -NH₂; -NO₂; -NHR^{11d}; -NR^{12d}R^{13d}; -OR^{14d}; -SR^{15d}; -C(=O)-OR^{22d};
für einen Rest ausgewählt aus der Gruppe bestehend aus Cyclopropyl, Cyclobutyl, Cyclopentyl, Cyclohexyl, Cyclohexenyl Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-. -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Tetrazolyl, (1,3)-Benzodioxolyl, (1,4)-Benzodioxanyl, (2,3)-Dihydrothieno[3,4-b][1,4]dioxinyl, Benzo[b]furanyl, Indolyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C, -(CH₂)-. -(CH₂)₂- oder -(CH₂)₃-Gruppe gebunden sein kann und/oder jeweils ggf.
mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, -N(C₂H₅)₂, -NH-S(=O)₂-CH₃, -NH-S(=O₂)-C₂H₅, -NH-S(=O)₂-CH(CH₃)₂, -NO₂, -O-CF₃, -S-CF₃, SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R^{11d}, R^{12d}, R^{13d}, R^{14d}, R^{15d} und R^{22d}, unabhängig voneinander, jeweils
für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus 2,3-Dihydro-1H-indenyl, Piperidinyl, Pyrrolidino Cyclopropyl, Cyclobutyl, Oxetanyl, Cyclopentyl und Cyclohexyl stehen, der jeweils über eine -CH₂-O-, - CH₂-CH₂-O-, -CH₂-CH₂-O-CH₂-, -CH₂-CH(CH₃)-O-CH₂-, -(CH₂)-, - (CH₂)₂- oder-(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, - C(=O)-O-C(CH₃)₃ substituiert sein kann;
oder für einen Rest ausgewählt aus der Gruppe bestehend aus -(CH₂)-Pyridinyl, -(CH₂)₂-Pyridinyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF3, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R^{12d} und R^{13d} jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl, -CH₂-OH, -CH₂-CH₂-OH, =CH₂, -C(=O)-O-CH₃, - C(=O)-O-C₂H₅, -C(=O)-O-C(CH₃)₃, -C(=O)-CH₃, -C(=O)C₄H₅, -C(=O)-C(CH₃)₃, -CN, -CH₂-N(CH₃)₂, -CH₂-N(C₂H₅)₂, -CH₂-NH-CH₃, -CH₂-NH-C₂H₃, -N-[C(=O)-C₂H₅]-Phenyl, -N-[C(=O)-CH₃]-Phenyl, -CH₂-O-CH₃, -CH₂-O-CH₂-CH₃, -NH₂, - NH-CH₃, -NH-C₂H₅, -N(CH₃)₂, -N(C₂H₅)₂, -NH-Phenyl, -N(CH₃)-Phenyl, - N(C₂H₅)-Phenyl, Oxo (=O), Thioxo (=S), -OH, F, Cl, Br, -CF₃, -O-CH₃, -O-C₂H₅, -C₄C(CH₃)₃, -O-CH(CH₃)₂, -O-CH₂-CH₂-CH₂-CH₃, Methyl, Ethyl,
Isopropyl, n-Propyl, n-Butyl, tert-Butyt sek-Butyl, Cyclohexyl, Cyclopentyl, (4,5)-Dihydroisoxazolyl, Thiazolyl, (1,2,5)-Thiadiazolyl, Thiophenyl, Phenethyl, Piperidinyl, Pyrrolidinyl, -O-Phenyl, -O-C(=O)-CH₃, -O-C(=O)-C₂H₅, -O-C(=O)-C(CH₃)₃, -(CH₂)-Pyridinyl, Pyridinyl, Phenyl und Benzyl substituiert sein kann, wobei jeweils der zyklische Teil der Reste Oxetanyl, -N-[C(=O)-C₂H₅]-Phenyl, - N-[C(=O)CH₃]-Phenyl, (4,5)-Dihydroisoxazolyl, Thiazolyl, (1,2,5)-Thiadiazolyl, Thiophenyl, Phenethyl, -NH-Phenyl, -N(CH₃)-Phenyl, -N(C₂H₅)-Phenyl, -(CH₂)-Pyridinyl, Pyridinyl, Phenyl, -O-Phenyl und Benzyl ggf. mit 1, 2, 3, 4 oder 5 Substituenten unabhängig voneinander ausgewählt aus der Gruppe
bestehend aus -CF₃, -OH, -O-CH₃, -O-C₂H₅, F, Cl, Br, Methyl, Ethyl, Isopropyl, n-Propyl, n-Butyl, tert-Butyl und sek-Butyl substituiert sein kann;
R^{25d} und R^{26d}, unabhängig voneinander, jeweils für einen Wasserstoff-Rest;
für einen Alkyl-Rest ausgewählt aus der Gruppe bestehend aus -CH₂-OH, -CH₂-CH₂-OH, -CH₂-CH₂-CH₂-OH, -CH₂-CH₂-CH,-CH₂-OH, Isopropyl, n-Butyl, sek-Butyl, Isobutyl, Methyl, Ethyl und n-Propyl;
mit der Maßgabe, dass R^{25d} und R^{26d} nicht jeweils für einen Wasserstoff-Rest stehen;
oder
R^{25d} und R^{26d} jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

15. Verbindungen der allgemeinen Formel Id gemäß Ansprüch 14, 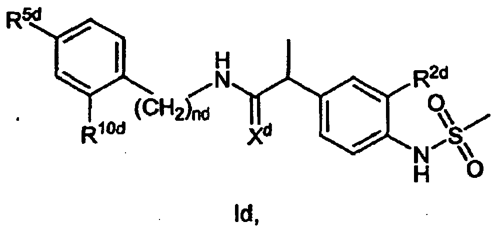 worin X^{d}, nd, R^{2d}, R^{5d} und R^{10d} wie in Anspruch 25 definiert sind;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

16. Verbindungen der allgemeinen Formel Ie1 gemäß einem oder mehreren der
Ansprüche 1 bis 12, 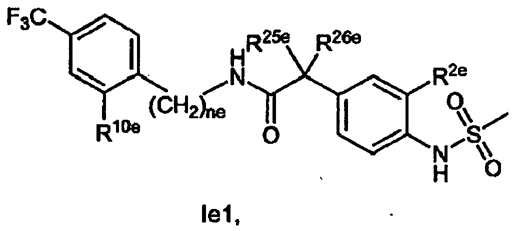 worin
ne für 0, 1 oder 2 steht;
R^{2e} für Methyl; -O-CH₃; F; Cl; Br oder I steht;
R^{10e} für -CN; -OH; -NH₂; -NO₂; -NHR^{11e}; -NR^{12e}R^{13e}; -OR^{14e}; -SR^{15e};
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Tetrazolyl, (1,3)-Benzodioxolyl, (1,4)-Benzodioxanyl, Indolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C-, -(CH₂), -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, -N(C₂H₅)₂, -NH-S(=O)₂-CH₃, -NH-S(=O₂)-C₂H₅, - NH-S(=O)₂-CH(CH₃)₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R^{11e}, R^{12e}, R^{13e}, R^{14e} und R^{15e}, unabhängig voneinander, jeweils
für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus Oxetanyl, 2,3-Dihydro-1H-indenyl, Piperidinyl, Pyrrolidinyl, Cyclopropyl, Cyclobutyl, Cyclopentyl und Cyclohexyl stehen, der jeweils über eine - CH₂-O-, -CH₂-CH₂-O-, -CH₂-CH₂-O-CH₂-, -CH₂-CH(CH₃)-O-CH₂-, - (CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, -(CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF3, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R^{12e} und R^{13e} jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-blcyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl, -CH₂-OH, -CH₂-CH₂-OH, =CH₂, -C(=O)-O-CH₃, - C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, -NH₂, - NH-CH₃, -NH-C₂H₅, -N(CH₃)₂, -N(C₂H₅)₂, -NH-Phenyl, -N(CH₃)-Phenyl, - N(C₂H₅)-Phenyl, Oxo (=O), Thioxo (=S), -OH, F, Cl, Br, -CF₃, -O-CH₃, -0-C₂H₅, -O-C(CH₃)₃, -O-CH(CH₃)₂, -O-CH₂-CH₂-CH₂-CH₃, Methyl, Ethyl,
Isopropyl, n-Propyl, n-Butyl, tert-Butyl, sek-Butyl, Cyclohexyl, Cyclopentyl, (4,5)-Dihydroisoxazolyl, Thiazolyl, (1,2,5)-Thiadiazolyl, Thiophenyl Phenethyl,
Piperidinyl, Pyrrolidinyl, -O-Phenyl, -O-C(=O)-CH₃, -O-C(=O)-C₂H₅, -O-C(=O)-C(CH₃)₃, -(CH₂)-Pyndinyl, Pyridinyl, Phenyl und Benzyl substituiert sein kann, wobei jeweils der zyklische Teil der Reste Oxetanyl, -N-[C(=O)-C₂H₅]-Phenyl, - N-[C(=O)-CH₃]-Phenyl, (4,5)-Dihydroisoxazolyl, Thiazolyl, (1,2,5)-Thiadiazolyl, Thiophenol, Phenethyl, -NH-Phenyl, -N(CH₃)-Phenyl, -N(C₂H₅)-Phenyl, -(CH₂)-Pyridinyl, Pyridinyl, Phenyl, -O-Phenyl und Benzyl ggf. mit 1, 2, 3, 4 oder 5 Substituenten unabhängig voneinander ausgewählt aus der Gruppe bestehend aus -CF₃, -OH, -O-CH₃, -O-C₂H₅, F, Cl, Br, Methyl, Ethyl, Isopropyl, n-Propyl, n-Butyl, tert-Butyl und sek-Butyl substituiert sein kann;
R^{25e} und R^{26e}, unabhängig voneinander, jeweils für einen Wasserstoff-Rest;
für einen Alkyl-Rest ausgewählt aus der Gruppe bestehend aus -CH₂-OH, -CH₂-CH₂-OH, -CH₂-CH₂-CH₂-OH, -CH₂-CH₂-CH₂-CH₂-OH, Isopropyl, n-Butyl, sek-Butyl, Isobutyl, Methyl, Ethyl und n-Propyl;
mit der Maßgabe, dass R^{25e} und R^{26e} nicht jeweils für einen Wasserstoff-Rest stehen;
oder
R^{25e} und R^{26e} jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

17. Verbindungen der allgemeinen Formel Ie gemäß Anspruch 16, 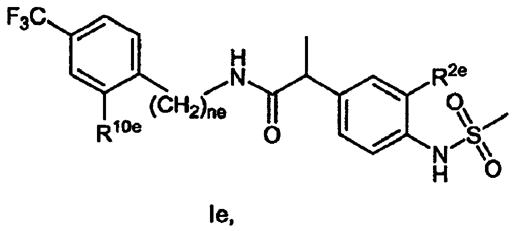 worin
ne, R^{10e} und R^{2e} wie in Anspruch 27 definiert sind,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

18. Verbindungen gemäß Anspruch 17, **dadurch gekennzeichnet, dass** ne für 1 steht;
R^{2e} für F steht;
R^{10e} für -CN; -OH; -NH₂; -NO₂; -NHR^{11e}; -NR^{12e}R^{13e}; -OR^{14e}; -SR¹⁵⁰;
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)O-C₂H₅, -C(=O)-O-C(CH₃)₃. -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Tetrazolyl, (1,3)-Benzodioxolyl, (1,4)-Benzodioxanyl, Indolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH)-, -C≡C, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, -N(C₂H₅)₂, -NH-S(=O)₂-CH₃, -NH-S(=O₂)-C₂H₅, - NH-S(=O)₂-CH(CH₃)₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R^{11e}, R^{12e}, R^{13e}, R^{14e} und R^{15e}, unabhängig voneinander, jeweils
für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus Oxetanyl, 2,3-Dihydro-1H-indenyl, Piperidinyl, Pyrrolidinyl, Cyclopropyl, Cyclobutyl, Cyclopentyl und Cyclohexyl stehen, der jeweils über eine -
CH₂-O-, -CH₂-CH₂-O-, -CH₂-CH₂-O-CH₂-, -CH₂-CH(CH₃)-O-CH₂-, - (CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, -(CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF3, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R^{12e} und R^{13e} jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl, -CH₂-OH, -CH₂-CH₂-OH, =CH₂, -C(=O)-O-CH₃, - C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, -NH₂, - NH-CH₃, -NH-C₂H₅, -N(CH₃)₂, -N(C₂H₅)₂, -NH-Phenyl, -N(CH₃)-Phenyl, - N(C₂H₅)-Phenyl, Oxo (=O), Thioxo (=S), -OH, F, Cl, Br, -CF₃, -O-CH₃, -O-C₂H₅, -O-C(CH₃)₃, -O-CH(CH₃)₂, -O-CH₂-CH₂-CH₂-CH₃, Methyl, Ethyl, Isopropyl, n-Propyl, n-Butyl, tert-Butyl, sek-Butyl, Cyclohexyl, Cyclopentyl, (4,5)-Dihydroisoxazolyl, Thiazolyl, (1,2,5)-Thiadiazolyl, Thiophenyl, Phenethyl, Piperidinyl, Pyrrolidinyl, -O-Phenyl, -O-C(=O)-CH₃, -O-C(=O)-C₂H₅, -O-C(=O)-C(CH₃)₃, -(CH₂)-Pyridinyl, Pyridinyl, Phenyl und Benzyl substituiert sein kann, wobei jeweils der zyklische Teil der Reste Oxetanyl, N-[C(=O)-C₂H₅]-Phenyl - N-[C(=O)-CH₃]-Phenyl, (4,5)-Dihydroisoxazolyl, Thiazol (1,2,5)-Thiadiazolyl, Thiophenyl, Phenethyl, -NH-Phenyl, -N(CH₃)-Phenyl, -N(C₂H₅)-Phenyl, -(CH₂)-Pyridinyl, Pyridinyl, Phenyl, -O-Phenyl und Benzyl ggf. mit 1, 2, 3, 4 oder 5 Substituenten unabhängig voneinander ausgewählt aus der Gruppe bestehend aus -CF₃, -OH, -O-CH₃, -O-C₂H₅, F, Cl, Br, Methyl, Ethyl, Isopropyl, n-Propyl, n-Butyl, tert-Butyl und sek-Butyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

19. Verbindungen der allgemeinen Formel If1 gemäß einem oder mehreren der
Ansprüche 1 bis 12, 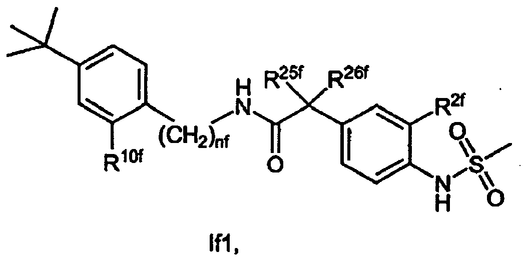 worin
nf für 0, 1 oder 2 steht;
R^{2f} für Methyl; -O-CH₃, F; Cl; Br oder I steht;
R^{10f} für -CN; -OH; -NH₂; -NO₂; -NHR^{11f}; -NR^{12f}R^{13f}; -OR^{14f}; -SR^{15f};
für einen Rest ausgewählt aus der Gruppe bestehend aus Cyclopropyl, Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus (1,3)-Benzodioxolyl, (1,4)-Benzodioxanyl, Indolyl, Tetrazolyl, (2,3)-Dihydrothieno[3,4-b][1,4]dioxinyl, Benzo[b]furanyl, Phenyl, Naphthyl, Oxazolyl, Thiazolyl, Imidazolyl, Pyrimidinyl, Thiophenyl, Furanyl und Pyridinyl, der jeweils über eine -(CH=CH), -C≡C-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -N(CH₃)₂, -N(C₂H₅)₂, -NH-S(=O)₂-CH₃, -NH-S(=O₂)-C₂H₅, - NH-S(=O)₂-CH(CH₃)₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R^{11f}, R^{12f}, R^{13f}, R^{14f} und R^{15f}, unabhängig voneinander, jeweils
für einen Rest ausgewählt aus der Gruppe bestehend aus Methyl, Ethyl, n-Propyl, Isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus Oxetanyl, 2,3-Dihydro-1H-indenyl, Piperidinyl, Pyrrolidinyl, Cyclopropyl, Cyclobutyl, Cyclopentyl und Cyclohexyl stehen, der jeweils über eine - CH₂-O-, -CH₂-CH₂-O-, -CH₂-CH₂-O-CH₂-, -CH₂-CH(CH₃)-O-CH₂-, - (CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, -(CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF3, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R^{12f} und R^{13f} jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl, -CH₂-OH, -CH₂-CH₂-OH, =CH₂, -C(=O)-O-CH₃, - C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, -NH₂, - NH-CH₃, -NH-C₂H₅, -N(CH₃)₂, -N(C₂H₅)₂, -NH-Phenyl, -N(CH₃)-Phenyl, - N(C₂H₅)-Phenyl, Oxo (=O), Thioxo (=S), -OH, F, Cl, Br, -CF₃, -O-CH₃, -O-C₂H₅, -O-C(CH₃)₃, -O-CH(CH₃)₂, -O-CH₂-CH₂-CH₂-CH₃, Methyl, Ethyl,
Isopropyl, n-Propyl, n-Butyl, tert-Butyl, sek-Butyl, Cyclohexyl, Cyclopentyl, (4,5)-Dihydroisoxazolyl, Thiazolyl, (1,2,5)-Thiadiazolyl, Thiophenyl, Phenethyl, Piperidinyl, Pyrrolidinyl, -O-Phenyl, -O-C(=O)-CH₃, -O-C(=O)-C₂H₅, -O-C(=O)-C(CH₃)₃, -(CH₂)-Pyridinyl, Pyridinyl, Phenyl und Benzyl substituiert sein kann, wobei jeweils der zyklische Teil der Reste Oxetanyl, -N-[C(=O)-C₂H₅]-Phenyl, - N-[C(=O)-CH₃]-Phenyl, (4,5)-Dihydroisoxazolyl, Thiazolyl, (1,2,5)-Thiadiazolyl, Thiophenyl, Phenethyl, -NH-Phenyl, -N(CH₃)-Phenyl, -N(C₂H₅)-Phenyl, -(CH₂)-Pyridinyl, Pyridinyl, Phenyl, -O-Phenyl und Benzyl ggf. mit 1, 2, 3, 4 oder 5 Substituenten unabhängig voneinander ausgewählt aus der Gruppe bestehend aus -CF₃, -OH, -O-CH₃, -O-C₂H₅, F, Cl, Br, Methyl, Ethyl, Isopropyl, n-Propyl, n-Butyl, tert-Butyl und sek-Butyl substituiert sein kann;
R^{25f} und R^{26f}, unabhängig voneinander, jeweils für einen Wasserstoff-Rest;
für einen Alkyl-Rest ausgewählt aus der Gruppe bestehend aus -CH₂-OH, -CH₂-CH₂-OH, -CH₂-CH₂-CH₂-OH, -CH₂-CH₂-CH₂-CH₂-OH, Isopropyl, n-Butyl, sek-Butyl, Isobutyl, Methyl, Ethyl und n-Propyl;
mit der Maßgabe, dass R^{25f} und R^{26f} nicht jeweils für einen Wasserstoff-Rest stehen;
oder
R^{25f} und R^{26f} jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

20. Verbindungen der allgemeinen Formel If gemäß Anspruch 19, 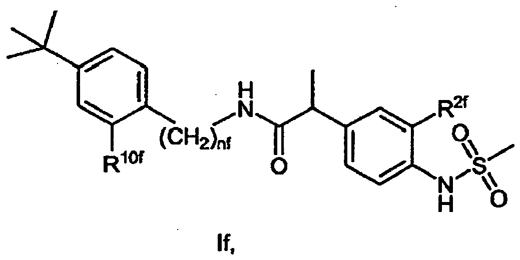 worin
nf, R^{10f} und R^{2f} wie in Anspruch 30 definiert sind;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

21. Verbindungen gemäß Anspruch 20, **dadurch gekennzeichnet, dass** nf für 1 steht;
R^{2f} für F steht;
R^{10f} für -CN; -OH; -NH₂; -NO₂; -NHR^{11f}; -NR^{12f}R^{13f}; -OR^{14f}; -SR^{15f};
für einen Rest ausgewählt aus der Gruppe bestehend aus Cyclopropyl,
Cyclobutyl, Cyclopentyl, Cyclohexyl, Cyclohexenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C=C- oder -C≡C-CH₂-Gruppe an das Grundgerüst gebunden ist und ggf. jeweils mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), Methyl, Ethyl, n-Propyl, lsopropyl, n-Butyl, sek-Butyl, Isobutyl, tert-Butyl und n-Pentyl substituiert sein kann;
oder für einen Rest ausgewählt aus der Gruppe bestehend aus (1,3)-Benzodioxolyl, (1,4)-Benzodioxanyl, Indolyl, Tetrazolyl, (2,3)-Dihydrothieno[3,4-b][1,4]dioxinyl, Benzo[b]furanyl, Phenyl, Naphthyl, Oxazotyl, Thiazolyl, Imidazolyl, Pyrimidinyl, Thiophenyl, Furanyl und Pyridinyl, der jeweils über eine -(CH=CH)-, -C≡C-, -(CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, **-**O-C₂H₅, -NH₂, -N(CH₃)₂, -N(C₂H₅)₂, -NH-S(=O)₂-CH₃, -NH-S(=O₂)C₂H₅, - NH-S(=O)₂-CH(CH₃)₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl und tert-Butyl substituiert sein kann, steht;
R^{11f}, R^{12f}, R^{13f}, R^{14f} und R^{15f}, unabhängig voneinander, jeweils
für einen Rest ausgewählt aus der Gruppe bestehend aus Methyl, Ethyl, n-Propyl, isopropyl, n-Butyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-O-Phenyl, -CH₂-CH₂-CH₂-O-CH₃, Ethenyl, Propenyl, 2-Butenyl, 3-Butenyl, 2-Pentenyl und 3-Pentenyl
stehen;
für einen Rest ausgewählt aus der Gruppe bestehend aus Oxetanyl, 2,3-Dihydro-1H-indenyl, Piperidinyl, Pyrrolidinyl, Cyclopropyl, Cyclobutyl, Cyclopentyl und Cyclohexyl stehen, der jeweils über eine - CH₂-O-, -CH₂-CH₂-O-, -CH₂-CH₂-O-CH₂-, -CH₂-CH(CH₃)-O-CH₂-, - (CH₂)-, -(CH₂)₂- oder -(CH₂)₃-Gruppe gebunden sein kann und/oder jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyt, tert-Butyl, n-Pentyl, n-Hexyl, -C(=O)-O-CH₃, -C(=O)-O-C₂H₅, -C(=O)-O-CH(CH₃)₂, -C(=O)-O-C(CH₃)₃ substituiert sein kann;
oder für einen Rest ausgewählt aus der Gruppe bestehend aus -(CH₂)-Pyridinyl, -(CH₂)₂-Pyridinyl, Benzyl, Phenethyl, Phenyl, Naphthyl, Thiophenyl, Furanyl, Pyrrolyl und Pyridinyl, wobei der Rest jeweils ggf. mit 1, 2, 3, 4 oder 5 Substituenten unabhängig voneinander ausgewählt aus der Gruppe bestehend aus -CF₃, F, Cl, Br, -O-CH₃, -O-C₂H₅, Methyl, Ethyl, n-Propyl, Isopropyl, n-Butyl, sek-Butyl, Isobutyl und tert-Butyl substituiert sein kann, stehen;
oder
R^{12f} und R^{13f} jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazoto[5,4-c]pyridinyl, Pyrrolidinyl, Piperidinyl, (1,2,3,6)-Tetrahydropyridinyl, Piperazinyl, Morpholinyl, Azepanyl, Diazepanyl und Thiomorpholinyl bilden, der ggf. jeweils mit 1 oder 2 Substituenten unabhängig voneinander ausgewählt aus der Gruppe bestehend aus -CH₂-O-CH₂-Oxetanyl, -O-CH₂-Oxetanyl, -CH₂-OH, -CH₂-CH₂-OH, =CH₂, -C(=O)-O-CH₃, - C(=C-O-C₂H₅, -C(=O)-O-C(CH₃)₃, -C(=O)-CH₃, -C(=O)-C₂H₅, -C(=O)-C(CH₃)₃, -CN, -CH₂N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂O-CH₂-CH₃, -CH₂-O-CH₃, -NH₂, -NH-CH₃, -NH-C₂H₅, -N(CH₃)₂, -N(C₂H₅)₂, -NH-Phenyl, -N(CH₃)-Phenyl, -N(C₂H₅)Phenyl, Oxo (=O), Thioxo (=S), -OH, F, Cl. Br, -CF₃, -O-CH₃, -O-C₂H₅, -O-C(CH₃)₃, -O-CH(CH₃)₂, -O-CH₂-CH₂-CH₂-CH₃, Methyl, Ethyl, Isopropyl, n-Propyl, n-Butyl, tert-Butyl, sek-Butyl, Piperidinyl, Pymolidinyl, -O-Phenyl, -O-C(=O)-CH₃, -O-C(=O)-C₂H₅, -O-C(=O)-C(CH₃)₃, -(CH₂)-Pyridinyl, Cyclohexyl, Cyclopentyl, (4,5)-Dihydroisoxazolyl, Thiazolyl, (1,2,5)-Thiadiazolyl, Thiophenyl, Phenethyl, Pyridinyl, Phenyl und Benzyl substituiert sein kann, wobei jeweils der zyklische Teil der Reste Oxetanyl, -N-[C(=O)-C₂H₅]-Phenyl, -N-[C(=O)-CH₃]-Phenyl, (4,5)-Dihydroisoxazolyl, Thiazolyt, (1,2,5)-Thiadiazolyl, Thiophenyl, Phenethyl, -NH-Phenyl, -N(CH₃)-Phenyl, -N(C₂H₅)-Phenyl, -(CH₂)-Pyridinyl, Pyridinyl, Phenyl, -O-Phenyl und Benzyl ggf. mit 1, 2, 3, 4 oder 5 Substituenten unabhängig voneinander ausgewählt aus der Gruppe bestehend aus -CF₃, - OH, -O-CH₃, -O-C₂H₅, F, Cl, Br, Methyl, Ethyl, Isopropyl, n-Propyl, n-Butyl, tert-Butyl und sek-Butyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

22. Verbindungen gemäß einem oder mehreren der Anspruche 1 bis 21 ausgewählt aus der Gruppe bestehend aus
3 2-(3-Fluor-4-(methylsulfonylamino)phenyl)-N-((-piperidin-1-yl)-4-(trifluormethyl)benzyl)propanamid
30 N-(2-Cyano-4-(trifluormethyl)benzyl)-2-(3-fluor-4-(methylsulfonylamino)phenyl)propanamid
31 (S)-2-(3-fluor-4-(methylsulfonylamino)phenyl)-N-(2-(piperidin-1-yl)-4-(trifluormethyl)benzyl)propanamid
32 (R)-2-(3-Fluor-4-(methylsulfonylamino)phenyl)-N-(2-(plperidin-1-yl)-4-(trifluormethyl)benzyl)propanamid
33 2-(3-Fluor-4-(methylsulfonylamino)phenyl)-N-(2-morpholino-4-(trifluormethyl)benzyl)propanamid
34 2-(3-fluor-4-(methylsulfonylamino)phenyl)-N-(2-(pyrrolidin-1-yl)-4-(trifluormethyl)benzyl)propanamid
35 N-(2-(Dimethylamino)-4-(trifluormethyl)benzyl)-2-(3-fluor-4-(methylsulfonylamino)phenyl)propanamid
36 N-(2-(Diethylamino)-4-(trifluormethyl)benzyl)-2-(3-fluor-4-(methylsulfonylamino)phenyl)propanamid
37 N-(2-(Dipropylamino)-4-(trifluormethyl)benzyl)-2-(3-fluor-4-(methylsulfonylamino)phenyl)propanamid
38 2-(3-Fluor-4-(methylsulfonylamino)phenyl)-N-(2-hydroxy-4-(trifluormethyl)benzyl)propanamid
39 2-(3-Fluor4-(methylsulfonylamino)phenyl)-N-(2-methoxy-4-(trifluormethyl)benzyl)propanamid
40 N-(2-Butoxy-4-(trifluormethyl)benzyl)-2-(3-fluor-4-(methylsulfonylamino)phenyl)propanamid
41 2-(3-Fluor-4-(methylsulfonylamino)phenyl)-N-(2-sopropoxy-4-(trifluormethyl)benzyl)propanamid
42 N-(2-(Cyclopentyloxy)-4-(trifluormethyl)benzyl)-2-(3-fluor-4-(methylsulfonylamino)phenyl)propanamid
43 2-(3-Fluor-4-(methytsulfonylamino)phenyl)-N-((5-(trifluormethyl)biphenyl-2-yl)methyl)propanamid
44 2-(3-fluor-4-(methylsulfonylamino)phenyl)-N-((4'-fluor-5-(trifluormethy)biphenyl-2-yl)methyl)propanamid
45 2-(3-Fluor-4-(methylsulfonylamino)phenyl)-N-(2-(pyridin-2-yl)-4-(trifluormethyl)benzyl)propanamid
46 2-(3-Fluor-4-(methylsulfonylamino)phenyl)-N-(2-(pyridin-3-yl)-4-(trifluormethyl)benzyl)propanamid
47 2-(3-Fluor-4-(methytsutfonylamino)phenyl)-N-(2-(pyrimidin-2-yl)-4-(triftuormethyl)benzyl)propanamid
48 2-(3-Fluor-4-(methylsulfonylamino)phenyl)-N-(2-(thiazol-2-yl)-4-(trifluormethyl)benzyl)propanamid
49 2-(3-Fluor-4-(methylsulfonylamino)phenyl)-N-(2-(oxazol-2-yl)-4-(trifluormethyl)benzyl)propanamid
50 N-(2-(1H-Imidazol-2-yl)-4-(trifluormethyl)benzyl)-2-(3-fluor-4-(methylsulfonylamino)phenyl)propanamid
58 2-(3-fluor-4-(methylsulfonylamino)phenyl)-N-((2-piperidin-1-yl)-4-(trifluormethyl)phenyl)propanamid
60 2-(3-Fluor-4-(methylsulfonylamino)phenyl)-N-(2-(piperidin-1-yl)-4-(trifluormethyl)phenethyl)propanamid
61 N-(2-Amino-4-(trifluormethyl)benzyl)-2-(3-fluor-4-(methylsulfonylamino)phenyl)propanamid
62 2-(3-Fluor-4-(methylsulfonylamino)phenyl)-N-(2-nitro-4-(trifluormethyl)benzyl)propanamid
63 N-(4-tert-Butyl-2-(piperidin-1-yl)benzyl)-2-(fluor-4-(methylsulfonylamino)phenyl)propanamid
66 2-(3-Chlor-4-(methylsulfonylamino)phenyl)-N-(2-(piperidin-1-yl)-4-(trifluormethyl)benzyl)propanamid
68 2-(3-Brom-4-(methylsulfonylamino)phenyl)-N-(2-(piperidin-1-yl)-4-(trifluormethyl)benzyl)propanamid
69 2-(3-Brom-4-(methylsulfonylamino)phenyl)-N-(2-(pyrrolidin-1-yl)-4-(trifluormethyl)benzyl)propanamid
70 N-(4-tert-Butyl-2-cyanobenzyl)-2-(3-fluor-4-(methylsulfonylamino)phenyl)propanamid
71 N-((6-(Chlordiflourmethy)-2-(pipendin-1-yl)pyridin-3-yl)methyl)-2-(3-fluor-(4-methylsulfonylamino)phenyl)propanamid
82 - 2-(3-Fluor-4-(methylsulfonylamino)phenyl)-N-(2-(4-methylpiperidin-1-yl)-4-(trifuormethyl)benzyl)propanamid;
83 (S)-2-(3-Fluor-4-methansulfonylamino-phenyl)-N-[2-(4-methyl-piperidin-1-yl)-4-trifluormethyl-benzyl]-propionamid
84 (R)-2-(3-Fluor-4-methansulfonylamino-phenyl)-N-[2-(4-methyl-piperidin-1-yl)-4-trifluormethyl-benzyl]-propionamid
98 2-(3-Fluor-4-methansulfonylamino-phenyl)-N-(2-isobutoxy-4-trifluormethyl-benzyl)-propionamid
99 (R)-2-(3-Fluor-4-methansulfonylamino-phenyl)-N-(2-isobutoxy-4-trifluormethyl-benzyl)-propionamid
108 N-(2-Butoxy-4-tert-butyl-benzyl)-2-(3-fluor-4-methansulfonylamino-phenyl)-propionamid
114 2-(3-Fluor-4-methansulfonylamino-phenyl)-N-(2-fluor-4-trifluormethyl-benzyl)-propionamid
133 N-(4-tert-Butyl-2-isobutoxy-benzyl)-2-(3-fluor-4-methansulfonylamino-phenyl)-propionamid
152 N-[4-tert-Butyl-2-(2,2-dimethyl-propoxy)benzyl]-2-(3-fluor-4-methansulfonylamino-phenyl)-propionamid
153 N-(4-tert-Butyl-2-pentyloxy-benzyl)-2-(3-fluor-4-methansulfonylamino-phenyl)-propionamid
154 N-(4-tert-Butyl-2-cyclohexyloxy-benzyl)-2-(3-fluor-4-methansulfonylamino-phenyl)-propionamid
155 N-(4-tert-Butyl-2-cyclopentyloxy-benzyl)-2-(3-fluor-4-methansulfonylamino-phenyl)-propionamid
174 N-(2-Azepan-1-yl-4-trifluormethyl-benzyl)-2-(3-fluor-4-methansulfonylamino-phenyl)-propionamid
206 2-(3-Fluor-4-methansulfonylamino-phenyl)-N-[4-trifuormethyl-2-(4-trifluormethyl-benzyloxy)-benzyl]-propionamid
209 N-(2-Benzyloxy-4-trifluormethyl-benzyl)-2-(3-fluor-4-methansulfonylamino-phenyl)-propionamid
216 2-(3-Fluor-4-methansulfonylamino-phenyl)-N-[2-(4-phenyl-piperazin-1-yl)-4-trifluormethyl-benzyl]-propionamid
217 N-(2-Azocan-1-yl-4-trifluormethyl-benzyl)-2-(3-fluor-4-methansulfonylamino-phenyl)-propionamid
218 N-[2-(4,4-Dimethyl-piperidin-1-yl)-4-trifluomethyl-benzyl]-2-(3-fluor-4-methansulfonylamino-phenyl)-propionamid
229 N-[2-(4-Ethyl-piperdin-1-yl)-4-trifluormethyl-benzyl]-2-(3-fluor-4-methansulfonylamino-phenyl)-propionamid
230 2-(3-fluor-4-methansulfonylamino-phenyl)-N-[4-trifluormethyl-2-(4-trifluormethyl-piperidin-1-yl)-benzyl]-propionamid
231 N-[2-(4-Benzyl-piperidin-1-yl)-4-trifluormethyl-benzyl]-2-(3-fluor-4-methansulfonylamino-phenyl)-propionamid
238 N-(2-Cyclohexytoxy-4-trifluormethyl-benzyl)-2-(3-fluor-4-methansulfonylamino-phenyl)-propionamid
239 (R)-N-(2-Cyclohexyloxy-4-trifluormethyl-benzyl)-2-(3-fluor-4-methansulfonylamino-phenyl)-propionamid
246 2-(3-Fluor-4-methansulfonylamino-phenyl)-N-[2-(4-p-tolyl-piperazin-1-yl)-4-trifluormethyl-benzyl]-propionamid
247 2-(3-Fluor-4-methansulfonylarnino-phenyl)-N-[2-(4-m-tolyl-piperazin-1-yl)-4-trifluormethyl-benryl]-propionamid
248 2-(3-Fluor-4-methansulfonylamino-phenyl)-N-{4-trifluormethyl-2-[4-(4-trifluormethylphenyl)-piperazin-1-yl]-benzyl}-propionamid
249 2-(3-Fluor-4-methansulfonylamino-phenyl)-N-{2-[4-(4-methoxy-phenyl)-piperazin-1-yl]-4-trifluormethyl-benryl}-propionamid
255 2-(3-Fluor-4-methansulfonylamino-phenyl)-N-{2-[4-(4-fluor-phenyl)-piperldln-1-yl]-4-trifluormethyl-benzyl}-proplonamid
319 N-[2-(4-Cydohexyl-piperazin-1-y)-4-trifluormethy-benzyl]-2-(3-fluor-4-methansulfonylamino-phenyl)-propionamid
320 N-(4'-tert-Butyl-5-trifluormethyl-biphenyl-2-ylmethyl)-2-(3-fluor-4-methansulfonylamino-phenyl)-propionamid
321 2-(3-Fluor-4-methansulfonylamino-phenyl)-N-(4'-methoxy-5-trifluormethyl-biphenyl-2-ylmethyl)-propionamid
322 N-(3'-Chlor-5-trifluormethyl-biphenyl-2-ylmethyl)-2-(3-fluor-4-methansulfonylamino-phenyl)-propionamid
323 2-(3-Fluor-4-methansulfonylamino-phenyl)-N-(3'-fluor-5-trifluormethyl-biphenyl-2-ylmethyl)-propionamid
324 N-(3'-Chlor-4'-fluor-5-trifluormethyl-biphenyl-2-ytmethyl)-2-(3-fluor-4-methansulfonylamino-phenyl)-propionamid
325 N-(3',4'-Dimethoxy-5-trifluormethyl-biphenyl-2-ylmethyl)-2-(3-fluor-4-methansufonylamino-phenyl)-propionamid
350. N-(3',5'-Dimethoxy-5-trifluormethyl-biphenyl-2-ylmethyl)-2-(3-fluor-4-methansulfonylamino-phenyl)-propionamid
jeweils ggf. in Form eines ihrer reinen Stereoisomeren, Insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

23. Verbindungen gemäß einem oder mehreren der Ansprüche 1 bis 22, **dadurch gekennzeichnet, dass** sie im FLIPR-Assay mit CHO K1-Zellen, die mit dem menschlichen VR1-Gen transfiziert wurden, in einer Konzentration kleiner 2000 nM, bevorzugt kleiner 1000 nM, besonders bevorzugt kleiner 300 nM, ganz besonders bevorzugt kleiner 100 nM, noch weiter bevorzugt kleiner 75 nM, darüber hinaus bevorzugt kleiner 50 nM, am weitesten bevorzugt kleiner 10 nM, eine 50-prozentige Verdrängung von Capsaicin bewirken, dass in einer Konzentration von 100 nM vorliegt.

24. Verfahren zur Herstellung einer Verbindung gemäß einem oder mehreren der Ansprüche 1 bis 23, **dadurch gekennzeichnet, dass** wenigstens eine Verbindung der allgemeinen Formel II, 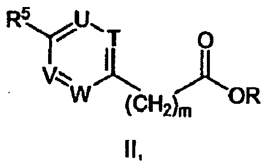 worin R⁵, U, T, V und W die Bedeutung gemäß einem oder mehreren der Ansprüche 1 bis, 23 haben, m für 0, 1, 2 oder 3 steht und R für Wasserstoff oder für einen C₁₋₆-Alkyl-Rest steht, in einem Reaktionsmedium, in Gegenwart wenigstens eines Reduktionsmittels, vorzugsweise in Gegenwart wenigstens eines Reduktionsmittels ausgewählt aus der Gruppe bestehend aus Natriumhydrid, Natrium, Kaliumhydrid, Lithiumaluminiumhydrid, Natriumborhydrid und Di(isobutyl)aluminiumhydrid
zu wenigstens einer Verbindung der allgemeinen Formel III, 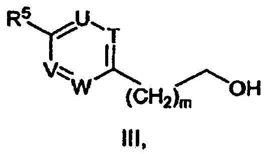 worin R⁵, U, T, V und W die Bedeutung gemäß einem oder mehreren der Ansprüche 1 bis 23 haben und m für 0, 1, 2 oder 3 steht, umgesetzt wird und diese ggf. gereinigt und/oder isoliert wird,
und wenigstens eine Verbindung der allgemeinen Formel 111 in einem Reaktionsmedium in Gegenwart von Diphenylphosphorylazid oder in Gegenwart von HN₃ zu wenigstens einer Verbindung der allgemeinen Formel IV, 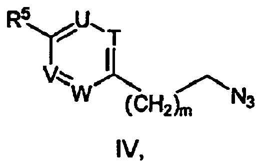 worin R⁵, U, T, V und W die Bedeutung gemäß einem oder mehreren der Ansprüche 1 bis 23 haben und m für 0, 1, 2 oder 3 steht, umgesetzt wird und diese ggf. gereinigt und/oder isoliert wird,
und wenigstens eine Verbindung der allgemeinen Formel IV in einem Reaktionsmedium in Gegenwart wenigstens eines Reduktionsmittels, vorzugsweise in Gegenwart wenigstens eines Reduktionsmittels ausgewählt aus der Gruppe bestehend aus Natriumhydrid, Kaliumhydrid, Lithiumatuminiumhydrid, Natriumborhydrid und Di(isobutyl)aluminiumhydrid oder in einem Reaktionsmedium in Gegenwart eines Katalysators, vorzugsweise in Gegenwart eines Katalysators basierend auf Platin oder
Palladium, besonders bevorzugt in Gegenwart von Palladium auf Kohle, und in Gegenwart von Wasserstoff oder in Gegenwart von Hydrazin
oder in einem Reaktionsmedium in Gegenwart von Triphenylphosphin zu wenigstens einer Verbindung der allgemeinen Formel V, 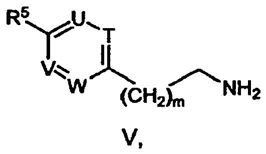 worin R⁵, U, T, V und W die Bedeutung gemäß einem oder mehreren der Ansprüche 1 bis 23 haben und m für 0, 1, 2 oder 3 steht, umgesetzt wird und diese ggf. gereinigt und/oder Isoliert wird,
oder wenigstens eine Verbindung der allgemeinen Formel VI, 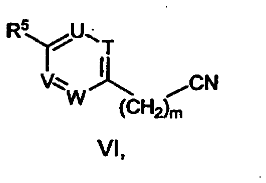 worin R⁵, U, T, V und W die Bedeutung gemäß einem oder mehreren der Ansprüche 1 bis 23 haben und m für 0, 1, 2 oder 3 steht, In einem Reaktionsmedium
in Gegenwart wenigstens eines Katalysators, vorzugsweise in Gegenwart wenigstens eines Katalysators basierend auf Palladium oder Platin, besonders bevorzugt in Gegenwart von Palladium auf Kohle, unter einer Wasserstoffatmosphäre, ggf. in Gegenwart wenigstens einer Säure, vorzugsweise in Gegenwart von Salzsäure,
oder in Gegenwart wenigstens eines Reduktionsmittels ausgewählt aus der Gruppe bestehend aus BH₃•S(CH₃)₂, Lithiumaluminiumhydrid und
Natriumborhydrid, ggf. in Gegenwart von NiCl₂,
zu wenigstens einer Verbindung der allgemeinen Formel V, ggf. in Form eines entsprechenden Salzes, vorzugsweise in Form eines entsprechenden Hydrochlorids, umgesetzt wird und diese ggf. gereinigt und/oder isoliert wird,
und wenigstens eine Verbindung der allgemeinen Formel V mit wenigstens einer Verbindung der allgemeinen Formel VII, 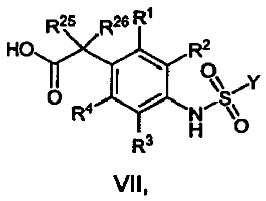 worin Y, R¹, R², R³, R⁴, R²⁵ und R²⁶ die Bedeutung gemäß einem oder mehreren der Ansprüche 1 bis 23 haben, in einem Reaktionsmedium, ggf. in Gegenwart wenigstens eines geeigneten Kopplungsmittels, ggf. in Gegenwart wenigstens einer Base,
oder mit wenigstens einer Verbindung der allgemeinen Formel VIII, 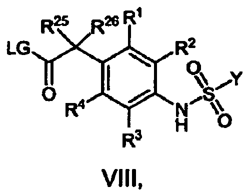 worin Y, R¹ R², R³, R⁴, R²⁵ und R²⁶ die Bedeutung gemäß einem oder mehreren der Ansprüche 1 bis 23 haben und LG für eine Abgangsgruppe, vorzugsweise für ein Chlor- oder Bromatom steht, in einem Reaktionsmedium, ggf. in Gegenwart wenigstens einer Base, zu wenigstens einer Verbindung der allgemeinen Formel Ih, 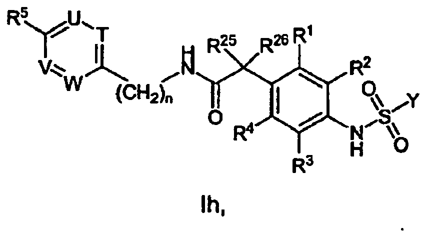 worin Y, T, U, V, W, R¹, R², R³, R⁴, R⁵, R²⁵ und R²⁶, die Bedeutung gemäß einem oder mehreren der Ansprüche 1 bis 23 haben und n für 1, 2, 3 oder 4 steht, umgesetzt wird und diese ggf. gereinigt und/oder isoliert wird,
und ggf. wenigstens eine Verbindung der allgemeinen Formel Ih in einem Reaktionsmedium mit wenigstens einer Verbindung der allgemeinen Formel IX, 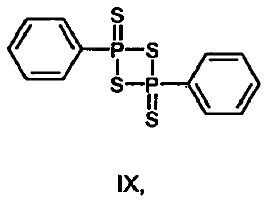 worin die Phenyl-Reste jeweils mit 1 oder 2 Substituenten unabhängig voneinander ausgewählt aus der Gruppe bestehend aus Methoxy, Phenoxy, Cl, Methyl und Br, bevorzugt jeweils mit einem Phenoxy-Rest oder Methoxy Rest, besonders bevorzugt jeweils mit einem Methoxy-Rest in para-Position, substituiert sind, oder mit Phosphorpentasulfid, zu wenigstens einer Verbindung der allgemeinen Formel Ik, 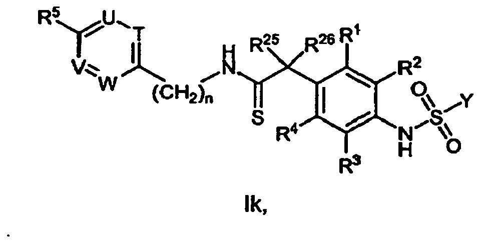 worin Y, T, U, V. W, R¹ R², R³, R⁴, R⁵, R²⁵ und R²⁶, die Bedeutung gemäß einem oder mehreren der Ansprüche 1 bis 23 haben und n für 1, 2, 3 oder 4 steht, umgesetzt wird und diese ggf. gereinigt und/oder isoliert wird.

25. Verfahren zur Herstellung wenigstens einer Verbindung gemäß einem oder mehreren der Ansprüche 1 bis 23, **dadurch gekennzeichnet, dass** wenigstens eine Verbindung der allgemeinen Formel X, 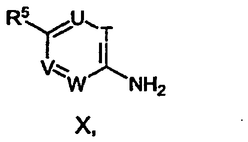 worin R⁵, U, T, V und W die Bedeutung gemäß einem oder mehreren der Anspruche 1 bis 23 haben, mit wenigstens einer Verbindung der allgemeinen Formel VII, 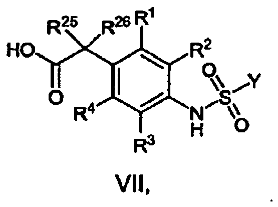 worin Y, R¹, R², R³, R⁴, R²⁵ und R²⁶ die Bedeutung gemäß einem oder mehreren der Ansprüche 1 bis 23 haben, in einem Reaktionsmedium, ggf. in Gegenwart wenigstens eines geeigneten Kopplungsmittels, ggf. in Gegenwart wenigstens einer Base,
oder mit wenigstens einer Verbindung der allgemeinen Formel VIII, 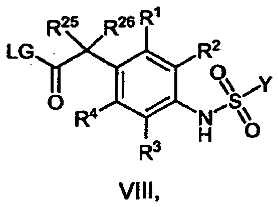 worin Y, R¹, R², R³, R⁴, R²⁵ und R²⁶ die Bedeutung gemäß einem oder mehreren der Ansprüche 1 bis 23 haben und LG für eine Abgangsgruppe, vorzugsweise für ein Chlor- oder Bromatom steht, in einem Reaktionsmedium, ggf. in Gegenwart wenigstens einer Base, zu wenigstens einer Verbindung der allgemeinen Formel Im, 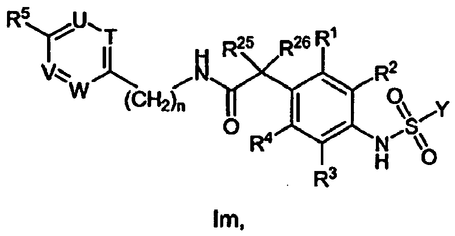 worin Y, T, U, V, W, R¹, R², R³, R⁴, R⁵, R²⁵ und R²⁶ die Bedeutung gemäß einem oder mehreren der Ansprüche 1 bis 23 haben, umgesetzt wird und diese ggf. gereinigt und/oder isoliert wird,
und ggf. wenigstens eine Verbindung der allgemeinen Formel Im in einem Reaktionsmedium mit wenigstens einer Verbindung der allgemeinen Formel IX, 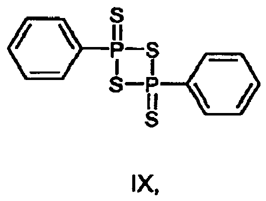 worin die Phenyl-Reste jeweils mit 1 oder 2 Substituenten unabhängig voneinander ausgewählt aus der Gruppe bestehend aus Methoxy, Phenoxy, Cl, Methyl und Br, bevorzugt jeweils mit einem Phenoxy-Rest oder Methoxy-Rest, besonders bevorzugt jeweils mit einem Methoxy-Rest in para-Position, substituiert sind, oder mit Phosphorpentasulfid, zu wenigstens einer Verbindung der allgemeinen Formel In, 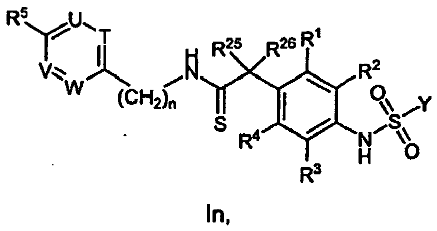 worin Y, T, U, V, W, R¹, R², R³, R⁴, R⁵, R²⁵ und R²⁶ die Bedeutung gemäß einem oder mehreren der Ansprüche 1 bis 23 haben, umgesetzt wird und diese ggf. gereinigt und/oder isoliert wird.

26. Arzneimittel enthaltend wenigstens eine Verbindung gemäß einem oder mehreren der Ansprüche 1 bis 23 und ggf. einen oder mehrere physiologisch verträgliche Hilfsstoffe.

27. Arzneimittel gemäß Anspruch 26 zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Hyperalgesie; Allodynie; Kausalgie und Migräne.

28. Arzneimittel gemäß Anspruch 26 zur Behandlung und/oder Prophylaxe von einer oder mehreren Erkrankungen ausgewählt aus der Gruppe bestehend aus Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; und Epilepsie.

29. Arzneimittel gemäß Anspruch 26 zur Behandlung und/oder Prophylaxe von einer oder mehreren Erkrankungen ausgewählt aus der Gruppe bestehend aus Atemwegserkrankungen, vorzugsweise ausgewählt aus der Gruppe
bestehend aus Asthma, Bronchitis und Lungenentzündung; Husten; Harninkontinenz; einer überaktiven Blase (overactive bladder, OAB); Erkrankungen und/oder Verletzungen des Magen-Darm-Trakts; Zwötffingerdarmgeschwüren; Magengeschwüren; Reizdarmsyndrom; Schlaganfällen; Augenreizungen; Hautreizungen; neurotischen Hauterkrankungen; allergischen Hautkrankheiten; Psiorasis; Vltiligo; Herpes simplex; Entzündungen, vorzugsweise Entzündungen des Darmes, der Augen, der Blase, der Haut oder der Nasenschleimhaut, Diarrhöe; Pruritus; Osteoporose; Arthritis; Osteoarthritis, rheumatischen Erkrankungen; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit, zur Diurese; zur Antinatriurese; zur Beeinflussung des kardiovaskulären Systems; zur Vigilanzsteigerung; zur Behandlung von Wunden und/oder Verbrennungen; zur Behandlung von durchtrennten Nerven; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanli, Arvanil, SDZ-249665, SDZ-249482, Nuvanil und Capsavanil.

30. Verwendung wenigstens einer Verbindung gemäß einem oder mehreren der Ansprüche 1 bis 26 zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz,
neuropathischem Schmerz und visceralem Schmerz; Gelenkschmerz; Hyperalgesie; Allodynie; Kausalgie und Migräne.

31. Verwendung wenigstens einer Verbindung gemäß einem oder mehreren der Ansprüche 1 bis 26 zur Herstellung eines Arzneimittels zur Behandlung und/oder Prophylaxe von einer oder mehreren Erkrankungen ausgewählt aus der Gruppe bestehend aus Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; und Epilepsie.

32. Verwendung wenigstens einer Verbindung gemäß einem oder mehreren der Ansprüche 1 bis 26 zur Herstellung eines Arzneimittels zur Behandlung und/oder Prophylaxe von einer oder mehreren Erkrankungen ausgewählt aus der Gruppe bestehend aus Atemwegserkrankungen, vorzugsweise ausgewählt aus der Gruppe bestehend aus Asthma, Bronchitis und Lungenentzündung; Husten; Harninkontinenz; einer überaktiven Blase (overactive bladder, OAB); Erkrankungen und/oder Verletzungen des Magen-Darm-Trakts; Zwölffingerdarmgeschwüren; Magengeschwüren; Reizdarmsyndrom; Schlaganfällen; Augenreizungen; Hautreizungen; neurotischen Hauterkrankungen; allergischen Hautkrankheiten; Psiorasis; Vitiligo, Herpes simplex; Entzündungen, vorzugsweise Entzündungen des Darmes, der Augen, der Blase, der Haut oder der Nasenschleimhaut; Diarrhöe; Pruritus; Osteoporose; Arthritis; Osteoarthritis; rheumatischen Erkrankungen; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opiolden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeft; zur Diurese; zur Antinatriurese; zur Beeinflussung des kardiovaskulären Systems; zur
Vigilanzsteigerung; zur Behandlung von Wunden und/oder Verbrennungen; zur Behandlung von durchtrennten Nerven; zur Libidosteigerung; zur Modulation der Bewegungsaktivität; zur Anxlolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.
